(19)

Europäisches
Patentamt
European
Patent Office
Office européen
des brevets

(11)  **EP 2 302 073 A1**

(12)  **EUROPEAN PATENT APPLICATION**

(43) Date of publication:
　　　**30.03.2011  Bulletin 2011/13**

(51) Int Cl.:
　　　*C12Q 1/68* (2006.01)

(21) Application number: **10174924.0**

(22) Date of filing: **18.07.2005**

(84) Designated Contracting States:
　　　**AT BE BG CH CY CZ DE DK EE ES FI FR GB GR
　　　HU IE IS IT LI LT LU LV MC NL PL PT RO SE SI
　　　SK TR**

(30) Priority:　**18.07.2004  EP 04016926**

(62) Document number(s) of the earlier application(s) in
　　　accordance with Art. 76 EPC:
　　　**08009974.0 / 1 961 827
　　　05768093.6 / 1 769 086**

(27) Previously filed application:
　　　**18.07.2005 EP 08009974**

(71) Applicant: **Epigenomics AG
　　　10178 Berlin (DE)**

(72) Inventors:
　　　• **Lesche, Ralf
　　　　13156 Berlin (DE)**
　　　• **Fassbender, Anne
　　　　10243 Berlin (DE)**
　　　• **Jünemann, Klaus
　　　　22609 Hamburg (DE)**
　　　• **Foekens, John
　　　　2908 XA Rotterdam (Capelle a/d Ijssel) (NL)**
　　　• **Martens, John
　　　　3033 BK Rotterdam (NL)**

Remarks:
　　　•The complete document including Reference Tables
　　　and the Sequence Listing can be downloaded from
　　　the EPO website
　　　•This application was filed on 01-09-2010 as a
　　　divisional application to the application mentioned
　　　under INID code 62.

(54)　　**Epigenetic methods and nucleic acids for the detection of breast cell proliferative disorders**

(57)　　The present application provides methods and nucleic acids for the detection and differentiation of breast cell proliferative disorders. This is achieved by the analysis of the methylation of a panel of genes, or subsets thereof. The invention may be used for the detection and/or differentiation of a variety of tissue types including breast cancer and benign breast disorders as well as other cancers and tissue types.

**EP 2 302 073 A1**

**Description**

FIELD OF THE INVENTION

The present invention relates to genomic DNA sequences that exhibit altered CpG methylation patterns in disease states relative to normal. Particular embodiments provide methods, nucleic acids, nucleic acid arrays and kits useful for detecting, or for detecting and differentiating between or among breast cell proliferative disorders.

BACKGROUND

The etiology of pathogenic states is known to involve modified methylation patterns of individual genes or of the genome. 5-methylcytosine, in the context of CpG dinucleotide sequences, is the most frequent covalently modified base in the DNA of eukaryotic cells, and plays a role in the regulation of transcription, genetic imprinting, and tumorigenesis. The identification and quantification of 5-methylcytosine sites in a specific specimen, or between or among a plurality of specimens, is thus of considerable interest, not only in research, but particularly for the molecular diagnoses of various diseases.

*Correlation of aberrant DNA methylation with cancer.* Aberrant DNA methylation within CpG 'islands' is characterized by *hyper-* or *hypomethylation* of CpG dinucleotide sequences leading to abrogation or over-expression of a broad spectrum of genes, and is among the earliest and most common alterations found in, and correlated with human malignancies. Additionally, abnormal methylation has been shown to occur in CpG-rich regulatory elements in intronic and coding parts of genes for certain tumors. In colon cancer, for example, aberrant DNA methylation constitutes one of the most prominent alterations and inactivates many tumor suppressor genes such as p14ARF, p16INK4a, THBS1, MINT2, and MINT31 and DNA mismatch repair genes such as hMLH1.

In contrast to the specific hypermethylation of tumor suppressor genes, an overall hypomethylation of DNA can be observed in tumor cells. This decrease in global methylation can be detected early, far before the development of frank tumor formation. A correlation between hypomethylation and increased gene expression has been determined for many oncogenes.

*Breast cancer.* In American women, breast cancer is the most frequently diagnosed cancer. 1 out of 8 women will develop breast cancer during her life time. Breast cancer is the second leading cause of cancer death and in women aged 40-55, breast cancer is the leading cause of death. For 2004, 215990 new cases of breast cancer and 40110 deaths are estimated in the US alone with comparable numbers in Europe.

*Breast cancer classification.*

*1. Breast cancer*

The vast majority of breast neoplasms are of epithelial origin with ductal carcinomas representing 80% of all tumors. In addition, there are several breast cancer subtypes that are less common. Medullary and lobular carcinomas are both found in about 5% of patients diagnosed with breast cancer. Other less frequent tumor histologies include pure tubular carcinoma, mucinous or colloid carcinoma, papillary carcinoma, and Paget's disease. These cancers have substantially better prognoses, especially when found in a node-negative stage. Carcinocarcinomas and adenocystic tumors occur only sporadically.

Ductal carcinoma in situ (DCIS) is a noninvasive, precancerous condition. DCIS can progress to become invasive cancer, but estimates of the likelihood of this vary widely. Some people include DCIS in breast cancer statistics. The frequency of the diagnosis of DCIS has increased markedly in the United States since the widespread use of screening mammography. In 1998, DCIS accounted for about 18% of all newly-diagnosed invasive plus noninvasive breast tumors in the United States. Very few cases of DCIS present as a palpable mass; 80% are diagnosed by mammography alone.

*2.Benign Breast Conditions*

The most common benign breast conditions include fibrocystic breast condition, benign breast tumors, and breast inflammation. Fibrocystic disease is the most frequent benign breast condition affecting every second woman at least once during her life time. Symptoms of fibrocystic breasts in the breast include cysts (accumulated packets of fluid), fibrosis (formation of scar-like connective tissue), lumpiness, areas of thickening, tenderness, or breast pain. Fibrocystic breasts can sometimes make breast cancer more difficult to detect with mammography.

Fibroadenomas are common benign breast tumors often too small to feel by hand, though occasionally, they may grow to be several inches in diameter. Fibroadenomas are made up of both glandular and stromal (connective) breast tissue and usually occur in women between 20-30 years of age. According to the American Cancer Society, African-American women are affected with fibroadenomas more often than women of other racial or ethnic groups. Phyllodes tumors are also benign breast tumors in the glandular and stroma breast tissues but are far less common than fibroadenomas. The difference between phyllodes tumors and fibroadenomas is that there is an overgrowth of the fibro-connective tissue in phyllodes tumors. Phyllodes tumors are usually benign but on very rare occasions, they may be malignant (cancerous) and could metastasize. Granular cell tumors are usually found in the mouth or skin but may rarely be detected in the breast as well. However, granular cell tumors do not indicate higher risk for developing breast cancer. Mastitis is an inflammation of breast tissue that commonly occurs during breast feeding.

*TNM Stage*

The TNM classification was devised by the International Union Against Cancer (UICC) and accepted by the American Joint Commission on Cancer Staging. TNM is based on the clinical features of tumor (T), the regional lymph nodes (N), and the presence or absence of distant metastases (M). The tumor is characterized by its size, so that a T1 is a tumor under 2 cm, a T2 is 2 to 5 cm, and a T3 is over 5 cm. Breast carcinomas in situ are labeled Tis and distinguished in ductal carcinoma in situ (DCIS), lobular carcinoma *in situ* (LCIS) and Paget's disease. Similarly, N0 represents negative or normal regional lymph nodes and M0 absence of distant metastases, respectively. Involvement of the ipsilateral axillary lymph nodes is the most reliable and reproducible prognostic indicator for primary breast cancer. In general, 50 to 70% of patients with positive lymph nodes have a relapse, whereas only 20 to 35% of patients with all lymph nodes negative for metastatic disease have a relapse after loco-regional treatments only.

*Grade*

Tumor grade reflects the differentiation status of the tumor. Breast cancer differentiation is usually described as well (grade 1), moderately (grade 2) or poorly (grade 3) differentiated, respectively. Poorly differentiated tumors tend to be more aggressive. Eighty-six percent of patients having tumors of good nuclear grade survived for 8 years as opposed to 64% in whom the nuclear grade was scored as poor.

*Hormone receptor status*

Hormone receptor levels are important parameters to classify breast cancer both with respect to prognosis as well as for treatment planning. Patients with ER-positive tumor tend to have a more indolent course and to metastasize preferentially to soft tissue and bone; conversely, those with ER-negative tumors relapse earlier, and metastases to liver, lung, and central nervous system are more likely. ER-positive tumors are more often well differentiated and are associated with other favorable prognostic characteristics. Although patients with ER-positive tumors tend to have better short-term disease-free and overall survival rates than do patients with ER-negative tumors, the differences between the two groups tend to diminish or even disappear with time. PsR appeared in some studies to be a more valuable prognostic indicator than the ER. In addition, high levels of estrogen receptor expression are predictors of a favorable response to endocrine therapy.

*Age and menopausal status*

A large study with long follow-up indicated that women 45 to 49 years of age had the best prognosis and that the very young (those under age 35 years) or elderly patients had the worst breast cancer survival. However, when other, more important tumor characteristics are considered, age and menopausal status are not important prognostic indicators.

*Diagnosis and treatment.*

*Diagnosis*

Breast cancer is often diagnosed as a palpable mass by self examination of the patient or during a clinical breast examination by a physician. The increasing use of mammography in breast cancer screening has resulted in many cancers being found already in a stage where no palpable mass is detectable. Suspicious masses are usually followed up by further imaging such as ultra sound or MRI and definitive diagnosis is confirmed by needle biopsy.

*Screening*

The American Cancer Society currently recommends the following guidelines for the detection of breast cancer in women who are asymptomatic:

- Women 20 years of age and older should perform breast self-examination (BSE) every month.

- Women 20-39 should have a physical examination of the breast (CBE or clinical breast exam) at least every three years, performed by health care professional such as a physician, physician assistant, nurse or nurse practitioner. CBE may often be received in the same appointment as a Pap smear.

- Women 20-39 should also perform monthly BSE.

- Women 40 and older should have a physical examination of the breast (CBE or clinical breast exam) every year, performed by a health care professional, such as a physician, physician assistant, nurse or nurse practitioner. CBE can often be performed in the same visit as a mammogram. Monthly BSE should also be performed.

- Women 40 years of age and older should have a screening mammogram every year in addition to annual CBE and monthly BSE.

*3. Breast Self Examination (BSE) and Clinical Breast Examination (CBE)*

In 2001, new analyses were conducted for clinical breast exam and breast self-exam. There is no direct, but some indirect, evidence that CBE decreases breast cancer mortality. CBE has an overall sensitivity of 54%, which varies with the patient's age and size of the mass, as well as the provider's skill in clinical examination. In the absence of direct evidence, the recommendation for CBE was based on consensus opinion. Regarding breast self exam, a meta-analysis of 6 large controlled trials found no reduction in the relative risk of mortality in women performing BSE vs. those not performing BSE (0.94, 95% C.I. 0.83-1.06). Recently it was further questioned whether routine BSE reduces breast cancer mortality and might even harm. Despite this evidence breast self examination (BSE) and clinical breast examination (CBE) are both still part of the current guidelines for breast cancer screening.

*4. Mammography*

Mammography is currently the only exam approved by the U.S. Food and Drug Administration (FDA) to screen for breast cancer in asymptomatic women. Randomly controlled trials conducted in the US and several European countries have demonstrated that routine mammography screening can reduce breast cancer mortality by 20-40% if performed annually. On average the sensitivity of mammography is up to 85%. However, mammography is less sensitive in patients with dense breast or benign breast conditions such as fibrocystic changes or lumps. Five to 10 percent of screening mammogram results are abnormal and require more testing. False positive rates are higher in younger women due to higher density of their breasts.

In the US, mammography screening has been established for the general population. Although mammography involves low dose radiation and discomfort to the tested person, the compliance rate is around 70%. The average charge for screening mammography is $141, $101 technical, $40 for interpretation. Despite the obvious success of mammography, growing difficulty has been reported to recruit trained staff, both doctors and nurses, for mammography clinics resulting in a 8% decline of sites over the last 4 years. It was speculated that this might be due to long hours, low reimbursement, heavy regulation and fear of lawsuits.

*5.MRI screening*

MRI imaging is mainly used to follow up positive mammography results. MRI is very sensitive, is well suited to analyze dense breasts and younger women, but is slower, more expensive, and is more difficult to guide breast biopsies. Currently, trials are ongoing to assess MRI for screening in high risk populations such as carriers of breast cancer susceptibility gene mutations

*Breast cancer therapy.*

Due to current screening programs and the accessibility to self-examination, breast cancer is diagnosed comparatively early: in about 65% of all newly diagnosed cases, the cancer is limited to the breast and has not yet spread to the lymph nodes. Therefore, for most patients diagnosed with organ confined breast cancer the suggested primary therapeutic intervention is surgery often followed by radiation therapy.

Although the tumor can be completely removed by surgery in most early stage breast cancer patients. However, without further therapy, about one third of these will develop metastases during follow-up. It is thought that this is due to occult metastases (or micrometastases) already present at the time of surgery. Based on this observation, systemic adjuvant treatment has been introduced also for node-negative breast cancers. Systemic adjuvant therapy is administered after surgical removal of the tumor, and has been shown to reduce the risk of recurrence significantly (Early Breast Cancer Trialists' Collaborative Group, 1998). Several types of adjuvant treatment are available: endocrine treatment (for hormone receptor positive tumors), different chemotherapy regimens, and novel agents like Herceptin. The treatment regimen for the individual patient is chosen according to guidelines such as St. Gallen or NIH which are based on the pathological classification of the tumor (mainly TNM, grade, ER status).

Based on figures from the American Cancer Society, the prognosis of breast cancer is clearly correlated with cancer stage. The earlier a tumor is detected the better the prognosis for survival. Therefore, breast cancer screening tests that detect cancer at an early result in a reduction of breast cancer mortality.

*Methylation and breast cancer.*

*Epigenetic regulation such as DNA methylation has been established as a frequent and early event in carcinogenesis In particular for breast cancer, the contribution of DNA methylation has been well documented.* Widschwendter and Jones (.Oncogene. 2002 Aug 12; 21(35): 5462-82.*) demonstrated in a recent review that DNA methylation changes have been reported in the context of all relevant steps in breast carcinogenesis including 1) evasion of apoptosis, 2) insensitivity to antigrowth signals, 3) self-sufficiency in growth signals, 4) limitless replicative potential, 5) tissue invasion and metastasis and 6) sustained angiogenesis. Less well documented is the role of DNA methylation in early pre cancerous lesions such as DCIS and in less frequent cancerous lesions such as lobular carcinoma. Fackler et al. showed that 95% of DCIS lesions (N=44) showed hypermethylation of at least one out or 5 genes including RASSF1A, HIN-1, RAR-beta, Cyclin D2 and Twist. The same authors compared frequency of hypermethylation of these genes between ductal and lobular carcinomas and found similar methylation patterns except for TWIST which was found less hypermethylated in lobular carcinomas. Similarly, Lehmann et al. reported very early stage changes in methylation of both RASSF1A and stratifin and found more frequent inactivation of DAPK in lobular carcinomas compared with ductal carcinomas. Early stage methylation changes in DCIS for RASSF1A and stratifin were confirmed by other groups.*

In order to screen an asymptomatic population, candidate molecular markers have to be detectable in remote samples in order to allow for non invasive screening assays. It is well established that DNA from breast tumors can be detected as free nucleic acid in serum and plasma. Alternatively fluids obtained directly from the breast such as nipple aspirate fluid (NAF) or ductal lavage have also been suggested as a body fluid source for molecular testing. However, since the procedures to obtain NAF and ductal lavage have been questioned for their reliability and are uncomfortable for the patient, blood based testing is clearly preferred for screening assays.

Blood based molecular cancer screening assays are faced with the challenge to detect minute amounts of tumor DNA in a background of normal DNA from other tissues. Tumor markers that are based on DNA methylation can be detected using assays that amplify DNA in a methylation specific way such as MSP or HeavyMethyl™. These assays allow highly sensitive detection of few copies of methylated DNA in a background of excess normal DNA

*Multifactorial approach.* Cancer diagnostics has traditionally relied upon the detection of single molecular markers (e.g. gene mutations, elevated PSA levels). Unfortunately, cancer is a disease state in which single markers have typically failed to detect or differentiate many forms of the disease. Thus, assays that recognize only a single marker have been shown to be of limited predictive value, as well be discussed briefly herein. A successful approach currently being pursued in methylation based cancer diagnostics and the screening, diagnosis, and therapeutic monitoring of such diseases is the use of a selection of multiple markers. The multiplexed analytical approach is particularly well suited for cancer diagnostics since cancer is not a simple disease, this multi-factorial "panel" approach is consistent with the heterogeneous nature of cancer, both cytologically and clinically.

Key to the successful implementation of a panel approach to methylation based diagnostic tests is the design and development of optimized panels of markers that can characterize and distinguish disease states. This patent application describes an efficient and unique panel of genes the methylation analysis of one or a combination of the members of the panel enabling the detection of cell proliferative disorders of the prostate with a particularly high sensitivity, specificity and/or predictive value.

*Development of medical tests.* Two key evaluative measures of any medical screening or diagnostic test are its sensitivity and specificity, which measure how well the test performs to accurately detect all affected individuals without exception, and without falsely including individuals who do not have the target disease (predictive value). Historically, many diagnostic tests have been criticized due to poor sensitivity and specificity.

A true positive (TP) result is where the test is positive and the condition is present. A false positive (FP) result is where the test is positive but the condition is not present. A true negative (TN) result is where the test is negative and the condition is not present. A false negative (FN) result is where the test is negative but the condition is not present.

Sensitivity = TP/(TP+FN)
Specificity = TN/(FP+TN)
Predictive value = TP/(TP+FP)

Sensitivity is a measure of a test's ability to correctly detect the target disease in an individual being tested. A test having poor sensitivity produces a high rate of false negatives, i.e., individuals who have the disease but are falsely identified as being free of that particular disease. The potential danger of a false negative is that the diseased individual will remain undiagnosed and untreated for some period of time, during which the disease may progress to a later stage wherein treatments, if any, may be less effective. An example of a test that has low sensitivity is a protein-based blood test for HIV. This type of test exhibits poor sensitivity because it fails to detect the presence of the virus until the disease is well established and the virus has invaded the bloodstream in substantial numbers. In contrast, an example of a test that has high sensitivity is viral-load detection using the polymerase chain reaction (PCR). High sensitivity is achieved because this type of test can detect very small quantities of the virus. High sensitivity is particularly important when the consequences of missing a diagnosis are high.

Specificity, on the other hand, is a measure of a test's ability to identify accurately patients who are free of the disease state. A test having poor specificity produces a high rate of false positives, i.e., individuals who are falsely identified as having the disease. A drawback of false positives is that they force patients to undergo unnecessary medical procedures treatments with their attendant risks, emotional and financial stresses, and which could have adverse effects on the patient's health. A feature of diseases which makes it difficult to develop diagnostic tests with high specificity is that disease mechanisms, particularly in cancer, often involve a plurality of genes and proteins. Additionally, certain proteins may be elevated for reasons unrelated to a disease state. An example of a test that has high specificity is a gene-based test that can detect a p53 mutation. Specificity is important when the cost or risk associated with further diagnostic procedures or further medical intervention are very high.

*Methylation analysis of breast fluids.* The detectability of methylation in bodily fluids of breast cancer patients has been established. Silva et al. (Br J Cancer. 1999 Jun;80(8):1262-4.) described the detection of methylated p16INK4a exon 1 in the plasma of five of eight tested breast cancer patients with p16INK4a exon 1 tumor methylation. The sensitivity of the analysis has since been improved by analyzing a panel of genes. Krassenstein et al. (Clin Cancer Res. 2004 Jan 1;10(1 Pt 1):28-32.) described the analysis of a panel of six genes (GSTP1, RARB2, p16INK4a, p14ARF, RASSF1A and DAPK)in matched tumor and nipple aspirate fluid (herein also referred to as NAF). Using a small sample set (22 tumors, 5 healthy patients and 5 benign breast patients) it was established that at least one gene of the panel was methylated in the tumor, and that this methylation could be detected in the NAF of 18 of the 22 breast cancer.

Although the study by Krassenstein et al. confirms that methylation observed in tumour tissue can be detected in breast derived fluid there are several technical problems associated with providing a bodily fluid based test, some of which were acknowledged but not satisfactorily resolved. Firstly, the markers used by Krassenstein were not specifically methylated in breast cancer, but were general cancer markers methylated in a range of cancers. Therefore, if said panel were to be utilized in a clinical setting it is probable that there would be an increased number of false positives, where methylated DNA from cancers of other tissues (or free-floating DNA therefrom) would be detected.

Although Krassenstein et al. aimed to provide a breast cancer screening test, patient compliance with a procedure such as NAF (or e.g. ductal lavage) would likely be low, except in the most at-risk populations. The preferred sample type for a screening test with high patient compliance would more preferably be blood based. The performance of a panel tested on tissue or NAF is no indicator as to its performance on a blood sample. Therefore, any panel would have to be validated on blood samples in order to establish that the markers were not also methylated in blood.

These issues were both shortly addressed by Evron et al. (Lancet. 2001 Apr 28;357(9265):1335-6.). In this study a gene panel consisting of Cyclin D2, RARB and Twist was selected based on factors including their methylation status in white blood cells. The methylation status of these genes was then analyzed in matched tissue and ductal lavage fluid. The gene panel detected invasive breast cancer in 48 of 50 samples.

The main aim of a screening test is the detection of low grade tumors, such as DCIS, higher grade tumors are easily detected by e.g. self-examination and mammography and are harder to treat. This was not enabled by Krassenstein et al. The sample set used consisted of mostly high grade (2 and 3) tumors. There was only one grade 1 tumor. Therefore it may be concluded the suitability of the panel for the detection of low grade tumors has not been fully established, in particular as it is not possible to confirm if the NAF sample in question tested positive for methylation. Furthermore, it is generally assumed that methylation is a progressive feature of tumorigenesis. One would conclude that it is in the detection of the early stages of tumorigenesis that a highly specifically selected panel of genetic markers as opposed to a panel of markers selected for their methylation status in high grade tumor tissue would be required. The panel investigated by Evron et al. detected only 8 out of 14 cases of DCIS thus confirming that a panel suited to the detection of breast cancers is not necessarily suitable for detection of DCIS.

One can summarize the state of the art in that methylation gene panels for the analysis of breast fluids are known, however, there are deficiencies associated with all said panels. Due to these technical difficulties none of these gene panels fulfill the requirements of a breast cancer screening test, namely that the test is suitable for use in bodily fluids, most preferably blood and that the test be capable of detecting early stage cell proliferative disorders such as DCIS.

The use of a panel of methylation markers consisting *RASSF1A, TWIST, Cyclin D2* and *HIN1* for the differentiation of invasive breast carcinoma from normal breast tissue was recently described by Fackler *et al.* By use of a highly sensitive quantitative multiplexed methylation-specific PCR assay the study detected the presence of promoter hypermethylation of the gene panel in breast cancer (as opposed to normal breast tissue) with a sensitivity of 84% and a specificity of 94%. Therefore, the study is significant in establishing the use of methylation markers for the sensitive detection of breast cancer cells in a background of non-cancerous cells. However, the samples used by Fackler et al., were tissue based (including those extracted by means of ductal lavage), thus it has not been established whether such a sensitivity and specificity would be obtainable using body fluid based samples. Furthermore, the same panel proved unable of detecting ductal carcinoma *in situ.* Therefore, the method is unsuitable for use as an early screening test.

## SUMMARY OF THE INVENTION

The present invention provides novel methods for detecting and/or distinguishing between breast cell proliferative disorders.

The invention achieves solves this longstanding need in the art by providing a panel of genes and genomic sequences according to Table 3, the methylation status of CpG positions of these genes and/or their promoter regions are indicative of breast cell proliferative disorders or features thereof. Preferred selections and combinations of genes are provided, the methylation analysis of which enable the differentiation and detection of various classes of breast cell proliferative disorders, namely:

- Detection of breast cell proliferative disorders.
- Detection of early stage breast cancers, including differentiation from benign breast cell proliferative disorders.
- Differentiation of breast cancers from other cancers
- Detection of breast cancer cells in a background of blood or fluids derived therefrom.
- Differentiation of DCIS from benign breast cell proliferative disorders (including healthy breast tissue.

In order to enable this analysis the invention provides a method for the analysis of biological samples for genomic methylation associated with the development of breast cell proliferative disorders. Said method is characterized in that at least one nucleic acid, or a fragment thereof, from the group consisting of SEQ ID NO: 1 to SEQ ID NO: 118 is/are contacted with a reagent or series of reagents capable of distinguishing between methylated and non methylated CpG dinucleotides within the genomic sequence, or sequences of interest.

The present invention provides a method for ascertaining genetic and/or epigenetic parameters of genomic DNA. The method has utility for the improved diagnosis, differentiation and treatment of breast cell proliferative disorders, more specifically by enabling the improved identification of and differentiation between subclasses of said disorder. The invention presents several improvements over the state of the art. Although methylation assays for the detection of breast cancer in bodily fluids are known there is currently no assay that fulfills the criteria of being validated in blood and capable of detecting DCIS with a suitable accuracy for commercial approval.

The source may be any suitable source, such as cell lines, histological slides, biopsies, paraffin-embedded tissue, bodily fluids, blood plasma, blood serum, whole blood, isolated blood cells, cells isolated from the blood and all possible combinations thereof. It is preferred that said sources of DNA are bodily fluids selected from the group consisting urine, nipple aspirate fluid , lymphatic fluid, ductal lavage fluid, fine needle aspirate, blood plasma, blood serum, whole blood, isolated blood cells, cells isolated from the blood.

Specifically, the present invention provides a method for detecting breast cell proliferative disorders, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of SEQ ID NO:1 to SEQ ID NO:118, said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO:493 to SEQ ID NO:964, and contiguous regions thereof corresponding to the target sequence.

Additional embodiments provide a method for the detection of breast cell proliferative disorders, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO:493 to SEQ ID NO:964, and complements thereof, wherein the treated DNA or the fragment thereof is either amplified to produce an amplificate, or is not amplified; and determining, based on a presence or absence of, or on a property of said amplificate, the methylation state of at least one CpG dinucleotide sequence selected from the group consisting of SEQ ID NO: to SEQ ID NO: 118, or an average, or a value reflecting an average methylation state of a plurality of CpG dinucleotide sequences thereof. Preferably, at least one such hybridizing nucleic acid molecule or peptide nucleic acid molecule is bound to a solid phase. Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a sequence selected from the group consisting of SEQ ID NO:493 to SEQ ID NO:964, and complements thereof; hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO:493 to SEQ ID NO:964, and complements thereof; hybridizing at least one nucleic acid molecule comprising a contiguous sequence at least 9 nucleotides in length that is complementary to, or hybridizes under moderately stringent or stringent conditions to a sequence selected from the group consisting of SEQ ID NO:493 to SEQ ID NO:964, and complements thereof, and extending at least one such hybridized nucleic acid molecule by at least one nucleotide base; and sequencing of the amplificate.

Further embodiments provide a method for the analysis of breast cell proliferative disorders, comprising: obtaining a biological sample having subject genomic DNA; extracting the genomic DNA; contacting the genomic DNA, or a fragment thereof, comprising one or more sequences selected from the group consisting of SEQ ID NO: to SEQ ID NO:118 or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of one or more sequences selected from the group consisting of SEQ ID NO: to SEQ ID NO:118, or an average, or a value reflecting an average methylation state of a plurality of CpG dinucleotide sequences thereof. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within sequences from the group consisting of SEQ ID NO: to SEQ ID NO:118.

## DETAILED DESCRIPTION OF THE INVENTION

<u>Definitions:</u>

The term "Observed/Expected Ratio" ("O/E Ratio") refers to the frequency of CpG dinucleotides within a particular DNA sequence, and corresponds to the [number of CpG sites / (number of C bases x number of G bases)] x band length for each fragment.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG CpG dinucleotide sequences) within a DNA sequence include "unmethylated," "fully-methylated" and "hemi-methylated."

The term "hemi-methylation" or "hemimethylation" refers to the methylation state of a palindromic CpG methylation site, where only a single cytosine in one of the two CpG dinucleotide sequences of the palindromic CpG methylation site is methylated (*e.g.*, 5'-CC$^M$GG-3' (top strand): 3'-GGCC-5' (bottom strand)).

The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity).The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an *increased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a *decreased* presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight™" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo and Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong and Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley and Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (*e.g.*, a head-to-tail composite of SEQ ID NO:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

Overview:

The present invention provides for molecular genetic markers that have novel utility for the analysis of methylation patterns associated with the development of breast cell proliferative disorders. Said markers may be used for detecting or distinguishing between breast cell proliferative disorders, thereby providing improved means for the detection, classification and treatment of said disorders.

*Bisulfite modification of DNA is an art-recognized tool used to assess CpG methylation status.* 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, *e.g.*, PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (*e.g.*, Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek and Walter, Nat Genet. 1997 17:275-6, 1997), subjected to one or more primer extension reactions (Gonzalgo and Jones, Nucleic Acids Res., 25:2529-31, 1997; WO 95/00669; U.S. Patent No. 6,251,594) to analyze individual cytosine positions, or treated by enzymatic digestion (Xiong and Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg and Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 97/46705 and WO 95/15373).

The present invention provides for the use of the bisulfite technique , in combination with one or more methylation assays, for determination of the methylation status of CpG dinucleotide sequences within sequences from the group consisting of SEQ ID NO: 1 to SEQ ID NO: 118. According to the present invention, determination of the methylation status of CpG dinucleotide sequences within sequences from the group consisting of SEQ ID NO: to SEQ ID NO:118 has diagnostic and prognostic utility.

*Methylation Assay Procedures.* Various methylation assay procedures are known in the art, and can be used in conjunction with the present invention. These assays allow for determination of the methylation state of one or a plurality of CpG dinucleotides (*e.g.*, CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, *e.g.*, the method described by Sadri and Hornsby (Nucl. Acids Res. 24:5058-5059, 1996), or COBRA (Combined Bisulfite Restriction Analysis) (Xiong and Laird, Nucleic Acids Res. 25:2532-2534, 1997).

*COBRA.* COBRA analysis is a quantitative methylation assay useful for determining DNA methylation levels at specific gene loci in small amounts of genomic DNA (Xiong and Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89: 1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (*e.g.*, as might be found in a typical COBRA-based kit) for COBRA analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe; and labeled nucleotides. Additionally, bisulfite conversion reagents may include: DNA denaturation buffer; sulfonation buffer; DNA recovery reagents or kits (*e.g.*, precipitation, ultrafiltration, affinity column); desulfonation buffer; and DNA recovery components.

Preferably, assays such as "MethyLightTM" (a fluorescence-based real-time PCR technique) (Eads et al., Cancer Res. 59:2302-2306, 1999), Ms-SNuPE (Methylation-sensitive Single Nucleotide Primer Extension) reactions (Gonzalgo and Jones, Nucleic Acids Res. 25:2529-2531, 1997), methylation-specific PCR ("MSP"; Herman et al., Proc. Natl. Acad. Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight*™. The MethyLight™ assay is a high-throughput quantitative methylation assay that utilizes fluorescence-based real-time PCR (TaqMan™) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; *e.g.*, with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10˚C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (*e.g.*, as might be found in a typical MethyLight™-based kit) for MethyLight™ analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE technique is a quantitative method for assessing methylation differences at specific CpG sites based on bisulfite treatment of DNA, followed by single-nucleotide primer extension (Gonzalgo and Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (*e.g.*, microdissected pathology sections), and it avoids utilization of restriction enzymes for determining the methylation status at CpG sites.

Typical reagents (*e.g.*, as might be found in a typical Ms-SNuPE-based kit) for Ms-SNuPE analysis may include, but are not limited to: PCR primers for specific gene (or bisulfite treated DNA sequence or CpG island); optimized PCR buffers and deoxynucleotides; gel extraction kit; positive control primers; Ms-SNuPE primers for specific gene; reaction buffer (for the Ms-SNuPE reaction); and labeled nucleotides. Additionally, bisulfite conversion reagents may include: DNA denaturation buffer; sulfonation buffer; DNA recovery regents or kit (*e.g.*, precipitation, ultrafiltration, affinity column); desulfonation buffer; and DNA recovery components.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1 % methylated alleles of a given CpG island locus, and can be performed on DNA extracted from paraffin-embedded samples. Typical reagents (*e.g.*, as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

*MCA.* The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (*e.g.*, as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Genomic Sequences according to SEQ ID NO:1 to SEQ ID NO:118, and non-naturally occurring treated variants thereof according to SEQ ID NO:493 to SEQ ID NO:964, were determined to have utility for the detection, classification and/or treatment of breast cell proliferative disorders.

In one embodiment the invention provides a method for detecting and/or for detecting and distinguishing between or among breast cell proliferative disorders in a subject. Said method comprises the following steps

i) contacting genomic DNA isolated from bodily fluids obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence, and

ii) detecting, or detecting and distinguishing between or among breast cell proliferative disorders.

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Body fluids are the preferred source of the DNA; particularly preferred are blood plasma, blood serum, whole blood, isolated blood cells and cells isolated from the blood.

The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

The treated DNA is then analyzed in order to determine the methylation state of one or more target gene sequences (prior to the treatment) associated with the development of breast carcinoma. It is particularly preferred that the target region comprises, or hybridizes under stringent conditions to at least 16 contiguous nucleotides of at least one gene or genomic sequence selected from the group consisting the genes and genomic sequences as listed in Table 3. It is further preferred that the sequences of said genes in Table 3 as described in the accompanying sequence listing are analyzed. The method of analysis may be selected from those known in the art, including those listed herein. Particularly preferred are MethyLight™, MSP and the use of blocking oligonucleotides as will be described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO: 493 to SEQ ID NO: 964 and sequences complementary thereto.

Aberrant methylation, more preferably hypermethylation of one or more genes or genomic sequences taken from those listed in Table 3 are associated with the presence of breast carcinoma. Analysis of one or a plurality of the sequences enables detecting, or detecting and distinguishing between or among breast cell proliferative disorders.

In one embodiment, the method discloses the use of one or more genes or genomic sequences selected from the group consisting of APC, ARH1/NOEY2, BRCA2, CCND2, CDKN1A, CDKN2A, SEQ ID NO:9, DAPK1, SEQ ID NO: 2, EYA4, FHIT, GSTP1, HIC1, IGFBP7, MLH1, PGR, SERPINB5, RARB, SFN, SOD2, TGFBR2, THRB, TIMP3, TP73, NME1, CDH13, THBS1, TMS1/ASC, ESR1, IL6, APAF1, CASP8, SYK, HOXA5, FABP3, RASSF1A, SEQ ID NO:3, RARA, TWIST, ESR2, PLAU, STAT1, SEQ ID NO:4, BRCA1, LOT1, PRSS8, SNCG, TPM1, GPC3, CLDN7, SLC19A1, GJB2, SLIT2, IGSF4, MCT1, HS3ST2, PRDM2, ALX4, SEQ ID NO:5, SEQ ID NO:6, SEQ ID NO:7, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), SASH1, S100A7, BCL11B, SEQ ID NO:51, MGC34831, SEQ ID NO:54, PDLIM1, MSF, SEQ ID NO:14, SEQ ID NO:15, SEQ ID NO:16, SEQ ID NO:17, SEQ ID NO:18, SEQ ID NO:19, PRDM6, RAP2B, NR2E1, PCDH7, DKK3, RTTN, SNAP25, SEQ ID NO:26, GIRK2, SEQ ID NO:28, SEQ ID NO:29, ARL7, SEQ ID NO:31, THH, HOXB13, SEQ ID NO:35, MGC10561, LMX1A, SENP3, GS1, TITF1, SEQ ID NO:42, DDX51, SEQ ID NO:117, SEQ ID NO:45, SEQ ID NO:46, 060279, and SEQ ID NO:48 as markers for the detection of breast cancers.

The use of said genes and/or sequences may be enabled by means of any analysis of the expression of the gene, by means of mRNA expression analysis or protein expression analysis. However, in the most preferred embodiment of the invention, the detection of breast cell proliferative disorders is enabled by means of analysis of the methylation status of said genes or genomic sequences and their promoter or regulatory elements. Methods for the methylation analysis of genes are described herein.

It is also preferred that the expression level of only one gene or genomic sequence from the group consisting of PRDM2, PLAU, GSTP1, SLIT2, CCND2, HOXA5, RASSF1A, HS3ST2, ARH1/NOEY2, SCGB3A1, LIMK-1, SEQ ID NO:6, SEQ ID NO:3, SEQ ID NO:18, SEQ ID NO:7, SEQ ID NO:41, SEQ IDNO:22, SEQ ID NO:46, SEQ ID NO:13, and SEQ ID NO:31 is analyzed. It is particularly preferred that this is carried out by means of methylation analysis.

In one embodiment the method discloses the use of one or more genes or genomic sequences selected from the group consisting of ARH1/NOEY2, CCND2, CDKN1A, CDKN2A, DAPK1, SEQ ID NO:2, EYA4, FHIT, GSTP1, HIC1, IGFBP7, SERPINB5, TERT, TGFBR2, THRB, TIMP3, TP73, NME1, CDH13, THBS1, TMS1/ASC, IL6, APAF1, SYK, HOXA5, FABP3, RASSF1A, SEQ ID NO:3, TWIST, ESR2, PLAU, STAT1, SEQ ID NO:4, LOT1, GPC3, CLDN7, GJB2, SLIT2, IGSF4, MCT1, PRDM2, ALX4, SEQ ID NO:5, SEQ ID NO:6, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), LIM DOMAIN KINASE 1 (EC 2.7.1.37) (LIMK-1), MGC34831, SEQ ID NO:54, MSF, SEQ ID NO:16, SEQ ID NO:17, SEQ ID NO:18, SEQ ID NO:19, PRDM6, NR2E1, PCDH7, RTTN, SNAP25, SEQ ID NO:26, SEQ ID NO:28, ARL7, SEQ ID NO:31, THH, HOXB13, SEQ ID NO:35, MGC10561, LMX1A, SENP3, TITF1, SEQ ID NO:42, DDX51, SEQ ID NO: 45, 060279, SEQ ID NO:48 as markers for the differentiation of breast cancers from other cancers. Said use of the genes and/or sequences may be enabled by means of any analysis of the expression of the gene, by means of mRNA expression analysis or protein expression analysis. However, in the most preferred embodiment of the invention, the detection of breast cell proliferative disorders is enabled by means of analysis of the methylation status of said genes or genomic sequences and their promoter or regulatory elements. Methods for the methylation analysis of genes are described herein.

It is also preferred that the expression level of only one gene or genomic sequence from the group consisting PRDM2, GSTP1, ALX4, HOXA5, PLAU, RASSF1A, IGSF4, SLIT2, DAPK1, CDKN1A, SEQ ID NO:38, SEQ IDNO:35, LIMK-1, SEQ ID NO:39', SEQ ID NO: 10, SEQ ID NO: 26, SEQ ID NO: 8, SEQ IDNO:22, SEQ ID NO:18, and SEQ ID NO:47 is analyzed. It is particularly preferred that this is carried out by means of methylation analysis.

In one embodiment the method discloses the use of one or more genes or genomic sequences selected from the group consisting of APC, ARH1/NOEY2, CCND2, CDH1, CDKN1A, CDKN2A, SEQ ID NO:9, DAPK1, SEQ ID NO: 2, EYA4, FHIT, GSTP1, HIC1, IGFBP7, PGR, SERPINB5, RARB, SFN, SOD2, TERT, TGFBR2, THRB, TIMP3, NME1, CDH13, THBS1, TMS1/ASC, ESR1, IL6, APAF1, CASP8, SYK, HOXA5, FABP3, RASSF1A, SEQ ID NO:3, TWIST, ESR2, PLAU, STAT1, SEQ ID NO:4, BRCA1, LOT1, PRSS8, SNCG, GPC3, CLDN7, SLC19A1, GJB2, SLIT2, IGSF4, MCT1, HS3ST2, PRDM2, ALX4, SEQ ID NO:5, SEQ ID NO:6, SEQ ID NO:7, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), SASH1, S100A7, BCL11B, SEQ ID NO:5 1, MGC34831, SEQ ID NO:54, PDLIM1, MSF, SEQ ID NO:14, SEQ ID NO:15, SEQ ID NO:16, SEQ ID NO:17, SEQ ID NO:18, SEQ ID NO:19, PRDM6, RAP2B, NR2E1, PCDH7, DKK3, RTTN, SNAP25, SEQ ID NO:26, GIRK2, SEQ ID NO:28, SEQ ID NO:29, ARL7, SEQ ID NO:31, THH, HOXB13, SEQ ID NO:35, SEQ ID NO:36, MGC10561, LMX1A, SENP3, GS1, TITF1, SEQ ID NO:42, DDX51, SEQ ID NO:117, SEQ ID NO:46, 060279, and SEQ ID NO:48 as markers for the detection of breast cancer cells within blood or blood derived fluids by differentiation of breast cancer cells from peripheral blood lymphocytes. Said use of the genes and/or sequences may be enabled by means of any analysis of the expression of the gene, by means of mRNA expression analysis or protein expression analysis. However, in the most preferred embodiment of the invention, the detection of breast cell proliferative disorders is enabled by means of analysis of the methylation status of said genes or genomic sequences and their promoter or regulatory elements. Methods for the methylation analysis of genes are described herein.

It is also preferred that the expression level of only one gene or genomic sequence from the group consisting EYA4, PRDM2, SERPINB5, TWIST, STAT1, ALX4, IGFBP7, DAPK1, THBS1, PLAU, SEQ ID NO:20, SEQ ID NO:38, SEQ ID NO: 8, SEQ ID NO:37, SEQ ID NO: 10, SEQ IDNO:35, SEQ ID NO:47, SEQ ID NO:6, LIMK-1 and SEQ ID NO: 46 is analyzed. It is particularly preferred that this is carried out by means of methylation analysis.

In one embodiment the method discloses the use of one or more genes or genomic sequences selected from the group consisting of APC, ARH1/NOEY2, CCND2, CDKN1A, CDKN2A, SEQ ID NO:9, SEQ ID NO:2, EYA4, FHIT, GSTP1, HIC1, IGFBP7, MLH1, PGR, SERPINB5, RARB, SOD2, TERT, TGFBR2, THRB, TIMP3, TP73, CDH13, THBS1, TMS1/ASC, ESR1, APAF1, CASP8, SYK, HOXA5, FABP3, RASSF1A, SEQ ID NO:3, RARA, TWIST, ESR2, PLAU, SEQ ID NO:4, SNCG, SLC19A1, GJB2, SLIT2, IGSF4, MCT1, HS3ST2, PRDM2, SEQ ID NO:6, SEQ ID NO:7, SEQ ID NO:8, SCGB3A1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), SASH1, S100A7, BCL11B, SEQ ID NO:51, MGC34831, SEQ ID NO:54, PDLIM1, MSF, SEQ ID NO:14, SEQ ID NO:16, SEQ ID NO:17, SEQ ID NO:18, SEQ ID NO:19, NR2E1, PCDH7, DKK3, RTTN, SNAP25, GIRK2, SEQ ID NO:28, SEQ ID NO:29, ARL7, SEQ ID NO:31, THH, HOXB13, SEQ ID NO:36, LMX1A, SENP3, TITF1, SEQ ID NO:42, DDX51, SEQ ID NO:117 , SEQ ID NO:46, 060279, and SEQ ID NO:48 as markers for the differentiation of DCIS from benign breast disorders. The term "benign breast disorders" as used herein shall be taken to include healthy breast tissue, fibroadenoma, fibrocystic disease and atypical ductal hyperplasia. Said use of the genes and/or sequences may be enabled by means of any analysis of the expression of the gene, by means of mRNA expression analysis or protein expression analysis. However, in the most preferred embodiment of the invention, the detection of breast cell proliferative disorders is enabled by means of analysis of the methylation status of said genes or genomic sequences and their promoter or regulatory elements. Methods for the methylation analysis of genes are described herein.

It is also preferred that the expression level of only one gene or genomic sequence from the group consisting HS3ST2, SLIT2, RASSF1A, GSTP1, GJB2, IGFBP7, CDH13, ARH1/NOEY2, SCGB3A1, FHIT, SEQ ID NO: 27, LIMK-1, SEQ ID NO:46, SEQ ID NO:3, SEQ ID NO: 117, SEQ ID NO:48, SEQ ID NO:41, SEQ ID NO:4, SEQ ID NO:6, and SEQ ID NO:24 is analyzed. It is particularly preferred that this is carried out by means of methylation analysis.

In one embodiment the method discloses the use of one or more genes or genomic sequences selected from the group consisting of ARH1/NOEY2, CCND2, CDKN1A, CDKN2A, SEQ ID NO:9, DAPK1, SEQ ID NO:2, EYA4, FHIT, GSTP1, HIC1, IGFBP7, SERPINB5, TERT, TGFBR2, THRB, TIMP3, TP73, NME1, CDH13, THBS1, TMS1/ASC, IL6, APAF1, SYK, HOXA5, FABP3, RASSF1A, SEQ ID NO:3, TWIST, ESR2, PLAU, STAT1, SEQ ID NO:4, LOT1, GPC3, CLDN7, GJB2, SLIT2, IGSF4, MCT1, PRDM2, ALX4, SEQ ID NO:5, SEQ ID NO:6, SEQ ID NO:7, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), BCL11B, SEQ ID NO:51, MGC34831, SEQ ID NO:54, PDLIM1, MSF, SEQ ID NO:14, SEQ ID NO:16, SEQ ID NO:17, SEQ ID NO:18, SEQ ID NO:19, PRDM6, NR2E1, PCDH7, DKK3, RTTN, SNAP25, SEQ ID NO:26, GIRK2, SEQ ID NO:28, SEQ ID NO:29, ARL7, SEQ ID NO:31, THH, HOXB13, SEQ ID NO:35, SEQ ID NO:36, MGC10561, LMX1A, SENP3, GS1, TITF1, SEQ ID NO:42, DDX51, SEQ ID NO:117 , SEQ ID NO:45, SEQ ID NO:46, 060279, and SEQ ID NO:48 as markers for the differentiation of breast cancer from benign breast disorders. The term benign breast disorders as used herein shall be taken to include healthy breast tissue, fibroadenoma, fibrocystic disease and atypical ductal hyperplasia. Said use of the genes and/or sequences may be enabled by means of any analysis of the expression of the gene, by means of mRNA expression analysis or protein expression analysis. However, in the most preferred embodiment of the invention, the detection of breast cell proliferative disorders is enabled by means of analysis of the methylation status of said genes or genomic sequences and their promoter or regulatory elements. Methods for the methylation analysis of genes are described herein.

It is also preferred that the expression level of only one gene or genomic sequence from the group consisting SLIT2, HS3ST2, HOXA5, ARH1/NOEY2, IGFBP7, PLAU, CDH13, TIMP3, CCND2, GSTP1, SEQ ID NO: 117, LIMK-1, SEQ ID NO:46, SEQ ID NO:6, SEQ ID NO:7, SEQ ID NO:3, SEQ ID NO:41,SEQ ID NO: 27,SEQ ID NO:31, and SEQ ID NO:4 is analyzed. It is particularly preferred that this is carried out by means of methylation analysis.

Aberrant levels of mRNA expression of the genes, genomic sequences or genes regulated by genomic sequences according to Table 3 are associated with breast cell proliferative disorders. Accordingly, increased or decreased levels of expression of said genes or sequences are associable with the development of breast cancers and other breast cell proliferative disorders.

To detect the presence of mRNA encoding a gene or genomic sequence in a detection system for breast cancer, a sample is obtained from a patient. The sample can be a tissue biopsy sample or a sample of blood, plasma, serum or the like. The sample may be treated to extract the nucleic acids contained therein. The resulting nucleic acid from the sample is subjected to gel electrophoresis or other separation techniques. Detection involves contacting the nucleic acids and in particular the mRNA of the sample with a DNA sequence serving as a probe to form hybrid duplexes. The stringency of hybridization is determined by a number of factors during hybridization and during the washing procedure, including temperature, ionic strength, length of time and concentration of formamide. These factors are outlined in, for example, Sambrook et al. (Molecular Cloning: A Laboratory Manual, 2d ed., 1989). Detection of the resulting duplex is usually accomplished by the use of labeled probes. Alternatively, the probe may be unlabeled, but may be detectable by specific binding with a ligand which is labeled, either directly or indirectly. Suitable labels and methods for labeling probes and ligands are known in the art, and include, for example, radioactive labels which may be incorporated by known methods (e.g., nick translation or kinasing) 'biotin' fluorescent groups, chemiluminescent groups (e.g., dioxetanes, particularly triggered dioxetanes), enzymes, antibodies, and the like.

In order to increase the sensitivity of the detection in a sample of mRNA transcribed from the gene or genomic sequence, the technique of reverse transcription/polymerization chain reaction can be used to amplify cDNA transcribed from the mRNA. The method of reverse transcription /PCR is well known in the art (for example, see Watson and Fleming, supra).

The reverse transcription /PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and EYA4 specific primers. (Belyavsky et al, Nucl Acid Res 17:2919-2932, 1989; Krug and Berger, Methods in Enzymology, Academic Press,N.Y., Vol.152, pp. 316-325, 1987 which are incorporated by reference)

The present invention may also be described in certain embodiments as a kit for use in detecting a breast cell proliferative disorder state through testing of a biological sample. A representative kit may comprise one or more nucleic acid segments that selectively hybridize to the mRNA and a container for each of the one or more nucleic acid segments. In certain embodiments the nucleic acid segments may be combined in a single tube. In further embodiments, the nucleic acid segments may also include a pair of primers for amplifying the target mRNA. Such kits may also include any buffers, solutions, solvents, enzymes, nucleotides, or other components for hybridization, amplification or detection reactions. Preferred kit components include reagents for reverse transcription-PCR, in situ hybridization, Northern analysis and/or RPA

The present invention further provides for methods to detect the presence of the polypeptide encoded by said genes or gene sequences in a sample obtained from a patient.

Aberrant levels of polypeptide expression of the polypeptides encoded by the genes, genomic sequences or genes regulated by genomic sequences of the group consisting all genes and genomic sequences of genomic regions listed in Table 3 are associated with breast carcinoma. Accordingly over or under expression of said polypeptides are associable with the development of breast carcinoma and other breast cell proliferative disorders.

Any method known in the art for detecting proteins can be used. Such methods include, but are not limited to immunodiffusion, immunoelectrophoresis, immunochemical methods, binder-ligand assays, immunohistochemical techniques, agglutination and complement assays. (for example see Basic and Clinical Immunology, Sites and Terr, eds., Appleton and Lange, Norwalk, Conn. pp 217-262, 1991 which is incorporated by reference). Preferred are binder-ligand immunoassay methods including reacting antibodies with an epitope or epitopes and competitively displacing a labeled protein or derivative thereof.

Certain embodiments of the present invention comprise the use of antibodies specific to the polypeptide encoded by the genes or genomic sequences of the group consisting all genes and genomic sequences of genomic regions as listed in Table 3, below.

Such antibodies may be useful for diagnostic and prognostic applications in detecting the disease state, by comparing a patient's levels of breast disease marker expression to expression of the same markers in normal individuals. In certain embodiments production of monoclonal or polyclonal antibodies can be induced by the use of the coded polypeptide as antigene. Such antibodies may in turn be used to detect expressed proteins as markers for human disease states. The levels of such proteins present in the peripheral blood or tissue sample of a patient may be quantified by conventional methods. Antibody-protein binding may be detected and quantified by a variety of means known in the art, such as labeling with fluorescent or radioactive ligands. The invention further comprises kits for performing the above-mentioned procedures, wherein such kits contain antibodies specific for the investigated polypeptides.

Numerous competitive and non-competitive protein binding immunoassays are well known in the art. Antibodies employed in such assays may be unlabeled, for example as used in agglutination tests, or labeled for use a wide variety of assay methods. Labels that can be used include radionuclides, enzymes, fluorescers, chemiluminescers, enzyme substrates or cofactors, enzyme inhibitors, particles, dyes and the like for use in radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art. One approach for preparing antibodies to a protein is the selection and preparation of an amino acid sequence of all or part of the protein, chemically synthesizing the sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In a further embodiment the present invention is based upon the analysis of methylation levels within two or more genes or genomic sequences taken from the group consisting all genes and genomic sequences of genomic regions listed in Table 3 and/or their regulatory sequences. It is further preferred that the sequences of said genes or genomic sequences are as according to SEQ ID NO: 1 to SEQ ID NO:118.

Particular embodiments of the present invention provide a novel application of the analysis of methylation levels and/or patterns within said sequences that enables a precise detection, characterization and/or treatment of breast cell proliferative disorders. Early detection of breast cell proliferative disorders is directly linked with disease prognosis, and the disclosed method thereby enables the physician and patient to make better and more informed treatment decisions.

FURTHER IMPROVEMENTS

The present invention provides novel uses for genomic sequences selected from the group consisting of SEQ ID NO: to SEQ ID NO: 118. Additional embodiments provide modified variants, in particular chemically modified variants, of SEQ ID NO: to SEQ ID NO: 118, as well as oligonucleotides and/or PNA-oligomers for analysis of cytosine methylation patterns within the group consisting SEQ ID NO:1 to SEQ ID NO: 118.

An objective of the invention comprises analysis of the methylation state of one or more CpG dinucleotides within at least one of the genomic sequences selected from the group consisting of SEQ ID NO:1 to SEQ ID NO: 118 and sequences complementary thereto.

The disclosed invention provides treated nucleic acids, derived from genomic SEQ ID NO: 1 to SEQ ID NO: 118, wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid comprising a sequence of at least 16 contiguous nucleotide bases in length of a sequence selected from the group consisting of SEQ ID NO:493 TO SEQ ID NO:964 , wherein said sequence comprises at least one CpG, TpA or CpA dinucleotide and sequences complementary thereto. The sequences of SEQ ID NO:493 TO SEQ ID NO:964 provide non-naturally occurring modified versions of the nucleic acid according to SEQ ID NO:1 to SEQ ID NO: 118, wherein the modification of each genomic sequence results in the synthesis of a nucleic acid having a sequence that is unique and distinct from said genomic sequence as follows. For each sense strand genomic DNA, *e.g.*, SEQ ID NO: 1, four converted versions are disclosed. A first version wherein "C". → "T," but "CpG" remains "CpG" (*i.e.,* corresponds to case where, for the genomic sequence, all "C" residues of CpG dinucleotide sequences are methylated and are thus not converted); a second version discloses the complement of the disclosed genomic DNA sequence (i.e. antisense strand), wherein "C" → "T," but "CpG" remains "CpG" (*i.e.*, corresponds to case where, for all "C" residues of CpG dinucleotide sequences are methylated and are thus not converted). The 'upmethylated' converted sequences of SEQ ID NO: to SEQ ID NO: 118 correspond to SEQ ID NO:493 to SEQ ID NO: 728. A third chemically converted version of each genomic sequences is provided, wherein "C". → "T" for all "C" residues, including those of "CpG" dinucleotide sequences (*i.e.*, corresponds to case where, for the genomic sequences, all "C" residues of CpG dinucleotide sequences are unmethylated); a final chemically converted version of each sequence, discloses the complement of the disclosed genomic DNA sequence (i.e. *antisense* strand), wherein "C" → "T" for all "C" residues, including those of "CpG" dinucleotide sequences (*i.e.*, corresponds to case where, for the complement (*anti*sense strand) of each genomic sequence, all "C" residues of CpG dinucleotide sequences are *un*methylated). The 'downmethylated' converted sequences of SEQ ID NO:1 to SEQ ID NO:118 correspond to SEQ ID NO: 729 to SEQ ID NO:964.

In an alternative preferred embodiment, such analysis comprises the use of an oligonucleotide or oligomer for detecting the cytosine methylation state within genomic or treated (chemically modified) DNA, according to SEQ ID NO:

1 to SEQ ID NO:964. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NO: 493 to SEQ ID NO:964 and/or sequences complementary thereto, or to a genomic sequence according to SEQ ID NO:1 to SEQ ID NO:118 and/or sequences complementary thereto.

Thus, the present invention includes nucleic acid molecules (*e.g.*, oligonucleotides and peptide nucleic acid (PNA) molecules (PNA-oligomers)) that hybridize under moderately stringent and/or stringent hybridization conditions to all or a portion of the sequences SEQ ID NO:1 to SEQ ID NO:964, or to the complements thereof. The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO:1 to SEQ ID NO:964, or to the complements thereof.

Hybridizing nucleic acids of the type described herein can be used, for example, as a primer (*e.g.*, a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO: to SEQ ID NO: 118 (such as allelic variants and SNPs), rather than identical, it is useful to first establish the lowest temperature at which only homologous hybridization occurs with a particular concentration of salt (*e.g.*, SSC or SSPE). Then, assuming that 1% mismatching results in a 1 °C decrease in the Tm, the temperature of the final wash in the hybridization reaction is reduced accordingly (for example, if sequences having > 95% identity with the probe are sought, the final wash temperature is decreased by 5°C). In practice, the change in Tm can be between 0.5°C and 1.5°C per 1% mismatch.

Examples of inventive oligonucleotides of length X (in nucleotides), as indicated by polynucleotide positions with reference to, *e.g.*, SEQ ID NO: 1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

n to (n + (X-1));
where n=1, 2, 3,...(Y-(X-1));
where Y equals the length (nucleotides or base pairs) of SEQ ID NO:1 (6435);
where X equals the common length (in nucleotides) of each oligonucleotide in the set (*e.g.,* X=20 for a set of consecutively overlapping 20-mers); and
where the number (Z) of consecutively overlapping oligomers of length X for a given SEQ ID NO of length Y is equal to Y-(X-1).

For example Z= 6435-19= 6416 for either sense or antisense sets of SEQ ID NO:1, where X=20.

Preferably, the set is limited to those oligomers that comprise at least one CpG, TpG or CpA dinucleotide.

Examples of inventive 20-mer oligonucleotides include the following set of 6416 oligomers (and the antisense set complementary thereto), indicated by polynucleotide positions with reference to SEQ ID NO:1: 1-20, 2-21, 3-22, 4-23, 5-24, etc....... 6397-6416.

Preferably, the set is limited to those oligomers that comprise at least one CpG, TpG or CpA dinucleotide.

Likewise, examples of inventive 25-mer oligonucleotides include the following set of 2,256 oligomers (and the antisense set complementary thereto), indicated by polynucleotide positions with reference to SEQ ID NO: 1: 1-25, 2-26, 3-27, 4-28, 5-29, etc....... 2254-2278, 2255-2279 and 2256-2280.

Preferably, the set is limited to those oligomers that comprise at least one CpG, TpG or CpA dinucleotide.

The present invention encompasses, for *each* of SEQ ID NO: to SEQ ID NO:964 (sense and antisense), multiple consecutively overlapping sets of oligonucleotides or modified oligonucleotides of length X, where, *e.g.*, X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO:1 to SEQ ID NO:118. Preferred sets of such oligonucleotides or modified oligonucleotides of length X are those consecutively overlapping sets of oligomers corresponding to SEQ ID NO:1 to SEQ ID NO:964 (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, *e.g.*, a chromophore, fluorophore, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of each of the CpG dinucleotides of genomic sequences SEQ ID NO:1 to SEQ ID NO:118 and sequences complementary thereto, or to the corresponding CpG, TpG or CpA dinucleotide within a sequence of the treated nucleic acids according to SEQ ID NO:493 to SEQ ID NO:964 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state in treated genomic DNA (SEQ ID NO:493 to SEQ ID NO:964), or in genomic DNA (SEQ ID NO:1 to SEQ ID NO:118 and sequences complementary thereto). These probes enable diagnosis, classification and/or therapy of genetic and epigenetic parameters of breast cell proliferative disorders. The set of oligomers may also be used for detecting single nucleotide polymorphisms (SNPs) in treated genomic DNA (SEQ ID NO:493 to SEQ ID NO:964), or in genomic DNA (SEQ ID NO: 1 to SEQ ID NO:118 and sequences complementary thereto).

In preferred embodiments, at least one, and more preferably all members of a set of oligonucleotides is/are bound to a solid phase.

In further embodiments, the present invention provides a set of at least two (2) oligonucleotides that are used as 'primer' oligonucleotides for amplifying DNA sequences of one of SEQ ID NO: to SEQ ID NO:964 and sequences complementary thereto, or segments thereof.

It is anticipated that the oligonucleotides may constitute all or part of an "array" or "DNA chip" (*i.e.*, an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.*, each analyte) can be uniquely bound by a unique label and thus detected (each label is directly counted, resulting in a digital read-out of each molecular species in the mixture).

It is particularly preferred that the oligomers according to the invention are utilized for at least one of: detection of; detection and differentiation between or among subclasses of; diagnosis of; prognosis of; treatment of; monitoring of; and treatment and monitoring of breast cell proliferative disorders. This is enabled by use of said sets for the differentiation and/or detection of the tissue types according to table 14. Particularly preferred are those sets of oligomer that comprise at least two oligonucleotides selected from one of the following sets of oligonucleotides.

In one embodiment of the method, breast cancer tissue is detected. This is achieved by analysis of the methylation status of at least one target sequence comprising, or hybridizing under stringent conditions to at least 16 contiguous nucleotides of a gene or sequence selected from the group consisting of APC, ARH1/NOEY2, BRCA2, CCND2, CDKN1A, CDKN2A, SEQ ID NO:9, DAPK1, SEQ ID NO:2, EYA4, FHIT, GSTP1, HIC1, IGFBP7, MLH1, PGR, SERPINB5, RARB, SFN, SOD2, TGFBR2, THRB, TIMP3, TP73, NME1, CDH13, THBS1, TMS1/ASC, ESR1, IL6, APAF1, CASP8, SYK, HOXA5, FABP3, RASSF1A, SEQ ID NO:3, RARA, TWIST, ESR2, PLAU, STAT1, SEQ ID NO:4, BRCA1, LOT1, PRSS8, SNCG, TPM1, GPC3, CLDN7, SLC19A1, GJB2, SLIT2, IGSF4, MCT1, HS3ST2, PRDM2, ALX4, SEQ ID NO:5, SEQ ID NO:6, SEQ ID NO:7, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), SASH1, S100A7, BCL11B, SEQ ID NO:51, MGC34831, SEQ ID NO:54, PDLIM1, MSF, SEQ ID NO:14, SEQ ID NO:15, SEQ ID NO:16, SEQ ID NO:17, SEQ ID NO:18, SEQ ID NO:19, PRDM6, RAP2B, NR2E1, PCDH7, DKK3, RTTN, SNAP25, SEQ ID NO:26, GIRK2, SEQ ID NO:28, SEQ ID NO:29, ARL7, SEQ ID NO:31, THH, HOXB13, SEQ ID NO:35, MGC10561, LMX1A, SENP3, GS1, TITF1, SEQ ID NO:42, DDX51, SEQ ID NO:117, SEQ ID NO:45, SEQ ID NO:46, 060279, and SEQ ID NO:48 and complements thereof.

In a further embodiment the methylation status of at least one target sequence comprising, or hybridizing under stringent conditions to at least 16 contiguous nucleotides of a gene or sequence selected from the group consisting of PRDM2, PLAU, GSTP1, SLIT2, CCND2, HOXA5, RASSF1A, HS3ST2, ARH1/NOEY2, SCGB3A1, LIMK-1, SEQ ID NO: 6, SEQ ID NO:3, SEQ ID NO:18, SEQ ID NO:7, SEQ ID NO:41, SEQ IDNO:22, SEQ ID NO:46, SEQ ID NO:13, and SEQ ID NO:31 and complements thereof.

In both cases this is preferably achieved by use of a set consisting of at least one oligonucleotide, and more preferably at least two selected from one of the groups consisting of 1475 - 1477, 1477, 1478, 1478, 1479, 1479, 1480, 1480 - 1497, 1497, 1498, 1498, 1499, 1499, 1500, 1500, 1501, 1501, 1502, 1502 -1511,1511, 1512, 1512 - 1527, 1527, 1528, 1528 - 1557, 1557, 1558, 1558 - 1567, 1567, 1568, 1568, 1569, 1569, 1570, 1570 - 1573, 1573, 1574, 1574 - 1607, 1607, 1608, 1608 - 1619, 1619, 1620, 1620 - 1623, 1623, 1624, 1624 - 1639, 1639, 1640, 1640 - 1655, 1655, 1656, 1656 - 1693, 1693, 1694, 1694, 1695, 1695, 1696, 1696, 1697, 1697 - 1702, 1702, 1703, 1703 - 1706, 1706, 1706, 1706, 1707, 1707, 1707, 1707 - 1720, 1720, 1721, 1721 - 1724, 1724, 1725, 1725 - 1728, 1728, 1729, 1729 - 1734, 1734, 1735, 1735 - 1738, 1738, 1739, 1739 - 1752, 1752, 1753, 1753 - 1758, 1758, 1759, 1759 - 1762, 1762, 1763, 1763 - 1770, 1770, 1771, 1771 - 1778, 1778, 1779, 1779, 1780, 1780, 1781, 1781 - 1790, 1790, 1791, 1791 - 1798, 1798, 1799, 1799 - 1838, 1838, 1839, 1839, 1840, 1840, 1841, 1841 - 1852, 1852, 1853, 1853, 1854, 1854, 1855, 1855 - 1870, 1870, 1871, 1871 - 1874, 1874, 1875, 1875 - 1878, 1878, 1879, 1879, 1880, 1880, 1881, 1881, 1882, 1882, 1883, 1883 - 1886, 1886, 1887, 1887 - 1890, 1890, 1891, 1891 - 1906, 1906, 1907, 1907 - 1940, 1940, 1941, 1941, 1942, 1942, 1943, 1943 - 1946, 1946, 1947, 1947 - 1952, 1952, 1953, 1953 - 1966, 1966, 1967, 1967, 1968, 1968, 1969, 1969, 1970, 1970, 1971, 1971 - 1978, 1978, 1979, 1979 - 1982, 1982, 1983, 1983 - 1992, 1992, 1993, 1993 - 1998, 1998, 1999, 1999 - 2006, 2006, 2007, 2007 - 2016, 2016, 2017, 2017 - 2022, 2022, 2023, 2023 - 2030, 2030, 2031, 2031 - 2036, 2036, 2037, 2037 - 2050, 2050, 2051, 2051 - 2060, 2060, 2061, 2061 - 2070, 2070, 2071, 2071 - 2076, 2076, 2077, 2077 - 2104, 2104, 2105, 2105, 2106, 2106, 2107, 2107 - 2114, 2114, 2115, 2115 - 2128, 2128, 2129, 2129, 2130, 2130, 2131, 2131 - 2134, 2134, 2135, 2135, 2136, 2136, 2137, 2137, 2138, 2138, 2139, and 2139.

In one embodiment of the method, breast cancer tissue is differentiated from other cancers. This is achieved by analysis of the methylation status of at least one target sequence comprising, or hybridizing under stringent conditions to at least 16 contiguous nucleotides of a gene or sequence selected from the group consisting of APC, ARH1/NOEY2, BRCA2, CCND2, CDKN1A, CDKN2A, SEQ ID NO:9, DAPK1, SEQ ID NO:2, EYA4, FHIT, GSTP1, HIC1, IGFBP7, MLH1, PGR, SERPINB5, RARB, SFN, SOD2, TGFBR2, THRB, TIMP3, TP73, NME1, CDH13, THBS1, TMS1/ASC, ESR1, IL6, APAF1, CASP8, SYK, HOXA5, FABP3, RASSF1A, SEQ ID NO:3, RARA, TWIST, ESR2, PLAU, STAT1, SEQ ID NO:4, BRCA1, LOT1, PRSS8, SNCG, TPM1, GPC3, CLDN7, SLC19A1, GJB2, SLIT2, IGSF4, MCT1, HS3ST2, PRDM2, ALX4, SEQ ID NO:5, SEQ ID NO:6, SEQ ID NO:7, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), SASH1, S100A7, BCL11B, SEQ ID NO:51, MGC34831, SEQ ID NO:54, PDLIM1, MSF, SEQ ID NO:14, SEQ ID NO:15, SEQ ID NO:16, SEQ ID NO:17, SEQ ID NO:18, SEQ ID NO:19, PRDM6, RAP2B, NR2E1, PCDH7, DKK3, RTTN, SNAP25, SEQ ID NO:26, GIRK2, SEQ ID NO:28, SEQ ID NO:29, ARL7, SEQ ID NO: 31, THH, HOXB13, SEQ ID NO:35, MGC10561, LMX1A, SENP3, GS1, TITF1, SEQ ID NO:42, DDX51, SEQ ID NO: 117, SEQ ID NO:45, SEQ ID NO:46, 060279, and SEQ ID NO:48 and complements thereof. In a further embodiment the methylation status of at least one target sequence comprising, or hybridizing under stringent conditions to at least 16 contiguous nucleotides of a gene or sequence selected from the group consisting PRDM2, GSTP1, ALX4, HOXA5, PLAU, RASSF1A, IGSF4, SLIT2, DAPK1, CDKN1A, SEQ ID NO:38, SEQ IDNO:35, LIMK-1, SEQ ID NO:39', SEQ ID NO: 10, SEQ ID NO: 26, SEQ ID NO: 8, SEQ IDNO:22, SEQ ID NO:18, and SEQ ID NO:47 and complements thereof.

In both cases this is preferably achieved by use of a set consisting of at least one oligonucleotide, and more preferably at least two selected from one of the groups consisting of 1475, 1476, 1485 - 1497, 1497, 1498, 1498, 1499, 1499, 1500, 1500, 1501, 1501, 1502, 1502 - 1504, 1509 - 1511, 1511, 1512, 1512 - 1522, 1525 - 1527, 1527, 1528, 1528 - 1540, 1551 - 1554, 1569, 1569, 1570, 1570 - 1573, 1573, 1574, 1574 - 1580, 1585, 1586, 1591 - 1600, 1603, 1604, 1609 - 1619, 1619, 1620, 1620 - 1623, 1623, 1624, 1624 - 1626, 1629, 1630, 1633 - 1639, 1639, 1640, 1640 - 1642, 1649, 1650, 1667, 1668, 1675 - 1680, 1683 - 1688, 1710 - 1717, 1724, 1724, 1725, 1725 - 1728, 1728, 1729, 1729 - 1731, 1736 - 1738, 1738, 1739, 1739, 1748, 1749, 1752, 1752, 1753, 1753, 1760, 1761, 1764, 1765, 1774 - 1778, 1778, 1779, 1779, 1780, 1780, 1781, 1781 - 1783, 1788, 1789, 1792 - 1798, 1798, 1799, 1799 - 1801, 1804 - 1819, 1830, 1831, 1858 - 1865, 1870, 1870, 1871, 1871, 1874, 1874, 1875, 1875 - 1878, 1878, 1879, 1879, 1880, 1880, 1881, 1881, 1882, 1882, 1883, 1883, 1900, 1901, 1904 - 1906, 1906, 1907, 1907 - 1909, 1916, 1917, 1924 - 1931, 1942, 1942, 1943, 1943, 1948, 1949, 1952, 1952, 1953, 1953 - 1957, 1966, 1966, 1967, 1967, 1968, 1968, 1969, 1969, 1970, 1970, 1971, 1971, 1980 - 1982, 1982, 1983, 1983 - 1985, 1994 - 1998, 1998, 1999, 1999, 2004, 2005, 2010 - 2015, 2020, 2021, 2028 - 2030, 2030, 2031, 2031 - 2033, 2036, 2036, 2037, 2037 - 2049, 2054 - 2059, 2066 - 2070, 2070, 2071, 2071 - 2073, 2076, 2076, 2077, 2077, 2080 - 2089, 2092 - 2099, 2104, 2104, 2105, 2105, 2108, 2109, 2116 - 2119, 2130, 2130, 2131, 2131 - 2134, 2134, 2135, 2135, 2136, 2136, 2137, 2137, 2138, 2138, 2139, 2139, 2140, 2140, 2141, 2141 - 2144, 2144, 2145, 2145, 2146, 2146 - 2149, 2149, 2150, 2150 - 2154, 2163, 2164, 2169, 2170, and 2177 - 2180.

In one further embodiment of the method, breast cancer cells are detected against a background of blood or blood derived fluids by the differentiation of breast cancer cells from peripheral blood lymphocytes. This is achieved by analysis of the methylation status of at least one target sequence comprising, or hybridizing under stringent conditions to at least 16 contiguous nucleotides of a gene or sequence selected from the group consisting APC, ARH1/NOEY2, CCND2, CDH1, CDKN1A, CDKN2A, SEQ ID NO:9, DAPK1, SEQ ID NO:2, EYA4, FHIT, GSTP1, HIC1, IGFBP7, PGR, SERPINB5, RARB, SFN, SOD2, TERT, TGFBR2, THRB, TIMP3, NME1, CDH13, THBS1, TMS1/ASC, ESR1, IL6, APAF1, CASP8, SYK, HOXA5, FABP3, RASSF1A, SEQ ID NO:3, TWIST, ESR2, PLAU, STAT1, SEQ ID NO:4, BRCA1, LOT1, PRSS8, SNCG, GPC3, CLDN7, SLC19A1, GJB2, SLIT2, IGSF4, MCT1, HS3ST2, PRDM2, ALX4, SEQ ID NO: 5, SEQ ID NO:6, SEQ ID NO:7, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), SASH1, S100A7, BCL11B, SEQ ID NO:51, MGC34831, SEQ ID NO:54, PDLIM1, MSF, SEQ ID NO:14, SEQ ID NO: 15, SEQ ID NO:16, SEQ ID NO:17, SEQ ID NO:18, SEQ ID NO:19, PRDM6, RAP2B, NR2E1, PCDH7, DKK3, RTTN, SNAP25, SEQ ID NO:26, GIRK2, SEQ ID NO:28, SEQ ID NO:29, ARL7, SEQ ID NO:31, THH, HOXB13, SEQ ID NO: 35, SEQ ID NO:36, MGC10561, LMX1A, SENP3, GS1, TITF1, SEQ ID NO:42, DDX51, SEQ ID NO:117 , SEQ ID NO: 46, 060279, and SEQ ID NO:48 and complements thereof.

In a further embodiment the methylation status of at least one target sequence comprising, or hybridizing under stringent conditions to at least 16 contiguous nucleotides of a gene or sequence selected from the group consisting EYA4, PRDM2, SERPINB5, TWIST, STAT1, ALX4, IGFBP7, DAPK1, THBS1, PLAU, SEQ ID NO:20, SEQ ID NO:38, SEQ ID NO: 8, SEQ ID NO:37, SEQ ID NO: 10, SEQ IDNO:35, SEQ ID NO:47, SEQ ID NO:6, LIMK-1 and SEQ ID NO: 46 and complements thereof.

In both cases, this is preferably achieved by use of a set consisting of at least one oligonucleotide, and more preferably at least two selected from one of the groups consisting of 1475 - 1477, 1477, 1478, 1478, 1479, 1479, 1480, 1480, 1485 - 1497, 1497, 1498, 1498, 1501, 1501, 1502, 1502 - 1511, 1511, 1512, 1512 - 1522, 1525 - 1527, 1527, 1528, 1528 - 1540, 1543 - 1546, 1549 - 1557, 1557, 1558, 1558 - 1567, 1567, 1568, 1568, 1569, 1569, 1570, 1570 - 1572, 1583 - 1596, 1599, 1600, 1603, 1604, 1607, 1607, 1608, 1608 - 1619, 1619, 1620, 1620 - 1623, 1623, 1624, 1624 - 1626, 1631 - 1639, 1639, 1640, 1640, 1645, 1646, 1649 - 1655, 1655, 1656, 1656 - 1660, 1663 - 1668, 1671 - 1693, 1693, 1694, 1694, 1695, 1695, 1696, 1696, 1697, 1697 - 1699, 1704 - 1706, 1706, 1707, 1707 - 1720, 1720, 1721, 1721, 1724, 1724, 1725, 1725 - 1728, 1728, 1729, 1729 - 1733, 1738, 1738, 1739, 1739, 1742, 1743, 1746, 1747, 1750, 1751, 1758, 1758, 1759, 1759 - 1762, 1762, 1763, 1763 - 1770, 1770, 1771, 1771 - 1778, 1778, 1779, 1779, 1782 - 1789, 1792 - 1798, 1798, 1799, 1799 - 1838, 1838, 1839, 1839, 1840, 1840, 1841, 1841 - 1845, 1848 - 1852, 1852, 1853, 1853, 1854, 1854, 1855, 1855 - 1869, 1872, 1873, 1878, 1878, 1879, 1879, 1880, 1880, 1881, 1881, 1882, 1882, 1883, 1883 - 1885, 1888 - 1890, 1890, 1891, 1891 - 1906, 1906, 1907, 1907 - 1935, 1938 - 1940, 1940, 1941, 1941, 1942, 1942, 1943, 1943 - 1946, 1946, 1947, 1947 - 1952, 1952, 1953, 1953 - 1966, 1966, 1967, 1967, 1968, 1968, 1969, 1969, 1970, 1970, 1971, 1971 - 1973, 1976 - 1978, 1978, 1979, 1979 - 1982, 1982, 1983, 1983 - 1987, 1990 - 1992, 1992, 1993, 1993 - 1998, 1998, 1999, 1999 - 2006, 2006, 2007, 2007 - 2016, 2016, 2017, 2017 - 2022, 2022, 2023, 2023 - 2025, 2028 - 2030, 2030, 2031, 2031 - 2036, 2036, 2037, 2037 - 2049, 2052 - 2060, 2060, 2061, 2061 - 2070, 2070, 2071, 2071 - 2076, 2076, 2077, 2077 - 2104, 2104, 2105, 2105, 2106, 2106, 2107, 2107 - 2114, 2114, 2115, 2115 - 2117, 2120 - 2128, 2128, 2129, 2129, 2130, 2130, 2131, 2131 - 2134, 2134, 2135, 2135, 2136, 2136, 2137, 2137, 2138, 2138, 2139, 2139, 2140, 2140, 2141, 2141 - 2143, 2147 - 2149, 2149, 2150, 2150 - 2156, 2161, 2162, 2171, 2172, 2181, 2181, 2182, 2182 - 2187, 2187, 2188, 2188 - 2199, 2199, 2200, and 2200 - 2218.

In one embodiment of the method, DCIS is differentiated from benign breast cell proliferative disorders. This is achieved by analysis of the methylation status of at least one target sequence comprising, or hybridizing under stringent conditions to at least 16 contiguous nucleotides of a gene or sequence selected from the group consisting of APC, ARH1/NOEY2, CCND2, CDKN1A, CDKN2A, SEQ ID NO:9, SEQ ID NO:2, EYA4, FHIT, GSTP1, HIC1, IGFBP7, MLH1, PGR, SERPINB5, RARB, SOD2, TERT, TGFBR2, THRB, TIMP3, TP73, CDH13, THBS1, TMS1/ASC, ESR1, APAF1, CASP8, SYK, HOXA5, FABP3, RASSF1A, SEQ ID NO:3, RARA, TWIST, ESR2, PLAU, SEQ ID NO:4, SNCG, SLC19A1, GJB2, SLIT2, IGSF4, MCT1, HS3ST2, PRDM2, SEQ ID NO:6, SEQ ID NO:7, SEQ ID NO:8, SCGB3A1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), SASH1, S100A7, BCL11B, SEQ ID NO:51, MGC34831, SEQ ID NO:54, PDLIM1, MSF, SEQ ID NO:14, SEQ ID NO:16, SEQ ID NO:17, SEQ ID NO:18, SEQ ID NO:19, NR2E1, PCDH7, DKK3, RTTN, SNAP25, GIRK2, SEQ ID NO:28, SEQ ID NO:29, ARL7, SEQ ID NO:31, THH, HOXB13, SEQ ID NO:36, LMX1A, SENP3, TITF1, SEQ ID NO:42, DDX51, SEQ ID NO: 117, SEQ ID NO:46, 060279, and SEQ ID NO:48 and complements thereof.

In a further embodiment the methylation status of at least one target sequence comprising, or hybridizing under stringent conditions to at least 16 contiguous nucleotides of a gene or sequence selected from the group consisting of HS3ST2, SLIT2, RASSF1A, GSTP1, GJB2, IGFBP7, CDH13, ARH1/NOEY2, SCGB3A1, FHIT, SEQ ID NO: 27, LIMK-1, SEQ ID NO:46, SEQ ID NO:3, SEQ ID NO: 117, SEQ ID NO:48, SEQ ID NO:41, SEQ ID NO:4, SEQ ID NO:6, and SEQ ID NO:24 and complements thereof.

In both cases this is preferably achieved by use of a set consisting of at least one oligonucleotide, and more preferably at least two selected from one of the groups consisting of 1475 - 1477, 1477, 1478, 1478, 1479, 1479, 1480, 1480 - 1482, 1485 - 1497, 1497, 1498, 1498, 1501, 1501, 1502, 1502 - 1508, 1513, 1514, 1517, 1518, 1521 - 1527, 1527, 1528, 1528 - 1534, 1541 - 1544, 1547 - 1550, 1555 - 1557, 1557, 1558, 1558 - 1560, 1567, 1567, 1568, 1568, 1569, 1569, 1570, 1570 - 1573, 1573, 1574, 1574 - 1578, 1581 - 1592, 1595 - 1600, 1605 - 1607, 1607, 1608, 1608 - 1618, 1623, 1623, 1624, 1624 - 1626, 1631 - 1636, 1639, 1639, 1640, 1640, 1663 - 1668, 1671 - 1676, 1689, 1690, 1695, 1695, 1696, 1696, 1697, 1697 - 1701, 1706, 1706, 1707, 1707 - 1713, 1720, 1720, 1721, 1721, 1726 - 1728, 1728, 1729, 1729, 1732 - 1734, 1734, 1735, 1735, 1740, 1741, 1746 - 1752, 1752, 1753, 1753, 1756 - 1758, 1758, 1759, 1759, 1772 - 1778, 1778, 1779, 1779, 1780, 1780, 1781, 1781, 1784 - 1790, 1790, 1791, 1791 - 1798, 1798, 1799, 1799 - 1805, 1812 - 1835, 1842 - 1845, 1848, 1849, 1852, 1852, 1853, 1853, 1854, 1854, 1855, 1855 - 1869, 1872, 1873, 1876 - 1878, 1878, 1879, 1879, 1882, 1882, 1883, 1883 - 1886, 1886, 1887, 1887 - 1890, 1890, 1891, 1891 - 1895, 1902 - 1906, 1906, 1907, 1907, 1910, 1911, 1914 - 1923, 1932 - 1935, 1938 - 1940, 1940, 1941, 1941, 1952, 1952, 1953, 1953, 1958 - 1963, 1966, 1966, 1967, 1967, 1968, 1968, 1969, 1969, 1970, 1970, 1971, 1971, 1974 - 1977, 1980 - 1982, 1982, 1983, 1983 - 1987, 1992, 1992, 1993, 1993, 1996 - 1998, 1998, 1999, 1999 - 2005, 2010 - 2013, 2028 - 2030, 2030, 2031, 2031 - 2036, 2036, 2037, 2037 - 2039, 2042 - 2050, 2050, 2051, 2051, 2060, 2060, 2061, 2061 - 2070, 2070, 2071, 2071 - 2076, 2076, 2077, 2077, 2092, 2093, 2104, 2104, 2105, 2105, 2106, 2106, 2107, 2107, 2110, 2111, 2116, 2117, 2120 - 2127, 2130, 2130, 2131, 2131 - 2134, 2134, 2135, 2135, 2136, 2136, 2137, 2137, 2138, 2138, 2139, 2139, 2155, 2156, 2161, 2162, 2183, 2184, 2187, 2187, 2188, 2188, 2199, 2199, 2200, 2200 - 2202, and 2219 - 2222

In one embodiment of the method, breast cancer is differentiated from benign breast cell proliferative disorders. This is achieved by analysis of the methylation status of at least one target sequence comprising, or hybridizing under stringent conditions to at least 16 contiguous nucleotides of a gene or sequence selected from the group consisting of ARH1/NOEY2, CCND2, CDKN1A, CDKN2A, SEQ ID NO:9, DAPK1, SEQ ID NO:2, EYA4, FHIT, GSTP1, HIC1, IGFBP7, SERPINB5, TERT, TGFBR2, THRB, TIMP3, TP73, NME1, CDH13, THBS1, TMS1/ASC, IL6, APAF1, SYK, HOXA5, FABP3, RASSF1A, SEQ ID NO:3, TWIST, ESR2, PLAU, STAT1, SEQ ID NO:4, LOT1, GPC3, CLDN7, GJB2, SLIT2, IGSF4, MCT1, PRDM2, ALX4, SEQ ID NO:5, SEQ ID NO:6, SEQ ID NO:7, SEQ ID NO:8, SCGB3A1, SEQ ID NO:1, PROSTAGLANDIN E2 RECEPTOR, EP4 SUBTYPE (PROSTANOID EP4 RECEPTOR) (PGE RECEPTOR, EP4 SUBTYPE), ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR), LIM DOMAIN KINASE 1 (EC 2.7.1.37) (LIMK-1), BCL11B, SEQ ID NO:51, MGC34831, SEQ ID NO:54, PDLIM1, MSF, SEQ ID NO:14, SEQ ID NO:16, SEQ ID NO:17, SEQ ID NO:18, SEQ ID NO:19, PRDM6, NR2E1, PCDH7, DKK3, RTTN, SNAP25, SEQ ID NO:26, GIRK2, SEQ ID NO:28, SEQ ID NO:29, ARL7, SEQ ID NO:31, THH, HOXB13, SEQ ID NO: 35, SEQ ID NO:36, MGC10561, LMX1A, SENP3, GS1, TITF1, SEQ ID NO:42, DDX51, SEQ ID NO:117, SEQ ID NO: 45, SEQ ID NO:46, 060279, and SEQ ID NO:48 and complements thereof.

In a further embodiment the methylation status of at least one target sequence comprising, or hybridizing under stringent conditions to at least 16 contiguous nucleotides of a gene or sequence selected from the group consisting SLIT2, HS3ST2, HOXA5, ARH1/NOEY2, IGFBP7, PLAU, CDH13, TIMP3, CCND2, GSTP1, SEQ ID NO: 117, LIMK-1, SEQ ID NO:46, SEQ ID NO:6, SEQ ID NO:7, SEQ ID NO:3, SEQ ID NO:41, SEQ ID NO: 27,SEQ ID NO:31, and SEQ ID NO:4 and complements thereof.

In both cases this is preferably achieved by use of a set consisting of at least one oligonucleotide, and more preferably at least two selected from one of the groups consisting of 1475, 1476, 1485 - 1497, 1497, 1498, 1498, 1499, 1499, 1500, 1500, 1501, 1501, 1502, 1502 - 1504, 1509 - 1511, 1511, 1512, 1512 - 1522, 1525 - 1527, 1527, 1528, 1528 - 1540, 1551 - 1554, 1569, 1569, 1570, 1570 - 1573, 1573, 1574, 1574 - 1580, 1585, 1586, 1591 - 1600, 1603, 1604, 1609 - 1619, 1619, 1620, 1620 - 1623, 1623, 1624, 1624 - 1626, 1629, 1630, 1633 - 1639, 1639, 1640, 1640 - 1642, 1649, 1650, 1667, 1668, 1675 - 1680, 1683 - 1688, 1710 - 1717, 1724, 1724, 1725, 1725 - 1728, 1728, 1729, 1729 - 1731, 1736 - 1738, 1738, 1739, 1739, 1748, 1749, 1752, 1752, 1753, 1753, 1760, 1761, 1764, 1765, 1774 - 1778, 1778, 1779, 1779, 1780, 1780, 1781, 1781 - 1783, 1788, 1789, 1792 - 1798, 1798, 1799, 1799 - 1835, 1842 - 1852, 1852, 1853, 1853, 1854, 1854, 1855, 1855 - 1870, 1870, 1871, 1871 - 1873, 1876 - 1878, 1878, 1879, 1879, 1880, 1880, 1881, 1881, 1882, 1882, 1883, 1883 - 1886, 1886, 1887, 1887 - 1890, 1890, 1891, 1891 - 1895, 1898, 1899, 1902 - 1906, 1906, 1907, 1907 - 1925, 1932 - 1940, 1940, 1941, 1941, 1942, 1942, 1943, 1943, 1958 - 1966, 1966, 1967, 1967, 1968, 1968, 1969, 1969, 1970, 1970, 1971, 1971 - 1977, 1980 - 1982, 1982, 1983, 1983 - 1987, 1992, 1992, 1993, 1993, 1998, 1998, 1999, 1999 - 2006, 2006, 2007, 2007, 2010 - 2015, 2020 - 2022, 2022, 2023, 2023, 2028 - 2030, 2030, 2031, 2031 - 2036, 2036, 2037, 2037 - 2045, 2048 - 2050, 2050, 2051, 2051 - 2057, 2060, 2060, 2061, 2061 - 2070, 2070, 2071, 2071 - 2076, 2076, 2077, 2077 - 2079, 2082, 2083, 2086, 2087, 2092 - 2101, 2104, 2104, 2105, 2105, 2106, 2106, 2107, 2107 - 2111, 2116, 2117, 2120 - 2128, 2128, 2129, 2129, 2130, 2130, 2131, 2131 - 2134, 2134, 2135, 2135, 2136, 2136, 2137, 2137, 2138, 2138, 2139, 2139, 2140, 2140, 2141, 2141 - 2144, 2144, 2145, 2145, 2146, 2146 - 2149, 2149, 2150, 2150 - 2167, 2167, 2168, 2168 - 2175, 2175, 2176, and 2176.

The present invention further provides a method for ascertaining genetic and/or epigenetic parameters of the genomic sequences according to SEQ ID NO: to SEQ ID NO: 118 within a subject by analyzing cytosine methylation and single nucleotide polymorphisms. Said method comprising contacting a nucleic acid comprising one or more of SEQ ID NO: to SEQ ID NO: 118 in a biological sample obtained from said subject with at least one reagent or a series of reagents, wherein said reagent or series of reagents, distinguishes between methylated and non-methylated CpG dinucleotides within the target nucleic acid.

Preferably, said method comprises the following steps: In the *first step,* a sample of the tissue to be analyzed is obtained. The source may be any suitable source, such as cell lines, histological slides, biopsies, paraffin-embedded tissue, bodily fluids, blood plasma, blood serum, whole blood, isolated blood cells, cells isolated from the blood and all possible combinations thereof. It is preferred that said sources of DNA are bodily fluids selected from the group consisting urine, nipple aspirate fluid , lymphatic fluid, ductal lavage fluid, fine needle aspirate, blood plasma, blood serum, whole blood, isolated blood cells, cells isolated from the blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Once the nucleic acids have been extracted, the genomic double stranded DNA is used in the analysis.

In the *second step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO:493 to SEQ ID NO:964 and sequences complementary thereto.

In an alternate embodiment of the method, the methylation status of pre-selected CpG positions within the nucleic acid sequences comprising one or more of SEQ ID NO: to SEQ ID NO:118 may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO:493 to SEQ ID NO: 964 and sequences complementary thereto, wherein the base sequence of said oligomers comprises at least one CpG dinucleotide.

A further preferred embodiment of the method comprises the use of *blocker* oligonucleotides. The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (*e.g.*, with excess blocker), degradation of the blocker oligonucleotide will be substantially precluded. This is because the polymerase will not extend the primer toward, and through (in the 5'-3' direction) the blocker - a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NO:493 to SEQ ID NO:964 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, *e.g.*, matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas and Hillenkamp, Anal Chem., 60:2299-301, 1988). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut and Beck, Current Innovations and Future Trends, 1:147-57, 1995). The sensitivity with respect to nucleic acid analysis is approximately 100-times less than for peptides, and decreases disproportionally with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallization. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut and Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In *the fourth step* of the method, the amplificates obtained during the third step of the method are analyzed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesized in *step three* are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO:1 to SEQ ID NO:118, and the equivalent positions within SEQ ID NO:493 to SEQ ID NO:964. Said oligonucleotides may also be present in the form of peptide nucleic acids. The non-hybridized amplificates are then removed. The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridized to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; *also see* United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a non-extendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethylLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the *fifth step* of the method comprises the use of template-directed oligonucleotide extension, such as MS-SNuPE as described by Gonzalgo and Jones, Nucleic Acids Res. 25:2529-2531, 1997.

In yet a further embodiment of the method, the *fifth step* of the method comprises sequencing and subsequent sequence analysis of the amplificate generated in the *third step* of the method (Sanger F., et al., Proc Natl Acad Sci USA 74:5463-5467, 1977).

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides*, and further, wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO:493 to SEQ ID NO:964 and sequences complementary thereto, wherein the base sequence of said oligomers comprises at least one CpG dinucleotide.

In an alternative, and most preferred embodiment of the method, the subsequent amplification of d) is carried out in the presence of *blocking oligonucleotides*, as described above. Said *blocking oligonucleotides* comprising a sequence having a length of at least 9 nucleotides which hybridizes to a treated nucleic acid sequence according to one of SEQ ID NO:493 to SEQ ID NO:964 and sequences complementary thereto, wherein the base sequence of said oligomers comprises at least one CpG, TpG or CpA dinucleotide. Step e) of the method, namely the detection of the specific amplificates indicative of the methylation status of one or more CpG positions according to SEQ ID NO: 1 to SEQ ID NO: 118 is carried out by means of real-time detection methods as described above.

Additional embodiments of the invention provide a method for the analysis of the methylation status of genomic DNA according to the invention (SEQ ID NO: 1 to SEQ ID NO: 118, and complements thereof) without the need for pretreatment.

In the *first step* of such additional embodiments, the genomic DNA sample is isolated from tissue or cellular sources. Preferably, such sources include cell lines, histological slides, body fluids, or tissue embedded in paraffin. In the *second step*, the genomic DNA is extracted. Extraction may be by means that are standard to one skilled in the art, including but not limited to the use of detergent lysates, sonification and vortexing with glass beads. Once the nucleic acids have been extracted, the genomic double-stranded DNA is used in the analysis. In a preferred embodiment, the DNA may be cleaved prior to the treatment, and this may be by any means standard in the state of the art, in particular with methylation-sensitive restriction endonucleases.

In the *third step*, the DNA is then digested with one or more methylation sensitive restriction enzymes. The digestion is carried out such that hydrolysis of the DNA at the restriction site is informative of the methylation status of a specific CpG dinucleotide.

In the *fourth step*, which is optional but a preferred embodiment, the restriction fragments are amplified. This is preferably carried out using a polymerase chain reaction, and said amplificates may carry suitable detectable labels as discussed above, namely fluorophore labels, radionuclides and mass labels.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

Subsequent to the determination of the methylation state of the genomic nucleic acids the presence, absence or subclass of breast cell proliferative disorder is deduced based upon the methylation state of at least one CpG dinucleotide sequence of SEQ ID NO: to SEQ ID NO: 118 , or an average, or a value reflecting an average methylation state of a plurality of CpG dinucleotide sequences of SEQ ID NO:1 to SEQ ID NO:118.

Example for Diagnostic Assays for breast cell proliferative disorders

The present invention enables diagnosis of events which are disadvantageous to patients or individuals in which important genetic and/or epigenetic parameters within one or more of SEQ ID NO: to SEQ ID NO:118 may be used as markers. Said parameters obtained by means of the present invention may be compared to another set of genetic and/or epigenetic parameters, the differences serving as the basis for a diagnosis and/or prognosis of events which are disadvantageous to patients or individuals.

More specifically the present invention enables the screening of at-risk populations for the early detection of breast cancers. Further embodiments of the method may also be used as alternatives to cytological screening for the differentiation of breast carcinomas from benign breast cell proliferative disorders.

Specifically, the present invention provides for diagnostic cancer assays based on measurement of differential methylation of one or more CpG dinucleotide sequences of SEQ ID NO: to SEQ ID NO: 118, or of sub-regions thereof that comprise such a CpG dinucleotide sequence. Typically, such assays involve obtaining a tissue sample from a test tissue, performing an assay to measure the methylation status of at least one of one or more CpG dinucleotide sequences of SEQ ID NO: to SEQ ID NO: 118 derived from the tissue sample, relative to a control sample, or a known standard and making a diagnosis or prognosis based thereon.

In particular preferred embodiments, inventive oligomers are used to assess the CpG dinucleotide methylation status, such as those based on SEQ ID NO:1 to SEQ ID NO:964, or arrays thereof, as well as in kits based thereon and useful for the diagnosis and/or prognosis of breast cell proliferative disorders.

Example for Kits

Moreover, an additional aspect of the present invention is a kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NO: to SEQ ID NO:964; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight ™, HeavyMethyl™, COBRA, and nucleic acid sequencing. However, a kit along the lines of the present invention can also contain only part of the aforementioned components.

While the present invention has been described with specificity in accordance with certain of its preferred embodiments, the following examples serve only to illustrate the invention and are not intended to limit the invention within the principles and scope of the broadest interpretations and equivalent configurations thereof.

Microarray analysis

To evaluate marker candidates a significant number of patient and control samples was analyzed using the applicant's proprietary methylation sensitive Microarray technology. For the Microarray study two gene panels were analyzed on a collection of initially 475 samples.

Patient Samples

An overview of patient samples collected for the Microarray study is provided in Table 22.

*Table 22: patient samples collected for the Microarray study*

| Sub Diagnosis | # used | Sub Diagnosis | # used |
|---|---|---|---|
| **Early sporadic breast cancer** | **259** | **Benign breast conditions** | **84** |
| IDCT1N0postER+ | 24 | fibroadenoma | 45 |
| IDCT1N0postER- | 25 | fibrocystic disease | 6 |
| IDCT1N0preER+ | 24 | ductal hyperplasia | 3 |
| IDCT1N0preER- | 24 | normal breast epithelium | 12 |
| IDCT1N12postER+ | 27 | normal breast tissue | 18 |
| IDCT1N12postER- | 24 | **Other cancers** | **75** |
| IDCT1N12preER+ | 24 | colon | 13 |
| IDCT1N12preER- | 24 | lung | 19 |
| ILC caucasian ER+ T1N0 | 16 | endometrial | 16 |
| ILC caucasian ER+ T1N12 | 8 | ovarian | 16 |
| DCIS caucasian ER+/- | 39 | others | 11 |
| **genetic breast cancer** | **23** | **Lymphocyte controls** | **34** |
| | | age group old | 13 |
| | | age group young | 13 |
| BRCA1 | 23 | male controls | 8 |
| | | **Total** | **475** |

Patient Samples: Breast Cancer

Early stage breast cancer samples were obtained from Erasmus Medical Centre, Rotterdam, The Netherlands. The tumor cell proportion was estimated to be on average 60%, ranging from 30% to 90%. Infiltrating ductal carcinomas (IDC) represented the largest histological subtype. Among the IDC patient samples, estrogen receptor (ER) positive and negative, pre- and post menopausal as well as aggressive (node positive) and less aggressive (node negative) tumors were included. In addition, infiltrating lobular carcinomas (ILC) and ductal carcinomas in situ (DCIS) were analyzed. For all DCIS samples, the diagnosis was confirmed by pathology review and estimates of tumor cell content were provided. Finally, tumors with confirmed BRCA1 mutations were analyzed to assess whether genetic breast cancer samples, which represent about 5-10% of all breast cancer incidences, would show different DNA methylation patterns.

Samples: benign breast conditions

Normal breast samples were obtained primarily from breast reductions or from patient samples that were originally diagnosed with DCIS but were classified to be normal during a pathology review. To analyze DNA methylation patterns in breast epithelial cells, from which breast cancer is almost exclusively derived, epithelial cells were sorted using epithelial cell surface markers. In addition, samples with the diagnosis fibroadenoma, fibrocystic disease and atypical ductal hyperplasia were included into the group benign breast conditions.

Samples: Other Cancers

To evaluate the marker performance on other cancer samples, tumor DNA from several other cancer types was analyzed. Emphasis was put on the most frequently occurring cancer classes in women (lung, colon) and tumors of the female reproductive system (endometrium, ovary).

Lymphocytes

Age matched female lymphocyte samples were used to assess the methylation level of candidate markers in blood cells.

Control Samples

For control purposes additional samples were included in both Microarray studies. In order to control the quality and the functionality of detection oligos, artificially up- and downmethylated DNA (Promega) was used. 8 male lymphocyte samples were included to allow for a positive control of the overall Microarray process by comparing differential methylation between male and female lymphocytes samples.

Gene selection

An initial selection of 63 candidate genes or sequences were identified. In addition, one gene fragment, ELK1, which was known to differentiate DNA of male from female origin, was included as a positive control. The gene panel for the second Microarray study consisted of 59 sequences initially identified using AP-PCR, MCA or DMH and confirmed by sequencing and the ELK1 gene as a positive control. It has to be noted that not all sequences of the second panel do not currently map to known genes. Candidate markers from both chips are fully listed in Table 3.

DNA Extraction

Samples were received from external collaborators or commercial providers either as frozen tissues, cell nuclei pellets, or extracted genomic DNA. DNA from tissue samples and cell nuclei pellets was isolated at using the QiaAmp Mini Kit (Qiagen, Hilden, Germany; No: 51306).
The DNA quality of all delivered and extracted samples was first assessed by photometrical measurements. Extinction at 260 nm and 280 nm was measured, A260/280 ratios were determined and the resulting DNA concentration were calculated.
After photometrical measurements each genomic DNA sample was analyzed by gel electrophoresis to assess the integrity of the DNA. Only minor signs of degradation were observed, indicating good overall quality of the extracted DNA.

PCR establishment and Multiplex PCR optimization

To amplify all gene fragments, PCR assays were designed to match bisulfite treated DNA and to allow amplification independent of the methylation status of the respective fragment. A standardized primer design workflow optimized by the applicant for bisulfite treated DNA was employed. Individual PCR assays were considered established when successful amplification on bisulfite treated lymphocyte DNA was reproduced in triplicate and no background amplification of genomic DNA was detectable, ensuring bisulfite DNA specific amplification. Primers are listed in Table 1.
To allow efficient amplification, individual PCR assays were combined into multiplex PCR (mPCR) assays usually combining up to 8 primer pairs into one mPCR assay. Several multiplex PCR sets were calculated based on the primer sequences of the individual PCR amplificates and tested on lymphocyte DNA. Based on ALF express analyses the best performing combination of multiplex PCR sets were chosen.

Bisulfite Treatment and Multiplex PCR

Total genomic DNA of all samples and controls was bisulfite treated converting unmethylated cytosines to uracil. Methylated cytosines are conserved. Bisulfite treatment was performed according to the applicant's optimized proprietary bisulfite treatment procedure. In order to avoid a potential process-bias, the samples were randomized into processing batches. The patient samples were first grouped according to the major diagnosis classes:

- ILC

- IDC, N0, ER=neg

- IDC, N0, ER=pos

- IDC, N1 or N2, ER=neg

- IDC, N1 or N2, ER=pos

- Other Tumors:

- benign disease / DCIS

- lymphocytes:

- normal breast and epithelial cells

- BRCA1

Batches of 50 samples were created. Each batch contained the same proportion of samples from the major diagnosis classes. Two independent bisulfite reactions were performed for each DNA sample. 10 ng of bisulfite treated DNA was used for each multiplex PCR (mPCR) reaction. In order to monitor the mPCR results, two methods were used: ALF analysis and gel electrophoresis.

Hybridization

All PCR products from each individual sample were then hybridized to glass slides carrying a pair of immobilized oligonucleotides for each CpG position under analysis. For hybridizations, the samples were grouped into processing batches in order to avoid a potential process-bias. The samples were processed in batches of 80 samples randomized for bisulfite batches. Each detection oligonucleotide was designed to hybridize to the bisulphite converted sequence around one CpG site which was either originally unmethylated (TG) or methylated (CG). See Table 2 for further details of all hybridization oligonucleotides used (both informative and non-informative.) Hybridization conditions were selected to allow the detection of the single nucleotide differences between the TG and CG variants.
Fluorescent signals from each hybridized oligonucleotide were detected using genepix scanner and software. Ratios for the two signals (from the CG oligonucleotide and the TG oligonucleotide used to analyze each CpG position) were calculated based on comparison of intensity of the fluorescent signals.
The samples were processed in batches of 80 samples randomized for sex, diagnosis, tissue, and bisulphite batch For each bisulfite treated DNA sample 2 hybridizations were performed. This means that for each sample a total number of 4 chips were processed.

Data Analysis

For the analysis of chip data, Epigenomics' proprietary software ('Episcape') was used. EpiScape contains a data warehouse that supports queries to sample, genome and laboratory management databases, respectively. It encompasses a variety of statistical tools for analyzing and visualizing methylation array data. In the following sections we summarize the most important data analysis techniques that were applied for analyzing the data.

From Raw Hybridization Intensities to Methylation Ratios

The log methylation ratio (log(CG/TG)) at each CpG position is determined according to a standardized preprocessing pipeline that includes the following steps:

- For each spot the median background pixel intensity is subtracted from the median foreground pixel intensity. This gives a good estimate of background corrected hybridization intensities.
- For both CG and TG detection oligonucleotides of each CpG position the background corrected median of the 4 redundant spot intensities is taken.
- For each chip and each CG/TG oligo pair, the log(CG/TG) ratio is calculated.
- For each sample the median of log(CG/TG) intensities over the redundant chip repetitions is taken.

This log ratio has the property that the hybridization noise has approximately constant variance over the full range of possible methylation rates (see e.g. Huber W, Von Heydebreck A, Sultmann H, Poustka A, Vingron M. 2002. Variance stabilization applied to Microarray data calibration and to the quantification of differential expression. Bioinformatics. 18 Suppl 1: S96-S104.)

Principle Component Analysis

Principle component analysis (PCA) projects measurement vectors (e.g. chip data, methylation profiles on several CpG sites etc.) onto a new coordinate system. The new coordinate axes are referred to as principal components. The first principal component spans the direction of largest variance of the data. Subsequent components are ordered by decreasing variance and are orthogonal to each other. Different CpG positions contribute with different weights to the extension of the data cloud along different components. PCA is an unsupervised technique, i.e. it does not take into account any group or label information of the data points (for further details see e.g. Ripley, B. D. 1996. Pattern Recognition and Neural Networks, Cambridge, UK, Cambridge University Press.).

PCA is typically used to project high dimensional data (in our case methylation-array data) onto lower dimensional subspaces in order to visualize or extract features with high variance from the data. In the present report we used 2 dimensional projections for statistical quality control of the data. We investigated the effect of different process parameters on the chip data in order to rule out that changing process parameters caused large alterations in the measurement values.

A robust version of PCA was used to detect single outlier chips and exclude them from further analysis (Model F, Koenig T, Piepenbrock C, Adorjan P. 2002. Statistical process control for large scale Microarray experiments. Bioinformatics. 18 Suppl 1:S155-163.).

## $T^2$ Control Charts

To control the general stability of the chip production process we use methods from the field of multivariate statistical process control (MVSPC). Our major tool is the $T^2$ control chart, which is used to detect significant deviations of the chip process from normal working conditions (Model F, Koenig T, Piepenbrock C, Adorjan P. 2002. Statistical process control for large scale Microarray experiments. Bioinformatics. 18 Suppl 1:S155-163.).

- Order the chip data with respect to a process parameter (e.g. hybridization data or spotting robot).
- Define a historic data set, which describes the chip process under normal working conditions (e.g. the first 75 hybridized chips). In the chart, data from the historical data set are indicated by a special plot symbol.
- Compute the distance of every new chip to the historic data set. If the distance of several consecutive chips exceeds a given control limit the process has to be regarded as out of control.

Use of $T^2$ charts to monitor the chip production process allows us to efficiently detect and eliminate most systematic error sources.

Comparison of Groups: univariate methods

Wilcoxon rank sum tests are used to compare groups (e.g. male vs. female lymphocytes) in terms of measurement values of single CpG sites. A significant test result (p<0.05) indicates a shift between the distributions of the respective methylation log-ratios, i.e. *log(CG/TG)*.

For the comparison of up- vs. down methylated chips hybridized with Promega DNA, Fisher scores are used to rank single CpG sites according to their discriminatory power. For each methylation ratio y = *CG/TG*, the Fisher score is calculated as

$$\frac{(\bar{y}_1 - \bar{y}_2)^2}{S_1^2 + S_2^2},$$

where $\bar{y}_i$ and $S_i^2$ denote mean and variance of group i, respectively.

Comparison of groups: multivariate methods

As referred to herein a marker (sometimes also simply referred to as gene or amplicon) is a genomic region of interest (also referred to herein using the abbreviation ROI). The ROI usually comprises several CpG positions. For testing the null hypothesis that a marker has no predictive power we use the likelihood ratio test for logistic regression models (see Venables, W. N. and Ripley, B. D. Modem Applied Statistics with S-PLUS, 3rd Ed. edition. New York: Springer, 2002.). The logistic regression model for a single marker is a linear combination of methylation measurements from all CpG positions in the respective ROI. The fitted logistic regression model is compared to a constant probability model that is independent of methylation and represents the null hypothesis. The p-value of the marker is computed via the likelihood ratio test.
A significant p-value for a marker means that the methylation of this ROI has some systematic correlation to the question of interest as given by the sample classes. In general a significant p-value does not necessarily imply a good classification performance. However, because with logistic regression we use a linear predictor as the basis of our test statistic small p-values will be indicative of a good clinical performance.

Multiple Test Corrections

Performing a large number of tests at the 5% level will lead to a large number of false positive test results. If there are no differences between groups, the probability of rejecting at least one hypothesis of equality is nearly 1, if about 200 tests are performed. Correction for multiplicity is therefore necessary to reliably conclude that a test result is really significant. A conservative, but simple method is the Bonferroni correction which multiplies all p-values by the number of tests performed, where corrected values > 1 are censored to 1.0.
Bonferroni corrections are used for all analyses. The correction helps to avoid spurious findings, however, it is a very conservative method and false negative results ("missed markers") are a frequent consequence. Therefore, results corrected by the less conservative False Discovery Rate (FDR) methods are also given.

Class prediction by supervised learning

In order to give a reliable estimate of how well the CpG ensemble of a selected marker can differentiate between different tissue classes we can determine its prediction accuracy by classification. For that purpose we calculate a methylation profile-based prediction function using a certain set of tissue samples with a specific class label. This step is called training and it exploits the prior knowledge represented by the data labels. The prediction accuracy of that function is then tested on a set of independent samples. As a method of choice, we use the support vector machine (SVM) algorithm (see e.g. Cristiannini, N. and Shawe-Taylor, J. An introduction to support vector machines. Cambridge, UK: Cambridge University Press, 2000.; Duda, R. O., Hart, P. E., and Stork, D. G. Pattern Classification. New York: John Wiley & Sons, 2001.) to learn the prediction function. For this report, sensitivity and specificity are weighted equally. This is achieved by setting the risk associated with false positive and false negative classifications to be inversely proportional to the respective class sizes. Therefore sensitivity and specificity of the resulting classifier can be expected to be approximately equal. Note that this weighting can be adapted according to the clinical requirements.

Estimating the performance of the tissue class prediction: Cross Validation

With limited sample size the cross-validation method provides an effective and reliable estimate for the prediction accuracy of a discriminator function, and therefore in addition to the significance of the markers we provide cross-validation accuracy, sensitivity and specificity estimates. For each classification task, the samples were partitioned into 5 groups of approximately equal size. Then the learning algorithm was trained on 4 of these 5 sample groups. The predictor obtained by this method was then tested on the remaining group of independent test samples. The number of correct positive and negative classifications was counted over 10 runs for the learning algorithm for all possible choices of the independent test group without using any knowledge obtained from the previous runs. This procedure was repeated on 10 random permutations of the sample set giving a better estimate of the prediction performance than if performed by simply splitting the samples into one training sample set and one independent test set.

Results

Our first step in the analysis of the array data was to identify discriminatory markers when comparing breast cancer samples with benign breast conditions. Since the preferred aim of the test is to detect early lesions we focused on differentiating between DCIS and benign breast conditions. In order to meet the requirements of a blood based screening test we analyzed the performance of the marker candidate genes in differentiating breast cancer from lymphocytes and cancer from other origins, respectively.

Finally, we compared all breast cancer samples against all other control samples and found that a large number of markers meet the specified statistical criteria with this sample set.

For every comparison, two ways of analyzing the data are presented. Multivariate logistic regression analyses were performed and resulting p-values corrected for multiple testing according to Bonferroni reported. Separate models are fitted for each amplificate, comprising methylation ratios measured by 2-6 detection oligo pairs. A marker is preferred, if a p-value below 0,05 after Bonferroni correction for multiple testing is observed. In addition, linear support vector machine (SVM) algorithms were trained and accuracy, sensitivity and specificity to distinguish the respective classes were estimated based on cross validation.

In addition, co methylation of CpG sites was assessed by analyzing individual detection oligos in univariate analyses. Co methylation is assumed if at least two detection oligos of the same gene fragment differentiate the respective classes with statistical significance ($p < 0.05$ after Bonferroni correction for multiple testing).

Breast cancer vs. benign breast conditions

Microarray 1: See table 12 for results.

In this comparison the positive was 263 breast cancer samples consisting of 197 IDC, 24 ILC, 22 DCIS and 20 BRCA1 samples, respectively. The negative class consisted 28 normal breast samples and 46 breast samples with a benign breast condition, including fibroadenoma, fibrocystic disease and atypical ductal hyperplasia. Based on multivariate logistic regression analysis 49 markers differentiate between breast cancer and benign breast conditions with statistical significance. Of these, 28 markers fulfilled the co methylation criterion with at least two detection oligos based on univariate analysis.

Microarray 2: See table 13 for results.

In this comparison the positive class was 256 breast cancer samples which are consisting of 191 IDC, 22 ILC, 21 DCIS and 22 BRCA1 samples, respectively. The negative consisted 26 normal breast samples and 42 breast samples with a benign breast condition, including fibroadenoma, fibrocystic disease and atypical ductal hyperplasia. Based on multivariate logistic regression 48 markers differentiate between breast cancer and benign breast conditions with statistical significance. Of these, 32 markers fulfilled the co methylation criterion with at least two detection oligos based on univariate analysis

DCIS vs. benign breast conditions

Microarray 1: See table 10 for results.

In this comparison the positive class was 263 breast cancer samples consisting of 197 IDC, 24 ILC, 22 DCIS and 20 BRCA1 samples, respectively. The negative class is composed of 28 normal breast samples and 46 breast samples with a benign breast condition, including fibroadenoma, fibrocystic disease and atypical ductal hyperplasia. Based on multivariate logistic regression analysis 31 markers differentiate between breast cancer and benign breast conditions with statistical significance. Of these, 13 markers fulfilled the co methylation criterion with at least two detection oligos statistically significant based on univariate analysis.

Microarray 2: See table 11 for results.

In this comparison the positive class was 256 breast cancer samples consisting 191 IDC, 22 ILC, 21 DCIS and 22 BRCA1 samples, respectively. The negative class i consisted of 26 normal breast samples and 42 breast samples with a benign breast condition, including fibroadenoma, fibrocystic disease and atypical ductal hyperplasia. Based on multivariate logistic regression 35 markers differentiate between breast cancer and benign breast conditions with statistical significance. Of these, 17 markers fulfilled the co methylation criterion with at least two detection oligos statistically significant based on univariate analysis.

Breast cancer vs. lymphocytes

Microarray 1: See table 8 for results.

In this comparison the positive class was 263 breast cancer samples which consisted 197 IDC, 24 ILC, 22 DCIS and 20 BRCA1 samples, respectively. The negative class consisted of 28 lymphocyte samples. Based on multivariate logistic regression analysis 49 markers differentiate between breast cancer and benign breast conditions with statistical significance. Of these, 30 markers fulfilled the co methylation criterion with at least two detection oligos statistically significant based on univariate analysis.

Microarray2: See table 9 for results.

In this comparison the positive class was 256 breast cancer samples which consisted of 191 IDC, 22 ILC, 21 DCIS and 22 BRCA1 samples, respectively. The negative class consisted of 34 lymphocyte samples. Based on multivariate logistic regression, 47 markers differentiate between breast cancer and benign breast conditions with statistical significance. Of these, 37 markers fulfilled the co methylation criterion with at least two detection oligos statistically significant based on univariate analysis.

Breast cancer vs. other cancers

Microarray 1: See table 6 for results.

In this comparison the positive class was 263 breast cancer samples which consisted of 197 IDC, 24 ILC, 22 DCIS and 20 BRCA1 samples, respectively. The negative class consisted of 71 other cancer samples which is composed of 12 colon samples, 19 lung samples, 16 ovary samples and 28 further samples of smaller tissue groups. Based on multivariate logistic regression analysis 28 markers differentiate between breast cancer and benign breast conditions with statistical significance. Of these, 16 markers fulfilled the co methylation criterion with at least two detection oligos statistically significant based on univariate analysis.

Microarray 2: See table 7 for results.

In this comparison the positive class was 256 breast cancer samples which consisted of 191 IDC, 22 ILC, 21 DCIS and 22 BRCA1 samples, respectively. The negative class consisted of 73 other cancer samples which consisted of 18 colon samples, 18 lung samples, 16 ovary samples and 21 further samples of smaller tissue groups. Based on multivariate logistic regression 25 markers differentiate between breast cancer and benign breast conditions with statistical significance. Of these, 15 markers fulfilled the co methylation criterion with at least two detection oligos statistically significant based on univariate analysis.

Breast cancer vs. all other controls

Microarray 1: See table 4 for results.

In this comparison the positive class was 263 breast cancer samples which consisted of 197 IDC, 24 ILC, 22 DCIS and 20 BRCA1 samples, respectively. The negative class consisted of 74 breast samples (normal and benign disease), 71 other cancer samples and 28 lymphocyte samples. Based on multivariate logistic regression analysis 50 markers differentiate between breast cancer and benign breast conditions with statistical significance. Of these, 29 markers fulfilled the co methylation criterion with at least two detection oligos statistically significant based on univariate analysis.

Microarray 2: See table 5 for results.

In this comparison the positive class was 256 breast cancer samples which consisted of 191 IDC, 22 ILC, 21 DCIS and 22 BRCA1 samples, respectively. The negative class consisted of 68 breast samples (normal and benign disease), 73 other cancer samples and 34 lymphocyte samples. Based on multivariate logistiv regression 48 markers differentiate between breast cancer and benign breast conditions with statistical significance. Of these, 32 markers fulfilled the co methylation criterion with at least two detection oligos statistically significant based on univariate analysis.

Table 1: Primer according to Example 1.

| No: | Primer: | Amplificate Length: |
|---|---|---|
| 1. MSF (SEQ ID NO: 13) | AATAAACAACCCTCCCCTC (SEQ ID NO: 973) TGGAGATTATTGTGTGAGTTTT (SEQ ID NO: 972) | 423 |
| 2. SEQ ID NO:14 (SEQ ID NO: 14) | CCAAAAACCTCTACATTCAAAC (SEQ ID NO: 975) AAAAAGGGGATTAGTGGGT (SEQ ID NO: 974) | 358 |
| 3. SEQ ID NO:15 (SEQ ID NO: 15) | ATTTTAGGTGTAAGTTTAAGGTTGT (SEQ ID NO: 976) ATCTACCTTTCCCCACCC (SEQ ID NO: 977) | 473 |
| 4. SEQ ID NO: 16 (SEQ ID NO: 16) | ACACAACTAAAACCCTCAAATC (SEQ ID NO: 979) AGGGAAAGGAGGTAGAGGT (SEQ ID NO: 978) | 262 |
| 5. SEQ ID NO:17 (SEQ ID NO: 17) | ATCACACCCTCCCAAAAC (SEQ ID NO: 981) AGTAGGAATTGTTTATAGATATGTTGA (SEQ ID NO: 980) | 385 |
| 6. SEQ ID NO: 18 (SEQ ID NO: 18) | ACTCCCCAAAATCCCACT (SEQ ID NO: 983) TGTTTTAGGTTTGATGGATTAGA (SEQ ID NO: 982) | 488 |
| 7. SEQ ID NO: 19 (SEQ ID NO: 19) | AAAACCCCATCTCTACAACC (SEQ ID NO: 985) AATGAGAGGGAAAATGAAAGT (SEQ ID NO: 984) | 498 |
| 8. PRDM6 (SEQ ID NO: 20) | ACTTCCAATCTTAAAAACCAAA (SEQ ID NO: 987) GGAGGAGAGGATGAAGTTTTA (SEQ ID NO: 986) | 272 |
| 9. RAP2B (SEQ ID NO: 21) | CTCAACCTACAAACAAATCTTAAC (SEQ ID NO: 989) AGGGGTAGGGGAGTGTTT (SEQ ID NO: 988) | 176 |
| 10. NR2E 1 (SEQ ID NO: 22) | CACCCCTACAACCCAAAC (SEQ ID NO: 991) GGTGGAGGGAAGATTAGTGTA (SEQ ID NO: 990) | 495 |
| 11. NR2E (SEQ ID NO: 22) | CCTTAAAAACCTCCAACCC (SEQ ID NO: 993) GGTTGTTGAAAGAAGTTAGTTTG (SEQ ID NO: 992) | 343 |
| 12. PCDH7 (SEQ ID NO: 23) | TCAATTCAAAAACACAACCAA (SEQ ID NO: 995) AATTTTAGAGAGTTGGAGAAAGAT (SEQ ID NO: 994) | 448 |
| 13. DKK3 (SEQ ID NO: 24) | TAGGTATAGTAGGGTGGTTTTAAGT (SEQ ID NO: 996) AAAACTCCAACATCACAAATAA (SEQ ID NO: 997) | 338 |
| 14. RTTN (SEQ ID NO: 25) | TTCAAAAACAAACCACTAATACC (SEQ ID NO: 999) GTGATTTTAGAGAGGTTGGAAG (SEQ ID NO: 998) | 438 |

(continued)

| No: | Primer: | Amplificate Length: |
|---|---|---|
| 15. BCL11B (SEQ ID NO: 50) | CCCCAAACTATAACCAACTTTA (SEQ ID NO: 1001) TATGGGATTGGAAGGGAT (SEQ ID NO: 1000) | 381 |
| 16. COL5A1 (SEQ ID NO: 53) | GATTAGTAGGAGGGGTTGTTTA (SEQ ID NO: 1002) AAATCCCACATCTACATATCATC (SEQ ID NO: 1003) | 500 |
| 17. SEQ ID NO:51 (SEQ ID NO: 51) | GTAGGGGTGGAGTGAAGG (SEQ ID NO: 1004) CACTCAAAACTCCCAAATAAAA (SEQ ID NO: 1005) | 404 |
| 18. MGC34831 (SEQ ID NO: 52) | AATGAGAGTAGGGTTTGAAATTAG (SEQ ID NO: 1006) TATCCAAAAACTTCACCTCAAC (SEQ ID NO: 1007) | 273 |
| 19. SEQ ID NO:54 (SEQ ID NO: 54) | TGGGATGAAGAAGTAGTGTGTT (SEQ ID NO: 1008) CTCCAACTAAACATTAAATAAATCTCA (SEQ ID NO: 1009) | 398 |
| 20. PDLIM (SEQ ID NO: 55) | GGTTTATTTGTTGGGTGGTTA (SEQ ID NO: 1010) CTATCAAAACCTACTTCCCTCTC (SEQ ID NO: 1011) | 440 |
| 21. KOX7 (SEQ ID NO: 49) | ACCACCACAAATATCCCAA (SEQ ID NO: 1013) GAGGTTAAGGGATGGTTTTATT (SEQ ID NO: 1012) | 423 |
| 22. SEQ ID NO:3 (SEQ ID NO: 3) | TCCTCAACTCTACAAACCTAAAA (SEQ ID NO: 1015) GTAGGGGAGGGAAGTAGATGT (SEQ ID NO: 1014) | 408 |
| 23. SEQ ID NO:4 (SEQ ID NO: 4) | TGTTTGGGTTAGATGGGG (SEQ ID NO: 1016) ATTCTCAACCACCAAAATCTAC (SEQ ID NO: 1017) | 378 |
| 24. SEQ ID NO:1 (SEQ ID NO: 1) | CCTCTAATTCCTATCAATCACC (SEQ ID NO: 1019) TTAGAAGTGAAAGTAGAAGGGTTT (SEQ ID NO: 1018) | 435 |
| 25. SEQ ID NO:9 (SEQ ID NO: 9) | GAAAGGAGAGGTTAAAGGTTG (SEQ ID NO: 1020) AACTCACTTAACTCCAATCCC (SEQ ID NO: 1021) | 696 |
| 26. SEQ ID NO:5 (SEQ ID NO: 5) | TTCTATTAAAACCCAACTCCTC (SEQ ID NO: 1023) ATAAGGGGAATTGTTGTAGGTT (SEQ ID NO: 1022) | 395 |
| 27. SEQ ID NO:6 (SEQ ID NO: 6) | AGGGAGTTAAGTAAGGGGTTAG (SEQ ID NO: 1024) AACACCAACAAAATATCCATCT (SEQ ID NO: 1025) | 442 |

(continued)

| No: | Primer: | Amplificate Length: |
|---|---|---|
| 28. SEQ ID NO:8 (SEQ ID NO: 8) | TAGTAGTTTGAAGAAGGGGAAG (SEQ ID NO: 1026) AAACATTCCTAAAATCACAAAAA (SEQ ID NO: 1027) | 373 |
| 29. SEQ ID NO:6 (SEQ ID NO: 6) | AGATGGATATTTTGTTGGTGTT (SEQ ID NO: 1028) TACACAATTATACCTTTCAAACAAT (SEQ ID NO: 1029) | 250 |
| 30. SEQ ID NO:7 (SEQ ID NO: 7) | CAAACCCAATTCTCAATATCC (SEQ ID NO: 1031) GAAGTTGTTGTATATGAGGTTGTTA (SEQ ID NO: 1030) | 434 |
| 31. PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GAAGAGGAATGGGAAAATTAG (SEQ ID NO: 1032) TCACCAACAAAATACCCAA (SEQ ID NO: 1033) | 500 |
| 32. PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AACCATCAACCATACCTATTTC (SEQ ID NO: 1035) TGAGTAAGATGATTATTTGGATTT (SEQ ID NO: 1034) | 467 |
| 33. ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | CCACTCACTCAACCCATAA (SEQ ID NO: 1037) GTGTGAGGTTTGGGTATTTTT (SEQ ID NO: 1036) | 398 |
| 34. LIM DOMAIN KINASE 1 (EC 2.7.1.37) (LIMK-1) (SEQ ID NO: 12) | AAACCCTACTTCCTACAAACAA (SEQ ID NO: 1039) AGGGAGGTTTGGTGTATTTT (SEQ ID NO: 1038) | 420 |
| 36. HOXB13 (SEQ ID NO: 34) | CTCACACTCTTTAACCTTTTCC (SEQ ID NO: 1041) GAGAGGGATATGAGGGTTTTT (SEQ ID NO: 1040) | 496 |
| 37. SEQ ID NO: 35 | TAAATCCCCCAACACATACTAC (SEQ ID NO: 1043) GTTTATTGGTTTTTATAGATTAAGG (SEQ ID NO: 1042) | 473 |
| 38. SEQ ID NO: 36 | AGGGGTGAGTTTTATTAGAGGT (SEQ ID NO: 1044) CTAAACCCCAATCTCTCCA (SEQ ID NO: 1045) | 479 |
| 39. MGC10561 (SEQ ID NO: 37) | AAAACCATAAAATCCCACATAA (SEQ ID NO: 1047) TTTTTGGGAGGGAAGAGT (SEQ ID NO: 1046) | 486 |
| 40. LMX1A (SEQ ID NO: 38) | AACAACACTCTTACCCTTATCC (SEQ ID NO: 1049) TGAATGTGGAGGATGAGATAGT (SEQ ID NO: 1048) | 490 |

(continued)

| No: | Primer: | Amplificate Length: |
|---|---|---|
| 41. SENP3 (SEQ ID NO: 39) | GGTTAAGGAAGGTAGGAGATTT (SEQ ID NO: 1050) CTTCCAAACTAAAAACCACTTT (SEQ ID NO: 1051) | 466 |
| 42. GS1 (SEQ ID NO: 40) | ATCACCATTCAAAATACAAATATC (SEQ ID NO: 1053) GTTTTGGAGATTAGTTGGGG (SEQ ID NO: 1052) | 470 |
| 43. TITF1 (SEQ ID NO:41) | CTTACTCCCTCAATACAAAACC (SEQ ID NO: 1055) GAGAGAAAGAAGTTGGGTGAT (SEQ ID NO: 1054) | 467 |
| 44. SEQ ID NO:42 (SEQ ID NO: 42) | GAGAAAAAGAGGGAGATTATTG (SEQ ID NO: 1056) CCACATTCAAAAACACAAATAC (SEQ ID NO: 1057) | 336 |
| 45. DDX51 (SEQ ID NO: 43) | CAAACTCACTCTATAACCTCCC (SEQ ID NO: 1059) TTTAGGGGTTTGGATTTTG (SEQ ID NO: 1058) | 482 |
| 46. SEQ ID NO: 117 | GGTTAGGGTGGGTAAAGGTAG (SEQ ID NO: 1060) CCTTTCCTTCAAATAACAAAAC (SEQ ID NO: 1061) | 288 |
| 47. Q8NAN2 (SEQ ID NO: 44) | TTGTATAGGAAAGTAGGAGGGTT (SEQ ID NO: 1062) AAACAAAATAATCTTTCACATCC (SEQ ID NO: 1063) | 307 |
| 48. SEQ ID NO:45 (SEQ ID NO: 45) | CAAAACAAATTAACTCCCCC (SEQ ID NO: 1065) AGGGTGATAGTATAAAGGAAATTG (SEQ ID NO: 1064) | 461 |
| 49. SEQ ID NO:46 (SEQ ID NO: 46) | TCCTCTACCCTTCACCTACC (SEQ ID NO: 1067) TGGAAGATAATGTGTTATTTAGTATTT (SEQ ID NO: 1066) | 383 |
| 50. 060279 (SEQ ID NO: 47) | TTAAAAGGGAATGGATATAGAGTT (SEQ ID NO: 1068) ACTTTCCTAAAATCTCCAAAAA (SEQ ID NO: 1069) | 485 |
| 51. SEQ ID NO:48 (SEQ ID NO: 48) | TATTTTTGGGGATGAAGAAAAT (SEQ ID NO: 1070) ACAAAATCTCCTTTCATTTAACA (SEQ ID NO: 1071) | 484 |
| 52. SEQ ID NO:2 (SEQ ID NO: 2) | TCCAATAAACACAAACCTAAATC (SEQ ID NO: 1212) ATATGGGATTGATGGAAGATAG (SEQ ID NO: 1213) | 471 |
| 53. SNAP25 (SEQ ID NO: 33) | ACTCCACCACAATTACAACCTA (SEQ ID NO: 1075) AGAAAGAGGGGGTTTTATTATT (SEQ ID NO: 1074) | 352 |

(continued)

| No: | Primer: | Amplificate Length: |
|---|---|---|
| 54. SEQ ID NO:26 (SEQ ID NO: 26) | CTTTACCCATCAACAACCCTA (SEQ ID NO: 1077) GGAGGTGTGATTTTATTGTTTG (SEQ ID NO: 1076) | 257 |
| 55. GIRK2 (SEQ ID NO: 27) | ACCTTCCACAAAAACAATAAAC (SEQ ID NO: 1079) TTTTAAGAGAGGAGATTATGATTGA (SEQ ID NO: 1078) | 457 |
| 56. SEQ ID NO:28 (SEQ ID NO: 28) | GTTGAGAGGTAAGGTATGAAGG (SEQ ID NO: 1080) TTAATTCCCCTCTTTAACCTAATAA (SEQ ID NO: 1081) | 477 |
| 57. SEQ ID NO:28 (SEQ ID NO: 28) | TACAAAAATAAACTCAAAATCCCTA (SEQ ID NO: 1083) TTTTTAAGGGAAAGTGGAGG (SEQ ID NO: 1082) | 477 |
| 58. SEQ ID NO:29 (SEQ ID NO: 29) | GAGAGAAATGGGTGATGAAGT (SEQ ID NO: 1084) CCCAACAAATAAAACCCC (SEQ ID NO: 1085) | 493 |
| 59. ARL7 (SEQ ID NO: 30) | TTGAATGTAAGGAGAGGTGG (SEQ ID NO: 1086) AAATCTCTACACCCAAATACAAA (SEQ ID NO: 1087) | 385 |
| 60. SEQ ID NO:31 (SEQ ID NO: 31) | CTCAACAAATAAACTTCACAAAA (SEQ ID NO: 1089) TTTTTGGAGATAGTAGGAAGGG (SEQ ID NO: 1088) | 435 |
| 61. THH (SEQ ID NO: 32) | GTTTTAGGAAAGGGAGAGGG (SEQ ID NO: 1090) TTAATTCACTCCCACCAATAAA (SEQ ID NO: 1091) | 475 |
| 62. APC (SEQ ID NO: 65) | TCAACTACCATCAACTTCCTTA (SEQ ID NO: 1092) AATTTATTTTTAGTGTTGTAGTGGG (SEQ ID NO: 1093) | 491 |
| 63. ESR1 (SEQ ID NO: 75) | AACTATACTCTTTTTCCAAATAACC (SEQ ID NO: 1095) TTTTAGGGGGATTTTGAGTT (SEQ ID NO: 1094) | 197 |
| 64. MLH1 (SEQ ID NO: 89) | TTTTAGGAGTGAAGGAGGTTA (SEQ ID NO: 1096) ATATCCAACCAATAAAAACAAA (SEQ ID NO: 1097) | 442 |
| 65. TIMP3 (SEQ ID NO: 103) | TTTGTTTTAGGAGAGGGGTAG (SEQ ID NO: 1098) CTTAAATCCACCCAAACTTAAC (SEQ ID NO: 1099) | 487 |
| 66. CDKN I A (SEQ ID NO: 67) | AGTTAGAAAGGGGGTTTATTTT (SEQ ID NO: 1100) TCTCTCACCTCCTCTAAATACC (SEQ ID NO: 1101) | 452 |

(continued)

| No: | Primer: | Amplificate Length: |
|---|---|---|
| 67. TP53 (SEQ ID NO: 80) | AATAATTTCCACCAATTCTACC (SEQ ID NO: 1103) TTAGTTTTGAGTATATGGGAGGG (SEQ ID NO: 1102) | 251 |
| 68. GSTP1 (SEQ ID NO: 59) | ATTTGGGAAAGAGGGAAAG (SEQ ID NO: 1104) TAAAAACTCTAAACCCCATCC (SEQ ID NO: 1105) | 301 |
| 69. PGR (SEQ ID NO: 83) | ATGATTGAGTTGAAGGTAAAGG (SEQ ID NO: 1106) TCCAAAACACTATCCAACAAT (SEQ ID NO: 1107) | 347 |
| 70. RARB (SEQ ID NO: 88) | GGGAGTTTTTAAGTTTTGTGAG (SEQ ID NO: 1108) AATCATTTACCATTTTCCAAAC (SEQ ID NO: 1109) | 415 |
| 71. CASP8 (SEQ ID NO: 71) | ACCCCCTTCCCTACTAAAC (SEQ ID NO: 1110) TTAGGGTTAAATGAAAAAGAAAA (SEQ ID NO: 1111) | 436 |
| 72. SNCG (SEQ ID NO: 73) | ACCCTTTAACCACCACACTAT (SEQ ID NO: 1112) TTGGGTTGAGTTAGTAGGAGTT (SEQ ID NO: 1113) | 444 |
| 73. TGFBR2 (SEQ ID NO: 93) | GGATGGTTAGAGAGTTGAAATG (SEQ ID NO: 1114) AAAATCCTACACTTCCTACAACA (SEQ ID NO: 1115) | 374 |
| 74. THBS1 (SEQ ID NO: 81) | CCCCCTTCACTTTCTAACTAA (SEQ ID NO: 1117) AGAGGATGGTTTTGGAGTTT (SEQ ID NO: 1116) | 497 |
| 75. CDH1 (SEQ ID NO: 79) | CCCTTTCTAATCCCAAATCT (SEQ ID NO: 1119) GTTTTAAGGGTTTATGGTTGG (SEQ ID NO: 1118) | 421 |
| 76. DAPK1 (SEQ ID NO: 98) | GGTGGGTATGTGTGTAGAGAA (SEQ ID NO: 1120) AAACCCAAAACACTTCAACTC (SEQ ID NO: 1121) | 407 |
| 77. TP73 (SEQ ID NO: 86) | AGAGGGGATAGTAGGAGGA (SEQ ID NO: 1122) ACTACAAATAAAAAACCCAAAAC (SEQ ID NO: 1123) | 322 |
| 78. SFN (SEQ ID NO: 69) | TTTTTGATGAGGTGGTTGTT (SEQ ID NO: 1124) AAACAAATCTTAAACCCTAATCC (SEQ ID NO: 1125) | 489 |
| 79. HIC1 (SEQ ID NO: 85) | TTTTTAAAAGGGGGTTTTAGGT (SEQ ID NO: 1126) TACCCTTCCAAAAACTAAAAAAAC (SEQ ID NO: 1127) | 589 |
| 80. RASSF1A (SEQ ID NO: 90) | AGTGGGTAGGTTAAGTGTGTTG (SEQ ID NO: 1128) CCCCAAAATCCAAACTAAA (SEQ ID NO: 1129) | 319 |

(continued)

| No: | Primer: | Amplificate Length: |
|---|---|---|
| 81. BRCA1 (SEQ ID NO: 66) | TTGGAAGAGTAGAGGTTAGAGG (SEQ ID NO: 1130) TACCCCAAAACATCACTTAAAC (SEQ ID NO: 1131) | 425 |
| 82. APAF1 (SEQ ID NO: 82) | TTGGGGTGTGTTTATTTGTAT (SEQ ID NO: 1132) CTCCCCTAAATCTCTACAACC (SEQ ID NO: 1133) | 451 |
| 83. TMS1/ASC (SEQ ID NO: 84) | GGATTAAGGGTGTAGTAAGGAA (SEQ ID NO: 1134) TCTCCAAATAAAAACTAACCAAC (SEQ ID NO: 1135) | 364 |
| 84. HOXA5 (SEQ ID NO: 78) | AATCCTACCTAATAACCTCTAAAAAT (SEQ ID NO: 1137) GGGGAAATAAAGTTGTTGTAAA (SEQ ID NO: 1136) | 361 |
| 85. SASH (SEQ ID NO: 102) | TTTTGTAGTGGGTTTAATTGTTTT (SEQ ID NO: 1138) ATAACTTACCAAAATACCCATCA (SEQ ID NO: 1139) | 500 |
| 86. BRCA2 (SEQ ID NO: 56) | ACCCACCCAAACCTAACT (SEQ ID NO: 1141) GGTTGGTAGAGATAAAAGGGTA (SEQ ID NO: 1140) | 388 |
| 87. NME1 (SEQ ID NO: 107) | CACCCAAACTAAAATACCCTAA (SEQ ID NO: 1143) GGAAAAAGTTGATAAATTGGAA (SEQ ID NO: 1142) | 306 |
| 88. TPM1 (SEQ ID NO: 110) | GGGAAAGGGTAGGTAGAAAATA (SEQ ID NO: 1144) ACACCCAACCATTAAAATCC (SEQ ID NO: 1145) | 386 |
| 89. SOD2 (SEQ ID NO: 105) | CCTATCCTAAAATAAATCCCAA (SEQ ID NO: 1147) GGAGAAAGGAGGTTGTAGGTT (SEQ ID NO: 1146) | 441 |
| 90. THRB (SEQ ID NO: 106) | GGGTATTGGTAATTTGGTTAGA (SEQ ID NO: 1148) CACACAACTTCCTCATCATAAA (SEQ ID NO: 1149) | 452 |
| 91. TWIST (SEQ ID NO: 100) | GTGGGGATGAAATGGTTATAG (SEQ ID NO: 1150) AACCCCTTAAAATTCCAAAA (SEQ ID NO: 1151) | 354 |
| 92. IL6 (SEQ ID NO: 99) | CAAAAACAAACTCCCAACTATAC (SEQ ID NO: 1153) AAGGTGGGTATGGATTTTAGA (SEQ ID NO: 1152) | 485 |
| 93. RARA (SEQ ID NO: 108) | CATATACATTTCCATCCTTCCT (SEQ ID NO: 1155) ATAAATATAGGTAGAGGGGGTTTT (SEQ ID NO: 1154) | 305 |
| 94. CDH13 (SEQ ID NO: 70) | GGAAAAGTGGAATTAGTTGGTA (SEQ ID NO: 1156) TCTTCCCTACCTAAAACAAAAA (SEQ ID NO: 1157) | 407 |

(continued)

| No: | Primer: | Amplificate Length: |
|---|---|---|
| 95. CLDN7 (SEQ ID NO: 87) | TCCTTTCTTCCCAACAAATA (SEQ ID NO: 1159) TTGAGTGTAAAGGGTTTAGGTT (SEQ ID NO: 1158) | 345 |
| 96. ESR2 (SEQ ID NO: 91) | AGTTGGAGAAATTGAAAAGATTA (SEQ ID NO: 1160) TAACAAACCCAAAACCTCTCTA (SEQ ID NO: 1161) | 441 |
| 97. IGFBP7 (SEQ ID NO: 94) | CCTCTCTCTACATCCCCAAA (SEQ ID NO: 1163) GGGAGAAGGTTATTATTTAGGTT (SEQ ID NO: 1162) | 414 |
| 98. FHIT (SEQ ID NO: 76) | GGGAGGTAAGTTTAAGTGGAAT (SEQ ID NO: 1164) ACTACACCCCCAAAACCA (SEQ ID NO: 1165) | 302 |
| 99. SERPINB5 (SEQ ID NO: 68) | TTTTTGTTTAATGGGTAGTTATTTT (SEQ ID NO: 1166) CTCCTCTCCTACTCAAACCTC (SEQ ID NO: 1167) | 353 |
| 100. LOT (SEQ ID NO: 95) | AGAGGAAAGAGAGTAGTTGGTG (SEQ ID NO: 1168) TATAAAATTCTCCCAACCAAAA (SEQ ID NO: 1169) | 479 |
| 101. PLAU (SEQ ID NO: 62) | GTGATATTTGGGGATTGTTATT (SEQ ID NO: 1170) ACTCCCTCCCCTATCTTACA (SEQ ID NO: 1171) | 479 |
| 102. PRSS8 (SEQ ID NO: 72) | GGTTTGTAGTTTTGAAAGGATT (SEQ ID NO: 1172) AATACATTATCCCCACCCTAC (SEQ ID NO: 1173) | 481 |
| 103. S100A7 (SEQ ID NO: 96) | TATCCTACCCCCATAACTATCA (SEQ ID NO: 1175) AGTTTGAGATTTGGTTTATTTTG (SEQ ID NO: 1174) | 243 |
| 104. SLC19A1 (SEQ ID NO: 116) | TAGGGGAAAGTGATTTTGA (SEQ ID NO: 1176) TTTTCCTTTTAACACCCTAAAAC (SEQ ID NO: 1177) | 459 |
| 105. SYK (SEQ ID NO: 60) | GTGGGTTTTGGGTAGTTATAGA (SEQ ID NO: 1178) TAACCTCCTCTCCTTACCAA (SEQ ID NO: 1179) | 485 |
| 106. TERT (SEQ ID NO: 92) | TAGGTTTTGGATGTTAGGGATT (SEQ ID NO: 1180) ACCACCTCCTCACCTAAACT (SEQ ID NO: 1181) | 483 |
| 107. GJB2 (SEQ ID NO: 111) | ATTTTGAGGGGAGAAAGAAG (SEQ ID NO: 1182) CCCATAAATTCCCCAAATAC (SEQ ID NO: 1183) | 450 |
| 108. FABP3 (SEQ ID NO: 77) | TAGGAAAGGGAGAAGGTTTTAT (SEQ ID NO: 1184) CTATTCCCCAATCTTAACCAA (SEQ ID NO: 1185) | 488 |
| 109. GPC3 (SEQ ID NO: 118) | TCAAATCTAACAAACCACAAAA (SEQ ID NO: 1187) AATTATTGTAGAGGGGTTGTAGG (SEQ ID NO: 1186) | 489 |

(continued)

| No: | Primer: | Amplificate Length: |
|---|---|---|
| 110. ARH1/NOEY2 (SEQ ID NO: 97) | TGGTAGAAGAGTAGTATTAGTGGTTT (SEQ ID NO: 1188) CCAATCCCTTTTCCAAATA (SEQ ID NO: 1189) | 279 |
| 111. SLIT2 (SEQ ID NO: 112) | AAATTACCCAAACATCCTTCA (SEQ ID NO: 1191) AGGTATAAGATAGAAGAGGGGATTT (SEQ ID NO: 1190) | 309 |
| 112. HS3ST2 (SEQ ID NO: 113) | CAAAAACTTCTCCAAAAATCC (SEQ ID NO: 1193) TATAATGAGGGTTTGGGGTAAT (SEQ ID NO: 1192) | 171 |
| 113. STAT1 (SEQ ID NO: 109) | TGTTTTGGTAGTGAAGAGGATT (SEQ ID NO: 1194) AACATTAAACCCTTCCATCTTT (SEQ ID NO: 1195) | 372 |
| 114. PRDM2 (SEQ ID NO: 114) | CCAACTCATCTCCAACCTATAC (SEQ ID NO: 1197) AGAATTAAAAAGATTGAAGGAGG (SEQ ID NO: 1196) | 438 |
| 115. MCT1 (SEQ ID NO: 101) | CTCTATCACTTCTTCCCTCTCA (SEQ ID NO: 1199) AATTTTAGGGAGTTAGGATGGTA (SEQ ID NO: 1198) | 430 |
| 116. IGSF4 (SEQ ID NO: 74) | TAATCCCCAACTTCCTTAAAAA (SEQ ID NO: 1201) GGAGTGGAGAGTAAGAAGGTAG (SEQ ID NO: 1200) | 489 |
| 117. SCGB3A1 (SEQ ID NO: 115) | TTTAGTGTTTAATGTTTGGGGT (SEQ ID NO: 1202) CAATTCCTAACTCCCTAATCC (SEQ ID NO: 1203) | 395 |
| 118. AR (SEQ ID NO: 61) | AATATAGGGAGGTTTAGGGTTT (SEQ ID NO: 1204) TAACCATACATTTCTCATCCAA (SEQ ID NO: 1205) | 424 |
| 119. EYA4 (SEQ ID NO: 58) | GGAAGAGGTGATTAAATGGAT (SEQ ID NO: 1206) CCCAAAAATCAAACAACAA (SEQ ID NO: 1207) | 226 |
| 120. LEF1 (SEQ ID NO: 63) | ATAGAATGGTTAGGGGGTATTT (SEQ ID NO: 1209) TACAAATATCAACCTCTCTCCC (SEQ ID NO: 1208) | 484 |
| 121. ALX4 (SEQ ID NO: 64) | CTCCTCCTTCCAACAAAAA (SEQ ID NO: 1210) GTTTAGAGGTTTTGGGATGATT (SEQ ID NO: 1211) | 487 |
| 122. SEQ ID NO:2 (SEQ ID NO: 2) | TCCAATAAACACAAACCTAAATC (SEQ ID NO: 1212) ATATGGGATTGATGGAAGATAG (SEQ ID NO: 1213) | 471 |
| 123. CCND2 (SEQ ID NO: 104) | GGAGGAAGGAGGTGAAGA (SEQ ID NO: 1214) CCCCTACATCTACTAACAAACC (SEQ ID NO: 1215) | 378 |

(continued)

| No: | Primer: | Amplificate Length: |
|---|---|---|
| 124. CDKN2A (SEQ ID NO: 57) | GGGGTTGGTTGGTTATTAGA (SEQ ID NO: 1216) AACCCTCTACCCACCTAAAT (SEQ ID NO: 1217) | 256 |

Table 2: Detection oligonucleotides according to Example 2.

| No: | Gene | Oligo: |
|---|---|---|
| 1 | SEQ ID NO:5 (SEQ ID NO: 5) | AATGAGCGAGAAAGTA (SEQ ID NO: 1970) |
| 2 | SEQ ID NO:5 (SEQ ID NO: 5) | AGAATGAGTGAGAAAGT (SEQ ID NO: 1971) |
| 3 | SEQ ID NO:5 (SEQ ID NO: 5) | ATTAAACGGGATGGTT (SEQ ID NO: 1972) |
| 4 | SEQ ID NO:5 (SEQ ID NO: 5) | AATATTAAATGGGATGGT (SEQ ID NO: 1973) |
| 5 | SEQ ID NO:5 (SEQ ID NO: 5) | GAGTTGCGAGGATTTT (SEQ ID NO: 1974) |
| 6 | SEQ ID NO:5 (SEQ ID NO: 5) | GGAGTTGTGAGGATTT (SEQ ID NO: 1975) |
| 7 | SEQ ID NO:5 (SEQ ID NO: 5) | TATGAGGTCGTATTGG (SEQ ID NO: 2196) |
| 8 | SEQ ID NO:5 (SEQ ID NO: 5) | TATGAGGTTGTATTGGT (SEQ ID NO: 2197) |
| 9 | SEQ ID NO:5 (SEQ ID NO: 5) | GGGAATCGTTGATTTT (SEQ ID NO: 1976) |
| 10 | SEQ ID NO:5 (SEQ ID NO: 5) | AGGGGAATTGTTGATT(SEQ ID NO: 1977) |
| 11 | SEQ ID NO:6 (SEQ ID NO: 6) | AAGTTTATCGGCGTTT (SEQ ID NO: 1838) |
| 12 | SEQ ID NO:6 (SEQ ID NO: 6) | AGAAGTTTATTGGTGTTT (SEQ ID NO: 1839) |
| 13 | SEQ ID NO:6 (SEQ ID NO: 6) | ATTTCGGAATTTAAGCGT (SEQ ID NO: 1840) |
| 14 | SEQ ID NO:6 (SEQ ID NO: 6) | TTTTGGAATTTAAGTGTT (SEQ ID NO: 1841) |
| 15 | SEQ ID NO:6 (SEQ ID NO: 6) | TAATTTCGGACGCGGA (SEQ ID NO: 1842) |
| 16 | SEQ ID NO:6 (SEQ ID NO: 6) | TTTTGGATGTGGAGGA (SEQ ID NO: 1843) |
| 17 | SEQ ID NO:6 (SEQ ID NO: 6) | TTACGGTGAAGGCGGA (SEQ ID NO: 1844) |
| 18 | SEQ ID NO:6 (SEQ ID NO: 6) | TTATGGTGAAGGTGGA (SEQ ID NO: 1845) |
| 19 | SEQ ID NO:6 (SEQ ID NO: 6) | TTTCGGTTTTCGTTAAT (SEQ ID NO: 1846) |
| 20 | SEQ ID NO:6 (SEQ ID NO: 6) | TTTGGTTTTTGTTAATTTAG (SEQ ID NO: 1847) |
| 21 | SEQ ID NO:6 (SEQ ID NO: 6) | TGTGCGAAGTTAACGT (SEQ ID NO: 1848) |
| 22 | SEQ ID NO:6 (SEQ ID NO: 6) | TTGTGTGAAGTTAATGT (SEQ ID NO: 1849) |
| 23 | SEQ ID NO:7 (SEQ ID NO: 7) | TAGTCGTCGTGTAGGA (SEQ ID NO: 1978) |
| 24 | SEQ ID NO:7 (SEQ ID NO: 7) | TAGGTAGTTGTTGTGTA (SEQ ID NO: 1979) |
| 25 | SEQ ID NO:7 (SEQ ID NO: 7) | TATAGGTACGCGATGA (SEQ ID NO: 1980) |
| 26 | SEQ ID NO:7 (SEQ ID NO: 7) | AGGTATGTGATGAGGA (SEQ ID NO: 1981) |
| 27 | SEQ ID NO:7 (SEQ ID NO: 7) | TATGGTTACGTACGAG (SEQ ID NO: 1982) |
| 28 | SEQ ID NO:7 (SEQ ID NO: 7) | ATGGTTATGTATGAGTTT (SEQ ID NO: 1983) |
| 29 | SEQ ID NO:7 (SEQ ID NO: 7) | ATGATTTGCGTTACGT (SEQ ID NO: 1984) |
| 30 | SEQ ID NO:7 (SEQ ID NO: 7) | ATGATTTGTGTTATGTTT (SEQ ID NO: 1985) |
| 31 | SEQ ID NO:7 (SEQ ID NO: 7) | TAACGTTGTGGTTCGAA (SEQ ID NO: 1986) |
| 32 | SEQ ID NO:7 (SEQ ID NO: 7) | TAATGTTGTGGTTTGAA (SEQ ID NO: 1987) |

(continued)

| No: | Gene | Oligo: |
|---|---|---|
| 33 | SEQ ID NO:8 (SEQ ID NO: 8) | ATAGGGCGGGATTTTA (SEQ ID NO: 2186) |
| 34 | SEQ ID NO:8 (SEQ ID NO: 8) | GATAGGGTGGGATTTT (SEQ ID NO: 2187) |
| 35 | SEQ ID NO:8 (SEQ ID NO: 8) | ATAAAGCGGGGTTTTA (SEQ ID NO: 1850) |
| 36 | SEQ ID NO:8 (SEQ ID NO: 8) | GGATAAAGTGGGGTTT (SEQ ID NO: 1851) |
| 37 | SEQ ID NO:8 (SEQ ID NO: 8) | AGGAGGCGAGAAATTT (SEQ ID NO: 1852) |
| 38 | SEQ ID NO:8 (SEQ ID NO: 8) | GAGGAGGTGAGAAATT (SEQ ID NO: 1853) |
| 39 | SEQ ID NO:8 (SEQ ID NO: 8) | AGAAATTTCGGGGTAG (SEQ ID NO: 1854) |
| 40 | SEQ ID NO:8 (SEQ ID NO: 8) | GAAATTTTGGGGTAGTA (SEQ ID NO: 1855) |
| 41 | SEQ ID NO:8 (SEQ ID NO: 8) | GGTAGTATCGTTTATAGA (SEQ ID NO: 1856) |
| 42 | SEQ ID NO:8 (SEQ ID NO: 8) | GGGGTAGTATTGTTTATA (SEQ ID NO: 1857) |
| 43 | SEQ ID NO:1 (SEQ ID NO: 1) | GGTCGGCGTTGATTTTA (SEQ ID NO: 1988) |
| 44 | SEQ ID NO:1 (SEQ ID NO: 1) | GGTTGGTGTTGATTTTA (SEQ ID NO: 1989) |
| 45 | SEQ ID NO:1 (SEQ ID NO: 1) | GATTCGAACGGATTTT (SEQ ID NO: 1990) |
| 46 | SEQ ID NO:1 (SEQ ID NO: 1) | GGGATTTGAATGGATTT (SEQ ID NO: 1991) |
| 47 | SEQ ID NO:1 (SEQ ID NO: 1) | TGGGTCGGGATTCGAA (SEQ ID NO: 1992) |
| 48 | SEQ ID NO:1 (SEQ ID NO: 1) | TGGGTTGGGATTTGAA (SEQ ID NO: 1993) |
| 49 | SEQ ID NO:1 (SEQ ID NO: 1) | GTCGGAAGTTTCGGGA (SEQ ID NO: 1994) |
| 50 | SEQ ID NO:1 (SEQ ID NO: 1) | GTTGGAAGTTTTGGGAT (SEQ ID NO: 1995) |
| 51 | SEQ ID NO:1 (SEQ ID NO: 1) | TGGATATCGTAGGGTA (SEQ ID NO: 1996) |
| 52 | SEQ ID NO:1 (SEQ ID NO: 1) | TGGATATTGTAGGGTAG (SEQ ID NO: 1997) |
| 53 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGTGTATCGTTTTTCGG (SEQ ID NO: 1858) |
| 54 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAGTGTATTGTTTTTTGG (SEQ ID NO: 1859) |
| 55 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TTCGTTTACGGTAGTT (SEQ ID NO: 1218) |
| 56 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | ATTTTTGTTTATGGTAGTT (SEQ ID NO: 1219) |
| 57 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TGCGTATCGTTAGTTA (SEQ ID NO: 1860) |
| 58 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTTGTGTATTGTTAG (SEQ ID NO: 1861) |
| 59 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1998) |

(continued)

| No: | Gene | Oligo: |
|---|---|---|
| 60 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1999) |
| 61 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | ATTATTTCGGCGGTGA (SEQ ID NO: 1862) |
| 62 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GATTATTTTGGTGGTGA (SEQ ID NO: 1863) |
| 63 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GGCGTCGAAAGTCGTT (SEQ ID NO: 2000) |
| 64 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GGTGTTGAAAGTTGTTG (SEQ ID NO: 2001) |
| 65 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAAGTCGCGTAAGGAG (SEQ ID NO: 1864) |
| 66 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AAGTTGTGTAAGGAGTA (SEQ ID NO: 1865) |
| 67 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAATCGTTTGTTTACGT (SEQ ID NO: 2002) |
| 68 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AATTGTTTGTTTATGTAGT (SEQ ID NO: 2003) |
| 69 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATCGCGAGTTTGGA (SEQ ID NO: 1866) |
| 70 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATTGTGAGTTTGGAG (SEQ ID NO: 1867) |
| 71 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTACGGAGGCGTTTTA (SEQ ID NO: 2004) |
| 72 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTATGGAGGTGTTTT (SEQ ID NO: 2005) |
| 73 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | AGGCGAATTTATCGGG (SEQ ID NO: 2006) |

(continued)

| No: | Gene | Oligo: |
|---|---|---|
| 74 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | GGTGAATTTATTGGGG (SEQ ID NO: 2007) |
| 75 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTCGAAGTAGGCGT (SEQ ID NO: 2008) |
| 76 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B 1-BINDING FACTOR) (HB1F) (CYP7A PROMOTER BINDING FACTOR) (SEQ ID NO: 11) | TAGTTGAAGTAGGTGTT (SEQ ID NO: 2009) |
| 77 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTCGACGAAGTTTT (SEQ ID NO: 2010) |
| 78 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTGATGAAGTTTTGTT (SEQ ID NO: 2011) |
| 79 | LIM DOMAIN KINASE 1 (EC 2.7.1.37) (LIMK-1) (SEQ ID NO: 12) | TGTAGTCGGGAGGTTA (SEQ ID NO: 2012) |
| 80 | LIM DOMAIN KINASE 1 (EC 2.7.1.37) (LIMK-1) (SEQ ID NO: 12) | TGTAGTTGGGAGGTTA (SEQ ID NO: 2013) |
| 81 | LIM DOMAIN KINASE 1 (EC 2.7.1.37) (LIMK-1) (SEQ ID NO: 12) | GGATTATCGCGGGGGT (SEQ ID NO: 2014) |
| 82 | LIM DOMAIN KINASE 1 (EC 2.7.1.37) (LIMK-1) (SEQ ID NO: 12) | GGATTATTGTGGGGGT (SEQ ID NO: 2015) |
| 83 | LIM DOMAIN KINASE 1 (EC 2.7.1.37) (LIMK-1) (SEQ ID NO: 12) | GTCGGTAGTTTATCGGAT (SEQ ID NO: 2016) |
| 84 | LIM DOMAIN KINASE 1 (EC 2.7.1.37) (LIMK-1) (SEQ ID NO: 12) | GTTGGTAGTTTATTGGAT (SEQ ID NO: 2017) |
| 85 | LIM DOMAIN KINASE 1 (EC 2.7.1.37) (LIMK-1) (SEQ ID NO: 12) | TAGGAGACGTTACGTT (SEQ ID NO: 2018) |
| 86 | LIM DOMAIN KINASE 1 (EC 2.7.1.37) (LIMK-1) (SEQ ID NO: 12) | AGATGTTATGTTAGGGT (SEQ ID NO: 2019) |
| 87 | KOX7 (SEQ ID NO: 49) | TAATTAGGCGCGAGGT (SEQ ID NO: 1220) |
| 88 | KOX7 (SEQ ID NO: 49) | TTAGGTGTGAGGTAGA (SEQ ID NO: 1221) |
| 89 | KOX7 (SEQ ID NO: 49) | TTAAAGTCGTGGGCGG (SEQ ID NO: 1222) |
| 90 | KOX7 (SEQ ID NO: 49) | TTAAAGTTGTGGGTGG (SEQ ID NO: 1223) |
| 91 | KOX7 (SEQ ID NO: 49) | GGTCGGGTTATAACGTA (SEQ ID NO: 1224) |

(continued)

| No: | Gene | Oligo: |
|-----|------|--------|
| 92 | KOX7 (SEQ ID NO: 49) | GGGTTGGGGTTATAATGT (SEQ ID NO: 1225) |
| 93 | KOX7 (SEQ ID NO: 49) | TAAACGTTTTTCGGGA (SEQ ID NO: 1226) |
| 94 | KOX7 (SEQ ID NO: 49) | GGTAAATGTTTTTTGGG (SEQ ID NO: 1227) |
| 95 | BCL11B (SEQ ID NO: 50) | TAGGTTTCGATTCGTT (SEQ ID NO: 2020) |
| 96 | BCL11B (SEQ ID NO: 50) | TTAGGTTTTGATTTGTTT (SEQ ID NO: 2021) |
| 97 | BCL11B (SEQ ID NO: 50) | AAGTCGTCGGAGTTAG (SEQ ID NO: 2022) |
| 98 | BCL11B (SEQ ID NO: 50) | GTGAAGTTGTTGGAGT (SEQ ID NO: 2023) |
| 99 | BCL11B (SEQ ID NO: 50) | TTGAGGCGTTACGGTT (SEQ ID NO: 2024) |
| 100 | BCL11B (SEQ ID NO: 50) | TTGAGGTGTTATGGTT (SEQ ID NO: 2025) |
| 101 | BCL11B (SEQ ID NO: 50) | TTCGGGACGAAAATAA (SEQ ID NO: 1228) |
| 102 | BCL11B (SEQ ID NO: 50) | AAGATTTTTGGGATGAA (SEQ ID NO: 1229) |
| 103 | BCL11B (SEQ ID NO: 50) | GTTTTCGTTTACGGAT (SEQ ID NO: 1230) |
| 104 | BCL11B (SEQ ID NO: 50) | TGTTTTTGTTTATGGATT (SEQ ID NO: 1231) |
| 105 | SEQ ID NO:51 (SEQ ID NO: 51) | TAAATTTCGGACGAGT (SEQ ID NO: 1232) |
| 106 | SEQ ID NO:51 (SEQ ID NO: 51) | TTTAAATTTTGGATGAGTT (SEQ ID NO: 1233) |
| 107 | SEQ ID NO:51 (SEQ ID NO:51) | TTTACGGAGTTTCGGT (SEQ ID NO: 1234) |
| 108 | SEQ ID NO:51 (SEQ ID NO: 51) | TTTTATGGAGTTTTGGT (SEQ ID NO: 1235) |
| 109 | SEQ ID NO:51 (SEQ ID NO: 51) | GTCGGGGTCGATTCGA (SEQ ID NO: 1868) |
| 110 | SEQ ID NO:51 (SEQ ID NO: 51) | GTTGGGGTTGATTTGAT (SEQ ID NO: 1869) |
| 111 | SEQ ID NO:51 (SEQ ID NO: 51) | AGGATTCGTTTGCGTT (SEQ ID NO: 1870) |
| 112 | SEQ ID NO:51 (SEQ ID NO: 51) | AAGGATTTGTTTGTGTT (SEQ ID NO: 1871) |
| 113 | SEQ ID NO:51 (SEQ ID NO: 51) | TAGACGTTAGAGAACGT (SEQ ID NO: 1236) |
| 114 | SEQ ID NO:51 (SEQ ID NO: 51) | AGATGTTAGAGAATGTTT (SEQ ID NO: 1237) |
| 115 | MGC34831 (SEQ ID NO: 52) | ATTAGCGTTTGGCGTT (SEQ ID NO: 1872) |
| 116 | MGC34831 (SEQ ID NO: 52) | AATTAGTGTTTGGTGTT (SEQ ID NO: 1873) |
| 117 | MGC34831 (SEQ ID NO: 52) | GTTTAGCGACGGTCGT (SEQ ID NO: 1874) |
| 118 | MGC34831 (SEQ ID NO: 52) | TGTTTAGTGATGGTTGT (SEQ ID NO: 1875) |
| 119 | MGC34831 (SEQ ID NO: 52) | GTCGGACGGATTTATA (SEQ ID NO: 2210) |
| 120 | MGC34831 (SEQ ID NO: 52) | TGGTTGGATGGATTTAT (SEQ ID NO: 2211) |
| 121 | MGC34831 (SEQ ID NO: 52) | TTCGTTTTTCGGGATA (SEQ ID NO: 1238) |
| 122 | MGC34831 (SEQ ID NO: 52) | TTTGTTTTTTGGGATATG (SEQ ID NO: 1239) |
| 123 | COL5A1 (SEQ ID NO: 53) | AGGGATAGCGTTTTTT (SEQ ID NO: 1240) |
| 124 | COL5A1 (SEQ ID NO: 53) | AGGGATAGTGTTTTTTG (SEQ ID NO: 1241) |
| 125 | COL5A1 (SEQ ID NO: 53) | TAAGTGTTCGGGGGTA (SEQ ID NO: 1242) |
| 126 | COL5A1 (SEQ ID NO: 53) | TAAGTGTTTGGGGGTA (SEQ ID NO: 1243) |
| 127 | COL5A1 (SEQ ID NO: 53) | TACGAGGCGGGTAAAT (SEQ ID NO: 1244) |
| 128 | COL5A1 (SEQ ID NO: 53) | GTTTATGAGGTGGGTA (SEQ ID NO: 1245) |
| 129 | COL5A1 (SEQ ID NO: 53) | TATAGGCGTTTAGTTTG (SEQ ID NO: 1246) |

(continued)

| No: | Gene | Oligo: |
|---|---|---|
| 130 | COL5A1 (SEQ ID NO: 53) | ATTATAGGTGTTTAGTTTG (SEQ ID NO: 1247) |
| 131 | COL5A1 (SEQ ID NO: 53) | TTAAATCGGGTAGGGT (SEQ ID NO: 1248) |
| 132 | COL5A1 (SEQ ID NO: 53) | TTTAAATTGGGTAGGGT (SEQ ID NO: 1249) |
| 133 | SEQ ID NO:54 (SEQ ID NO: 54) | TGTTATCGCGTAGGTT (SEQ ID NO: 1250) |
| 134 | SEQ ID NO:54 (SEQ ID NO: 54) | GTGTTATTGTGTAGGTT (SEQ ID NO: 1251) |
| 135 | SEQ ID NO:54 (SEQ ID NO: 54) | AGCGATGTATTCGTTT (SEQ ID NO: 1252) |
| 136 | SEQ ID NO:54 (SEQ ID NO: 54) | GAGAGTGATGTATTTGT (SEQ ID NO: 1253) |
| 137 | SEQ ID NO:54 (SEQ ID NO: 54) | TGTGTAGCGGCGATTA (SEQ ID NO: 1876) |
| 138 | SEQ ID NO:54 (SEQ ID NO: 54) | GTGTGTAGTGGTGATT (SEQ ID NO: 1877) |
| 139 | SEQ ID NO:54 (SEQ ID NO: 54) | ATTAGCGTTTGGTCGG (SEQ ID NO: 1878) |
| 140 | SEQ ID NO:54 (SEQ ID NO: 54) | ATTAGTGTTTGGTTGGG (SEQ ID NO: 1879) |
| 141 | SEQ ID NO:54 (SEQ ID NO: 54) | GACGGGGCGAGTTAGT (SEQ ID NO: 1254) |
| 142 | SEQ ID NO:54 (SEQ ID NO: 54) | GATGGGGTGAGTTAGT (SEQ ID NO: 1255) |
| 143 | PDLIM1 (SEQ ID NO: 55) | GATTCGCGGGGATAGA (SEQ ID NO: 1256) |
| 144 | PDLIM1 (SEQ ID NO: 55) | GATTTGTGGGGATAGA (SEQ ID NO: 1257) |
| 145 | PDLIM1 (SEQ ID NO: 55) | TAATCGCGAGGGTTAG (SEQ ID NO: 1258) |
| 146 | PDLIM1 (SEQ ID NO: 55) | GGTAATTGTGAGGGTT (SEQ ID NO: 1259) |
| 147 | PDLIM1 (SEQ ID NO: 55) | TAGGTCGCGTAGTCGT (SEQ ID NO: 1880) |
| 148 | PDLIM1 (SEQ ID NO: 55) | TTAGGTTGTGTAGTTGT (SEQ ID NO: 1881) |
| 149 | PDLIM1 (SEQ ID NO: 55) | GAGTTTCGTCGAGAGA (SEQ ID NO: 1260) |
| 150 | PDLIM1 (SEQ ID NO: 55) | GGAGTTTTGTTGAGAGA (SEQ ID NO: 1261) |
| 151 | PDLIM1 (SEQ ID NO: 55) | AGAGCGCGAGTTAGAT (SEQ ID NO: 1262) |
| 152 | PDLIM1 (SEQ ID NO: 55) | AGAGAGTGTGAGTTAGA (SEQ ID NO: 1263) |
| 153 | MSF (SEQ ID NO: 13) | GTTTCGAAATTGGCGT (SEQ ID NO: 2026) |
| 154 | MSF (SEQ ID NO: 13) | TTTGAAATTGGTGTGG (SEQ ID NO: 2027) |
| 155 | MSF (SEQ ID NO: 13) | TTCGGTTTACGGGTTGTA (SEQ ID NO: 2028) |
| 156 | MSF (SEQ ID NO: 13) | TTTGGTTTATGGGTTGTA (SEQ ID NO: 2029) |
| 157 | MSF (SEQ ID NO: 13) | TTACGGTTCGATTTTG (SEQ ID NO: 2030) |
| 158 | MSF (SEQ ID NO: 13) | TATGGTTTGATTTTGGG (SEQ ID NO: 2031) |
| 159 | MSF (SEQ ID NO: 13) | TTCGTACGTAGGTTTT (SEQ ID NO: 1264) |
| 160 | MSF (SEQ ID NO: 13) | TTTGTTTGTATGTAGGTT (SEQ ID NO: 1265) |
| 161 | MSF (SEQ ID NO: 13) | TAGGAAAGCGTACGTA (SEQ ID NO: 1266) |
| 162 | MSF (SEQ ID NO: 13) | AGGAAAGTGTATGTATTT (SEQ ID NO: 1267) |
| 163 | SEQ ID NO:14 (SEQ ID NO: 14) | TAAGGCGTTTTCGATA (SEQ ID NO: 2032) |
| 164 | SEQ ID NO: 14 (SEQ ID NO: 14) | GTAAGGTGTTTTTGATAT (SEQ ID NO: 2033) |
| 165 | SEQ ID NO:14 (SEQ ID NO: 14) | TGGGTGTACGCGTGAA (SEQ ID NO: 2034) |
| 166 | SEQ ID NO:14 (SEQ ID NO: 14) | TGTATGTGTGAAGGGG (SEQ ID NO: 2035) |
| 167 | SEQ ID NO: 14 (SEQ ID NO: 14) | AAATACGTATTTTCGGT (SEQ ID NO: 1268) |

(continued)

| No: | Gene | Oligo: |
|---|---|---|
| 168 | SEQ ID NO: 14 (SEQ ID NO: 14) | ATATGTATTTTTGGTAGTT (SEQ ID NO: 1269) |
| 169 | SEQ ID NO: 15 (SEQ ID NO: 15) | AATCGTGCGGTTGATA (SEQ ID NO: 1882) |
| 170 | SEQ ID NO: 15 (SEQ ID NO: 15) | TGTAGAATTGTGTGGT (SEQ ID NO: 1883) |
| 171 | SEQ ID NO:15 (SEQ ID NO: 15) | TTGCGTAGAAAATTCGAG (SEQ ID NO: 1884) |
| 172 | SEQ ID NO: 15 (SEQ ID NO: 15) | TTGTGTAGAAAATTTGAG (SEQ ID NO: 1885) |
| 173 | SEQ ID NO:15 (SEQ ID NO: 15) | AAAATTCGAGGTCGGG (SEQ ID NO: 1886) |
| 174 | SEQ ID NO:15 (SEQ ID NO: 15) | AAAATTTGAGGTTGGG (SEQ ID NO: 1887) |
| 175 | SEQ ID NO:15 (SEQ ID NO: 15) | AATAGGCGATGTACGG (SEQ ID NO: 2214) |
| 176 | SEQ ID NO:15 (SEQ ID NO: 15) | TAGGTGATGTATGGGT (SEQ ID NO: 2215) |
| 177 | SEQ ID NO:15 (SEQ ID NO: 15) | TAATCGAGTTTAGCGG (SEQ ID NO: 2216) |
| 178 | SEQ ID NO:15 (SEQ ID NO: 15) | TTAATTGAGTTTAGTGGT (SEQ ID NO: 2217) |
| 179 | SEQ ID NO: 16 (SEQ ID NO: 16) | ATCGTTGGTCGGATTT (SEQ ID NO: 2036) |
| 180 | SEQ ID NO:16 (SEQ ID NO: 16) | TAGGATTGTTGGTTGGA (SEQ ID NO: 2037) |
| 181 | SEQ ID NO:16 (SEQ ID NO: 16) | AGGAGTTTTCGTGTCGT (SEQ ID NO: 2038) |
| 182 | SEQ ID NO: 16 (SEQ ID NO: 16) | AGGAGTTTTTGTGTTGT (SEQ ID NO: 2039) |
| 183 | SEQ ID NO: 16 (SEQ ID NO: 16) | TTACGGATAGGGCGAT (SEQ ID NO: 2040) |
| 184 | SEQ ID NO:16 (SEQ ID NO: 16) | TATGGATAGGGTGATTT (SEQ ID NO: 2041) |
| 185 | SEQ ID NO: 16 (SEQ ID NO: 16) | AGGCGTTGTCGGTGAT (SEQ ID NO: 2042) |
| 186 | SEQ ID NO: 16 (SEQ ID NO: 16) | AGGTGTTGTTGGTGATA (SEQ ID NO: 2043) |
| 187 | SEQ ID NO:16 (SEQ ID NO: 16) | AAGTAGATCGGGACGA (SEQ ID NO: 1270) |
| 188 | SEQ ID NO:16 (SEQ ID NO: 16) | TAGATTGGGATGAGGA (SEQ ID NO: 1271) |
| 189 | SEQ ID NO:17 (SEQ ID NO: 17) | TACGTTTCGGGTTTGTTA (SEQ ID NO: 2044) |
| 190 | SEQ ID NO: 17 (SEQ ID NO: 17) | TATGTTTTGGGTTTGTTA (SEQ ID NO: 2045) |
| 191 | SEQ ID NO: 17 (SEQ ID NO: 17) | ATTTAGTCGTGCGTTT (SEQ ID NO: 2046) |
| 192 | SEQ ID NO: 17 (SEQ ID NO: 17) | TGATTTAGTTGTGTGTT (SEQ ID NO: 2047) |
| 193 | SEQ ID NO:17 (SEQ ID NO: 17) | GGTGATTTCGACGGTT (SEQ ID NO: 1272) |
| 194 | SEQ ID NO:17 (SEQ ID NO: 17) | AGGTGATTTTGATGGTT (SEQ ID NO: 1273) |
| 195 | SEQ ID NO:17 (SEQ ID NO: 17) | AGTAGCGTAATTTCGA (SEQ ID NO: 1274) |
| 196 | SEQ ID NO:17 (SEQ ID NO: 17) | AGTGTAATTTTGAGGTG (SEQ ID NO: 1275) |
| 197 | SEQ ID NO:17 (SEQ ID NO: 17) | TATGGCGCGTATGTAT (SEQ ID NO: 1276) |
| 198 | SEQ ID NO: 17 (SEQ ID NO: 17) | GATTATGGTGTGTATGT (SEQ ID NO: 1277) |
| 199 | SEQ ID NO: 18 (SEQ ID NO: 18) | TTTACGCGGGGTTTTA (SEQ ID NO: 2048) |
| 200 | SEQ ID NO: 18 (SEQ ID NO: 18) | TTTATGTGGGGTTTTAG (SEQ ID NO: 2049) |
| 201 | SEQ ID NO:18 (SEQ ID NO: 18) | TTACGTCGTTATTAGGT (SEQ ID NO: 2050) |
| 202 | SEQ ID NO: 18 (SEQ ID NO: 18) | TTTTATGTTGTTATTAGGT (SEQ ID NO: 2051) |
| 203 | SEQ ID NO: 18 (SEQ ID NO: 18) | TATTTGGACGTCGGGT (SEQ ID NO: 2052) |
| 204 | SEQ ID NO: 18 (SEQ ID NO: 18) | TATTTGGATGTTGGGT (SEQ ID NO: 2053) |
| 205 | SEQ ID NO: 18 (SEQ ID NO: 18) | GAGGCGTATTAGGTCGG (SEQ ID NO: 2054) |

(continued)

| No: | Gene | Oligo: |
|-----|------|--------|
| 206 | SEQ ID NO:18 (SEQ ID NO: 18) | AGGTGTATTAGGTTGGG (SEQ ID NO: 2055) |
| 207 | SEQ ID NO: 18 (SEQ ID NO: 18) | AAAGCGGAGTCGTTAG (SEQ ID NO: 2056) |
| 208 | SEQ ID NO: 18 (SEQ ID NO: 18) | AGTGGAGTTGTTAGGT (SEQ ID NO: 2057) |
| 209 | SEQ ID NO: 19 (SEQ ID NO: 19) | AGGTTTTCGTTGTAGTA (SEQ ID NO: 2058) |
| 210 | SEQ ID NO: 19 (SEQ ID NO: 19) | TAGGTTTTTGTTGTAGTA (SEQ ID NO: 2059) |
| 211 | SEQ ID NO:19 (SEQ ID NO: 19) | TTTGATATCGAGGGAG (SEQ ID NO: 2198) |
| 212 | SEQ ID NO: 19 (SEQ ID NO: 19) | TTTGATATTGAGGGAGG (SEQ ID NO: 2199) |
| 213 | SEQ ID NO: 19 (SEQ ID NO: 19) | GGTGTACGGAGGAAAG (SEQ ID NO: 2200) |
| 214 | SEQ ID NO:19 (SEQ ID NO: 19) | GGTGTATGGAGGAAAG (SEQ ID NO: 2201) |
| 215 | SEQ ID NO: 19 (SEQ ID NO: 19) | TGAGATTCGTTTTTTAAA (SEQ ID NO: 2060) |
| 216 | SEQ ID NO:19 (SEQ ID NO: 19) | GGTGAGATTTGTTTTTTA (SEQ ID NO: 2061) |
| 217 | PRDM6 (SEQ ID NO: 20) | AGTTTTAAGCGTTTGGT (SEQ ID NO: 2062) |
| 218 | PRDM6 (SEQ ID NO: 20) | AGTTTTAAGTGTTTGGT (SEQ ID NO: 2063) |
| 219 | PRDM6 (SEQ ID NO: 20) | GAAGTTCGGATTTCGG (SEQ ID NO: 2064) |
| 220 | PRDM6 (SEQ ID NO: 20) | GGAAGTTTGGATTTTGG (SEQ ID NO: 2065) |
| 221 | PRDM6 (SEQ ID NO: 20) | TTGTCGGGTTACGGGA (SEQ ID NO: 2066) |
| 222 | PRDM6 (SEQ ID NO: 20) | GTTGGGTTATGGGAGA (SEQ ID NO: 2067) |
| 223 | PRDM6 (SEQ ID NO: 20) | TTCGTAGAATTGTCGAAG (SEQ ID NO: 2068) |
| 224 | PRDM6 (SEQ ID NO: 20) | TTTGTAGAATTGTTGAAG (SEQ ID NO: 2069) |
| 225 | RAP2B (SEQ ID NO: 21) | AGATGTTTCGATTTGTT (SEQ ID NO: 1278) |
| 226 | RAP2B (SEQ ID NO: 21) | GAGATGTTTTGATTTGTT (SEQ ID NO: 1279) |
| 227 | RAP2B (SEQ ID NO: 21) | GGGTAATCGTTAGATTT (SEQ ID NO: 1280) |
| 228 | RAP2B (SEQ ID NO: 21) | GGTAATTGTTAGATTTGG (SEQ ID NO: 1281) |
| 229 | RAP2B (SEQ ID NO: 21) | GGTCGGGTAATCGTTA (SEQ ID NO: 2070) |
| 230 | RAP2B (SEQ ID NO: 21) | GTTGGGTAATTGTTAGA (SEQ ID NO: 2071) |
| 231 | RAP2B (SEQ ID NO: 21) | AGGAAGCGTTTTCGTT (SEQ ID NO: 2072) |
| 232 | RAP2B (SEQ ID NO: 21) | AGAGGAAGTGTTTTTGT (SEQ ID NO: 2073) |
| 233 | NR2E1 (SEQ ID NO: 22) | AATGTAGCGGCGTTAT (SEQ ID NO: 2074) |
| 234 | NR2E1 (SEQ ID NO: 22) | TAAATGTAGTGGTGTTAT (SEQ ID NO: 2075) |
| 235 | NR2E1 (SEQ ID NO: 22) | GTCGTTATCGGTTTGGA (SEQ ID NO: 2076) |
| 236 | NR2E1 (SEQ ID NO: 22) | GTTGTTATTGGTTTGGA (SEQ ID NO: 2077) |
| 237 | NR2E1 (SEQ ID NO: 22) | GACGTAAGTTTCGGGT (SEQ ID NO: 2078) |
| 238 | NR2E1 (SEQ ID NO: 22) | GGATGTAAGTTTTGGG (SEQ ID NO: 2079) |
| 239 | NR2E1 (SEQ ID NO: 22) | TTTTTAGTCGCGAGAA (SEQ ID NO: 2080) |
| 240 | NR2E1 (SEQ ID NO: 22) | TTTTTAGTTGTGAGAAGT (SEQ ID NO: 2081) |
| 241 | NR2E1 (SEQ ID NO: 22) | TTCGGGTGATATCGTTT (SEQ ID NO: 2082) |
| 242 | NR2E1 (SEQ ID NO: 22) | TTTGGGTGATATTGTTT (SEQ ID NO: 2083) |
| 243 | NR2E1 (SEQ ID NO: 22) | AGGCGAGTCGGAGTTT (SEQ ID NO: 2084) |

(continued)

| No: | Gene | Oligo: |
|---|---|---|
| 244 | NR2E1 (SEQ ID NO: 22) | AGGTGAGTTGGAGTTTT (SEQ ID NO: 2085) |
| 245 | NR2E1 (SEQ ID NO: 22) | TAAGTCGAGCGAGTTT (SEQ ID NO: 2086) |
| 246 | NR2E1 (SEQ ID NO: 22) | TAGTAAGTTGAGTGAGT (SEQ ID NO: 2087) |
| 247 | NR2E1 (SEQ ID NO: 22) | AGGGACGCGAAAATTT (SEQ ID NO: 2088) |
| 248 | NR2E1 (SEQ ID NO: 22) | GGAGGGATGTGAAAAT (SEQ ID NO: 2089) |
| 249 | PCDH7 (SEQ ID NO: 23) | ATCGTAGTCGGTTTTA (SEQ ID NO: 2090) |
| 250 | PCDH7 (SEQ ID NO: 23) | GATTATTGTAGTTGGTTT (SEQ ID NO: 2091) |
| 251 | PCDH7 (SEQ ID NO: 23) | AAAGTGTAGCGTTATTT (SEQ ID NO: 1282) |
| 252 | PCDH7 (SEQ ID NO: 23) | AAAGTGTAGTGTTATTTAT (SEQ ID NO: 1283) |
| 253 | PCDH7 (SEQ ID NO: 23) | TTGGAAATTTCGATATTAT (SEQ ID NO: 1284) |
| 254 | PCDH7 (SEQ ID NO: 23) | TGGAAATTTTGATATTATTT (SEQ ID NO: 1285) |
| 255 | DKK3 (SEQ ID NO: 24) | ATTCGTTTTAGTTCGAG (SEQ ID NO: 1888) |
| 256 | DKK3 (SEQ ID NO: 24) | ATTTGTTTTAGTTTGAGT (SEQ ID NO: 1889) |
| 257 | DKK3 (SEQ ID NO: 24) | TTCGATCGTTTTAGGA (SEQ ID NO: 1890) |
| 258 | DKK3 (SEQ ID NO: 24) | AGTTTTGATTGTTTTAGG (SEQ ID NO: 1891) |
| 259 | DKK3 (SEQ ID NO: 24) | TTTCGGAACGCGATTA (SEQ ID NO: 1286) |
| 260 | DKK3 (SEQ ID NO: 24) | GTGTTTTGGAATGTGA (SEQ ID NO: 1287) |
| 261 | DKK3 (SEQ ID NO: 24) | ATTCGTTCGGAGACGG (SEQ ID NO: 1892) |
| 262 | DKK3 (SEQ ID NO: 24) | TTTGTTTGGAGATGGG (SEQ ID NO: 1893) |
| 263 | DKK3 (SEQ ID NO: 24) | GAAAAGACGCGATTTT (SEQ ID NO: 1894) |
| 264 | DKK3 (SEQ ID NO: 24) | GAAAAGATGTGATTTTATT (SEQ ID NO: 1895) |
| 265 | RTTN (SEQ ID NO: 25) | AATGGTCGTGTTACGT (SEQ ID NO: 1288) |
| 266 | RTTN (SEQ ID NO: 25) | ATGGTTGTGTTATGTTT (SEQ ID NO: 1289) |
| 267 | RTTN (SEQ ID NO: 25) | TAGATTGACGGGACGA (SEQ ID NO: 2228) |
| 268 | RTTN (SEQ ID NO: 25) | TAGATTGATGGGATGAG (SEQ ID NO: 2229) |
| 269 | RTTN (SEQ ID NO: 25) | TAGTGGCGCGGTAGTT (SEQ ID NO: 2092) |
| 270 | RTTN (SEQ ID NO: 25) | TAGTGGTGTGGTAGTTT (SEQ ID NO: 2093) |
| 271 | RTTN (SEQ ID NO: 25) | TAGGACGTGTTTTCGG (SEQ ID NO: 2094) |
| 272 | RTTN (SEQ ID NO: 25) | AGGATGTGTTTTTGGG (SEQ ID NO: 2095) |
| 273 | RTTN (SEQ ID NO: 25) | TTGCGGAATTTATCGTT (SEQ ID NO: 1290) |
| 274 | RTTN (SEQ ID NO:25) | TGTGGAATTTATTGTTTT (SEQ ID NO: 1291) |
| 275 | SNAP25 (SEQ ID NO: 33) | TATTTCGAGTTAGAGTTACGA (SEQ ID NO: 2096) |
| 276 | SNAP25 (SEQ ID NO: 33) | TATTTTGAGTTAGAGTTATGA (SEQ ID NO: 2097) |
| 277 | SNAP25 (SEQ ID NO: 33) | ATACGGAATATCGTATTT (SEQ ID NO: 2098) |
| 278 | SNAP25 (SEQ ID NO: 33) | GTGTATATATGGAATATTGT (SEQ ID NO: 2099) |
| 279 | SNAP25 (SEQ ID NO: 33) | GTTATTATTCGGTACGT (SEQ ID NO: 2202) |
| 280 | SNAP25 (SEQ ID NO: 33) | AGTTATTATTTGGTATGTAT (SEQ ID NO: 2203) |
| 281 | SEQ ID NO:26 (SEQ ID NO: 26) | TTAAGTATCGAGGCGT (SEQ ID NO: 2100) |

(continued)

| No: | Gene | Oligo: |
|---|---|---|
| 282 | SEQ ID NO:26 (SEQ ID NO: 26) | TTTTAAGTATTGAGGTGT (SEQ ID NO: 2101) |
| 283 | SEQ ID NO:26 (SEQ ID NO: 26) | AATTTTGGTCGTTTAGT (SEQ ID NO: 2102) |
| 284 | SEQ ID NO:26 (SEQ ID NO: 26) | AAATTTTGGTTGTTTAGT (SEQ ID NO: 2103) |
| 285 | SEQ ID NO:26 (SEQ ID NO: 26) | TTCGTATTGACGTTAAT (SEQ ID NO: 2104) |
| 286 | SEQ ID NO:26 (SEQ ID NO: 26) | TTTGTATTGATGTTAATAGA (SEQ ID NO: 2105) |
| 287 | GIRK2 (SEQ ID NO: 27) | AGTTGTTCGTAGGCGA (SEQ ID NO: 2106) |
| 288 | GIRK2 (SEQ ID NO: 27) | AGTTGTTTGTAGGTGA (SEQ ID NO: 2107) |
| 289 | GIRK2 (SEQ ID NO: 27) | TTATTTCGTTCGTAGTT (SEQ ID NO: 2108) |
| 290 | GIRK2 (SEQ ID NO: 27) | TTTGTTTGTAGTTAGGTA (SEQ ID NO: 2109) |
| 291 | GIRK2 (SEQ ID NO: 27) | TTGAAATTCGTACGGG (SEQ ID NO: 1292) |
| 292 | GIRK2 (SEQ ID NO: 27) | AAATTTGTATGGGGGG (SEQ ID NO: 1293) |
| 293 | GIRK2 (SEQ ID NO: 27) | TTAGTCGAAAGGCGAG (SEQ ID NO: 2110) |
| 294 | GIRK2 (SEQ ID NO: 27) | TAGTTGAAAGGTGAGG (SEQ ID NO: 2111) |
| 295 | SEQ ID NO:28 (SEQ ID NO: 28) | GAGTAACGCGTGTGAT (SEQ ID NO: 1294) |
| 296 | SEQ ID NO:28 (SEQ ID NO: 28) | TATGAGTAATGTGTGTG (SEQ ID NO: 1295) |
| 297 | SEQ ID NO:28 (SEQ ID NO: 28) | TTTTCGGTAAGTTTACGG (SEQ ID NO: 1296) |
| 298 | SEQ ID NO:28 (SEQ ID NO: 28) | TTTTTGGTAAGTTTATGG (SEQ ID NO: 1297) |
| 299 | SEQ ID NO:28 (SEQ ID NO: 28) | ATAAGACGGAGTTCGT (SEQ ID NO: 1298) |
| 300 | SEQ ID NO:28 (SEQ ID NO: 28) | AAGATGGAGTTTGTTTG (SEQ ID NO: 1299) |
| 301 | SEQ ID NO:28 (SEQ ID NO: 28) | ATTAGGCGAGTTTCGT (SEQ ID NO: 2112) |
| 302 | SEQ ID NO:28 (SEQ ID NO: 28) | TTAGGTGAGTTTTGTTT (SEQ ID NO: 2113) |
| 303 | SEQ ID NO:28 (SEQ ID NO: 28) | TATCGACGTTTTTGGT (SEQ ID NO: 1896) |
| 304 | SEQ ID NO:28 (SEQ ID NO: 28) | TATTGATGTTTTTGGTTT (SEQ ID NO: 1897) |
| 305 | SEQ ID NO:28 (SEQ ID NO: 28) | ATTCGGTGAAGCGATT (SEQ ID NO: 1300) |
| 306 | SEQ ID NO:28 (SEQ ID NO: 28) | ATTTGGTGAAGTGATTT (SEQ ID NO: 1301) |
| 307 | SEQ ID NO:28 (SEQ ID NO: 28) | GTTCGTAGCGGTAGGT (SEQ ID NO: 1302) |
| 308 | SEQ ID NO:28 (SEQ ID NO: 28) | TTTGTAGTGGTAGGTGA (SEQ ID NO: 1303) |
| 309 | SEQ ID NO:28 (SEQ ID NO: 28) | TGGACGGAGACGAAAG (SEQ ID NO: 1304) |
| 310 | SEQ ID NO:28 (SEQ ID NO: 28) | GGATGGAGATGAAAGGA (SEQ ID NO: 1305) |
| 311 | SEQ ID NO:29 (SEQ ID NO: 29) | TTGATGCGGAGTTCGA (SEQ ID NO: 1898) |
| 312 | SEQ ID NO:29 (SEQ ID NO: 29) | TTGATGTGGAGTTTGA (SEQ ID NO: 1899) |
| 313 | SEQ ID NO:29 (SEQ ID NO: 29) | TTCGAAGCGTGTATTA (SEQ ID NO: 2188) |
| 314 | SEQ ID NO:29 (SEQ ID NO: 29) | GGGTTTGAAGTGTGTA (SEQ ID NO: 2189) |
| 315 | SEQ ID NO:29 (SEQ ID NO: 29) | TTTTCGCGTTTGCGAA (SEQ ID NO: 2190) |
| 316 | SEQ ID NO:29 (SEQ ID NO: 29) | TTTGTGTTTGTGAAAGG (SEQ ID NO: 2191) |
| 317 | SEQ ID NO:29 (SEQ ID NO: 29) | TAGGAACGGTAGGCGG (SEQ ID NO: 1900) |
| 318 | SEQ ID NO:29 (SEQ ID NO: 29) | TAGGAATGGTAGGTGG (SEQ ID NO: 1901) |
| 319 | SEQ ID NO:29 (SEQ ID NO: 29) | GTCGGAGGCGTTTGAG (SEQ ID NO: 1902) |

(continued)

| No: | Gene | Oligo: |
|-----|------|--------|
| 320 | SEQ ID NO:29 (SEQ ID NO: 29) | GTTGGAGGTGTTTGAGA (SEQ ID NO: 1903) |
| 321 | ARL7 (SEQ ID NO: 30) | TAGATTTCGTAGTTTTTTA (SEQ ID NO: 1904) |
| 322 | ARL7 (SEQ ID NO: 30) | TAGATTTTGTAGTTTTTTAA (SEQ ID NO: 1905) |
| 323 | ARL7 (SEQ ID NO: 30) | TGGGTACGTTTACGGT (SEQ ID NO: 1906) |
| 324 | ARL7 (SEQ ID NO: 30) | TGGGTATGTTTATGGTT (SEQ ID NO: 1907) |
| 325 | ARL7 (SEQ ID NO: 30) | GAAGAAATCGTTTTTGT (SEQ ID NO: 1908) |
| 326 | ARL7 (SEQ ID NO: 30) | GAAGAAATTGTTTTTGTT (SEQ ID NO: 1909) |
| 327 | ARL7 (SEQ ID NO: 30) | TAGTAGGATCGGTTTTT (SEQ ID NO: 1910) |
| 328 | ARL7 (SEQ ID NO: 30) | ATAGTAGGATTGGTTTTT (SEQ ID NO: 1911) |
| 329 | SEQ ID NO:31 (SEQ ID NO: 31) | TTTACGTAGGGCGATT (SEQ ID NO: 2114) |
| 330 | SEQ ID NO:31 (SEQ ID NO: 31) | ATTTTTATGTAGGGTGAT (SEQ ID NO: 2115) |
| 331 | SEQ ID NO:31 (SEQ ID NO: 31) | AACGTTGCGTTGGGTA (SEQ ID NO: 1912) |
| 332 | SEQ ID NO:31 (SEQ ID NO: 31) | AATGTTGTGTTGGGTAA (SEQ ID NO: 1913) |
| 333 | SEQ ID NO:31 (SEQ ID NO: 31) | TAGCGTTTCGTGGTTA (SEQ ID NO: 1914) |
| 334 | SEQ ID NO:31 (SEQ ID NO: 31) | GTAGTGTTTTGTGGTTA (SEQ ID NO: 1915) |
| 335 | SEQ ID NO:31 (SEQ ID NO: 31) | GATACGGTTAGGCGGG (SEQ ID NO: 2116) |
| 336 | SEQ ID NO:31 (SEQ ID NO: 31) | GATATGGTTAGGTGGG (SEQ ID NO: 2117) |
| 337 | SEQ ID NO:31 (SEQ ID NO: 31) | TTCGGGCGTTTTATAT (SEQ ID NO: 2118) |
| 338 | SEQ ID NO:31 (SEQ ID NO: 31) | GGTTTGGGTGTTTTATA (SEQ ID NO: 2119) |
| 339 | THH (SEQ ID NO: 32) | TTCGGAAGAAAAGCGAAT (SEQ ID NO: 1916) |
| 340 | THH (SEQ ID NO: 32) | TTTGGAAGAAAAGTGAAT (SEQ ID NO: 1917) |
| 341 | THH (SEQ ID NO: 32) | GTTCGTTGGCGTAAAT (SEQ ID NO: 1918) |
| 342 | THH (SEQ ID NO: 32) | GGTTTGTTGGTGTAAAT (SEQ ID NO: 1919) |
| 343 | THH (SEQ ID NO: 32) | AGTACGTGTATATCGG (SEQ ID NO: 1306) |
| 344 | THH (SEQ ID NO: 32) | TAGTATGTGTATATTGGAA (SEQ ID NO: 1307) |
| 345 | THH (SEQ ID NO: 32) | TATTCGGAAGTGATCGG (SEQ ID NO: 1920) |
| 346 | THH (SEQ ID NO: 32) | TATTTGGAAGTGATTGG (SEQ ID NO: 1921) |
| 347 | HOXB13 (SEQ ID NO: 34) | GTCGTTATTATTTCGAGG (SEQ ID NO: 2120) |
| 348 | HOXB13 (SEQ ID NO: 34) | GTTGTTATTATTTTGAGGA (SEQ ID NO: 2121) |
| 349 | HOXB 13 (SEQ ID NO: 34) | TTCGTAGAATCGAAGT (SEQ ID NO: 2220) |
| 350 | HOXB13 (SEQ ID NO: 34) | TAATTTGTAGAATTGAAGT (SEQ ID NO: 2221) |
| 351 | HOXB13 (SEQ ID NO: 34) | TTACGATTGAGCGTAT (SEQ ID NO: 2222) |
| 352 | HOXB13 (SEQ ID NO: 34) | TATGATTGAGTGTATAGG (SEQ ID NO: 2223) |
| 353 | HOXB13 (SEQ ID NO: 34) | AAGTTAGCGGGTTCGT (SEQ ID NO: 2122) |
| 354 | HOXB13 (SEQ ID NO: 34) | AAGTTAGTGGGTTTGT (SEQ ID NO: 2123) |
| 355 | HOXB13 (SEQ ID NO: 34) | TTGGTCGCGTAGTAAA (SEQ ID NO: 2124) |
| 356 | HOXB13 (SEQ ID NO: 34) | TGGTTGTGTAGTAAAGT (SEQ ID NO: 2125) |
| 357 | (SEQ ID NO: 35) | GGAGGTGCGAATTTAA (SEQ ID NO: 2126) |

(continued)

| No: | Gene | Oligo: |
|---|---|---|
| 358 | (SEQ ID NO: 35) | GGGAGGTGTGAATTTA (SEQ ID NO: 2127) |
| 359 | (SEQ ID NO: 35) | AAATAGTCGTTTGGGA (SEQ ID NO: 2128) |
| 360 | (SEQ ID NO: 35) | AAATAGTTGTTTGGGAG (SEQ ID NO: 2129) |
| 361 | (SEQ ID NO: 35) | AGAAAATATACGAGATTTAT (SEQ ID NO: 2130) |
| 362 | (SEQ ID NO: 35) | AGAAAATATATGAGATTTATT (SEQ ID NO: 2131) |
| 363 | (SEQ ID NO: 35) | TAAAGACGGGAAGAGA (SEQ ID NO: 2132) |
| 364 | (SEQ ID NO: 35) | ATAAAGATGGGAAGAGA (SEQ ID NO: 2133) |
| 365 | (SEQ ID NO: 36) | TTCGTTGGAGATCGTAA (SEQ ID NO: 2192) |
| 366 | (SEQ ID NO: 36) | GTTTGTTGGAGATTGTA (SEQ ID NO: 2193) |
| 367 | (SEQ ID NO: 36) | TTATTTGCGTTTCGAA (SEQ ID NO: 2218) |
| 368 | (SEQ ID NO: 36) | AATTATTTGTGTTTTGAAT (SEQ ID NO: 2219) |
| 369 | (SEQ ID NO: 36) | TTGTCGTGATAGCGTT (SEQ ID NO: 1308) |
| 370 | (SEQ ID NO: 36) | TTTGTTGTGATAGTGTT (SEQ ID NO: 1309) |
| 371 | (SEQ ID NO: 36) | GGACGTTGCGATTGTA (SEQ ID NO: 2194) |
| 372 | (SEQ ID NO: 36) | GATGTTGTGATTGTAGT (SEQ ID NO: 2195) |
| 373 | MGC10561 (SEQ ID NO: 37) | ATATTTAGCGTAGTTATTT (SEQ ID NO: 2204) |
| 374 | MGC10561 (SEQ ID NO: 37) | TAGTATATTTAGTGTAGTTAT (SEQ ID NO: 2205) |
| 375 | MGC10561 (SEQ ID NO: 37) | TTTTTTACGTTAAGGGG (SEQ ID NO: 2134) |
| 376 | MGC10561 (SEQ ID NO: 37) | TTTTTATGTTAAGGGGG (SEQ ID NO: 2135) |
| 377 | MGC10561 (SEQ ID NO: 37) | TTTGTTTTTCGAGTAGA (SEQ ID NO: 2136) |
| 378 | MGC10561 (SEQ ID NO: 37) | TGTTTTTTGAGTAGAGG (SEQ ID NO: 2137) |
| 379 | LMX1A (SEQ ID NO: 38) | GTCGGGTTTTTCGGAA (SEQ ID NO: 2138) |
| 380 | LMX1A (SEQ ID NO: 38) | GTTGGGTTTTTTGGAAG (SEQ ID NO: 2139) |
| 381 | LMX1A (SEQ ID NO: 38) | GTCGAGATTTTATCGAAA (SEQ ID NO: 2140) |
| 382 | LMX1A (SEQ ID NO: 3 8) | GTTGAGATTTTATTGAAAG (SEQ ID NO: 2141) |
| 383 | LMX1A (SEQ ID NO: 38) | TTCGTTTTTAACGTGG (SEQ ID NO: 2224) |
| 384 | LMX1A (SEQ ID NO: 38) | TTTGTTTTTAATGTGGTT (SEQ ID NO: 2225) |
| 385 | LMX1A (SEQ ID NO: 38) | AGTATTCGGGCGGGTA (SEQ ID NO: 2142) |
| 386 | LMX1A (SEQ ID NO: 38) | GTAGTATTTGGGTGGG (SEQ ID NO: 2143) |
| 387 | LMX1A (SEQ ID NO: 3 8) | AACGAAATTACGTGTAT (SEQ ID NO: 2144) |
| 388 | LMX1A (SEQ ID NO: 38) | TGAAATGAAATTATGTGTA (SEQ ID NO: 2145) |
| 389 | SENP3 (SEQ ID NO: 39) | TATTCGGATCGGGTTT (SEQ ID NO: 1922) |
| 390 | SENP3 (SEQ ID NO: 39) | TTTATTTGGATTGGGTT (SEQ ID NO: 1923) |
| 391 | SENP3 (SEQ ID NO: 39) | TAGTCGAAAGTAGGACGT (SEQ ID NO: 1924) |
| 392 | SENP3 (SEQ ID NO: 39) | TAGTTGAAAGTAGGATGT (SEQ ID NO: 1925) |
| 393 | SENP3 (SEQ ID NO: 39) | AGGACGTTTTTGATCGG (SEQ ID NO: 1926) |
| 394 | SENP3 (SEQ ID NO: 39) | AGGATGTTTTTGATTGG (SEQ ID NO: 1927) |
| 395 | SENP3 (SEQ ID NO: 39) | AGTTATCGTTAGGAGGG (SEQ ID NO: 1928) |

(continued)

| No: | Gene | Oligo: |
|---|---|---|
| 396 | SENP3 (SEQ ID NO: 39) | AGTTATTGTTAGGAGGG (SEQ ID NO: 1929) |
| 397 | GS1 (SEQ ID NO: 40) | GAAGCGACGTTTATTT (SEQ ID NO: 1310) |
| 398 | GS (SEQ ID NO: 40) | GGGAAGTGATGTTTATT (SEQ ID NO: 1311) |
| 399 | GS1 (SEQ ID NO: 40) | TGCGAGTTAGCGGTTA (SEQ ID NO: 2146) |
| 400 | GS1 (SEQ ID NO: 40) | TTGTGAGTTAGTGGTT (SEQ ID NO: 2147) |
| 401 | GS1 (SEQ ID NO: 40) | AATCGTTAGTTCGGTT (SEQ ID NO: 1312) |
| 402 | GS1 (SEQ ID NO: 40) | TGAATTGTTAGTTTGGT (SEQ ID NO: 1313) |
| 403 | GS1 (SEQ ID NO: 40) | TAGAGGTTCGAGCGTA (SEQ ID NO: 1314) |
| 404 | GS1 (SEQ ID NO: 40) | AGAGGTTTGAGTGTAG (SEQ ID NO: 1315) |
| 405 | GS1 (SEQ ID NO: 40) | AGTTTCGAGGTTTTCGG (SEQ ID NO: 2148) |
| 406 | GS1 (SEQ ID NO: 40) | AAGTTTTGAGGTTTTTGG (SEQ ID NO: 2149) |
| 407 | TITF1 (SEQ ID NO: 41) | GTCGTGGGGATCGTAT (SEQ ID NO: 2150) |
| 408 | TITF1 (SEQ ID NO: 41) | GTTGTGGGGATTGTAT (SEQ ID NO: 2151) |
| 409 | TITF1 (SEQ ID NO: 41) | TTTTTGCGTAAACGTA (SEQ ID NO: 1316) |
| 410 | TITF1 (SEQ ID NO: 41) | TTGTGTAAATGTAGGGT (SEQ ID NO: 1317) |
| 411 | TITF1 (SEQ ID NO: 41) | GGTTCGTTTAAGTTCGG (SEQ ID NO: 2152) |
| 412 | TITF1 (SEQ ID NO: 41) | GGTTTGTTTAAGTTTGG (SEQ ID NO: 2153) |
| 413 | TITF1 (SEQ ID NO: 41) | TTAGGTCGCGTTTGTA (SEQ ID NO: 2154) |
| 414 | TITF1 (SEQ ID NO: 41) | AGGTTGTGTTTGTAGA (SEQ ID NO: 2155) |
| 415 | TITF1 (SEQ ID NO: 41) | TATTTCGTTTTCGTAATT (SEQ ID NO: 2156) |
| 416 | TITF (SEQ ID NO: 41) | TTTGTTTTTGTAATTAGATT (SEQ ID NO: 2157) |
| 417 | SEQ ID NO:42 (SEQ ID NO: 42) | AGGTCGAATAAAGCGT (SEQ ID NO: 2212) |
| 418 | SEQ ID NO:42 (SEQ ID NO: 42) | GAGGTTGAATAAAGTGT (SEQ ID NO: 2213) |
| 419 | SEQ ID NO:42 (SEQ ID NO: 42) | TTCGGAAGTTTAACGAGG (SEQ ID NO: 1318) |
| 420 | SEQ ID NO:42 (SEQ ID NO: 42) | TTTGGAAGTTTAATGAGG (SEQ ID NO: 1319) |
| 421 | SEQ ID NO:42 (SEQ ID NO: 42) | GAGTCGGGTTGCGATG (SEQ ID NO: 1930) |
| 422 | SEQ ID NO:42 (SEQ ID NO: 42) | GGAGTTGGGTTGTGAT (SEQ ID NO: 1931) |
| 423 | SEQ ID NO:42 (SEQ ID NO: 42) | ATGGGTTGCGATTTTTA (SEQ ID NO: 1932) |
| 424 | SEQ ID NO:42 (SEQ ID NO: 42) | ATGGGTTGTGATTTTTA (SEQ ID NO: 1933) |
| 425 | DDX51 (SEQ ID NO: 43) | TAGGCGAGCGTTAGGT (SEQ ID NO: 2206) |
| 426 | DDX51 (SEQ ID NO: 43) | GGTGAGTGTTAGGTTA (SEQ ID NO: 2207) |
| 427 | DDX51 (SEQ ID NO: 43) | AGATTTTCGGCGAGAT (SEQ ID NO: 2158) |
| 428 | DDX51 (SEQ ID NO: 43) | ATTTTTGGTGAGATAGG (SEQ ID NO: 2159) |
| 429 | DDX51 (SEQ ID NO: 43) | TACGTGTGGTTTTCGGTA (SEQ ID NO: 2160) |
| 430 | DDX51 (SEQ ID NO: 43) | TATGTGTGGTTTTTGGTA (SEQ ID NO: 2161) |
| 431 | DDX51 (SEQ ID NO: 43) | TGCGTTTATCGTTTTAG (SEQ ID NO: 1320) |
| 432 | DDX51 (SEQ ID NO: 43) | TGTGTTTATTGTTTTAGG (SEQ ID NO: 1321) |
| 433 | DDX51 (SEQ ID NO: 43) | AACGTGCGGGGGTTTTT (SEQ ID NO: 2162) |

(continued)

| No: | Gene | Oligo: |
|---|---|---|
| 434 | DDX51 (SEQ ID NO: 43) | TGAATGTGTGGGGTTT (SEQ ID NO: 2163) |
| 435 | (SEQ ID NO: 117) | TAGCGGTTTTTTAGCGTA (SEQ ID NO: 1934) |
| 436 | (SEQ ID NO: 117) | AGTGGTTTTTTAGTGTAT (SEQ ID NO: 1935) |
| 437 | (SEQ ID NO: 117) | AGATGCGCGGGTAGAT (SEQ ID NO: 1936) |
| 438 | (SEQ ID NO: 117) | AGATGTGTGGGTAGAT (SEQ ID NO: 1937) |
| 439 | (SEQ ID NO: 117) | AGATAGTTCGTATTCGT (SEQ ID NO: 1938) |
| 440 | (SEQ ID NO: 117) | GTAGATAGTTTGTATTTGT (SEQ ID NO: 1939) |
| 441 | (SEQ ID NO: 117) | TTTGTTCGTAACGTTT (SEQ ID NO: 1940) |
| 442 | (SEQ ID NO: 117) | TGTTTGTAATGTTTAGAG (SEQ ID NO: 1941) |
| 443 | Q8NAN2 (SEQ ID NO: 44) | AGTTTCGCGAGGGGTT (SEQ ID NO: 1322) |
| 444 | Q8NAN2 (SEQ ID NO: 44) | AGAGTTTTGTGAGGGG (SEQ ID NO: 1323) |
| 445 | Q8NAN2 (SEQ ID NO: 44) | TTTTTACGTTAGAGGGA (SEQ ID NO: 1324) |
| 446 | Q8NAN2 (SEQ ID NO: 44) | TTTTTATGTTAGAGGGAG (SEQ ID NO: 1325) |
| 447 | SEQ ID NO:45 (SEQ ID NO: 45) | AGCGAACGTTTATTTT (SEQ ID NO: 2164) |
| 448 | SEQ ID NO:45 (SEQ ID NO: 45) | TAGGAGAGTGAATGTTT (SEQ ID NO: 2165) |
| 449 | SEQ ID NO:45 (SEQ ID NO: 45) | TAATGTCGATCGGATT (SEQ ID NO: 1326) |
| 450 | SEQ ID NO:45 (SEQ ID NO: 45) | ATGTTGATTGGATTTGT (SEQ ID NO: 1327) |
| 451 | SEQ ID NO:45 (SEQ ID NO: 45) | TGTAGTATCGGGGGAG (SEQ ID NO: 2208) |
| 452 | SEQ ID NO:45 (SEQ ID NO: 45) | TGTAGTATTGGGGGAG (SEQ ID NO: 2209) |
| 453 | SEQ ID NO:45 (SEQ ID NO: 45) | TTTTTTACGGGCGATA (SEQ ID NO: 1328) |
| 454 | SEQ ID NO:45 (SEQ ID NO: 45) | TTTTATGGGTGATAGGT (SEQ ID NO: 1329) |
| 455 | SEQ ID NO:46 (SEQ ID NO: 46) | GAGTCGGGTTATCGTT (SEQ ID NO: 2166) |
| 456 | SEQ ID NO:46 (SEQ ID NO: 46) | GGAGTTGGGTTATTGT (SEQ ID NO: 2167) |
| 457 | SEQ ID NO:46 (SEQ ID NO: 46) | TTACGGATGTTTCGGT (SEQ ID NO: 2168) |
| 458 | SEQ ID NO:46 (SEQ ID NO: 46) | TTTATGGATGTTTTGGT (SEQ ID NO: 2169) |
| 459 | SEQ ID NO:46 (SEQ ID NO: 46) | TGACGTATTTTCGGTT (SEQ ID NO: 2170) |
| 460 | SEQ ID NO:46 (SEQ ID NO: 46) | GGTGATGTATTTTTGGT (SEQ ID NO: 2171) |
| 461 | SEQ ID NO:46 (SEQ ID NO: 46) | ATAGTCGGAATCGTTG (SEQ ID NO: 2172) |
| 462 | SEQ ID NO:46 (SEQ ID NO: 46) | TAGTTGGAATTGTTGTT (SEQ ID NO: 2173) |
| 463 | 060279 (SEQ ID NO: 47) | AGTAAACGAATAAGAAGT (SEQ ID NO: 1942) |
| 464 | 060279 (SEQ ID NO: 47) | AAGTAAATGAATAAGAAGT (SEQ ID NO: 1943) |
| 465 | 060279 (SEQ ID NO: 47) | TACGTTTTTTCGGATTA (SEQ ID NO: 1944) |
| 466 | 060279 (SEQ ID NO: 47) | TATGTTTTTTTGGATTAAG (SEQ ID NO: 1945) |
| 467 | 060279 (SEQ ID NO: 47) | TAATTATCGGCGGTGT (SEQ ID NO: 1946) |
| 468 | 060279 (SEQ ID NO: 47) | ATTATTGGTGGTGTTTT (SEQ ID NO: 1947) |
| 469 | 060279 (SEQ ID NO: 47) | GGACGGCGGAAAATTA (SEQ ID NO: 1948) |
| 470 | 060279 (SEQ ID NO: 47) | AGGGATGGTGGAAAAT (SEQ ID NO: 1949) |
| 471 | SEQ ID NO:48 (SEQ ID NO: 48) | TATTTGGCGATTCGGA (SEQ ID NO: 1950) |

(continued)

| No: | Gene | Oligo: |
|-----|------|--------|
| 472 | SEQ ID NO:48 (SEQ ID NO: 48) | TGGTGATTTGGAGATT (SEQ ID NO: 1951) |
| 473 | SEQ ID NO:48 (SEQ ID NO: 48) | TAGGGTTACGTGTCGG (SEQ ID NO: 1952) |
| 474 | SEQ ID NO:48 (SEQ ID NO: 48) | TAGGGTTATGTGTTGG (SEQ ID NO: 1953) |
| 475 | SEQ ID NO:48 (SEQ ID NO: 48) | AACGAATTTTTCGATATT (SEQ ID NO: 1954) |
| 476 | SEQ ID NO:48 (SEQ ID NO: 48) | TTTAATGAATTTTTTGATATT (SEQ ID NO: 1955) |
| 477 | SEQ ID NO:48 (SEQ ID NO: 48) | AAAATCGTATGCGTGT (SEQ ID NO: 1956) |
| 478 | SEQ ID NO:48 (SEQ ID NO: 48) | GAAAATTGTATGTGTGTG (SEQ ID NO: 1957) |
| 479 | SEQ ID NO:9 (SEQ ID NO: 9) | AGGTATTTCGCGATAT (SEQ ID NO: 1330) |
| 480 | SEQ ID NO:9 (SEQ ID NO: 9) | AGGTATTTTGTGATATTTT (SEQ ID NO: 1331) |
| 481 | SEQ ID NO:9 (SEQ ID NO: 9) | GTTTTTCGATTTACGTT (SEQ ID NO: 1332) |
| 482 | SEQ ID NO:9 (SEQ ID NO: 9) | TAGGTTTTTTGATTTATGT (SEQ ID NO: 1333) |
| 483 | SEQ ID NO:9 (SEQ ID NO: 9) | TTACGGTTCGGTTATT (SEQ ID NO: 1334) |
| 484 | SEQ ID NO:9 (SEQ ID NO: 9) | AGGTTTTATGGTTTGGT (SEQ ID NO: 1335) |
| 485 | SEQ ID NO:9 (SEQ ID NO: 9) | GTTTCGCGTTTAGGGA (SEQ ID NO: 1958) |
| 486 | SEQ ID NO:9 (SEQ ID NO: 9) | GTTTTGTGTTTAGGGAT (SEQ ID NO: 1959) |
| 487 | SEQ ID NO:9 (SEQ ID NO: 9) | AGTGTTCGTCGTAGTT (SEQ ID NO: 1960) |
| 488 | SEQ ID NO:9 (SEQ ID NO: 9) | TGAGTGTTTGTTGTAGT (SEQ ID NO: 1961) |
| 489 | SEQ ID NO:2 (SEQ ID NO: 2) | ATTGGGTTTCGCGTAGG (SEQ ID NO: 2174) |
| 490 | SEQ ID NO:2 (SEQ ID NO: 2) | ATTGGGTTTTGTGTAGG (SEQ ID NO: 2175) |
| 491 | SEQ ID NO:2 (SEQ ID NO: 2) | TAGTAGTCGGCGGGAG (SEQ ID NO: 2176) |
| 492 | SEQ ID NO:2 (SEQ ID NO: 2) | TAGTAGTTGGTGGGAG (SEQ ID NO: 2177) |
| 493 | SEQ ID NO:2 (SEQ ID NO: 2) | TGTCGTACGTTATGTT (SEQ ID NO: 2226) |
| 494 | SEQ ID NO:2 (SEQ ID NO: 2) | GGTGTTGTATGTTATGT (SEQ ID NO: 2227) |
| 495 | SEQ ID NO:2 (SEQ ID NO: 2) | TTGATTGGCGGACGAG (SEQ ID NO: 1699) |
| 496 | SEQ ID NO:2 (SEQ ID NO: 2) | TTGATTGGTGGATGAG (SEQ ID NO: 1700) |
| 497 | SEQ ID NO:3 (SEQ ID NO: 3) | TTGCGTTAATTCGGTA (SEQ ID NO: 2178) |
| 498 | SEQ ID NO:3 (SEQ ID NO: 3) | AATTTGTGTTAATTTGGT (SEQ ID NO: 2179) |
| 499 | SEQ ID NO:3 (SEQ ID NO: 3) | AGTCGGGAGAGCGAAA (SEQ ID NO: 2180) |
| 500 | SEQ ID NO:3 (SEQ ID NO: 3) | AGTTGGGAGAGTGAAA (SEQ ID NO: 2181) |
| 501 | SEQ ID NO:3 (SEQ ID NO: 3) | GGTCGAAGAGTCGGGA (SEQ ID NO: 2182) |
| 502 | SEQ ID NO:3 (SEQ ID NO: 3) | GGTTGAAGAGTTGGGA (SEQ ID NO: 2183) |
| 503 | SEQ ID NO:3 (SEQ ID NO: 3) | ATGTTAGCGGGTCGAA (SEQ ID NO: 2184) |
| 504 | SEQ ID NO:3 (SEQ ID NO: 3) | ATGTTAGTGGGTTGAA (SEQ ID NO: 2185) |
| 505 | SEQ ID NO:4 (SEQ ID NO: 4) | TTTTGTTCGCGTTGAA (SEQ ID NO: 1962) |
| 506 | SEQ ID NO:4 (SEQ ID NO: 4) | TTGTTTGTGTTGAAGTA (SEQ ID NO: 1963) |
| 507 | SEQ ID NO:4 (SEQ ID NO: 4) | GGGTCGCGAGGTAGTT (SEQ ID NO: 1964) |
| 508 | SEQ ID NO:4 (SEQ ID NO: 4) | TGGGTTGTGAGGTAGT (SEQ ID NO: 1965) |
| 509 | SEQ ID NO:4 (SEQ ID NO: 4) | TTTGTGCGACGTTATT (SEQ ID NO: 1966) |

(continued)

| No: | Gene | Oligo: |
|---|---|---|
| 510 | SEQ ID NO:4 (SEQ ID NO: 4) | GGTTTGTGTGATGTTAT (SEQ ID NO: 1967) |
| 511 | SEQ ID NO:4 (SEQ ID NO: 4) | ATGGCGGTTTCGATTT (SEQ ID NO: 1968) |
| 512 | SEQ ID NO:4 (SEQ ID NO: 4) | GATGGTGGTTTTGATTT (SEQ ID NO: 1969) |
| 513 | HS3ST2 (SEQ ID NO: 113) | GAATCGGAGAGGCGAG (SEQ ID NO: 1664) |
| 514 | HS3ST2 (SEQ ID NO: 113) | AATTGGAGAGGTGAGG (SEQ ID NO: 1665) |
| 515 | HS3ST2 (SEQ ID NO: 113) | GGGTAATCGTTTGGTA (SEQ ID NO: 1666) |
| 516 | HS3ST2 (SEQ ID NO: 113) | GGGTAATTGTTTGGTAT (SEQ ID NO: 1667) |
| 517 | PRDM2 (SEQ ID NO: 114) | TGTAGAGACGACGATT (SEQ ID NO: 1668) |
| 518 | PRDM2 (SEQ ID NO: 114) | ATTGTAGAGATGATGATT (SEQ ID NO: 1669) |
| 519 | PRDM2 (SEQ ID NO: 114) | AGAGCGCGGTAGTAGT (SEQ ID NO: 1670) |
| 520 | PRDM2 (SEQ ID NO: 114) | TGAGAGTGTGGTAGTA (SEQ ID NO: 1671) |
| 521 | PRDM2 (SEQ ID NO: 114) | TGTTCGCGATGTTTTA (SEQ ID NO: 1672) |
| 522 | PRDM2 (SEQ ID NO: 114) | TGTTTGTGATGTTTTAGT (SEQ ID NO: 1673) |
| 523 | PRDM2 (SEQ ID NO: 114) | AGTATATAAACGTAGATTTT (SEQ ID NO: 1674) |
| 524 | PRDM2 (SEQ ID NO: 114) | AAGTATATAAATGTAGATTTT (SEQ ID NO: 1675) |
| 525 | ALX4 (SEQ ID NO: 64) | AAGTCGATCGTTTTGT (SEQ ID NO: 1676) |
| 526 | ALX4 (SEQ ID NO: 64) | TGGAAGTTGATTGTTTT (SEQ ID NO: 1677) |
| 527 | ALX4 (SEQ ID NO: 64) | ATATCGTTCGGGGGAA (SEQ ID NO: 1827) |
| 528 | ALX4 (SEQ ID NO: 64) | ATTGTTTGGGGGAATT (SEQ ID NO: 1336) |
| 529 | ALX4 (SEQ ID NO: 64) | TATTGCGAGGATTCGG (SEQ ID NO: 1678) |
| 530 | ALX4 (SEQ ID NO: 64) | ATTGTGAGGATTTGGT (SEQ ID NO: 1679) |
| 531 | ALX4 (SEQ ID NO: 64) | TTCGTAGCGTAGGGTT (SEQ ID NO: 1680) |
| 532 | ALX4 (SEQ ID NO: 64) | TTTGTAGTGTAGGGTTT (SEQ ID NO: 1681) |
| 533 | LEF1 (SEQ ID NO: 63) | TGAGATTTCGTTTATTGA (SEQ ID NO: 1337) |
| 534 | LEF1 (SEQ ID NO: 63) | ATTGAGATTTTGTTTATTG (SEQ ID NO: 1338) |
| 535 | LEF1 (SEQ ID NO: 63) | TTGTTTTTAACGAAATTAT (SEQ ID NO: 1339) |
| 536 | LEF1 (SEQ ID NO: 63) | TGTTTTTAATGAAATTATTTA (SEQ ID NO: 1340) |
| 537 | LEF1 (SEQ ID NO: 63) | TTGGAAGTCGAAGAGA (SEQ ID NO: 1341) |
| 538 | LEF1 (SEQ ID NO: 63) | TTTGGAAGTTGAAGAGA (SEQ ID NO: 1342) |
| 539 | LEF1 (SEQ ID NO: 63) | TGGATTTTTCGAGTAATT (SEQ ID NO: 1343) |
| 540 | LEF1 (SEQ ID NO: 63) | TTGGATTTTTTGAGTAATT (SEQ ID NO: 1344) |
| 541 | SCGB3A (SEQ ID NO: 115) | GATAGACGAGTGAGGA (SEQ ID NO: 1345) |
| 542 | SCGB3A1 (SEQ ID NO: 115) | GATAGATGAGTGAGGAT (SEQ ID NO: 1346) |
| 543 | SCGB3A1 (SEQ ID NO: 115) | TATTGGCGTCGAGGTT (SEQ ID NO: 1828) |
| 544 | SCGB3A1 (SEQ ID NO: 115) | TATTGGTGTTGAGGTT (SEQ ID NO: 1829) |
| 545 | SCGB3A1 (SEQ ID NO: 115) | GGCGTAGAAGGCGTTT (SEQ ID NO: 1823) |
| 546 | SCGB3A1 (SEQ ID NO: 115) | GGTGTAGAAGGTGTTT (SEQ ID NO: 1824) |
| 547 | SCGB3A1 (SEQ ID NO: 115) | TAGTGTTCGACGTTGT (SEQ ID NO: 1468) |

(continued)

| No: | Gene | Oligo: |
|---|---|---|
| 548 | SCGB3A1 (SEQ ID NO: 115) | TAGTGTTTGATGTTGTT (SEQ ID NO: 1469) |
| 549 | SASH1 (SEQ ID NO: 102) | TAGATTCGAGGTGCGG (SEQ ID NO: 1470) |
| 550 | SASH1 (SEQ ID NO: 102) | TAGATTTGAGGTGTGG (SEQ ID NO: 1471) |
| 551 | SASH1 (SEQ ID NO: 102) | ATTCGGTATTCGGGTAG (SEQ ID NO: 1472) |
| 552 | SASH1 (SEQ ID NO: 102) | ATTTGGTATTTGGGTAG (SEQ ID NO: 1473) |
| 553 | SASH1 (SEQ ID NO: 102) | ATTGGGACGTACGGTT (SEQ ID NO: 1785) |
| 554 | SASH1 (SEQ ID NO: 102) | GATTGGGATGTATGGT (SEQ ID NO: 1786) |
| 555 | SASH1 (SEQ ID NO: 102) | AGTCGGAATCGGAGTT (SEQ ID NO: 1474) |
| 556 | SASH1 (SEQ ID NO: 102) | GTTGGAATTGGAGTTTA (SEQ ID NO: 1475) |
| 557 | S100A7 (SEQ ID NO: 96) | TATAGTCGGGGTGATA (SEQ ID NO: 1682) |
| 558 | S100A7 (SEQ ID NO: 96) | TTTATAGTTGGGGTGAT (SEQ ID NO: 1683) |
| 559 | S100A7 (SEQ ID NO: 96) | AGTCGGGCGTTAGTAA (SEQ ID NO: 1684) |
| 560 | S100A7 (SEQ ID NO: 96) | GAGTTGGGTGTTAGTA (SEQ ID NO: 1685) |
| 561 | S100A7 (SEQ ID NO: 96) | GGATGGCGGAAGTTTA (SEQ ID NO: 1686) |
| 562 | S100A7 (SEQ ID NO: 96) | GGATGGTGGAAGTTTA (SEQ ID NO: 1687) |
| 563 | APC (SEQ ID NO: 65) | GATTCGTATTTCGTAGT (SEQ ID NO: 1688) |
| 564 | APC (SEQ ID NO: 65) | TTTGTATTTTGTAGTGGG (SEQ ID NO: 1347) |
| 565 | APC (SEQ ID NO: 65) | AGCGTTTTGGTTCGTAT (SEQ ID NO: 1689) |
| 566 | APC (SEQ ID NO: 65) | AGTGTTTTGGTTTGTAT (SEQ ID NO: 1690) |
| 567 | APC (SEQ ID NO: 65) | TTAATCGGCGGGTTTT (SEQ ID NO: 1691) |
| 568 | APC (SEQ ID NO: 65) | AGTTAATTGGTGGGTT (SEQ ID NO: 1692) |
| 569 | APC (SEQ ID NO: 65) | ATTTTCGAGTTCGGTA (SEQ ID NO: 1693) |
| 570 | APC (SEQ ID NO: 65) | TTTTTGAGTTTGGTAGT (SEQ ID NO: 1694) |
| 571 | ARH1/NOEY2 (SEQ ID NO: 97) | TGTTATCGTTAACGATT (SEQ ID NO: 1476) |
| 572 | ARH1/NOEY2 (SEQ ID NO: 97) | AGGTGTTATTGTTAATGA (SEQ ID NO: 1477) |
| 573 | ARH1/NOEY2 (SEQ ID NO: 97) | TAAAACGTTCGGTAGG (SEQ ID NO: 1478) |
| 574 | ARH1/NOEY2 (SEQ ID NO: 97) | TTTAAAATGTTTGGTAGG (SEQ ID NO: 1479) |
| 575 | ARH1/NOEY2 (SEQ ID NO: 97) | TGTATTCGTCGTTAGG (SEQ ID NO: 1480) |
| 576 | ARH1/NOEY2 (SEQ ID NO: 97) | AATGTATTTGTTGTTAGG (SEQ ID NO: 1481) |
| 577 | ARH1/NOEY2 (SEQ ID NO: 97) | TTAGACGGAGTTCGGA (SEQ ID NO: 1482) |
| 578 | ARH1/NOEY2 (SEQ ID NO: 97) | TAGATGGAGTTTGGAGA (SEQ ID NO: 1483) |
| 579 | ARH1/NOEY2 (SEQ ID NO: 97) | TAGACGTAGGCGTATT (SEQ ID NO: 1484) |
| 580 | ARH1/NOEY2 (SEQ ID NO: 97) | GGGTAGATGTAGGTGT (SEQ ID NO: 1485) |
| 581 | BRCA2 (SEQ ID NO: 56) | AGTATACGTATTATTCGG (SEQ ID NO: 1348) |
| 582 | BRCA2 (SEQ ID NO: 56) | AGTATATGTATTATTTGGAA (SEQ ID NO: 1349) |
| 583 | BRCA2 (SEQ ID NO: 56) | TAGAAGTTCGCGTTAT (SEQ ID NO: 1350) |
| 584 | BRCA2 (SEQ ID NO: 56) | GAAGTTTGTGTTATAAGT (SEQ ID NO: 1351) |
| 585 | BRCA2 (SEQ ID NO: 56) | TATCGTCGTAAAAGATAT (SEQ ID NO: 1352) |

(continued)

| No: | Gene | Oligo: |
|---|---|---|
| 586 | BRCA2 (SEQ ID NO: 56) | GATTTATTGTTGTAAAAGAT (SEQ ID NO: 1353) |
| 587 | BRCA2 (SEQ ID NO: 56) | ATTCGTTTTAGAGGCGTA (SEQ ID NO: 1695) |
| 588 | BRCA2 (SEQ ID NO: 56) | ATTTGTTTTAGAGGTGTA (SEQ ID NO: 1696) |
| 589 | BRCA2 (SEQ ID NO: 56) | TTTCGTTACGTTGGAT (SEQ ID NO: 1354) |
| 590 | BRCA2 (SEQ ID NO: 56) | TTTTTGTTATGTTGGATT (SEQ ID NO: 1355) |
| 591 | CDH1 (SEQ ID NO: 79) | TATAGACGCGGTGATT (SEQ ID NO: 1356) |
| 592 | CDH1 (SEQ ID NO: 79) | TATAGATGTGGTGATTTT (SEQ ID NO: 1357) |
| 593 | CDH1 (SEQ ID NO: 79) | TTGACGGTTCGTTTAT (SEQ ID NO: 1358) |
| 594 | CDH1 (SEQ ID NO: 79) | GGAGTTGATGGTTTGT (SEQ ID NO: 1359) |
| 595 | CDH1 (SEQ ID NO: 79) | ATTAGTAGCGCGGATT (SEQ ID NO: 1360) |
| 596 | CDH1 (SEQ ID NO: 79) | AATTAGTAGTGTGGATTT (SEQ ID NO: 1361) |
| 597 | CDH1 (SEQ ID NO: 79) | AGGTATCGTTTTTCGT (SEQ ID NO: 1832) |
| 598 | CDH1 (SEQ ID NO: 79) | GAGGTATTGTTTTTTGTA (SEQ ID NO: 1833) |
| 599 | CDH1 (SEQ ID NO: 79) | TTCGGAGGTATCGTTT (SEQ ID NO: 1362) |
| 600 | CDH1 (SEQ ID NO: 79) | TTTTGGAGGTATTGTTT (SEQ ID NO: 1363) |
| 601 | CDKN1A (SEQ ID NO: 67) | AGCGTATTAACGTAGG (SEQ ID NO: 1364) |
| 602 | CDKN1A (SEQ ID NO: 67) | TTTAGTGTATTAATGTAGG (SEQ ID NO: 1365) |
| 603 | CDKN1A (SEQ ID NO: 67) | TGTAGTACGCGAGGTT (SEQ ID NO: 1366) |
| 604 | CDKN1A (SEQ ID NO: 67) | TGTAGTATGTGAGGTTT (SEQ ID NO: 1367) |
| 605 | CDKN1A (SEQ ID NO: 67) | ATTAGGGTCGCGTTGA (SEQ ID NO: 1494) |
| 606 | CDKN1A (SEQ ID NO: 67) | ATTAGGGTTGTGTTGA (SEQ ID NO: 1495) |
| 607 | CDKN1A (SEQ ID NO: 67) | TTCGTCGAGGTATCGA (SEQ ID NO: 1368) |
| 608 | CDKN1A (SEQ ID NO: 67) | TTTGTTGAGGTATTGAG (SEQ ID NO: 1369) |
| 609 | DAPK1 (SEQ ID NO: 98) | ATAGATCGTTTAGCGT (SEQ ID NO: 1370) |
| 610 | DAPK1 (SEQ ID NO: 98) | ATTGTTTAGTGTTTGGG (SEQ ID NO: 1371) |
| 611 | DAPK1 (SEQ ID NO: 98) | TTTTATTTCGCGAGGA (SEQ ID NO: 1372) |
| 612 | DAPK1 (SEQ ID NO: 98) | TATTTTGTGAGGAGGG (SEQ ID NO: 1373) |
| 613 | DAPK1 (SEQ ID NO: 98) | TTTCGGAGATTCGGTT (SEQ ID NO: 1502) |
| 614 | DAPK1 (SEQ ID NO: 98) | TTTGGAGATTTGGTTTT (SEQ ID NO: 1503) |
| 615 | DAPK1 (SEQ ID NO: 98) | TTTTAGCGTCGGGGAG (SEQ ID NO: 1504) |
| 616 | DAPK1 (SEQ ID NO: 98) | TTTTAGTGTTGGGGAG (SEQ ID NO: 1505) |
| 617 | SEQ ID NO:2 (SEQ ID NO: 2) | TAGGGGTTCGATTAGG (SEQ ID NO: 1834) |
| 618 | SEQ ID NO:2 (SEQ ID NO: 2) | AGGGGTTTGATTAGGG (SEQ ID NO: 1835) |
| 619 | SEQ ID NO:2 (SEQ ID NO: 2) | TTGAATTGCGGTTAGA (SEQ ID NO: 1803) |
| 620 | SEQ ID NO:2 (SEQ ID NO: 2) | TTGAATTGTGGTTAGAG (SEQ ID NO: 1804) |
| 621 | SEQ ID NO:2 (SEQ ID NO: 2) | TAGGTATACGAAAGAGTA (SEQ ID NO: 1697) |
| 622 | SEQ ID NO:2 (SEQ ID NO: 2) | TTAGGTATATGAAAGAGTA (SEQ ID NO: 1698) |
| 623 | SEQ ID NO:2 (SEQ ID NO: 2) | TGTCGTACGTTATGTT (SEQ ID NO: 2226) |

(continued)

| No: | Gene | Oligo: |
|---|---|---|
| 624 | SEQ ID NO:2 (SEQ ID NO: 2) | GGTGTTGTATGTTATGT (SEQ ID NO: 2227) |
| 625 | SEQ ID NO:2 (SEQ ID NO: 2) | TTGATTGGCGGACGAG (SEQ ID NO: 1699) |
| 626 | SEQ ID NO:2 (SEQ ID NO: 2) | TTGATTGGTGGATGAG (SEQ ID NO: 1700) |
| 627 | EYA4 (SEQ ID NO: 58) | GGTATAAAATCGTAAATTTT (SEQ ID NO: 1506) |
| 628 | EYA4 (SEQ ID NO: 58) | GGGTATAAAATTGTAAATTT (SEQ ID NO: 1507) |
| 629 | EYA4 (SEQ ID NO: 58) | TATGTAGTCGCGTAGT (SEQ ID NO: 1508) |
| 630 | EYA4 (SEQ ID NO: 58) | TTTATGTAGTTGTGTAGT (SEQ ID NO: 1509) |
| 631 | EYA4 (SEQ ID NO: 58) | GTTTAGATACGAAATGTT (SEQ ID NO: 1510) |
| 632 | EYA4 (SEQ ID NO: 58) | GTTTAGATATGAAATGTTAT (SEQ ID NO: 1511) |
| 633 | EYA4 (SEQ ID NO: 58) | AGTTTTGACGTCGTTT (SEQ ID NO: 1512) |
| 634 | EYA4 (SEQ ID NO: 58) | TTGATGTTGTTTTGGAA (SEQ ID NO: 1513) |
| 635 | FHIT (SEQ ID NO: 76) | TTTTCGTTTTACGAGG (SEQ ID NO: 1374) |
| 636 | FHIT (SEQ ID NO: 76) | TTTTGTTTTATGAGGGT (SEQ ID NO: 1375) |
| 637 | FHIT (SEQ ID NO: 76) | TTTAAGTATCGATTTTAGT (SEQ ID NO: 1787) |
| 638 | FHIT (SEQ ID NO: 76) | TAAGTATTGATTTTAGTTTTA (SEQ ID NO: 1788) |
| 639 | FHIT (SEQ ID NO: 76) | GTTACGTTAGCGGGTT (SEQ ID NO: 1514) |
| 640 | FHIT (SEQ ID NO: 76) | GGTTATGTTAGTGGGT (SEQ ID NO: 1515) |
| 641 | FHIT (SEQ ID NO: 76) | TTCGGGGACGTTATAG (SEQ ID NO: 1516) |
| 642 | FHIT (SEQ ID NO: 76) | GGTTTGGGGATGTTAT (SEQ ID NO: 1517) |
| 643 | GSTP1 (SEQ ID NO: 59) | GGCGATTTCGGGGATT (SEQ ID NO: 1518) |
| 644 | GSTP1 (SEQ ID NO: 59) | GGTGATTTTGGGGATTT (SEQ ID NO: 1519) |
| 645 | GSTP1 (SEQ ID NO: 59) | GACGTTCGGGGTGTAG (SEQ ID NO: 1520) |
| 646 | GSTP1 (SEQ ID NO: 59) | GATGTTTGGGGTGTAG (SEQ ID NO: 1521) |
| 647 | GSTP1 (SEQ ID NO: 59) | AGTTCGCGGGATTTTT (SEQ ID NO: 1522) |
| 648 | GSTP1 (SEQ ID NO: 59) | GGAGTTTGTGGGATTT (SEQ ID NO: 1523) |
| 649 | GSTP1 (SEQ ID NO: 59) | AGTTTTCGTTATTAGTGA (SEQ ID NO: 1524) |
| 650 | GSTP1 (SEQ ID NO: 59) | TAGTTTTTGTTATTAGTGA (SEQ ID NO: 1525) |
| 651 | HIC (SEQ ID NO: 85) | TTTTGTCGTACGGAAT (SEQ ID NO: 1376) |
| 652 | HIC1 (SEQ ID NO: 85) | AGTTTTTGTTGTATGGA (SEQ ID NO: 1377) |
| 653 | HIC1 (SEQ ID NO: 85) | TATCGAAGTTTTCGGG (SEQ ID NO: 1526) |
| 654 | HIC1 (SEQ ID NO: 85) | TATTGAAGTTTTTGGGT (SEQ ID NO: 1527) |
| 655 | HIC1 (SEQ ID NO: 85) | TAGCGGTTATTTCGGT (SEQ ID NO: 1528) |
| 656 | HIC1 (SEQ ID NO: 85) | TTTAGTGGTTATTTTGGT (SEQ ID NO: 1529) |
| 657 | HIC1 (SEQ ID NO: 85) | TACGTTTTTCGTAGCGT (SEQ ID NO: 1530) |
| 658 | HIC1 (SEQ ID NO: 85) | ATTATGTTTTTTGTAGTGT (SEQ ID NO: 1531) |
| 659 | HIC1 (SEQ ID NO: 85) | TTCGGTTTTGTCGTATA (SEQ ID NO: 1532) |
| 660 | HIC1 (SEQ ID NO: 85) | TTTGGTTTTGTTGTATAG (SEQ ID NO: 1533) |
| 661 | IGFBP7 (SEQ ID NO: 94) | TAGTCGCGGAATGTTA (SEQ ID NO: 1701) |

(continued)

| No: | Gene | Oligo: |
|-----|------|--------|
| 662 | IGFBP7 (SEQ ID NO: 94) | TTGGTAGTTGTGGAAT (SEQ ID NO: 1702) |
| 663 | IGFBP7 (SEQ ID NO: 94) | ATTTTTTCGCGGGTAT (SEQ ID NO: 1703) |
| 664 | IGFBP7 (SEQ ID NO: 94) | TTTTTGTGGGTATTTTAG (SEQ ID NO: 1704) |
| 665 | IGFBP7 (SEQ ID NO: 94) | GGTATATTCGACGGGG (SEQ ID NO: 1705) |
| 666 | IGFBP7 (SEQ ID NO: 94) | GGGTATATTTGATGGGG (SEQ ID NO: 1706) |
| 667 | IGFBP7 (SEQ ID NO: 94) | GGTACGAGCGTTTTTT (SEQ ID NO: 1707) |
| 668 | IGFBP7 (SEQ ID NO: 94) | TGGGTATGAGTGTTTT (SEQ ID NO: 1708) |
| 669 | IGFBP7 (SEQ ID NO: 94) | AAAGCGTATTTAATTCGT (SEQ ID NO: 1709) |
| 670 | IGFBP7 (SEQ ID NO: 94) | AGTGTATTTAATTTGTGTT (SEQ ID NO: 1710) |
| 671 | MLH1 (SEQ ID NO: 89) | AAGTCGTTTTGACGTA (SEQ ID NO: 1378) |
| 672 | MLH1 (SEQ ID NO: 89) | TAAGTTGTTTTGATGTAG (SEQ ID NO: 1379) |
| 673 | MLH1 (SEQ ID NO: 89) | AGGCGGCGATAGATTA (SEQ ID NO: 1534) |
| 674 | MLH1 (SEQ ID NO: 89) | ATGAGGTGGTGATAGA (SEQ ID NO: 1535) |
| 675 | MLH1 (SEQ ID NO: 89) | TATCGTTTTTCGGAGG (SEQ ID NO: 1380) |
| 676 | MLH1 (SEQ ID NO: 89) | TATTGTTTTTTGGAGGT (SEQ ID NO: 1381) |
| 677 | MLH1 (SEQ ID NO: 89) | TAGAGTTTCGTCGATT (SEQ ID NO: 1382) |
| 678 | MLH1 (SEQ ID NO: 89) | AGAGTTTTGTTGATTTTT (SEQ ID NO: 1383) |
| 679 | PGR (SEQ ID NO: 83) | TTTCGAGGTCGGATTT (SEQ ID NO: 1536) |
| 680 | PGR (SEQ ID NO: 83) | TTTGAGGTTGGATTTTT (SEQ ID NO: 1537) |
| 681 | PGR (SEQ ID NO: 83) | GTGTCGTTTAGTCGTA (SEQ ID NO: 1538) |
| 682 | PGR (SEQ ID NO: 83) | GTTGTTTAGTTGTAGGT (SEQ ID NO: 1539) |
| 683 | PGR (SEQ ID NO: 83) | TTCGACGAAAAGACGT (SEQ ID NO: 1789) |
| 684 | PGR (SEQ ID NO: 83) | TTTTTTCGACGAAAAGA (SEQ ID NO: 1540) |
| 685 | PGR (SEQ ID NO: 83) | AGGATTTTTTTGATGAAA (SEQ ID NO: 1541) |
| 686 | PGR (SEQ ID NO: 83) | TTTTTGATGAAAAGATGT (SEQ ID NO: 1790) |
| 687 | SERPINB5 (SEQ ID NO: 68) | TTATTAACGTGTTTGAGA (SEQ ID NO: 1542) |
| 688 | SERPINB5 (SEQ ID NO: 68) | TTATTAATGTGTTTGAGAA (SEQ ID NO: 1543) |
| 689 | SERPINB5 (SEQ ID NO: 68) | ATTGTCGTACGTATGT (SEQ ID NO: 1544) |
| 690 | SERPINB5 (SEQ ID NO: 68) | AGAGGATTGTTGTATGTA (SEQ ID NO: 1545) |
| 691 | SERPINB5 (SEQ ID NO: 68) | TTTTTTGTTCGAATATGT (SEQ ID NO: 1546) |
| 692 | SERPINB5 (SEQ ID NO: 68) | TTTGTTTGAATATGTTGG (SEQ ID NO: 1547) |
| 693 | RARB (SEQ ID NO: 88) | ATCGAAAGTTTATTCGTATA (SEQ ID NO: 1791) |
| 694 | RARB (SEQ ID NO: 88) | TTATTGAAAGTTTATTTGTA (SEQ ID NO: 1792) |
| 695 | RARB (SEQ ID NO: 88) | ATGTCGAGAACGCGAG (SEQ ID NO: 1548) |
| 696 | RARB (SEQ ID NO: 88) | GGATGTTGAGAATGTGA (SEQ ID NO: 1549) |
| 697 | RARB (SEQ ID NO: 88) | AGCGATTCGAGTAGGG (SEQ ID NO: 1550) |
| 698 | RARB (SEQ ID NO: 88) | AGTGATTTGAGTAGGG (SEQ ID NO: 1551) |
| 699 | RARB (SEQ ID NO: 88) | TAGGATTCGGAACGTA (SEQ ID NO: 1552) |

(continued)

| No: | Gene | Oligo: |
|-----|------|--------|
| 700 | RARB (SEQ ID NO: 88) | GGTAGGATTTGGAATGT (SEQ ID NO: 1553) |
| 701 | SFN (SEQ ID NO: 69) | TAGTACGGTGTCGTAT (SEQ ID NO: 1554) |
| 702 | SFN (SEQ ID NO: 69) | GTTTAGTATGGTGTTGT (SEQ ID NO: 1555) |
| 703 | SFN (SEQ ID NO: 69) | TACGTATTTCGGGTTT (SEQ ID NO: 1556) |
| 704 | SFN (SEQ ID NO: 69) | TATTTATGTATTTTGGGTT (SEQ ID NO: 1557) |
| 705 | SFN (SEQ ID NO: 69) | TTCGTTTTTCGTAGGAG (SEQ ID NO: 1558) |
| 706 | SFN (SEQ ID NO: 69) | TTTGTTTTTTGTAGGAGA (SEQ ID NO: 1559) |
| 707 | SFN (SEQ ID NO: 69) | TTTATAGCGTTCGGTT (SEQ ID NO: 1830) |
| 708 | SFN (SEQ ID NO: 69) | ATAGTGTTTGGTTTGTT (SEQ ID NO: 1831) |
| 709 | SOD2 (SEQ ID NO: 105) | GTCGTTTAGTCGGTTTA (SEQ ID NO: 1711) |
| 710 | SOD2 (SEQ ID NO: 105) | GTTGTTTAGTTGGTTTAT (SEQ ID NO: 1712) |
| 711 | SOD2 (SEQ ID NO: 105) | TATTAGGCGGTTGCGG (SEQ ID NO: 1713) |
| 712 | SOD2 (SEQ ID NO: 105) | TATTAGGTGGTTGTGG (SEQ ID NO: 1714) |
| 713 | SOD2 (SEQ ID NO: 105) | TACGGTTCGAAGGTTT (SEQ ID NO: 1715) |
| 714 | SOD2 (SEQ ID NO: 105) | AGTTGGTATGGTTTGA (SEQ ID NO: 1716) |
| 715 | SOD2 (SEQ ID NO: 105) | GTTTCGGTAATTTCGT (SEQ ID NO: 1384) |
| 716 | SOD2 (SEQ ID NO: 105) | GGTTTTGGTAATTTTGTT (SEQ ID NO: 1385) |
| 717 | TERT (SEQ ID NO: 92) | AAGACGTATTGTTCGT (SEQ ID NO: 1386) |
| 718 | TERT (SEQ ID NO: 92) | AAGATGTATTGTTTGTTG (SEQ ID NO: 1387) |
| 719 | TERT (SEQ ID NO: 92) | AGGTGTACGGTTTCGT (SEQ ID NO: 1793) |
| 720 | TERT (SEQ ID NO: 92) | AGGTGTATGGTTTTGT (SEQ ID NO: 1794) |
| 721 | TERT (SEQ ID NO: 92) | TATAACGAACGTCGTT (SEQ ID NO: 1795) |
| 722 | TERT (SEQ ID NO: 92) | AGGTATAATGAATGTTGT (SEQ ID NO: 1796) |
| 723 | TERT (SEQ ID NO: 92) | ATTACGCGTAGTGTTT (SEQ ID NO: 1388) |
| 724 | TERT (SEQ ID NO: 92) | TGGGAATTATGTGTAGT (SEQ ID NO: 1389) |
| 725 | TGFBR2 (SEQ ID NO: 93) | AAGACGGTTAGGTCGG (SEQ ID NO: 1825) |
| 726 | TGFBR2 (SEQ ID NO: 93) | AAGATGGTTAGGTTGG (SEQ ID NO: 1826) |
| 727 | TGFBR2 (SEQ ID NO: 93) | TTTTATTTCGAAGCGG (SEQ ID NO: 1390) |
| 728 | TGFBR2 (SEQ ID NO: 93) | TTTGAAGTGGGTTTATT (SEQ ID NO: 1391) |
| 729 | TGFBR2 (SEQ ID NO: 93) | ATGGGGCGGACGAATA (SEQ ID NO: 1560) |
| 730 | TGFBR2 (SEQ ID NO: 93) | ATGGGGTGGATGAATA (SEQ ID NO: 1561) |
| 731 | TGFBR2 (SEQ ID NO: 93) | ATAACGTATTAGGAGCGG (SEQ ID NO: 1392) |
| 732 | TGFBR2 (SEQ ID NO: 93) | ATAATGTATTAGGAGTGG (SEQ ID NO: 1393) |
| 733 | THRB (SEQ ID NO: 106) | GAGCGTCGTAAGAATT (SEQ ID NO: 1394) |
| 734 | THRB (SEQ ID NO: 106) | GGAGTGTTGTAAGAATT (SEQ ID NO: 1395) |
| 735 | THRB (SEQ ID NO: 106) | GGGCGGTTAAGTCGAG (SEQ ID NO: 1562) |
| 736 | THRB (SEQ ID NO: 106) | GGGTGGTTAAGTTGAG (SEQ ID NO: 1563) |
| 737 | THRB (SEQ ID NO: 106) | TTTAATCGTAGCGGTT (SEQ ID NO: 1396) |

(continued)

| No: | Gene | Oligo: |
|---|---|---|
| 738 | THRB (SEQ ID NO: 106) | AATTGTAGTGGTTTTGT (SEQ ID NO: 1397) |
| 739 | THRB (SEQ ID NO: 106) | TAGCGTCGAATTTAGT (SEQ ID NO: 1398) |
| 740 | THRB (SEQ ID NO: 106) | TTAGTGTTGAATTTAGTG (SEQ ID NO: 1399) |
| 741 | TIMP3 (SEQ ID NO: 103) | TTATTAACGGAGGAAGG (SEQ ID NO: 1564) |
| 742 | TIMP3 (SEQ ID NO: 103) | TATTAATGGAGGAAGGG (SEQ ID NO: 1565) |
| 743 | TIMP3 (SEQ ID NO: 103) | TTCGTTATGTGTACGGAA (SEQ ID NO: 1566) |
| 744 | TIMP3 (SEQ ID NO: 103) | TTTGTTATGTGTATGGAA (SEQ ID NO: 1567) |
| 745 | TIMP3 (SEQ ID NO: 103) | GAGTATTTCGAGTTTGT (SEQ ID NO: 1568) |
| 746 | TIMP3 (SEQ ID NO: 103) | AGTATTTTGAGTTTGTATT (SEQ ID NO: 1569) |
| 747 | TIMP3 (SEQ ID NO: 103) | TAAGCGTTAATCGAGT (SEQ ID NO: 1570) |
| 748 | TIMP3 (SEQ ID NO: 103) | TAGGTAAGTGTTAATTGA (SEQ ID NO: 1571) |
| 749 | TP53 (SEQ ID NO: 80) | AGGATTTTCGTCGATT (SEQ ID NO: 1400) |
| 750 | TP53 (SEQ ID NO: 80) | TTAGGATTTTTGTTGATTT (SEQ ID NO: 1401) |
| 751 | TP53 (SEQ ID NO: 80) | TAATTCGTTTGTCGGA (SEQ ID NO: 1402) |
| 752 | TP53 (SEQ ID NO: 80) | ATTTGTTTGTTGGAGGA (SEQ ID NO: 1403) |
| 753 | TP53 (SEQ ID NO: 80) | TGGATAGTCGTTATGAT (SEQ ID NO: 1404) |
| 754 | TP53 (SEQ ID NO: 80) | TTGGATAGTTGTTATGAT (SEQ ID NO: 1405) |
| 755 | TP53 (SEQ ID NO: 80) | AATTTTTGCGAGGTTT (SEQ ID NO: 1406) |
| 756 | TP53 (SEQ ID NO: 80) | TAATTTTTGTGAGGTTTT (SEQ ID NO: 1407) |
| 757 | TP73 (SEQ ID NO: 86) | TAGGATTCGCGTTTTT (SEQ ID NO: 1572) |
| 758 | TP73 (SEQ ID NO: 86) | GGTAGGATTTGTGTTTT (SEQ ID NO: 1573) |
| 759 | TP73 (SEQ ID NO: 86) | TTTTTCGCGAGCGTTA (SEQ ID NO: 1408) |
| 760 | TP73 (SEQ ID NO: 86) | TTTTGTGAGTGTTAAGT (SEQ ID NO: 1409) |
| 761 | TP73 (SEQ ID NO: 86) | GGGTCGATTTAATTTCGA (SEQ ID NO: 1410) |
| 762 | TP73 (SEQ ID NO: 86) | GGGTTGATTTAATTTTGA (SEQ ID NO: 1411) |
| 763 | TP73 (SEQ ID NO: 86) | TTTCGGAGTTGCGAGT (SEQ ID NO: 1574) |
| 764 | TP73 (SEQ ID NO: 86) | TAGTTTTGGAGTTGTGA (SEQ ID NO: 1575) |
| 765 | NME1 (SEQ ID NO: 107) | AGTCGAGATTGCGTTA (SEQ ID NO: 1805) |
| 766 | NME1 (SEQ ID NO: 107) | AGTTGAGATTGTGTTAG (SEQ ID NO: 1806) |
| 767 | NME1 (SEQ ID NO: 107) | ATCGTTTGAATTCGGGA (SEQ ID NO: 1807) |
| 768 | NME1 (SEQ ID NO: 107) | ATTGTTTGAATTTGGGA (SEQ ID NO: 1808) |
| 769 | NME1 (SEQ ID NO: 107) | AATTCGAGATTAGTTCGG (SEQ ID NO: 1717) |
| 770 | NME1 (SEQ ID NO: 107) | AATTTGAGATTAGTTTGG (SEQ ID NO: 1718) |
| 771 | NME1 (SEQ ID NO: 107) | TTTTAGTACGTTGGAAA (SEQ ID NO: 1809) |
| 772 | NME1 (SEQ ID NO: 107) | TTAGTATGTTGGAAAGTA (SEQ ID NO: 1810) |
| 773 | CDH 13 (SEQ ID NO: 70) | TAAAACGAGGGAGCGT (SEQ ID NO: 1576) |
| 774 | CDH13 (SEQ ID NO: 70) | AAAATGAGGGAGTGTT (SEQ ID NO: 1577) |
| 775 | CDH13 (SEQ ID NO: 70) | TAGTCGCGTGTATGAA (SEQ ID NO: 1578) |

(continued)

| No: | Gene | Oligo: |
|-----|------|--------|
| 776 | CDH 13 (SEQ ID NO: 70) | TGTAGTTGTGTGTATGA (SEQ ID NO: 1579) |
| 777 | CDH13 (SEQ ID NO: 70) | ATGAAAACGTCGTCGG (SEQ ID NO: 1580) |
| 778 | CDH13 (SEQ ID NO: 70) | AATGAAAATGTTGTTGG (SEQ ID NO: 1581) |
| 779 | CDH13 (SEQ ID NO: 70) | TAGTCGAGAATTTCGT (SEQ ID NO: 1582) |
| 780 | CDH13 (SEQ ID NO: 70) | TGTAGTTGAGAATTTTGT (SEQ ID NO: 1583) |
| 781 | THBS1 (SEQ ID NO: 81) | TTGGCGCGTAATTTTT (SEQ ID NO: 1811) |
| 782 | THBS1 (SEQ ID NO: 81) | TGTTTGGTGTGTAATTT (SEQ ID NO: 1812) |
| 783 | THBS1 (SEQ ID NO: 81) | TAAAGGGGCGTTCGTA (SEQ ID NO: 1719) |
| 784 | THBS1 (SEQ ID NO: 81) | AAGGGGTGTTTGTATT (SEQ ID NO: 1720) |
| 785 | THBS1 (SEQ ID NO: 81) | GGTTAGTTCGGGCGTA (SEQ ID NO: 1721) |
| 786 | THBS1 (SEQ ID NO: 81) | GGTTAGTTTGGGTGTA (SEQ ID NO: 1722) |
| 787 | THBS1 (SEQ ID NO: 81) | TTGTGCGTTCGGAGTA (SEQ ID NO: 1723) |
| 788 | THBS1 (SEQ ID NO: 81) | TGTGTTTGGAGTAGAG (SEQ ID NO: 1724) |
| 789 | TMS1/ASC (SEQ ID NO: 84) | TTCGTTTCGGAGTCGA (SEQ ID NO: 1584) |
| 790 | TMS1/ASC (SEQ ID NO: 84) | TTTGTTTTGGAGTTGAT (SEQ ID NO: 1585) |
| 791 | TMS1/ASC (SEQ ID NO: 84) | TTCGGAGTCGATTTTT (SEQ ID NO: 1412) |
| 792 | TMS1/ASC (SEQ ID NO: 84) | GTTTTGGAGTTGATTTTT (SEQ ID NO: 1413) |
| 793 | TMS1/ASC (SEQ ID NO: 84) | ATTTGATCGTCGAGGA (SEQ ID NO: 1414) |
| 794 | TMS1/ASC (SEQ ID NO: 84) | TTGATTGTTGAGGAGT (SEQ ID NO: 1415) |
| 795 | TMS1/ASC (SEQ ID NO: 84) | AGGGTTACGGGCGTAT (SEQ ID NO: 1416) |
| 796 | TMS1/ASC (SEQ ID NO: 84) | AGGGTTATGGGTGTAT (SEQ ID NO: 1417) |
| 797 | ESR1 (SEQ ID NO: 75) | AAATCGGCGGGTTATT (SEQ ID NO: 1725) |
| 798 | ESR1 (SEQ ID NO: 75) | AGAAATTGGTGGGTTA (SEQ ID NO: 1726) |
| 799 | ESR1 (SEQ ID NO: 75) | AGATCGTGTTTTCGTA (SEQ ID NO: 1836) |
| 800 | ESR1 (SEQ ID NO: 75) | ATTGTGTTTTTGTAGGG (SEQ ID NO: 1837) |
| 801 | ESR1 (SEQ ID NO: 75) | AGTTGCGGACGGTTTA (SEQ ID NO: 1727) |
| 802 | ESR1 (SEQ ID NO: 75) | AGTTGTGGATGGTTTA (SEQ ID NO: 1728) |
| 803 | IL6 (SEQ ID NO: 99) | AGATGTCGTCGAGGAT (SEQ ID NO: 1813) |
| 804 | IL6 (SEQ ID NO: 99) | ATGTTGTTGAGGATGTA (SEQ ID NO: 1814) |
| 805 | IL6 (SEQ ID NO: 99) | TATGCGTATCGGGTTT (SEQ ID NO: 1418) |
| 806 | IL6 (SEQ ID NO: 99) | ATGTGTATTGGGTTTTT (SEQ ID NO: 1419) |
| 807 | IL6 (SEQ ID NO: 99) | TTCGGTTATACGTAGG (SEQ ID NO: 1729) |
| 808 | IL6 (SEQ ID NO: 99) | TTTTGGTTATATGTAGGG (SEQ ID NO: 1730) |
| 809 | IL6 (SEQ ID NO: 99) | AGTTTAGTCGGTTTCGT (SEQ ID NO: 1731) |
| 810 | IL6 (SEQ ID NO: 99) | AGTTTAGTTGGTTTTGT (SEQ ID NO: 1732) |
| 811 | APAF1 (SEQ ID NO: 82) | GATTCGCGAAGATTTG (SEQ ID NO: 1420) |
| 812 | APAF1 (SEQ ID NO: 82) | GATTTGTGAAGATTTGAG (SEQ ID NO: 1421) |
| 813 | APAF1 (SEQ ID NO: 82) | GTGTCGTAGCGGTATT (SEQ ID NO: 1586) |

(continued)

| No: | Gene | Oligo: |
|-----|------|--------|
| 814 | APAF1 (SEQ ID NO: 82) | GGTGTTGTAGTGGTAT (SEQ ID NO: 1587) |
| 815 | APAF1 (SEQ ID NO: 82) | AGTAGCGTCGGGTTTT (SEQ ID NO: 1588) |
| 816 | APAF1 (SEQ ID NO: 82) | GAGTAGTGTTGGGTTT (SEQ ID NO: 1589) |
| 817 | APAF1 (SEQ ID NO: 82) | TGGACGTTTATTTCGT (SEQ ID NO: 1422) |
| 818 | APAF1 (SEQ ID NO: 82) | GTGGATGTTTATTTTGTT (SEQ ID NO: 1423) |
| 819 | CASP8 (SEQ ID NO: 71) | AGGTAACGGTTTAGAG (SEQ ID NO: 1424) |
| 820 | CASP8 (SEQ ID NO: 71) | AGGTAATGGTTTAGAGA (SEQ ID NO: 1425) |
| 821 | CASP8 (SEQ ID NO: 71) | TGTATTCGAGGCGGTA (SEQ ID NO: 1733) |
| 822 | CASP8 (SEQ ID NO: 71) | TTGTATTTGAGGTGGT (SEQ ID NO: 1734) |
| 823 | CASP8 (SEQ ID NO: 71) | ATTTTTTAAACGGGTTTA (SEQ ID NO: 1735) |
| 824 | CASP8 (SEQ ID NO: 71) | TTTTAAATGGGTTTATAGG (SEQ ID NO: 1736) |
| 825 | CASP8 (SEQ ID NO: 71) | ATCGTAGTTTTCGAGT (SEQ ID NO: 1737) |
| 826 | CASP8 (SEQ ID NO: 71) | ATTGTAGTTTTTGAGTTT (SEQ ID NO: 1738) |
| 827 | SYK (SEQ ID NO: 60) | GAAGTTATCGCGTTGG (SEQ ID NO: 1590) |
| 828 | SYK (SEQ ID NO: 60) | AGAAGTTATTGTGTTGG (SEQ ID NO: 1591) |
| 829 | SYK (SEQ ID NO: 60) | GATCGATGCGGTTTAT (SEQ ID NO: 1592) |
| 830 | SYK (SEQ ID NO: 60) | GGGATTGATGTGGTTT (SEQ ID NO: 1593) |
| 831 | SYK (SEQ ID NO: 60) | GGCGTTTTAGTCGATT (SEQ ID NO: 1594) |
| 832 | SYK (SEQ ID NO: 60) | GGTGTTTTAGTTGATTTT (SEQ ID NO: 1595) |
| 833 | SYK (SEQ ID NO: 60) | TTATTCGGTCGGGATT (SEQ ID NO: 1596) |
| 834 | SYK (SEQ ID NO: 60) | TTTATTTGGTTGGGATT (SEQ ID NO: 1597) |
| 835 | HOXA5 (SEQ ID NO: 78) | TTCGAGTTCGGTTGAA (SEQ ID NO: 1739) |
| 836 | HOXA5 (SEQ ID NO: 78) | GTTTGAGTTTGGTTGAA (SEQ ID NO: 1740) |
| 837 | HOXA5 (SEQ ID NO: 78) | TAGTTTTCGGTCGGAA (SEQ ID NO: 1741) |
| 838 | HOXA5 (SEQ ID NO: 78) | TAGTTTTTGGTTGGAAG (SEQ ID NO: 1742) |
| 839 | HOXA5 (SEQ ID NO: 78) | TAATTCGATTTCGGTTT (SEQ ID NO: 1743) |
| 840 | HOXA5 (SEQ ID NO: 78) | TTTAATTTGATTTTGGTTT (SEQ ID NO: 1744) |
| 841 | HOXA5 (SEQ ID NO: 78) | ATCGGTAGTTGACGGTT (SEQ ID NO: 1745) |
| 842 | HOXA5 (SEQ ID NO: 78) | ATTGGTAGTTGATGGTT (SEQ ID NO: 1746) |
| 843 | FABP3 (SEQ ID NO: 77) | GATGGGCGTATTAGTT (SEQ ID NO: 1598) |
| 844 | FABP3 (SEQ ID NO: 77) | GGGATGGGTGTATTAG (SEQ ID NO: 1599) |
| 845 | FABP3 (SEQ ID NO: 77) | GTGATGCGAGGGTTAT (SEQ ID NO: 1600) |
| 846 | FABP3 (SEQ ID NO: 77) | GTGATGTGAGGGTTAT (SEQ ID NO: 1601) |
| 847 | FABP3 (SEQ ID NO: 77) | TAAAGCGGTAGTTCGG (SEQ ID NO: 1602) |
| 848 | FABP3 (SEQ ID NO: 77) | AAGTGGTAGTTTGGGT (SEQ ID NO: 1603) |
| 849 | FABP3 (SEQ ID NO: 77) | TATTGGCGTTGACGTA (SEQ ID NO: 1604) |
| 850 | FABP3 (SEQ ID NO: 77) | TGGTGTTGATGTAGGT (SEQ ID NO: 1605) |
| 851 | RASSF1A (SEQ ID NO: 90) | TACGGGTATTTTCGCGT (SEQ ID NO: 1606) |

(continued)

| No: | Gene | Oligo: |
|---|---|---|
| 852 | RASSF1A (SEQ ID NO: 90) | ATATGGGTATTTTTGTGT (SEQ ID NO: 1607) |
| 853 | RASSF1A (SEQ ID NO: 90) | AGAGCGCGTTTAGTTT (SEQ ID NO: 1608) |
| 854 | RASSF1A (SEQ ID NO: 90) | GAGAGTGTGTTTAGTTT (SEQ ID NO: 1609) |
| 855 | RASSF1A (SEQ ID NO: 90) | AGTAAATCGGATTAGGA (SEQ ID NO: 1610) |
| 856 | RASSF1A (SEQ ID NO: 90) | AGTAAATTGGATTAGGAG (SEQ ID NO: 1611) |
| 857 | RARA (SEQ ID NO: 108) | TTCGGGATGTACGTTT (SEQ ID NO: 1747) |
| 858 | RARA (SEQ ID NO: 108) | TTTGGGATGTATGTTTT (SEQ ID NO: 1748) |
| 859 | RARA (SEQ ID NO: 108) | GAATTAGTATCGGTTTTT (SEQ ID NO: 1749) |
| 860 | RARA (SEQ ID NO: 108) | ATTAGTATTGGTTTTTGG (SEQ ID NO: 1750) |
| 861 | RARA (SEQ ID NO: 108) | AAAGTTTCGTTTAAGGT (SEQ ID NO: 1426) |
| 862 | RARA (SEQ ID NO: 108) | AAAGTTTTGTTTAAGGTG (SEQ ID NO: 1427) |
| 863 | RARA (SEQ ID NO: 108) | TTTCGGCGAGTAAAGT (SEQ ID NO: 1428) |
| 864 | RARA (SEQ ID NO: 108) | TTTTTGGTGAGTAAAGT (SEQ ID NO: 1429) |
| 865 | TWIST (SEQ ID NO: 100) | AGTAAAGGCGTTGCGT (SEQ ID NO: 1612) |
| 866 | TWIST (SEQ ID NO: 100) | AGTAAAGGTGTTGTGT (SEQ ID NO: 1613) |
| 867 | TWIST (SEQ ID NO: 100) | TATTTTTCGAGGCGTA (SEQ ID NO: 1614) |
| 868 | TWIST (SEQ ID NO: 100) | TTTTGAGGTGTAGTTTT (SEQ ID NO: 1615) |
| 869 | TWIST (SEQ ID NO: 100) | ATTGGGTCGTTGTAGA (SEQ ID NO: 1616) |
| 870 | TWIST (SEQ ID NO: 100) | ATTGGGTTGTTGTAGA (SEQ ID NO: 1617) |
| 871 | TWIST (SEQ ID NO: 100) | TAGGTCGGGACGTAAA (SEQ ID NO: 1618) |
| 872 | TWIST (SEQ ID NO: 100) | AGTAGGTTGGGATGTA (SEQ ID NO: 1619) |
| 873 | AR (SEQ ID NO: 61) | TTAAGAGGCGAAAAGT (SEQ ID NO: 1430) |
| 874 | AR (SEQ ID NO: 61) | AAGAGGTGAAAAGTAGT (SEQ ID NO: 1431) |
| 875 | AR (SEQ ID NO: 61) | AATATGCGAAGGTAATT (SEQ ID NO: 1432) |
| 876 | AR (SEQ ID NO: 61) | TTAAAATATGTGAAGGTAA (SEQ ID NO: 1433) |
| 877 | AR (SEQ ID NO: 61) | AAGTAAGCGGTTGTAT (SEQ ID NO: 1434) |
| 878 | AR (SEQ ID NO: 61) | AAGTAAGTGGTTGTATAT (SEQ ID NO: 1435) |
| 879 | AR (SEQ ID NO: 61) | ATTGAGCGTTTATGTG (SEQ ID NO: 1436) |
| 880 | AR (SEQ ID NO: 61) | ATTGAGTGTTTATGTGT (SEQ ID NO: 1437) |
| 881 | ESR2 (SEQ ID NO: 91) | GAGTATTTTCGAATCGA (SEQ ID NO: 1620) |
| 882 | ESR2 (SEQ ID NO: 91) | GGAGTATTTTTGAATTGA (SEQ ID NO: 1621) |
| 883 | ESR2 (SEQ ID NO: 91) | ATAAGCGATTTAACGAT (SEQ ID NO: 1622) |
| 884 | ESR2 (SEQ ID NO: 91) | AAGTGATTTAATGATAAGT (SEQ ID NO: 1623) |
| 885 | ESR2 (SEQ ID NO: 91) | ATTTCGAGGATTACGT (SEQ ID NO: 1438) |
| 886 | ESR2 (SEQ ID NO: 91) | ATATTTTGAGGATTATGTT (SEQ ID NO: 1439) |
| 887 | ESR2 (SEQ ID NO: 91) | TTTACGTGATCGAGTT (SEQ ID NO: 1624) |
| 888 | ESR2 (SEQ ID NO: 91) | AGTTTATGTGATTGAGTT (SEQ ID NO: 1625) |
| 889 | PLAU (SEQ ID NO: 62) | TATTTGTCGCGTTGAT (SEQ ID NO: 1626) |

(continued)

| No: | Gene | Oligo: |
|-----|------|--------|
| 890 | PLAU (SEQ ID NO: 62) | ATTTGTTGTGTTGATGA (SEQ ID NO: 1627) |
| 891 | PLAU (SEQ ID NO: 62) | TGTAATTCGGGGATTT (SEQ ID NO: 1628) |
| 892 | PLAU (SEQ ID NO: 62) | TTGTAATTTGGGGATTT (SEQ ID NO: 1629) |
| 893 | PLAU (SEQ ID NO: 62) | TTGGAGATCGCGTTTT (SEQ ID NO: 1630) |
| 894 | PLAU (SEQ ID NO: 62) | TTGGAGATTGTGTTTTT (SEQ ID NO: 1631) |
| 895 | PLAU (SEQ ID NO: 62) | GAGCGTTGCGGAAGTA (SEQ ID NO: 1632) |
| 896 | PLAU (SEQ ID NO: 62) | GAGTGTTGTGGAAGTA (SEQ ID NO: 1633) |
| 897 | STAT1 (SEQ ID NO: 109) | GTTATTTTCGAGAGTTG (SEQ ID NO: 1634) |
| 898 | STAT1 (SEQ ID NO: 109) | GTTATTTTTGAGAGTTGT (SEQ ID NO: 1635) |
| 899 | STAT1 (SEQ ID NO: 109) | AGGTTAACGTTCGTAT (SEQ ID NO: 1440) |
| 900 | STAT1 (SEQ ID NO: 109) | TTAGGTTAATGTTTGTATT (SEQ ID NO: 1441) |
| 901 | STAT1 (SEQ ID NO: 109) | TAATTTCGATAGTTTTGG (SEQ ID NO: 1442) |
| 902 | STAT1 (SEQ ID NO: 109) | TATAATTTTGATAGTTTTGG (SEQ ID NO: 1443) |
| 903 | STAT1 (SEQ ID NO: 109) | ATATGATTTCGGAATTTTA (SEQ ID NO: 1797) |
| 904 | STAT1 (SEQ ID NO: 109) | ATATGATTTTGGAATTTTAA (SEQ ID NO: 1798) |
| 905 | BRCA1 (SEQ ID NO: 66) | AGTTTCGAGAGACGTT (SEQ ID NO: 1636) |
| 906 | BRCA1 (SEQ ID NO: 66) | AGAGTTTTGAGAGATGT (SEQ ID NO: 1637) |
| 907 | BRCA1 (SEQ ID NO: 66) | TTTCGTGGTAACGGAA (SEQ ID NO: 1638) |
| 908 | BRCA1 (SEQ ID NO: 66) | TTTGTGGTAATGGAAAA (SEQ ID NO: 1639) |
| 909 | BRCA1 (SEQ ID NO: 66) | AAAGCGCGGGAATTAT (SEQ ID NO: 1640) |
| 910 | BRCA1 (SEQ ID NO: 66) | GGAAAAGTGTGGGAAT (SEQ ID NO: 1641) |
| 911 | BRCA1 (SEQ ID NO: 66) | TTTACGTTATTCGGGG (SEQ ID NO: 1444) |
| 912 | BRCA1 (SEQ ID NO: 66) | TATGTTATTTGGGGGTA (SEQ ID NO: 1445) |
| 913 | LOT (SEQ ID NO: 95) | ATGGGTACGTTTAAGG (SEQ ID NO: 1642) |
| 914 | LOT1 (SEQ ID NO: 95) | TGGGTATGTTTAAGGG (SEQ ID NO: 1643) |
| 915 | LOT1 (SEQ ID NO: 95) | AAATTAGTTACGTTATTTAA (SEQ ID NO: 1644) |
| 916 | LOT1 (SEQ ID NO: 95) | TGAAATTAGTTATGTTATTTA (SEQ ID NO: 1645) |
| 917 | LOT1 (SEQ ID NO: 95) | GAGTTTCGTGGTTGAA (SEQ ID NO: 1799) |
| 918 | LOT1 (SEQ ID NO: 95) | GAGTTTTGTGGTTGAA (SEQ ID NO: 1800) |
| 919 | LOT1 (SEQ ID NO: 95) | ATGTCGGTTATTACGT (SEQ ID NO: 1646) |
| 920 | LOT1 (SEQ ID NO: 95) | TGTTGGTTATTATGTAGA (SEQ ID NO: 1647) |
| 921 | PRSS8 (SEQ ID NO: 72) | AGTTGGCGGAGTTTAG (SEQ ID NO: 1648) |
| 922 | PRSS8 (SEQ ID NO: 72) | AGTTGGTGGAGTTTAG (SEQ ID NO: 1649) |
| 923 | PRSS8 (SEQ ID NO: 72) | ATTTTGCGTGGTTAGA (SEQ ID NO: 1446) |
| 924 | PRSS8 (SEQ ID NO: 72) | GATTTTGTGTGGTTAGA (SEQ ID NO: 1447) |
| 925 | PRSS8 (SEQ ID NO: 72) | TTGGTGATTCGTTTATAT (SEQ ID NO: 1650) |
| 926 | PRSS8 (SEQ ID NO: 72) | GTTGGTGATTTGTTTATA (SEQ ID NO: 1651) |
| 927 | PRSS8 (SEQ ID NO: 72) | TGTTCGTTTCGGATAT (SEQ ID NO: 1652) |

(continued)

| No: | Gene | Oligo: |
|---|---|---|
| 928 | PRSS8 (SEQ ID NO: 72) | TTTGTTTTGGATATTTTAG (SEQ ID NO: 1653) |
| 929 | SNCG (SEQ ID NO: 73) | TGCGGTAGTATTCGAGT (SEQ ID NO: 1751) |
| 930 | SNCG (SEQ ID NO: 73) | TGTGGTAGTATTTGAGT (SEQ ID NO: 1752) |
| 931 | SNCG (SEQ ID NO: 73) | TATCGGGGATAGTCGTT (SEQ ID NO: 1815) |
| 932 | SNCG (SEQ ID NO: 73) | TATTGGGGATAGTTGTT (SEQ ID NO: 1816) |
| 933 | SNCG (SEQ ID NO: 73) | TATCGGCGTTAATAGG (SEQ ID NO: 1817) |
| 934 | SNCG (SEQ ID NO: 73) | TATTGGTGTTAATAGGAG (SEQ ID NO: 1818) |
| 935 | SNCG (SEQ ID NO: 73) | ATTGTACGTAGGGTTG (SEQ ID NO: 1819) |
| 936 | SNCG (SEQ ID NO: 73) | TTTTATTGTATGTAGGGT (SEQ ID NO: 1820) |
| 937 | TPM1 (SEQ ID NO: 110) | TTGATTCGCGTTCGTA (SEQ ID NO: 1654) |
| 938 | TPM1 (SEQ ID NO: 110) | TGATTTGTGTTTGTAGA (SEQ ID NO: 1655) |
| 939 | TPM1 (SEQ ID NO: 110) | AAGAGTCGTTTTAGGT (SEQ ID NO: 1448) |
| 940 | TPM1 (SEQ ID NO: 110) | TAAGAGTTGTTTTAGGTT (SEQ ID NO: 1449) |
| 941 | TPM1 (SEQ ID NO: 110) | GTCGAGAGCGTATTGA (SEQ ID NO: 1450) |
| 942 | TPM1 (SEQ ID NO: 110) | GGTGTTGAGAGTGTAT (SEQ ID NO: 1451) |
| 943 | TPM1 (SEQ ID NO: 110) | GGCGACGCGTATTTAT (SEQ ID NO: 1452) |
| 944 | TPM1 (SEQ ID NO: 110) | GGGGTGATGTGTATTT (SEQ ID NO: 1453) |
| 945 | GPC3 (SEQ ID NO: 118) | AATAGTCGCGTTTAGG (SEQ ID NO: 1753) |
| 946 | GPC3 (SEQ ID NO: 118) | TAGTTGTGTTTAGGGAT (SEQ ID NO: 1754) |
| 947 | GPC3 (SEQ ID NO: 118) | TTTAACGTAGTTTTGATCGG (SEQ ID NO: 1755) |
| 948 | GPC3 (SEQ ID NO: 118) | TTTAATGTAGTTTTGATTGG (SEQ ID NO: 1756) |
| 949 | GPC3 (SEQ ID NO: 118) | TTTTTCGGGAATCGTT (SEQ ID NO: 1454) |
| 950 | GPC3 (SEQ ID NO: 118) | AGTTTTTTGGGAATTGT (SEQ ID NO: 1455) |
| 951 | GPC3 (SEQ ID NO: 118) | TTCGAGCGCGTTGATT (SEQ ID NO: 1456) |
| 952 | GPC3 (SEQ ID NO: 118) | TAGGTTTGAGTGTGTT (SEQ ID NO: 1457) |
| 953 | CLDN7 (SEQ ID NO: 87) | TTACGTTAAGTCGGGT (SEQ ID NO: 1757) |
| 954 | CLDN7 (SEQ ID NO: 87) | AGTTATGTTAAGTTGGG (SEQ ID NO: 1758) |
| 955 | CLDN7 (SEQ ID NO: 87) | TAGCGTTTTAGGCGTA (SEQ ID NO: 1759) |
| 956 | CLDN7 (SEQ ID NO: 87) | TAGTGTTTTAGGTGTATT (SEQ ID NO: 1760) |
| 957 | CLDN7 (SEQ ID NO: 87) | TTAGGGGCGTTTCGTA (SEQ ID NO: 1761) |
| 958 | CLDN7 (SEQ ID NO: 87) | TTAGGGGTGTTTTGTAG (SEQ ID NO: 1762) |
| 959 | CLDN7 (SEQ ID NO: 87) | TAGAATTCGGCGGGGA (SEQ ID NO: 1763) |
| 960 | CLDN7 (SEQ ID NO: 87) | TAGAATTTGGTGGGGA (SEQ ID NO: 1764) |
| 961 | SLC19A1 (SEQ ID NO: 116) | TATAAGTTCGTACGGG (SEQ ID NO: 1458) |
| 962 | SLC19A1 (SEQ ID NO: 116) | ATAAGTTTGTATGGGTTA (SEQ ID NO: 1459) |
| 963 | SLC19A1 (SEQ ID NO: 116) | GTCGTGCGGTTTTTAA (SEQ ID NO: 1656) |
| 964 | SLC19A (SEQ ID NO: 116) | GGTTGTGTGGTTTTTAA (SEQ ID NO: 1657) |
| 965 | SLC19A1 (SEQ ID NO: 116) | TTACGAAGGCGGTTTA (SEQ ID NO: 1658) |

(continued)

| No: | Gene | Oligo: |
|---|---|---|
| 966 | SLC19A1 (SEQ ID NO: 116) | TTTTATGAAGGTGGTTT (SEQ ID NO: 1659) |
| 967 | SLC19A1 (SEQ ID NO: 116) | GGTTCGATTCGCGTTT (SEQ ID NO: 1460) |
| 968 | SLC19A1 (SEQ ID NO: 116) | TGGGTTTGATTTGTGTT (SEQ ID NO: 1461) ) |
| 969 | GJB2 (SEQ ID NO: 111) | GGATTTCGTCGGTATT (SEQ ID NO: 1660) |
| 970 | GJB2 (SEQ ID NO: 111) | GGGGATTTTGTTGGTA (SEQ ID NO: 1661) |
| 971 | GJB2 (SEQ ID NO: 111) | TTTCGCGATTCGAATA (SEQ ID NO: 1462) |
| 972 | GJB2 (SEQ ID NO: 111) | TAGTTTTTGTGATTTGAA (SEQ ID NO: 1463) |
| 973 | GJB2 (SEQ ID NO: 111) | TTTCGTATTATGCGGA (SEQ ID NO: 1801) |
| 974 | GJB2 (SEQ ID NO: 111) | TTTGTATTATGTGGAGTA (SEQ ID NO: 1802) |
| 975 | GJB2 (SEQ ID NO: 111) | GAATTTCGTTTACGGT (SEQ ID NO: 1662) |
| 976 | GJB2 (SEQ ID NO: 111) | TTGAATTTTGTTTATGGT (SEQ ID NO: 1663) |
| 977 | SLIT2 (SEQ ID NO: 112) | TTCGATAGTTAACGATG (SEQ ID NO: 1765) |
| 978 | SLIT2 (SEQ ID NO: 112) | TTTGATAGTTAATGATGGT (SEQ ID NO: 1766) |
| 979 | SLIT2 (SEQ ID NO: 112) | ATTTCGTCGTAGTTTG (SEQ ID NO: 1767) |
| 980 | SLIT2 (SEQ ID NO: 112) | TTTTGTTGTAGTTTGGA (SEQ ID NO: 1768) |
| 981 | SLIT2 (SEQ ID NO: 112) | TAGCGGGTTCGTAGTA (SEQ ID NO: 1769) |
| 982 | SLIT2 (SEQ ID NO: 112) | TTAGTGGGTTTGTAGTA (SEQ ID NO: 1770) |
| 983 | SLIT2 (SEQ ID NO: 112) | AAGGCGCGGAAGTTTA (SEQ ID NO: 1771) |
| 984 | SLIT2 (SEQ ID NO: 112) | AAGGTGTGGAAGTTTA (SEQ ID NO: 1772) |
| 985 | IGSF4 (SEQ ID NO: 74) | AGGTAGATCGAGGAGG (SEQ ID NO: 1773) |
| 986 | IGSF4 (SEQ ID NO: 74) | AGGTAGATTGAGGAGG (SEQ ID NO: 1774) |
| 987 | IGSF4 (SEQ ID NO: 74) | TAGTCGTAGAGTCGGG (SEQ ID NO: 1775) |
| 988 | IGSF4 (SEQ ID NO: 74) | GTTGTAGAGTTGGGTT (SEQ ID NO: 1776) |
| 989 | IGSF4 (SEQ ID NO: 74) | TTAGCGGTAGATTCGG (SEQ ID NO: 1464) |
| 990 | IGSF4 (SEQ ID NO: 74) | AGTGGTAGATTTGGGG (SEQ ID NO: 1465) |
| 991 | IGSF4 (SEQ ID NO: 74) | TAGGTTTTCGGATTGA (SEQ ID NO: 1777) |
| 992 | IGSF4 (SEQ ID NO: 74) | GTAGGTTTTTGGATTGA (SEQ ID NO: 1778) |
| 993 | MCT1 (SEQ ID NO: 101) | ATTTTACGTAGGCGTT (SEQ ID NO: 1779) |
| 994 | MCT1 (SEQ ID NO: 101) | GATTTTATGTAGGTGTTT (SEQ ID NO: 1780) |
| 995 | MCT1 (SEQ ID NO: 101) | AGTTAGTCGCGTTTTA (SEQ ID NO: 1781) |
| 996 | MCT1 (SEQ ID NO: 101) | AGAGTTAGTTGTGTTTTA (SEQ ID NO: 1782) |
| 997 | MCT1 (SEQ ID NO: 101) | AGTCGTTATTCGGAAA (SEQ ID NO: 1821) |
| 998 | MCT1 (SEQ ID NO: 10 1) | TGTAGTTGTTATTTGGAA (SEQ ID NO: 1822) |
| 999 | MCT1 (SEQ ID NO: 101) | TATACGAGGAAGGTCGG (SEQ ID NO: 1783) |
| 1000 | MCT1 (SEQ ID NO: 101) | TATATGAGGAAGGTTGG (SEQ ID NO: 1784) |
| 1001 | CCND2 (SEQ ID NO: 104) | AACGTTATTAGATCGTAT (SEQ ID NO: 1466) |
| 1002 | CCND2 (SEQ ID NO: 104) | AGAAATGTTATTAGATTGT (SEQ ID NO: 1467) |
| 1003 | CCND2 (SEQ ID NO: 104) | TTAGGGTCGATCGTGT (SEQ ID NO: 1486) |

(continued)

| No: | Gene | Oligo: |
|---|---|---|
| 1004 | CCND2 (SEQ ID NO: 104) | TAGGGTTGATTGTGTT (SEQ ID NO: 1487) |
| 1005 | CCND2 (SEQ ID NO: 104) | TTATCGTAGTCGGTTT (SEQ ID NO: 1488) |
| 1006 | CCND2 (SEQ ID NO: 104) | GATTTTATTGTAGTTGGT (SEQ ID NO:1489) |
| 1007 | CCND2 (SEQ ID NO: 104) | GAGTGAGGCGCGAAAT (SEQ ID NO: 1490) |
| 1008 | CCND2 (SEQ ID NO: 104) | GAGTGAGGTGTGAAAT (SEQ ID NO: 1491) |
| 1009 | CCND2 (SEQ ID NO: 104) | AAGGATCGAGCGTGGA (SEQ ID NO: 1492) |
| 1010 | CCND2 (SEQ ID NO: 104) | AAGGATTGAGTGTGGA (SEQ ID NO: 1493) |
| 1011 | CDKN2A (SEQ ID NO: 57) | GGCGTTGTTTAACGTAT (SEQ ID NO: 1496) |
| 1012 | CDKN2A (SEQ ID NO: 57) | GGGTGTTGTTTAATGTA (SEQ ID NO: 1497) |
| 1013 | CDKN2A (SEQ ID NO: 57) | AATAGTTACGGTCGGA (SEQ ID NO: 1498) |
| 1014 | CDKN2A (SEQ ID NO: 57) | AGTTATGGTTGGAGGT (SEQ ID NO: 1499) |
| 1015 | CDKN2A (SEQ ID NO: 57) | GTCGGAGGTCGATTTA (SEQ ID NO: 1500) |
| 1016 | CDKN2A (SEQ ID NO: 57) | GGTTGGAGGTTGATTTA (SEQ ID NO: 1501) |

Table 3: Genes and sequences

| Gene | Genomic SEQ ID NO: | Sense methylated converted SEQ ID NO: | Antisense methylated converted SEQ ID NO: | Sense unmethylated converted ID NO: | Antisense unmethylated SEQconverted SEQ ID NO: |
|---|---|---|---|---|---|
| Genomic region | 1 | 493 | 494 | 729 | 730 |
| Genomic region | 2 | 495 | 496 | 731 | 732 |
| Genomic region | 3 | 497 | 498 | 733 | 734 |
| Genomic region | 4 | 499 | 500 | 735 | 736 |
| Genomic region | 5 | 501 | 502 | 737 | 738 |
| Genomic region | 6 | 503 | 504 | 739 | 740 |
| Genomic region | 7 | 505 | 506 | 741 | 742 |
| Genomic region | 8 | 507 | 508 | 743 | 744 |
| Genomic region | 9 | 509 | 510 | 745 | 746 |
| PROSTAGLANDIN E2 RECEPTOR | 10 | 511 | 512 | 747 | 748 |
| ORPHAN NUCLEAR RECEPTOR NR5A2 | 11 | 513 | 514 | 749 | 750 |
| LIM DOMAIN KINASE 1 (LIMK-1) | 12 | 515 | 516 | 751 | 752 |
| MSF | 13 | 517 | 518 | 753 | 754 |
| Genomic region | 14 | 519 | 520 | 755 | 756 |
| Genomic region | 15 | 521 | 522 | 757 | 758 |
| Genomic region | 16 | 523 | 524 | 759 | 760 |

(continued)

| Gene | Genomic SEQ ID NO: | Sense methylated converted SEQ ID NO: | Antisense methylated converted SEQ ID NO: | Sense unmethylated converted ID NO: | Antisense unmethylated SEQconverted SEQ ID NO: |
|---|---|---|---|---|---|
| Genomic region | 17 | 525 | 526 | 761 | 762 |
| Genomic region | 18 | 527 | 528 | 763 | 764 |
| Genomic region | 19 | 529 | 530 | 765 | 766 |
| PRDM6 | 20 | 531 | 532 | 767 | 768 |
| RAP2B | 21 | 533 | 534 | 769 | 770 |
| NR2E1 | 22 | 535 | 536 | 771 | 772 |
| PCDH7 | 23 | 537 | 538 | 773 | 774 |
| DKK3 | 24 | 539 | 540 | 775 | 776 |
| RTTN | 25 | 541 | 542 | 777 | 778 |
| Genomic region | 26 | 543 | 544 | 779 | 780 |
| GIRK2 | 27 | 545 | 546 | 781 | 782 |
| Genomic region | 28 | 547 | 548 | 783 | 784 |
| Genomic region | 29 | 549 | 550 | 785 | 786 |
| ARL7 | 30 | 551 | 552 | 787 | 788 |
| Genomic region | 31 | 553 | 554 | 789 | 790 |
| THH | 32 | 555 | 556 | 791 | 792 |
| SNAP25 | 33 | 557 | 558 | 793 | 794 |
| HOXB13 | 34 | 559 | 560 | 795 | 796 |
| Genomic region | 35 | 561 | 562 | 797 | 798 |
| Genomic region | 36 | 563 | 564 | 799 | 800 |
| MGC10561 | 37 | 565 | 566 | 801 | 802 |
| LMX1A | 38 | 567 | 568 | 803 | 804 |
| SENP3 | 39 | 569 | 570 | 805 | 806 |
| GS1 | 40 | 571 | 572 | 807 | 808 |
| TITF1 | 41 | 573 | 574 | 809 | 810 |
| SEQ ID NO:42 | 42 | 575 | 576 | 811 | 812 |
| DDX51 | 43 | 577 | 578 | 813 | 814 |
| Q8NAN2 | 44 | 579 | 580 | 815 | 816 |
| Genomic region | 45 | 581 | 582 | 817 | 818 |
| Genomic region | 46 | 583 | 584 | 819 | 820 |
| 060279 | 47 | 585 | 586 | 821 | 822 |
| Genomic region | 48 | 587 | 588 | 823 | 824 |
| KOX7 | 49 | 589 | 590 | 825 | 826 |
| BCL11B | 50 | 591 | 592 | 827 | 828 |
| Genomic region | 51 | 593 | 594 | 829 | 830 |
| MGC34831 | 52 | 595 | 596 | 831 | 832 |

(continued)

| Gene | Genomic SEQ ID NO: | Sense methylated converted SEQ ID NO: | Antisense methylated converted SEQ ID NO: | Sense unmethylated converted ID NO: | Antisense unmethylated SEQconverted SEQ ID NO: |
|---|---|---|---|---|---|
| COL5A1 | 53 | 597 | 598 | 833 | 834 |
| Genomic region | 54 | 599 | 600 | 835 | 836 |
| PDLIM1 | 55 | 601 | 602 | 837 | 838 |
| BRCA2 | 56 | 603 | 604 | 839 | 840 |
| CDKN2A | 57 | 605 | 606 | 841 | 842 |
| EYA4 | 58 | 607 | 608 | 843 | 844 |
| GSTP1 | 59 | 609 | 610 | 845 | 846 |
| SYK | 60 | 611 | 612 | 847 | 848 |
| AR | 61 | 613 | 614 | 849 | 850 |
| PLAU | 62 | 615 | 616 | 851 | 852 |
| LEF1 | 63 | 617 | 618 | 853 | 854 |
| ALX4 | 64 | 619 | 620 | 855 | 856 |
| APC | 65 | 621 | 622 | 857 | 858 |
| BRCA1 | 66 | 623 | 624 | 859 | 860 |
| CDKN1A | 67 | 625 | 626 | 861 | 862 |
| SERPINB5 | 68 | 627 | 628 | 863 | 864 |
| SFN | 69 | 629 | 630 | 865 | 866 |
| CDH13 | 70 | 631 | 632 | 867 | 868 |
| CASP8 | 71 | 633 | 634 | 869 | 870 |
| PRSS8 | 72 | 635 | 636 | 871 | 872 |
| SNCG | 73 | 637 | 638 | 873 | 874 |
| IGSF4 | 74 | 639 | 640 | 875 | 876 |
| ESR1 | 75 | 641 | 642 | 877 | 878 |
| FHIT | 76 | 643 | 644 | 879 | 880 |
| FABP3 | 77 | 645 | 646 | 881 | 882 |
| HOXA5 | 78 | 647 | 648 | 883 | 884 |
| CDH1 | 79 | 649 | 650 | 885 | 886 |
| TP53 | 80 | 651 | 652 | 887 | 888 |
| THBS1 | 81 | 653 | 654 | 889 | 890 |
| APAF1 | 82 | 655 | 656 | 891 | 892 |
| PGR | 83 | 657 | 658 | 893 | 894 |
| TMS1/ASC | 84 | 659 | 660 | 895 | 896 |
| HIC1 | 85 | 661 | 662 | 897 | 898 |
| TP73 | 86 | 663 | 664 | 899 | 900 |
| CLDN7 | 87 | 665 | 666 | 901 | 902 |
| RARB | 88 | 667 | 668 | 903 | 904 |

(continued)

| Gene | Genomic SEQ ID NO: | Sense methylated converted SEQ ID NO: | Antisense methylated converted SEQ ID NO: | Sense unmethylated converted ID NO: | Antisense unmethylated SEQconverted SEQ ID NO: |
|---|---|---|---|---|---|
| MLH1 | 89 | 669 | 670 | 905 | 906 |
| RASSF1A | 90 | 671 | 672 | 907 | 908 |
| ESR2 | 91 | 673 | 674 | 909 | 910 |
| TERT | 92 | 675 | 676 | 911 | 912 |
| TGFBR2 | 93 | 677 | 678 | 913 | 914 |
| IGFBP7 | 94 | 679 | 680 | 915 | 916 |
| LOT1 | 95 | 681 | 682 | 917 | 918 |
| S100A7 | 96 | 683 | 684 | 919 | 920 |
| ARH1/NOEY2 | 97 | 685 | 686 | 921 | 922 |
| DAPK1 | 98 | 687 | 688 | 923 | 924 |
| IL6 | 99 | 689 | 690 | 925 | 926 |
| TWIST | 100 | 691 | 692 | 927 | 928 |
| MCT1 | 101 | 693 | 694 | 929 | 930 |
| SASH1 | 102 | 695 | 696 | 931 | 932 |
| TIMP3 | 103 | 697 | 698 | 933 | 934 |
| CCND2 | 104 | 699 | 700 | 935 | 936 |
| SOD2 | 105 | 701 | 702 | 937 | 938 |
| THRB | 106 | 703 | 704 | 939 | 940 |
| NME1 | 107 | 705 | 706 | 941 | 942 |
| RARA | 108 | 707 | 708 | 943 | 944 |
| STAT1 | 109 | 709 | 710 | 945 | 946 |
| TPM1 | 110 | 711 | 712 | 947 | 948 |
| GJB2 | 111 | 713 | 714 | 949 | 950 |
| SLIT2 | 112 | 715 | 716 | 951 | 952 |
| HS3ST2 | 113 | 717 | 718 | 953 | 954 |
| PRDM2 | 114 | 719 | 720 | 955 | 956 |
| SCGB3A1 | 115 | 721 | 722 | 957 | 958 |
| SLC19A1 | 116 | 723 | 724 | 959 | 960 |
| Genomic region | 117 | 725 | 726 | 961 | 962 |
| GPC3 | 118 | 727 | 728 | 963 | 964 |

Table 4: Breast cancer vs. all other controls Chip 1

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| PRDM2 | SEQ ID NO:114 | 0,781 | 0,798 | 0,754 | 3,34E-39 | 2,04E-37 | 2,04E-37 | 4 | 3 |
| PLAU | SEQ ID NO:62 | 0,769 | 0,781 | 0,751 | 3,87E-35 | 2,36E-33 | 1,18E-33 | 4 | 4 |
| GSTP1 | SEQ ID NO:59 | 0,733 | 0,627 | 0,894 | 2,85E-32 | 1,74E-30 | 5,79E-31 | 4 | 4 |
| SLIT2 | SEQ ID NO:112 | 0,756 | 0,779 | 0,72 | 9,67E-32 | 5,90E-30 | 1,47E-30 | 4 | 4 |
| CCND2 | SEQ ID NO:104 | 0,732 | 0,724 | 0,745 | 4,10E-27 | 2,50E-25 | 5,00E-26 | 6 | 4 |
| HOXA5 | SEQ ID NO:78 | 0,689 | 0,676 | 0,71 | 5,15E-24 | 3,14E-22 | 5,23E-23 | 4 | 2 |
| RASSF1A | SEQ ID NO:90 | 0,727 | 0,702 | 0,766 | 7,95E-24 | 4,85E-22 | 6,93E-23 | 3 | 3 |
| HS3ST2 | SEQ ID NO:113 | 0,739 | 0,825 | 0,609 | 1,62E-22 | 9,90E-21 | 1,24E-21 | 2 | 2 |
| ARH1/NOEY2 | SEQ ID NO:97 | 0,737 | 0,685 | 0,815 | 8,93E-20 | 5,45E-18 | 6,06E-19 | 5 | 5 |
| SCGB3A1 | SEQ ID NO:115 | 0,706 | 0,711 | 0,697 | 1,02E-18 | 6,19E-17 | 6,19E-18 | 4 | 1 |
| THBS1 | SEQ ID NO:81 | 0,722 | 0,829 | 0,56 | 1,41E-18 | 8,59E-17 | 7,81E-18 | 4 | 3 |
| TIMP3 | SEQ ID NO:103 | 0,667 | 0,623 | 0,735 | 3,62E-18 | 2,21E-16 | 1,84E-17 | 4 | 4 |
| IGSF4 | SEQ ID NO:74 | 0,696 | 0,713 | 0,669 | 3,23E-17 | 1,97E-15 | 1,52E-16 | 4 | 2 |
| CDKN1A | SEQ ID NO:67 | 0,67 | 0,748 | 0,551 | 8,09E-17 | 4,93E-15 | 3,52E-16 | 4 | 1 |
| IGFBP7 | SEQ ID NO:94 | 0,681 | 0,746 | 0,582 | 1,67E-16 | 1,02E-14 | 6,78E-16 | 5 | 3 |
| SYK | SEQ ID NO:60 | 0,685 | 0,678 | 0,694 | 5,57E-16 | 3,40E-14 | 2,12E-15 | 4 | 3 |
| HIC1 | SEQ ID NO:85 | 0,645 | 0,707 | 0,55 | 6,28E-16 | 3,83E-14 | 2,25E-15 | 5 | 4 |
| TMS1/ASC | SEQ ID NO:84 | 0,673 | 0,765 | 0,533 | 2,40E-15 | 1,47E-13 | 8,15E-15 | 4 | 1 |
| TWIST | SEQ ID NO:100 | 0,659 | 0,771 | 0,487 | 3,17E-15 | 1,93E-13 | 9,99E-15 | 4 | 3 |
| APAF1 | SEQ ID NO:82 | 0,656 | 0,65 | 0,665 | 3,27E-15 | 2,00E-13 | 9,99E-15 | 5 | 2 |
| THRB | SEQ ID NO:106 | 0,68 | 0,721 | 0,619 | 6,19E-15 | 3,78E-13 | 1,80E-14 | 4 | 1 |
| CDH13 | SEQ ID NO:70 | 0,685 | 0,676 | 0,698 | 1,20E-14 | 7,33E-13 | 3,33E-14 | 4 | 3 |
| GJB2 | SEQ ID NO:111 | 0,626 | 0,537 | 0,761 | 2,06E-14 | 1,26E-12 | 5,47E-14 | 4 | 1 |
| ALX4 | SEQ ID NO:64 | 0,685 | 0,833 | 0,46 | 2,29E-14 | 1,40E-12 | 5,82E-14 | 4 | 3 |
| SERPINB5 | SEQ ID NO:68 | 0,716 | 0,87 | 0,481 | 7,91E-14 | 4,82E-12 | 1,93E-13 | 3 | 3 |

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| FABP3 | SEQ ID NO:77 | 0,658 | 0,603 | 0,742 | 8,69E-13 | 5,30E-11 | 2,04E-12 | 4 | 3 |
| DAPK1 | SEQ ID NO:98 | 0,65 | 0,764 | 0,478 | 2,40E-11 | 1,47E-09 | 5,43E-11 | 2 | 1 |
| FHIT | SEQ ID NO:76 | 0,63 | 0,6 | 0,675 | 5,00E-11 | 3,05E-09 | 1,09E-10 | 3 | 1 |
| MCT1 | SEQ ID NO:101 | 0,667 | 0,743 | 0,552 | 1,37E-10 | 8,33E-09 | 2,87E-10 | 4 | 3 |
| APC | SEQ ID NO:65 | 0,595 | 0,431 | 0,844 | 6,33E-10 | 3,86E-08 | 1,29E-09 | 4 | 4 |
| CDKN2A | SEQ ID NO:57 | 0,614 | 0,479 | 0,819 | 6,73E-10 | 4,11E-08 | 1,32E-09 | 3 | 3 |
| EYA4 | SEQ ID NO:58 | 0,627 | 0,803 | 0,358 | 2,35E-09 | 1,43E-07 | 4,48E-09 | 4 | 1 |
| S100A7 | SEQ ID NO:96 | 0,658 | 0,627 | 0,704 | 5,32E-09 | 3,25E-07 | 9,84E-09 | 3 | 2 |
| GPC3 | SEQ ID NO:118 | 0,623 | 0,653 | 0,578 | 1,17E-08 | 7,11 E-07 | 2,09E-08 | 4 | 1 |
| SLC19A1 | SEQ ID NO:116 | 0,603 | 0,598 | 0,61 | 2,17E-08 | 1,32E-06 | 3,78E-08 | 3 | 2 |
| SOD2 | SEQ ID NO: 105 | 0,632 | 0,657 | 0,592 | 4,55E-08 | 2,77E-06 | 7,71E-08 | 4 | 2 |
| ESR2 | SEQ ID NO:91 | 0,544 | 0,337 | 0,857 | 6,27E-08 | 3,82E-06 | 1,03E-07 | 4 | 1 |
| LOT1 | SEQ ID NO:95 | 0,605 | 0,594 | 0,621 | 4,53E-07 | 2,76E-05 | 7,28E-07 | 4 | 2 |
| SASH1 | SEQ ID NO:102 | 0,613 | 0,619 | 0,605 | 4,76E-07 | 2,90E-05 | 7,45E-07 | 4 | 2 |
| TGFBR2 | SEQ ID NO:93 | 0,609 | 0,554 | 0,692 | 4,89E-07 | 2,98E-05 | 7,46E-07 | 4 | 1 |
| CLDN7 | SEQ ID NO:87 | 0,622 | 0,716 | 0,48 | 1,83E-06 | 1,11E-04 | 2,72E-06 | 4 | 1 |
| RARA | SEQ ID NO:108 | 0,564 | 0,483 | 0,686 | 5,30E-06 | 3,23E-04 | 7,70E-06 | 4 | 1 |
| IL6 | SEQ ID NO:99 | 0,601 | 0,586 | 0,624 | 1,47E-05 | 8,98E-04 | 2,09E-05 | 4 | 0 |
| PRSS8 | SEQ ID NO:72 | 0,558 | 0,595 | 0,502 | 3,66E-05 | 2,23E-03 | 5,07E-05 | 4 | 0 |
| CASP8 | SEQ ID NO:71 | 0,579 | 0,53 | 0,653 | 3,98E-05 | 2,43E-03 | 5,40E-05 | 4 | 1 |
| SFN | SEQ ID NO:69 | 0,599 | 0,677 | 0,479 | 4,24E-05 | 2,58E-03 | 5,62E-05 | 4 | 1 |
| RARB | SEQ ID NO:88 | 0,549 | 0,474 | 0,662 | 4,61E-05 | 2,81E-03 | 5,98E-05 | 4 | 1 |
| NME1 | SEQ ID NO:107 | 0,59 | 0,635 | 0,522 | 1,46E-04 | 8,91E-03 | 1,86E-04 | 4 | 1 |
| ELK1 | SEQ ID NO:2 | 0,596 | 0,677 | 0,473 | 1,88E-04 | 1,15E-02 | 2,34E-04 | 5 | 0 |
| PGR | SEQ ID NO:83 | 0,592 | 0,63 | 0,532 | 7,29E-04 | 4,44E-02 | 8,89E-04 | 4 | 0 |

(continued)

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| BRCA1 | SEQ ID NO:66 | 0,543 | 0,468 | 0,658 | 4,79E-03 | 2,92E-01 | 5,72E-03 | 4 | 0 |
| ESR1 | SEQ ID NO:75 | 0,606 | 0,654 | 0,533 | 6,06E-03 | 3,69E-01 | 7,10E-03 | 3 | 1 |
| STAT1 | SEQ ID NO:109 | 0,583 | 0,646 | 0,488 | 9,12E-03 | 5,56E-01 | 1,05E-02 | 4 | 0 |
| SNCG | SEQ ID NO:73 | 0,566 | 0,676 | 0,398 | 2,04E-02 | 1,00E+00 | 2,31E-02 | 4 | 0 |
| MLH1 | SEQ ID NO:89 | 0,53 | 0,414 | 0,706 | 2,60E-02 | 1,00E+00 | 2,88E-02 | 4 | 0 |
| BRCA2 | SEQ ID NO:56 | 0,565 | 0,567 | 0,562 | 3,43E-02 | 1,00E+00 | 3,74E-02 | 5 | 0 |
| TP73 | SEQ ID NO:86 | 0,534 | 0,496 | 0,592 | 4,43E-02 | 1,00E+00 | 4,74E-02 | 4 | 0 |
| TERT | SEQ ID NO:92 | 0,537 | 0,5 | 0,592 | 1,16E-01 | 1,00E+00 | 1,22E-01 | 4 | 0 |
| TP53 | SEQ ID NO:80 | 0,513 | 0,44 | 0,625 | 2,09E-01 | 1,00E+00 | 2,16E-01 | 4 | 0 |
| TPM1 | SEQ ID NO:110 | 0,518 | 0,405 | 0,689 | 2,35E-01 | 1,00E+00 | 2,39E-01 | 4 | 0 |
| CDH1 | SEQ ID NO:79 | 0,482 | 0,51 | 0,44 | 6,17E-01 | 1,00E+00 | 6,17E-01 | 5 | 0 |

Table 5: Breast cancer vs. all other controls Chip 2

| SEQ ID NO: | Accuracy | Sensitivity | Specificity | p-value | P-value (Bonferroni) | P-value FDR | n Oligos | n Significant oligos |
|---|---|---|---|---|---|---|---|---|
| SEQ ID NO:12 | 0,773 | 0,764 | 0,786 | 1,02E-37 | 5,50E-36 | 5,50E-36 | 4 | 3 |
| SEQ ID NO:6 | 0,777 | 0,821 | 0,712 | 1,01E-32 | 5,47E-31 | 2,73E-31 | 6 | 6 |
| SEQ ID NO:3 | 0,754 | 0,73 | 0,789 | 3,11E-32 | 1,68E-30 | 5,60E-31 | 4 | 4 |
| SEQ ID NO:18 | 0,722 | 0,7 | 0,754 | 1,43E-28 | 7,72E-27 | 1,93E-27 | 5 | 5 |
| SEQ ID NO:7 | 0,753 | 0,824 | 0,65 | 2,18E-26 | 1,18E-24 | 2,36E-25 | 5 | 3 |
| SEQ ID NO:41 | 0,74 | 0,797 | 0,658 | 3,97E-26 | 2,15E-24 | 3,58E-25 | 5 | 4 |
| SEQ ID NO:22 | 0,726 | 0,677 | 0,796 | 1,87E-25 | 1,01E-23 | 1,44E-24 | 8 | 8 |
| SEQ ID NO:46 | 0,727 | 0,74 | 0,707 | 2,74E-25 | 1,48E-23 | 1,85E-24 | 4 | 4 |
| SEQ ID NO:13 | 0,681 | 0,622 | 0,767 | 6,60E-22 | 3,56E-20 | 3,96E-21 | 4 | 3 |
| SEQ ID NO:31 | 0,69 | 0,647 | 0,753 | 1,06E-21 | 5,74E-20 | 5,74E-21 | 5 | 5 |
| SEQ ID NO:34 | 0,695 | 0,684 | 0,711 | 4,06E-21 | 2,19E-19 | 1,99E-20 | 5 | 2 |
| SEQ ID NO:27 | 0,714 | 0,702 | 0,733 | 1,58E-20 | 8,52E-19 | 7,10E-20 | 4 | 3 |
| SEQ ID NO:10 | 0,697 | 0,816 | 0,521 | 3,12E-20 | 1,69E-18 | 1,30E-19 | 9 | 8 |
| SEQ ID NO:38 | 0,69 | 0,818 | 0,502 | 9,42E-20 | 5,09E-18 | 3,63E-19 | 5 | 3 |
| SEQ ID NO:47 | 0,684 | 0,745 | 0,593 | 1,06E-19 | 5,74E-18 | 3,83E-19 | 4 | 3 |
| SEQ ID NO:117 | 0,703 | 0,716 | 0,683 | 1,39E-19 | 7,49E-18 | 4,68E-19 | 4 | 4 |
| SEQ ID NO:48 | 0,723 | 0,746 | 0,69 | 1,47E-19 | 7,95E-18 | 4,68E-19 | 4 | 4 |
| SEQ ID NO:30 | 0,695 | 0,586 | 0,855 | 7,60E-19 | 4,10E-17 | 2,28E-18 | 4 | 4 |
| SEQ ID NO:4 | 0,692 | 0,641 | 0,766 | 1,66E-18 | 8,95E-17 | 4,71E-18 | 4 | 3 |
| SEQ ID NO:39 | 0,678 | 0,695 | 0,654 | 1,70E-17 | 9,16E-16 | 4,58E-17 | 4 | 4 |
| SEQ ID NO:1 | 0,683 | 0,741 | 0,598 | 1,83E-17 | 9,88E-16 | 4,70E-17 | 5 | 4 |

(continued)

| SEQ ID NO: | Accuracy | Sensitivity | Specificity | p-value | P-value (Bonferroni) | P-value FDR | n Oligos | n Significant oligos |
|---|---|---|---|---|---|---|---|---|
| SEQ ID NO:35 | 0,686 | 0,78 | 0,549 | 8,08E-16 | 4,37E-14 | 1,98E-15 | 4 | 4 |
| SEQ ID NO:43 | 0,697 | 0,687 | 0,711 | 1,66E-15 | 8,97E-14 | 3,90E-15 | 4 | 1 |
| SEQ ID NO:54 | 0,659 | 0,597 | 0,75 | 1,15E-14 | 6,21E-13 | 2,59E-14 | 5 | 2 |
| SEQ ID NO:25 | 0,657 | 0,648 | 0,67 | 3,14E-14 | 1,70E-12 | 6,79E-14 | 5 | 2 |
| SEQ ID NO:42 | 0,667 | 0,648 | 0,695 | 6,97E-14 | 3,76E-12 | 1,45E-13 | 4 | 2 |
| SEQ ID NO:29 | 0,684 | 0,727 | 0,621 | 1,53E-13 | 8,25E-12 | 3,05E-13 | 5 | 3 |
| SEQ ID NO:2 | 0,642 | 0,704 | 0,551 | 1,74E-13 | 9,38E-12 | 3,35E-13 | 4 | 1 |
| SEQ ID NO:32 | 0,631 | 0,623 | 0,643 | 4,22E-12 | 2,28E-10 | 7,85E-12 | 3 | 1 |
| SEQ ID NO:23 | 0,64 | 0,609 | 0,686 | 6,07E-12 | 3,28E-10 | 1,07E-11 | 3 | |
| SEQ ID NO:11 | 0,652 | 0,64 | 0,67 | 6,12E-12 | 3,30E-10 | 1,07E-11 | 4 | 4 |
| SEQ ID NO:16 | 0,631 | 0,614 | 0,655 | 8,28E-11 | 4,47E-09 | 1,40E-10 | 5 | 2 |
| SEQ ID NO:17 | 0,643 | 0,652 | 0,631 | 1,68E-10 | 9,09E-09 | 2,75E-10 | 5 | 1 |
| SEQ ID NO:28 | 0,639 | 0,613 | 0,677 | 3,66E-10 | 1,97E-08 | 5,81E-10 | 7 | 2 |
| SEQ ID NO:51 | 0,615 | 0,524 | 0,749 | 8,46E-10 | 4,57E-08 | 1,31E-09 | 4 | 1 |
| SEQ ID NO:19 | 0,595 | 0,539 | 0,677 | 2,07E-09 | 1,12E-07 | 3,11E-09 | 4 | 1 |
| SEQ ID NO:20 | 0,626 | 0,806 | 0,363 | 2,96E-09 | 1,60E-07 | 4,32E-09 | 4 | 1 |
| SEQ ID NO:14 | 0,607 | 0,496 | 0,769 | 8,74E-09 | 4,72E-07 | 1,24E-08 | 3 | 1 |
| SEQ ID NO:24 | 0,614 | 0,505 | 0,774 | 6,78E-08 | 3,66E-06 | 9,39E-08 | 5 | 3 |
| SEQ ID NO:33 | 0,598 | 0,468 | 0,789 | 1,33E-07 | 7,20E-06 | 1,80E-07 | 3 | 0 |
| SEQ ID NO:50 | 0,599 | 0,5 | 0,743 | 2,21E-07 | 1,19E-05 | 2,90E-07 | 5 | 2 |
| SEQ ID NO:26 | 0,644 | 0,75 | 0,489 | 1,03E-06 | 5,57E-05 | 1,33E-06 | 3 | 1 |

(continued)

| SEQ ID NO: | Accuracy | Sensitivity | Specificity | p-value | P-value (Bonferroni) | P-value FDR | n Oligos | n Significant oligos |
|---|---|---|---|---|---|---|---|---|
| SEQ ID NO:15 | 0,597 | 0,668 | 0,492 | 1,15E-05 | 6,21E-04 | 1,44E-05 | 5 | 0 |
| SEQ ID NO:40 | 0,6 | 0,569 | 0,646 | 1,78E-05 | 9,59E-04 | 2,18E-05 | 5 | 1 |
| SEQ ID NO:37 | 0,582 | 0,635 | 0,504 | 2,43E-05 | 1,31E-03 | 2,91E-05 | 3 | 1 |
| SEQ ID NO:9 | 0,603 | 0,538 | 0,698 | 3,25E-05 | 1,75E-03 | 3,81E-05 | 5 | 1 |
| SEQ ID NO:5 | 0,592 | 0,613 | 0,561 | 5,90E-05 | 3,18E-03 | 6,77E-05 | 5 | 3 |
| SEQ ID NO:8 | 0,64 | 0,863 | 0,315 | 1,41E-04 | 7,61E-03 | 1,59E-04 | 5 | 0 |
| SEQ ID NO:52 | 0,606 | 0,695 | 0,475 | 1,72E-03 | 9,31E-02 | 1,90E-03 | 4 | 0 |
| SEQ ID NO:21 | 0,57 | 0,516 | 0,649 | 1,14E-02 | 6,14E-01 | 1,23E-02 | 4 | 0 |
| SEQ ID NO:36 | 0,521 | 0,54 | 0,493 | 2,59E-02 | 1,00E+00 | 2,74E-02 | 4 | 0 |
| SEQ ID NO:55 | 0,514 | 0,332 | 0,78 | 2,98E-02 | 1,00E+00 | 3,09E-02 | 5 | 0 |
| SEQ ID NO:45 | 0,553 | 0,65 | 0,411 | 6,73E-02 | 1,00E+00 | 6,86E-02 | 4 | 0 |
| SEQ ID NO:44 | 0,496 | 0,402 | 0,632 | 5,14E-01 | 1,00E+00 | 5,14E-01 | 2 | 0 |

Table 6: Breast cancer vs. other cancer Chip 1

| Gene Name | SEQ NO: | ID Accuracy | Sensitivity | Specificity | p-value | P-value (Bonferroni) | P-value FDR | n Oligos | n Significant oligos |
|---|---|---|---|---|---|---|---|---|---|
| PRDM2 | SEQ ID NO: 114 | 0,852 | 0,897 | 0,687 | 8,85E-26 | 5,40E-24 | 5,40E-24 | 4 | 1 |
| GSTP1 | SEQ ID NO:59 | 0,686 | 0,638 | 0,862 | 2,40E-14 | 1,47E-12 | 7,34E-13 | 4 | 4 |
| ALX4 | SEQ ID NO:64 | 0,77 | 0,821 | 0,583 | 4,88E-14 | 2,98E-12 | 9,92E-13 | 4 | 3 |
| HOXA5 | SEQ ID NO:78 | 0,689 | 0,683 | 0,708 | 8,11E-13 | 4,95E-11 | 1,24E-11 | 4 | 2 |
| PLAU | SEQ ID NO:62 | 0,693 | 0,707 | 0,642 | 2,29E-12 | 1,40E-10 | 2,80E-11 | 4 | 3 |
| RASSF1A | SEQ ID NO:90 | 0,714 | 0,721 | 0,689 | 1,10E-10 | 6,68E-09 | 1,11E-09 | 3 | 3 |
| IGSF4 | SEQ ID NO:74 | 0,716 | 0,735 | 0,644 | 2,13E-10 | 1,30E-08 | 1,86E-09 | 4 | 1 |
| SLIT2 | SEQ ID NO:112 | 0,674 | 0,726 | 0,479 | 5,43E-10 | 3,31E-08 | 4,14E-09 | 4 | 2 |
| DAPK1 | SEQ ID NO:98 | 0,713 | 0,757 | 0,549 | 1,05E-09 | 6,41E-08 | 7,13E-09 | 2 | 1 |
| CDKN1A | SEQ ID NO:67 | 0,693 | 0,725 | 0,576 | 1,34E-09 | 8,17E-08 | 8,17E-09 | 4 | 1 |
| IGFBP7 | SEQ ID NO:94 | 0,7 | 0,702 | 0,693 | 2,79E-09 | 1,70E-07 | 1,55E-08 | 5 | 3 |
| THBS1 | SEQ ID NO:81 | 0,735 | 0,791 | 0,528 | 4,76E-09 | 2,90E-07 | 2,42E-08 | 4 | 3 |
| CCND2 | SEQ ID NO:104 | 0,688 | 0,707 | 0,617 | 3,74E-08 | 2,28E-06 | 1,75E-07 | 6 | 2 |
| APAF1 | SEQ ID NO:82 | 0,631 | 0,632 | 0,628 | 1,20E-07 | 7,34E-06 | 5,25E-07 | 5 | 2 |
| EYA4 | SEQ ID NO:58 | 0,719 | 0,748 | 0,613 | 1,82E-07 | 1,11E-05 | 7,40E-07 | 4 | 2 |
| SCGB3A1 | SEQ ID NO: 115 | 0,649 | 0,671 | 0,568 | 2,56E-07 | 1,56E-05 | 9,25E-07 | 4 | 1 |
| SYK | SEQ ID NO:60 | 0,647 | 0,653 | 0,625 | 2,65E-07 | 1,62E-05 | 9,25E-07 | 4 | 2 |
| HIC1 | SEQ ID NO:85 | 0,654 | 0,69 | 0,524 | 2,73E-07 | 1,67E-05 | 9,25E-07 | 5 | 2 |
| CDH13 | SEQ ID NO:70 | 0,573 | 0,503 | 0,831 | 3,52E-07 | 2,15E-05 | 1,13E-06 | 4 | 0 |
| TIMP3 | SEQ ID NO:103 | 0,619 | 0,617 | 0,627 | 1,45E-06 | 8,84E-05 | 4,42E-06 | 4 | 3 |
| LOT1 | SEQ ID NO:95 | 0,632 | 0,626 | 0,652 | 1,57E-06 | 9,56E-05 | 4,55E-06 | 4 | 1 |
| THRB | SEQ ID NO:106 | 0,723 | 0,776 | 0,53 | 1,89E-06 | 1,15E-04 | 5,24E-06 | 4 | 1 |
| TMS1/ASC | SEQ ID NO:84 | 0,702 | 0,781 | 0,411 | 2,77E-06 | 1,69E-04 | 7,34E-06 | 4 | 1 |
| IL6 | SEQ ID NO:99 | 0,654 | 0,669 | 0,596 | 1,69E-05 | 1,03E-03 | 4,30E-05 | 4 | 1 |
| TWIST | SEQ ID NO: 100 | 0,696 | 0,755 | 0,479 | 1,92E-05 | 1,17E-03 | 4,69E-05 | 4 | 2 |

(continued)

| Gene Name | SEQ NO: | ID Accuracy | Sensitivity | Specificity | p-value | P-value (Bonferroni) | P-value FDR | n Oligos | n Significant oligos |
|---|---|---|---|---|---|---|---|---|---|
| CLDN7 | SEQ ID NO:87 | 0,63 | 0,67 | 0,482 | 3,67E-04 | 2,24E-02 | 8,62E-04 | 4 | 0 |
| FHIT | SEQ ID NO:76 | 0,601 | 0,613 | 0,556 | 7,43E-04 | 4,53E-02 | 1,63E-03 | 3 | 0 |
| ARH1/NOEY2 | SEQ ID NO:97 | 0,634 | 0,646 | 0,587 | 7,50E-04 | 4,57E-02 | 1,63E-03 | 5 | 2 |
| PGR | SEQ ID NO:83 | 0,649 | 0,642 | 0,673 | 1,34E-03 | 8,17E-02 | 2,82E-03 | 4 | 0 |
| NME1 | SEQ ID NO:107 | 0,588 | 0,597 | 0,556 | 2,10E-03 | 1,28E-01 | 4,27E-03 | 4 | 1 |
| SERPINB5 | SEQ ID NO:68 | 0,665 | 0,709 | 0,503 | 3,24E-03 | 1,98E-01 | 6,38E-03 | 3 | 0 |
| GPC3 | SEQ ID NO:118 | 0,619 | 0,651 | 0,503 | 3,42E-03 | 2,09E-01 | 6,52E-03 | 4 | 0 |
| ESR2 | SEQ ID NO:91 | 0,395 | 0,267 | 0,869 | 3,53E-03 | 2,15E-01 | 6,53E-03 | 4 | 0 |
| MCT1 | SEQ ID NO:101 | 0,713 | 0,818 | 0,321 | 9,75E-03 | 5,95E-01 | 1,75E-02 | 4 | 0 |
| HS3ST2 | SEQ ID NO:113 | 0,749 | 0,892 | 0,22 | 1,07E-02 | 6,54E-01 | 1,84E-02 | 2 | 0 |
| GJB2 | SEQ ID NO:111 | 0,543 | 0,516 | 0,642 | 1,08E-02 | 6,62E-01 | 1,84E-02 | 4 | 0 |
| ELK1 | SEQ ID NO:2 | 0,603 | 0,634 | 0,49 | 2,21E-02 | 1,00E+00 | 3,61E-02 | 5 | 0 |
| STAT1 | SEQ ID NO:109 | 0,618 | 0,651 | 0,496 | 2,25E-02 | 1,00E+00 | 3,61E-02 | 4 | 0 |
| TGFBR2 | SEQ ID NO:93 | 0,608 | 0,669 | 0,382 | 2,34E-02 | 1,00E+00 | 3,66E-02 | 4 | 0 |
| TERT | SEQ ID NO:92 | 0,552 | 0,549 | 0,566 | 2,41E-02 | 1,00E+00 | 3,67E-02 | 4 | 0 |
| PRSS8 | SEQ ID NO:72 | 0,578 | 0,592 | 0,525 | 4,66E-02 | 1,00E+00 | 6,93E-02 | 4 | 0 |
| SOD2 | SEQ ID NO:105 | 0,587 | 0,602 | 0,534 | 6,76E-02 | 1,00E+00 | 9,82E-02 | 4 | 0 |
| S100A7 | SEQ ID NO:96 | 0,568 | 0,594 | 0,47 | 7,29E-02 | 1,00E+00 | 1,03E-01 | 3 | 0 |
| FABP3 | SEQ ID NO:77 | 0,504 | 0,466 | 0,648 | 7,78E-02 | 1,00E+00 | 1,08E-01 | 4 | 0 |
| SFN | SEQ ID NO:69 | 0,597 | 0,637 | 0,448 | 8,88E-02 | 1,00E+00 | 1,18E-01 | 4 | 0 |
| RARA | SEQ ID NO:108 | 0,442 | 0,369 | 0,711 | 8,97E-02 | 1,00E+00 | 1,18E-01 | 4 | 0 |
| CDH1 | SEQ ID NO:79 | 0,539 | 0,559 | 0,463 | 9,06E-02 | 1,00E+00 | 1,18E-01 | 5 | 0 |
| APC | SEQ ID NO:65 | 0,578 | 0,605 | 0,476 | 1,21E-01 | 1,00E+00 | 1,54E-01 | 4 | 0 |
| BRCA2 | SEQ ID NO:56 | 0,577 | 0,585 | 0,546 | 1,26E-01 | 1,00E+00 | 1,57E-01 | 5 | 0 |
| SASH1 | SEQ ID NO:102 | 0,548 | 0,553 | 0,53 | 1,58E-01 | 1,00E+00 | 1,93E-01 | 4 | 0 |

(continued)

| Gene Name | SEQ NO: | ID Accuracy | Sensitivity | Specificity | p-value | P-value (Bonferroni) | P-value FDR | n Oligos | n Significant oligos |
|---|---|---|---|---|---|---|---|---|---|
| CASP8 | SEQ ID NO:71 | 0,534 | 0,533 | 0,534 | 1,63E-01 | 1,00E+00 | 1,95E-01 | 4 | 0 |
| CDKN2A | SEQ ID NO:57 | 0,487 | 0,448 | 0,632 | 1,74E-01 | 1,00E+00 | 2,04E-01 | 3 | 0 |
| ESR1 | SEQ ID NO:75 | 0,602 | 0,654 | 0,408 | 2,14E-01 | 1,00E+00 | 2,47E-01 | 3 | 0 |
| RARB | SEQ ID NO:88 | 0,577 | 0,622 | 0,413 | 2,61E-01 | 1,00E+00 | 2,95E-01 | 4 | 0 |
| SNCG | SEQ ID NO:73 | 0,573 | 0,635 | 0,342 | 3,53E-01 | 1,00E+00 | 3,92E-01 | 4 | 0 |
| BRCA1 | SEQ ID NO:66 | 0,421 | 0,362 | 0,642 | 3,69E-01 | 1,00E+00 | 4,02E-01 | 4 | 0 |
| SLC19A1 | SEQ ID NO:116 | 0,455 | 0,437 | 0,523 | 4,30E-01 | 1,00E+00 | 4,60E-01 | 3 | 0 |
| TP73 | SEQ ID NO:86 | 0,484 | 0,463 | 0,559 | 5,24E-01 | 1,00E+00 | 5,51E-01 | 4 | 0 |
| TP53 | SEQ ID NO:80 | 0,472 | 0,463 | 0,503 | 6,77E-01 | 1,00E+00 | 7,00E-01 | 4 | 0 |
| MLH1 | SEQ ID NO:89 | 0,56 | 0,621 | 0,332 | 7,29E-01 | 1,00E+00 | 7,42E-01 | 4 | 0 |
| TPM1 | SEQ ID NO:110 | 0,514 | 0,554 | 0,369 | 8,30E-01 | 1,00E+00 | 8,30E-01 | 4 | 0 |

Table 7: Breast cancer vs. other cancer Chip 2

| SEQ ID NO: | Accuracy | Sensitivity | Specificity | P-value | P-value (Bonferroni) | P-value FDR | n Oligos | n Significant oligos |
|---|---|---|---|---|---|---|---|---|
| SEQ ID NO:38 | 0,75 | 0,775 | 0,662 | 1,91E-14 | 1,03E-12 | 1,03E-12 | 5 | 4 |
| SEQ ID NO:35 | 0,73 | 0,771 | 0,592 | 5,32E-13 | 2,87E-11 | 1,44E-11 | 4 | 4 |
| SEQ ID NO:12 | 0,74 | 0,784 | 0,577 | 5,86E-12 | 3,17E-10 | 1,01E-10 | 4 | 3 |
| SEQ ID NO:39 | 0,71 | 0,733 | 0,626 | 7,49E-12 | 4,05E-10 | 1,01E-10 | 4 | 4 |
| SEQ ID NO:10 | 0,79 | 0,886 | 0,455 | 9,63E-11 | 5,20E-09 | 1,04E-09 | 9 | 5 |
| SEQ ID NO:26 | 0,74 | 0,773 | 0,623 | 1,88E-10 | 1,01E-08 | 1,69E-09 | 3 | 3 |
| SEQ ID NO:8 | 0,64 | 0,616 | 0,716 | 2,30E-10 | 1,24E-08 | 1,77E-09 | 5 | 5 |
| SEQ ID NO:22 | 0,69 | 0,692 | 0,681 | 6,56E-10 | 3,54E-08 | 4,43E-09 | 8 | 6 |
| SEQ ID NO:18 | 0,67 | 0,664 | 0,692 | 1,53E-09 | 8,24E-08 | 9,15E-09 | 5 | 2 |
| SEQ ID NO:47 | 0,73 | 0,788 | 0,534 | 4,22E-09 | 2,28E-07 | 2,22E-08 | 4 | 1 |
| SEQ ID NO:5 | 0,73 | 0,745 | 0,677 | 4,53E-09 | 2,44E-07 | 2,22E-08 | 5 | 3 |
| SEQ ID NO:3 | 0,65 | 0,642 | 0,666 | 6,75E-09 | 3,65E-07 | 3,04E-08 | 4 | 2 |
| SEQ ID NO:1 | 0,67 | 0,69 | 0,578 | 1,35E-08 | 7,29E-07 | 5,61E-08 | 5 | 2 |
| SEQ ID NO:6 | 0,68 | 0,726 | 0,523 | 1,25E-06 | 6,76E-05 | 4,83E-06 | 6 | 2 |
| SEQ ID NO:41 | 0,72 | 0,744 | 0,656 | 1,44E-06 | 7,76E-05 | 5,17E-06 | 5 | 1 |
| SEQ ID NO:33 | 0,66 | 0,677 | 0,581 | 3,97E-06 | 2,15E-04 | 1,34E-05 | 3 | 1 |
| SEQ ID NO:13 | 0,59 | 0,573 | 0,64 | 1,19E-05 | 6,44E-04 | 3,79E-05 | 4 | 2 |
| SEQ ID NO:2 | 0,65 | 0,716 | 0,438 | 1,62E-05 | 8,72E-04 | 4,85E-05 | 4 | 0 |
| SEQ ID NO:25 | 0,63 | 0,628 | 0,629 | 1,72E-05 | 9,26E-04 | 4,88E-05 | 5 | 1 |
| SEQ ID NO:54 | 0,61 | 0,578 | 0,704 | 3,32E-05 | 1,79E-03 | 8,95E-05 | 5 | 1 |
| SEQ ID NO:30 | 0,59 | 0,55 | 0,725 | 1,21E-04 | 6,55E-03 | 3,12E-04 | 4 | 4 |

(continued)

| SEQ ID NO: | Accuracy | Sensitivity | Specificity | P-value | P-value (Bonferroni) | P-value FDR | n Oligos | n Significant oligos |
|---|---|---|---|---|---|---|---|---|
| SEQ ID NO:32 | 0,65 | 0,659 | 0,596 | 1,37E-04 | 7,39E-03 | 3,36E-04 | 3 | 1 |
| SEQ ID NO:31 | 0,59 | 0,578 | 0,642 | 1,44E-04 | 7,75E-03 | 3,37E-04 | 5 | 1 |
| SEQ ID NO:23 | 0,6 | 0,6 | 0,614 | 1,89E-04 | 1,02E-02 | 4,26E-04 | 3 | 0 |
| SEQ ID NO:34 | 0,66 | 0,682 | 0,56 | 7,24E-04 | 3,91E-02 | 1,56E-03 | 5 | 0 |
| SEQ ID NO:48 | 0,69 | 0,746 | 0,501 | 9,60E-04 | 5,18E-02 | 1,99E-03 | 4 | 1 |
| SEQ ID NO:27 | 0,62 | 0,631 | 0,563 | 1,38E-03 | 7,44E-02 | 2,76E-03 | 4 | 0 |
| SEQ ID NO:28 | 0,59 | 0,572 | 0,652 | 1,84E-03 | 9,95E-02 | 3,55E-03 | 7 | 1 |
| SEQ ID NO:7 | 0,61 | 0,648 | 0,456 | 2,91E-03 | 1,57E-01 | 5,42E-03 | 5 | 0 |
| SEQ ID NO:42 | 0,63 | 0,676 | 0,449 | 3,05E-03 | 1,65E-01 | 5,49E-03 | 4 | 0 |
| SEQ ID NO:37 | 0,59 | 0,6 | 0,555 | 3,41E-03 | 1,84E-01 | 5,94E-03 | 3 | 0 |
| SEQ ID NO:19 | 0,55 | 0,544 | 0,548 | 6,25E-03 | 3,37E-01 | 1,05E-02 | 4 | 0 |
| SEQ ID NO:46 | 0,54 | 0,526 | 0,592 | 6,73E-03 | 3,63E-01 | 1,10E-02 | 4 | 0 |
| SEQ ID NO:29 | 0,59 | 0,635 | 0,415 | 7,70E-03 | 4,16E-01 | 1,22E-02 | 5 | 0 |
| SEQ ID NO:20 | 0,6 | 0,63 | 0,477 | 7,88E-03 | 4,26E-01 | 1,22E-02 | 4 | 0 |
| SEQ ID NO:17 | 0,61 | 0,635 | 0,523 | 8,17E-03 | 4,41E-01 | 1,23E-02 | 5 | 0 |
| SEQ ID NO:16 | 0,58 | 0,584 | 0,56 | 9,05E-03 | 4,89E-01 | 1,32E-02 | 5 | 0 |
| SEQ ID NO:45 | 0,62 | 0,655 | 0,515 | 1,27E-02 | 6,88E-01 | 1,81E-02 | 4 | 0 |
| SEQ ID NO:4 | 0,55 | 0,523 | 0,632 | 2,94E-02 | 1,00E+00 | 4,07E-02 | 4 | 0 |
| SEQ ID NO:14 | 0,48 | 0,411 | 0,73 | 3,58E-02 | 1,00E+00 | 4,83E-02 | 3 | 0 |
| SEQ ID NO:15 | 0,56 | 0,564 | 0,552 | 3,73E-02 | 1,00E+00 | 4,91E-02 | 5 | 0 |
| SEQ ID NO:43 | 0,54 | 0,525 | 0,581 | 4,70E-02 | 1,00E+00 | 6,04E-02 | 4 | 0 |

(continued)

| SEQ ID NO: | Accuracy | Sensitivity | Specificity | P-value | P-value (Bonferroni) | P-value FDR | n Oligos | n Significant oligos |
|---|---|---|---|---|---|---|---|---|
| SEQ ID NO:40 | 0,54 | 0,564 | 0,467 | 7,94E-02 | 1,00E+00 | 9,97E-02 | 5 | 0 |
| SEQ ID NO:11 | 0,56 | 0,596 | 0,438 | 1,26E-01 | 1,00E+00 | 1,54E-01 | 4 | 0 |
| SEQ ID NO:9 | 0,56 | 0,591 | 0,473 | 1,32E-01 | 1,00E+00 | 1,58E-01 | 5 | 0 |
| SEQ ID NO:36 | 0,54 | 0,558 | 0,471 | 1,42E-01 | 1,00E+00 | 1,66E-01 | 4 | 0 |
| SEQ ID NO:117 | 0,54 | 0,526 | 0,568 | 1,46E-01 | 1,00E+00 | 1,68E-01 | 4 | 0 |
| SEQ ID NO:55 | 0,55 | 0,568 | 0,473 | 3,06E-01 | 1,00E+00 | 3,45E-01 | 5 | 0 |
| SEQ ID NO:51 | 0,55 | 0,586 | 0,408 | 3,77E-01 | 1,00E+00 | 4,15E-01 | 4 | 0 |
| SEQ ID NO:50 | 0,47 | 0,437 | 0,574 | 4,14E-01 | 1,00E+00 | 4,47E-01 | 5 | 0 |
| SEQ ID NO:21 | 0,48 | 0,451 | 0,59 | 4,40E-01 | 1,00E+00 | 4,66E-01 | 4 | 0 |
| SEQ ID NO:24 | 0,48 | 0,421 | 0,663 | 4,94E-01 | 1,00E+00 | 5,13 E-01 | 5 | 0 |
| SEQ ID NO:52 | 0,48 | 0,448 | 0,589 | 5,90E-01 | 1,00E+00 | 6,01E-01 | 4 | 0 |
| SEQ ID NO:44 | 0,56 | 0,618 | 0,344 | 9,16E-01 | 1,00E+00 | 9,16E-01 | 2 | 0 |

Table 8: Breast cancer vs. lymphocytes Chip 1

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| EYA4 | SEQ ID NO:58 | 1 | 1 | 1 | 9,16E-39 | 5,59E-37 | 3,76E-37 | 4 | 4 |
| PRDM2 | SEQ ID NO:114 | 0,984 | 0,985 | 0,971 | 1,23E-38 | 7,52E-37 | 3,76E-37 | 4 | 4 |
| SERPINB5 | SEQ ID NO:68 | 0,986 | 0,984 | 0,996 | 3,96E-37 | 2,42E-35 | 8,05E-36 | 3 | 3 |
| TWIST | SEQ ID NO:100 | 0,962 | 0,962 | 0,964 | 8,80E-34 | 5,37E-32 | 1,34E-32 | 4 | 4 |
| STAT1 | SEQ ID NO: 109 | 0,97 | 0,973 | 0,936 | 9,90E-32 | 6,04E-30 | 1,21E-30 | 4 | 1 |
| ALX4 | SEQ ID NO:64 | 0,949 | 0,953 | 0,911 | 2,21E-31 | 1,35E-29 | 2,24E-30 | 4 | 3 |
| IGFBP7 | SEQ ID NO:94 | 0,941 | 0,941 | 0,939 | 1,24E-28 | 7,58E-27 | 1,08E-27 | 5 | 4 |
| DAPK1 | SEQ ID NO:98 | 0,905 | 0,9 | 0,954 | 3,26E-26 | 1,99E-24 | 2,49E-25 | 2 | 1 |
| THBS1 | SEQ ID NO:81 | 0,93 | 0,934 | 0,889 | 2,02E-25 | 1,23E-23 | 1,37E-24 | 4 | 2 |
| PLAU | SEQ ID NO:62 | 0,923 | 0,927 | 0,882 | 3,43E-24 | 2,09E-22 | 2,09E-23 | 4 | 3 |
| PRSS8 | SEQ ID NO:72 | 0,871 | 0,881 | 0,775 | 2,25E-22 | 1,37E-20 | 1,25E-21 | 4 | 2 |
| S100A7 | SEQ ID NO:96 | 0,886 | 0,879 | 0,95 | 3,83E-21 | 2,34E-19 | 1,95E-20 | 3 | 3 |
| SFN | SEQ ID NO:69 | 0,88 | 0,882 | 0,861 | 9,22E-20 | 5,62E-18 | 4,33E-19 | 4 | 3 |
| NME1 | SEQ ID NO: 107 | 0,807 | 0,802 | 0,854 | 1,53E-18 | 9,34E-17 | 6,67E-18 | 4 | 4 |
| SLIT2 | SEQ ID NO:112 | 0,848 | 0,847 | 0,854 | 1,76E-18 | 1,08E-16 | 7,17E-18 | 4 | 4 |
| CDKN1A | SEQ ID NO:67 | 0,877 | 0,881 | 0,846 | 5,07E-18 | 3,09E-16 | 1,93E-17 | 4 | 1 |
| LOT1 | SEQ ID NO:95 | 0,819 | 0,819 | 0,818 | 8,85E-17 | 5,40E-15 | 3,18E-16 | 4 | 3 |
| HOXA5 | SEQ ID NO:78 | 0,808 | 0,807 | 0,821 | 4,95E-16 | 3,02E-14 | 1,68E-15 | 4 | 2 |
| CCND2 | SEQ ID NO:104 | 0,822 | 0,822 | 0,825 | 7,82E-15 | 4,77E-13 | 2,51E-14 | 6 | 4 |
| IGSF4 | SEQ ID NO:74 | 0,864 | 0,878 | 0,732 | 3,82E-14 | 2,33E-12 | 1,17E-13 | 4 | 1 |
| SCGB3A1 | SEQ ID NO:115 | 0,807 | 0,798 | 0,886 | 4,11E-14 | 2,51E-12 | 1,19E-13 | 4 | 2 |
| RASSF1A | SEQ ID NO:90 | 0,759 | 0,74 | 0,939 | 4,74E-14 | 2,89E-12 | 1,31E-13 | 3 | 3 |
| HIC1 | SEQ ID NO:85 | 0,856 | 0,867 | 0,754 | 5,66E-14 | 3,45E-12 | 1,50E-13 | 5 | 4 |
| GPC3 | SEQ ID NO:118 | 0,771 | 0,768 | 0,793 | 1,27E-13 | 7,76E-12 | 3,23E-13 | 4 | 1 |
| SNCG | SEQ ID NO:73 | 0,793 | 0,781 | 0,914 | 7,47E-13 | 4,56E-11 | 1,82E-12 | 4 | 4 |

(continued)

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| APC | SEQ ID NO:65 | 0,769 | 0,761 | 0,846 | 8,49E-13 | 5,18E-11 | 1,99E-12 | 4 | 3 |
| GSTP1 | SEQ ID NO:59 | 0,747 | 0,738 | 0,832 | 3,71E-12 | 2,26E-10 | 8,38E-12 | 4 | 3 |
| MCT1 | SEQ ID NO:101 | 0,819 | 0,817 | 0,832 | 5,61E-12 | 3,42E-10 | 1,22E-11 | 4 | 2 |
| SLC19A1 | SEQ ID NO:116 | 0,754 | 0,756 | 0,736 | 9,04E-12 | 5,52E-10 | 1,90E-11 | 3 | 2 |
| IL6 | SEQ ID NO:99 | 0,807 | 0,81 | 0,786 | 1,08E-11 | 6,57E-10 | 2,19E-11 | 4 | 1 |
| HS3ST2 | SEQ ID NO:113 | 0,776 | 0,774 | 0,793 | 5,22E-11 | 3,18E-09 | 1,03E-10 | 2 | 2 |
| FABP3 | SEQ ID NO:77 | 0,753 | 0,752 | 0,761 | 1,73E-10 | 1,06E-08 | 3,30E-10 | 4 | 2 |
| SOD2 | SEQ ID NO: 105 | 0,777 | 0,787 | 0,686 | 5,83E-09 | 3,56E-07 | 1,08E-08 | 4 | 1 |
| THRB | SEQ ID NO:106 | 0,797 | 0,824 | 0,539 | 2,75E-08 | 1,67E-06 | 4,93E-08 | 4 | 1 |
| CDH13 | SEQ ID NO:70 | 0,701 | 0,689 | 0,821 | 3,44E-08 | 2,10E-06 | 5,99E-08 | 4 | 4 |
| GJB2 | SEQ ID NO:111 | 0,633 | 0,612 | 0,825 | 4,24E-08 | 2,58E-06 | 7,18E-08 | 4 | 1 |
| APAF1 | SEQ ID NO:82 | 0,723 | 0,725 | 0,696 | 6,25E-08 | 3,81E-06 | 1,03E-07 | 5 | 1 |
| CLDN7 | SEQ ID NO:87 | 0,728 | 0,73 | 0,704 | 1,66E-06 | 1,01E-04 | 2,66E-06 | 4 | 1 |
| ARH1/NOEY2 | SEQ ID NO:97 | 0,713 | 0,699 | 0,843 | 2,18E-06 | 1,33E-04 | 3,41E-06 | 5 | 3 |
| SYK | SEQ ID NO:60 | 0,712 | 0,705 | 0,771 | 4,91E-06 | 2,99E-04 | 7,48E-06 | 4 | 2 |
| ESR1 | SEQ ID NO:75 | 0,686 | 0,672 | 0,821 | 5,17E-06 | 3,15E-04 | 7,69E-06 | 3 | 1 |
| ELK1 | SEQ ID NO:2 | 0,848 | 0,907 | 0,296 | 6,48E-06 | 3,95E-04 | 9,41E-06 | 5 | 0 |
| PGR | SEQ ID NO:83 | 0,757 | 0,77 | 0,632 | 9,28E-06 | 5,66E-04 | 1,32E-05 | 4 | 1 |
| FHIT | SEQ ID NO:76 | 0,665 | 0,663 | 0,686 | 3,60E-05 | 2,19E-03 | 4,99E-05 | 3 | 0 |
| TGFBR2 | SEQ ID NO:93 | 0,634 | 0,634 | 0,639 | 4,05E-05 | 2,47E-03 | 5,49E-05 | 4 | 1 |
| TIMP3 | SEQ ID NO:103 | 0,737 | 0,738 | 0,729 | 8,00E-05 | 4,88E-03 | 1,06E-04 | 4 | 1 |
| TERT | SEQ ID NO:92 | 0,626 | 0,615 | 0,725 | 9,27E-05 | 5,66E-03 | 1,20E-04 | 4 | 0 |
| TMS1/ASC | SEQ ID NO:84 | 0,746 | 0,777 | 0,454 | 2,42E-04 | 1,48E-02 | 3,08E-04 | 4 | 1 |
| CDKN2A | SEQ ID NO:57 | 0,718 | 0,714 | 0,761 | 7,51E-04 | 4,58E-02 | 9,35E-04 | 3 | 2 |
| SASH1 | SEQ ID NO:102 | 0,61 | 0,601 | 0,7 | 2,93E-03 | 1,79E-01 | 3,57E-03 | 4 | 0 |

(continued)

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| BRCA1 | SEQ ID NO:66 | 0,602 | 0,603 | 0,589 | 3,54E-03 | 2,16E-01 | 4,23E-03 | 4 | 0 |
| CASP8 | SEQ ID NO:71 | 0,678 | 0,685 | 0,607 | 5,31E-03 | 3,24E-01 | 6,23E-03 | 4 | 0 |
| CDH1 | SEQ ID NO:79 | 0,653 | 0,649 | 0,689 | 6,12E-03 | 3,73E-01 | 7,04E-03 | 5 | 0 |
| RARB | SEQ ID NO:88 | 0,493 | 0,471 | 0,704 | 1,43E-02 | 8,73E-01 | 1,62E-02 | 4 | 0 |
| ESR2 | SEQ ID NO:91 | 0,504 | 0,484 | 0,7 | 1,79E-02 | 1,00E+00 | 1,99E-02 | 4 | 0 |
| TPM1 | SEQ ID NO:110 | 0,556 | 0,538 | 0,718 | 1,30E-01 | 1,00E+00 | 1,42E-01 | 4 | 0 |
| RARA | SEQ ID NO:108 | 0,498 | 0,498 | 0,5 | 1,44E-01 | 1,00E+00 | 1,55E-01 | 4 | 0 |
| TP73 | SEQ ID NO:86 | 0,521 | 0,524 | 0,489 | 3,04E-01 | 1,00E+00 | 3,20E-01 | 4 | 0 |
| BRCA2 | SEQ ID NO:56 | 0,611 | 0,636 | 0,375 | 3,85E-01 | 1,00E+00 | 3,98E-01 | 5 | 0 |
| TP53 | SEQ ID NO:80 | 0,583 | 0,6 | 0,425 | 3,95E-01 | 1,00E+00 | 3,98E-01 | 4 | 0 |
| MLH1 | SEQ ID NO:89 | 0,471 | 0,467 | 0,507 | 3,98E-01 | 1,00E+00 | 3,98E-01 | 4 | 0 |

Table 9: Breast cancer vs. lymphocytes Chip 2

| SEQ ID NO: | Accuracy | Sensitivity | Specificity | P-value | P-value (Bonferroni) | P-value FDR | n Oligos | n Significant oligos |
|---|---|---|---|---|---|---|---|---|
| SEQ ID NO:20 | 0,997 | 0,998 | 0,985 | 3,34E-44 | 1,81E-42 | 1,81E-42 | 4 | 4 |
| SEQ ID NO:38 | 0,992 | 0,997 | 0,953 | 2,65E-43 | 1,43E-41 | 7,16E-42 | 5 | 4 |
| SEQ ID NO:8 | 0,981 | 0,985 | 0,95 | 1,18E-42 | 6,39E-41 | 1,86E-41 | 5 | 4 |
| SEQ ID NO:37 | 0,992 | 0,995 | 0,976 | 1,38E-42 | 7,45E-41 | 1,86E-41 | 3 | 3 |
| SEQ ID NO:10 | 0,977 | 0,986 | 0,909 | 4,54E-40 | 2,45E-38 | 4,91E-39 | 9 | 8 |
| SEQ ID NO:35 | 0,964 | 0,97 | 0,926 | 1,48E-37 | 7,98E-36 | 1,33E-36 | 4 | 4 |
| SEQ ID NO:47 | 0,963 | 0,968 | 0,926 | 2,48E-36 | 1,34E-34 | 1,91E-35 | 4 | 3 |
| SEQ ID NO:6 | 0,936 | 0,942 | 0,894 | 6,90E-34 | 3,72E-32 | 4,65E-33 | 6 | 6 |
| SEQ ID NO:12 | 0,922 | 0,92 | 0,935 | 6,99E-32 | 3,77E-30 | 4,19E-31 | 4 | 3 |
| SEQ ID NO:46 | 0,922 | 0,927 | 0,882 | 5,28E-30 | 2,85E-28 | 2,85E-29 | 4 | 4 |
| SEQ ID NO:1 | 0,915 | 0,917 | 0,903 | 2,25E-29 | 1,21E-27 | 1,10E-28 | 5 | 5 |
| SEQ ID NO:41 | 0,919 | 0,916 | 0,935 | 3,12E-29 | 1,68E-27 | 1,40E-28 | 5 | 4 |
| SEQ ID NO:22 | 0,93 | 0,927 | 0,95 | 4,54E-29 | 2,45E-27 | 1,88E-28 | 8 | 7 |
| SEQ ID NO:26 | 0,902 | 0,894 | 0,968 | 1,16E-28 | 6,28E-27 | 4,48E-28 | 3 | 3 |
| SEQ ID NO:7 | 0,901 | 0,897 | 0,929 | 2,78E-28 | 1,50E-26 | 1,00E-27 | 5 | 3 |
| SEQ ID NO:15 | 0,883 | 0,882 | 0,888 | 1,97E-27 | 1,06E-25 | 6,39E-27 | 5 | 4 |
| SEQ ID NO:29 | 0,887 | 0,877 | 0,965 | 2,01E-27 | 1,09E-25 | 6,39E-27 | 5 | 2 |
| SEQ ID NO:18 | 0,89 | 0,892 | 0,871 | 2,51E-25 | 1,35E-23 | 7,52E-25 | 5 | 4 |
| SEQ ID NO:34 | 0,877 | 0,879 | 0,856 | 4,95E-25 | 2,68E-23 | 1,41E-24 | 5 | 2 |
| SEQ ID NO:25 | 0,864 | 0,856 | 0,921 | 4,95E-24 | 2,67E-22 | 1,34E-23 | 5 | 2 |

(continued)

| SEQ ID NO: | Accuracy | Sensitivity | Specificity | P-value | P-value (Bonferroni) | P-value FDR | n Oligos | n Significant oligos |
|---|---|---|---|---|---|---|---|---|
| SEQ ID NO:16 | 0,857 | 0,856 | 0,862 | 1,17E-23 | 6,31E-22 | 3,01E-23 | 5 | 3 |
| SEQ ID NO:2 | 0,852 | 0,85 | 0,868 | 2,85E-22 | 1,54E-20 | 7,01E-22 | 4 | 2 |
| SEQ ID NO:117 | 0,83 | 0,825 | 0,865 | 5,73E-20 | 3,10E-18 | 1,35E-19 | 4 | 4 |
| SEQ ID NO:3 | 0,829 | 0,826 | 0,85 | 1,05E-18 | 5,66E-17 | 2,36E-18 | 4 | 4 |
| SEQ ID NO:48 | 0,822 | 0,817 | 0,856 | 1,20E-18 | 6,48E-17 | 2,59E-18 | 4 | 4 |
| SEQ ID NO:4 | 0,824 | 0,818 | 0,871 | 2,48E-17 | 1,34E-15 | 5,15E-17 | 4 | 3 |
| SEQ ID NO:11 | 0,801 | 0,805 | 0,771 | 3,41E-16 | 1,84E-14 | 6,69E-16 | 4 | 4 |
| SEQ ID NO:5 | 0,853 | 0,845 | 0,915 | 3,47E-16 | 1,87E-14 | 6,69E-16 | 5 | 4 |
| SEQ ID NO:54 | 0,757 | 0,745 | 0,844 | 1,83E-15 | 9,91E-14 | 3,42E-15 | 5 | 2 |
| SEQ ID NO:13 | 0,742 | 0,728 | 0,844 | 6,80E-15 | 3,67E-13 | 1,22E-14 | 4 | 3 |
| SEQ ID NO:43 | 0,783 | 0,774 | 0,856 | 1,57E-13 | 8,48E-12 | 2,74E-13 | 4 | 1 |
| SEQ ID NO:31 | 0,777 | 0,772 | 0,809 | 1,77E-13 | 9,57E-12 | 2,99E-13 | 5 | 5 |
| SEQ ID NO:28 | 0,783 | 0,766 | 0,906 | 2,28E-12 | 1,23E-10 | 3,73E-12 | 7 | 1 |
| SEQ ID NO:32 | 0,744 | 0,752 | 0,691 | 2,54E-11 | 1,37E-09 | 4,04E-11 | 3 | 1 |
| SEQ ID NO:27 | 0,753 | 0,742 | 0,832 | 4,05E-11 | 2,19E-09 | 6,25E-11 | 4 | 3 |
| SEQ ID NO:23 | 0,702 | 0,698 | 0,738 | 1,12E-09 | 6,07E-08 | 1,68E-09 | 3 | 1 |
| SEQ ID NO:42 | 0,757 | 0,753 | 0,782 | 1,15E-09 | 6,21E-08 | 1,68E-09 | 4 | 1 |
| SEQ ID NO:51 | 0,654 | 0,634 | 0,803 | 1,35E-09 | 7,28E-08 | 1,92E-09 | 4 | 2 |
| SEQ ID NO:17 | 0,714 | 0,713 | 0,721 | 2,47E-08 | 1,33E-06 | 3,41E-08 | 5 | 1 |
| SEQ ID NO:52 | 0,713 | 0,728 | 0,603 | 6,09E-08 | 3,29E-06 | 8,22E-08 | 4 | 2 |
| SEQ ID NO:30 | 0,656 | 0,623 | 0,906 | 7,96E-08 | 4,30E-06 | 1,05E-07 | 4 | 4 |

(continued)

| SEQ ID NO: | Accuracy | Sensitivity | Specificity | P-value | P-value (Bonferroni) | P-value FDR | n Oligos | n Significant oligos |
|---|---|---|---|---|---|---|---|---|
| SEQ ID NO:24 | 0,643 | 0,608 | 0,903 | 1,26E-07 | 6,79E-06 | 1,62E-07 | 5 | 2 |
| SEQ ID NO:40 | 0,684 | 0,683 | 0,688 | 7,22E-07 | 3,90E-05 | 9,07E-07 | 5 | 1 |
| SEQ ID NO:50 | 0,63 | 0,609 | 0,791 | 9,04E-07 | 4,88E-05 | 1,11E-06 | 5 | 2 |
| SEQ ID NO:19 | 0,667 | 0,664 | 0,685 | 1,55E-06 | 8,36E-05 | 1,86E-06 | 4 | 1 |
| SEQ ID NO:21 | 0,617 | 0,597 | 0,765 | 8,69E-06 | 4,69E-04 | 1,02E-05 | 4 | 1 |
| SEQ ID NO:14 | 0,597 | 0,575 | 0,762 | 1,12E-04 | 6,04E-03 | 1,29E-04 | 3 | 1 |
| SEQ ID NO:9 | 0,603 | 0,579 | 0,785 | 1,35E-03 | 7,29E-02 | 1,52E-03 | 5 | 0 |
| SEQ ID NO:33 | 0,481 | 0,45 | 0,721 | 1,01E-02 | 5,47E-01 | 1,12E-02 | 3 | 0 |
| SEQ ID NO:39 | 0,588 | 0,583 | 0,626 | 1,21E-02 | 6,52E-01 | 1,30E-02 | 4 | 0 |
| SEQ ID NO:55 | 0,472 | 0,445 | 0,674 | 1,94E-02 | 1,00E+00 | 2,06E-02 | 5 | 0 |
| SEQ ID NO:36 | 0,596 | 0,595 | 0,603 | 2,85E-02 | 1,00E+00 | 2,96E-02 | 4 | 0 |
| SEQ ID NO:45 | 0,501 | 0,509 | 0,444 | 3,22E-01 | 1,00E+00 | 3,29E-01 | 4 | 0 |
| SEQ ID NO:44 | 0,343 | 0,309 | 0,606 | 8,51E-01 | 1,00E+00 | 8,51E-01 | 2 | 0 |

Table 10: DCIS vs. benign breast conditions Chip 1

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| HS3ST2 | SEQ ID NO:113 | 0,88 | 0,795 | 0,905 | 1,10E-13 | 6,73E-12 | 6,73E-12 | 2 | 2 |
| SLIT2 | SEQ ID NO:112 | 0,86 | 0,845 | 0,864 | 2,28E-13 | 1,39E-11 | 6,95E-12 | 4 | 4 |
| RASSF1A | SEQ ID NO:90 | 0,88 | 0,877 | 0,882 | 3,27E-12 | 2,00E-10 | 6,65E-11 | 3 | 3 |
| GSTP1 | SEQ ID NO:59 | 0,88 | 0,691 | 0,942 | 3,25E-11 | 1,98E-09 | 4,96E-10 | 4 | 3 |
| GJB2 | SEQ ID NO:111 | 0,82 | 0,732 | 0,843 | 1,17E-09 | 7,15E-08 | 1,43E-08 | 4 | 1 |
| IGFBP7 | SEQ ID NO:94 | 0,84 | 0,786 | 0,861 | 1,48E-09 | 9.01E-08 | 1,50E-08 | 5 | 1 |
| CDH13 | SEQ ID NO:70 | 0,83 | 0,741 | 0,851 | 5,67E-09 | 3,46E-07 | 4,94E-08 | 4 | 4 |
| ARH1/NOEY2 | SEQ ID NO:97 | 0,84 | 0,777 | 0,857 | 1,57E-08 | 9,57E-07 | 1,20E-07 | 5 | 4 |
| SCGB3A1 | SEQ ID NO:115 | 0,82 | 0,832 | 0,814 | 6,51E-08 | 3,97E-06 | 4,41E-07 | 4 | 1 |
| FHIT | SEQ ID NO:76 | 0,79 | 0,814 | 0,781 | 1,23E-07 | 7,50E-06 | 7,50E-07 | 3 | 1 |
| CCND2 | SEQ ID NO:104 | 0,76 | 0,723 | 0,768 | 1,65E-07 | 1,00E-05 | 9,13E-07 | 6 | 2 |
| HIC1 | SEQ ID NO:85 | 0,79 | 0,659 | 0,828 | 2,32E-07 | 1,41E-05 | 1,18E-06 | 5 | 1 |
| APC | SEQ ID NO:65 | 0,73 | 0,6 | 0,773 | 4,29E-07 | 2,62E-05 | 2,01E-06 | 4 | 4 |
| TIMP3 | SEQ ID NO:103 | 0,77 | 0,605 | 0,82 | 5,93E-07 | 3,62E-05 | 2,58E-06 | 4 | 3 |
| IGSF4 | SEQ ID NO:74 | 0,75 | 0,732 | 0,753 | 2,17E-06 | 1,32E-04 | 8,83E-06 | 4 | 1 |
| RARB | SEQ ID NO:88 | 0,77 | 0,614 | 0,818 | 2,53E-06 | 1,54E-04 | 9,65E-06 | 4 | 2 |
| PRDM2 | SEQ ID NO:114 | 0,8 | 0,8 | 0,793 | 3,88E-06 | 2,36E-04 | 1,39E-05 | 4 | 1 |
| PLAU | SEQ ID NO:62 | 0,74 | 0,609 | 0,776 | 4,86E-06 | 2,96E-04 | 1,57E-05 | 4 | 2 |
| CDKN2A | SEQ ID NO:57 | 0,76 | 0,577 | 0,815 | 4,88E-06 | 2,98E-04 | 1,57E-05 | 3 | 0 |
| SLC19A1 | SEQ ID NO:116 | 0,7 | 0,6 | 0,723 | 1,20E-05 | 7,35E-04 | 3,67E-05 | 3 | 0 |
| ELK1 | SEQ ID NO:2 | 0,69 | 0,795 | 0,655 | 1,42E-05 | 8,67E-04 | 4,13E-05 | 5 | 0 |
| FABP3 | SEQ ID NO:77 | 0,8 | 0,655 | 0,846 | 1,80E-05 | 1,10E-03 | 5,00E-05 | 4 | 2 |
| HOXA5 | SEQ ID NO:78 | 0,75 | 0,618 | 0,785 | 2,40E-05 | 1,46E-03 | 6,36E-05 | 4 | 2 |
| SNCG | SEQ ID NO:73 | 0,7 | 0,655 | 0,708 | 4,07E-05 | 2,48E-03 | 1,03E-04 | 4 | 1 |
| TGFBR2 | SEQ ID NO:93 | 0,76 | 0,65 | 0,789 | 4,35E-05 | 2,65E-03 | 1,06E-04 | 4 | 1 |

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| PGR | SEQ ID NO:83 | 0,71 | 0,641 | 0,732 | 5,96E-05 | 3,64E-03 | 1,40E-04 | 4 | 1 |
| THRB | SEQ ID NO:106 | 0,68 | 0,545 | 0,726 | 1,72E-04 | 1,05E-02 | 3,88E-04 | 4 | 1 |
| EYA4 | SEQ ID NO:58 | 0,74 | 0,741 | 0,741 | 3,42E-04 | 2,08E-02 | 7,44E-04 | 4 | 0 |
| APAF1 | SEQ ID NO:82 | 0,72 | 0,659 | 0,736 | 5,53E-04 | 3,37E-02 | 1,16E-03 | 5 | 1 |
| SERPINB5 | SEQ ID NO:68 | 0,69 | 0,573 | 0,719 | 6,00E-04 | 3,66E-02 | 1,22E-03 | 3 | 0 |
| SASH 1 | SEQ ID NO:102 | 0,69 | 0,527 | 0,742 | 7,38E-04 | 4,50E-02 | 1,45E-03 | 4 | 1 |
| MLH1 | SEQ ID NO:89 | 0,73 | 0,659 | 0,751 | 8,31E-04 | 5,07E-02 | 1,58E-03 | 4 | 0 |
| CASP8 | SEQ ID NO:71 | 0,65 | 0,645 | 0,654 | 1,86E-03 | 1.13E-01 | 3,43E-03 | 4 | 0 |
| RARA | SEQ ID NO:108 | 0,66 | 0,486 | 0,707 | 2,27E-03 | 1,38E-01 | 4,07E-03 | 4 | 0 |
| TERT | SEQ ID NO:92 | 0,67 | 0,664 | 0,67 | 2,89E-03 | 1,76E-01 | 5,04E-03 | 4 | 0 |
| SOD2 | SEQ ID NO:105 | 0,69 | 0,591 | 0,72 | 3,25E-03 | 1,98E-01 | 5,50E-03 | 4 | 0 |
| SYK | SEQ ID NO:60 | 0,64 | 0,527 | 0,669 | 3,85E-03 | 2,35E-01 | 6,20E-03 | 4 | 0 |
| TWIST | SEQ ID NO:100 | 0,65 | 0,659 | 0,645 | 3,86E-03 | 2,36E-01 | 6,20E-03 | 4 | 0 |
| S100A7 | SEQ ID NO:96 | 0,72 | 0,486 | 0,784 | 5,17E-03 | 3,15E-01 | 8,09E-03 | 3 | 0 |
| ESR2 | SEQ ID NO:91 | 0,66 | 0,573 | 0,688 | 7,05E-03 | 4,30E-01 | 1,08E-02 | 4 | 0 |
| ESR1 | SEQ ID NO:75 | 0,64 | 0,782 | 0,603 | 9,45E-03 | 5,76E-01 | 1,40E-02 | 3 | 0 |
| MCT1 | SEQ ID NO:101 | 0,65 | 0,641 | 0,655 | 9,62E-03 | 5,87E-01 | 1,40E-02 | 4 | 0 |
| TP73 | SEQ ID NO:86 | 0,65 | 0,641 | 0,649 | 1,06E-02 | 6,47E-01 | 1,50E-02 | 4 | 0 |
| TMS1/ASC | SEQ ID NO:84 | 0,57 | 0,568 | 0,564 | 1,54E-02 | 9,40E-01 | 2,14E-02 | 4 | 0 |
| THBS1 | SEQ ID NO:81 | 0,58 | 0,618 | 0,566 | 1,82E-02 | 1,00E+00 | 2,47E-02 | 4 | 0 |
| PRSS8 | SEQ ID NO:72 | 0,6 | 0,618 | 0,6 | 2,71E-02 | 1,00E+00 | 3,59E-02 | 4 | 0 |
| ALX4 | SEQ ID NO:64 | 0,63 | 0,395 | 0,7 | 4,40E-02 | 1,00E+00 | 5,71E-02 | 4 | 0 |
| DAPK1 | SEQ ID NO:98 | 0,57 | 0,6 | 0,559 | 7,28E-02 | 1,00E+00 | 9,25E-02 | 2 | 0 |
| TPM1 | SEQ ID NO:110 | 0,59 | 0,473 | 0,63 | 9,68E-02 | 1,00E+00 | 1,21E-01 | 4 | 0 |
| CDKN1A | SEQ ID NO:67 | 0,59 | 0,645 | 0,577 | 1,09E-01 | 1,00E+00 | 1,33E-01 | 4 | 0 |

EP 2 302 073 A1

(continued)

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| CDH1 | SEQ ID NO:79 | 0,58 | 0,436 | 0,628 | 1,28E-01 | 1,00E+00 | 1,53E-01 | 5 | 0 |
| IL6 | SEQ ID NO:99 | 0,6 | 0,45 | 0,639 | 1,44E-01 | 1,00E+00 | 1,69E-01 | 4 | 0 |
| CLDN7 | SEQ ID NO:87 | 0,55 | 0,609 | 0,526 | 1,88E-01 | 1,00E+00 | 2,16E-01 | 4 | 0 |
| GPC3 | SEQ ID NO:118 | 0,52 | 0,482 | 0,532 | 2,00E-01 | 1,00E+00 | 2,26E-01 | 4 | 0 |
| STAT1 | SEQ ID NO:109 | 0,56 | 0,618 | 0,545 | 4,52E-01 | 1,00E+00 | 5.01E-01 | 4 | 0 |
| SFN | SEQ ID NO:69 | 0,49 | 0,345 | 0,536 | 5,20E-01 | 1,00E+00 | 5,67E-01 | 4 | 0 |
| BRCA2 | SEQ ID NO:56 | 0,55 | 0,423 | 0,581 | 5,57E-01 | 1,00E+00 | 5,96E-01 | 5 | 0 |
| NME1 | SEQ ID NO:107 | 0,48 | 0,35 | 0,52 | 7,13E-01 | 1,00E+00 | 7,50E-01 | 4 | 0 |
| TP53 | SEQ ID NO:80 | 0,51 | 0,409 | 0,545 | 7,47E-01 | 1,00E+00 | 7,72E-01 | 4 | 0 |
| BRCA1 | SEQ ID NO:66 | 0,47 | 0,318 | 0,515 | 8,59E-01 | 1,00E+00 | 8,73E-01 | 4 | 0 |
| LOT1 | SEQ ID NO:95 | 0,54 | 0,232 | 0,628 | 8,86E-01 | 1,00E+00 | 8,86E-01 | 4 | 0 |

Table 11: DCIS vs. benign breast conditions Chip 2

| SEQ ID NO | Accuracy | Sensitivity | Specificity | P-value | P-value (Bonferroni) | P-value FDR | n Oligos | n Significant oligos |
|---|---|---|---|---|---|---|---|---|
| SEQ ID NO:27 | 0,892 | 0,864 | 0,901 | 2,28E-16 | 1,23E-14 | 1,23E-14 | 4 | 3 |
| SEQ ID NO:12 | 0,86 | 0,818 | 0,874 | 1,63E-13 | 8,80E-12 | 4,40E-12 | 4 | 3 |
| SEQ ID NO:46 | 0,91 | 0,859 | 0,926 | 3,75E-13 | 2,03E-11 | 6,76E-12 | 4 | 3 |
| SEQ ID NO:3 | 0,89 | 0,827 | 0,91 | 9,50E-12 | 5,13E-10 | 1,18E-10 | 4 | 3 |
| SEQ ID NO:117 | 0,876 | 0,791 | 0,903 | 1,09E-11 | 5,88E-10 | 1,18E-10 | 4 | 4 |
| SEQ ID NO:48 | 0,804 | 0,845 | 0,791 | 3,79E-11 | 2,05E-09 | 3,41E-10 | 4 | 2 |
| SEQ ID NO:41 | 0,874 | 0,836 | 0,887 | 7,60E-11 | 4,10E-09 | 5,86E-10 | 5 | 3 |
| SEQ ID NO:4 | 0,792 | 0,664 | 0,834 | 3,02E-10 | 1,63E-08 | 2,04E-09 | 4 | 4 |
| SEQ ID NO:6 | 0,871 | 0,859 | 0,875 | 6,76E-10 | 3,65E-08 | 4,06E-09 | 6 | 5 |
| SEQ ID NO:24 | 0,849 | 0,768 | 0,875 | 7,78E-10 | 4,20E-08 | 4,20E-09 | 5 | 2 |
| SEQ ID NO:31 | 0,823 | 0,718 | 0,857 | 8,60E-10 | 4,65E-08 | 4,22E-09 | 5 | 5 |
| SEQ ID NO:51 | 0,87 | 0,695 | 0,926 | 2,10E-09 | 1,14E-07 | 9,46E-09 | 4 | 1 |
| SEQ ID NO:7 | 0,833 | 0,745 | 0,862 | 2,52E-09 | 1,36E-07 | 1,05E-08 | 5 | 3 |
| SEQ ID NO:13 | 0,813 | 0,768 | 0,828 | 4,22E-09 | 2,28E-07 | 1,63E-08 | 4 | 3 |
| SEQ ID NO:30 | 0,84 | 0,764 | 0,865 | 1,11E-08 | 6.01E-07 | 4,01E-08 | 4 | 3 |
| SEQ ID NO:42 | 0,829 | 0,745 | 0,856 | 4,47E-08 | 2,42E-06 | 1,51E-07 | 4 | 1 |
| SEQ ID NO:16 | 0,847 | 0,691 | 0,897 | 2,98E-07 | 1,61E-05 | 9,48E-07 | 5 | 1 |
| SEQ ID NO:34 | 0,778 | 0,659 | 0,816 | 3,18E-07 | 1,72E-05 | 9,55E-07 | 5 | 1 |
| SEQ ID NO:22 | 0,797 | 0,705 | 0,826 | 1.01E-06 | 5,43E-05 | 2,86E-06 | 8 | 4 |
| SEQ ID NO:17 | 0,758 | 0,718 | 0,771 | 1,65E-06 | 8,91E-05 | 4,45E-06 | 5 | 1 |
| SEQ ID NO:54 | 0,758 | 0,709 | 0,774 | 1,96E-06 | 1,06E-04 | 5,05E-06 | 5 | 2 |

(continued)

| SEQ ID NO | Accuracy | Sensitivity | Specificity | P-value | P-value (Bonferroni) | P-value FDR | n Oligos | n Significant oligos |
|---|---|---|---|---|---|---|---|---|
| SEQ ID NO:50 | 0,731 | 0,573 | 0,782 | 3.01E-06 | 1,62E-04 | 7,38E-06 | 5 | 1 |
| SEQ ID NO:33 | 0,804 | 0,532 | 0,893 | 4,52E-06 | 2,44E-04 | 1,06E-05 | 3 | 0 |
| SEQ ID NO:38 | 0,73 | 0,641 | 0,759 | 9,90E-06 | 5,35E-04 | 2,23E-05 | 5 | 0 |
| SEQ ID NO:10 | 0,732 | 0,623 | 0,768 | 1,22E-05 | 6,61E-04 | 2,64E-05 | 9 | 1 |
| SEQ ID NO:9 | 0,834 | 0,545 | 0,928 | 1,69E-05 | 9,11E-04 | 3,50E-05 | 5 | 0 |
| SEQ ID NO:18 | 0,764 | 0,6 | 0,818 | 2,16E-05 | 1,16E-03 | 4,31E-05 | 5 | 2 |
| SEQ ID NO:23 | 0,773 | 0,591 | 0,832 | 4,56E-05 | 2,46E-03 | 8,79E-05 | 3 | 1 |
| SEQ ID NO:2 | 0,692 | 0,677 | 0,697 | 1,49E-04 | 8,06E-03 | 2,76E-04 | 4 | 1 |
| SEQ ID NO:32 | 0,666 | 0,591 | 0,69 | 1,53E-04 | 8,27E-03 | 2,76E-04 | 3 | 1 |
| SEQ ID NO:29 | 0,711 | 0,591 | 0,75 | 1,65E-04 | 8,90E-03 | 2,82E-04 | 5 | 0 |
| SEQ ID NO:39 | 0,713 | 0,7 | 0,718 | 1,67E-04 | 9,03E-03 | 2,82E-04 | 4 | 1 |
| SEQ ID NO:43 | 0,72 | 0,673 | 0,735 | 2,13E-04 | 1,15E-02 | 3,49E-04 | 4 | 1 |
| SEQ ID NO:11 | 0,71 | 0,527 | 0,769 | 2,74E-04 | 1,48E-02 | 4,31E-04 | 4 | 1 |
| SEQ ID NO:47 | 0,728 | 0,586 | 0,774 | 2,80E-04 | 1,51E-02 | 4,31E-04 | 4 | 0 |
| SEQ ID NO:26 | 0,637 | 0,682 | 0,622 | 1,45E-03 | 7,84E-02 | 2,18E-03 | 3 | 0 |
| SEQ ID NO:52 | 0,646 | 0,823 | 0,588 | 2,25E-03 | 1,21E-01 | 3,28E-03 | 4 | 0 |
| SEQ ID NO:25 | 0,647 | 0,627 | 0,653 | 2,58E-03 | 1,40E-01 | 3,67E-03 | 5 | 0 |
| SEQ ID NO:14 | 0,699 | 0,505 | 0,762 | 3,07E-03 | 1.66E-01 | 4,25E-03 | 3 | 0 |
| SEQ ID NO:20 | 0,586 | 0,55 | 0,597 | 7,37E-03 | 3,98E-01 | 9,95E-03 | 4 | 0 |
| SEQ ID NO:19 | 0,658 | 0,518 | 0,703 | 1,16E-02 | 6,27E-01 | 1,53E-02 | 4 | 0 |
| SEQ ID NO:28 | 0,658 | 0,514 | 0,704 | 1,93E-02 | 1,00E+00 | 2,48E-02 | 7 | 0 |

(continued)

| SEQ ID NO | Accuracy | Sensitivity | Specificity | P-value | P-value (Bonferroni) | P-value FDR | n Oligos | n Significant oligos |
|---|---|---|---|---|---|---|---|---|
| SEQ ID NO:8 | 0,642 | 0,641 | 0,643 | 3,10E-02 | 1,00E+00 | 3,90E-02 | 5 | 0 |
| SEQ ID NO:40 | 0,681 | 0,536 | 0,728 | 4,06E-02 | 1,00E+00 | 4,99E-02 | 5 | 0 |
| SEQ ID NO:1 | 0,58 | 0,445 | 0,624 | 6,69E-02 | 1,00E+00 | 7,95E-02 | 5 | 0 |
| SEQ ID NO:35 | 0,633 | 0,459 | 0,69 | 6,77E-02 | 1,00E+00 | 7,95E-02 | 4 | 0 |
| SEQ ID NO:15 | 0,606 | 0,518 | 0,634 | 8,76E-02 | 1,00E+00 | 1,00E-01 | 5 | 0 |
| SEQ ID NO:36 | 0,566 | 0,577 | 0,562 | 9,68E-02 | 1,00E+00 | 1,09E-01 | 4 | 0 |
| SEQ ID NO:55 | 0,667 | 0,409 | 0,75 | 1,00E-01 | 1,00E+00 | 1,10E-01 | 5 | 0 |
| SEQ ID NO:45 | 0,607 | 0,427 | 0,665 | 1,57E-01 | 1,00E+00 | 1,69E-01 | 4 | 0 |
| SEQ ID NO:5 | 0,597 | 0,468 | 0,638 | 2,14E-01 | 1,00E+00 | 2,27E-01 | 5 | 0 |
| SEQ ID NO:21 | 0,62 | 0,491 | 0,662 | 2,22E-01 | 1,00E+00 | 2,31E-01 | 4 | 0 |
| SEQ ID NO:37 | 0,636 | 0,391 | 0,715 | 3,37E-01 | 1,00E+00 | 3,44E-01 | 3 | 0 |
| SEQ ID NO:44 | 0,55 | 0,186 | 0,668 | 6,49E-01 | 1,00E+00 | 6,49E-01 | 2 | 0 |

Table 12: Chip 1 Breast cancer vs. benign breast conditions

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| SLIT2 | SEQ ID NO:11 | 20,836 | 0,829 | 0,861 | 3,16E-37 | 1,92E-35 | 0,00 | 4 | 4 |
| HS3ST2 | SEQ ID NO:11 | 30,852 | 0,854 | 0,843 | 4,37E-33 | 2,66E-31 | 0,00 | 2 | 2 |
| HOXA5 | SEQ ID NO:78 | 0,782 | 0,784 | 0,776 | 5,67E-28 | 3,46E-26 | 0,00 | 4 | 4 |
| ARH1/NOEY2 | SEQ ID NO:97 | 0,771 | 0,727 | 0,924 | 2,55E-24 | 1,56E-22 | 0,00 | 5 | 5 |
| IGFBP7 | SEQ ID NO:94 | 0,769 | 0,759 | 0,801 | 4,30E-24 | 2,62E-22 | 0,00 | 5 | 3 |
| PLAU | SEQ ID NO:62 | 0,774 | 0,765 | 0,805 | 3,29E-23 | 2,01E-21 | 0,00 | 4 | 3 |
| CDH13 | SEQ ID NO:70 | 0,741 | 0,702 | 0,881 | 1,54E-20 | 9,42E-19 | 0,00 | 4 | 4 |
| TIMP3 | SEQ ID NO:103 | 0,715 | 0,694 | 0,789 | 1,37E-19 | 8,34E-18 | 0,00 | 4 | 4 |
| CCND2 | SEQ ID NO: 104 | 0,729 | 0,716 | 0,776 | 7,23E-19 | 4,41E-17 | 0,00 | 6 | 4 |
| GSTP1 | SEQ ID NO:59 | 0,686 | 0,632 | 0,88 | 3,12E-18 | 1,90E-16 | 0,00 | 4 | 4 |
| APC | SEQ ID NO:65 | 0,733 | 0,731 | 0,739 | 6,84E-18 | 4,18E-16 | 0,00 | 4 | 4 |
| SCGB3A1 | SEQ ID NO:115 | 0,748 | 0,745 | 0,761 | 5,71E-16 | 3,48E-14 | 0,00 | 4 | 1 |
| GJB2 | SEQ ID NO:111 | 0,679 | 0,638 | 0,824 | 9,16E-16 | 5,59E-14 | 0,00 | 4 | 1 |
| CDKN2A | SEQ ID NO:57 | 0,669 | 0,614 | 0,862 | 1,30E-15 | 7,94E-14 | 0,00 | 3 | 3 |
| PRDM2 | SEQ ID NO:114 | 0,718 | 0,696 | 0,795 | 2,85E-14 | 1,74E-12 | 0,00 | 4 | 3 |
| FABP3 | SEQ ID NO:77 | 0,691 | 0,651 | 0,834 | 1,33E-13 | 8,14E-12 | 0,00 | 4 | 4 |
| S100A7 | SEQ ID NO:96 | 0,693 | 0,643 | 0,874 | 1,18E-12 | 7,22E-11 | 0,00 | 3 | 1 |
| SNCG | SEQ ID NO:73 | 0,68 | 0,681 | 0,676 | 1,65E-12 | 1,01E-10 | 0,00 | 4 | 4 |
| RASSF1A | SEQ ID NO:90 | 0,708 | 0,692 | 0,766 | 8,00E-12 | 4,88E-10 | 0,00 | 3 | 3 |
| TMS1/ASC | SEQ ID NO:84 | 0,71 | 0,735 | 0,619 | 9,47E-12 | 5,77E-10 | 0,00 | 4 | 1 |
| FHIT | SEQ ID NO:76 | 0,688 | 0,664 | 0,774 | 1,23E-11 | 7,48E-10 | 0,00 | 3 | 1 |
| SERPINB5 | SEQ ID NO:68 | 0,698 | 0,696 | 0,703 | 1,23E-11 | 7,48E-10 | 0,00 | 3 | 1 |
| SYK | SEQ ID NO:60 | 0,699 | 0,708 | 0,669 | 1,52E-11 | 9,25E-10 | 0,00 | 4 | 2 |
| TWIST | SEQ ID NO:100 | 0,689 | 0,683 | 0,711 | 2,88E-11 | 1,76E-09 | 0,00 | 4 | 1 |
| HIC1 | SEQ ID NO:85 | 0,669 | 0,672 | 0,659 | 3,46E-11 | 2,11E-09 | 0,00 | 5 | 3 |

(continued)

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| SLC19A1 | SEQ ID NO:116 | 0,646 | 0,635 | 0,685 | 2,48E-10 | 1,52E-08 | 0,00 | 3 | 2 |
| APAF1 | SEQ ID NO:82 | 0,667 | 0,653 | 0,715 | 2,85E-10 | 1,74E-08 | 0,00 | 5 | 1 |
| RARB | SEQ ID NO:88 | 0,602 | 0,56 | 0,751 | 8,36E-10 | 5,10E-08 | 0,00 | 4 | 3 |
| TGFBR2 | SEQ ID NO:93 | 0,622 | 0,574 | 0,791 | 1,18E-09 | 7,19E-08 | 0,00 | 4 | 1 |
| MCT1 | SEQ ID NO:101 | 0,719 | 0,748 | 0,616 | 1,42E-09 | 8,66E-08 | 0,00 | 4 | 2 |
| SOD2 | SEQ ID NO:105 | 0,667 | 0,653 | 0,716 | 4,08E-09 | 2,49E-07 | 0,00 | 4 | 1 |
| THRB | SEQ ID NO:106 | 0,663 | 0,642 | 0,738 | 4,27E-09 | 2,61E-07 | 0,00 | 4 | 1 |
| IGSF4 | SEQ ID NO:74 | 0,674 | 0,66 | 0,723 | 4,38E-09 | 2,67E-07 | 0,00 | 4 | 1 |
| SASH1 | SEQ ID NO: 102 | 0,657 | 0,656 | 0,661 | 7,22E-09 | 4,41E-07 | 0,00 | 4 | 2 |
| THBS1 | SEQ ID NO:81 | 0,676 | 0,719 | 0,523 | 1,60E-08 | 9,76E-07 | 0,00 | 4 | 2 |
| ESR1 | SEQ ID NO:75 | 0,694 | 0,724 | 0,585 | 3,85E-08 | 2,35E-06 | 0,00 | 3 | 2 |
| EYA4 | SEQ ID NO:58 | 0,647 | 0,631 | 0,704 | 4,36E-08 | 2,66E-06 | 0,00 | 4 | 3 |
| RARA | SEQ ID NO:108 | 0,623 | 0,594 | 0,73 | 1,07E-07 | 6,51E-06 | 0,00 | 4 | 1 |
| CASP8 | SEQ ID NO:71 | 0,611 | 0,6 | 0,649 | 1,45E-07 | 8,85E-06 | 0,00 | 4 | 1 |
| ALX4 | SEQ ID NO:64 | 0,588 | 0,543 | 0,747 | 1,68E-07 | 1,02E-05 | 0,00 | 4 | 1 |
| ESR2 | SEQ ID NO:91 | 0,55 | 0,5 | 0,731 | 1,27E-06 | 7,72E-05 | 0,00 | 4 | 0 |
| NME1 | SEQ ID NO: 107 | 0,581 | 0,538 | 0,732 | 1,60E-06 | 9,74E-05 | 0,00 | 4 | 2 |
| ELK1 | SEQ ID NO:2 | 0,681 | 0,693 | 0,638 | 1,78E-06 | 1,08E-04 | 0,00 | 5 | 0 |
| MLH1 | SEQ ID NO:89 | 0,535 | 0,468 | 0,773 | 1,80E-06 | 1,10E-04 | 0,00 | 4 | 1 |
| PGR | SEQ ID NO:83 | 0,636 | 0,645 | 0,601 | 2,41E-06 | 1,47E-04 | 0,00 | 4 | 2 |
| TERT | SEQ ID NO:92 | 0,663 | 0,668 | 0,642 | 2,07E-05 | 1,26E-03 | 0,00 | 4 | 2 |
| TP73 | SEQ ID NO:86 | 0,625 | 0,624 | 0,63 | 6,76E-05 | 4,12E-03 | 0,00 | 4 | 1 |
| CLDN7 | SEQ ID NO:87 | 0,622 | 0,638 | 0,566 | 1,52E-04 | 9,25E-03 | 0,00 | 4 | 1 |
| IL6 | SEQ ID NO:99 | 0,567 | 0,552 | 0,622 | 2,09E-04 | 1,28E-02 | 0,00 | 4 | 0 |
| CDKN1A | SEQ ID NO:67 | 0,6 | 0,619 | 0,531 | 1,22E-03 | 7,43E-02 | 0,00 | 4 | 1 |

| Gene Name | SEQ ID NO: | Accuracy | Sensitivity | Specificity | p-value | P-value (Bonferroni) | P-value FDR | n Oligos | n Significant oligos |
|---|---|---|---|---|---|---|---|---|---|
| GPC3 | SEQ ID NO:118 | 0,563 | 0,565 | 0,557 | 1,41E-03 | 8,59E-02 | 0,00 | 4 | 0 |
| LOT1 | SEQ ID NO:95 | 0,513 | 0,437 | 0,784 | 2,46E-03 | 1,50E-01 | 0,00 | 4 | 0 |
| STAT1 | SEQ ID NO:109 | 0,61 | 0,616 | 0,586 | 3,20E-03 | 1,95E-01 | 0,00 | 4 | 0 |
| SFN | SEQ ID NO:69 | 0,545 | 0,549 | 0,53 | 5,58E-03 | 3,40E-01 | 0,01 | 4 | 0 |
| PRSS8 | SEQ ID NO:72 | 0,542 | 0,532 | 0,578 | 6,37E-03 | 3,88E-01 | 0,01 | 4 | 0 |
| TPM1 | SEQ ID NO:110 | 0,524 | 0,479 | 0,684 | 1,73E-02 | 1,00E+00 | 0,02 | 4 | 0 |
| BRCA2 | SEQ ID NO:56 | 0,558 | 0,553 | 0,576 | 2,76E-02 | 1,00E+00 | 0,03 | 5 | 0 |
| BRCA2 | SEQ ID NO:66 | 0,531 | 0,487 | 0,684 | 4,70E-02 | 1,00E+00 | 0,05 | 4 | 0 |
| TP53 | SEQ ID NO:80 | 0,484 | 0,449 | 0,607 | 1,87E-01 | 1,00E+00 | 0,19 | 4 | 0 |
| CDH1 | SEQ ID NO:79 | 0,534 | 0,534 | 0,531 | 3,69E-01 | 1,00E+00 | 0,38 | 5 | 0 |
| DAPK1 | SEQ ID NO:98 | 0,46 | 0,466 | 0,439 | 9,43E-01 | 1,00E+00 | 0,94 | 2 | 0 |

Table 13: Chip 2 Breast cancer vs. benign breast conditions

| SEQ ID NO | Accuracy | Sensitivity | Specificity | P-value | P-value (Bonferroni) | P-value FDR | n Oligos | n Significant oligos |
|---|---|---|---|---|---|---|---|---|
| SEQ ID NO:117 | 0,86 | 0,839 | 0,94 | 2,44E-39 | 1,32E-37 | 1,32E-37 | 4 | 4 |
| SEQ ID NO:12 | 0,852 | 0,839 | 0,899 | 5,84E-36 | 3,15E-34 | 1,58E-34 | 4 | 3 |
| SEQ ID NO:46 | 0,821 | 0,801 | 0,899 | 6,41E-32 | 3,46E-30 | 1,15E-30 | 4 | 4 |
| SEQ ID NO:6 | 0,858 | 0,85 | 0,89 | 1,94E-31 | 1,05E-29 | 2,61E-30 | 6 | 6 |
| SEQ ID NO:7 | 0,838 | 0,828 | 0,878 | 1,08E-29 | 5,86E-28 | 1,17E-28 | 5 | 4 |
| SEQ ID NO:3 | 0,834 | 0,815 | 0,906 | 2,11E-29 | 1,14E-27 | 1,66E-28 | 4 | 4 |
| SEQ ID NO:41 | 0,824 | 0,821 | 0,837 | 2,16E-29 | 1,17E-27 | 1,66E-28 | 5 | 4 |
| SEQ ID NO:27 | 0,802 | 0,787 | 0,857 | 7,96E-29 | 4,30E-27 | 5,37E-28 | 4 | 3 |
| SEQ ID NO:31 | 0,825 | 0,816 | 0,862 | 1,14E-27 | 6,17E-26 | 6,85E-27 | 5 | 5 |
| SEQ ID NO:4 | 0,767 | 0,752 | 0,822 | 4,61E-22 | 2,49E-20 | 2,49E-21 | 4 | 4 |
| SEQ ID NO:51 | 0,73 | 0,686 | 0,897 | 1,82E-21 | 9,83E-20 | 8,94E-21 | 4 | 2 |
| SEQ ID NO:18 | 0,773 | 0,762 | 0,816 | 4,66E-21 | 2,52E-19 | 2,10E-20 | 5 | 3 |
| SEQ ID NO:47 | 0,761 | 0,745 | 0,824 | 9,81E-20 | 5,30E-18 | 4,07E-19 | 4 | 2 |
| SEQ ID NO:16 | 0,745 | 0,722 | 0,829 | 4,98E-18 | 2,69E-16 | 1,92E-17 | 5 | 1 |
| SEQ ID NO:34 | 0,701 | 0,664 | 0,838 | 5,22E-17 | 2,82E-15 | 1,88E-16 | 5 | 2 |
| SEQ ID NO:42 | 0,747 | 0,728 | 0,819 | 9,31E-17 | 5,03E-15 | 3,14E-16 | 4 | 2 |
| SEQ ID NO:13 | 0,695 | 0,655 | 0,844 | 1,61E-15 | 8,69E-14 | 5,11E-15 | 4 | 3 |
| SEQ ID NO:39 | 0,73 | 0,729 | 0,731 | 2,04E-15 | 1,10E-13 | 6,11E-15 | 4 | 4 |
| SEQ ID NO:43 | 0,731 | 0,72 | 0,769 | 3,58E-15 | 1,93E-13 | 1,02E-14 | 4 | 1 |
| SEQ ID NO:29 | 0,691 | 0,66 | 0,807 | 4,12E-15 | 2,23E-13 | 1,11E-14 | 5 | 4 |
| SEQ ID NO:22 | 0,723 | 0,7 | 0,812 | 8,23E-15 | 4,45E-13 | 2,12E-14 | 8 | 7 |

(continued)

| SEQ ID NO | Accuracy | Sensitivity | Specificity | P-value | P-value (Bonferroni) | P-value FDR | n Oligos | n Significant oligos |
|---|---|---|---|---|---|---|---|---|
| SEQ ID NO:30 | 0,69 | 0,625 | 0,935 | 1,09E-14 | 5,89E-13 | 2,68E-14 | 4 | 4 |
| SEQ ID NO:11 | 0,688 | 0,67 | 0,756 | 1,90E-14 | 1,03E-12 | 4,46E-14 | 4 | 4 |
| SEQ ID NO:10 | 0,69 | 0,674 | 0,749 | 8,91E-14 | 4,81E-12 | 2,00E-13 | 9 | 4 |
| SEQ ID NO:8 | 0,709 | 0,702 | 0,735 | 1,01E-13 | 5,47E-12 | 2,19E-13 | 5 | 5 |
| SEQ ID NO:48 | 0,727 | 0,718 | 0,762 | 7,74E-13 | 4,18E-11 | 1,61E-12 | 4 | 4 |
| SEQ ID NO:50 | 0,631 | 0,602 | 0,738 | 7,90E-12 | 4,27E-10 | 1,58E-11 | 5 | 2 |
| SEQ ID NO:33 | 0,631 | 0,586 | 0,803 | 2,75E-11 | 1,49E-09 | 5,31E-11 | 3 | 2 |
| SEQ ID NO:24 | 0,606 | 0,56 | 0,778 | 1,94E-10 | 1,05E-08 | 3,62E-10 | 5 | 3 |
| SEQ ID NO:26 | 0,678 | 0,687 | 0,646 | 3,57E-10 | 1,93E-08 | 6,42E-10 | 3 | 2 |
| SEQ ID NO:54 | 0,642 | 0,623 | 0,713 | 2,19E-09 | 1,18E-07 | 3,81E-09 | 5 | 2 |
| SEQ ID NO:38 | 0,665 | 0,653 | 0,712 | 2,52E-09 | 1,36E-07 | 4,25E-09 | 5 | 1 |
| SEQ ID NO:25 | 0,661 | 0,643 | 0,728 | 7,27E-09 | 3,93E-07 | 1,19E-08 | 5 | 1 |
| SEQ ID NO:32 | 0,636 | 0,618 | 0,706 | 1,59E-08 | 8,58E-07 | 2,52E-08 | 3 | 1 |
| SEQ ID NO:5 | 0,664 | 0,635 | 0,774 | 4,69E-08 | 2,53E-06 | 7,24E-08 | 5 | 0 |
| SEQ ID NO:23 | 0,648 | 0,622 | 0,744 | 5,74E-08 | 3,10E-06 | 8,62E-08 | 3 | 1 |
| SEQ ID NO:17 | 0,636 | 0,599 | 0,774 | 1,17E-07 | 6,32E-06 | 1,71E-07 | 5 | 2 |
| SEQ ID NO:1 | 0,64 | 0,638 | 0,647 | 1,33E-07 | 7,17E-06 | 1,89E-07 | 5 | 1 |
| SEQ ID NO:14 | 0,601 | 0,571 | 0,715 | 1,58E-07 | 8,55E-06 | 2,19E-07 | 3 | 1 |
| SEQ ID NO:52 | 0,69 | 0,705 | 0,635 | 3,78E-07 | 2,04E-05 | 5,11E-07 | 4 | 2 |
| SEQ ID NO:9 | 0,642 | 0,619 | 0,728 | 5,19E-07 | 2,80E-05 | 6,83E-07 | 5 | 1 |
| SEQ ID NO:19 | 0,602 | 0,567 | 0,734 | 1,23E-06 | 6,64E-05 | 1,58E-06 | 4 | 1 |

(continued)

| SEQ ID NO | Accuracy | Sensitivity | Specificity | P-value | P-value (Bonferroni) | P-value FDR | n Oligos | n Significant oligos |
|---|---|---|---|---|---|---|---|---|
| SEQ ID NO:40 | 0,611 | 0,582 | 0,722 | 3,21E-06 | 1,73E-04 | 4,03E-06 | 5 | 1 |
| SEQ ID NO:35 | 0,623 | 0,641 | 0,553 | 7,05E-05 | 3,81E-03 | 8,65E-05 | 4 | 0 |
| SEQ ID NO:2 | 0,594 | 0,569 | 0,69 | 1,02E-04 | 5,50E-03 | 1,22E-04 | 4 | 1 |
| SEQ ID NO:28 | 0,596 | 0,567 | 0,704 | 1,21E-04 | 6,52E-03 | 1,42E-04 | 7 | 2 |
| SEQ ID NO:36 | 0,583 | 0,584 | 0,579 | 4,02E-04 | 2,17E-02 | 4,61E-04 | 4 | 1 |
| SEQ ID NO:20 | 0,564 | 0,538 | 0,662 | 7,11E-04 | 3,84E-02 | 8,00E-04 | 4 | 1 |
| SEQ ID NO:37 | 0,619 | 0,636 | 0,554 | 5,71E-03 | 3,08E-01 | 6,29E-03 | 3 | 0 |
| SEQ ID NO:55 | 0,425 | 0,33 | 0,781 | 9,30E-02 | 1,00E+00 | 1,00E-01 | 5 | 0 |
| SEQ ID NO:15 | 0,533 | 0,511 | 0,613 | 1,09E-O1 | 1,00E+00 | 1,16E-01 | 5 | 0 |
| SEQ ID NO:44 | 0,488 | 0,454 | 0,615 | 1,43E-01 | 1,00E+00 | 1,48E-01 | 2 | 0 |
| SEQ ID NO:21 | 0,528 | 0,521 | 0,556 | 1,53E-01 | 1,00E+00 | 1,55E-01 | 4 | 0 |
| SEQ ID NO:45 | 0,525 | 0,521 | 0,54 | 1,95E-01 | 1,00E+00 | 1,95E-01 | 4 | 0 |

Table 14: Sample overview

| Sample No. | main class | subclass | sex | age | Menopausal status |
|---|---|---|---|---|---|
| 1 | benign breast | Normal Breast | f | 45 | |
| 2 | benign breast | Normal Breast | f | 45 | |
| 3 | benign breast | Normal Breast | f | 17 | |
| 4 | benign breast | Normal Breast | f | 20 | |
| 5 | benign breast | Normal Breast | f | 19 | |
| 6 | benign breast | Normal Breast | f | 33 | |
| 7 | benign breast | Normal Breast | f | 36 | |
| 8 | benign breast | Normal Breast | f | 37 | |
| 9 | benign breast | Normal Breast | f | 57 | |
| 10 | benign breast | Normal Breast | f | 50 | |
| 11 | benign breast | Normal Breast | f | 53 | |
| 12 | benign breast | Normal Breast | f | 77 | |
| 13 | other cancer | ovarian cancer | f | 83 | |

(continued)

| Sample No. | main class | subclass | sex | age | Menopausal status |
|---|---|---|---|---|---|
| 14 | other cancer | ovarian cancer | f | 30 | |
| 15 | other cancer | ovarian cancer | f | 53 | |
| 16 | other cancer | ovarian cancer | f | 56 | |
| 17 | other cancer | ovarian cancer | f | 47 | |
| 18 | other cancer | ovarian cancer | f | 63 | |
| 19 | other cancer | ovarian cancer | f | 55 | |
| 20 | other cancer | ovarian cancer | f | 58 | |
| 21 | other cancer | ovarian cancer | f | 49 | |
| 22 | other cancer | ovarian cancer | f | 52 | |
| 23 | other cancer | endometrial cancer | f | 53 | |
| 24 | other cancer | endometrial cancer | f | 46 | |
| 25 | other cancer | endometrial cancer | f | 64 | |
| 26 | other cancer | endometrial cancer | f | 51 | |
| 27 | other cancer | endometrial cancer | f | 80 | |
| 28 | other cancer | endometrial cancer | f | 68 | |
| 29 | other cancer | endometrial cancer | f | 70 | |
| 30 | other cancer | endometrial cancer | f | 77 | |
| 31 | other cancer | endometrial cancer | f | 92 | |
| 32 | other cancer | endometrial cancer | f | 27 | |
| 33 | other cancer | endometrial cancer | f | 52 | |
| 34 | other cancer | endometrial cancer | f | 61 | |
| 35 | other cancer | ovarian cancer | f | 52 | |
| 36 | other cancer | ovarian cancer | f | 65 | |
| 37 | other cancer | ovarian cancer | f | na | |
| 38 | other cancer | ovarian cancer | f | 54 | |
| 39 | other cancer | ovarian cancer | f | 52 | |
| 40 | other cancer | ovarian cancer | f | 68 | |
| 41 | other cancer | endometrial cancer | f | 74 | |
| 42 | other cancer | endometrial cancer | f | 30 | |
| 43 | other cancer | endometrial cancer | f | 44 | |
| 44 | other cancer | endometrial cancer | f | 58 | |
| 45 | benign breast | Normal Breast | f | 78 | |
| 46 | benign breast | Normal Breast | f | 37 | |
| 47 | benign breast | Normal Breast | f | 48 | |
| 48 | benign breast | Normal Breast | f | 37 | |
| 49 | benign breast | Normal Breast | f | 36 | |
| 50 | benign breast | Normal Breast | f | 43 | |
| 51 | benign breast | Normal Breast | f | 41 | |

(continued)

| Sample No. | main class | subclass | sex | age | Menopausal status |
|---|---|---|---|---|---|
| 52 | benign breast | Normal Breast | f | 48 | |
| 53 | benign breast | Normal Breast | f | 35 | |
| 54 | benign breast | Normal Breast | f | 41 | pre |
| 55 | benign breast | Normal Breast | f | 48 | |
| 56 | benign breast | Normal Breast | f | 39 | |
| 57 | benign breast | Normal Breast | f | 69 | |
| 58 | benign breast | Normal Breast | f | 52 | |
| 59 | benign breast | Normal Breast | f | 41 | |
| 60 | benign breast | Normal Breast | f | 43 | |
| 61 | benign breast | Normal Breast | f | 36 | |
| 62 | benign breast | Normal Breast | f | 36 | |
| 63 | other cancer | lung cancer | f | - | |
| 64 | other cancer | lung cancer | f | - | |
| 65 | other cancer | lung cancer | f | - | |
| 66 | other cancer | lung cancer | f | - | |
| 67 | other cancer | lung cancer | f | 78 | |
| 68 | other cancer | lung cancer | f | 60 | |
| 69 | other cancer | lung cancer | f | 54 | |
| 70 | other cancer | lung cancer | f | - | |
| 71 | other cancer | lung cancer | f | 67 | post |
| 72 | other cancer | lung cancer | f | 56 | post |
| 73 | other cancer | lung cancer | f | 51 | pre |
| 74 | other cancer | lung cancer | f | 76 | post |
| 75 | other cancer | lung cancer | f | 41 | |
| 76 | other cancer | lung cancer | f | 66 | post |
| 77 | lymphocytes | lymphocytes | f | 60 | |
| 78 | lymphocytes | lymphocytes | f | 57 | |
| 79 | lymphocytes | lymphocytes | f | 61 | |
| 80 | lymphocytes | lymphocytes | f | 61 | |
| 81 | lymphocytes | lymphocytes | f | 49 | |
| 82 | lymphocytes | lymphocytes | f | 48 | |
| 83 | lymphocytes | lymphocytes | f | 42 | |
| 84 | lymphocytes | lymphocytes | f | 44 | |
| 85 | lymphocytes | lymphocytes | f | 47 | |
| 86 | lymphocytes | lymphocytes | f | 47 | |
| 87 | lymphocytes | lymphocytes | f | 49 | |
| 88 | lymphocytes | lymphocytes | f | 55 | |
| 89 | lymphocytes | lymphocytes | f | 55 | |

(continued)

| Sample No. | main class | subclass | sex | age | Menopausal status |
|---|---|---|---|---|---|
| 90 | lymphocytes | lymphocytes | f | 55 | |
| 91 | lymphocytes | lymphocytes | m | 49 | |
| 92 | lymphocytes | lymphocytes | f | 65 | |
| 93 | lymphocytes | lymphocytes | f | 82 | |
| 94 | lymphocytes | lymphocytes | f | 64 | |
| 95 | lymphocytes | lymphocytes | f | 74 | |
| 96 | lymphocytes | lymphocytes | f | 37 | |
| 97 | lymphocytes | lymphocytes | f | 37 | |
| 98 | lymphocytes | lymphocytes | f | 42 | |
| 99 | lymphocytes | lymphocytes | f | 28 | |
| 100 | lymphocytes | lymphocytes | f | 58 | |
| 101 | lymphocytes | lymphocytes | f | 67 | |
| 102 | lymphocytes | lymphocytes | f | 38 | |
| 103 | other cancer | lung cancer | f | 55 | |
| 104 | other cancer | lung cancer | f | 56 | |
| 105 | other cancer | lung cancer | f | 68 | |
| 106 | other cancer | lung cancer | f | 80 | |
| 107 | lymphocytes | lymphocytes | m | NA | |
| 108 | lymphocytes | lymphocytes | m | NA | |
| 109 | lymphocytes | lymphocytes | m | NA | |
| 110 | lymphocytes | lymphocytes | m | NA | |
| 111 | lymphocytes | lymphocytes | m | NA | |
| 112 | lymphocytes | lymphocytes | m | NA | |
| 113 | lymphocytes | lymphocytes | m | NA | |
| 114 | lymphocytes | lymphocytes | m | NA | |
| 115 | brca | IDC | f | 69 | post |
| 116 | brca | IDC | f | 69 | post |
| 117 | brca | IDC | f | 33 | pre |
| 118 | brca | IDC | f | 54 | pre |
| 119 | brca | IDC | f | 86 | post |
| 120 | brca | IDC | f | 62 | post |
| 121 | brca | IDC | f | 30 | pre |
| 122 | brca | IDC | f | 33 | pre |
| 123 | brca | IDC | f | 78 | post |
| 124 | brca | IDC | f | 34 | pre |
| 125 | brca | IDC | f | 46 | pre |
| 126 | brca | IDC | f | 61 | post |
| 127 | brca | IDC | f | 44 | pre |

(continued)

| Sample No. | main class | subclass | sex | age | Menopausal status |
|---|---|---|---|---|---|
| 128 | brca | IDC | f | 68 | post |
| 129 | brca | IDC | f | 57 | post |
| 130 | brca | IDC | f | 70 | post |
| 131 | brca | IDC | f | 42 | pre |
| 132 | brca | IDC | f | 37 | pre |
| 133 | brca | IDC | f | 45 | pre |
| 134 | brca | IDC | f | 47 | pre |
| 135 | brca | IDC | f | 35 | pre |
| 136 | brca | IDC | f | 49 | pre |
| 137 | brca | IDC | f | 40 | post |
| 138 | brca | IDC | f | 75 | post |
| 139 | brca | IDC | f | 76 | post |
| 140 | brca | IDC | f | 69 | post |
| 141 | brca | IDC | f | 46 | pre |
| 142 | brca | IDC | f | 50 | pre |
| 143 | brca | IDC | f | 44 | pre |
| 144 | brca | IDC | f | 47 | pre |
| 145 | brca | ILC | f | 47 | pre |
| 146 | brca | IDC | f | 56 | post |
| 147 | brca | IDC | f | 40 | pre |
| 148 | brca | IDC | f | 38 | pre |
| 149 | brca | ILC | f | 50 | pre |
| 150 | brca | IDC | f | 42 | pre |
| 151 | brca | ILC | f | 55 | pre |
| 152 | brca | IDC | f | 62 | post |
| 153 | brca | IDC | f | 63 | post |
| 154 | brca | IDC | f | 38 | pre |
| 155 | brca | IDC | f | 32 | pre |
| 156 | brca | IDC | f | 59 | post |
| 157 | brca | IDC | f | 53 | post |
| 158 | brca | IDC | f | 38 | pre |
| 159 | brca | IDC | f | 43 | pre |
| 160 | brca | IDC | f | 46 | pre |
| 161 | brca | IDC | f | 47 | pre |
| 162 | brca | IDC | f | 52 | pre |
| 163 | brca | IDC | f | 55 | post |
| 164 | brca | IDC | f | 68 | post |
| 165 | brca | IDC | f | 68 | post |

(continued)

| Sample No. | main class | subclass | sex | age | Menopausal status |
|---|---|---|---|---|---|
| 166 | brca | IDC | f | 63 | post |
| 167 | brca | IDC | f | 49 | post |
| 168 | brca | IDC | f | 47 | pre |
| 169 | brca | IDC | f | 50 | pre |
| 170 | brca | IDC | f | 66 | post |
| 171 | brca | ILC | f | 48 | pre |
| 172 | brca | IDC | f | 56 | post |
| 173 | brca | IDC | f | 53 | post |
| 174 | brca | IDC | f | 33 | pre |
| 175 | brca | IDC | f | 48 | pre |
| 176 | brca | ILC | f | 40 | pre |
| 177 | brca | ILC | f | 44 | pre |
| 178 | brca | IDC | f | 79 | post |
| 179 | brca | IDC | f | 78 | post |
| 180 | brca | IDC | f | 30 | pre |
| 181 | brca | IDC | f | 44 | pre |
| 182 | brca | IDC | f | 45 | pre |
| 183 | brca | ILC | f | 47 | pre |
| 184 | brca | ILC | f | 51 | pre |
| 185 | brca | IDC | f | 65 | post |
| 186 | brca | IDC | f | 70 | post |
| 187 | brca | IDC | f | 51 | pre |
| 188 | brca | IDC | f | 67 | post |
| 189 | brca | IDC | f | 44 | pre |
| 190 | brca | IDC | f | 64 | post |
| 191 | brca | IDC | f | 72 | post |
| 192 | brca | ILC | f | 42 | pre |
| 193 | brca | IDC | f | 41 | pre |
| 194 | brca | ILC | f | 37 | pre |
| 195 | brca | IDC | f | 65 | post |
| 196 | brca | IDC | f | 67 | post |
| 197 | brca | IDC | f | 62 | post |
| 198 | brca | IDC | f | 51 | pre |
| 199 | brca | IDC | f | 55 | post |
| 200 | brca | IDC | f | 53 | post |
| 201 | brca | IDC | f | 74 | post |
| 202 | brca | ILC | f | 45 | pre |
| 203 | brca | ILC | f | 46 | pre |

(continued)

| Sample No. | main class | subclass | sex | age | Menopausal status |
|---|---|---|---|---|---|
| 204 | brca | IDC | f | 85 | post |
| 205 | brca | IDC | f | 72 | post |
| 206 | brca | IDC | f | 48 | pre |
| 207 | brca | IDC | f | 62 | post |
| 208 | brca | IDC | f | 52 | post |
| 209 | brca | IDC | f | 45 | pre |
| 210 | brca | IDC | f | 35 | pre |
| 211 | brca | IDC | f | 71 | post |
| 212 | brca | IDC | f | 61 | post |
| 213 | brca | IDC | f | 49 | pre |
| 214 | brca | IDC | f | 24 | pre |
| 215 | brca | IDC | f | 48 | pre |
| 216 | brca | IDC | f | 51 | post |
| 217 | brca | IDC | f | 61 | post |
| 218 | brca | IDC | f | 55 | post |
| 219 | brca | IDC | f | 87 | post |
| 220 | brca | IDC | f | 51 | post |
| 221 | brca | IDC | f | 43 | pre |
| 222 | brca | IDC | f | 65 | post |
| 223 | brca | IDC | f | 59 | post |
| 224 | brca | IDC | f | 53 | post |
| 225 | brca | IDC | f | 51 | pre |
| 226 | brca | IDC | f | 31 | pre |
| 227 | brca | IDC | f | 54 | post |
| 228 | brca | IDC | f | 60 | post |
| 229 | brca | ILC | f | 49 | pre |
| 230 | brca | IDC | f | 46 | pre |
| 231 | brca | IDC | f | 74 | post |
| 232 | brca | IDC | f | 47 | pre |
| 233 | brca | IDC | f | 46 | pre |
| 234 | brca | IDC | f | 44 | pre |
| 235 | brca | ILC | f | 48 | pre |
| 236 | brca | IDC | f | 51 | post |
| 237 | brca | ILC | f | 35 | pre |
| 238 | brca | IDC | f | 32 | pre |
| 239 | brca | IDC | f | 40 | pre |
| 240 | brca | IDC | f | 43 | pre |
| 241 | brca | IDC | f | 60 | post |

(continued)

| Sample No. | main class | subclass | sex | age | Menopausal status |
|---|---|---|---|---|---|
| 242 | brca | IDC | f | 76 | post |
| 243 | brca | IDC | f | 48 | pre |
| 244 | brca | IDC | f | 48 | pre |
| 245 | brca | IDC | f | 33 | pre |
| 246 | brca | IDC | f | 58 | post |
| 247 | brca | IDC | f | 62 | post |
| 248 | brca | IDC | f | 46 | post |
| 249 | brca | IDC | f | 63 | post |
| 250 | brca | IDC | f | 64 | post |
| 251 | brca | IDC | f | 46 | pre |
| 252 | brca | IDC | f | 47 | pre |
| 253 | brca | ILC | f | 44 | pre |
| 254 | brca | IDC | f | 38 | pre |
| 255 | brca | ILC | f | 38 | pre |
| 256 | brca | IDC | f | 48 | pre |
| 257 | brca | IDC | f | 50 | pre |
| 258 | brca | IDC | f | 66 | post |
| 259 | brca | IDC | f | 33 | pre |
| 260 | brca | IDC | f | 59 | post |
| 261 | brca | IDC | f | 35 | pre |
| 262 | brca | IDC | f | 59 | post |
| 263 | brca | IDC | f | 65 | post |
| 264 | brca | IDC | f | 56 | post |
| 265 | brca | IDC | f | 52 | post |
| 266 | brca | IDC | f | 47 | pre |
| 267 | brca | IDC | f | 75 | post |
| 268 | brca | IDC | f | 29 | pre |
| 269 | brca | IDC | f | 70 | post |
| 270 | brca | IDC | f | 79 | post |
| 271 | brca | IDC | f | 63 | post |
| 272 | brca | ILC | f | 37 | pre |
| 273 | brca | IDC | f | 69 | post |
| 274 | brca | IDC | f | 57 | post |
| 275 | brca | IDC | f | 72 | post |
| 276 | brca | IDC | f | 79 | post |
| 277 | brca | IDC | f | 53 | post |
| 278 | brca | IDC | f | 52 | pre |
| 279 | brca | IDC | f | 48 | pre |

(continued)

| Sample No. | main class | subclass | sex | age | Menopausal status |
|---|---|---|---|---|---|
| 280 | brca | ILC | f | 44 | pre |
| 281 | brca | IDC | f | 50 | pre |
| 282 | brca | IDC | f | 61 | post |
| 283 | brca | IDC | f | 57 | post |
| 284 | brca | IDC | f | 64 | post |
| 285 | brca | IDC | f | 43 | pre |
| 286 | brca | ILC | f | 46 | pre |
| 287 | brca | IDC | f | 62 | post |
| 288 | brca | IDC | f | 32 | pre |
| 289 | brca | IDC | f | 40 | pre |
| 290 | brca | IDC | f | 65 | post |
| 291 | brca | IDC | f | 47 | pre |
| 292 | brca | IDC | f | 45 | pre |
| 293 | brca | IDC | f | 51 | pre |
| 294 | brca | IDC | f | 62 | post |
| 295 | brca | IDC | f | 72 | post |
| 296 | brca | IDC | f | 52 | post |
| 297 | brca | IDC | f | 52 | post |
| 298 | brca | IDC | f | 46 | pre |
| 299 | brca | IDC | f | 61 | post |
| 300 | brca | IDC | f | 51 | pre |
| 301 | brca | IDC | f | 48 | pre |
| 302 | brca | IDC | f | 47 | pre |
| 303 | brca | IDC | f | 44 | pre |
| 304 | brca | IDC | f | 52 | pre |
| 305 | brca | IDC | f | 55 | post |
| 306 | brca | IDC | f | 42 | pre |
| 307 | brca | IDC | f | 49 | pre |
| 308 | brca | IDC | f | 52 | post |
| 309 | brca | IDC | f | 69 | post |
| 310 | brca | IDC | f | 64 | post |
| 311 | brca | IDC | f | 40 | pre |
| 312 | brca | IDC | f | 45 | pre |
| 313 | brca | IDC | f | 66 | post |
| 314 | brca | IDC | f | 64 | post |
| 315 | brca | IDC | f | 53 | post |
| 316 | brca | ILC | f | 51 | pre |
| 317 | brca | IDC | f | 54 | post |

(continued)

| Sample No. | main class | subclass | sex | age | Menopausal status |
|---|---|---|---|---|---|
| 318 | brca | IDC | f | 52 | pre |
| 319 | brca | IDC | f | 45 | pre |
| 320 | brca | ILC | f | 54 | pre |
| 321 | brca | IDC | f | 52 | pre |
| 322 | brca | IDC | f | 47 | pre |
| 323 | brca | ILC | f | 41 | pre |
| 324 | brca | IDC | f | 56 | post |
| 325 | brca | IDC | f | 72 | post |
| 326 | brca | ILC | f | 41 | pre |
| 327 | brca | IDC | f | 54 | pre |
| 328 | brca | IDC | f | 36 | pre |
| 329 | brca | IDC | f | 44 | pre |
| 330 | brca | IDC | f | 71 | post |
| 331 | brca | IDC | f | 38 | pre |
| 332 | brca | IDC | f | 27 | pre |
| 333 | brca | IDC | f | 42 | pre |
| 334 | brca | IDC | f | 51 | pre |
| 335 | brca | DCIS | f | 49 | |
| 336 | brca | DCIS | f | 96 | |
| 337 | brca | DCIS | f | 39 | |
| 338 | benign breast | fibroadenoma | f | 36 | |
| 339 | benign breast | fibroadenoma | f | 42 | |
| 340 | benign breast | fibroadenoma | f | 24 | |
| 341 | benign breast | fibroadenoma | f | 27 | |
| 342 | benign breast | fibroadenoma | f | 42 | |
| 343 | benign breast | fibroadenoma | f | 50 | |
| 344 | benign breast | fibroadenoma | f | 43 | |
| 345 | benign breast | fibroadenoma | f | 43 | |
| 346 | benign breast | fibroadenoma | f | 33 | |
| 347 | benign breast | fibrocystic changes | f | 54 | |
| 348 | benign breast | fibrocystic changes | f | 61 | |
| 349 | benign breast | fibrocystic disease | f | 35 | |
| 350 | benign breast | fibrocystic changes | f | 44 | |
| 351 | benign breast | fibrocystic disease | f | 45 | |
| 352 | benign breast | Breast Benign Disease | f | 45 | |
| 353 | brca | BRCA1 | f | 44 | |
| 354 | brca | BRCA1 | f | 31 | |
| 355 | brca | BRCA1 | f | 39 | |

(continued)

| Sample No. | main class | subclass | sex | age | Menopausal status |
|---|---|---|---|---|---|
| 356 | brca | BRCA1 | f | 32 | |
| 357 | brca | BRCA1 | f | 44 | |
| 358 | brca | BRCA1 | f | 35 | |
| 359 | brca | BRCA1 | f | 32 | |
| 360 | brca | BRCA1 | f | 32 | |
| 361 | brca | BRCA1 | f | 29 | |
| 362 | brca | BRCA1 | f | 47 | |
| 363 | brca | BRCA1 | f | 49 | |
| 364 | brca | BRCA1 | f | 37 | |
| 365 | brca | BRCA1 | f | 32 | |
| 366 | brca | BRCA1 | f | 50 | |
| 367 | brca | BRCA1 | f | 35 | |
| 368 | brca | BRCA1 | f | 56 | |
| 369 | brca | BRCA1 | f | 49 | |
| 370 | brca | BRCA1 | f | 44 | |
| 371 | brca | BRCA1 | f | 45 | |
| 372 | brca | BRCA1 | f | 37 | |
| 373 | brca | BRCA1 | f | 37 | |
| 374 | brca | BRCA1 | f | 29 | |
| 375 | brca | BRCA1 | f | 41 | |
| 376 | brca | DCIS | f | 59 | |
| 377 | brca | IDC | f | 61 | |
| 378 | brca | DCIS | f | 46 | |
| 379 | brca | DCIS | f | 38 | |
| 380 | brca | DCIS | f | 45 | |
| 381 | brca | DCIS | f | 46 | |
| 382 | benign breast | Normal Breast | f | 44 | |
| 383 | brca | DCIS | f | 58 | |
| 384 | brca | DCIS | f | 52 | |
| 385 | brca | DCIS | f | 60 | |
| 386 | brca | DCIS | f | 46 | |
| 387 | brca | DCIS | f | 60 | |
| 388 | brca | DCIS | f | 59 | |
| 389 | brca | DCIS | f | 60 | |
| 390 | brca | DCIS | f | 62 | |
| 391 | brca | DCIS | f | 39 | |
| 392 | brca | DCIS | f | 50 | |
| 393 | brca | DCIS | f | 62 | |

(continued)

| Sample No. | main class | subclass | sex | age | Menopausal status |
|---|---|---|---|---|---|
| 394 | brca | IDC | f | 84 | |
| 395 | benign breast | Normal Breast | f | 55 | |
| 396 | brca | DCIS | f | 41 | |
| 397 | brca | IDC | f | 32 | |
| 398 | brca | IDC | f | 49 | |
| 399 | brca | DCIS | f | 43 | |
| 400 | brca | DCIS | f | 48 | |
| 401 | brca | DCIS | f | 45 | |
| 402 | brca | IDC | f | 33 | |
| 403 | benign breast | Normal Breast | f | 71 | |
| 404 | benign breast | Normal Breast | f | 40 | |
| 405 | benign breast | Normal Breast | f | 40 | |
| 406 | brca | IDC | f | 42 | |
| 407 | brca | DCIS | f | - | |
| 408 | benign breast | Normal Breast | f | - | |
| 409 | brca | IDC | f | 58 | |
| 410 | brca | IDC | f | 70 | |
| 411 | brca | DCIS | f | 43 | |
| 412 | brca | DCIS | f | 83 | |
| 413 | brca | DCIS | f | 74 | |
| 414 | brca | DCIS | f | 28 | |
| 415 | benign breast | fibroadenoma | f | 38 | |
| 416 | benign breast | fibroadenoma | f | 39 | |
| 417 | benign breast | fibroadenoma | f | 39 | |
| 418 | benign breast | fibroadenoma | f | 38 | |
| 419 | benign breast | fibroadenoma | f | 51 | |
| 420 | benign breast | fibroadenoma | f | 58 | |
| 421 | benign breast | fibroadenoma | f | 50 | |
| 422 | benign breast | fibroadenoma | f | 58 | |
| 423 | benign breast | fibroadenoma | f | 39 | |
| 424 | benign breast | fibroadenoma | f | 41 | |
| 425 | benign breast | fibroadenoma | f | 15 | |
| 426 | benign breast | fibroadenoma | f | 42 | |
| 427 | benign breast | adenosis | f | 14 | |
| 428 | benign breast | fibroadenoma | f | 26 | |
| 429 | benign breast | Normal Breast | f | 31 | |
| 430 | benign breast | fibroadenoma | f | - | |
| 431 | benign breast | Normal Breast | f | 30 | |

(continued)

| Sample No. | main class | subclass | sex | age | Menopausal status |
|---|---|---|---|---|---|
| 432 | benign breast | fibroadenoma | f | 46 | |
| 433 | benign breast | fibroadenoma | f | 53 | |
| 434 | benign breast | fibroadenoma | f | 43 | |
| 435 | benign breast | fibroadenoma | f | 20 | |
| 436 | benign breast | fibroadenoma | f | 44 | |
| 437 | benign breast | Normal Breast | f | 45 | |
| 438 | benign breast | fibroadenoma | f | 27 | |
| 439 | benign breast | fibroadenoma | f | 63 | |
| 440 | benign breast | fibroadenoma | f | 44 | |
| 441 | benign breast | fibroadenoma | f | 40 | |
| 442 | benign breast | fibroadenoma | f | 45 | |
| 443 | benign breast | fibroadenoma | f | 41 | |
| 444 | benign breast | fibroadenoma | f | 41 | |
| 445 | benign breast | Normal Breast | f | 41 | |
| 446 | benign breast | fibroadenoma | f | 35 | |
| 447 | benign breast | fibroadenoma | f | 33 | |
| 448 | benign breast | fibroadenoma | f | - | |
| 449 | benign breast | fibroadenoma | f | 24 | |
| 450 | benign breast | fibroadenoma | f | - | |
| 451 | lymphocytes | lymphocytes | f | 77 | |
| 452 | lymphocytes | lymphocytes | m | 50 | |
| 453 | lymphocytes | lymphocytes | f | 74 | |
| 454 | other cancer | colorectal cancer | f | 0 | |
| 455 | other cancer | colorectal cancer | f | 0 | |
| 456 | lymphocytes | lymphocytes | f | 53 | |
| 457 | other cancer | colorectal cancer | f | 0 | |
| 458 | lymphocytes | lymphocytes | f | 72 | |
| 459 | other cancer | colorectal cancer | f | 0 | |
| 460 | other cancer | colorectal cancer | f | 0 | |
| 461 | lymphocytes | lymphocytes | f | 71 | |
| 462 | lymphocytes | lymphocytes | f | 69 | |
| 463 | lymphocytes | lymphocytes | f | 53 | |
| 464 | other cancer | colorectal cancer | f | 0 | |
| 465 | lymphocytes | lymphocytes | f | 51 | |
| 466 | other cancer | colorectal cancer | f | 0 | |
| 467 | lymphocytes | lymphocytes | f | 50 | |
| 468 | other cancer | colorectal cancer | f | 53 | |
| 469 | other cancer | colorectal cancer | f | 46 | |

(continued)

| Sample No. | main class | subclass | sex | age | Menopausal status |
|---|---|---|---|---|---|
| 470 | other cancer | colorectal cancer | f | 62 | |
| 471 | other cancer | colorectal cancer | f | 78 | |
| 472 | other cancer | colorectal cancer | f | 55 | |
| 473 | other cancer | colorectal cancer | f | 53 | |
| 474 | other cancer | colorectal cancer | f | 60 | |
| 475 | other cancer | colorectal cancer | f | 84 | |
| 476 | other cancer | colorectal cancer | f | 69 | |
| 477 | other cancer | colorectal cancer | f | 66 | |
| 478 | other cancer | colorectal cancer | f | NA | |
| 479 | other cancer | colorectal cancer | f | 45 | |
| 480 | other cancer | colorectal cancer | f | 75 | |
| 481 | other cancer | lung cancer | f | 69 | |
| 482 | other cancer | colorectal cancer | f | 73 | |
| 483 | other cancer | colorectal cancer | f | 73 | |
| 484 | other cancer | colorectal cancer | f | 72 | |
| 485 | other cancer | colorectal cancer | m | 72 | |
| 486 | other cancer | liver cancer | f | 66 | |
| 487 | other cancer | colorectal cancer | m | 88 | |
| 488 | other cancer | colorectal cancer | f | 65 | |
| 489 | other cancer | colorectal cancer | f | 54 | |
| 490 | other cancer | colorectal cancer | m | 76 | |
| 491 | other cancer | liver cancer | f | NA | |
| 492 | other cancer | stomach cancer | f | 82 | |
| 493 | other cancer | stomach cancer | f | 85 | |
| 494 | other cancer | stomach cancer | f | 60 | |
| 495 | other cancer | stomach cancer | f | 55 | |
| 496 | other cancer | stomach cancer | f | 56 | |
| 497 | other cancer | stomach cancer | f | 63 | |
| 498 | other cancer | cancer esophagus | f | 57 | |
| 499 | other cancer | cancer esophagus | f | 45 | |
| 500 | other cancer | cancer esophagus | f | 71 | |

Table 15: Sample grading

| Sample No. | T | N | M | grade | ER |
|---|---|---|---|---|---|
| 1 | | | | | |
| 2 | | | | | |
| 3 | | | | | |
| 4 | | | | | |

(continued)

| Sample No. | T | N | M | grade | ER |
|---|---|---|---|---|---|
| 5 | | | | | |
| 6 | | | | | |
| 7 | | | | | |
| 8 | | | | | |
| 9 | | | | | |
| 10 | | | | | |
| 11 | | | | | |
| 12 | | | | | |
| 13 | | | | | |
| 14 | | | | | |
| 15 | | | | | |
| 16 | | | | | |
| 17 | | | | | |
| 18 | | | | | |
| 19 | | | | | |
| 20 | | | | | |
| 21 | | | | | |
| 22 | | | | | |
| 23 | | | | | |
| 24 | | | | | |
| 25 | | | | | |
| 26 | | | | | |
| 27 | | | | | |
| 28 | | | | | |
| 29 | | | | | |
| 30 | | | | | |
| 31 | | | | | |
| 32 | | | | | |
| 33 | | | | | |
| 34 | | | | | |
| 35 | | | | | |
| 36 | | | | | |
| 37 | | | | | |
| 38 | | | | | |
| 39 | | | | | |
| 40 | | | | | |
| 41 | | | | | |
| 42 | | | | | |

(continued)

| Sample No. | T | N | M | grade | ER |
|---|---|---|---|---|---|
| 43 | | | | | |
| 44 | | | | | |
| 45 | | | | | |
| 46 | | | | | |
| 47 | | | | | |
| 48 | | | | | |
| 49 | | | | | |
| 50 | | | | | |
| 51 | | | | | |
| 52 | | | | | |
| 53 | | | | | |
| 54 | | | | | |
| 55 | | | | | |
| 56 | | | | | |
| 57 | | | | | |
| 58 | | | | | |
| 59 | | | | | |
| 60 | | | | | |
| 61 | | | | | |
| 62 | | | | | |
| 63 | | | | | |
| 64 | | | | | |
| 65 | | | | | |
| 66 | | | | | |
| 67 | | | | | |
| 68 | | | | | |
| 69 | | | | | |
| 70 | | | | | |
| 71 | | | | | |
| 72 | | | | | |
| 73 | | | | | |
| 74 | | | | | |
| 75 | | | | | |
| 76 | | | | | |
| 77 | | | | | |
| 78 | | | | | |
| 79 | | | | | |
| 80 | | | | | |

(continued)

| Sample No. | T | N | M | grade | ER |
|---|---|---|---|---|---|
| 81 | | | | | |
| 82 | | | | | |
| 83 | | | | | |
| 84 | | | | | |
| 85 | | | | | |
| 86 | | | | | |
| 87 | | | | | |
| 88 | | | | | |
| 89 | | | | | |
| 90 | | | | | |
| 91 | | | | | |
| 92 | | | | | |
| 93 | | | | | |
| 94 | | | | | |
| 95 | | | | | |
| 96 | | | | | |
| 97 | | | | | |
| 98 | | | | | |
| 99 | | | | | |
| 100 | | | | | |
| 101 | | | | | |
| 102 | | | | | |
| 103 | | | | | |
| 104 | | | | | |
| 105 | | | | | |
| 106 | | | | | |
| 107 | | | | | |
| 108 | | | | | |
| 109 | | | | | |
| 110 | | | | | |
| 111 | | | | | |
| 112 | | | | | |
| 113 | | | | | |
| 114 | | | | | |
| 115 | T1 | N0 | M0 | poor | pos |
| 116 | T1 | N1 | M0 | poor | pos |
| 117 | T1 | N1 | M0 | | neg |
| 118 | T1 | N1 | M0 | | pos |

(continued)

| Sample No. | T | N | M | grade | ER |
|---|---|---|---|---|---|
| 119 | T1 | N1 | M0 | poor | neg |
| 120 | T1 | N0 | M0 | poor | pos |
| 121 | T1 | N0 | M0 | poor | neg |
| 122 | T1 | N1 | M0 | poor | neg |
| 123 | T1 | N1 | M0 | poor | neg |
| 124 | T1 | N1 | M0 | poor | pos |
| 125 | T1 | N0 | M0 | poor | pos |
| 126 | T1 | N1 | M0 | poor | neg |
| 127 | T1 | N1 | M0 | poor | pos |
| 128 | T1 | N0 | M0 | poor | neg |
| 129 | T1 | N0 | M0 | poor | neg |
| 130 | T1 | N1 | M0 |  | pos |
| 131 | T1 | N0 | M0 |  | pos |
| 132 | T1 | N1 | M0 | mod | neg |
| 133 | T1 | N1 | M0 | poor | pos |
| 134 | T1 | N0 | M0 | mod | pos |
| 135 | T1 | N0 | M0 | poor | pos |
| 136 | T1 | N1 | M0 | poor | neg |
| 137 | T1 | N1 | M0 |  | pos |
| 138 | T1 | N1 | M0 | mod | pos |
| 139 | T1 | N1 | M0 | mod | neg |
| 140 | T1 | N0 | M0 |  | pos |
| 141 | T1 | N0 | M0 |  | pos |
| 142 | T1 | N0 | M0 |  | neg |
| 143 | T1 | N0 | M0 |  | pos |
| 144 | T1 | N0 | M0 |  | pos |
| 145 | T1 | N2 | M0 | poor | pos |
| 146 | T1 | N0 | M0 | mod | pos |
| 147 | T1 | N0 | M0 | poor | pos |
| 148 | T1 | N1 | M0 | poor | neg |
| 149 | T1 | N0 | M0 | mod | pos |
| 150 | T1 | N1 | M0 |  | neg |
| 151 | T1 | N1 | M0 | poor | pos |
| 152 | T1 | N1 | M0 | poor | neg |
| 153 | T1 | N1 | M0 | poor | pos |
| 154 | T1 | N0 | M0 | poor | pos |
| 155 | T1 | N0 | M0 |  | neg |
| 156 | T1 | N0 | M0 | poor | neg |

(continued)

| Sample No. | T | N | M | grade | ER |
|---|---|---|---|---|---|
| 157 | T1 | N0 | M0 | | neg |
| 158 | T1 | N0 | M0 | poor | neg |
| 159 | T1 | N1 | M0 | | neg |
| 160 | T1 | N0 | M0 | poor | pos |
| 161 | T1 | N1 | M0 | poor | neg |
| 162 | T1 | N1 | M0 | | pos |
| 163 | T1 | N0 | M0 | | pos |
| 164 | T1 | N0 | M0 | poor | pos |
| 165 | T1 | N1 | M0 | poor | pos |
| 166 | T1 | N1 | M0 | poor | pos |
| 167 | T1 | N0 | M0 | poor | neg |
| 168 | T1 | N0 | M0 | poor | pos |
| 169 | T1 | N0 | M0 | poor | neg |
| 170 | T1 | N1 | M0 | poor | neg |
| 171 | T1 | N0 | M0 | poor | pos |
| 172 | T1 | N0 | M0 | poor | pos |
| 173 | T1 | N1 | M0 | | pos |
| 174 | T1 | N0 | M0 | poor | pos |
| 175 | T1 | N0 | M0 | | neg |
| 176 | T1 | N0 | M0 | | pos |
| 177 | T1 | N0 | M0 | | pos |
| 178 | T1 | N0 | M0 | mod | pos |
| 179 | T1 | N0 | M0 | poor | neg |
| 180 | T1 | N0 | M0 | poor | neg |
| 181 | T1 | N0 | M0 | poor | neg |
| 182 | T1 | N2 | M0 | | pos |
| 183 | T1 | N0 | M0 | poor | pos |
| 184 | T1 | N0 | M0 | | pos |
| 185 | T1 | N0 | M0 | | pos |
| 186 | T1 | N0 | M0 | | pos |
| 187 | T1 | N1 | M0 | | pos |
| 188 | T1 | N2 | M0 | poor | pos |
| 189 | T1 | N0 | M0 | | pos |
| 190 | T1 | N0 | M0 | poor | pos |
| 191 | T1 | N0 | M0 | | neg |
| 192 | T1 | N1 | M0 | poor | pos |
| 193 | T1 | N2 | M0 | poor | pos |
| 194 | T1 | N1 | M0 | | pos |

(continued)

| Sample No. | T | N | M | grade | ER |
|---|---|---|---|---|---|
| 195 | T1 | N0 | M0 | poor | neg |
| 196 | T1 | N1 | M0 |  | pos |
| 197 | T1 | N1 | M0 |  | pos |
| 198 | T1 | N1 | M0 | good | neg |
| 199 | T1 | N1 | M0 |  | neg |
| 200 | T1 | N0 | M0 | poor | neg |
| 201 | T1 | N0 | M0 |  | neg |
| 202 | T1 | N1 | M0 |  | pos |
| 203 | T1 | N0 | M0 | mod | pos |
| 204 | T1 | N0 | M0 |  | pos |
| 205 | T1 | N0 | M0 |  | pos |
| 206 | T1 | N0 | M0 |  | neg |
| 207 | T1 | N1 | M0 |  | neg |
| 208 | T1 | N1 | M0 |  | pos |
| 209 | T1 | N1 | M0 | poor | pos |
| 210 | T1 | N1 | M0 | poor | neg |
| 211 | T1 | N1 | M0 | poor | neg |
| 212 | T1 | N1 | M0 | poor | pos |
| 213 | T1 | N1 | M0 | poor | neg |
| 214 | T1 | N1 | M0 | poor | pos |
| 215 | T1 | N1 | M0 | poor | neg |
| 216 | T1 | N2 | M0 | poor | neg |
| 217 | T1 | N1 | M0 | mod | pos |
| 218 | T1 | N1 | M0 | poor | neg |
| 219 | T1 | N1 | M0 |  | pos |
| 220 | T1 | N0 | M0 | poor | neg |
| 221 | T1 | N0 | M0 | mod | pos |
| 222 | T1 | N0 | M0 | poor | neg |
| 223 | T1 | N0 | M0 | mod | pos |
| 224 | T1 | N0 | M0 | poor | neg |
| 225 | T1 | N0 | M0 |  | neg |
| 226 | T1 | N0 | M0 | poor | neg |
| 227 | T1 | N1 | M0 | poor | pos |
| 228 | T1 | N0 | M0 | poor | neg |
| 229 | T1 | N0 | M0 |  | pos |
| 230 | T1 | N1 | M0 | poor | pos |
| 231 | T1 | N1 | M0 | poor | neg |
| 232 | T1 | N0 | M0 | poor | neg |

(continued)

| Sample No. | T | N | M | grade | ER |
|---|---|---|---|---|---|
| 233 | T1 | N2 | M0 | poor | pos |
| 234 | T1 | N1 | M0 | | pos |
| 235 | T1 | N0 | M0 | poor | pos |
| 236 | T1 | N1 | M0 | | pos |
| 237 | T1 | N0 | M0 | | pos |
| 238 | T1 | N2 | M0 | mod | pos |
| 239 | T1 | N0 | M0 | | pos |
| 240 | T1 | N0 | M0 | | neg |
| 241 | T1 | N0 | M0 | poor | pos |
| 242 | T1 | N1 | M0 | | pos |
| 243 | T1 | N0 | M0 | | pos |
| 244 | T1 | N 1 | M0 | poor | pos |
| 245 | T1 | N0 | M0 | poor | pos |
| 246 | T1 | N2 | M0 | poor | pos |
| 247 | T1 | N1 | M0 | | pos |
| 248 | T1 | N0 | M0 | poor | neg |
| 249 | T1 | N0 | M0 | poor | neg |
| 250 | T1 | N1 | M0 | mod | neg |
| 251 | T1 | N1 | M0 | poor | pos |
| 252 | T1 | N1 | M0 | poor | neg |
| 253 | T1 | N0 | M0 | poor | pos |
| 254 | T1 | N1 | M0 | poor | neg |
| 255 | T1 | N1 | M0 | | pos |
| 256 | T1 | N0 | M0 | mod | pos |
| 257 | T1 | N1 | M0 | | pos |
| 258 | T1 | N1 | M0 | poor | pos |
| 259 | T1 | N1 | M0 | mod | pos |
| 260 | T1 | N0 | M0 | poor | pos |
| 261 | T1 | N0 | M0 | | pos |
| 262 | T1 | N0 | M0 | poor | neg |
| 263 | T1 | N0 | M0 | poor | pos |
| 264 | T1 | N1 | M0 | | neg |
| 265 | T1 | N0 | M0 | poor | neg |
| 266 | T1 | N1 | M0 | poor | pos |
| 267 | T1 | N0 | M0 | | neg |
| 268 | T1 | N1 | M0 | poor | pos |
| 269 | T1 | N1 | M0 | poor | pos |
| 270 | T1 | N0 | M0 | mod | pos |

(continued)

| Sample No. | T | N | M | grade | ER |
|---|---|---|---|---|---|
| 271 | T1 | N1 | M0 | poor | pos |
| 272 | T1 | N0 | M0 | poor | pos |
| 273 | T1 | N1 | M0 | mod | neg |
| 274 | T1 | N1 | M0 | poor | neg |
| 275 | T1 | N1 | M0 | poor | pos |
| 276 | T1 | N1 | M0 | mod | pos |
| 277 | T1 | N0 | M0 | | pos |
| 278 | T1 | N0 | M0 | good | neg |
| 279 | T1 | N0 | M0 | poor | neg |
| 280 | T1 | N0 | M0 | | pos |
| 281 | T1 | N0 | M0 | mod | pos |
| 282 | T1 | N0 | M0 | mod | pos |
| 283 | T1 | N0 | M0 | poor | neg |
| 284 | T1 | N0 | M0 | poor | neg |
| 285 | T1 | N0 | M0 | poor | neg |
| 286 | T1 | N0 | M0 | poor | pos |
| 287 | T1 | N0 | M0 | | pos |
| 288 | T1 | N0 | M0 | poor | neg |
| 289 | T1 | N1 | M0 | poor | pos |
| 290 | T1 | N1 | M0 | poor | neg |
| 291 | T1 | N1 | M0 | poor | neg |
| 292 | T1 | N1 | M0 | poor | neg |
| 293 | T1 | N1 | M0 | poor | neg |
| 294 | T1 | N2 | M0 | poor | neg |
| 295 | T1 | N1 | M0 | poor | pos |
| 296 | T1 | N1 | M0 | mod | neg |
| 297 | T1 | N0 | M0 | | neg |
| 298 | T1 | N1 | M0 | | neg |
| 299 | T1 | N0 | M0 | | neg |
| 300 | T1 | N0 | M0 | poor | neg |
| 301 | T1 | N1 | M0 | | pos |
| 302 | T1 | N2 | M0 | poor | neg |
| 303 | T1 | N0 | M0 | mod | pos |
| 304 | T1 | N1 | M0 | poor | neg |
| 305 | T1 | N1 | M0 | poor | pos |
| 306 | T1 | N1 | M0 | | neg |
| 307 | T1 | N0 | M0 | mod | pos |
| 308 | T1 | N0 | M0 | poor | neg |

(continued)

| Sample No. | T | N | M | grade | ER |
|---|---|---|---|---|---|
| 309 | T1 | N1 | M0 | poor | neg |
| 310 | T1 | N1 | M0 | poor | neg |
| 311 | T1 | N1 | M0 | | neg |
| 312 | T1 | N1 | M0 | | neg |
| 313 | T1 | N2 | M0 | poor | neg |
| 314 | T1 | N0 | M0 | | pos |
| 315 | T1 | N0 | M0 | mod | neg |
| 316 | T1 | N1 | M0 | poor | pos |
| 317 | T1 | N1 | M0 | poor | neg |
| 318 | T1 | N0 | M0 | mod | pos |
| 319 | T1 | N0 | M0 | poor | pos |
| 320 | T1 | N0 | M0 | | pos |
| 321 | T1 | N0 | M0 | mod | neg |
| 322 | T1 | N0 | M0 | poor | neg |
| 323 | T1 | N1 | M0 | | pos |
| 324 | T1 | N1 | M0 | poor | neg |
| 325 | T1 | N0 | M0 | good | pos |
| 326 | T1 | N0 | M0 | | pos |
| 327 | T1 | N0 | M0 | poor | neg |
| 328 | T1 | N0 | M0 | poor | neg |
| 329 | T1 | N1 | M0 | | pos |
| 330 | T1 | N0 | M0 | mod | pos |
| 331 | T1 | N1 | M0 | mod | pos |
| 332 | T1 | N1 | M0 | poor | neg |
| 333 | T1 | N0 | M0 | | neg |
| 334 | T1 | N0 | M0 | mod | neg |
| 335 | Tis | N0 | Mx | | pos |
| 336 | Tis | N0 | Mx | | |
| 337 | Tis | N0 | Mx | | |
| 338 | | | | | |
| 339 | | | | | |
| 340 | | | | | |
| 341 | | | | | |
| 342 | | | | | |
| 343 | | | | | |
| 344 | | | | | |
| 345 | | | | | |
| 346 | | | | | |

(continued)

| Sample No. | T | N | M | grade | ER |
|---|---|---|---|---|---|
| 347 | | | | | |
| 348 | | | | | |
| 349 | | | | | |
| 350 | | | | | |
| 351 | | | | | |
| 352 | | | | | |
| 353 | T1 | N0 | M0 | poor | |
| 354 | | | | poor | |
| 355 | | | | | |
| 356 | T1 | N0 | M0 | poor | |
| 357 | T1 | N0 | M0 | 2e prim,unknown | |
| 358 | T1 | N0 | M0 | poor | |
| 359 | T1 | N0 | M0 | unknown | |
| 360 | T1 | N0 | M0 | | |
| 361 | T1 | N0 | M0 | poor | |
| 362 | T1 | N0 | M0 | | |
| 363 | T1 | N0 | M0 | LR | |
| 364 | T1 | N0 | M0 | poor | |
| 365 | T1 | N0 | M0 | poor | |
| 366 | T1 | N0 | M0 | poor | |
| 367 | T1 | N0 | M0 | | |
| 368 | T1 | N0 | M0 | | |
| 369 | T1 | N0 | M0 | | |
| 370 | T1 | N0 | M0 | missing | |
| 371 | T1 | N0 | M0 | poor | |
| 372 | T1 | N0 | M0 | poor | |
| 373 | T1 | N0 | M0 | mod | |
| 374 | T1 | N0 | M0 | poor | |
| 375 | T1 | N0 | M0 | | |
| 376 | | | | | |
| 377 | | | | | |
| 378 | | | | | |
| 379 | | | | | |
| 380 | | | | | |
| 381 | | | | | |
| 382 | | | | | |
| 383 | | | | poor | |
| 384 | | | | poor | |

(continued)

| Sample No. | T | N | M | grade | ER |
|---|---|---|---|---|---|
| 385 | | | | | |
| 386 | | | | | |
| 387 | | | | | |
| 388 | | | | | |
| 389 | | | | poor | |
| 390 | | | | | |
| 391 | | | | | |
| 392 | | | | | |
| 393 | | | | | |
| 394 | | | | | |
| 395 | | | | | |
| 396 | | | | | |
| 397 | | | | | |
| 398 | | | | | |
| 399 | | | | | |
| 400 | | | | | |
| 401 | | | | | |
| 402 | | | | | |
| 403 | | | | | |
| 404 | | | | | |
| 405 | | | | | |
| 406 | | | | LR, poor | |
| 407 | | | | | |
| 408 | | | | | |
| 409 | | | | | |
| 410 | | | | | |
| 411 | | | | | |
| 412 | | | | | |
| 413 | | | | | |
| 414 | | | | poor | |
| 415 | | | | | |
| 416 | | | | | |
| 417 | | | | | |
| 418 | | | | | |
| 419 | | | | | |
| 420 | | | | | |
| 421 | | | | | |
| 422 | | | | | |

(continued)

| Sample No. | T | N | M | grade | ER |
|---|---|---|---|---|---|
| 423 | | | | | |
| 424 | | | | | |
| 425 | | | | | |
| 426 | | | | | |
| 427 | | | | | |
| 428 | | | | | |
| 429 | | | | | |
| 430 | | | | | |
| 431 | | | | | |
| 432 | | | | | |
| 433 | | | | | |
| 434 | | | | | |
| 435 | | | | | |
| 436 | | | | | |
| 437 | | | | | |
| 438 | | | | | |
| 439 | | | | | |
| 440 | | | | | |
| 441 | | | | | |
| 442 | | | | | |
| 443 | | | | | |
| 444 | | | | | |
| 445 | | | | | |
| 446 | | | | | |
| 447 | | | | | |
| 448 | | | | | |
| 449 | | | | | |
| 450 | | | | | |
| 451 | | | | | |
| 452 | | | | | |
| 453 | | | | | |
| 454 | | | | | |
| 455 | | | | | |
| 456 | | | | | |
| 457 | | | | | |
| 458 | | | | | |
| 459 | | | | | |
| 460 | | | | | |

(continued)

| Sample No. | T | N | M | grade | ER |
|---|---|---|---|---|---|
| 461 | | | | | |
| 462 | | | | | |
| 463 | | | | | |
| 464 | | | | | |
| 465 | | | | | |
| 466 | | | | | |
| 467 | | | | | |
| 468 | | | | | |
| 469 | | | | | |
| 470 | | | | | |
| 471 | | | | | |
| 472 | | | | | |
| 473 | | | | | |
| 474 | | | | | |
| 475 | | | | | |
| 476 | | | | | |
| 477 | | | | | |
| 478 | | | | | |
| 479 | | | | | |
| 480 | | | | | |
| 481 | | | | | |
| 482 | | | | | |
| 483 | | | | | |
| 484 | | | | | |
| 485 | | | | | |
| 486 | | | | | |
| 487 | | | | | |
| 488 | | | | | |
| 489 | | | | | |
| 490 | | | | | |
| 491 | | | | | |
| 492 | | | | | |
| 493 | | | | | |
| 494 | | | | | |
| 495 | | | | | |
| 496 | | | | | |
| 497 | | | | | |
| 498 | | | | | |

(continued)

| Sample No. | T | N | M | grade | ER |
|---|---|---|---|---|---|
| 499 | | | | | |
| 500 | | | | | |

Table 16: Breast cancer vs. all other controls

| No: | Gene | Oligo: |
|---|---|---|
| 1 | SCGB3A1 (SEQ ID NO: 115) | TAGTGTTCGACGTTGT (SEQ ID NO: 1475) |
| 2 | SCGB3A1 (SEQ ID NO: 115) | TAGTGTTTGATGTTGTT (SEQ ID NO: 1476) |
| 3 | SASH1 (SEQ ID NO: 102) | TAGATTCGAGGTGCGG (SEQ ID NO: 1477) |
| 4 | SASH1 (SEQ ID NO: 102) | TAGATTTGAGGTGTGG (SEQ ID NO: 1478) |
| 5 | SASH1 (SEQ ID NO: 102) | TAGATTCGAGGTGCGG (SEQ ID NO: 1477) |
| 6 | SASH1 (SEQ ID NO: 102) | TAGATTTGAGGTGTGG (SEQ ID NO: 1478) |
| 7 | SASH1 (SEQ ID NO: 102) | ATTCGGTATTCGGGTAG (SEQ ID NO: 1479) |
| 8 | SASH1 (SEQ ID NO: 102) | ATTTGGTATTTGGGTAG (SEQ ID NO: 1480) |
| 9 | SASH1 (SEQ ID NO: 102) | ATTCGGTATTCGGGTAG (SEQ ID NO: 1479) |
| 10 | SASH1 (SEQ ID NO: 102) | ATTTGGTATTTGGGTAG (SEQ ID NO: 1480) |
| 11 | SASH1 (SEQ ID NO: 102) | AGTCGGAATCGGAGTT (SEQ ID NO: 1481) |
| 12 | SASH1 (SEQ ID NO: 102) | GTTGGAATTGGAGTTTA (SEQ ID NO: 1482) |
| 13 | ARH1/NOEY2 (SEQ ID NO: 97) | TGTTATCGTTAACGATT (SEQ ID NO: 1483) |
| 14 | ARH1/NOEY2 (SEQ ID NO: 97) | AGGTGTTATTGTTAATGA (SEQ ID NO: 1484) |
| 15 | ARH1/NOEY2 (SEQ ID NO: 97) | TAAAACGTTCGGTAGG (SEQ ID NO: 1485) |
| 16 | ARH1/NOEY2 (SEQ ID NO: 97) | TTTAAAATGTTTGGTAGG (SEQ ID NO: 1486) |
| 17 | ARH1/NOEY2 (SEQ ID NO: 97) | TGTATTCGTCGTTAGG (SEQ ID NO: 1487) |
| 18 | ARH1/NOEY2 (SEQ ID NO: 97) | AATGTATTTGTTGTTAGG (SEQ ID NO: 1488) |
| 19 | ARH1/NOEY2 (SEQ ID NO: 97) | TTAGACGGAGTTCGGA (SEQ ID NO: 1489) |
| 20 | ARH1/NOEY2 (SEQ ID NO: 97) | TAGATGGAGTTTGGAGA (SEQ ID NO: 1490) |
| 21 | ARH1/NOEY2 (SEQ ID NO: 97) | TAGACGTAGGCGTATT (SEQ ID NO: 1491) |
| 22 | ARH1/NOEY2 (SEQ ID NO: 97) | GGGTAGATGTAGGTGT (SEQ ID NO: 1492) |
| 23 | CCND2 (SEQ ID NO: 104) | TTAGGGTCGATCGTGT (SEQ ID NO: 1493) |
| 24 | CCND2 (SEQ ID NO: 104) | TAGGGTTGATTGTGTT (SEQ ID NO: 1494) |
| 25 | CCND2 (SEQ ID NO: 104) | TTATCGTAGTCGGTTT (SEQ ID NO: 1495) |
| 26 | CCND2 (SEQ ID NO: 104) | GATTTTATTGTAGTTGGT (SEQ ID NO: 1496) |
| 27 | CCND2 (SEQ ID NO: 104) | GAGTGAGGCGCGAAAT (SEQ ID NO: 1497) |
| 28 | CCND2 (SEQ ID NO: 104) | GAGTGAGGTGTGAAAT (SEQ ID NO: 1498) |
| 29 | CCND2 (SEQ ID NO: 104) | GAGTGAGGCGCGAAAT (SEQ ID NO: 1497) |
| 30 | CCND2 (SEQ ID NO: 104) | GAGTGAGGTGTGAAAT (SEQ ID NO: 1498) |
| 31 | CCND2 (SEQ ID NO: 104) | AAGGATCGAGCGTGGA (SEQ ID NO: 1499) |
| 32 | CCND2 (SEQ ID NO: 104) | AAGGATTGAGTGTGGA (SEQ ID NO: 1500) |
| 33 | CCND2 (SEQ ID NO: 104) | AAGGATCGAGCGTGGA (SEQ ID NO: 1499) |

(continued)

| No: | Gene | Oligo: |
|---|---|---|
| 34 | CCND2 (SEQ ID NO: 104) | AAGGATTGAGTGTGGA (SEQ ID NO: 1500) |
| 35 | CDKN1A (SEQ ID NO: 67) | ATTAGGGTCGCGTTGA (SEQ ID NO: 1501) |
| 36 | CDKN1A (SEQ ID NO: 67) | ATTAGGGTTGTGTTGA (SEQ ID NO: 1502) |
| 37 | CDKN1A (SEQ ID NO: 67) | ATTAGGGTCGCGTTGA (SEQ ID NO: 1501) |
| 38 | CDKN1A (SEQ ID NO: 67) | ATTAGGGTTGTGTTGA (SEQ ID NO: 1502) |
| 39 | CDKN2A (SEQ ID NO: 57) | GGCGTTGTTTAACGTAT (SEQ ID NO: 1503) |
| 40 | CDKN2A (SEQ ID NO: 57) | GGGTGTTGTTTAATGTA (SEQ ID NO: 1504) |
| 41 | CDKN2A (SEQ ID NO: 57) | AATAGTTACGGTCGGA (SEQ ID NO: 1505) |
| 42 | CDKN2A (SEQ ID NO: 57) | AGTTATGGTTGGAGGT (SEQ ID NO: 1506) |
| 43 | CDKN2A (SEQ ID NO: 57) | GTCGGAGGTCGATTTA (SEQ ID NO: 1507) |
| 44 | CDKN2A (SEQ ID NO: 57) | GGTTGGAGGTTGATTTA (SEQ ID NO: 1508) |
| 45 | DAPK1 (SEQ ID NO: 98) | TTTCGGAGATTCGGTT (SEQ ID NO: 1509) |
| 46 | DAPK1 (SEQ ID NO: 98) | TTTGGAGATTTGGTTTT (SEQ ID NO: 1510) |
| 47 | DAPK1 (SEQ ID NO: 98) | TTTTAGCGTCGGGGAG (SEQ ID NO: 1511) |
| 48 | DAPK1 (SEQ ID NO: 98) | TTTTAGTGTTGGGGAG (SEQ ID NO: 1512) |
| 49 | DAPK1 (SEQ ID NO: 98) | TTTTAGCGTCGGGGAG (SEQ ID NO: 1511) |
| 50 | DAPK (SEQ ID NO: 98) | TTTTAGTGTTGGGGAG (SEQ ID NO: 1512) |
| 51 | EYA4 (SEQ ID NO: 58) | GGTATAAAATCGTAAATTTT (SEQ ID NO: 1513) |
| 52 | EYA4 (SEQ ID NO: 58) | GGGTATAAAATTGTAAATTT (SEQ ID NO: 1514) |
| 53 | EYA4 (SEQ ID NO: 58) | TATGTAGTCGCGTAGT (SEQ ID NO: 1515) |
| 54 | EYA4 (SEQ ID NO: 58) | TTTATGTAGTTGTGTAGT (SEQ ID NO: 1516) |
| 55 | EYA4 (SEQ ID NO: 58) | GTTTAGATACGAAATGTT (SEQ ID NO: 1517) |
| 56 | EYA4 (SEQ ID NO: 58) | GTTTAGATATGAAATGTTAT (SEQ ID NO: 1518) |
| 57 | EYA4 (SEQ ID NO: 58) | AGTTTTGACGTCGTTT (SEQ ID NO: 1519) |
| 58 | EYA4 (SEQ ID NO: 58) | TTGATGTTGTTTTGGAA (SEQ ID NO: 1520) |
| 59 | FHIT (SEQ ID NO: 76) | GTTACGTTAGCGGGTT (SEQ ID NO: 1521) |
| 60 | FHIT (SEQ ID NO: 76) | GGTTATGTTAGTGGGT (SEQ ID NO: 1522) |
| 61 | FHIT (SEQ ID NO: 76) | TTCGGGGACGTTATAG (SEQ ID NO: 1523) |
| 62 | FHIT (SEQ ID NO: 76) | GGTTTGGGGATGTTAT (SEQ ID NO: 1524) |
| 63 | GSTPI1 (SEQ ID NO: 59) | GGCGATTTCGGGGATT (SEQ ID NO: 1525) |
| 64 | GSTP1 (SEQ ID NO: 59) | GGTGATTTTGGGGATTT (SEQ ID NO: 1526) |
| 65 | GSTP1 (SEQ ID NO: 59) | GACGTTCGGGGTGTAG (SEQ ID NO: 1527) |
| 66 | GSTP1 (SEQ ID NO: 59) | GATGTTTGGGGTGTAG (SEQ ID NO: 1528) |
| 67 | GSTP1 (SEQ ID NO: 59) | GACGTTCGGGGTGTAG (SEQ ID NO: 1527) |
| 68 | GSTP1 (SEQ ID NO: 59) | GATGTTTGGGGTGTAG (SEQ ID NO: 1528) |
| 69 | GSTP1 (SEQ ID NO: 59) | AGTTCGCGGGATTTTT (SEQ ID NO: 1529) |
| 70 | GSTP1 (SEQ ID NO: 59) | GGAGTTTGTGGGATTT (SEQ ID NO: 1530) |
| 71 | GSTP1 (SEQ ID NO: 59) | AGTTTTCGTTATTAGTGA (SEQ ID NO: 1531) |

(continued)

| No: | Gene | Oligo: |
|-----|------|--------|
| 72 | GSTP1 (SEQ ID NO: 59) | TAGTTTTTGTTATTAGTGA (SEQ ID NO: 1532) |
| 73 | HIC1 (SEQ ID NO: 85) | TATCGAAGTTTTCGGG (SEQ ID NO: 1533) |
| 74 | HIC1 (SEQ ID NO: 85) | TATTGAAGTTTTTGGGT (SEQ ID NO: 1534) |
| 75 | HIC1 (SEQ ID NO: 85) | TAGCGGTTATTTCGGT (SEQ ID NO: 1535) |
| 76 | HIC1 (SEQ ID NO: 85) | TTTAGTGGTTATTTTGGT (SEQ ID NO: 1536) |
| 77 | HIC1 (SEQ ID NO: 85) | TACGTTTTTCGTAGCGT (SEQ ID NO: 1537) |
| 78 | HIC1 (SEQ ID NO: 85) | ATTATGTTTTTTGTAGTGT (SEQ ID NO: 1538) |
| 79 | HIC1 (SEQ ID NO: 85) | TTCGGTTTTGTCGTATA (SEQ ID NO: 1539) |
| 80 | HIC1 (SEQ ID NO: 85) | TTTGGTTTTGTTGTATAG (SEQ ID NO: 1540) |
| 81 | MLH1 (SEQ ID NO: 89) | AGGCGGCGATAGATTA (SEQ ID NO: 1541) |
| 82 | MLH1 (SEQ ID NO: 89) | ATGAGGTGGTGATAGA (SEQ ID NO: 1542) |
| 83 | PGR (SEQ ID NO: 83) | TTTCGAGGTCGGATTT (SEQ ID NO: 1543) |
| 84 | PGR (SEQ ID NO: 83) | TTTGAGGTTGGATTTTT (SEQ ID NO: 1544) |
| 85 | PGR (SEQ ID NO: 83) | GTGTCGTTAGTCGTA (SEQ ID NO: 1545) |
| 86 | PGR (SEQ ID NO: 83) | GTTGTTTAGTTGTAGGT (SEQ ID NO: 1546) |
| 87 | PGR (SEQ ID NO: 83) | TTTTTTCGACGAAAAGA (SEQ ID NO: 1547) |
| 88 | PGR (SEQ ID NO: 83) | AGGATTTTTTTGATGAAA (SEQ ID NO: 1548) |
| 89 | SERPINB5 (SEQ ID NO: 68) | TTATTAACGTGTTTGAGA (SEQ ID NO: 1549) |
| 90 | SERPINB5 (SEQ ID NO: 68) | TTATTAATGTGTTTGAGAA (SEQ ID NO: 1550) |
| 91 | SERPINB5 (SEQ ID NO: 68) | ATTGTCGTACGTATGT (SEQ ID NO: 1551) |
| 92 | SERPINB5 (SEQ ID NO: 68) | AGAGGATTGTTGTATGTA (SEQ ID NO: 1552) |
| 93 | SERPINB5 (SEQ ID NO: 68) | TTTTTTGTTCGAATATGT (SEQ ID NO: 1553) |
| 94 | SERPINB5 (SEQ ID NO: 68) | TTTGTTTGAATATGTTGG (SEQ ID NO: 1554) |
| 95 | RARB (SEQ ID NO: 88) | ATGTCGAGAACGCGAG (SEQ ID NO: 1555) |
| 96 | RARB (SEQ ID NO: 88) | GGATGTTGAGAATGTGA (SEQ ID NO: 1556) |
| 97 | RARB (SEQ ID NO: 88) | AGCGATTCGAGTAGGG (SEQ ID NO: 1557) |
| 98 | RARB (SEQ ID NO: 88) | AGTGATTTGAGTAGGG (SEQ ID NO: 1558) |
| 99 | RARB (SEQ ID NO: 88) | AGCGATTCGAGTAGGG (SEQ ID NO: 1557) |
| 100 | RARB (SEQ ID NO: 88) | AGTGATTTGAGTAGGG (SEQ ID NO: 1558) |
| 101 | RARB (SEQ ID NO: 88) | TAGGATTCGGAACGTA (SEQ ID NO: 1559) |
| 102 | RARB (SEQ ID NO: 88) | GGTAGGATTTGGAATGT (SEQ ID NO: 1560) |
| 103 | SFN (SEQ ID NO: 69) | TAGTACGGTGTCGTAT (SEQ ID NO: 1561) |
| 104 | SFN (SEQ ID NO: 69) | GTTTAGTATGGTGTTGT (SEQ ID NO: 1562) |
| 105 | SFN (SEQ ID NO: 69) | TACGTATTTCGGGTTT (SEQ ID NO: 1563) |
| 106 | SFN (SEQ ID NO: 69) | TATTTATGTATTTTGGGTT (SEQ ID NO: 1564) |
| 107 | SFN (SEQ ID NO: 69) | TTCGTTTTTCGTAGGAG (SEQ ID NO: 1565) |
| 108 | SFN (SEQ ID NO: 69) | TTTGTTTTTTGTAGGAGA (SEQ ID NO: 1566) |
| 109 | TGFBR2 (SEQ ID NO: 93) | ATGGGGCGGACGAATA (SEQ ID NO: 1567) |

(continued)

| No: | Gene | Oligo: |
|-----|------|--------|
| 110 | TGFBR2 (SEQ ID NO: 93) | ATGGGGTGGATGAATA (SEQ ID NO: 1568) |
| 111 | TGFBR2 (SEQ ID NO: 93) | ATGGGGCGGACGAATA (SEQ ID NO: 1567) |
| 112 | TGFBR2 (SEQ ID NO: 93) | ATGGGGTGGATGAATA (SEQ ID NO: 1568) |
| 113 | THRB (SEQ ID NO: 106) | GGGCGGTTAAGTCGAG (SEQ ID NO: 1569) |
| 114 | THRB (SEQ ID NO: 106) | GGGTGGTTAAGTTGAG (SEQ ID NO: 1570) |
| 115 | THRB (SEQ ID NO: 106) | GGGCGGTTAAGTCGAG (SEQ ID NO: 1569) |
| 116 | THRB (SEQ ID NO: 106) | GGGTGGTTAAGTTGAG (SEQ ID NO: 1570) |
| 117 | TIMP3 (SEQ ID NO: 103) | TTATTAACGGAGGAAGG (SEQ ID NO: 1571) |
| 118 | TIMP3 (SEQ ID NO: 103) | TATTAATGGAGGAAGGG (SEQ ID NO: 1572) |
| 119 | TIMP3 (SEQ ID NO: 103) | TTCGTTATGTGTACGGAA (SEQ ID NO: 1573) |
| 120 | TIMP3 (SEQ ID NO: 103) | TTTGTTATGTGTATGGAA (SEQ ID NO: 1574) |
| 121 | TIMP3 (SEQ ID NO: 103) | TTCGTTATGTGTACGGAA (SEQ ID NO: 1573) |
| 122 | TIMP3 (SEQ ID NO: 103) | TTTGTTATGTGTATGGAA (SEQ ID NO: 1574) |
| 123 | TIMP3 (SEQ ID NO: 103) | GAGTATTTCGAGTTTGT (SEQ ID NO: 1575) |
| 124 | TIMP3 (SEQ ID NO: 103) | AGTATTTTGAGTTTGTATT (SEQ ID NO: 1576) |
| 125 | TIMP3 (SEQ ID NO: 103) | TAAGCGTTAATCGAGT (SEQ ID NO: 1577) |
| 126 | TIMP3 (SEQ ID NO: 103) | TAGGTAAGTGTTAATTGA (SEQ ID NO: 1578) |
| 127 | TP73 (SEQ ID NO: 86) | TAGGATTCGCGTTTTT (SEQ ID NO: 1579) |
| 128 | TP73 (SEQ ID NO: 86) | GGTAGGATTTGTGTTTT (SEQ ID NO: 1580) |
| 129 | TP73 (SEQ ID NO: 86) | TTTCGGAGTTGCGAGT (SEQ ID NO: 1581) |
| 130 | TP73 (SEQ ID NO: 86) | TAGTTTTGGAGTTGTGA (SEQ ID NO: 1582) |
| 131 | CDH13 (SEQ ID NO: 70) | TAAAACGAGGGAGCGT (SEQ ID NO: 1583) |
| 132 | CDH13 (SEQ ID NO: 70) | AAAATGAGGGAGTGTT (SEQ ID NO: 1584) |
| 133 | CDH13 (SEQ ID NO: 70) | TAGTCGCGTGTATGAA (SEQ ID NO: 1585) |
| 134 | CDH13 (SEQ ID NO: 70) | TGTAGTTGTGTGTATGA (SEQ ID NO: 1586) |
| 135 | CDH13 (SEQ ID NO: 70) | ATGAAAACGTCGTCGG (SEQ ID NO: 1587) |
| 136 | CDH13 (SEQ ID NO: 70) | AATGAAAATGTTGTTGG (SEQ ID NO: 1588) |
| 137 | CDH13 (SEQ ID NO: 70) | TAGTCGAGAATTTCGT (SEQ ID NO: 1589) |
| 138 | CDH13 (SEQ ID NO: 70) | TGTAGTTGAGAATTTTGT (SEQ ID NO: 1590) |
| 139 | TMS1/ASC (SEQ ID NO: 84) | TTCGTTTCGGAGTCGA (SEQ ID NO: 1591) |
| 140 | TMS1/ASC (SEQ ID NO: 84) | TTTGTTTTGGAGTTGAT (SEQ ID NO: 1592) |
| 141 | APAF1 (SEQ ID NO: 82) | GTGTCGTAGCGGTATT (SEQ ID NO: 1593) |
| 142 | APAF1 (SEQ ID NO: 82) | GGTGTTGTAGTGGTAT (SEQ ID NO: 1594) |
| 143 | APAF1 (SEQ ID NO: 82) | AGTAGCGTCGGGTTTT (SEQ ID NO: 1595) |
| 144 | APAF1 (SEQ ID NO: 82) | GAGTAGTGTTGGGTTT (SEQ ID NO: 1596) |
| 145 | SYK (SEQ ID NO: 60) | GAAGTTATCGCGTTGG (SEQ ID NO: 1597) |
| 146 | SYK (SEQ ID NO: 60) | AGAAGTTATTGTGTTGG (SEQ ID NO: 1598) |
| 147 | SYK (SEQ ID NO: 60) | GATCGATGCGGTTTAT (SEQ ID NO: 1599) |

(continued)

| No: | Gene | Oligo: |
|---|---|---|
| 148 | SYK (SEQ ID NO: 60) | GGGATTGATGTGGTTT (SEQ ID NO: 1600) |
| 149 | SYK (SEQ ID NO: 60) | GGCGTTTTAGTCGATT (SEQ ID NO: 1601) |
| 150 | SYK (SEQ ID NO: 60) | GGTGTTTTAGTTGATTTT (SEQ ID NO: 1602) |
| 151 | SYK (SEQ ID NO: 60) | TTATTCGGTCGGGATT (SEQ ID NO: 1603) |
| 152 | SYK (SEQ ID NO: 60) | TTTATTTGGTTGGGATT (SEQ ID NO: 1604) |
| 153 | FABP3 (SEQ ID NO: 77) | GATGGGCGTATTAGTT (SEQ ID NO: 1605) |
| 154 | FABP3 (SEQ ID NO: 77) | GGGATGGGTGTATTAG (SEQ ID NO: 1606) |
| 155 | FABP3 (SEQ ID NO: 77) | GTGATGCGAGGGTTAT (SEQ ID NO: 1607) |
| 156 | FABP3 (SEQ ID NO: 77) | GTGATGTGAGGGTTAT (SEQ ID NO: 1608) |
| 157 | FABP3 (SEQ ID NO: 77) | GTGATGCGAGGGTTAT (SEQ ID NO: 1607) |
| 158 | FABP3 (SEQ ID NO: 77) | GTGATGTGAGGGTTAT (SEQ ID NO: 1608) |
| 159 | FABP3 (SEQ ID NO: 77) | TAAAGCGGTAGTTCGG (SEQ ID NO: 1609) |
| 160 | FABP3 (SEQ ID NO: 77) | AAGTGGTAGTTTGGGT (SEQ ID NO: 1610) |
| 161 | FABP3 (SEQ ID NO: 77) | TATTGGCGTTGACGTA (SEQ ID NO: 1611) |
| 162 | FABP3 (SEQ ID NO: 77) | TGGTGTTGATGTAGGT (SEQ ID NO: 1612) |
| 163 | RASSF I A (SEQ ID NO: 90) | TACGGGTATTTTCGCGT (SEQ ID NO: 1613) |
| 164 | RASSF1A (SEQ ID NO: 90) | ATATGGGTATTTTTGTGT (SEQ ID NO: 1614) |
| 165 | RASSF1A (SEQ ID NO: 90) | AGAGCGCGTTTAGTTT (SEQ ID NO: 1615) |
| 166 | RASSF1A (SEQ ID NO: 90) | GAGAGTGTGTTTAGTTT (SEQ ID NO: 1616) |
| 167 | RASSF1A (SEQ ID NO: 90) | AGTAAATCGGATTAGGA (SEQ ID NO: 1617) |
| 168 | RASSF1A (SEQ ID NO: 90) | AGTAAATTGGATTAGGAG (SEQ ID NO: 1618) |
| 169 | TWIST (SEQ ID NO: 100) | AGTAAAGGCGTTGCGT (SEQ ID NO: 1619) |
| 170 | TWIST (SEQ ID NO: 100) | AGTAAAGGTGTTGTGT (SEQ ID NO: 1620) |
| 171 | TWIST (SEQ ID NO: 100) | AGTAAAGGCGTTGCGT (SEQ ID NO: 1619) |
| 172 | TWIST (SEQ ID NO: 100) | AGTAAAGGTGTTGTGT (SEQ ID NO: 1620) |
| 173 | TWIST (SEQ ID NO: 100) | TATTTTTCGAGGCGTA (SEQ ID NO: 1621) |
| 174 | TWIST (SEQ ID NO: 100) | TTTTGAGGTGTAGTTTT (SEQ ID NO: 1622) |
| 175 | TWIST (SEQ ID NO: 100) | ATTGGGTCGTTGTAGA (SEQ ID NO: 1623) |
| 176 | TWIST (SEQ ID NO: 100) | ATTGGGTTGTTGTAGA (SEQ ID NO: 1624) |
| 177 | TWIST (SEQ ID NO: 100) | ATTGGGTCGTTGTAGA (SEQ ID NO: 1623) |
| 178 | TWIST (SEQ ID NO: 100) | ATTGGGTTGTTGTAGA (SEQ ID NO: 1624) |
| 179 | TWIST (SEQ ID NO: 100) | TAGGTCGGGACGTAAA (SEQ ID NO: 1625) |
| 180 | TWIST (SEQ ID NO: 100) | AGTAGGTTGGGATGTA (SEQ ID NO: 1626) |
| 181 | ESR2 (SEQ ID NO: 91) | GAGTATTTTCGAATCGA (SEQ ID NO: 1627) |
| 182 | ESR2 (SEQ ID NO: 91) | GGAGTATTTTTGAATTGA (SEQ ID NO: 1628) |
| 183 | ESR2 (SEQ ID NO: 91) | ATAAGCGATTTAACGAT (SEQ ID NO: 1629) |
| 184 | ESR2 (SEQ ID NO: 91) | AAGTGATTTAATGATAAGT (SEQ ID NO: 1630) |
| 185 | ESR2 (SEQ ID NO: 91) | TTTACGTGATCGAGTT (SEQ ID NO: 1631) |

(continued)

| No: | Gene | Oligo: |
|---|---|---|
| 186 | ESR2 (SEQ ID NO: 91) | AGTTTATGTGATTGAGTT (SEQ ID NO: 1632) |
| 187 | PLAU (SEQ ID NO: 62) | TATTTGTCGCGTTGAT (SEQ ID NO: 1633) |
| 188 | PLAU (SEQ ID NO: 62) | ATTTGTTGTGTTGATGA (SEQ ID NO: 1634) |
| 189 | PLAU (SEQ ID NO: 62) | TGTAATTCGGGGATTT (SEQ ID NO: 1635) |
| 190 | PLAU (SEQ ID NO: 62) | TTGTAATTTGGGGATTT (SEQ ID NO: 1636) |
| 191 | PLAU (SEQ ID NO: 62) | TTGGAGATCGCGTTTT (SEQ ID NO: 1637) |
| 192 | PLAU (SEQ ID NO: 62) | TTGGAGATTGTGTTTTT (SEQ ID NO: 1638) |
| 193 | PLAU (SEQ ID NO: 62) | GAGCGTTGCGGAAGTA (SEQ ID NO: 1639) |
| 194 | PLAU (SEQ ID NO: 62) | GAGTGTTGTGGAAGTA (SEQ ID NO: 1640) |
| 195 | PLAU (SEQ ID NO: 62) | GAGCGTTGCGGAAGTA (SEQ ID NO: 1639) |
| 196 | PLAU (SEQ ID NO: 62) | GAGTGTTGTGGAAGTA (SEQ ID NO: 1640) |
| 197 | STAT1 (SEQ ID NO: 109) | GTTATTTTCGAGAGTTG (SEQ ID NO: 1641) |
| 198 | STAT1 (SEQ ID NO: 109) | GTTATTTTTGAGAGTTGT (SEQ ID NO: 1642) |
| 199 | BRCA1 (SEQ ID NO: 66) | AGTTTCGAGAGACGTT (SEQ ID NO: 1643) |
| 200 | BRCA1 (SEQ ID NO: 66) | AGAGTTTTGAGAGATGT (SEQ ID NO: 1644) |
| 201 | BRCA1 (SEQ ID NO: 66) | TTTCGTGGTAACGGAA (SEQ ID NO: 1645) |
| 202 | BRCA1 (SEQ ID NO: 66) | TTTGTGGTAATGGAAAA (SEQ ID NO: 1646) |
| 203 | BRCA1 (SEQ ID NO: 66) | AAAGCGCGGGAATTAT (SEQ ID NO: 1647) |
| 204 | BRCA1 (SEQ ID NO: 66) | GGAAAAGTGTGGGAAT (SEQ ID NO: 1648) |
| 205 | LOT1 (SEQ ID NO: 95) | ATGGGTACGTTTAAGG (SEQ ID NO: 1649) |
| 206 | LOT1 (SEQ ID NO: 95) | TGGGTATGTTTAAGGG (SEQ ID NO: 1650) |
| 207 | LOT1 (SEQ ID NO: 95) | AAATTAGTTACGTTATTTAA (SEQ ID NO: 1651) |
| 208 | LOT1 (SEQ ID NO: 95) | TGAAATTAGTTATGTTATTTA (SEQ ID NO: 1652) |
| 209 | LOT1 (SEQ ID NO: 95) | ATGTCGGTTATTACGT (SEQ ID NO: 1653) |
| 210 | LOT1 (SEQ ID NO: 95) | TGTTGGTTATTATGTAGA (SEQ ID NO: 1654) |
| 211 | PRSS8 (SEQ ID NO: 72) | AGTTGGCGGAGTTTAG (SEQ ID NO: 1655) |
| 212 | PRSS8 (SEQ ID NO: 72) | AGTTGGTGGAGTTTAG (SEQ ID NO: 1656) |
| 213 | PRSS8 (SEQ ID NO: 72) | AGTTGGCGGAGTTTAG (SEQ ID NO: 1655) |
| 214 | PRSS8 (SEQ ID NO: 72) | AGTTGGTGGAGTTTAG (SEQ ID NO: 1656) |
| 215 | PRSS8 (SEQ ID NO: 72) | TTGGTGATTCGTTATAT (SEQ ID NO: 1657) |
| 216 | PRSS8 (SEQ ID NO: 72) | GTTGGTGATTTGTTTATA (SEQ ID NO: 1658) |
| 217 | PRSS8 (SEQ ID NO: 72) | TGTTCGTTTCGGATAT (SEQ ID NO: 1659) |
| 218 | PRSS8 (SEQ ID NO: 72) | TTTGTTTTGGATATTTTAG (SEQ ID NO: 1660) |
| 219 | TPM1 (SEQ ID NO: 110) | TTGATTCGCGTTCGTA (SEQ ID NO: 1661) |
| 220 | TPM1 (SEQ ID NO: 110) | TGATTTGTGTTTGTAGA (SEQ ID NO: 1662) |
| 221 | SLC19A1 (SEQ ID NO: 116) | GTCGTGCGGTTTTTAA (SEQ ID NO: 1663) |
| 222 | SLC19A1 (SEQ ID NO: 116) | GGTTGTGTGGTTTTTAA (SEQ ID NO: 1664) |
| 223 | SLC19A1 (SEQ ID NO: 116) | TTACGAAGGCGGTTTA (SEQ ID NO: 1665) |

(continued)

| No: | Gene | Oligo: |
|-----|------|--------|
| 224 | SLC19A1 (SEQ ID NO: 116) | TTTTATGAAGGTGGTTT (SEQ ID NO: 1666) |
| 225 | GJB2 (SEQ ID NO: 111) | GGATTTCGTCGGTATT (SEQ ID NO: 1667) |
| 226 | GJB2 (SEQ ID NO: 111) | GGGGATTTTGTTGGTA (SEQ ID NO: 1668) |
| 227 | GJB2 (SEQ ID NO: 111) | GAATTTCGTTTACGGT (SEQ ID NO: 1669) |
| 228 | GJB2 (SEQ ID NO: 111) | TTGAATTTTGTTTATGGT (SEQ ID NO: 1670) |
| 229 | HS3ST2 (SEQ ID NO: 113) | GAATCGGAGAGGCGAG (SEQ ID NO: 1671) |
| 230 | HS3ST2 (SEQ ID NO: 113) | AATTGGAGAGGTGAGG (SEQ ID NO: 1672) |
| 231 | HS3ST2 (SEQ ID NO: 113) | GGGTAATCGTTTGGTA (SEQ ID NO: 1673) |
| 232 | HS3ST2 (SEQ ID NO: 113) | GGGTAATTGTTTGGTAT (SEQ ID NO: 1674) |
| 233 | PRDM2 (SEQ ID NO: 114) | TGTAGAGACGACGATT (SEQ ID NO: 1675) |
| 234 | PRDM2 (SEQ ID NO: 114) | ATTGTAGAGATGATGATT (SEQ ID NO: 1676) |
| 235 | PRDM2 (SEQ ID NO: 114) | AGAGCGCGGTAGTAGT (SEQ ID NO: 1677) |
| 236 | PRDM2 (SEQ ID NO: 114) | TGAGAGTGTGGTAGTA (SEQ ID NO: 1678) |
| 237 | PRDM2 (SEQ ID NO: 114) | TGTTCGCGATGTTTTA (SEQ ID NO: 1679) |
| 238 | PRDM2 (SEQ ID NO: 114) | TGTTTGTGATGTTTTAGT (SEQ ID NO: 1680) |
| 239 | PRDM2 (SEQ ID NO: 114) | AGTATATAAACGTAGATTTT (SEQ ID NO: 1681) |
| 240 | PRDM2 (SEQ ID NO: 114) | AAGTATATAAATGTAGATTTT (SEQ ID NO: 1682) |
| 241 | ALX4 (SEQ ID NO: 64) | AAGTCGATCGTTTTGT (SEQ ID NO: 1683) |
| 242 | ALX4 (SEQ ID NO: 64) | TGGAAGTTGATTGTTTT (SEQ ID NO: 1684) |
| 243 | ALX4 (SEQ ID NO: 64) | TATTGCGAGGATTCGG (SEQ ID NO: 1685) |
| 244 | ALX4 (SEQ ID NO: 64) | ATTGTGAGGATTTGGT (SEQ ID NO: 1686) |
| 245 | ALX4 (SEQ ID NO: 64) | TTCGTAGCGTAGGGTT (SEQ ID NO: 1687) |
| 246 | ALX4 (SEQ ID NO: 64) | TTTGTAGTGTAGGGTTT (SEQ ID NO: 1688) |
| 247 | S 100A7 (SEQ ID NO: 96) | TATAGTCGGGGTGATA (SEQ ID NO: 1689) |
| 248 | S 100A7 (SEQ ID NO: 96) | TTTATAGTTGGGGTGAT (SEQ ID NO: 1690) |
| 249 | S100A7 (SEQ ID NO: 96) | AGTCGGGCGTTAGTAA (SEQ ID NO: 1691) |
| 250 | S100A7 (SEQ ID NO: 96) | GAGTTGGGTGTTAGTA (SEQ ID NO: 1692) |
| 251 | S100A7 (SEQ ID NO: 96) | GGATGGCGGAAGTTTA (SEQ ID NO: 1693) |
| 252 | S 100A7 (SEQ ID NO: 96) | GGATGGTGGAAGTTTA (SEQ ID NO: 1694) |
| 253 | S100A7 (SEQ ID NO: 96) | GGATGGCGGAAGTTTA (SEQ ID NO: 1693) |
| 254 | S100A7 (SEQ ID NO: 96) | GGATGGTGGAAGTTTA (SEQ ID NO: 1694) |
| 255 | APC (SEQ ID NO: 65) | GATTCGTATTTCGTAGT (SEQ ID NO: 1695) |
| 256 | APC (SEQ ID NO: 65) | GATTCGTATTTCGTAGT (SEQ ID NO: 1695) |
| 257 | APC (SEQ ID NO: 65) | AGCGTTTTGGTTCGTAT (SEQ ID NO: 1696) |
| 258 | APC (SEQ ID NO: 65) | AGTGTTTTGGTTTGTAT (SEQ ID NO: 1697) |
| 259 | APC (SEQ ID NO: 65) | AGCGTTTTGGTTCGTAT (SEQ ID NO: 1696) |
| 260 | APC (SEQ ID NO: 65) | AGTGTTTTGGTTTGTAT (SEQ ID NO: 1697) |
| 261 | APC (SEQ ID NO: 65) | TTAATCGGCGGGTTTT (SEQ ID NO: 1698) |

(continued)

| No: | Gene | Oligo: |
|-----|------|--------|
| 262 | APC (SEQ ID NO: 65) | AGTTAATTGGTGGGTT (SEQ ID NO: 1699) |
| 263 | APC (SEQ ID NO: 65) | ATTTTCGAGTTCGGTA (SEQ ID NO: 1700) |
| 264 | APC (SEQ ID NO: 65) | TTTTTGAGTTTGGTAGT (SEQ ID NO: 1701) |
| 265 | BRCA2 (SEQ ID NO: 56) | ATTCGTTTTAGAGGCGTA (SEQ ID NO: 1702) |
| 266 | BRCA2 (SEQ ID NO: 56) | ATTTGTTTTAGAGGTGTA (SEQ ID NO: 1703) |
| 267 | BRCA2 (SEQ ID NO: 56) | ATTCGTTTTAGAGGCGTA (SEQ ID NO: 1702) |
| 268 | BRCA2 (SEQ ID NO: 56) | ATTTGTTTTAGAGGTGTA (SEQ ID NO: 1703) |
| 269 | SEQ ID NO:2 (SEQ ID NO: 2) | TAGGTATACGAAAGAGTA (SEQ ID NO: 1704) |
| 270 | SEQ ID NO:2 (SEQ ID NO: 2) | TTAGGTATATGAAAGAGTA (SEQ ID NO: 1705) |
| 271 | SEQ ID NO:2 (SEQ ID NO: 2) | TTGATTGGCGGACGAG (SEQ ID NO: 1706) |
| 272 | SEQ ID NO:2 (SEQ ID NO: 2) | TTGATTGGTGGATGAG (SEQ ID NO: 1707) |
| 273 | SEQ ID NO:2 (SEQ ID NO: 2) | TTGATTGGCGGACGAG (SEQ ID NO: 1706) |
| 274 | SEQ ID NO:2 (SEQ ID NO: 2) | TTGATTGGTGGATGAG (SEQ ID NO: 1707) |
| 275 | IGFBP7 (SEQ ID NO: 94) | TAGTCGCGGAATGTTA (SEQ ID NO: 1708) |
| 276 | IGFBP7 (SEQ ID NO: 94) | TTGGTAGTTGTGGAAT (SEQ ID NO: 1709) |
| 277 | IGFBP7 (SEQ ID NO: 94) | ATTTTTTCGCGGGTAT (SEQ ID NO: 1710) |
| 278 | IGFBP7 (SEQ ID NO: 94) | TTTTTGTGGGTATTTTAG (SEQ ID NO: 1711) |
| 279 | IGFBP7 (SEQ ID NO: 94) | GGTATATTCGACGGGG (SEQ ID NO: 1712) |
| 280 | IGFBP7 (SEQ ID NO: 94) | GGGTATATTTGATGGGG (SEQ ID NO: 1713) |
| 281 | IGFBP7 (SEQ ID NO: 94) | GGTACGAGCGTTTTTT (SEQ ID NO: 1714) |
| 282 | IGFBP7 (SEQ ID NO: 94) | TGGGTATGAGTGTTTT (SEQ ID NO: 1715) |
| 283 | IGFBP7 (SEQ ID NO: 94) | AAAGCGTATTTAATTCGT (SEQ ID NO: 1716) |
| 284 | IGFBP7 (SEQ ID NO: 94) | AGTGTATTTAATTTGTGTT (SEQ ID NO: 1717) |
| 285 | SOD2 (SEQ ID NO: 105) | GTCGTTTAGTCGGTTTA (SEQ ID NO: 1718) |
| 286 | SOD2 (SEQ ID NO: 105) | GTTGTTTAGTTGGTTTAT (SEQ ID NO: 1719) |
| 287 | SOD2 (SEQ ID NO: 105) | TATTAGGCGGTTGCGG (SEQ ID NO: 1720) |
| 288 | SOD2 (SEQ ID NO: 105) | TATTAGGTGGTTGTGG (SEQ ID NO: 1721) |
| 289 | SOD2 (SEQ ID NO: 105) | TATTAGGCGGTTGCGG (SEQ ID NO: 1720) |
| 290 | SOD2 (SEQ ID NO: 105) | TATTAGGTGGTTGTGG (SEQ ID NO: 1721) |
| 291 | SOD2 (SEQ ID NO: 105) | TACGGTTCGAAGGTTT (SEQ ID NO: 1722) |
| 292 | SOD2 (SEQ ID NO: 105) | AGTTGGTATGGTTTGA (SEQ ID NO: 1723) |
| 293 | NME1 (SEQ ID NO: 107) | AATTCGAGATTAGTTCGG (SEQ ID NO: 1724) |
| 294 | NME1 (SEQ ID NO: 107) | AATTTGAGATTAGTTTGG (SEQ ID NO: 1725) |
| 295 | NME1 (SEQ ID NO: 107) | AATTCGAGATTAGTTCGG (SEQ ID NO: 1724) |
| 296 | NME1 (SEQ ID NO: 107) | AATTTGAGATTAGTTTGG (SEQ ID NO: 1725) |
| 297 | THBS (SEQ ID NO: 81) | TAAAGGGGCGTTCGTA (SEQ ID NO: 1726) |
| 298 | THBS (SEQ ID NO: 81) | AAGGGGTGTTTGTATT (SEQ ID NO: 1727) |
| 299 | THBS1 (SEQ ID NO: 81) | GGTTAGTTCGGGCGTA (SEQ ID NO: 1728) |

(continued)

| No: | Gene | Oligo: |
|-----|------|--------|
| 300 | THBS1 (SEQ ID NO: 81) | GGTTAGTTTGGGTGTA (SEQ ID NO: 1729) |
| 301 | THBS1 (SEQ ID NO: 81) | GGTTAGTTCGGGCGTA (SEQ ID NO: 1728) |
| 302 | THBS1 (SEQ ID NO: 81) | GGTTAGTTTGGGTGTA (SEQ ID NO: 1729) |
| 303 | THBS1 (SEQ ID NO: 81) | TTGTGCGTTCGGAGTA (SEQ ID NO: 1730) |
| 304 | THBS1 (SEQ ID NO: 81) | TGTGTTTGGAGTAGAG (SEQ ID NO: 1731) |
| 305 | ESR1 (SEQ ID NO: 75) | AAATCGGCGGGTTATT (SEQ ID NO: 1732) |
| 306 | ESR1 (SEQ ID NO: 75) | AGAAATTGGTGGGTTA (SEQ ID NO: 1733) |
| 307 | ESR1 (SEQ ID NO: 75) | AGTTGCGGACGGTTTA (SEQ ID NO: 1734) |
| 308 | ESR1 (SEQ ID NO: 75) | AGTTGTGGATGGTTTA (SEQ ID NO: 1735) |
| 309 | ESR1 (SEQ ID NO: 75) | AGTTGCGGACGGTTTA (SEQ ID NO: 1734) |
| 310 | ESR1 (SEQ ID NO: 75) | AGTTGTGGATGGTTTA (SEQ ID NO: 1735) |
| 311 | IL6 (SEQ ID NO: 99) | TTCGGTTATACGTAGG (SEQ ID NO: 1736) |
| 312 | IL6 (SEQ ID NO: 99) | TTTTGGTTATATGTAGGG (SEQ ID NO: 1737) |
| 313 | IL6 (SEQ ID NO: 99) | AGTTTAGTCGGTTTCGT (SEQ ID NO: 1738) |
| 314 | IL6 (SEQ ID NO: 99) | AGTTTAGTTGGTTTTGT (SEQ ID NO: 1739) |
| 315 | IL6 (SEQ ID NO: 99) | AGTTTAGTCGGTTTCGT (SEQ ID NO: 1738) |
| 316 | IL6 (SEQ ID NO: 99) | AGTTTAGTTGGTTTTGT (SEQ ID NO: 1739) |
| 317 | CASP8 (SEQ ID NO: 71) | TGTATTCGAGGCGGTA (SEQ ID NO: 1740) |
| 318 | CASP8 (SEQ ID NO: 71) | TTGTATTTGAGGTGGT (SEQ ID NO: 1741) |
| 319 | CASP8 (SEQ ID NO: 71) | ATTTTTTAAACGGGTTTA (SEQ ID NO: 1742) |
| 320 | CASP8 (SEQ ID NO: 71) | TTTTAAATGGGTTTATAGG (SEQ ID NO: 1743) |
| 321 | CASP8 (SEQ ID NO: 71) | ATCGTAGTTTTCGAGT (SEQ ID NO: 1744) |
| 322 | CASP8 (SEQ ID NO: 71) | ATTGTAGTTTTTGAGTTT (SEQ ID NO: 1745) |
| 323 | HOXA5 (SEQ ID NO: 78) | TTCGAGTTCGGTTGAA (SEQ ID NO: 1746) |
| 324 | HOXA5 (SEQ ID NO: 78) | GTTTGAGTTTGGTTGAA (SEQ ID NO: 1747) |
| 325 | HOXA5 (SEQ ID NO: 78) | TAGTTTTCGGTCGGAA (SEQ ID NO: 1748) |
| 326 | HOXA5 (SEQ ID NO: 78) | TAGTTTTTGGTTGGAAG (SEQ ID NO: 1749) |
| 327 | HOXA5 (SEQ ID NO: 78) | TAATTCGATTCGGTTT (SEQ ID NO: 1750) |
| 328 | HOXA5 (SEQ ID NO: 78) | TTTAATTTGATTTTGGTTT (SEQ ID NO: 1751) |
| 329 | HOXA5 (SEQ ID NO: 78) | ATCGGTAGTTGACGGTT (SEQ ID NO: 1752) |
| 330 | HOXA5 (SEQ ID NO: 78) | ATTGGTAGTTGATGGTT (SEQ ID NO: 1753) |
| 331 | HOXA5 (SEQ ID NO: 78) | ATCGGTAGTTGACGGTT (SEQ ID NO: 1752) |
| 332 | HOXA5 (SEQ ID NO: 78) | ATTGGTAGTTGATGGTT (SEQ ID NO: 1753) |
| 333 | RARA (SEQ ID NO: 108) | TTCGGGATGTACGTTT (SEQ ID NO: 1754) |
| 334 | RARA (SEQ ID NO: 108) | TTTGGGATGTATGTTTT (SEQ ID NO: 1755) |
| 335 | RARA (SEQ ID NO: 108) | GAATTAGTATCGGTTTTT (SEQ ID NO: 1756) |
| 336 | RARA (SEQ ID NO: 108) | ATTAGTATTGGTTTTTGG (SEQ ID NO: 1757) |
| 337 | SNCG (SEQ ID NO: 73) | TGCGGTAGTATTCGAGT (SEQ ID NO: 1758) |

(continued)

| No: | Gene | Oligo: |
|-----|------|--------|
| 338 | SNCG (SEQ ID NO: 73) | TGTGGTAGTATTTGAGT (SEQ ID NO: 1759) |
| 339 | SNCG (SEQ ID NO: 73) | TGCGGTAGTATTCGAGT (SEQ ID NO: 1758) |
| 340 | SNCG (SEQ ID NO: 73) | TGTGGTAGTATTTGAGT (SEQ ID NO: 1759) |
| 341 | GPC3 (SEQ ID NO: 118) | AATAGTCGCGTTTAGG (SEQ ID NO: 1760) |
| 342 | GPC3 (SEQ ID NO: 118) | TAGTTGTGTTTAGGGAT (SEQ ID NO: 1761) |
| 343 | GPC3 (SEQ ID NO: 118) | TTTAACGTAGTTTTGATCGG (SEQ ID NO: 1762) |
| 344 | GPC3 (SEQ ID NO: 118) | TTTAATGTAGTTTTGATTGG (SEQ ID NO: 1763) |
| 345 | GPC3 (SEQ ID NO: 118) | TTTAACGTAGTTTTGATCGG (SEQ ID NO: 1762) |
| 346 | GPC3 (SEQ ID NO: 118) | TTTAATGTAGTTTTGATTGG (SEQ ID NO: 1763) |
| 347 | CLDN7 (SEQ ID NO: 87) | TTACGTTAAGTCGGGT (SEQ ID NO: 1764) |
| 348 | CLDN7 (SEQ ID NO: 87) | AGTTATGTTAAGTTGGG (SEQ ID NO: 1765) |
| 349 | CLDN7 (SEQ ID NO: 87) | TAGCGTTTTAGGCGTA (SEQ ID NO: 1766) |
| 350 | CLDN7 (SEQ ID NO: 87) | TAGTGTTTTAGGTGTATT (SEQ ID NO: 1767) |
| 351 | CLDN7 (SEQ ID NO: 87) | TTAGGGGCGTTTCGTA (SEQ ID NO: 1768) |
| 352 | CLDN7 (SEQ ID NO: 87) | TTAGGGGTGTTTTGTAG (SEQ ID NO: 1769) |
| 353 | CLDN7 (SEQ ID NO: 87) | TAGAATTCGGCGGGGA (SEQ ID NO: 1770) |
| 354 | CLDN7 (SEQ ID NO: 87) | TAGAATTTGGTGGGGA (SEQ ID NO: 1771) |
| 355 | CLDN7 (SEQ ID NO: 87) | TAGAATTCGGCGGGGA (SEQ ID NO: 1770) |
| 356 | CLDN7 (SEQ ID NO: 87) | TAGAATTTGGTGGGGA (SEQ ID NO: 1771) |
| 357 | SLIT2 (SEQ ID NO: 112) | TTCGATAGTTAACGATG (SEQ ID NO: 1772) |
| 358 | SLIT2 (SEQ ID NO: 112) | TTTGATAGTTAATGATGGT (SEQ ID NO: 1773) |
| 359 | SLIT2 (SEQ ID NO: 112) | ATTTCGTCGTAGTTTG (SEQ ID NO: 1774) |
| 360 | SLIT2 (SEQ ID NO: 112) | TTTTGTTGTAGTTTGGA (SEQ ID NO: 1775) |
| 361 | SLIT2 (SEQ ID NO: 112) | TAGCGGGTTCGTAGTA (SEQ ID NO: 1776) |
| 362 | SLIT2 (SEQ ID NO: 112) | TTAGTGGGTTTGTAGTA (SEQ ID NO: 1777) |
| 363 | SLIT2 (SEQ ID NO: 112) | AAGGCGCGGAAGTTTA (SEQ ID NO: 1778) |
| 364 | SLIT2 (SEQ ID NO: 112) | AAGGTGTGGAAGTTTA (SEQ ID NO: 1779) |
| 365 | SLIT2 (SEQ ID NO: 112) | AAGGCGCGGAAGTTTA (SEQ ID NO: 1778) |
| 366 | SLIT2 (SEQ ID NO: 112) | AAGGTGTGGAAGTTTA (SEQ ID NO: 1779) |
| 367 | IGSF4 (SEQ ID NO: 74) | AGGTAGATCGAGGAGG (SEQ ID NO: 1780) |
| 368 | IGSF4 (SEQ ID NO: 74) | AGGTAGATTGAGGAGG (SEQ ID NO: 1781) |
| 369 | IGSF4 (SEQ ID NO: 74) | AGGTAGATCGAGGAGG (SEQ ID NO: 1780) |
| 370 | IGSF4 (SEQ ID NO: 74) | AGGTAGATTGAGGAGG (SEQ ID NO: 1781) |
| 371 | IGSF4 (SEQ ID NO: 74) | TAGTCGTAGAGTCGGG (SEQ ID NO: 1782) |
| 372 | IGSF4 (SEQ ID NO: 74) | GTTGTAGAGTTGGGTT (SEQ ID NO: 1783) |
| 373 | IGSF4 (SEQ ID NO: 74) | TAGGTTTTCGGATTGA (SEQ ID NO: 1784) |
| 374 | IGSF4 (SEQ ID NO: 74) | GTAGGTTTTTGGATTGA (SEQ ID NO: 1785) |
| 375 | MCT1 (SEQ ID NO: 101) | ATTTTACGTAGGCGTT (SEQ ID NO: 1786) |

(continued)

| No: | Gene | Oligo: |
|-----|------|--------|
| 376 | MCT1 (SEQ ID NO: 101) | GATTTTATGTAGGTGTTT (SEQ ID NO: 1787) |
| 377 | MCT1 (SEQ ID NO: 101) | AGTTAGTCGCGTTTTA (SEQ ID NO: 1788) |
| 378 | MCT1 (SEQ ID NO: 101) | AGAGTTAGTTGTGTTTTA (SEQ ID NO: 1789) |
| 379 | MCT1 (SEQ ID NO: 101) | TATACGAGGAAGGTCGG (SEQ ID NO: 1790) |
| 380 | MCT1 (SEQ ID NO: 101) | TATATGAGGAAGGTTGG (SEQ ID NO: 1791) |
| 381 | MCT1 (SEQ ID NO: 101) | TATACGAGGAAGGTCGG (SEQ ID NO: 1790) |
| 382 | MCT1 (SEQ ID NO: 101) | TATATGAGGAAGGTTGG (SEQ ID NO: 1791) |
| 383 | SEQ ID NO:6 (SEQ ID NO: 6) | AAGTTTATCGGCGTTT (SEQ ID NO: 1792) |
| 384 | SEQ ID NO:6 (SEQ ID NO: 6) | AGAAGTTTATTGGTGTTT (SEQ ID NO: 1793) |
| 385 | SEQ ID NO:6 (SEQ ID NO: 6) | ATTTCGGAATTTAAGCGT (SEQ ID NO: 1794) |
| 386 | SEQ ID NO:6 (SEQ ID NO: 6) | TTTTGGAATTTAAGTGTT (SEQ ID NO: 1795) |
| 387 | SEQ ID NO:6 (SEQ ID NO: 6) | TAATTTCGGACGCGGA (SEQ ID NO: 1796) |
| 388 | SEQ ID NO:6 (SEQ ID NO: 6) | TTTTGGATGTGGAGGA (SEQ ID NO: 1797) |
| 389 | SEQ ID NO:6 (SEQ ID NO: 6) | TTACGGTGAAGGCGGA (SEQ ID NO: 1798) |
| 390 | SEQ ID NO:6 (SEQ ID NO: 6) | TTATGGTGAAGGTGGA (SEQ ID NO: 1799) |
| 391 | SEQ ID NO:6 (SEQ ID NO: 6) | TTACGGTGAAGGCGGA (SEQ ID NO: 1798) |
| 392 | SEQ ID NO:6 (SEQ ID NO: 6) | TTATGGTGAAGGTGGA (SEQ ID NO: 1799) |
| 393 | SEQ ID NO:6 (SEQ ID NO: 6) | TTTCGGTTTTCGTTAAT (SEQ ID NO: 1800) |
| 394 | SEQ ID NO:6 (SEQ ID NO: 6) | TTTGGTTTTTGTTAATTTAG (SEQ ID NO: 1801) |
| 395 | SEQ ID NO:6 (SEQ ID NO: 6) | TGTGCGAAGTTAACGT (SEQ ID NO: 1802) |
| 396 | SEQ ID NO:6 (SEQ ID NO: 6) | TTGTGTGAAGTTAATGT (SEQ ID NO: 1803) |
| 397 | SEQ ID NO:8 (SEQ ID NO: 8) | ATAAAGCGGGGTTTTA (SEQ ID NO: 1804) |
| 398 | SEQ ID NO:8 (SEQ ID NO: 8) | GGATAAAGTGGGGTTT (SEQ ID NO: 1805) |
| 399 | SEQ ID NO:8 (SEQ ID NO: 8) | AGGAGGCGAGAAATTT (SEQ ID NO: 1806) |
| 400 | SEQ ID NO:8 (SEQ ID NO: 8) | GAGGAGGTGAGAAATT (SEQ ID NO: 1807) |
| 401 | SEQ ID NO:8 (SEQ ID NO: 8) | AGAAATTTCGGGGTAG (SEQ ID NO: 1808) |
| 402 | SEQ ID NO:8 (SEQ ID NO: 8) | GAAATTTTGGGGTAGTA (SEQ ID NO: 1809) |
| 403 | SEQ ID NO:8 (SEQ ID NO: 8) | GGTAGTATCGTTTATAGA (SEQ ID NO: 1810) |
| 404 | SEQ ID NO:8 (SEQ ID NO: 8) | GGGGTAGTATTGTTTATA (SEQ ID NO: 1811) |
| 405 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGTGTATCGTTTTCGG (SEQ ID NO: 1812) |
| 406 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAGTGTATTGTTTTTTGG (SEQ ID NO: 1813) |
| 407 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TGCGTATCGTTAGTTA (SEQ ID NO: 1814) |

(continued)

| No: | Gene | Oligo: |
|---|---|---|
| 408 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTTGTGTATTGTTAG (SEQ ID NO: 1815) |
| 409 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | ATTATTTCGGCGGTGA (SEQ ID NO: 1816) |
| 410 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GATTATTTTGGTGGTGA (SEQ ID NO: 1817) |
| 411 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAAGTCGCGTAAGGAG (SEQ ID NO: 1818) |
| 412 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AAGTTGTGTAAGGAGTA (SEQ ID NO: 1819) |
| 413 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATCGCGAGTTTGGA (SEQ ID NO: 1820) |
| 414 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATTGTGAGTTTGGAG (SEQ ID NO: 1821) |
| 415 | SEQ ID NO:51 (SEQ ID NO: 51) | GTCGGGGTCGATTCGA (SEQ ID NO: 1822) |
| 416 | SEQ ID NO:51 (SEQ ID NO: 51) | GTTGGGGTTGATTTGAT (SEQ ID NO: 1823) |
| 417 | SEQ ID NO:51 (SEQ ID NO: 51) | AGGATTCGTTTGCGTT (SEQ ID NO: 1824) |
| 418 | SEQ ID NO:51 (SEQ ID NO: 51) | AAGGATTTGTTTGTGTT (SEQ ID NO: 1825) |
| 419 | MGC34831 (SEQ ID NO: 52) | ATTAGCGTTTGGCGTT (SEQ ID NO: 1826) |
| 420 | MGC34831 (SEQ ID NO: 52) | AATTAGTGTTTGGTGTT (SEQ ID NO: 1827) |
| 421 | MGC34831 (SEQ ID NO: 52) | GTTTAGCGACGGTCGT (SEQ ID NO: 1828) |
| 422 | MGC34831 (SEQ ID NO: 52) | TGTTTAGTGATGGTTGT (SEQ ID NO: 1829) |
| 423 | SEQ ID NO:54 (SEQ ID NO: 54) | TGTGTAGCGGCGATTA (SEQ ID NO: 1830) |
| 424 | SEQ ID NO:54 (SEQ ID NO: 54) | GTGTGTAGTGGTGATT (SEQ ID NO: 1831) |
| 425 | SEQ ID NO:54 (SEQ ID NO: 54) | ATTAGCGTTTGGTCGG (SEQ ID NO: 1832) |
| 426 | SEQ ID NO:54 (SEQ ID NO: 54) | ATTAGTGTTTGGTTGGG (SEQ ID NO: 1833) |
| 427 | PDLIM1 (SEQ ID NO: 55) | TAGGTCGCGTAGTCGT (SEQ ID NO: 1834) |
| 428 | PDLIM1 (SEQ ID NO: 55) | TTAGGTTGTGTAGTTGT (SEQ ID NO: 1835) |
| 429 | SEQ ID NO: 15 (SEQ ID NO: 15) | AATCGTGCGGTTGATA (SEQ ID NO: 1836) |
| 430 | SEQ ID NO:15 (SEQ ID NO: 15) | TGTAGAATTGTGTGGT (SEQ ID NO: 1837) |
| 431 | SEQ ID NO:15 (SEQ ID NO: 15) | TTGCGTAGAAAATTCGAG (SEQ ID NO: 1838) |
| 432 | SEQ ID NO: 15 (SEQ ID NO: 15) | TTGTGTAGAAAATTTGAG (SEQ ID NO: 1839) |
| 433 | SEQ ID NO:15 (SEQ ID NO: 15) | TTGCGTAGAAAATTCGAG (SEQ ID NO: 1838) |
| 434 | SEQ ID NO:15 (SEQ ID NO: 15) | TTGTGTAGAAAATTTGAG (SEQ ID NO: 1839) |
| 435 | SEQ ID NO:15 (SEQ ID NO: 15) | AAAATTCGAGGTCGGG (SEQ ID NO: 1840) |

(continued)

| No: | Gene | Oligo: |
|---|---|---|
| 436 | SEQ ID NO: 15 (SEQ ID NO: 15) | AAAATTTGAGGTTGGG (SEQ ID NO: 1841) |
| 437 | SEQ ID NO:15 (SEQ ID NO: 15) | AAAATTCGAGGTCGGG (SEQ ID NO: 1840) |
| 438 | SEQ ID NO:15 (SEQ ID NO: 15) | AAAATTTGAGGTTGGG (SEQ ID NO: 1841) |
| 439 | DKK3 (SEQ ID NO: 24) | ATTCGTTTTAGTTCGAG (SEQ ID NO: 1842) |
| 440 | DKK3 (SEQ ID NO: 24) | ATTTGTTTTAGTTTGAGT (SEQ ID NO: 1843) |
| 441 | DKK3 (SEQ ID NO: 24) | TTCGATCGTTTTAGGA (SEQ ID NO: 1844) |
| 442 | DKK3 (SEQ ID NO: 24) | AGTTTTGATTGTTTTAGG (SEQ ID NO: 1845) |
| 443 | DKK3 (SEQ ID NO: 24) | ATTCGTTCGGAGACGG (SEQ ID NO: 1846) |
| 444 | DKK3 (SEQ ID NO: 24) | TTTGTTTGGAGATGGG (SEQ ID NO: 1847) |
| 445 | DKK3 (SEQ ID NO: 24) | GAAAAGACGCGATTTT (SEQ ID NO: 1848) |
| 446 | DKK3 (SEQ ID NO: 24) | GAAAAGATGTGATTTTATT (SEQ ID NO: 1849) |
| 447 | SEQ ID NO:28 (SEQ ID NO: 28) | TATCGACGTTTTTGGT (SEQ ID NO: 1850) |
| 448 | SEQ ID NO:28 (SEQ ID NO: 28) | TATTGATGTTTTTGGTTT (SEQ ID NO: 1851) |
| 449 | SEQ ID NO:29 (SEQ ID NO: 29) | TTGATGCGGAGTTCGA (SEQ ID NO: 1852) |
| 450 | SEQ ID NO:29 (SEQ ID NO: 29) | TTGATGTGGAGTTTGA (SEQ ID NO: 1853) |
| 451 | SEQ ID NO:29 (SEQ ID NO: 29) | TTGATGCGGAGTTCGA (SEQ ID NO: 1852) |
| 452 | SEQ ID NO:29 (SEQ ID NO: 29) | TTGATGTGGAGTTTGA (SEQ ID NO: 1853) |
| 453 | SEQ ID NO:29 (SEQ ID NO: 29) | TAGGAACGGTAGGCGG (SEQ ID NO: 1854) |
| 454 | SEQ ID NO:29 (SEQ ID NO: 29) | TAGGAATGGTAGGTGG (SEQ ID NO: 1855) |
| 455 | SEQ ID NO:29 (SEQ ID NO: 29) | TAGGAACGGTAGGCGG (SEQ ID NO: 1854) |
| 456 | SEQ ID NO:29 (SEQ ID NO: 29) | TAGGAATGGTAGGTGG (SEQ ID NO: 1855) |
| 457 | SEQ ID NO:29 (SEQ ID NO: 29) | GTCGGAGGCGTTTGAG (SEQ ID NO: 1856) |
| 458 | SEQ ID NO:29 (SEQ ID NO: 29) | GTTGGAGGTGTTTGAGA (SEQ ID NO: 1857) |
| 459 | ARL7 (SEQ ID NO: 30) | TAGATTTCGTAGTTTTTTA (SEQ ID NO: 1858) |
| 460 | ARL7 (SEQ ID NO: 30) | TAGATTTTGTAGTTTTTTAA (SEQ ID NO: 1859) |
| 461 | ARL7 (SEQ ID NO: 30) | TGGGTACGTTTACGGT (SEQ ID NO: 1860) |
| 462 | ARL7 (SEQ ID NO: 30) | TGGGTATGTTTATGGTT (SEQ ID NO: 1861) |
| 463 | ARL7 (SEQ ID NO: 30) | GAAGAAATCGTTTTTGT (SEQ ID NO: 1862) |
| 464 | ARL7 (SEQ ID NO: 30) | GAAGAAATTGTTTTTGTT (SEQ ID NO: 1863) |
| 465 | ARL7 (SEQ ID NO: 30) | TAGTAGGATCGGTTTTT (SEQ ID NO: 1864) |
| 466 | ARL7 (SEQ ID NO: 30) | ATAGTAGGATTGGTTTTT (SEQ ID NO: 1865) |
| 467 | SEQ ID NO:31 (SEQ ID NO: 31) | AACGTTGCGTTGGGTA (SEQ ID NO: 1866) |
| 468 | SEQ ID NO:31 (SEQ ID NO: 31) | AATGTTGTGTTGGGTAA (SEQ ID NO: 1867) |
| 469 | SEQ ID NO:31 (SEQ ID NO: 31) | TAGCGTTTCGTGGTTA (SEQ ID NO: 1868) |
| 470 | SEQ ID NO:31 (SEQ ID NO: 31) | GTAGTGTTTTGTGGTTA (SEQ ID NO: 1869) |
| 471 | THH (SEQ ID NO: 32) | TTCGGAAGAAAAGCGAAT (SEQ ID NO: 1870) |
| 472 | THH (SEQ ID NO: 32) | TTTGGAAGAAAAGTGAAT (SEQ ID NO: 1871) |
| 473 | THH (SEQ ID NO: 32) | TTCGGAAGAAAAGCGAAT (SEQ ID NO: 1870) |

(continued)

| No: | Gene | Oligo: |
|---|---|---|
| 474 | THH (SEQ ID NO: 32) | TTTGGAAGAAAAGTGAAT (SEQ ID NO: 1871) |
| 475 | THH (SEQ ID NO: 32) | GTTCGTTGGCGTAAAT (SEQ ID NO: 1872) |
| 476 | THH (SEQ ID NO: 32) | GGTTTGTTGGTGTAAAT (SEQ ID NO: 1873) |
| 477 | THH (SEQ ID NO: 32) | TATTCGGAAGTGATCGG (SEQ ID NO: 1874) |
| 478 | THH (SEQ ID NO: 32) | TATTTGGAAGTGATTGG (SEQ ID NO: 1875) |
| 479 | THH (SEQ ID NO: 32) | TATTCGGAAGTGATCGG (SEQ ID NO: 1874) |
| 480 | THH (SEQ ID NO: 32) | TATTTGGAAGTGATTGG (SEQ ID NO: 1875) |
| 481 | SENP3 (SEQ ID NO: 39) | TATTCGGATCGGGTTT (SEQ ID NO: 1876) |
| 482 | SENP3 (SEQ ID NO: 39) | TTTATTTGGATTGGGTT (SEQ ID NO: 1877) |
| 483 | SENP3 (SEQ ID NO: 39) | TAGTCGAAAGTAGGACGT (SEQ ID NO: 1878) |
| 484 | SENP3 (SEQ ID NO: 39) | TAGTTGAAAGTAGGATGT (SEQ ID NO: 1879) |
| 485 | SENP3 (SEQ ID NO: 39) | TAGTCGAAAGTAGGACGT (SEQ ID NO: 1878) |
| 486 | SENP3 (SEQ ID NO: 39) | TAGTTGAAAGTAGGATGT (SEQ ID NO: 1879) |
| 487 | SENP3 (SEQ ID NO: 39) | AGGACGTTTTTGATCGG (SEQ ID NO: 1880) |
| 488 | SENP3 (SEQ ID NO: 39) | AGGATGTTTTTGATTGG (SEQ ID NO: 1881) |
| 489 | SENP3 (SEQ ID NO: 39) | AGGACGTTTTTGATCGG (SEQ ID NO: 1880) |
| 490 | SENP3 (SEQ ID NO: 39) | AGGATGTTTTTGATTGG (SEQ ID NO: 1881) |
| 491 | SENP3 (SEQ ID NO: 39) | AGTTATCGTTAGGAGGG (SEQ ID NO: 1882) |
| 492 | SENP3 (SEQ ID NO: 39) | AGTTATTGTTAGGAGGG (SEQ ID NO: 1883) |
| 493 | SENP3 (SEQ ID NO: 39) | AGTTATCGTTAGGAGGG (SEQ ID NO: 1882) |
| 494 | SENP3 (SEQ ID NO: 39) | AGTTATTGTTAGGAGGG (SEQ ID NO: 1883) |
| 495 | SEQ ID NO:42 (SEQ ID NO: 42) | GAGTCGGGTTGCGATG (SEQ ID NO: 1884) |
| 496 | SEQ ID NO:42 (SEQ ID NO: 42) | GGAGTTGGGTTGTGAT (SEQ ID NO: 1885) |
| 497 | SEQ ID NO:42 (SEQ ID NO: 42) | ATGGGTTGCGATTTTTA (SEQ ID NO: 1886) |
| 498 | SEQ ID NO:42 (SEQ ID NO: 42) | ATGGGTTGTGATTTTTA (SEQ ID NO: 1887) |
| 499 | SEQ ID NO:42 (SEQ ID NO: 42) | ATGGGTTGCGATTTTTA (SEQ ID NO: 1886) |
| 500 | SEQ ID NO:42 (SEQ ID NO: 42) | ATGGGTTGTGATTTTTA (SEQ ID NO: 1887) |
| 501 | (SEQ ID NO: 117) | TAGCGGTTTTTTAGCGTA (SEQ ID NO: 1888) |
| 502 | (SEQ ID NO: 117) | AGTGGTTTTTTAGTGTAT (SEQ ID NO: 1889) |
| 503 | (SEQ ID NO: 117) | AGATGCGCGGGTAGAT (SEQ ID NO: 1890) |
| 504 | (SEQ ID NO: 117) | AGATGTGTGGGTAGAT (SEQ ID NO: 1891) |
| 505 | (SEQ ID NO: 117) | AGATGCGCGGGTAGAT (SEQ ID NO: 1890) |
| 506 | (SEQ ID NO: 117) | AGATGTGTGGGTAGAT (SEQ ID NO: 1891) |
| 507 | (SEQ ID NO: 117) | AGATAGTTCGTATTCGT (SEQ ID NO: 1892) |
| 508 | (SEQ ID NO: 117) | GTAGATAGTTTGTATTTGT (SEQ ID NO: 1893) |
| 509 | (SEQ ID NO: 117) | TTTGTTCGTAACGTTT (SEQ ID NO: 1894) |
| 510 | (SEQ ID NO: 117) | TGTTTGTAATGTTTAGAG (SEQ ID NO: 1895) |
| 511 | 060279 (SEQ ID NO: 47) | AGTAAACGAATAAGAAGT (SEQ ID NO: 1896) |

(continued)

| No: | Gene | Oligo: |
|---|---|---|
| 512 | 060279 (SEQ ID NO: 47) | AAGTAAATGAATAAGAAGT (SEQ ID NO: 1897) |
| 513 | 060279 (SEQ ID NO: 47) | TACGTTTTTTCGGATTA (SEQ ID NO: 1898) |
| 514 | 060279 (SEQ ID NO: 47) | TATGTTTTTTTGGATTAAG (SEQ ID NO: 1899) |
| 515 | 060279 (SEQ ID NO: 47) | TAATTATCGGCGGTGT (SEQ ID NO: 1900) |
| 516 | 060279 (SEQ ID NO: 47) | ATTATTGGTGGTGTTTT (SEQ ID NO: 1901) |
| 517 | 060279 (SEQ ID NO: 47) | GGACGGCGGAAAATTA (SEQ ID NO: 1902) |
| 518 | 060279 (SEQ ID NO: 47) | AGGGATGGTGGAAAAT (SEQ ID NO: 1903) |
| 519 | SEQ ID NO:48 (SEQ ID NO: 48) | TATTTGGCGATTCGGA (SEQ ID NO: 1904) |
| 520 | SEQ ID NO:48 (SEQ ID NO: 48) | TGGTGATTTGGAGATT (SEQ ID NO: 1905) |
| 521 | SEQ ID NO:48 (SEQ ID NO: 48) | TAGGGTTACGTGTCGG (SEQ ID NO: 1906) |
| 522 | SEQ ID NO:48 (SEQ ID NO: 48) | TAGGGTTATGTGTTGG (SEQ ID NO: 1907) |
| 523 | SEQ ID NO:48 (SEQ ID NO: 48) | TAGGGTTACGTGTCGG (SEQ ID NO: 1906) |
| 524 | SEQ ID NO:48 (SEQ ID NO: 48) | TAGGGTTATGTGTTGG (SEQ ID NO: 1907) |
| 525 | SEQ ID NO:48 (SEQ ID NO: 48) | AACGAATTTTTCGATATT (SEQ ID NO: 1908) |
| 526 | SEQ ID NO:48 (SEQ ID NO: 48) | TTTAATGAATTTTTTGATATT (SEQ ID NO: 1909) |
| 527 | SEQ ID NO:48 (SEQ ID NO: 48) | AAAATCGTATGCGTGT (SEQ ID NO: 1910) |
| 528 | SEQ ID NO:48 (SEQ ID NO: 48) | GAAAATTGTATGTGTGTG (SEQ ID NO: 1911) |
| 529 | SEQ ID NO:9 (SEQ ID NO: 9) | GTTTCGCGTTTAGGGA (SEQ ID NO: 1912) |
| 530 | SEQ ID NO:9 (SEQ ID NO: 9) | GTTTTGTGTTTAGGGAT (SEQ ID NO: 1913) |
| 531 | SEQ ID NO:9 (SEQ ID NO: 9) | AGTGTTCGTCGTAGTT (SEQ ID NO: 1914) |
| 532 | SEQ ID NO:9 (SEQ ID NO: 9) | TGAGTGTTTGTTGTAGT (SEQ ID NO: 1915) |
| 533 | SEQ ID NO:4 (SEQ ID NO: 4) | TTTTGTTCGCGTTGAA (SEQ ID NO: 1916) |
| 534 | SEQ ID NO:4 (SEQ ID NO: 4) | TTGTTTGTGTTGAAGTA (SEQ ID NO: 1917) |
| 535 | SEQ ID NO:4 (SEQ ID NO: 4) | GGGTCGCGAGGTAGTT (SEQ ID NO: 1918) |
| 536 | SEQ ID NO:4 (SEQ ID NO: 4) | TGGGTTGTGAGGTAGT (SEQ ID NO: 1919) |
| 537 | SEQ ID NO:4 (SEQ ID NO: 4) | TTTGTGCGACGTTATT (SEQ ID NO: 1920) |
| 538 | SEQ ID NO:4 (SEQ ID NO: 4) | GGTTTGTGTGATGTTAT (SEQ ID NO: 1921) |
| 539 | SEQ ID NO:4 (SEQ ID NO: 4) | ATGGCGGTTTCGATTT (SEQ ID NO: 1922) |
| 540 | SEQ ID NO:4 (SEQ ID NO: 4) | GATGGTGGTTTTGATTT (SEQ ID NO: 1923) |
| 541 | SEQ ID NO:5 (SEQ ID NO: 5) | AATGAGCGAGAAAGTA (SEQ ID NO: 1924) |
| 542 | SEQ ID NO:5 (SEQ ID NO: 5) | AGAATGAGTGAGAAAGT (SEQ ID NO: 1925) |
| 543 | SEQ ID NO:5 (SEQ ID NO: 5) | ATTAAACGGGATGGTT (SEQ ID NO: 1926) |
| 544 | SEQ ID NO:5 (SEQ ID NO: 5) | AATATTAAATGGGATGGT (SEQ ID NO: 1927) |
| 545 | SEQ ID NO:5 (SEQ ID NO: 5) | GAGTTGCGAGGATTTT (SEQ ID NO: 1928) |
| 546 | SEQ ID NO:5 (SEQ ID NO: 5) | GGAGTTGTGAGGATTT (SEQ ID NO: 1929) |
| 547 | SEQ ID NO:5 (SEQ ID NO: 5) | GGGAATCGTTGATTTT (SEQ ID NO: 1930) |
| 548 | SEQ ID NO:5 (SEQ ID NO: 5) | AGGGGAATTGTTGATT (SEQ ID NO: 1931) |
| 549 | SEQ ID NO:7 (SEQ ID NO: 7) | TAGTCGTCGTGTAGGA (SEQ ID NO: 1932) |

(continued)

| No: | Gene | Oligo: |
|---|---|---|
| 550 | SEQ ID NO:7 (SEQ ID NO: 7) | TAGGTAGTTGTTGTGTA (SEQ ID NO: 1933) |
| 551 | SEQ ID NO:7 (SEQ ID NO: 7) | TATAGGTACGCGATGA (SEQ ID NO: 1934) |
| 552 | SEQ ID NO:7 (SEQ ID NO: 7) | AGGTATGTGATGAGGA (SEQ ID NO: 1935) |
| 553 | SEQ ID NO:7 (SEQ ID NO: 7) | TATGGTTACGTACGAG (SEQ ID NO: 1936) |
| 554 | SEQ ID NO:7 (SEQ ID NO: 7) | ATGGTTATGTATGAGTTT (SEQ ID NO: 1937) |
| 555 | SEQ ID NO:7 (SEQ ID NO: 7) | ATGATTTGCGTTACGT (SEQ ID NO: 1938) |
| 556 | SEQ ID NO:7 (SEQ ID NO: 7) | ATGATTTGTGTTATGTTT (SEQ ID NO: 1939) |
| 557 | SEQ ID NO:7 (SEQ ID NO: 7) | TAACGTTGTGGTTCGAA (SEQ ID NO: 1940) |
| 558 | SEQ ID NO:7 (SEQ ID NO: 7) | TAATGTTGTGGTTTGAA (SEQ ID NO: 1941) |
| 559 | SEQ ID NO:7 (SEQ ID NO: 7) | TAACGTTGTGGTTCGAA (SEQ ID NO: 1940) |
| 560 | SEQ ID NO:7 (SEQ ID NO: 7) | TAATGTTGTGGTTTGAA (SEQ ID NO: 1941) |
| 561 | SEQ ID NO: (SEQ ID NO: 1) | GGTCGGCGTTGATTTTA (SEQ ID NO: 1942) |
| 562 | SEQ ID NO: (SEQ ID NO: 1) | GGTTGGTGTTGATTTTA (SEQ ID NO: 1943) |
| 563 | SEQ ID NO: (SEQ ID NO: 1) | GGTCGGCGTTGATTTTA (SEQ ID NO: 1942) |
| 564 | SEQ ID NO: (SEQ ID NO: 1) | GGTTGGTGTTGATTTTA (SEQ ID NO: 1943) |
| 565 | SEQ ID NO: (SEQ ID NO: 1) | GATTCGAACGGATTTT (SEQ ID NO: 1944) |
| 566 | SEQ ID NO: (SEQ ID NO: 1) | GGGATTTGAATGGATTT (SEQ ID NO: 1945) |
| 567 | SEQ ID NO: (SEQ ID NO: 1 ) | TGGGTCGGGATTCGAA (SEQ ID NO: 1946) |
| 568 | SEQ ID NO: (SEQ ID NO: 1) | TGGGTTGGGATTTGAA (SEQ ID NO: 1947) |
| 569 | SEQ ID NO: (SEQ ID NO: 1) | TGGGTCGGGATTCGAA (SEQ ID NO: 1946) |
| 570 | SEQ ID NO: (SEQ ID NO: 1) | TGGGTTGGGATTTGAA (SEQ ID NO: 1947) |
| 571 | SEQ ID NO:1 (SEQ ID NO: 1) | GTCGGAAGTTTCGGGA (SEQ ID NO: 1948) |
| 572 | SEQ ID NO: (SEQ ID NO: 1) | GTTGGAAGTTTTGGGAT (SEQ ID NO: 1949) |
| 573 | SEQ ID NO: (SEQ ID NO: 1) | TGGATATCGTAGGGTA (SEQ ID NO: 1950) |
| 574 | SEQ ID NO: (SEQ ID NO: 1) | TGGATATTGTAGGGTAG (SEQ ID NO: 1951) |
| 575 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 576 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |
| 577 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 578 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |
| 579 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GGCGTCGAAAGTCGTT (SEQ ID NO: 1954) |
| 580 | PROSTAGLANDIN E2 RECEPTOR, EP4 | GGTGTTGAAAGTTGTTG (SEQ ID NO: 1955) |

(continued)

| No: | Gene | Oligo: |
|---|---|---|
| | SUBTYPE (PROSTANOID EP4 RECEPTOR) (PGE RECEPTOR, EP4 SUBTYPE) (SEQ ID NO: 10) | |
| 581 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAATCGTTTGTTTACGT (SEQ ID NO: 1956) |
| 582 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AATTGTTTGTTTATGTAGT (SEQ ID NO: 1957) |
| 583 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTACGGAGGCGTTTTA (SEQ ID NO: 1958) |
| 584 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTATGGAGGTGTTTT (SEQ ID NO: 1959) |
| 585 | ORPHAN NUCLEAR RECEPTOR NR5A2 (ALPHA-1-FETOPROTEM TRANSCRIPTION FACTOR) (HEPATOCYTIC TRANSCRIPTION FACTOR) (B1-BINDING FACTOR) (HB 1F) (CYP7A PROMOTER BINDING FACTOR) (SEQ ID NO: 11) | AGGCGAATTTATCGGG (SEQ ID NO: 1960) |
| 586 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | GGTGAATTTATTGGGG (SEQ ID NO: 1961) |
| 587 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTCGAAGTAGGCGT (SEQ ID NO: 1962) |
| 588 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTTGAAGTAGGTGTT (SEQ ID NO: 1963) |
| 589 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTCGACGAAGTTTT (SEQ ID NO: 1964) |
| 590 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTGATGAAGTTTTGTT (SEQ ID NO: 1965) |

(continued)

| No: | Gene | Oligo: |
|---|---|---|
| 591 | LIM DOMAIN KINASE 1 (EC 2.7.1.37) (LIMK-1) (SEQ ID NO: 12) | TGTAGTCGGGAGGTTA (SEQ ID NO: 1966) |
| 592 | LIM DOMAIN KINASE 1 (EC 2.7.1.37) (LIMK-1) (SEQ ID NO: 12) | TGTAGTTGGGAGGTTA (SEQ ID NO: 1967) |
| 593 | LIM DOMAIN KINASE 1 (etc 2.7.1.37) (LIMK-1) (SEQ ID NO: 12) | TGTAGTCGGGAGGTTA (SEQ ID NO: 1966) |
| 594 | LIM DOMAIN KINASE 1 (EC 2.7.1.37) (LIMK-1) (SEQ ID NO: 12) | TGTAGTTGGGAGGTTA (SEQ ID NO: 1967) |
| 595 | LIM DOMAIN KINASE 1 (EC 2.7.1.37) (LIMK-1) (SEQ ID NO: 12) | GGATTATCGCGGGGGT (SEQ ID NO: 1968) |
| 596 | LIM DOMAIN KINASE 1 (EC 2.7.1.37) (LIMK-1) (SEQ ID NO: 12) | GGATTATTGTGGGGGT (SEQ ID NO: 1969) |
| 597 | LIM DOMAIN KINASE 1 (EC 2.7.1.37) (LIMK-1) (SEQ ID NO: 12) | GGATTATCGCGGGGGT (SEQ ID NO: 1968) |
| 598 | LIM DOMAIN KINASE 1 (EC 2.7.1.37) (LIMK-1) (SEQ ID NO: 12) | GGATTATTGTGGGGGT (SEQ ID NO: 1969) |
| 599 | LIM DOMAIN KINASE 1 (EC 2.7.1.37) (LIMK-1) (SEQ ID NO: 12) | GTCGGTAGTTTATCGGAT (SEQ ID NO: 1970) |
| 600 | LIM DOMAIN KINASE 1 (EC 2.7.1.37) (LIMK-1) (SEQ ID NO: 12) | GTTGGTAGTTTATTGGAT (SEQ ID NO: 1971) |
| 601 | LIM DOMAIN KINASE 1 (EC 2.7.1.37) (LIMK-1) (SEQ ID NO: 12) | GTCGGTAGTTTATCGGAT (SEQ ID NO: 1970) |
| 602 | LIM DOMAIN KINASE 1 (EC 2.7.1.37) (LIMK-1) (SEQ ID NO: 12) | GTTGGTAGTTTATTGGAT (SEQ ID NO: 1971) |
| 603 | LIM DOMAIN KINASE 1 (EC 2.7.1.37) (LIMK-1) (SEQ ID NO: 12) | TAGGAGACGTTACGTT (SEQ ID NO: 1972) |
| 604 | LIM DOMAIN KINASE 1 (EC 2.7.1.37) (LIMK-1) (SEQ ID NO: 12) | AGATGTTATGTTAGGGT (SEQ ID NO: 1973) |
| 605 | ECL11B (SEQ ID NO: 50) | TAGGTTTCGATTCGTT (SEQ ID NO: 1974) |
| 606 | BCL11B (SEQ ID NO: 50) | TTAGGTTTTGATTTGTTT (SEQ ID NO: 1975) |
| 607 | BCL11B (SEQ ID NO: 50) | AAGTCGTCGGAGTTAG (SEQ ID NO: 1976) |
| 608 | BCL11B (SEQ ID NO: 50) | GTGAAGTTGTTGGAGT (SEQ ID NO: 1977) |
| 609 | BCL11B (SEQ ID NO: 50) | TTGAGGCGTTACGGTT (SEQ ID NO: 1978) |
| 610 | BCL11B (SEQ ID NO: 50) | TTGAGGTGTTATGGTT (SEQ ID NO: 1979) |
| 611 | BCL11B (SEQ ID NO: 50) | TTGAGGCGTTACGGTT (SEQ ID NO: 1978) |
| 612 | BCL11B (SEQ ID NO: 50) | TTGAGGTGTTATGGTT (SEQ ID NO: 1979) |
| 613 | MSF (SEQ ID NO: 13) | GTTTCGAAATTGGCGT (SEQ ID NO: 1980) |
| 614 | MSF (SEQ ID NO: 13) | TTTGAAATTGGTGTGG (SEQ ID NO: 1981) |
| 615 | MSF (SEQ ID NO: 13) | TTCGGTTTACGGGTTGTA (SEQ ID NO: 1982) |
| 616 | MSF (SEQ ID NO: 13) | TTTGGTTTATGGGTTGTA (SEQ ID NO: 1983) |
| 617 | MSF (SEQ ID NO: 13) | TTCGGTTTACGGGTTGTA (SEQ ID NO: 1982) |
| 618 | MSF (SEQ ID NO: 13) | TTTGGTTTATGGGTTGTA (SEQ ID NO: 1983) |

(continued)

| No: | Gene | Oligo: |
|---|---|---|
| 619 | MSF (SEQ ID NO: 13) | TTACGGTTCGATTTTG (SEQ ID NO: 1984) |
| 620 | MSF (SEQ ID NO: 13) | TATGGTTTGATTTTGGG (SEQ ID NO: 1985) |
| 621 | SEQ ID NO: 14 (SEQ ID NO: 14) | TAAGGCGTTTTCGATA (SEQ ID NO: 1986) |
| 622 | SEQ ID NO:14 (SEQ ID NO: 14) | GTAAGGTGTTTTTGATAT (SEQ ID NO: 1987) |
| 623 | SEQ ID NO:14 (SEQ ID NO: 14) | TGGGTGTACGCGTGAA (SEQ ID NO: 1988) |
| 624 | SEQ ID NO:14 (SEQ ID NO: 14) | TGTATGTGTGAAGGGG (SEQ ID NO: 1989) |
| 625 | SEQ ID NO:16 (SEQ ID NO: 16) | ATCGTTGGTCGGATTT (SEQ ID NO: 1990) |
| 626 | SEQ ID NO: 16 (SEQ ID NO: 16) | TAGGATTGTTGGTTGGA (SEQ ID NO: 1991) |
| 627 | SEQ ID NO: 16 (SEQ ID NO: 16) | AGGAGTTTTCGTGTCGT (SEQ ID NO: 1992) |
| 628 | SEQ ID NO: 16 (SEQ ID NO: 16) | AGGAGTTTTTGTGTTGT (SEQ ID NO: 1993) |
| 629 | SEQ ID NO: 16 (SEQ ID NO: 16) | AGGAGTTTTCGTGTCGT (SEQ ID NO: 1992) |
| 630 | SEQ ID NO: 16 (SEQ ID NO: 16) | AGGAGTTTTTGTGTTGT (SEQ ID NO: 1993) |
| 631 | SEQ ID NO: 16 (SEQ ID NO: 16) | TTACGGATAGGGCGAT (SEQ ID NO: 1994) |
| 632 | SEQ ID NO: 16 (SEQ ID NO: 16) | TATGGATAGGGTGATTT (SEQ ID NO: 1995) |
| 633 | SEQ ID NO: 16 (SEQ ID NO: 16) | AGGCGTTGTCGGTGAT (SEQ ID NO: 1996) |
| 634 | SEQ ID NO:16 (SEQ ID NO: 16) | AGGTGTTGTTGGTGATA (SEQ ID NO: 1997) |
| 635 | SEQ ID NO: 17 (SEQ ID NO: 17) | TACGTTTCGGGTTTGTTA (SEQ ID NO: 1998) |
| 636 | SEQ ID NO: 17 (SEQ ID NO: 17) | TATGTTTTGGGTTTGTTA (SEQ ID NO: 1999) |
| 637 | SEQ ID NO: 17 (SEQ ID NO: 17) | TACGTTTCGGGTTTGTTA (SEQ ID NO: 1998) |
| 638 | SEQ ID NO: 17 (SEQ ID NO: 17) | TATGTTTTGGGTTTGTTA (SEQ ID NO: 1999) |
| 639 | SEQ ID NO: 17 (SEQ ID NO: 17) | ATTTAGTCGTGCGTTT (SEQ ID NO: 2000) |
| 640 | SEQ ID NO: 17 (SEQ ID NO: 17) | TGATTTAGTTGTGTGTT (SEQ ID NO: 2001) |
| 641 | SEQ ID NO: 18 (SEQ ID NO: 18) | TTTACGCGGGGTTTTA (SEQ ID NO: 2002) |
| 642 | SEQ ID NO: 18 (SEQ ID NO: 18) | TTTATGTGGGGTTTTAG (SEQ ID NO: 2003) |
| 643 | SEQ ID NO: 18 (SEQ ID NO: 18) | TTACGTCGTTATTAGGT (SEQ ID NO: 2004) |
| 644 | SEQ ID NO: 18 (SEQ ID NO: 18) | TTTTATGTTGTTATTAGGT (SEQ ID NO: 2005) |
| 645 | SEQ ID NO: 18 (SEQ ID NO: 18) | TATTTGGACGTCGGGT (SEQ ID NO: 2006) |
| 646 | SEQ ID NO: 18 (SEQ ID NO: 18) | TATTTGGATGTTGGGT (SEQ ID NO: 2007) |
| 647 | SEQ ID NO:18 (SEQ ID NO: 18) | TATTTGGACGTCGGGT (SEQ ID NO: 2006) |
| 648 | SEQ ID NO: 18 (SEQ ID NO: 18) | TATTTGGATGTTGGGT (SEQ ID NO: 2007) |
| 649 | SEQ ID NO: 18 (SEQ ID NO: 18) | GAGGCGTATTAGGTCGG (SEQ ID NO: 2008) |
| 650 | SEQ ID NO: 18 (SEQ ID NO: 18) | AGGTGTATTAGGTTGGG (SEQ ID NO: 2009) |
| 651 | SEQ ID NO: 18 (SEQ ID NO: 18) | AAAGCGGAGTCGTTAG (SEQ ID NO: 2010) |
| 652 | SEQ ID NO: 18 (SEQ ID NO: 18) | AGTGGAGTTGTTAGGT (SEQ ID NO: 2011) |
| 653 | SEQ ID NO:19 (SEQ ID NO: 19) | AGGTTTTCGTTGTAGTA (SEQ ID NO: 2012) |
| 654 | SEQ ID NO: 19 (SEQ ID NO: 19) | TAGGTTTTTGTTGTAGTA (SEQ ID NO: 2013) |
| 655 | SEQ ID NO:19 (SEQ ID NO: 19) | TGAGATTCGTTTTTTAAA (SEQ ID NO: 2014) |
| 656 | SEQ ID NO: 19 (SEQ ID NO: 19) | GGTGAGATTTGTTTTTTA (SEQ ID NO: 2015) |

(continued)

| No: | Gene | Oligo: |
|---|---|---|
| 657 | PRDM6 (SEQ ID NO: 20) | AGTTTTAAGCGTTTGGT (SEQ ID NO: 2016) |
| 658 | PRDM6 (SEQ ID NO: 20) | AGTTTTAAGTGTTTGGT (SEQ ID NO: 2017) |
| 659 | PRDM6 (SEQ ID NO: 20) | AGTTTTAAGCGTTTGGT (SEQ ID NO: 2016) |
| 660 | PRDM6 (SEQ ID NO: 20) | AGTTTTAAGTGTTTGGT (SEQ ID NO: 2017) |
| 661 | PRDM6 (SEQ ID NO: 20) | GAAGTTCGGATTTCGG (SEQ ID NO: 2018) |
| 662 | PRDM6 (SEQ ID NO: 20) | GGAAGTTTGGATTTTGG (SEQ ID NO: 2019) |
| 663 | PRDM6 (SEQ ID NO: 20) | TTGTCGGGTTACGGGA (SEQ ID NO: 2020) |
| 664 | PRDM6 (SEQ ID NO: 20) | GTTGGGTTATGGGAGA (SEQ ID NO: 2021) |
| 665 | PRDM6 (SEQ ID NO: 20) | TTCGTAGAATTGTCGAAG (SEQ ID NO: 2022) |
| 666 | PRDM6 (SEQ ID NO: 20) | TTTGTAGAATTGTTGAAG (SEQ ID NO: 2023) |
| 667 | PRDM6 (SEQ ID NO: 20) | TTCGTAGAATTGTCGAAG (SEQ ID NO: 2022) |
| 668 | PRDM6 (SEQ ID NO: 20) | TTTGTAGAATTGTTGAAG (SEQ ID NO: 2023) |
| 669 | RAP2B (SEQ ID NO: 21) | GGTCGGGTAATCGTTA (SEQ ID NO: 2024) |
| 670 | RAP2B (SEQ ID NO: 21) | GTTGGGTAATTGTTAGA (SEQ ID NO: 2025) |
| 671 | RAP2B (SEQ ID NO: 21) | AGGAAGCGTTTTCGTT (SEQ ID NO: 2026) |
| 672 | RAP2B (SEQ ID NO: 21) | AGAGGAAGTGTTTTTGT (SEQ ID NO: 2027) |
| 673 | NR2E1 (SEQ ID NO: 22) | AATGTAGCGGCGTTAT (SEQ ID NO: 2028) |
| 674 | NR2E1 (SEQ ID NO: 22) | TAAATGTAGTGGTGTTAT (SEQ ID NO: 2029) |
| 675 | NR2E1 (SEQ ID NO: 22) | GTCGTTATCGGTTTGGA (SEQ ID NO: 2030) |
| 676 | NR2E1 (SEQ ID NO: 22) | GTTGTTATTGGTTTGGA (SEQ ID NO: 2031) |
| 677 | NR2E1 (SEQ ID NO: 22) | GTCGTTATCGGTTTGGA (SEQ ID NO: 2030) |
| 678 | NR2E1 (SEQ ID NO: 22) | GTTGTTATTGGTTTGGA (SEQ ID NO: 2031) |
| 679 | NR2E1 (SEQ ID NO: 22) | GACGTAAGTTTCGGGT (SEQ ID NO: 2032) |
| 680 | NR2E1 (SEQ ID NO: 22) | GGATGTAAGTTTTGGG (SEQ ID NO: 2033) |
| 681 | NR2E1 (SEQ ID NO: 22) | TTTTTAGTCGCGAGAA (SEQ ID NO: 2034) |
| 682 | NR2E1 (SEQ ID NO: 22) | TTTTTAGTTGTGAGAAGT (SEQ ID NO: 2035) |
| 683 | NR2E1 (SEQ ID NO: 22) | TTCGGGTGATATCGTTT (SEQ ID NO: 2036) |
| 684 | NR2E1 (SEQ ID NO: 22) | TTTGGGTGATATTGTTT (SEQ ID NO: 2037) |
| 685 | NR2E1 (SEQ ID NO: 22) | TTCGGGTGATATCGTTT (SEQ ID NO: 2036) |
| 686 | NR2E1 (SEQ ID NO: 22) | TTTGGGTGATATTGTTT (SEQ ID NO: 2037) |
| 687 | NR2E1 (SEQ ID NO: 22) | AGGCGAGTCGGAGTTT (SEQ ID NO: 2038) |
| 688 | NR2E1 (SEQ ID NO: 22) | AGGTGAGTTGGAGTTTT (SEQ ID NO: 2039) |
| 689 | NR2E1 (SEQ ID NO: 22) | TAAGTCGAGCGAGTTT (SEQ ID NO: 2040) |
| 690 | NR2E1 (SEQ ID NO: 22) | TAGTAAGTTGAGTGAGT (SEQ ID NO: 2041) |
| 691 | NR2E1 (SEQ ID NO: 22) | AGGGACGCGAAAATTT (SEQ ID NO: 2042) |
| 692 | NR2E (SEQ ID NO: 22) | GGAGGGATGTGAAAAT (SEQ ID NO: 2043) |
| 693 | PCDH7 (SEQ ID NO: 23) | ATCGTAGTCGGTTTTA (SEQ ID NO: 2044) |
| 694 | PCDH7 (SEQ ID NO: 23) | GATTATTGTAGTTGGTTT (SEQ ID NO: 2045) |

(continued)

| No: | Gene | Oligo: |
|---|---|---|
| 695 | RTTN (SEQ ID NO: 25) | TAGTGGCGCGGTAGTT (SEQ ID NO: 2046) |
| 696 | RTTN (SEQ ID NO: 25) | TAGTGGTGTGGTAGTTT (SEQ ID NO: 2047) |
| 697 | RTTN (SEQ ID NO: 25) | TAGGACGTGTTTTCGG (SEQ ID NO: 2048) |
| 698 | RTTN (SEQ ID NO: 25) | AGGATGTGTTTTTGGG (SEQ ID NO: 2049) |
| 699 | SNAP25 (SEQ ID NO: 33) | TATTTCGAGTTAGAGTTACGA (SEQ ID NO: 2050) |
| 700 | SNAP25 (SEQ ID NO: 33) | TATTTTGAGTTAGAGTTATGA (SEQ ID NO: 2051) |
| 701 | SNAP25 (SEQ ID NO: 33) | TATTTCGAGTTAGAGTTACGA (SEQ ID NO: 2050) |
| 702 | SNAP25 (SEQ ID NO: 33) | TATTTTGAGTTAGAGTTATGA (SEQ ID NO: 2051) |
| 703 | SNAP25 (SEQ ID NO: 33) | ATACGGAATATCGTATTT (SEQ ID NO: 2052) |
| 704 | SNAP25 (SEQ ID NO: 33) | GTGTATATATGGAATATTGT (SEQ ID NO: 2053) |
| 705 | SEQ ID NO:26 (SEQ ID NO: 26) | TTAAGTATCGAGGCGT (SEQ ID NO: 2054) |
| 706 | SEQ ID NO:26 (SEQ ID NO: 26) | TTTTAAGTATTGAGGTGT (SEQ ID NO: 2055) |
| 707 | SEQ ID NO:26 (SEQ ID NO: 26) | AATTTTGGTCGTTTAGT (SEQ ID NO: 2056) |
| 708 | SEQ ID NO:26 (SEQ ID NO: 26) | AAATTTTGGTTGTTTAGT (SEQ ID NO: 2057) |
| 709 | SEQ ID NO:26 (SEQ ID NO: 26) | TTCGTATTGACGTTAAT (SEQ ID NO: 2058) |
| 710 | SEQ ID NO:26 (SEQ ID NO: 26) | TTTGTATTGATGTTAATAGA (SEQ ID NO: 2059) |
| 711 | GIRK2 (SEQ ID NO: 27) | AGTTGTTCGTAGGCGA (SEQ ID NO: 2060) |
| 712 | GIRK2 (SEQ ID NO: 27) | AGTTGTTTGTAGGTGA (SEQ ID NO: 2061) |
| 713 | GIRK2 (SEQ ID NO: 27) | AGTTGTTCGTAGGCGA (SEQ ID NO: 2060) |
| 714 | GIRK2 (SEQ ID NO: 27) | AGTTGTTTGTAGGTGA (SEQ ID NO: 2061) |
| 715 | GIRK2 (SEQ ID NO: 27) | TTATTTCGTTCGTAGTT (SEQ ID NO: 2062) |
| 716 | GIRK2 (SEQ ID NO: 27) | TTTGTTTGTAGTTAGGTA (SEQ ID NO: 2063) |
| 717 | GIRK2 (SEQ ID NO: 27) | TTAGTCGAAAGGCGAG (SEQ ID NO: 2064) |
| 718 | GIRK2 (SEQ ID NO: 27) | TAGTTGAAAGGTGAGG (SEQ ID NO: 2065) |
| 719 | SEQ ID NO:28 (SEQ ID NO: 28) | ATTAGGCGAGTTTCGT (SEQ ID NO: 2066) |
| 720 | SEQ ID NO:28 (SEQ ID NO: 28) | TTAGGTGAGTTTTGTTT (SEQ ID NO: 2067) |
| 721 | SEQ ID NO:31 (SEQ ID NO: 31) | TTTACGTAGGGCGATT (SEQ ID NO: 2068) |
| 722 | SEQ ID NO:31 (SEQ ID NO: 31) | ATTTTTATGTAGGGTGAT (SEQ ID NO: 2069) |
| 723 | SEQ ID NO:31 (SEQ ID NO: 31) | GATACGGTTAGGCGGG (SEQ ID NO: 2070) |
| 724 | SEQ ID NO:31 (SEQ ID NO: 31) | GATATGGTTAGGTGGG (SEQ ID NO: 2071) |
| 725 | SEQ ID NO:31 (SEQ ID NO: 31) | GATACGGTTAGGCGGG (SEQ ID NO: 2070) |
| 726 | SEQ ID NO:31 (SEQ ID NO: 31) | GATATGGTTAGGTGGG (SEQ ID NO: 2071) |
| 727 | SEQ ID NO:31 (SEQ ID NO: 31) | TTCGGGCGTTTTATAT (SEQ ID NO: 2072) |
| 728 | SEQ ID NO:31 (SEQ ID NO: 31) | GGTTTGGGTGTTTATA (SEQ ID NO: 2073) |
| 729 | HOXB 13 (SEQ ID NO: 34) | GTCGTTATTATTTCGAGG (SEQ ID NO: 2074) |
| 730 | HOXB 13 (SEQ ID NO: 34) | GTTGTTATTATTTTGAGGA (SEQ ID NO: 2075) |
| 731 | HOXB 13 (SEQ ID NO: 34) | AAGTTAGCGGGTTCGT (SEQ ID NO: 2076) |
| 732 | HOXB 13 (SEQ ID NO: 34) | AAGTTAGTGGGTTTGT (SEQ ID NO: 2077) |

(continued)

| No: | Gene | Oligo: |
|---|---|---|
| 733 | HOXB 13 (SEQ ID NO: 34) | AAGTTAGCGGGTTCGT (SEQ ID NO: 2076) |
| 734 | HOXB 13 (SEQ ID NO: 34) | AAGTTAGTGGGTTTGT (SEQ ID NO: 2077) |
| 735 | HOXB13 (SEQ ID NO: 34) | TTGGTCGCGTAGTAAA (SEQ ID NO: 2078) |
| 736 | HOXB 13 (SEQ ID NO: 34) | TGGTTGTGTAGTAAAGT (SEQ ID NO: 2079) |
| 737 | (SEQ ID NO: 35) | GGAGGTGCGAATTTAA (SEQ ID NO: 2080) |
| 738 | (SEQ ID NO: 35) | GGGAGGTGTGAATTTA (SEQ ID NO: 2081) |
| 739 | (SEQ ID NO: 35) | AAATAGTCGTTTGGGA (SEQ ID NO: 2082) |
| 740 | (SEQ ID NO: 35) | AAATAGTTGTTTGGGAG (SEQ ID NO: 2083) |
| 741 | (SEQ ID NO: 35) | AGAAAATATACGAGATTTAT (SEQ ID NO: 2084) |
| 742 | (SEQ ID NO: 35) | AGAAAATATATGAGATTTATT (SEQ ID NO: 2085) |
| 743 | (SEQ ID NO: 35) | TAAAGACGGGAAGAGA (SEQ ID NO: 2086) |
| 744 | (SEQ ID NO: 35) | ATAAAGATGGGAAGAGA (SEQ ID NO: 2087) |
| 745 | MGC10561 (SEQ ID NO: 37) | TTTTTTACGTTAAGGGG (SEQ ID NO: 2088) |
| 746 | MGC10561 (SEQ ID NO: 37) | TTTTTATGTTAAGGGGG (SEQ ID NO: 2089) |
| 747 | MGC10561 (SEQ ID NO: 37) | TTTGTTTTTCGAGTAGA (SEQ ID NO: 2090) |
| 748 | MGC10561 (SEQ ID NO: 37) | TGTTTTTTGAGTAGAGG (SEQ ID NO: 2091) |
| 749 | LMX1A (SEQ ID NO: 38) | GTCGGGTTTTTCGGAA (SEQ ID NO: 2092) |
| 750 | LMX1A (SEQ ID NO: 38) | GTTGGGTTTTTTGGAAG (SEQ ID NO: 2093) |
| 751 | LMX1A (SEQ ID NO: 38) | GTCGAGATTTTATCGAAA (SEQ ID NO: 2094) |
| 752 | LMX1A (SEQ ID NO: 38) | GTTGAGATTTTATTGAAAG (SEQ ID NO: 2095) |
| 753 | LMX1A (SEQ ID NO: 38) | AGTATTCGGGCGGGTA (SEQ ID NO: 2096) |
| 754 | LMX1A (SEQ ID NO: 38) | GTAGTATTTGGGTGGG (SEQ ID NO: 2097) |
| 755 | LMX1A (SEQ ID NO: 38) | AACGAAATTACGTGTAT (SEQ ID NO: 2098) |
| 756 | LMX1A (SEQ ID NO: 38) | TGAAATGAAATTATGTGTA (SEQ ID NO: 2099) |
| 757 | GS1 (SEQ ID NO: 40) | TGCGAGTTAGCGGTTA (SEQ ID NO: 2100) |
| 758 | GS1 (SEQ ID NO: 40) | TTGTGAGTTAGTGGTT (SEQ ID NO: 2101) |
| 759 | GS1 (SEQ ID NO: 40) | AGTTTCGAGGTTTTCGG (SEQ ID NO: 2102) |
| 760 | GS1 (SEQ ID NO: 40) | AAGTTTTGAGGTTTTTGG (SEQ ID NO: 2103) |
| 761 | TITF1 (SEQ ID NO: 41) | GTCGTGGGGATCGTAT (SEQ ID NO: 2104) |
| 762 | TITF1 (SEQ ID NO: 41) | GTTGTGGGGATTGTAT (SEQ ID NO: 2105) |
| 763 | TITF1 (SEQ ID NO: 41) | GTCGTGGGGATCGTAT (SEQ ID NO: 2104) |
| 764 | TITF1 (SEQ ID NO: 41) | GTTGTGGGGATTGTAT (SEQ ID NO: 2105) |
| 765 | TITF1 (SEQ ID NO: 41) | GGTTCGTTTAAGTTCGG (SEQ ID NO: 2106) |
| 766 | TITF1 (SEQ ID NO: 41) | GGTTTGTTTAAGTTTGG (SEQ ID NO: 2107) |
| 767 | TITF1 (SEQ ID NO: 41) | GGTTCGTTTAAGTTCGG (SEQ ID NO: 2106) |
| 768 | TITF1 (SEQ ID NO: 41) | GGTTTGTTTAAGTTTGG (SEQ ID NO: 2107) |
| 769 | TITF1 (SEQ ID NO: 41) | TTAGGTCGCGTTTGTA (SEQ ID NO: 2108) |
| 770 | TITF1 (SEQ ID NO: 41) | AGGTTGTGTTTGTAGA (SEQ ID NO: 2109) |

(continued)

| No: | Gene | Oligo: |
|---|---|---|
| 771 | TITF1 (SEQ ID NO: 41) | TATTTCGTTTTCGTAATT (SEQ ID NO: 2110) |
| 772 | TITF1 (SEQ ID NO: 41) | TTTGTTTTTGTAATTAGATT (SEQ ID NO: 2111) |
| 773 | DDX51 (SEQ ID NO: 43) | AGATTTTCGGCGAGAT (SEQ ID NO: 2112) |
| 774 | DDX51 (SEQ ID NO: 43) | ATTTTTGGTGAGATAGG (SEQ ID NO: 2113) |
| 775 | DDX51 (SEQ ID NO: 43) | TACGTGTGGTTTTCGGTA (SEQ ID NO: 2114) |
| 776 | DDX51 (SEQ ID NO: 43) | TATGTGTGGTTTTTGGTA (SEQ ID NO: 2115) |
| 777 | DDX51 (SEQ ID NO: 43) | TACGTGTGGTTTTCGGTA (SEQ ID NO: 2114) |
| 778 | DDX51 (SEQ ID NO: 43) | TATGTGTGGTTTTTGGTA (SEQ ID NO: 2115) |
| 779 | DDX51 (SEQ ID NO: 43) | AACGTGCGGGGTTTTT (SEQ ID NO:2116) |
| 780 | DDX51 (SEQ ID NO: 43) | TGAATGTGTGGGGTTT (SEQ ID NO: 2117) |
| 781 | SEQ ID NO:45 (SEQ ID NO: 45) | AGCGAACGTTTATTTT (SEQ ID NO: 2118) |
| 782 | SEQ ID NO:45 (SEQ ID NO: 45) | TAGGAGAGTGAATGTTT (SEQ ID NO: 2119) |
| 783 | SEQ ID NO:46 (SEQ ID NO: 46) | GAGTCGGGTTATCGTT (SEQ ID NO: 2120) |
| 784 | SEQ ID NO:46 (SEQ ID NO: 46) | GGAGTTGGGTTATTGT (SEQ ID NO: 2121) |
| 785 | SEQ ID NO:46 (SEQ ID NO: 46) | TTACGGATGTTTCGGT (SEQ ID NO: 2122) |
| 786 | SEQ ID NO:46 (SEQ ID NO: 46) | TTTATGGATGTTTTGGT (SEQ ID NO:2123) |
| 787 | SEQ ID NO:46 (SEQ ID NO: 46) | TGACGTATTTTCGGTT (SEQ ID NO: 2124) |
| 788 | SEQ ID NO:46 (SEQ ID NO: 46) | GGTGATGTATTTTTGGT (SEQ ID NO:2125) |
| 789 | SEQ ID NO:46 (SEQ ID NO: 46) | ATAGTCGGAATCGTTG (SEQ ID NO: 2126) |
| 790 | SEQ ID NO:46 (SEQ ID NO: 46) | TAGTTGGAATTGTTGTT (SEQ ID NO:2127) |
| 791 | SEQ ID NO:2 (SEQ ID NO: 2) | ATTGGGTTTCGCGTAGG (SEQ ID NO: 2128) |
| 792 | SEQ ID NO:2 (SEQ ID NO: 2) | ATTGGGTTTTGTGTAGG (SEQ ID NO: 2129) |
| 793 | SEQ ID NO:2 (SEQ ID NO: 2) | ATTGGGTTTCGCGTAGG (SEQ ID NO: 2128) |
| 794 | SEQ ID NO:2 (SEQ ID NO: 2) | ATTGGGTTTTGTGTAGG (SEQ ID NO: 2129) |
| 795 | SEQ ID NO:2 (SEQ ID NO: 2) | TAGTAGTCGGCGGGAG (SEQ ID NO: 2130) |
| 796 | SEQ ID NO:2 (SEQ ID NO: 2) | TAGTAGTTGGTGGGAG (SEQ ID NO:2131) |
| 797 | SEQ ID NO:2 (SEQ ID NO: 2) | TAGTAGTCGGCGGGAG (SEQ ID NO:2130) |
| 798 | SEQ ID NO:2 (SEQ ID NO: 2) | TAGTAGTTGGTGGGAG (SEQ ID NO: 2131) |
| 799 | SEQ ID NO:2 (SEQ ID NO: 2) | TTGATTGGCGGACGAG (SEQ ID NO: 1706) |
| 800 | SEQ ID NO:2 (SEQ ID NO: 2) | TTGATTGGTGGATGAG (SEQ ID NO: 1707) |
| 801 | SEQ ID NO:2 (SEQ ID NO: 2) | TTGATTGGCGGACGAG (SEQ ID NO: 1706) |
| 802 | SEQ ID NO:2 (SEQ ID NO: 2) | TTGATTGGTGGATGAG (SEQ ID NO: 1707) |
| 803 | SEQ ID NO:3 (SEQ ID NO: 3) | TTGCGTTAATTCGGTA (SEQ ID NO: 2132) |
| 804 | SEQ ID NO:3 (SEQ ID NO: 3) | AATTTGTGTTAATTTGGT (SEQ ID NO: 2133) |
| 805 | SEQ ID NO:3 (SEQ ID NO: 3) | AGTCGGGAGAGCGAAA (SEQ ID NO: 2134) |
| 806 | SEQ ID NO:3 (SEQ ID NO: 3) | AGTTGGGAGAGTGAAA (SEQ ID NO: 2135) |
| 807 | SEQ ID NO:3 (SEQ ID NO: 3) | AGTCGGGAGAGCGAAA (SEQ ID NO: 2134) |
| 808 | SEQ ID NO:3 (SEQ ID NO: 3) | AGTTGGGAGAGTGAAA (SEQ ID NO: 2135) |

(continued)

| No: | Gene | Oligo: |
|---|---|---|
| 809 | SEQ ID NO:3 (SEQ ID NO: 3) | GGTCGAAGAGTCGGGA (SEQ ID NO: 2136) |
| 810 | SEQ ID NO:3 (SEQ ID NO: 3) | GGTTGAAGAGTTGGGA (SEQ ID NO: 2137) |
| 811 | SEQ ID NO:3 (SEQ ID NO: 3) | GGTCGAAGAGTCGGGA (SEQ ID NO: 2136) |
| 812 | SEQ ID NO:3 (SEQ ID NO: 3) | GGTTGAAGAGTTGGGA (SEQ ID NO: 2137) |
| 813 | SEQ ID NO:3 (SEQ ID NO: 3) | ATGTTAGCGGGTCGAA (SEQ ID NO: 2138) |
| 814 | SEQ ID NO:3 (SEQ ID NO: 3) | ATGTTAGTGGGTTGAA (SEQ ID NO: 2139) |
| 815 | SEQ ID NO:3 (SEQ ID NO: 3) | ATGTTAGCGGGTCGAA (SEQ ID NO: 2138) |
| 816 | SEQ ID NO:3 (SEQ ID NO: 3) | ATGTTAGTGGGTTGAA (SEQ ID NO: 2139) |

**Table 17: Breast cancer vs. other cancer**

| No: | Gene | Oligo: |
|---|---|---|
| 1 | SCGB3A1 (SEQ ID NO: 115) | GGCGTAGAAGGCGTTT (SEQ ID NO: 2140) |
| 2 | SCGB3A1 (SEQ ID NO: 115) | GGTGTAGAAGGTGTTT (SEQ ID NO: 2141) |
| 3 | SCGB3A1 (SEQ ID NO: 115) | GGCGTAGAAGGCGTTT (SEQ ID NO: 2140) |
| 4 | SCGB3A1 (SEQ ID NO: 115) | GGTGTAGAAGGTGTTT (SEQ ID NO: 2141) |
| 5 | SCGB3A1 (SEQ ID NO: 115) | TAGTGTTCGACGTTGT (SEQ ID NO: 1475) |
| 6 | SCGB3A1 (SEQ ID NO: 115) | TAGTGTTTGATGTTGTT (SEQ ID NO: 1476) |
| 7 | ARH1/NOEY2 (SEQ ID NO: 97) | TAAAACGTTCGGTAGG (SEQ ID NO: 1485) |
| 8 | ARH1/NOEY2 (SEQ ID NO: 97) | TTTAAAATGTTTGGTAGG (SEQ ID NO: 1486) |
| 9 | ARH1/NOEY2 (SEQ ID NO: 97) | TGTATTCGTCGTTAGG (SEQ ID NO: 1487) |
| 10 | ARH1/NOEY2 (SEQ ID NO: 97) | AATGTATTTGTTGTTAGG (SEQ ID NO: 1488) |
| 11 | ARH1/NOEY2 (SEQ ID NO: 97) | TTAGACGGAGTTCGGA (SEQ ID NO: 1489) |
| 12 | ARH1/NOEY2 (SEQ ID NO: 97) | TAGATGGAGTTTGGAGA (SEQ ID NO: 1490) |
| 13 | ARH1/NOEY2 (SEQ ID NO: 97) | TAGACGTAGGCGTATT (SEQ ID NO: 1491) |
| 14 | ARH1/NOEY2 (SEQ ID NO: 97) | GGGTAGATGTAGGTGT (SEQ ID NO: 1492) |
| 15 | CCND2 (SEQ ID NO: 104) | TTAGGGTCGATCGTGT (SEQ ID NO: 1493) |
| 16 | CCND2 (SEQ ID NO: 104) | TAGGGTTGATTGTGTT (SEQ ID NO: 1494) |
| 17 | CCND2 (SEQ ID NO: 104) | TTATCGTAGTCGGTTT (SEQ ID NO: 1495) |
| 18 | CCND2 (SEQ ID NO: 104) | GATTTTATTGTAGTTGGT (SEQ ID NO: 1496) |
| 19 | CCND2 (SEQ ID NO: 104) | GAGTGAGGCGCGAAAT (SEQ ID NO: 1497) |
| 20 | CCND2 (SEQ ID NO: 104) | GAGTGAGGTGTGAAAT (SEQ ID NO: 1498) |
| 21 | CCND2 (SEQ ID NO: 104) | GAGTGAGGCGCGAAAT (SEQ ID NO: 1497) |
| 22 | CCND2 (SEQ ID NO: 104) | GAGTGAGGTGTGAAAT (SEQ ID NO: 1498) |
| 23 | CCND2 (SEQ ID NO: 104) | AAGGATCGAGCGTGGA (SEQ ID NO: 1499) |
| 24 | CCND2 (SEQ ID NO: 104) | AAGGATTGAGTGTGGA (SEQ ID NO: 1500) |
| 25 | CCND2 (SEQ ID NO: 104) | AAGGATCGAGCGTGGA (SEQ ID NO: 1499) |
| 26 | CCND2 (SEQ ID NO: 104) | AAGGATTGAGTGTGGA (SEQ ID NO: 1500) |
| 27 | CDKN1A (SEQ ID NO: 67) | ATTAGGGTCGCGTTGA (SEQ ID NO: 1501) |

(continued)

| No: | Gene | Oligo: |
|-----|------|--------|
| 28 | CDKN1A (SEQ ID NO: 67) | ATTAGGGTTGTGTTGA (SEQ ID NO: 1502) |
| 29 | CDKN1A (SEQ ID NO: 67) | ATTAGGGTCGCGTTGA (SEQ ID NO: 1501) |
| 30 | CDKN1A (SEQ ID NO: 67) | ATTAGGGTTGTGTTGA (SEQ ID NO: 1502) |
| 31 | CDKN2A (SEQ ID NO: 57) | GGCGTTGTTTAACGTAT (SEQ ID NO: 1503) |
| 32 | CDKN2A (SEQ ID NO: 57) | GGGTGTTGTTTAATGTA (SEQ ID NO: 1504) |
| 33 | DAPK1 (SEQ ID NO: 98) | TTTCGGAGATTCGGTT (SEQ ID NO: 1509) |
| 34 | DAPK1 (SEQ ID NO: 98) | TTTGGAGATTTGGTTTT (SEQ ID NO: 1510) |
| 35 | DAPK1 (SEQ ID NO: 98) | TTTTAGCGTCGGGGAG (SEQ ID NO: 1511) |
| 36 | DAPK1 (SEQ ID NO: 98) | TTTTAGTGTTGGGGAG (SEQ ID NO: 1512) |
| 37 | DAPK1 (SEQ ID NO: 98) | TTTTAGCGTCGGGGAG (SEQ ID NO: 1511) |
| 38 | DAPK1 (SEQ ID NO: 98) | TTTTAGTGTTGGGGAG (SEQ ID NO: 1512) |
| 39 | EYA4 (SEQ ID NO: 58) | GGTATAAAATCGTAAATTTT (SEQ ID NO: 1513) |
| 40 | EYA4 (SEQ ID NO: 58) | GGGTATAAAATTGTAAATTT (SEQ ID NO: 1514) |
| 41 | EYA4 (SEQ ID NO: 58) | TATGTAGTCGCGTAGT (SEQ ID NO: 1515) |
| 42 | EYA4 (SEQ ID NO: 58) | TTTATGTAGTTGTGTAGT (SEQ ID NO: 1516) |
| 43 | EYA4 (SEQ ID NO: 58) | GTTTAGATACGAAATGTT (SEQ ID NO: 1517) |
| 44 | EYA4 (SEQ ID NO: 58) | GTTTAGATATGAAATGTTAT (SEQ ID NO: 1518) |
| 45 | EYA4 (SEQ ID NO: 58) | AGTTTTGACGTCGTTT (SEQ ID NO: 1519) |
| 46 | EYA4 (SEQ ID NO: 58) | TTGATGTTGTTTTGGAA (SEQ ID NO: 1520) |
| 47 | FHIT (SEQ ID NO: 76) | GTTACGTTAGCGGGTT (SEQ ID NO: 1521) |
| 48 | FHIT (SEQ ID NO: 76) | GGTTATGTTAGTGGGT (SEQ ID NO: 1522) |
| 49 | GSTP1 (SEQ ID NO: 59) | GGCGATTTCGGGGATT (SEQ ID NO: 1525) |
| 50 | GSTP1 (SEQ ID NO: 59) | GGTGATTTTGGGGATTT (SEQ ID NO: 1526) |
| 51 | GSTP1 (SEQ ID NO: 59) | GACGTTCGGGGTGTAG (SEQ ID NO: 1527) |
| 52 | GSTP1 (SEQ ID NO: 59) | GATGTTTGGGGTGTAG (SEQ ID NO: 1528) |
| 53 | GSTP1 (SEQ ID NO: 59) | GACGTTCGGGGTGTAG (SEQ ID NO: 1527) |
| 54 | GSTP1 (SEQ ID NO: 59) | GATGTTTGGGGTGTAG (SEQ ID NO: 1528) |
| 55 | GSTP1 (SEQ ID NO: 59) | AGTTCGCGGGATTTTT (SEQ ID NO: 1529) |
| 56 | GSTP1 (SEQ ID NO: 59) | GGAGTTTGTGGGATTT (SEQ ID NO: 1530) |
| 57 | GSTP1 (SEQ ID NO: 59) | AGTTTTCGTTATTAGTGA (SEQ ID NO: 1531) |
| 58 | GSTP1 (SEQ ID NO: 59) | TAGTTTTTGTTATTAGTGA (SEQ ID NO: 1532) |
| 59 | HIC1 (SEQ ID NO: 85) | TATCGAAGTTTTCGGG (SEQ ID NO: 1533) |
| 60 | HIC1 (SEQ ID NO: 85) | TATTGAAGTTTTTGGGT (SEQ ID NO: 1534) |
| 61 | HIC1 (SEQ ID NO: 85) | TAGCGGTTATTTCGGT (SEQ ID NO: 1535) |
| 62 | HIC1 (SEQ ID NO: 85) | TTTAGTGGTTATTTTGGT (SEQ ID NO: 1536) |
| 63 | HIC1 (SEQ ID NO: 85) | TACGTTTTTCGTAGCGT (SEQ ID NO: 1537) |
| 64 | HIC1 (SEQ ID NO: 85) | ATTATGTTTTTTGTAGTGT (SEQ ID NO: 1538) |
| 65 | HIC1 (SEQ ID NO: 85) | TTCGGTTTTGTCGTATA (SEQ ID NO: 1539) |

(continued)

| No: | Gene | Oligo: |
|---|---|---|
| 66 | HIC1 (SEQ ID NO: 85) | TTTGGTTTTGTTGTATAG (SEQ ID NO: 1540) |
| 67 | SERPINB5 (SEQ ID NO: 68) | ATTGTCGTACGTATGT (SEQ ID NO: 1551) |
| 68 | SERPINB5 (SEQ ID NO: 68) | AGAGGATTGTTGTATGTA (SEQ ID NO: 1552) |
| 69 | SERPINB5 (SEQ ID NO: 68) | TTTTTTGTTCGAATATGT (SEQ ID NO: 1553) |
| 70 | SERPINB5 (SEQ ID NO: 68) | TTTGTTTGAATATGTTGG (SEQ ID NO: 1554) |
| 71 | TERT (SEQ ID NO: 92) | TATAACGAACGTCGTT (SEQ ID NO: 2142) |
| 72 | TERT (SEQ ID NO: 92) | AGGTATAATGAATGTTGT (SEQ ID NO: 2143) |
| 73 | TGFBR2 (SEQ ID NO: 93) | AAGACGGTTAGGTCGG (SEQ ID NO: 2144) |
| 74 | TGFBR2 (SEQ ID NO: 93) | AAGATGGTTAGGTTGG (SEQ ID NO: 2145) |
| 75 | TGFBR2 (SEQ ID NO: 93) | AAGACGGTTAGGTCGG (SEQ ID NO: 2144) |
| 76 | TGFBR2 (SEQ ID NO: 93) | AAGATGGTTAGGTTGG (SEQ ID NO: 2145) |
| 77 | THRB (SEQ ID NO: 106) | GGGCGGTTAAGTCGAG (SEQ ID NO: 1569) |
| 78 | THRB (SEQ ID NO: 106) | GGGTGGTTAAGTTGAG (SEQ ID NO: 1570) |
| 79 | THRB (SEQ ID NO: 106) | GGGCGGTTAAGTCGAG (SEQ ID NO: 1569) |
| 80 | THRB (SEQ ID NO: 106) | GGGTGGTTAAGTTGAG (SEQ ID NO: 1570) |
| 81 | TIMP3 (SEQ ID NO: 103) | TTATTAACGGAGGAAGG (SEQ ID NO: 1571) |
| 82 | TIMP3 (SEQ ID NO: 103) | TATTAATGGAGGAAGGG (SEQ ID NO: 1572) |
| 83 | TIMP3 (SEQ ID NO: 103) | TTCGTTATGTGTACGGAA (SEQ ID NO: 1573) |
| 84 | TIMP3 (SEQ ID NO: 103) | TTTGTTATGTGTATGGAA (SEQ ID NO: 1574) |
| 85 | TIMP3 (SEQ ID NO: 103) | TTCGTTATGTGTACGGAA (SEQ ID NO: 1573) |
| 86 | TIMP3 (SEQ ID NO: 103) | TTTGTTATGTGTATGGAA (SEQ ID NO: 1574) |
| 87 | TIMP3 (SEQ ID NO: 103) | GAGTATTTCGAGTTTGT (SEQ ID NO: 1575) |
| 88 | TIMP3 (SEQ ID NO: 103) | AGTATTTTGAGTTTGTATT (SEQ ID NO: 1576) |
| 89 | TIMP3 (SEQ ID NO: 103) | TAAGCGTTAATCGAGT (SEQ ID NO: 1577) |
| 90 | TIMP3 (SEQ ID NO: 103) | TAGGTAAGTGTTAATTGA (SEQ ID NO: 1578) |
| 91 | TP73 (SEQ ID NO: 86) | TAGGATTCGCGTTTTT (SEQ ID NO: 1579) |
| 92 | TP73 (SEQ ID NO: 86) | GGTAGGATTTGTGTTTT (SEQ ID NO: 1580) |
| 93 | CDH13 (SEQ ID NO: 70) | TAGTCGCGTGTATGAA (SEQ ID NO: 1585) |
| 94 | CDH13 (SEQ ID NO: 70) | TGTAGTTGTGTGTATGA (SEQ ID NO: 1586) |
| 95 | TMS1/ASC (SEQ ID NO: 84) | TTCGTTTCGGAGTCGA (SEQ ID NO: 1591) |
| 96 | TMS1/ASC (SEQ ID NO: 84) | TTTGTTTTGGAGTTGAT (SEQ ID NO: 1592) |
| 97 | APAF1 (SEQ ID NO: 82) | GTGTCGTAGCGGTATT (SEQ ID NO: 1593) |
| 98 | APAF1 (SEQ ID NO: 82) | GGTGTTGTAGTGGTAT (SEQ ID NO: 1594) |
| 99 | APAF1 (SEQ ID NO: 82) | AGTAGCGTCGGGTTTT (SEQ ID NO: 1595) |
| 100 | APAF1 (SEQ ID NO: 82) | GAGTAGTGTTGGGTTT (SEQ ID NO: 1596) |
| 101 | SYK (SEQ ID NO: 60) | GAAGTTATCGCGTTGG (SEQ ID NO: 1597) |
| 102 | SYK (SEQ ID NO: 60) | AGAAGTTATTGTGTTGG (SEQ ID NO: 1598) |
| 103 | SYK (SEQ ID NO: 60) | GATCGATGCGGTTTAT (SEQ ID NO: 1599) |

(continued)

| No: | Gene | Oligo: |
|---|---|---|
| 104 | SYK (SEQ ID NO: 60) | GGGATTGATGTGGTTT (SEQ ID NO: 1600) |
| 105 | SYK (SEQ ID NO: 60) | TTATTCGGTCGGGATT (SEQ ID NO: 1603) |
| 106 | SYK (SEQ ID NO: 60) | TTTATTTGGTTGGGATT (SEQ ID NO: 1604) |
| 107 | FABP3 (SEQ ID NO: 77) | TAAAGCGGTAGTTCGG (SEQ ID NO: 1609) |
| 108 | FABP3 (SEQ ID NO: 77) | AAGTGGTAGTTTGGGT (SEQ ID NO: 1610) |
| 109 | FABP3 (SEQ ID NO: 77) | TATTGGCGTTGACGTA (SEQ ID NO: 1611) |
| 110 | FABP3 (SEQ ID NO: 77) | TGGTGTTGATGTAGGT (SEQ ID NO: 1612) |
| 111 | RASSF1A (SEQ ID NO: 90) | TACGGGTATTTTCGCGT (SEQ ID NO: 1613) |
| 112 | RASSF1A (SEQ ID NO: 90) | ATATGGGTATTTTTGTGT (SEQ ID NO: 1614) |
| 113 | RASSF1A (SEQ ID NO: 90) | AGAGCGCGTTTAGTTT (SEQ ID NO: 1615) |
| 114 | RASSF1A (SEQ ID NO: 90) | GAGAGTGTGTTTAGTTT (SEQ ID NO: 1616) |
| 115 | RASSF1A (SEQ ID NO: 90) | AGTAAATCGGATTAGGA (SEQ ID NO: 1617) |
| 116 | RASSF1A (SEQ ID NO: 90) | AGTAAATTGGATTAGGAG (SEQ ID NO: 1618) |
| 117 | TWIST (SEQ ID NO: 100) | AGTAAAGGCGTTGCGT (SEQ ID NO: 1619) |
| 118 | TWIST (SEQ ID NO: 100) | AGTAAAGGTGTTGTGT (SEQ ID NO: 1620) |
| 119 | TWIST (SEQ ID NO: 100) | AGTAAAGGCGTTGCGT (SEQ ID NO: 1619) |
| 120 | TWIST (SEQ ID NO: 100) | AGTAAAGGTGTTGTGT (SEQ ID NO: 1620) |
| 121 | TWIST (SEQ ID NO: 100) | TATTTTTCGAGGCGTA (SEQ ID NO: 1621) |
| 122 | TWIST (SEQ ID NO: 100) | TTTTGAGGTGTAGTTTT (SEQ ID NO: 1622) |
| 123 | TWIST (SEQ ID NO: 100) | ATTGGGTCGTTGTAGA (SEQ ID NO: 1623) |
| 124 | TWIST (SEQ ID NO: 100) | ATTGGGTTGTTGTAGA (SEQ ID NO: 1624) |
| 125 | TWIST (SEQ ID NO: 100) | ATTGGGTCGTTGTAGA (SEQ ID NO: 1623) |
| 126 | TWIST (SEQ ID NO: 100) | ATTGGGTTGTTGTAGA (SEQ ID NO: 1624) |
| 127 | TWIST (SEQ ID NO: 100) | TAGGTCGGGACGTAAA (SEQ ID NO: 1625) |
| 128 | TWIST (SEQ ID NO: 100) | AGTAGGTTGGGATGTA (SEQ ID NO: 1626) |
| 129 | ESR2 (SEQ ID NO: 91) | ATAAGCGATTTAACGAT (SEQ ID NO: 1629) |
| 130 | ESR2 (SEQ ID NO: 91) | AAGTGATTTAATGATAAGT (SEQ ID NO: 1630) |
| 131 | PLAU (SEQ ID NO: 62) | TATTTGTCGCGTTGAT (SEQ ID NO: 1633) |
| 132 | PLAU (SEQ ID NO: 62) | ATTTGTTGTGTTGATGA (SEQ ID NO: 1634) |
| 133 | PLAU (SEQ ID NO: 62) | TGTAATTCGGGGATTT (SEQ ID NO: 1635) |
| 134 | PLAU (SEQ ID NO: 62) | TTGTAATTTGGGGATTT (SEQ ID NO: 1636) |
| 135 | PLAU (SEQ ID NO: 62) | TTGGAGATCGCGTTTT (SEQ ID NO: 1637) |
| 136 | PLAU (SEQ ID NO: 62) | TTGGAGATTGTGTTTTT (SEQ ID NO: 1638) |
| 137 | PLAU (SEQ ID NO: 62) | GAGCGTTGCGGAAGTA (SEQ ID NO: 1639) |
| 138 | PLAU (SEQ ID NO: 62) | GAGTGTTGTGGAAGTA (SEQ ID NO: 1640) |
| 139 | PLAU (SEQ ID NO: 62) | GAGCGTTGCGGAAGTA (SEQ ID NO: 1639) |
| 140 | PLAU (SEQ ID NO: 62) | GAGTGTTGTGGAAGTA (SEQ ID NO: 1640) |
| 141 | STAT1 (SEQ ID NO: 109) | GTTATTTTCGAGAGTTG (SEQ ID NO: 1641) |

(continued)

| No: | Gene | Oligo: |
|---|---|---|
| 142 | STAT1 (SEQ ID NO: 109) | GTTATTTTTGAGAGTTGT (SEQ ID NO: 1642) |
| 143 | LOT1 (SEQ ID NO: 95) | ATGGGTACGTTTAAGG (SEQ ID NO: 1649) |
| 144 | LOT1 (SEQ ID NO: 95) | TGGGTATGTTTAAGGG (SEQ ID NO: 1650) |
| 145 | GJB2 (SEQ ID NO: 111) | GGATTTCGTCGGTATT (SEQ ID NO: 1667) |
| 146 | GJB2 (SEQ ID NO: 111) | GGGGATTTTGTTGGTA (SEQ ID NO: 1668) |
| 147 | PRDM2 (SEQ ID NO: 114) | TGTAGAGACGACGATT (SEQ ID NO: 1675) |
| 148 | PRDM2 (SEQ ID NO: 114) | ATTGTAGAGATGATGATT (SEQ ID NO: 1676) |
| 149 | PRDM2 (SEQ ID NO: 114) | AGAGCGCGGTAGTAGT (SEQ ID NO: 1677) |
| 150 | PRDM2 (SEQ ID NO: 114) | TGAGAGTGTGGTAGTA (SEQ ID NO: 1678) |
| 151 | PRDM2 (SEQ ID NO: 114) | TGTTCGCGATGTTTTA (SEQ ID NO: 1679) |
| 152 | PRDM2 (SEQ ID NO: 114) | TGTTTGTGATGTTTTAGT (SEQ ID NO: 1680) |
| 153 | ALX4 (SEQ ID NO: 64) | AAGTCGATCGTTTTGT (SEQ ID NO: 1683) |
| 154 | ALX4 (SEQ ID NO: 64) | TGGAAGTTGATTGTTTT (SEQ ID NO: 1684) |
| 155 | ALX4 (SEQ ID NO: 64) | ATATCGTTCGGGGGAA (SEQ ID NO: 2146) |
| 156 | ALX4 (SEQ ID NO: 64) | ATATCGTTCGGGGGAA (SEQ ID NO: 2146) |
| 157 | ALX4 (SEQ ID NO: 64) | TATTGCGAGGATTCGG (SEQ ID NO: 1685) |
| 158 | ALX4 (SEQ ID NO: 64) | ATTGTGAGGATTTGGT (SEQ ID NO: 1686) |
| 159 | ALX4 (SEQ ID NO: 64) | TTCGTAGCGTAGGGTT (SEQ ID NO: 1687) |
| 160 | ALX4 (SEQ ID NO: 64) | TTTGTAGTGTAGGGTTT (SEQ ID NO: 1688) |
| 161 | IGFBP7 (SEQ ID NO: 94) | ATTTTTTCGCGGGTAT (SEQ ID NO: 1710) |
| 162 | IGFBP7 (SEQ ID NO: 94) | TTTTTGTGGGTATTTTAG (SEQ ID NO: 1711) |
| 163 | IGFBP7 (SEQ ID NO: 94) | GGTATATTCGACGGGG (SEQ ID NO: 1712) |
| 164 | IGFBP7 (SEQ ID NO: 94) | GGGTATATTTGATGGGG (SEQ ID NO: 1713) |
| 165 | IGFBP7 (SEQ ID NO: 94) | GGTACGAGCGTTTTTT (SEQ ID NO: 1714) |
| 166 | IGFBP7 (SEQ ID NO: 94) | TGGGTATGAGTGTTTT (SEQ ID NO: 1715) |
| 167 | IGFBP7 (SEQ ID NO: 94) | AAAGCGTATTTAATTCGT (SEQ ID NO: 1716) |
| 168 | IGFBP7 (SEQ ID NO: 94) | AGTGTATTTAATTTGTGTT (SEQ ID NO: 1717) |
| 169 | NME1 (SEQ ID NO: 107) | AGTCGAGATTGCGTTA (SEQ ID NO: 2147) |
| 170 | NME1 (SEQ ID NO: 107) | AGTTGAGATTGTGTTAG (SEQ ID NO: 2148) |
| 171 | NME1 (SEQ ID NO: 107) | ATCGTTTGAATTCGGGA (SEQ ID NO: 2149) |
| 172 | NME1 (SEQ ID NO: 107) | ATTGTTTGAATTTGGGA (SEQ ID NO: 2150) |
| 173 | NME1 (SEQ ID NO: 107) | ATCGTTTGAATTCGGGA (SEQ ID NO: 2149) |
| 174 | NME1 (SEQ ID NO: 107) | ATTGTTTGAATTTGGGA (SEQ ID NO: 2150) |
| 175 | NME1 (SEQ ID NO: 107) | AATTCGAGATTAGTTCGG (SEQ ID NO: 1724) |
| 176 | NME1 (SEQ ID NO: 107) | AATTTGAGATTAGTTTGG (SEQ ID NO: 1725) |
| 177 | NME1 (SEQ ID NO: 107) | AATTCGAGATTAGTTCGG (SEQ ID NO: 1724) |
| 178 | NME1 (SEQ ID NO: 107) | AATTTGAGATTAGTTTGG (SEQ ID NO: 1725) |
| 179 | NME1 (SEQ ID NO: 107) | TTTTAGTACGTTGGAAA (SEQ ID NO: 2151) |

(continued)

| No: | Gene | Oligo: |
|---|---|---|
| 180 | NME1 (SEQ ID NO: 107) | TTAGTATGTTGGAAAGTA (SEQ ID NO: 2152) |
| 181 | THBS1 (SEQ ID NO: 81) | TAAAGGGGCGTTCGTA (SEQ ID NO: 1726) |
| 182 | THBS1 (SEQ ID NO: 81) | AAGGGGTGTTTGTATT (SEQ ID NO: 1727) |
| 183 | THBS1 (SEQ ID NO: 81) | GGTTAGTTCGGGCGTA (SEQ ID NO: 1728) |
| 184 | THBS1 (SEQ ID NO: 81) | GGTTAGTTTGGGTGTA (SEQ ID NO: 1729) |
| 185 | THBS1 (SEQ ID NO: 81) | GGTTAGTTCGGGCGTA (SEQ ID NO: 1728) |
| 186 | THBS1 (SEQ ID NO: 81) | GGTTAGTTTGGGTGTA (SEQ ID NO: 1729) |
| 187 | THBS1 (SEQ ID NO: 81) | TTGTGCGTTCGGAGTA (SEQ ID NO: 1730) |
| 188 | THBS1 (SEQ ID NO: 81) | TGTGTTTGGAGTAGAG (SEQ ID NO: 1731) |
| 189 | IL6 (SEQ ID NO: 99) | TTCGGTTATACGTAGG (SEQ ID NO: 1736) |
| 190 | IL6 (SEQ ID NO: 99) | TTTTGGTTATATGTAGGG (SEQ ID NO: 1737) |
| 191 | IL6 (SEQ ID NO: 99) | AGTTTAGTCGGTTTCGT (SEQ ID NO: 1738) |
| 192 | IL6 (SEQ ID NO: 99) | AGTTTAGTTGGTTTTGT (SEQ ID NO: 1739) |
| 193 | IL6 (SEQ ID NO: 99) | AGTTTAGTCGGTTTCGT (SEQ ID NO: 1738) |
| 194 | IL6 (SEQ ID NO: 99) | AGTTTAGTTGGTTTTGT (SEQ ID NO: 1739) |
| 195 | HOXA5 (SEQ ID NO: 78) | TAGTTTTCGGTCGGAA (SEQ ID NO: 1748) |
| 196 | HOXA5 (SEQ ID NO: 78) | TAGTTTTTGGTTGGAAG (SEQ ID NO: 1749) |
| 197 | HOXA5 (SEQ ID NO: 78) | ATCGGTAGTTGACGGTT (SEQ ID NO: 1752) |
| 198 | HOXA5 (SEQ ID NO: 78) | ATTGGTAGTTGATGGTT (SEQ ID NO: 1753) |
| 199 | HOXA5 (SEQ ID NO: 78) | ATCGGTAGTTGACGGTT (SEQ ID NO: 1752) |
| 200 | HOXA5 (SEQ ID NO: 78) | ATTGGTAGTTGATGGTT (SEQ ID NO: 1753) |
| 201 | GPC3 (SEQ ID NO: 118) | AATAGTCGCGTTTAGG (SEQ ID NO: 1760) |
| 202 | GPC3 (SEQ ID NO: 118) | TAGTTGTGTTTAGGGAT (SEQ ID NO: 1761) |
| 203 | CLDN7 (SEQ ID NO: 87) | TTACGTTAAGTCGGGT (SEQ ID NO: 1764) |
| 204 | CLDN7 (SEQ ID NO: 87) | AGTTATGTTAAGTTGGG (SEQ ID NO: 1765) |
| 205 | SLIT2 (SEQ ID NO: 112) | ATTTCGTCGTAGTTTG (SEQ ID NO: 1774) |
| 206 | SLIT2 (SEQ ID NO: 112) | TTTTGTTGTAGTTTGGA (SEQ ID NO: 1775) |
| 207 | SLIT2 (SEQ ID NO: 112) | TAGCGGGTTCGTAGTA (SEQ ID NO: 1776) |
| 208 | SLIT2 (SEQ ID NO: 112) | TTAGTGGGTTTGTAGTA (SEQ ID NO: 1777) |
| 209 | SLIT2 (SEQ ID NO: 112) | AAGGCGCGGAAGTTTA (SEQ ID NO: 1778) |
| 210 | SLIT2 (SEQ ID NO: 112) | AAGGTGTGGAAGTTTA (SEQ ID NO: 1779) |
| 211 | SLIT2 (SEQ ID NO: 112) | AAGGCGCGGAAGTTTA (SEQ ID NO: 1778) |
| 212 | SLIT2 (SEQ ID NO: 112) | AAGGTGTGGAAGTTTA (SEQ ID NO: 1779) |
| 213 | IGSF4 (SEQ ID NO: 74) | AGGTAGATCGAGGAGG (SEQ ID NO: 1780) |
| 214 | IGSF4 (SEQ ID NO: 74) | AGGTAGATTGAGGAGG (SEQ ID NO: 1781) |
| 215 | IGSF4 (SEQ ID NO: 74) | AGGTAGATCGAGGAGG (SEQ ID NO: 1780) |
| 216 | IGSF4 (SEQ ID NO: 74) | AGGTAGATTGAGGAGG (SEQ ID NO: 1781) |
| 217 | IGSF4 (SEQ ID NO: 74) | TAGTCGTAGAGTCGGG (SEQ ID NO: 1782) |

(continued)

| No: | Gene | Oligo: |
|---|---|---|
| 218 | IGSF4 (SEQ ID NO: 74) | GTTGTAGAGTTGGGTT (SEQ ID NO: 1783) |
| 219 | MCT1 (SEQ ID NO: 101) | AGTTAGTCGCGTTTTA (SEQ ID NO: 1788) |
| 220 | MCT1 (SEQ ID NO: 101) | AGAGTTAGTTGTGTTTTA (SEQ ID NO: 1789) |
| 221 | 221 SEQ ID NO:6 (SEQ ID NO: 6) | AAGTTTATCGGCGTTT (SEQ ID NO: 1792) |
| 222 | SEQ ID NO:6 (SEQ ID NO: 6) | AGAAGTTTATTGGTGTTT (SEQ ID NO: 1793) |
| 223 | SEQ ID NO:6 (SEQ ID NO: 6) | ATTTCGGAATTTAAGCGT (SEQ ID NO: 1794) |
| 224 | SEQ ID NO:6 (SEQ ID NO: 6) | TTTTGGAATTTAAGTGTT (SEQ ID NO: 1795) |
| 225 | SEQ ID NO:6 (SEQ ID NO: 6) | TAATTTCGGACGCGGA (SEQ ID NO: 1796) |
| 226 | SEQ ID NO:6 (SEQ ID NO: 6) | TTTTGGATGTGGAGGA (SEQ ID NO: 1797) |
| 227 | SEQ ID NO:6 (SEQ ID NO: 6) | TTACGGTGAAGGCGGA (SEQ ID NO: 1798) |
| 228 | SEQ ID NO:6 (SEQ ID NO: 6) | TTATGGTGAAGGTGGA (SEQ ID NO: 1799) |
| 229 | SEQ ID NO:6 (SEQ ID NO: 6) | TTACGGTGAAGGCGGA (SEQ ID NO: 1798) |
| 230 | SEQ ID NO:6 (SEQ ID NO: 6) | TTATGGTGAAGGTGGA (SEQ ID NO: 1799) |
| 231 | SEQ ID NO:6 (SEQ ID NO: 6) | TTTCGGTTTTCGTTAAT (SEQ ID NO: 1800) |
| 232 | SEQ ID NO:6 (SEQ ID NO: 6) | TTTGGTTTTTGTTAATTTAG (SEQ ID NO: 1801) |
| 233 | SEQ ID NO:8 (SEQ ID NO: 8) | ATAGGGCGGGATTTTA (SEQ ID NO: 2153) |
| 234 | SEQ ID NO:8 (SEQ ID NO: 8) | GATAGGGTGGGATTTT (SEQ ID NO: 2154) |
| 235 | SEQ ID NO:8 (SEQ ID NO: 8) | ATAAAGCGGGGTTTTA (SEQ ID NO: 1804) |
| 236 | SEQ ID NO:8 (SEQ ID NO: 8) | GGATAAAGTGGGGTTT (SEQ ID NO: 1805) |
| 237 | SEQ ID NO:8 (SEQ ID NO: 8) | AGGAGGCGAGAAATTT (SEQ ID NO: 1806) |
| 238 | SEQ ID NO:8 (SEQ ID NO: 8) | GAGGAGGTGAGAAATT (SEQ ID NO: 1807) |
| 239 | SEQ ID NO:8 (SEQ ID NO: 8) | AGAAATTTCGGGGTAG (SEQ ID NO: 1808) |
| 240 | SEQ ID NO:8 (SEQ ID NO: 8) | GAAATTTTGGGGTAGTA (SEQ ID NO: 1809) |
| 241 | SEQ ID NO:8 (SEQ ID NO: 8) | GGTAGTATCGTTTATAGA (SEQ ID NO: 1810) |
| 242 | SEQ ID NO:8 (SEQ ID NO: 8) | GGGGTAGTATTGTTTATA (SEQ ID NO: 1811) |
| 243 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGTGTATCGTTTTTCGG (SEQ ID NO: 1812) |
| 244 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAGTGTATTGTTTTTTGG (SEQ ID NO: 1813) |
| 245 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TGCGTATCGTTAGTTA (SEQ ID NO: 1814) |
| 246 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTTGTGTATTGTTAG (SEQ ID NO: 1815) |
| 247 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | ATTATTTCGGCGGTGA (SEQ ID NO: 1816) |

(continued)

| No: | Gene | Oligo: |
|---|---|---|
| 28 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GATTATTTTGGTGGTGA (SEQ ID NO: 1817) |
| 249 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAAGTCGCGTAAGGAG (SEQ ID NO: 1818) |
| 250 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AAGTTGTGTAAGGAGTA (SEQ ID NO: 1819) |
| 251 | MGC34831 (SEQ ID NO: 52) | GTCGGACGGATTTATA (SEQ ID NO: 2177) |
| 252 | MGC34831 (SEQ ID NO: 52) | TGGTTGGATGGATTTAT (SEQ ID NO: 2178) |
| 253 | SEQ ID NO:54 (SEQ ID NO: 54) | TGTGTAGCGGCGATTA (SEQ ID NO: 1830) |
| 254 | SEQ ID NO:54 (SEQ ID NO: 54) | GTGTGTAGTGGTGATT (SEQ ID NO: 1831) |
| 255 | ARL7 (SEQ ID NO: 30) | TAGATTTCGTAGTTTTTTA (SEQ ID NO: 1858) |
| 256 | ARL7 (SEQ ID NO: 30) | TAGATTTTGTAGTTTTTTAA (SEQ ID NO: 1859) |
| 257 | ARL7 (SEQ ID NO: 30) | TGGGTACGTTTACGGT (SEQ ID NO: 1860) |
| 258 | ARL7 (SEQ ID NO: 30) | TGGGTATGTTTATGGTT (SEQ ID NO: 1861) |
| 259 | ARL7 (SEQ ID NO: 30) | GAAGAAATCGTTTTTGT (SEQ ID NO: 1862) |
| 260 | ARL7 (SEQ ID NO: 30) | GAAGAAATTGTTTTTGTT (SEQ ID NO: 1863) |
| 261 | ARL7 (SEQ ID NO: 30) | TAGTAGGATCGGTTTTT (SEQ ID NO: 1864) |
| 262 | ARL7 (SEQ ID NO: 30) | ATAGTAGGATTGGTTTTT (SEQ ID NO: 1865) |
| 263 | THH (SEQ ID NO: 32) | TTCGGAAGAAAAGCGAAT (SEQ ID NO: 1870) |
| 264 | THH (SEQ ID NO: 32) | TTTGGAAGAAAAGTGAAT (SEQ ID NO: 1871) |
| 265 | THH (SEQ ID NO: 32) | TTCGGAAGAAAAGCGAAT (SEQ ID NO: 1870) |
| 266 | THH (SEQ ID NO: 32) | TTTGGAAGAAAAGTGAAT (SEQ ID NO: 1871) |
| 267 | THH (SEQ ID NO: 32) | TATTCGGAAGTGATCGG (SEQ ID NO: 1874) |
| 268 | THH (SEQ ID NO: 32) | TATTTGGAAGTGATTGG (SEQ ID NO: 1875) |
| 269 | THH (SEQ ID NO: 32) | TATTCGGAAGTGATCGG (SEQ ID NO: 1874) |
| 270 | THH (SEQ ID NO: 32) | TATTTGGAAGTGATTGG (SEQ ID NO: 1875) |
| 271 | SENP3 (SEQ ID NO: 39) | TATTCGGATCGGGTTT (SEQ ID NO: 1876) |
| 272 | SENP3 (SEQ ID NO: 39) | TTTATTTGGATTGGGTT (SEQ ID NO: 1877) |
| 273 | SENP3 (SEQ ID NO: 39) | TAGTCGAAAGTAGGACGT (SEQ ID NO: 1878) |
| 274 | SENP3 (SEQ ID NO: 39) | TAGTTGAAAGTAGGATGT (SEQ ID NO: 1879) |
| 275 | SENP3 (SEQ ID NO: 39) | TAGTCGAAAGTAGGACGT (SEQ ID NO: 1878) |
| 276 | SENP3 (SEQ ID NO: 39) | TAGTTGAAAGTAGGATGT (SEQ ID NO: 1879) |
| 277 | SENP3 (SEQ ID NO: 39) | AGGACGTTTTTGATCGG (SEQ ID NO: 1880) |
| 278 | SENP3 (SEQ ID NO: 39) | AGGATGTTTTTGATTGG (SEQ ID NO: 1881) |
| 279 | SENP3 (SEQ ID NO: 39) | AGGACGTTTTTGATCGG (SEQ ID NO: 1880) |
| 280 | SENP3 (SEQ ID NO: 39) | AGGATGTTTTTGATTGG (SEQ ID NO: 1881) |
| 281 | SENP3 (SEQ ID NO: 39) | AGTTATCGTTAGGAGGG (SEQ ID NO: 1882) |

(continued)

| No: | Gene | Oligo: |
|---|---|---|
| 282 | SENP3 (SEQ ID NO: 39) | AGTTATTGTTAGGAGGG (SEQ ID NO: 1883) |
| 283 | SENP3 (SEQ ID NO: 39) | AGTTATCGTTAGGAGGG (SEQ ID NO: 1882) |
| 284 | SENP3 (SEQ ID NO: 39) | AGTTATTGTTAGGAGGG (SEQ ID NO: 1883) |
| 285 | SEQ ID NO:42 (SEQ ID NO: 42) | AGGTCGAATAAAGCGT (SEQ ID NO: 2179) |
| 286 | SEQ ID NO:42 (SEQ ID NO: 42) | GAGGTTGAATAAAGTGT (SEQ ID NO: 2180) |
| 287 | 060279 (SEQ ID NO: 47) | TAATTATCGGCGGTGT (SEQ ID NO: 1900) |
| 288 | 060279 (SEQ ID NO: 47) | ATTATTGGTGGTGTTTT (SEQ ID NO: 1901) |
| 289 | SEQ ID NO:48 (SEQ ID NO: 48) | TATTTGGCGATTCGGA (SEQ ID NO: 1904) |
| 290 | SEQ ID NO:48 (SEQ ID NO: 48) | TGGTGATTTGGAGATT (SEQ ID NO: 1905) |
| 291 | SEQ ID NO:48 (SEQ ID NO: 48) | TAGGGTTACGTGTCGG (SEQ ID NO: 1906) |
| 292 | SEQ ID NO:48 (SEQ ID NO: 48) | TAGGGTTATGTGTTGG (SEQ ID NO: 1907) |
| 293 | SEQ ID NO:48 (SEQ ID NO: 48) | TAGGGTTACGTGTCGG (SEQ ID NO: 1906) |
| 294 | SEQ ID NO:48 (SEQ ID NO: 48) | TAGGGTTATGTGTTGG (SEQ ID NO: 1907) |
| 295 | SEQ ID NO:48 (SEQ ID NO: 48) | AACGAATTTTTCGATATT (SEQ ID NO: 1908) |
| 296 | SEQ ID NO:48 (SEQ ID NO: 48) | TTTAATGAATTTTTTGATATT (SEQ ID NO: 1909) |
| 297 | SEQ ID NO:4 (SEQ ID NO: 4) | TTTTGTTCGCGTTGAA (SEQ ID NO: 1916) |
| 298 | SEQ ID NO:4 (SEQ ID NO: 4) | TTGTTTGTGTTGAAGTA (SEQ ID NO: 1917) |
| 299 | SEQ ID NO:5 (SEQ ID NO: 5) | AATGAGCGAGAAAGTA (SEQ ID NO: 1924) |
| 300 | SEQ ID NO:5 (SEQ ID NO: 5) | AGAATGAGTGAGAAAGT (SEQ ID NO: 1925) |
| 301 | SEQ ID NO:5 (SEQ ID NO: 5) | ATTAAACGGGATGGTT (SEQ ID NO: 1926) |
| 302 | SEQ ID NO:5 (SEQ ID NO: 5) | AATATTAAATGGGATGGT (SEQ ID NO: 1927) |
| 303 | SEQ ID NO:5 (SEQ ID NO: 5) | GAGTTGCGAGGATTTT (SEQ ID NO: 1928) |
| 304 | SEQ ID NO:5 (SEQ ID NO: 5) | GGAGTTGTGAGGATTT (SEQ ID NO: 1929) |
| 305 | SEQ ID NO:5 (SEQ ID NO: 5) | TATGAGGTCGTATTGG (SEQ ID NO: 2163) |
| 306 | SEQ ID NO:5 (SEQ ID NO: 5) | TATGAGGTTGTATTGGT (SEQ ID NO: 2164) |
| 307 | SEQ ID NO:5 (SEQ ID NO: 5) | GGGAATCGTTGATTTT (SEQ ID NO: 1930) |
| 308 | SEQ ID NO:5 (SEQ ID NO: 5) | AGGGGAATTGTTGATT (SEQ ID NO: 1931) |
| 309 | SEQ ID NO:1 (SEQ ID NO: 1) | GGTCGGCGTTGATTTTA (SEQ ID NO: 1942) |
| 310 | SEQ ID NO:1 (SEQ ID NO: 1) | GGTTGGTGTTGATTTTA (SEQ ID NO: 1943) |
| 311 | SEQ ID NO:1 (SEQ ID NO: 1) | GGTCGGCGTTGATTTTA (SEQ ID NO: 1942) |
| 312 | SEQ ID NO:1 (SEQ ID NO: 1) | GGTTGGTGTTGATTTTA (SEQ ID NO: 1943) |
| 313 | SEQ ID NO:1 (SEQ ID NO: 1) | GTCGGAAGTTTCGGGA (SEQ ID NO: 1948) |
| 314 | SEQ ID NO:1 (SEQ ID NO: 1) | GTTGGAAGTTTTGGGAT (SEQ ID NO: 1949) |
| 315 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 316 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |

(continued)

| No: | Gene | Oligo: |
|---|---|---|
| 317 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 318 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |
| 319 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GGCGTCGAAAGTCGTT (SEQ ID NO: 1954) |
| 320 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GGTGTTGAAAGTTGTTG (SEQ ID NO: 1955) |
| 321 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAATCGTTTGTTTACGT (SEQ ID NO: 1956) |
| 322 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AATTGTTTGTTTATGTAGT (SEQ ID NO: 1957) |
| 323 | LIM DOMAIN KINASE 1 (EC 2.7.1.37) (LIMK-1) (SEQ ID NO: 12) | TGTAGTCGGGAGGTTA (SEQ ID NO: 1966) |
| 324 | LIM DOMAIN KINASE 1 (EC 2.7.1.37) (LIMK-1) (SEQ ID NO: 12) | TGTAGTTGGGAGGTTA (SEQ ID NO: 1967) |
| 325 | LIM DOMAIN KINASE 1 (EC 2.7.1.37) (LIMK-1) (SEQ ID NO: 12) | TGTAGTCGGGAGGTTA (SEQ ID NO: 1966) |
| 326 | LIM DOMAIN KINASE 1 (EC 2.7.1.37) (LIMK-1) (SEQ ID NO: 12) | TGTAGTTGGGAGGTTA (SEQ ID NO: 1967) |
| 327 | LIM DOMAIN KINASE 1 (EC 2.7.1.37) (LIMK-1) (SEQ ID NO: 12) | GGATTATCGCGGGGGT (SEQ ID NO: 1968) |
| 328 | LIM DOMAIN KINASE 1 (EC 2.7.1.37) (LIMK-1) (SEQ ID NO: 12) | GGATTATTGTGGGGGT (SEQ ID NO: 1969) |
| 329 | LIM DOMAIN KINASE 1 (EC 2.7.1.37) (LIMK-1) (SEQ ID NO: 12) | GGATTATCGCGGGGGT (SEQ ID NO: 1968) |
| 330 | LIM DOMAIN KINASE 1 (EC 2.7.1.37) (LIMK-1) (SEQ ID NO: 12) | GGATTATTGTGGGGGT (SEQ ID NO: 1969) |
| 331 | LIM DOMAIN KINASE 1 (EC 2.7.1.37) (LIMK-1) (SEQ ID NO: 12) | GTCGGTAGTTTATCGGAT (SEQ ID NO: 1970) |
| 332 | LIM DOMAIN KINASE 1 (EC 2.7.1.37) (LIMK-1) (SEQ ID NO: 12) | GTTGGTAGTTTATTGGAT (SEQ ID NO: 1971) |
| 333 | LIM DOMAIN KINASE 1 (EC 2.7.1.37) (LIMK-1) (SEQ ID NO: 12) | GTCGGTAGTTTATCGGAT (SEQ ID NO: 1970) |
| 334 | LIM DOMAIN KINASE 1 (EC 2.7.1.37) (LIMK-1) (SEQ ID NO: 12) | GTTGGTAGTTTATTGGAT (SEQ ID NO: 1971) |
| 335 | MSF (SEQ ID NO: 13) | GTTTCGAAATTGGCGT (SEQ ID NO: 1980) |
| 336 | MSF (SEQ ID NO: 13) | TTTGAAATTGGTGTGG (SEQ ID NO: 1981) |
| 337 | MSF (SEQ ID NO: 13) | TTCGGTTTACGGGTTGTA (SEQ ID NO: 1982) |

(continued)

| No: | Gene | Oligo: |
|---|---|---|
| 338 | MSF (SEQ ID NO: 13) | TTTGGTTTATGGGTTGTA (SEQ ID NO: 1983) |
| 339 | MSF (SEQ ID NO: 13) | TTCGGTTTACGGGTTGTA (SEQ ID NO: 1982) |
| 340 | MSF (SEQ ID NO: 13) | TTTGGTTTATGGGTTGTA (SEQ ID NO: 1983) |
| 341 | MSF (SEQ ID NO: 13) | TTACGGTTCGATTTTG (SEQ ID NO: 1984) |
| 342 | MSF (SEQ ID NO: 13) | TATGGTTTGATTTTGGG (SEQ ID NO: 1985) |
| 343 | SEQ ID NO:16 (SEQ ID NO: 16) | TTACGGATAGGGCGAT (SEQ ID NO: 1994) |
| 344 | SEQ ID NO:16 (SEQ ID NO: 16) | TATGGATAGGGTGATTT (SEQ ID NO: 1995) |
| 345 | SEQ ID NO:16 (SEQ ID NO: 16) | AGGCGTTGTCGGTGAT (SEQ ID NO: 1996) |
| 346 | SEQ ID NO:16 (SEQ ID NO: 16) | AGGTGTTGTTGGTGATA (SEQ ID NO: 1997) |
| 347 | SEQ ID NO:17 (SEQ ID NO: 17) | TACGTTTCGGGTTTGTTA (SEQ ID NO: 1998) |
| 348 | SEQ ID NO:17 (SEQ ID NO: 17) | TATGTTTTGGGTTTGTTA (SEQ ID NO: 1999) |
| 349 | SEQ ID NO:17 (SEQ ID NO: 17) | TACGTTTCGGGTTTGTTA (SEQ ID NO: 1998) |
| 350 | SEQ ID NO:17 (SEQ ID NO: 17) | TATGTTTTGGGTTTGTTA (SEQ ID NO: 1999) |
| 351 | SEQ ID NO:18 (SEQ ID NO: 18) | TTACGTCGTTATTAGGT (SEQ ID NO: 2004) |
| 352 | SEQ ID NO:18 (SEQ ID NO: 18) | TTTTATGTTGTTATTAGGT (SEQ ID NO: 2005) |
| 353 | SEQ ID NO:18 (SEQ ID NO: 18) | AAAGCGGAGTCGTTAG (SEQ ID NO: 2010) |
| 354 | SEQ ID NO:18 (SEQ ID NO: 18) | AGTGGAGTTGTTAGGT (SEQ ID NO: 2011) |
| 355 | SEQ ID NO:19 (SEQ ID NO: 19) | AGGTTTTCGTTGTAGTA (SEQ ID NO: 2012) |
| 356 | SEQ ID NO:19 (SEQ ID NO: 19) | TAGGTTTTTGTTGTAGTA (SEQ ID NO: 2013) |
| 357 | SEQ ID NO:19 (SEQ ID NO: 19) | TGAGATTCGTTTTTTAAA (SEQ ID NO: 2014) |
| 358 | SEQ ID NO:19 (SEQ ID NO: 19) | GGTGAGATTTGTTTTTTA (SEQ ID NO: 2015) |
| 359 | PRDM6 (SEQ ID NO: 20) | TTGTCGGGTTACGGGA (SEQ ID NO: 2020) |
| 360 | PRDM6 (SEQ ID NO: 20) | GTTGGGTTATGGGAGA (SEQ ID NO: 2021) |
| 361 | NR2E1 (SEQ ID NO: 22) | AATGTAGCGGCGTTAT (SEQ ID NO: 2028) |
| 362 | NR2E1 (SEQ ID NO: 22) | TAAATGTAGTGGTGTTAT (SEQ ID NO: 2029) |
| 363 | NR2E1 (SEQ ID NO: 22) | GTCGTTATCGGTTTGGA (SEQ ID NO: 2030) |
| 364 | NR2E1 (SEQ ID NO: 22) | GTTGTTATTGGTTTGGA (SEQ ID NO: 2031) |
| 365 | NR2E1 (SEQ ID NO: 22) | GTCGTTATCGGTTTGGA (SEQ ID NO: 2030) |
| 366 | NR2E1 (SEQ ID NO: 22) | GTTGTTATTGGTTTGGA (SEQ ID NO: 2031) |
| 367 | NR2E1 (SEQ ID NO: 22) | GACGTAAGTTTCGGGT (SEQ ID NO: 2032) |
| 368 | NR2E1 (SEQ ID NO: 22) | GGATGTAAGTTTTGGG (SEQ ID NO: 2033) |
| 369 | NR2E1 (SEQ ID NO: 22) | TTCGGGTGATATCGTTT (SEQ ID NO: 2036) |
| 370 | NR2E1 (SEQ ID NO: 22) | TTTGGGTGATATTGTTT (SEQ ID NO: 2037) |
| 371 | NR2E1 (SEQ ID NO: 22) | TTCGGGTGATATCGTTT (SEQ ID NO: 2036) |
| 372 | NR2E1 (SEQ ID NO: 22) | TTTGGGTGATATTGTTT (SEQ ID NO: 2037) |
| 373 | NR2E1 (SEQ ID NO: 22) | AGGCGAGTCGGAGTTT (SEQ ID NO: 2038) |
| 374 | NR2E1 (SEQ ID NO: 22) | AGGTGAGTTGGAGTTTT (SEQ ID NO: 2039) |
| 375 | NR2E1 (SEQ ID NO: 22) | TAAGTCGAGCGAGTTT (SEQ ID NO: 2040) |

(continued)

| No: | Gene | Oligo: |
|---|---|---|
| 376 | NR2E1 (SEQ ID NO: 22) | TAGTAAGTTGAGTGAGT (SEQ ID NO: 2041) |
| 377 | NR2E1 (SEQ ID NO: 22) | AGGGACGCGAAAATTT (SEQ ID NO: 2042) |
| 378 | NR2E1 (SEQ ID NO: 22) | GGAGGGATGTGAAAAT (SEQ ID NO: 2043) |
| 379 | PCDH7 (SEQ ID NO: 23) | ATCGTAGTCGGTTTTA (SEQ ID NO: 2044) |
| 380 | PCDH7 (SEQ ID NO: 23) | GATTATTGTAGTTGGTTT (SEQ ID NO: 2045) |
| 381 | RTTN (SEQ ID NO: 25) | TAGTGGCGCGGTAGTT (SEQ ID NO: 2046) |
| 382 | RTTN (SEQ ID NO: 25) | TAGTGGTGTGGTAGTTT (SEQ ID NO: 2047) |
| 383 | RTTN (SEQ ID NO: 25) | TAGGACGTGTTTTCGG (SEQ ID NO: 2048) |
| 384 | RTTN (SEQ ID NO: 25) | AGGATGTGTTTTTGGG (SEQ ID NO: 2049) |
| 385 | SNAP25 (SEQ ID NO: 33) | GTTATTATTCGGTACGT (SEQ ID NO: 2169) |
| 386 | SNAP25 (SEQ ID NO: 33) | AGTTATTATTTGGTATGTAT (SEQ ID NO: 2170) |
| 387 | SEQ ID NO:26 (SEQ ID NO: 26) | TTAAGTATCGAGGCGT (SEQ ID NO: 2054) |
| 388 | SEQ ID NO:26 (SEQ ID NO: 26) | TTTTAAGTATTGAGGTGT (SEQ ID NO: 2055) |
| 389 | SEQ ID NO:26 (SEQ ID NO: 26) | AATTTTGGTCGTTTAGT (SEQ ID NO: 2056) |
| 390 | SEQ ID NO:26 (SEQ ID NO: 26) | AAATTTTGGTTGTTTAGT (SEQ ID NO: 2057) |
| 391 | SEQ ID NO:26 (SEQ ID NO: 26) | TTCGTATTGACGTTAAT (SEQ ID NO: 2058) |
| 392 | SEQ ID NO:26 (SEQ ID NO: 26) | TTTGTATTGATGTTAATAGA (SEQ ID NO: 2059) |
| 393 | SEQ ID NO:28 (SEQ ID NO: 28) | ATTAGGCGAGTTTCGT (SEQ ID NO: 2066) |
| 394 | SEQ ID NO:28 (SEQ ID NO: 28) | TTAGGTGAGTTTTGTTT (SEQ ID NO: 2067) |
| 395 | SEQ ID NO:31 (SEQ ID NO: 31) | TTTACGTAGGGCGATT (SEQ ID NO: 2068) |
| 396 | SEQ ID NO:31 (SEQ ID NO: 31) | ATTTTTATGTAGGGTGAT (SEQ ID NO: 2069) |
| 397 | SEQ ID NO:31 (SEQ ID NO: 31) | GATACGGTTAGGCGGG (SEQ ID NO: 2070) |
| 398 | SEQ ID NO:31 (SEQ ID NO: 31) | GATATGGTTAGGTGGG (SEQ ID NO: 2071) |
| 399 | SEQ ID NO:31 (SEQ ID NO: 31) | GATACGGTTAGGCGGG (SEQ ID NO: 2070) |
| 400 | SEQ ID NO:31 (SEQ ID NO: 31) | GATATGGTTAGGTGGG (SEQ ID NO: 2071) |
| 401 | SEQ ID NO:31 (SEQ ID NO: 31) | TTCGGGCGTTTTATAT (SEQ ID NO: 2072) |
| 402 | SEQ ID NO:31 (SEQ ID NO: 31) | GGTTTGGGTGTTTTATA (SEQ ID NO: 2073) |
| 403 | HOXB13 (SEQ ID NO: 34) | AAGTTAGCGGGTTCGT (SEQ ID NO: 2076) |
| 404 | HOXB13 (SEQ ID NO: 34) | AAGTTAGTGGGTTTGT (SEQ ID NO: 2077) |
| 405 | HOXB13 (SEQ ID NO: 34) | AAGTTAGCGGGTTCGT (SEQ ID NO: 2076) |
| 406 | HOXB13 (SEQ ID NO: 34) | AAGTTAGTGGGTTTGT (SEQ ID NO: 2077) |
| 407 | (SEQ ID NO: 35) | GGAGGTGCGAATTTAA (SEQ ID NO: 2080) |
| 408 | (SEQ ID NO: 35) | GGGAGGTGTGAATTTA (SEQ ID NO: 2081) |
| 409 | (SEQ ID NO: 35) | AAATAGTCGTTTGGGA (SEQ ID NO: 2082) |
| 410 | (SEQ ID NO: 35) | AAATAGTTGTTTGGGAG (SEQ ID NO: 2083) |
| 411 | (SEQ ID NO: 35) | AGAAAATATACGAGATTTAT (SEQ ID NO: 2084) |
| 412 | (SEQ ID NO: 35) | AGAAAATATATGAGATTTATT (SEQ ID NO: 2085) |
| 413 | (SEQ ID NO: 35) | TAAAGACGGGAAGAGA (SEQ ID NO: 2086) |

(continued)

| No: | Gene | Oligo: |
|---|---|---|
| 414 | (SEQ ID NO: 35) | ATAAAGATGGGAAGAGA (SEQ ID NO: 2087) |
| 415 | MGC10561 (SEQ ID NO: 37) | TTTTTTACGTTAAGGGG (SEQ ID NO: 2088) |
| 416 | MGC10561 (SEQ ID NO: 37) | TTTTTATGTTAAGGGGG (SEQ ID NO: 2089) |
| 417 | LMX1A (SEQ ID NO: 38) | GTCGGGTTTTTCGGAA (SEQ ID NO: 2092) |
| 418 | LMX1A (SEQ ID NO: 38) | GTTGGGTTTTTTGGAAG (SEQ ID NO: 2093) |
| 419 | LMX1A (SEQ ID NO: 38) | GTCGAGATTTTATCGAAA (SEQ ID NO: 2094) |
| 420 | LMX1A (SEQ ID NO: 38) | GTTGAGATTTTATTGAAAG (SEQ ID NO: 2095) |
| 421 | LMX1A (SEQ ID NO: 38) | AGTATTCGGGCGGGTA (SEQ ID NO: 2096) |
| 422 | LMX1A (SEQ ID NO: 38) | GTAGTATTTGGGTGGG (SEQ ID NO: 2097) |
| 423 | LMX1A (SEQ ID NO: 38) | AACGAAATTACGTGTAT (SEQ ID NO: 2098) |
| 424 | LMX1A (SEQ ID NO: 38) | TGAAATGAAATTATGTGTA (SEQ ID NO: 2099) |
| 425 | TITF1 (SEQ ID NO:41) | GTCGTGGGGATCGTAT (SEQ ID NO: 2104) |
| 426 | TITF1 (SEQ ID NO: 41) | GTTGTGGGGATTGTAT (SEQ ID NO: 2105) |
| 427 | TITF1 (SEQ ID NO: 41) | GTCGTGGGGATCGTAT (SEQ ID NO: 2104) |
| 428 | TITF1 (SEQ ID NO: 41) | GTTGTGGGGATTGTAT (SEQ ID NO: 2105) |
| 429 | TITF1 (SEQ ID NO: 41) | TTAGGTCGCGTTTGTA (SEQ ID NO: 2108) |
| 430 | TITF1 (SEQ ID NO: 41) | AGGTTGTGTTTGTAGA (SEQ ID NO: 2109) |
| 431 | DDX51 (SEQ ID NO: 43) | AACGTGCGGGGTTTTT (SEQ ID NO: 2116) |
| 432 | DDX51 (SEQ ID NO: 43) | TGAATGTGTGGGGTTT (SEQ ID NO: 2117) |
| 433 | SEQ ID NO:45 (SEQ ID NO: 45) | AGCGAACGTTTATTTT (SEQ ID NO: 2118) |
| 434 | SEQ ID NO:45 (SEQ ID NO: 45) | TAGGAGAGTGAATGTTT (SEQ ID NO: 2119) |
| 435 | SEQ ID NO:2 (SEQ ID NO: 2) | TAGTAGTCGGCGGGAG (SEQ ID NO: 2130) |
| 436 | SEQ ID NO:2 (SEQ ID NO: 2) | TAGTAGTTGGTGGGAG (SEQ ID NO: 2131) |
| 437 | SEQ ID NO:2 (SEQ ID NO: 2) | TAGTAGTCGGCGGGAG (SEQ ID NO: 2130) |
| 438 | SEQ ID NO:2 (SEQ ID NO: 2) | TAGTAGTTGGTGGGAG (SEQ ID NO: 2131) |
| 439 | SEQ ID NO:3 (SEQ ID NO: 3) | TTGCGTTAATTCGGTA (SEQ ID NO: 2132) |
| 440 | SEQ ID NO:3 (SEQ ID NO: 3) | AATTTGTGTTAATTTGGT (SEQ ID NO: 2133) |
| 441 | SEQ ID NO:3 (SEQ ID NO: 3) | AGTCGGGAGAGCGAAA (SEQ ID NO: 2134) |
| 442 | SEQ ID NO:3 (SEQ ID NO: 3) | AGTTGGGAGAGTGAAA (SEQ ID NO: 2135) |
| 443 | SEQ ID NO:3 (SEQ ID NO: 3) | AGTCGGGAGAGCGAAA (SEQ ID NO: 2134) |
| 444 | SEQ ID NO:3 (SEQ ID NO: 3) | AGTTGGGAGAGTGAAA (SEQ ID NO: 2135) |
| 445 | SEQ ID NO:3 (SEQ ID NO: 3) | GGTCGAAGAGTCGGGA (SEQ ID NO: 2136) |
| 446 | SEQ ID NO:3 (SEQ ID NO: 3) | GGTTGAAGAGTTGGGA (SEQ ID NO: 2137) |
| 447 | SEQ ID NO:3 (SEQ ID NO: 3) | GGTCGAAGAGTCGGGA (SEQ ID NO: 2136) |
| 448 | SEQ ID NO:3 (SEQ ID NO: 3) | GGTTGAAGAGTTGGGA (SEQ ID NO: 2137) |
| 449 | SEQ ID NO:3 (SEQ ID NO: 3) | ATGTTAGCGGGTCGAA (SEQ ID NO: 2138) |
| 450 | SEQ ID NO:3 (SEQ ID NO: 3) | ATGTTAGTGGGTTGAA (SEQ ID NO: 2139) |
| 451 | SEQ ID NO:3 (SEQ ID NO: 3) | ATGTTAGCGGGTCGAA (SEQ ID NO: 2138) |

(continued)

| No: | Gene | Oligo: |
|---|---|---|
| 452 | SEQ ID NO:3 (SEQ ID NO: 3) | ATGTTAGTGGGTTGAA (SEQ ID NO: 2139) |

Table 18: Breast cancer vs. lymphocytes

| No: | Gene | Oligo: |
|---|---|---|
| 1 | SCGB3A1 (SEQ ID NO: 115) | TATTGGCGTCGAGGTT (SEQ ID NO: 2181) |
| 2 | SCGB3A1 (SEQ ID NO: 115) | TATTGGTGTTGAGGTT (SEQ ID NO: 2182) |
| 3 | SCGB3A1 (SEQ ID NO: 115) | TATTGGCGTCGAGGTT (SEQ ID NO: 2181) |
| 4 | SCGB3A 1 (SEQ ID NO: 115) | TATTGGTGTTGAGGTT (SEQ ID NO: 2182) |
| 5 | SCGB3A1 (SEQ ID NO: 115) | GGCGTAGAAGGCGTTT (SEQ ID NO: 2140) |
| 6 | SCGB3A1 (SEQ ID NO: 115) | GGTGTAGAAGGTGTTT (SEQ ID NO: 2141) |
| 7 | SCGB3A1 (SEQ ID NO: 115) | GGCGTAGAAGGCGTTT (SEQ ID NO: 2140) |
| 8 | SCGB3A1 (SEQ ID NO: 115) | GGTGTAGAAGGTGTTT (SEQ ID NO: 2141) |
| 9 | SCGB3A1 (SEQ ID NO: 115) | TAGTGTTCGACGTTGT (SEQ ID NO: 1475) |
| 10 | SCGB3A1 (SEQ ID NO: 115) | TAGTGTTTGATGTTGTT (SEQ ID NO: 1476) |
| 11 | SASH1 (SEQ ID NO: 102) | TAGATTCGAGGTGCGG (SEQ ID NO: 1477) |
| 12 | SASH1 (SEQ ID NO: 102) | TAGATTTGAGGTGTGG (SEQ ID NO: 1478) |
| 13 | SASH1 (SEQ ID NO: 102) | TAGATTCGAGGTGCGG (SEQ ID NO: 1477) |
| 14 | SASH1 (SEQ ID NO: 102) | TAGATTTGAGGTGTGG (SEQ ID NO: 1478) |
| 15 | SASH1 (SEQ ID NO: 102) | ATTCGGTATTCGGGTAG (SEQ ID NO: 1479) |
| 16 | SASH1 (SEQ ID NO: 102) | ATTTGGTATTTGGGTAG (SEQ ID NO: 1480) |
| 17 | SASH1 (SEQ ID NO: 102) | ATTCGGTATTCGGGTAG (SEQ ID NO: 1479) |
| 18 | SASH 1 (SEQ ID NO: 102) | ATTTGGTATTTGGGTAG (SEQ ID NO: 1480) |
| 19 | ARH1/NOEY2 (SEQ ID NO: 97) | TAAAACGTTCGGTAGG (SEQ ID NO: 1485) |
| 20 | ARH1/NOEY2 (SEQ ID NO: 97) | TTTAAAATGTTTGGTAGG (SEQ ID NO: 1486) |
| 21 | ARH1/NOEY2 (SEQ ID NO: 97) | TGTATTCGTCGTTAGG (SEQ ID NO: 1487) |
| 22 | ARH1/NOEY2 (SEQ ID NO: 97) | AATGTATTTGTTGTTAGG (SEQ ID NO: 1488) |
| 23 | ARH1/NOEY2 (SEQ ID NO: 97) | TTAGACGGAGTTCGGA (SEQ ID NO: 1489) |
| 24 | ARH1/NOEY2 (SEQ ID NO: 97) | TAGATGGAGTTTGGAGA (SEQ ID NO: 1490) |
| 25 | ARH1/NOEY2 (SEQ ID NO: 97) | TAGACGTAGGCGTATT (SEQ ID NO: 1491) |
| 26 | ARH1/NOEY2 (SEQ ID NO: 97) | GGGTAGATGTAGGTGT (SEQ ID NO: 1492) |
| 27 | CCND2 (SEQ ID NO: 104) | TTAGGGTCGATCGTGT (SEQ ID NO: 1493) |
| 28 | CCND2 (SEQ ID NO: 104) | TAGGGTTGATTGTGTT (SEQ ID NO: 1494) |
| 29 | CCND2 (SEQ ID NO: 104) | TTATCGTAGTCGGTTT (SEQ ID NO: 1495) |
| 30 | CCND2 (SEQ ID NO: 104) | GATTTTATTGTAGTTGGT (SEQ ID NO: 1496) |
| 31 | CCND2 (SEQ ID NO: 104) | GAGTGAGGCGCGAAAT (SEQ ID NO: 1497) |
| 32 | CCND2 (SEQ ID NO: 104) | GAGTGAGGTGTGAAAT (SEQ ID NO: 1498) |
| 33 | CCND2 (SEQ ID NO: 104) | GAGTGAGGCGCGAAAT (SEQ ID NO: 1497) |
| 34 | CCND2 (SEQ ID NO: 104) | GAGTGAGGTGTGAAAT (SEQ ID NO: 1498) |

(continued)

| No: | Gene | Oligo: |
|---|---|---|
| 35 | CDKN1A (SEQ ID NO: 67) | ATTAGGGTCGCGTTGA (SEQ ID NO: 1501) |
| 36 | CDKN1A (SEQ ID NO: 67) | ATTAGGGTTGTGTTGA (SEQ ID NO: 1502) |
| 37 | CDKN1A (SEQ ID NO: 67) | ATTAGGGTCGCGTTGA (SEQ ID NO: 1501) |
| 38 | CDKN1A (SEQ ID NO: 67) | ATTAGGGTTGTGTTGA (SEQ ID NO: 1502) |
| 39 | CDKN2A (SEQ ID NO: 57) | GGCGTTGTTTAACGTAT (SEQ ID NO: 1503) |
| 40 | CDKN2A (SEQ ID NO: 57) | GGGTGTTGTTTAATGTA (SEQ ID NO: 1504) |
| 41 | CDKN2A (SEQ ID NO: 57) | AATAGTTACGGTCGGA (SEQ ID NO: 1505) |
| 42 | CDKN2A (SEQ ID NO: 57) | AGTTATGGTTGGAGGT (SEQ ID NO: 1506) |
| 43 | CDKN2A (SEQ ID NO: 57) | GTCGGAGGTCGATTTA (SEQ ID NO: 1507) |
| 44 | CDKN2A (SEQ ID NO: 57) | GGTTGGAGGTTGATTTA (SEQ ID NO: 1508) |
| 45 | DAPK1 (SEQ ID NO: 98) | TTTCGGAGATTCGGTT (SEQ ID NO: 1509) |
| 46 | DAPK1 (SEQ ID NO: 98) | TTTGGAGATTTGGTTTT (SEQ ID NO: 1510) |
| 47 | DAPK1 (SEQ ID NO: 98) | TTTTAGCGTCGGGGAG (SEQ ID NO: 1511) |
| 48 | DAPK1 (SEQ ID NO: 98) | TTTTAGTGTTGGGGAG (SEQ ID NO: 1512) |
| 49 | DAPK1 (SEQ ID NO: 98) | TTTTAGCGTCGGGGAG (SEQ ID NO: 1511) |
| 50 | DAPK1 (SEQ ID NO: 98) | TTTTAGTGTTGGGGAG (SEQ ID NO: 1512) |
| 51 | EYA4 (SEQ ID NO: 58) | GGTATAAAATCGTAAATTTT (SEQ ID NO: 1513) |
| 52 | EYA4 (SEQ ID NO: 58) | GGGTATAAAATTGTAAATTT (SEQ ID NO: 1514) |
| 53 | EYA4 (SEQ ID NO: 58) | TATGTAGTCGCGTAGT (SEQ ID NO: 1515) |
| 54 | EYA4 (SEQ ID NO: 58) | TTTATGTAGTTGTGTAGT (SEQ ID NO: 1516) |
| 55 | EYA4 (SEQ ID NO: 58) | GTTTAGATACGAAATGTT (SEQ ID NO: 1517) |
| 56 | EYA4 (SEQ ID NO: 58) | GTTTAGATATGAAATGTTAT (SEQ ID NO: 1518) |
| 57 | EYA4 (SEQ ID NO: 58) | AGTTTTGACGTCGTTT (SEQ ID NO: 1519) |
| 58 | EYA4 (SEQ ID NO: 58) | TTGATGTTGTTTTGGAA (SEQ ID NO: 1520) |
| 59 | FHIT (SEQ ID NO: 76) | TTTAAGTATCGATTTTAGT (SEQ ID NO: 2183) |
| 60 | FHIT (SEQ ID NO: 76) | TAAGTATTGATTTTAGTTTTA (SEQ ID NO: 2184) |
| 61 | FHIT (SEQ ID NO: 76) | GTTACGTTAGCGGGTT (SEQ ID NO: 1521) |
| 62 | FHIT (SEQ ID NO: 76) | GGTTATGTTAGTGGGT (SEQ ID NO: 1522) |
| 63 | GSTP1 (SEQ ID NO: 59) | GGCGATTTCGGGGATT (SEQ ID NO: 1525) |
| 64 | GSTP1 (SEQ ID NO: 59) | GGTGATTTTGGGGATTT (SEQ ID NO: 1526) |
| 65 | GSTP1 (SEQ ID NO: 59) | GACGTTCGGGGTGTAG (SEQ ID NO: 1527) |
| 66 | GSTP1 (SEQ ID NO: 59) | GATGTTTGGGGTGTAG (SEQ ID NO: 1528) |
| 67 | GSTP (SEQ ID NO: 59) | GACGTTCGGGGTGTAG (SEQ ID NO: 1527) |
| 68 | GSTP1 (SEQ ID NO: 59) | GATGTTTGGGGTGTAG (SEQ ID NO: 1528) |
| 69 | GSTP1 (SEQ ID NO: 59) | AGTTCGCGGGATTTTT (SEQ ID NO: 1529) |
| 70 | GSTP1 (SEQ ID NO: 59) | GGAGTTTGTGGGATTT (SEQ ID NO: 1530) |
| 71 | GSTP1 (SEQ ID NO: 59) | AGTTTTCGTTATTAGTGA (SEQ ID NO: 1531) |
| 72 | GSTP1 (SEQ ID NO: 59) | TAGTTTTTGTTATTAGTGA (SEQ ID NO: 1532) |

(continued)

| No: | Gene | Oligo: |
|---|---|---|
| 73 | HIC1 (SEQ ID NO: 85) | TATCGAAGTTTTCGGG (SEQ ID NO: 1533) |
| 74 | HIC1 (SEQ ID NO: 85) | TATTGAAGTTTTTGGGT (SEQ ID NO: 1534) |
| 75 | HIC1 (SEQ ID NO: 85) | TAGCGGTTATTTCGGT (SEQ ID NO: 1535) |
| 76 | HIC1 (SEQ ID NO: 85) | TTTAGTGGTTATTTTGGT (SEQ ID NO: 1536) |
| 77 | HIC1 (SEQ ID NO: 85) | TACGTTTTTCGTAGCGT (SEQ ID NO: 1537) |
| 78 | HIC1 (SEQ ID NO: 85) | ATTATGTTTTTTGTAGTGT (SEQ ID NO: 1538) |
| 79 | HIC1 (SEQ ID NO: 85) | TTCGGTTTTGTCGTATA (SEQ ID NO: 1539) |
| 80 | HIC 1 (SEQ ID NO: 85) | TTTGGTTTTGTTGTATAG (SEQ ID NO: 1540) |
| 81 | PGR (SEQ ID NO: 83) | TTTCGAGGTCGGATTT (SEQ ID NO: 1543) |
| 82 | PGR (SEQ ID NO: 83) | TTTGAGGTTGGATTTTT (SEQ ID NO: 1544) |
| 83 | PGR (SEQ ID NO: 83) | GTGTCGTTTAGTCGTA (SEQ ID NO: 1545) |
| 84 | PGR (SEQ ID NO: 83) | GTTGTTTAGTTGTAGGT (SEQ ID NO: 1546) |
| 85 | SERPINB5 (SEQ ID NO: 68) | TTATTAACGTGTTTGAGA (SEQ ID NO: 1549) |
| 86 | SERPINB5 (SEQ ID NO: 68) | TTATTAATGTGTTTGAGAA (SEQ ID NO: 1550) |
| 87 | SERPINB5 (SEQ ID NO: 68) | ATTGTCGTACGTATGT (SEQ ID NO: 1551) |
| 88 | SERPINB5 (SEQ ID NO: 68) | AGAGGATTGTTGTATGTA (SEQ ID NO: 1552) |
| 89 | SERPINB5 (SEQ ID NO: 68) | TTTTTTGTTCGAATATGT (SEQ ID NO: 1553) |
| 90 | SERPINB5 (SEQ ID NO: 68) | TTTGTTTGAATATGTTGG (SEQ ID NO: 1554) |
| 91 | RARB (SEQ ID NO: 88) | ATGTCGAGAACGCGAG (SEQ ID NO: 1555) |
| 92 | RARB (SEQ ID NO: 88) | GGATGTTGAGAATGTGA (SEQ ID NO: 1556) |
| 93 | RARB (SEQ ID NO: 88) | AGCGATTCGAGTAGGG (SEQ ID NO: 1557) |
| 94 | RARB (SEQ ID NO: 88) | AGTGATTTGAGTAGGG (SEQ ID NO: 1558) |
| 95 | RARB (SEQ ID NO: 88) | AGCGATTCGAGTAGGG (SEQ ID NO: 1557) |
| 96 | RARB (SEQ ID NO: 88) | AGTGATTTGAGTAGGG (SEQ ID NO: 1558) |
| 97 | RARB (SEQ ID NO: 88) | TAGGATTCGGAACGTA (SEQ ID NO: 1559) |
| 98 | RARB (SEQ ID NO: 88) | GGTAGGATTTGGAATGT (SEQ ID NO: 1560) |
| 99 | SFN (SEQ ID NO: 69) | TAGTACGGTGTCGTAT (SEQ ID NO: 1561) |
| 100 | SFN (SEQ ID NO: 69) | GTTTAGTATGGTGTTGT (SEQ ID NO: 1562) |
| 101 | SFN (SEQ ID NO: 69) | TACGTATTTCGGGTTT (SEQ ID NO: 1563) |
| 102 | SFN (SEQ ID NO: 69) | TATTTATGTATTTTGGGTT (SEQ ID NO: 1564) |
| 103 | SFN (SEQ ID NO: 69) | TTCGTTTTTCGTAGGAG (SEQ ID NO: 1565) |
| 104 | SFN (SEQ ID NO: 69) | TTTGTTTTTTTGTAGGAGA (SEQ ID NO: 1566) |
| 105 | SFN (SEQ ID NO: 69) | TTTATAGCGTTCGGTT (SEQ ID NO: 2185) |
| 106 | SFN (SEQ ID NO: 69) | ATAGTGTTTGGTTTGTT (SEQ ID NO: 2186) |
| 107 | TERT (SEQ ID NO: 92) | AGGTGTACGGTTTCGT (SEQ ID NO: 2187) |
| 108 | TERT (SEQ ID NO: 92) | AGGTGTATGGTTTTGT (SEQ ID NO: 2188) |
| 109 | TERT (SEQ ID NO: 92) | AGGTGTACGGTTTCGT (SEQ ID NO: 2187) |
| 110 | TERT (SEQ ID NO: 92) | AGGTGTATGGTTTTGT (SEQ ID NO: 2188) |

(continued)

| No: | Gene | Oligo: |
|-----|------|--------|
| 111 | TERT (SEQ ID NO: 92) | TATAACGAACGTCGTT (SEQ ID NO: 2142) |
| 112 | TERT (SEQ ID NO: 92) | AGGTATAATGAATGTTGT (SEQ ID NO: 2143) |
| 113 | TGFBR2 (SEQ ID NO: 93) | ATGGGGCGGACGAATA (SEQ ID NO: 1567) |
| 114 | TGFBR2 (SEQ ID NO: 93) | ATGGGGTGGATGAATA (SEQ ID NO: 1568) |
| 115 | TGFBR2 (SEQ ID NO: 93) | ATGGGGCGGACGAATA (SEQ ID NO: 1567) |
| 116 | TGFBR2 (SEQ ID NO: 93) | ATGGGGTGGATGAATA (SEQ ID NO: 1568) |
| 117 | THRB (SEQ ID NO: 106) | GGGCGGTTAAGTCGAG (SEQ ID NO: 1569) |
| 118 | THRB (SEQ ID NO: 106) | GGGTGGTTAAGTTGAG (SEQ ID NO: 1570) |
| 119 | THRB (SEQ ID NO: 106) | GGGCGGTTAAGTCGAG (SEQ ID NO: 1569) |
| 120 | THRB (SEQ ID NO: 106) | GGGTGGTTAAGTTGAG (SEQ ID NO: 1570) |
| 121 | TIMP3 (SEQ ID NO: 103) | TTATTAACGGAGGAAGG (SEQ ID NO: 1571) |
| 122 | TIMP3 (SEQ ID NO: 103) | TATTAATGGAGGAAGGG (SEQ ID NO: 1572) |
| 123 | CDH13 (SEQ ID NO: 70) | TAAAACGAGGGAGCGT (SEQ ID NO: 1583) |
| 124 | CDH13 (SEQ ID NO: 70) | AAAATGAGGGAGTGTT (SEQ ID NO: 1584) |
| 125 | CDH13 (SEQ ID NO: 70) | TAGTCGCGTGTATGAA (SEQ ID NO: 1585) |
| 126 | CDH13 (SEQ ID NO: 70) | TGTAGTTGTGTGTATGA (SEQ ID NO: 1586) |
| 127 | CDH13 (SEQ ID NO: 70) | ATGAAAACGTCGTCGG (SEQ ID NO: 1587) |
| 128 | CDH13 (SEQ ID NO: 70) | AATGAAAATGTTGTTGG (SEQ ID NO: 1588) |
| 129 | CDH13 (SEQ ID NO: 70) | TAGTCGAGAATTTCGT (SEQ ID NO: 1589) |
| 130 | CDH13 (SEQ ID NO: 70) | TGTAGTTGAGAATTTTGT (SEQ ID NO: 1590) |
| 131 | TMS1/ASC (SEQ ID NO: 84) | TTCGTTTCGGAGTCGA (SEQ ID NO: 1591) |
| 132 | TMS1/ASC (SEQ ID NO: 84) | TTTGTTTTGGAGTTGAT (SEQ ID NO: 1592) |
| 133 | APAF1 (SEQ ID NO: 82) | GTGTCGTAGCGGTATT (SEQ ID NO: 1593) |
| 134 | APAF1 (SEQ ID NO: 82) | GGTGTTGTAGTGGTAT (SEQ ID NO: 1594) |
| 135 | APAF1 (SEQ ID NO: 82) | AGTAGCGTCGGGTTTT (SEQ ID NO: 1595) |
| 136 | APAF1 (SEQ ID NO: 82) | GAGTAGTGTTGGGTTT (SEQ ID NO: 1596) |
| 137 | SYK (SEQ ID NO: 60) | GATCGATGCGGTTTAT (SEQ ID NO: 1599) |
| 138 | SYK (SEQ ID NO: 60) | GGGATTGATGTGGTTT (SEQ ID NO: 1600) |
| 139 | SYK (SEQ ID NO: 60) | TTATTCGGTCGGGATT (SEQ ID NO: 1603) |
| 140 | SYK (SEQ ID NO: 60) | TTTATTTGGTTGGGATT (SEQ ID NO: 1604) |
| 141 | FABP3 (SEQ ID NO: 77) | GTGATGCGAGGGTTAT (SEQ ID NO: 1607) |
| 142 | FABP3 (SEQ ID NO: 77) | GTGATGTGAGGGTTAT (SEQ ID NO: 1608) |
| 143 | FABP3 (SEQ ID NO: 77) | GTGATGCGAGGGTTAT (SEQ ID NO: 1607) |
| 144 | FABP3 (SEQ ID NO: 77) | GTGATGTGAGGGTTAT (SEQ ID NO: 1608) |
| 145 | FABP3 (SEQ ID NO: 77) | TAAAGCGGTAGTTCGG (SEQ ID NO: 1609) |
| 146 | FABP3 (SEQ ID NO: 77) | AAGTGGTAGTTTGGGT (SEQ ID NO: 1610) |
| 147 | FABP3 (SEQ ID NO: 77) | TATTGGCGTTGACGTA (SEQ ID NO: 1611) |
| 148 | FABP3 (SEQ ID NO: 77) | TGGTGTTGATGTAGGT (SEQ ID NO: 1612) |

(continued)

| No: | Gene | Oligo: |
|-----|------|--------|
| 149 | RASSF1A (SEQ ID NO: 90) | TACGGGTATTTTCGCGT (SEQ ID NO: 1613) |
| 150 | RASSF1A (SEQ ID NO: 90) | ATATGGGTATTTTTGTGT (SEQ ID NO: 1614) |
| 151 | RA SSF I A (SEQ ID NO: 90) | AGAGCGCGTTTAGTTT (SEQ ID NO: 1615) |
| 152 | RASSF1A (SEQ ID NO: 90) | GAGAGTGTGTTTAGTTT (SEQ ID NO: 1616) |
| 153 | RASSF1A (SEQ ID NO: 90) | AGTAAATCGGATTAGGA (SEQ ID NO: 1617) |
| 154 | RASSF1A (SEQ ID NO: 90) | AGTAAATTGGATTAGGAG (SEQ ID NO: 1618) |
| 155 | TWIST (SEQ ID NO: 100) | AGTAAAGGCGTTGCGT (SEQ ID NO: 1619) |
| 156 | TWIST (SEQ ID NO: 100) | AGTAAAGGTGTTGTGT (SEQ ID NO: 1620) |
| 157 | TWIST (SEQ ID NO: 100) | AGTAAAGGCGTTGCGT (SEQ ID NO: 1619) |
| 158 | TWIST (SEQ ID NO: 100) | AGTAAAGGTGTTGTGT (SEQ ID NO: 1620) |
| 159 | TWIST (SEQ ID NO: 100) | TATTTTTCGAGGCGTA (SEQ ID NO: 1621) |
| 160 | TWIST (SEQ ID NO: 100) | TTTTGAGGTGTAGTTTT (SEQ ID NO: 1622) |
| 161 | TWIST (SEQ ID NO: 100) | ATTGGGTCGTTGTAGA (SEQ ID NO: 1623) |
| 162 | TWIST (SEQ ID NO: 100) | ATTGGGTTGTTGTAGA (SEQ ID NO: 1624) |
| 163 | TWIST (SEQ ID NO: 100) | ATTGGGTCGTTGTAGA (SEQ ID NO: 1623) |
| 164 | TWIST (SEQ ID NO: 100) | ATTGGGTTGTTGTAGA (SEQ ID NO: 1624) |
| 165 | TWIST (SEQ ID NO: 100) | TAGGTCGGGACGTAAA (SEQ ID NO: 1625) |
| 166 | TWIST (SEQ ID NO: 100) | AGTAGGTTGGGATGTA (SEQ ID NO: 1626) |
| 167 | ESR2 (SEQ ID NO: 91) | TTTACGTGATCGAGTT (SEQ ID NO: 1631) |
| 168 | ESR2 (SEQ ID NO: 91) | AGTTTATGTGATTGAGTT (SEQ ID NO: 1632) |
| 169 | PLAU (SEQ ID NO: 62) | TATTTGTCGCGTTGAT (SEQ ID NO: 1633) |
| 170 | PLAU (SEQ ID NO: 62) | ATTTGTTGTGTTGATGA (SEQ ID NO: 1634) |
| 171 | PLAU (SEQ ID NO: 62) | TGTAATTCGGGGATTT (SEQ ID NO: 1635) |
| 172 | PLAU (SEQ ID NO: 62) | TTGTAATTTGGGGATTT (SEQ ID NO: 1636) |
| 173 | PLAU (SEQ ID NO: 62) | TTGGAGATCGCGTTTT (SEQ ID NO: 1637) |
| 174 | PLAU (SEQ ID NO: 62) | TTGGAGATTGTGTTTTT (SEQ ID NO: 1638) |
| 175 | PLAU (SEQ ID NO: 62) | GAGCGTTGCGGAAGTA (SEQ ID NO: 1639) |
| 176 | PLAU (SEQ ID NO: 62) | GAGTGTTGTGGAAGTA (SEQ ID NO: 1640) |
| 177 | PLAU (SEQ ID NO: 62) | GAGCGTTGCGGAAGTA (SEQ ID NO: 1639) |
| 178 | PLAU (SEQ ID NO: 62) | GAGTGTTGTGGAAGTA (SEQ ID NO: 1640) |
| 179 | STAT1 (SEQ ID NO: 109) | ATATGATTTCGGAATTTTA (SEQ ID NO: 2189) |
| 180 | STAT1 (SEQ ID NO: 109) | ATATGATTTTGGAATTTTAA (SEQ ID NO: 2190) |
| 181 | BRCA1 (SEQ ID NO: 66) | TTTCGTGGTAACGGAA (SEQ ID NO: 1645) |
| 182 | BRCA1 (SEQ ID NO: 66) | TTTGTGGTAATGGAAAA (SEQ ID NO: 1646) |
| 183 | LOT1 (SEQ ID NO: 95) | ATGGGTACGTTTAAGG (SEQ ID NO: 1649) |
| 184 | LOT1 (SEQ ID NO: 95) | TGGGTATGTTTAAGGG (SEQ ID NO: 1650) |
| 185 | LOT1 (SEQ ID NO: 95) | AAATTAGTTACGTTATTTAA (SEQ ID NO: 1651) |
| 186 | LOT1 (SEQ ID NO: 95) | TGAAATTAGTTATGTTATTTA (SEQ ID NO: 1652) |

(continued)

| No: | Gene | Oligo: |
|---|---|---|
| 187 | LOT1 (SEQ ID NO: 95) | ATGTCGGTTATTACGT (SEQ ID NO: 1653) |
| 188 | LOT1 (SEQ ID NO: 95) | TGTTGGTTATTATGTAGA (SEQ ID NO: 1654) |
| 189 | PRSS8 (SEQ ID NO: 72) | AGTTGGCGGAGTTTAG (SEQ ID NO: 1655) |
| 190 | PRSS8 (SEQ ID NO: 72) | AGTTGGTGGAGTTTAG (SEQ ID NO: 1656) |
| 191 | PRSS8 (SEQ ID NO: 72) | AGTTGGCGGAGTTTAG (SEQ ID NO: 1655) |
| 192 | PRSS8 (SEQ ID NO: 72) | AGTTGGTGGAGTTTAG (SEQ ID NO: 1656) |
| 193 | PRSS8 (SEQ ID NO: 72) | TTGGTGATTCGTTTATAT (SEQ ID NO: 1657) |
| 194 | PRSS8 (SEQ ID NO: 72) | GTTGGTGATTTGTTTATA (SEQ ID NO: 1658) |
| 195 | PRSS8 (SEQ ID NO: 72) | TGTTCGTTTCGGATAT (SEQ ID NO: 1659) |
| 196 | PRSS8 (SEQ ID NO: 72) | TTTGTTTTGGATATTTTAG (SEQ ID NO: 1660) |
| 197 | SLC19A1 (SEQ ID NO: 116) | GTCGTGCGGTTTTTAA (SEQ ID NO: 1663) |
| 198 | SLC19A1 (SEQ ID NO: 116) | GGTTGTGTGGTTTTTAA (SEQ ID NO: 1664) |
| 199 | SLC19A1 (SEQ ID NO: 116) | TTACGAAGGCGGTTTA (SEQ ID NO: 1665) |
| 200 | SLC19A1 (SEQ ID NO: 116) | TTTTATGAAGGTGGTTT (SEQ ID NO: 1666) |
| 201 | GJB2 (SEQ ID NO: 111) | GGATTTCGTCGGTATT (SEQ ID NO: 1667) |
| 202 | GJB2 (SEQ ID NO: 111) | GGGGATTTTGTTGGTA (SEQ ID NO: 1668) |
| 203 | HS3ST2 (SEQ ID NO: 113) | GAATCGGAGAGGCGAG (SEQ ID NO: 1671) |
| 204 | HS3ST2 (SEQ ID NO: 113) | AATTGGAGAGGTGAGG (SEQ ID NO: 1672) |
| 205 | HS3ST2 (SEQ ID NO: 113) | GGGTAATCGTTTGGTA (SEQ ID NO: 1673) |
| 206 | HS3ST2 (SEQ ID NO: 113) | GGGTAATTGTTTGGTAT (SEQ ID NO: 1674) |
| 207 | PRDM2 (SEQ ID NO: 114) | TGTAGAGACGACGATT (SEQ ID NO: 1675) |
| 208 | PRDM2 (SEQ ID NO: 114) | ATTGTAGAGATGATGATT (SEQ ID NO: 1676) |
| 209 | PRDM2 (SEQ ID NO: 114) | AGAGCGCGGTAGTAGT (SEQ ID NO: 1677) |
| 210 | PRDM2 (SEQ ID NO: 114) | TGAGAGTGTGGTAGTA (SEQ ID NO: 1678) |
| 211 | PRDM2 (SEQ ID NO: 114) | TGTTCGCGATGTTTTA (SEQ ID NO: 1679) |
| 212 | PRDM2 (SEQ ID NO: 114) | TGTTTGTGATGTTTTAGT (SEQ ID NO: 1680) |
| 213 | PRDM2 (SEQ ID NO: 114) | AGTATATAAACGTAGATTTT (SEQ ID NO: 1681) |
| 214 | PRDM2 (SEQ ID NO: 114) | AAGTATATAAATGTAGATTTT (SEQ ID NO: 1682) |
| 215 | ALX4 (SEQ ID NO: 64) | AAGTCGATCGTTTTGT (SEQ ID NO: 1683) |
| 216 | ALX4 (SEQ ID NO: 64) | TGGAAGTTGATTGTTTT (SEQ ID NO: 1684) |
| 217 | ALX4 (SEQ ID NO: 64) | TATTGCGAGGATTCGG (SEQ ID NO: 1685) |
| 218 | ALX4 (SEQ ID NO: 64) | ATTGTGAGGATTTGGT (SEQ ID NO: 1686) |
| 219 | ALX4 (SEQ ID NO: 64) | TTCGTAGCGTAGGGTT (SEQ ID NO: 1687) |
| 220 | ALX4 (SEQ ID NO: 64) | TTTGTAGTGTAGGGTTT (SEQ ID NO: 1688) |
| 221 | S 100A7 (SEQ ID NO: 96) | TATAGTCGGGGTGATA (SEQ ID NO: 1689) |
| 222 | S100A7 (SEQ ID NO: 96) | TTTATAGTTGGGGTGAT (SEQ ID NO: 1690) |
| 223 | S100A7 (SEQ ID NO: 96) | AGTCGGGCGTTAGTAA (SEQ ID NO: 1691) |
| 224 | S100A7 (SEQ ID NO: 96) | GAGTTGGGTGTTAGTA (SEQ ID NO: 1692) |

(continued)

| No: | Gene | Oligo: |
|---|---|---|
| 225 | S100A7 (SEQ ID NO: 96) | GGATGGCGGAAGTTTA (SEQ ID NO: 1693) |
| 226 | S100A7 (SEQ ID NO: 96) | GGATGGTGGAAGTTTA (SEQ ID NO: 1694) |
| 227 | S100A7 (SEQ ID NO: 96) | GGATGGCGGAAGTTTA (SEQ ID NO: 1693) |
| 228 | S100A7 (SEQ ID NO: 96) | GGATGGTGGAAGTTTA (SEQ ID NO: 1694) |
| 229 | APC (SEQ ID NO: 65) | GATTCGTATTTCGTAGT (SEQ ID NO: 1695) |
| 230 | APC (SEQ ID NO: 65) | GATTCGTATTTCGTAGT (SEQ ID NO: 1695) |
| 231 | APC (SEQ ID NO: 65) | AGCGTTTTGGTTCGTAT (SEQ ID NO: 1696) |
| 232 | APC (SEQ ID NO: 65) | AGTGTTTTGGTTTGTAT (SEQ ID NO: 1697) |
| 233 | APC (SEQ ID NO: 65) | AGCGTTTTGGTTCGTAT (SEQ ID NO: 1696) |
| 234 | APC (SEQ ID NO: 65) | AGTGTTTTGGTTTGTAT (SEQ ID NO: 1697) |
| 235 | APC (SEQ ID NO: 65) | TTAATCGGCGGGTTTT (SEQ ID NO: 1698) |
| 236 | APC (SEQ ID NO: 65) | AGTTAATTGGTGGGTT (SEQ ID NO: 1699) |
| 237 | CDH1 (SEQ ID NO: 79) | AGGTATCGTTTTTCGT (SEQ ID NO: 2191) |
| 238 | CDH1 (SEQ ID NO: 79) | GAGGTATTGTTTTTTGTA (SEQ ID NO: 2192) |
| 239 | SEQ ID NO:2 (SEQ ID NO: 2) | TAGGGGTTCGATTAGG (SEQ ID NO: 2193) |
| 240 | SEQ ID NO:2 (SEQ ID NO: 2) | AGGGGTTTGATTAGGG (SEQ ID NO: 2194) |
| 241 | SEQ ID NO:2 (SEQ ID NO: 2) | TAGGTATACGAAAGAGTA (SEQ ID NO: 1704) |
| 242 | SEQ ID NO:2 (SEQ ID NO: 2) | TTAGGTATATGAAAGAGTA (SEQ ID NO: 1705) |
| 243 | IGFBP7 (SEQ ID NO: 94) | TAGTCGCGGAATGTTA (SEQ ID NO: 1708) |
| 244 | IGFBP7 (SEQ ID NO: 94) | TTGGTAGTTGTGGAAT (SEQ ID NO: 1709) |
| 245 | IGFBP7 (SEQ ID NO: 94) | ATTTTTTCGCGGGTAT (SEQ ID NO: 1710) |
| 246 | IGFBP7 (SEQ ID NO: 94) | TTTTTGTGGGTATTTTAG (SEQ ID NO: 1711) |
| 247 | IGFBP7 (SEQ ID NO: 94) | GGTATATTCGACGGGG (SEQ ID NO: 1712) |
| 248 | IGFBP7 (SEQ ID NO: 94) | GGGTATATTTGATGGGG (SEQ ID NO: 1713) |
| 249 | IGFBP7 (SEQ ID NO: 94) | GGTACGAGCGTTTTTT (SEQ ID NO: 1714) |
| 250 | IGFBP7 (SEQ ID NO: 94) | TGGGTATGAGTGTTTT (SEQ ID NO: 1715) |
| 251 | IGFBP7 (SEQ ID NO: 94) | AAAGCGTATTTAATTCGT (SEQ ID NO: 1716) |
| 252 | IGFBP7 (SEQ ID NO: 94) | AGTGTATTTAATTTGTGTT (SEQ ID NO: 1717) |
| 253 | SOD2 (SEQ ID NO: 105) | GTCGTTTAGTCGGTTTA (SEQ ID NO: 1718) |
| 254 | SOD2 (SEQ ID NO: 105) | GTTGTTTAGTTGGTTTAT (SEQ ID NO: 1719) |
| 255 | SOD2 (SEQ ID NO: 105) | TATTAGGCGGTTGCGG (SEQ ID NO: 1720) |
| 256 | SOD2 (SEQ ID NO: 105) | TATTAGGTGGTTGTGG (SEQ ID NO: 1721) |
| 257 | SOD2 (SEQ ID NO: 105) | TATTAGGCGGTTGCGG (SEQ ID NO: 1720) |
| 258 | SOD2 (SEQ ID NO: 105) | TATTAGGTGGTTGTGG (SEQ ID NO: 1721) |
| 259 | NME1 (SEQ ID NO: 107) | AGTCGAGATTGCGTTA (SEQ ID NO: 2147) |
| 260 | NME (SEQ ID NO: 107) | AGTTGAGATTGTGTTAG (SEQ ID NO: 2148) |
| 261 | NME1 (SEQ ID NO: 107) | ATCGTTTGAATTCGGGA (SEQ ID NO: 2149) |
| 262 | NME (SEQ ID NO: 107) | ATTGTTTGAATTTGGGA (SEQ ID NO: 2150) |

(continued)

| No: | Gene | Oligo: |
|---|---|---|
| 263 | NME1 (SEQ ID NO: 107) | ATCGTTTGAATTCGGGA (SEQ ID NO: 2149) |
| 264 | NME1 (SEQ ID NO: 107) | ATTGTTTGAATTTGGGA (SEQ ID NO: 2150) |
| 265 | NME1 (SEQ ID NO: 107) | AATTCGAGATTAGTTCGG (SEQ ID NO: 1724) |
| 266 | NME1 (SEQ ID NO: 107) | AATTTGAGATTAGTTTGG (SEQ ID NO: 1725) |
| 267 | NME1 (SEQ ID NO: 107) | AATTCGAGATTAGTTCGG (SEQ ID NO: 1724) |
| 268 | NME1 (SEQ ID NO: 107) | AATTTGAGATTAGTTTGG (SEQ ID NO: 1725) |
| 269 | NME1 (SEQ ID NO: 107) | TTTTAGTACGTTGGAAA (SEQ ID NO: 2151) |
| 270 | NME1 (SEQ ID NO: 107) | TTAGTATGTTGGAAAGTA (SEQ ID NO: 2152) |
| 271 | THBS (SEQ ID NO: 81) | TAAAGGGGCGTTCGTA (SEQ ID NO: 1726) |
| 272 | THBS (SEQ ID NO: 81) | AAGGGGTGTTTGTATT (SEQ ID NO: 1727) |
| 273 | THBS1 (SEQ ID NO: 81) | GGTTAGTTCGGGCGTA (SEQ ID NO: 1728) |
| 274 | THBS1 (SEQ ID NO: 81) | GGTTAGTTTGGGTGTA (SEQ ID NO: 1729) |
| 275 | THBS1 (SEQ ID NO: 81) | GGTTAGTTCGGGCGTA (SEQ ID NO: 1728) |
| 276 | THBS1 (SEQ ID NO: 81) | GGTTAGTTTGGGTGTA (SEQ ID NO: 1729) |
| 277 | THBS1 (SEQ ID NO: 81) | TTGTGCGTTCGGAGTA (SEQ ID NO: 1730) |
| 278 | THBS1 (SEQ ID NO: 81) | TGTGTTTGGAGTAGAG (SEQ ID NO: 1731) |
| 279 | ESR1 (SEQ ID NO: 75) | AAATCGGCGGGGTTATT (SEQ ID NO: 1732) |
| 280 | ESR1 (SEQ ID NO: 75) | AGAAATTGGTGGGTTA (SEQ ID NO: 1733) |
| 281 | ESR1 (SEQ ID NO: 75) | AGATCGTGTTTTCGTA (SEQ ID NO: 2195) |
| 282 | ESR1 (SEQ ID NO: 75) | ATTGTGTTTTTGTAGGG (SEQ ID NO: 2196) |
| 283 | IL6 (SEQ ID NO: 99) | AGATGTCGTCGAGGAT (SEQ ID NO: 2197) |
| 284 | IL6 (SEQ ID NO: 99) | ATGTTGTTGAGGATGTA (SEQ ID NO: 2198) |
| 285 | IL6 (SEQ ID NO: 99) | AGTTTAGTCGGTTTCGT (SEQ ID NO: 1738) |
| 286 | IL6 (SEQ ID NO: 99) | AGTTTAGTTGGTTTTGT (SEQ ID NO: 1739) |
| 287 | IL6 (SEQ ID NO: 99) | AGTTTAGTCGGTTTCGT (SEQ ID NO: 1738) |
| 288 | IL6 (SEQ ID NO: 99) | AGTTTAGTTGGTTTTGT (SEQ ID NO: 1739) |
| 289 | CASP8 (SEQ ID NO: 71) | ATTTTTTAAACGGGTTTA (SEQ ID NO: 1742) |
| 290 | CASP8 (SEQ ID NO: 71) | TTTTAAATGGGTTTATAGG (SEQ ID NO: 1743) |
| 291 | HOXA5 (SEQ ID NO: 78) | TTCGAGTTCGGTTGAA (SEQ ID NO: 1746) |
| 292 | HOXA5 (SEQ ID NO: 78) | GTTTGAGTTTGGTTGAA (SEQ ID NO: 1747) |
| 293 | HOXA5 (SEQ ID NO: 78) | TAATTCGATTTCGGTTT (SEQ ID NO: 1750) |
| 294 | HOXA5 (SEQ ID NO: 78) | TTTAATTTGATTTTGGTTT (SEQ ID NO: 1751) |
| 295 | SNCG (SEQ ID NO: 73) | TGCGGTAGTATTCGAGT (SEQ ID NO: 1758) |
| 296 | SNCG (SEQ ID NO: 73) | TGTGGTAGTATTTGAGT (SEQ ID NO: 1759) |
| 297 | SNCG (SEQ ID NO: 73) | TGCGGTAGTATTCGAGT (SEQ ID NO: 1758) |
| 298 | SNCG (SEQ ID NO: 73) | TGTGGTAGTATTTGAGT (SEQ ID NO: 1759) |
| 299 | SNCG (SEQ ID NO: 73) | TATCGGGGATAGTCGTT (SEQ ID NO: 2199) |
| 300 | SNCG (SEQ ID NO: 73) | TATTGGGGATAGTTGTT (SEQ ID NO: 2200) |

(continued)

| No: | Gene | Oligo: |
|---|---|---|
| 301 | SNCG (SEQ ID NO: 73) | TATCGGGGATAGTCGTT (SEQ ID NO: 2199) |
| 302 | SNCG (SEQ ID NO: 73) | TATTGGGGATAGTTGTT (SEQ ID NO: 2200) |
| 303 | SNCG (SEQ ID NO: 73) | TATCGGCGTTAATAGG (SEQ ID NO: 2201) |
| 304 | SNCG (SEQ ID NO: 73) | TATTGGTGTTAATAGGAG (SEQ ID NO: 2202) |
| 305 | SNCG (SEQ ID NO: 73) | ATTGTACGTAGGGTTG (SEQ ID NO: 2203) |
| 306 | SNCG (SEQ ID NO: 73) | TTTTATTGTATGTAGGGT (SEQ ID NO: 2204) |
| 307 | GPC3 (SEQ ID NO: 118) | AATAGTCGCGTTTAGG (SEQ ID NO: 1760) |
| 308 | GPC3 (SEQ ID NO: 118) | TAGTTGTGTTTAGGGAT (SEQ ID NO: 1761) |
| 309 | GPC3 (SEQ ID NO: 118) | TTTAACGTAGTTTTGATCGG (SEQ ID NO: 1762) |
| 310 | GPC3 (SEQ ID NO: 118) | TTTAATGTAGTTTTGATTGG (SEQ ID NO: 1763) |
| 311 | GPC3 (SEQ ID NO: 118) | TTTAACGTAGTTTTGATCGG (SEQ ID NO: 1762) |
| 312 | GPC3 (SEQ ID NO: 118) | TTTAATGTAGTTTTGATTGG (SEQ ID NO: 1763) |
| 313 | CLDN7 (SEQ ID NO: 87) | TTACGTTAAGTCGGGT (SEQ ID NO: 1764) |
| 314 | CLDN7 (SEQ ID NO: 87) | AGTTATGTTAAGTTGGG (SEQ ID NO: 1765) |
| 315 | CLDN7 (SEQ ID NO: 87) | TAGCGTTTTAGGCGTA (SEQ ID NO: 1766) |
| 316 | CLDN7 (SEQ ID NO: 87) | TAGTGTTTTAGGTGTATT (SEQ ID NO: 1767) |
| 317 | CLDN7 (SEQ ID NO: 87) | TTAGGGGCGTTTCGTA (SEQ ID NO: 1768) |
| 318 | CLDN7 (SEQ ID NO: 87) | TTAGGGGTGTTTTGTAG (SEQ ID NO: 1769) |
| 319 | CLDN7 (SEQ ID NO: 87) | TAGAATTCGGCGGGGA (SEQ ID NO: 1770) |
| 320 | CLDN7 (SEQ ID NO: 87) | TAGAATTTGGTGGGGA (SEQ ID NO: 1771) |
| 321 | CLDN7 (SEQ ID NO: 87) | TAGAATTCGGCGGGGA (SEQ ID NO: 1770) |
| 322 | CLDN7 (SEQ ID NO: 87) | TAGAATTTGGTGGGGA (SEQ ID NO: 1771) |
| 323 | SLIT2 (SEQ ID NO: 112) | TTCGATAGTTAACGATG (SEQ ID NO: 1772) |
| 324 | SLIT2 (SEQ ID NO: 112) | TTTGATAGTTAATGATGGT (SEQ ID NO: 1773) |
| 325 | SLIT2 (SEQ ID NO: 112) | ATTTCGTCGTAGTTTG (SEQ ID NO: 1774) |
| 326 | SLIT2 (SEQ ID NO: 112) | TTTTGTTGTAGTTTGGA (SEQ ID NO: 1775) |
| 327 | SLIT2 (SEQ ID NO: 112) | TAGCGGGTTCGTAGTA (SEQ ID NO: 1776) |
| 328 | SLIT2 (SEQ ID NO: 112) | TTAGTGGGTTTGTAGTA (SEQ ID NO: 1777) |
| 329 | SLIT2 (SEQ ID NO: 112) | AAGGCGCGGAAGTTTA (SEQ ID NO: 1778) |
| 330 | SLIT2 (SEQ ID NO: 112) | AAGGTGTGGAAGTTTA (SEQ ID NO: 1779) |
| 331 | SLIT2 (SEQ ID NO: 112) | AAGGCGCGGAAGTTTA (SEQ ID NO: 1778) |
| 332 | SLIT2 (SEQ ID NO: 112) | AAGGTGTGGAAGTTTA (SEQ ID NO: 1779) |
| 333 | IGSF4 (SEQ ID NO: 74) | TAGTCGTAGAGTCGGG (SEQ ID NO: 1782) |
| 334 | IGSF4 (SEQ ID NO: 74) | GTTGTAGAGTTGGGTT (SEQ ID NO: 1783) |
| 335 | IGSF4 (SEQ ID NO: 74) | TAGGTTTTCGGATTGA (SEQ ID NO: 1784) |
| 336 | IGSF4 (SEQ ID NO: 74) | GTAGGTTTTTGGATTGA (SEQ ID NO: 1785) |
| 337 | MCT1 (SEQ ID NO: 101) | ATTTTACGTAGGCGTT (SEQ ID NO: 1786) |
| 338 | MCT1 (SEQ ID NO: 101) | GATTTTATGTAGGTGTTT (SEQ ID NO: 1787) |

(continued)

| No: | Gene | Oligo: |
|---|---|---|
| 339 | MCT1 (SEQ ID NO: 101) | AGTTAGTCGCGTTTTA (SEQ ID NO: 1788) |
| 340 | MCT1 (SEQ ID NO: 101) | AGAGTTAGTTGTGTTTTA (SEQ ID NO: 1789) |
| 341 | SEQ ID NO:6 (SEQ ID NO: 6) | AAGTTTATCGGCGTTT (SEQ ID NO: 1792) |
| 342 | SEQ ID NO:6 (SEQ ID NO: 6) | AGAAGTTTATTGGTGTTT (SEQ ID NO: 1793) |
| 343 | SEQ ID NO:6 (SEQ ID NO: 6) | ATTTCGGAATTTAAGCGT (SEQ ID NO: 1794) |
| 344 | SEQ ID NO:6 (SEQ ID NO: 6) | TTTTGGAATTTAAGTGTT (SEQ ID NO: 1795) |
| 345 | SEQ ID NO:6 (SEQ ID NO: 6) | TAATTTCGGACGCGGA (SEQ ID NO: 1796) |
| 346 | SEQ ID NO:6 (SEQ ID NO: 6) | TTTTGGATGTGGAGGA (SEQ ID NO: 1797) |
| 347 | SEQ ID NO:6 (SEQ ID NO: 6) | TTACGGTGAAGGCGGA (SEQ ID NO: 1798) |
| 348 | SEQ ID NO:6 (SEQ ID NO: 6) | TTATGGTGAAGGTGGA (SEQ ID NO: 1799) |
| 349 | SEQ ID NO:6 (SEQ ID NO: 6) | TTACGGTGAAGGCGGA (SEQ ID NO: 1798) |
| 350 | SEQ ID NO:6 (SEQ ID NO: 6) | TTATGGTGAAGGTGGA (SEQ ID NO: 1799) |
| 351 | SEQ ID NO:6 (SEQ ID NO: 6) | TTTCGGTTTTCGTTAAT (SEQ ID NO: 1800) |
| 352 | SEQ ID NO:6 (SEQ ID NO: 6) | TTTGGTTTTTGTTAATTTAG (SEQ ID NO: 1801) |
| 353 | SEQ ID NO:6 (SEQ ID NO: 6) | TGTGCGAAGTTAACGT (SEQ ID NO: 1802) |
| 354 | SEQ ID NO:6 (SEQ ID NO: 6) | TTGTGTGAAGTTAATGT (SEQ ID NO: 1803) |
| 355 | SEQ ID NO:8 (SEQ ID NO: 8) | ATAGGGCGGGATTTTA (SEQ ID NO: 2153) |
| 356 | SEQ ID NO:8 (SEQ ID NO: 8) | GATAGGGTGGGATTTT (SEQ ID NO: 2154) |
| 357 | SEQ ID NO:8 (SEQ ID NO: 8) | ATAAAGCGGGGTTTTA (SEQ ID NO: 1804) |
| 358 | SEQ ID NO:8 (SEQ ID NO: 8) | GGATAAAGTGGGGTTT (SEQ ID NO: 1805) |
| 359 | SEQ ID NO:8 (SEQ ID NO: 8) | AGGAGGCGAGAAATTT (SEQ ID NO: 1806) |
| 360 | SEQ ID NO:8 (SEQ ID NO: 8) | GAGGAGGTGAGAAATT (SEQ ID NO: 1807) |
| 361 | SEQ ID NO:8 (SEQ ID NO: 8) | AGAAATTTCGGGGTAG (SEQ ID NO: 1808) |
| 362 | SEQ ID NO:8 (SEQ ID NO: 8) | GAAATTTTGGGGTAGTA (SEQ ID NO: 1809) |
| 363 | SEQ ID NO:8 (SEQ ID NO: 8) | GGTAGTATCGTTATAGA (SEQ ID NO: 1810) |
| 364 | SEQ ID NO:8 (SEQ ID NO: 8) | GGGGTAGTATTGTTATA (SEQ ID NO: 1811) |
| 365 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGTGTATCGTTTTTCGG (SEQ ID NO: 1812) |
| 366 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAGTGTATTGTTTTTTGG (SEQ ID NO: 1813) |
| 367 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TGCGTATCGTTAGTTA (SEQ ID NO: 1814) |
| 368 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTTGTGTATTGTTAG (SEQ ID NO: 1815) |

(continued)

| No: | Gene | Oligo: |
|---|---|---|
| 369 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | ATTATTTCGGCGGTGA (SEQ ID NO: 1816) |
| 370 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GATTATTTTGGTGGTGA (SEQ ID NO: 1817) |
| 371 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAAGTCGCGTAAGGAG (SEQ ID NO: 1818) |
| 372 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AAGTTGTGTAAGGAGTA (SEQ ID NO: 1819) |
| 373 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATCGCGAGTTTGGA (SEQ ID NO: 1820) |
| 374 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATTGTGAGTTTGGAG (SEQ ID NO: 1821) |
| 375 | SEQ ID NO:51 (SEQ ID NO: 51) | GTCGGGGTCGATTCGA (SEQ ID NO: 1822) |
| 376 | SEQ ID NO:51 (SEQ ID NO: 51) | GTTGGGGTTGATTTGAT (SEQ ID NO: 1823) |
| 377 | SEQ ID NO:51 (SEQ ID NO: 51) | AGGATTCGTTTGCGTT (SEQ ID NO: 1824) |
| 378 | SEQ ID NO:51 (SEQ ID NO: 51) | AAGGATTTGTTTGTGTT (SEQ ID NO: 1825) |
| 379 | MGC34831 (SEQ ID NO: 52) | ATTAGCGTTTGGCGTT (SEQ ID NO: 1826) |
| 380 | MGC34831 (SEQ ID NO: 52) | AATTAGTGTTTGGTGTT (SEQ ID NO: 1827) |
| 381 | MGC34831 (SEQ ID NO: 52) | GTTTAGCGACGGTCGT (SEQ ID NO: 1828) |
| 382 | MGC34831 (SEQ ID NO: 52) | TGTTTAGTGATGGTTGT (SEQ ID NO: 1829) |
| 383 | SEQ ID NO:54 (SEQ ID NO: 54) | TGTGTAGCGGCGATTA (SEQ ID NO: 1830) |
| 384 | SEQ ID NO:54 (SEQ ID NO: 54) | GTGTGTAGTGGTGATT (SEQ ID NO: 1831) |
| 385 | SEQ ID NO:54 (SEQ ID NO: 54) | ATTAGCGTTTGGTCGG (SEQ ID NO: 1832) |
| 386 | SEQ ID NO:54 (SEQ ID NO: 54) | ATTAGTGTTTGGTTGGG (SEQ ID NO: 1833) |
| 387 | PDLIM1 (SEQ ID NO: 55) | TAGGTCGCGTAGTCGT (SEQ ID NO: 1834) |
| 388 | PDLIM1 (SEQ ID NO: 55) | TTAGGTTGTGTAGTTGT (SEQ ID NO: 1835) |
| 389 | SEQ ID N0:15 (SEQ ID NO: 15) | AATCGTGCGGTTGATA (SEQ ID NO: 1836) |
| 390 | SEQ ID NO:15 (SEQ ID NO: 15) | TGTAGAATTGTGTGGT (SEQ ID NO: 1837) |
| 391 | SEQ ID NO:15 (SEQ ID NO: 15) | TTGCGTAGAAAATTCGAG (SEQ ID NO: 1838) |
| 392 | SEQ ID NO:15 (SEQ ID NO: 15) | TTGTGTAGAAAATTTGAG (SEQ ID NO: 1839) |
| 393 | SEQ ID NO:15 (SEQ ID NO: 15) | TTGCGTAGAAAATTCGAG (SEQ ID NO: 1838) |
| 394 | SEQ ID NO: 15 (SEQ ID NO: 15) | TTGTGTAGAAAATTTGAG (SEQ ID NO: 1839) |
| 395 | SEQ ID NO: 15 (SEQ ID NO: 15) | AAAATTCGAGGTCGGG (SEQ ID NO: 1840) |
| 396 | SEQ ID NO: 15 (SEQ ID NO: 15) | AAAATTTGAGGTTGGG (SEQ ID NO: 1841) |
| 397 | SEQ ID NO: 15 (SEQ ID NO: 15) | AAAATTCGAGGTCGGG (SEQ ID NO: 1840) |

(continued)

| No: | Gene | Oligo: |
|---|---|---|
| 398 | SEQ ID NO:15 (SEQ ID NO: 15) | AAAATTTGAGGTTGGG (SEQ ID NO: 1841) |
| 399 | SEQ ID NO: 15 (SEQ ID NO: 15) | AATAGGCGATGTACGG (SEQ ID NO: 2205) |
| 400 | SEQ ID NO: 15 (SEQ ID NO: 15) | TAGGTGATGTATGGGT (SEQ ID NO: 2206) |
| 401 | SEQ ID NO: 15 (SEQ ID NO: 15) | TAATCGAGTTTAGCGG (SEQ ID NO: 2207) |
| 402 | SEQ ID NO: 15 (SEQ ID NO: 15) | TTAATTGAGTTTAGTGGT (SEQ ID NO: 2208) |
| 403 | DKK3 (SEQ ID NO: 24) | ATTCGTTTTAGTTCGAG (SEQ ID NO: 1842) |
| 404 | DKK3 (SEQ ID NO: 24) | ATTTGTTTTAGTTTGAGT (SEQ ID NO: 1843) |
| 405 | DKK3 (SEQ ID NO: 24) | TTCGATCGTTTTAGGA (SEQ ID NO: 1844) |
| 406 | DKK3 (SEQ ID NO: 24) | AGTTTTGATTGTTTTAGG (SEQ ID NO: 1845) |
| 407 | DKK3 (SEQ ID NO: 24) | GAAAAGACGCGATTTT (SEQ ID NO: 1848) |
| 408 | DKK3 (SEQ ID NO: 24) | GAAAAGATGTGATTTTATT (SEQ ID NO: 1849) |
| 409 | SEQ ID NO:28 (SEQ ID NO: 28) | TATCGACGTTTTTGGT (SEQ ID NO: 1850) |
| 410 | SEQ ID NO:28 (SEQ ID NO: 28) | TATTGATGTTTTTGGTTT (SEQ ID NO: 1851) |
| 411 | SEQ ID NO:29 (SEQ ID NO: 29) | TTGATGCGGAGTTCGA (SEQ ID NO: 1852) |
| 412 | SEQ ID NO:29 (SEQ ID NO: 29) | TTGATGTGGAGTTTGA (SEQ ID NO: 1853) |
| 413 | SEQ ID NO:29 (SEQ ID NO: 29) | TTGATGCGGAGTTCGA (SEQ ID NO: 1852) |
| 414 | SEQ ID NO:29 (SEQ ID NO: 29) | TTGATGTGGAGTTTGA (SEQ ID NO: 1853) |
| 415 | SEQ ID NO:29 (SEQ ID NO: 29) | TTCGAAGCGTGTATTA (SEQ ID NO: 2155) |
| 416 | SEQ ID NO:29 (SEQ ID NO: 29) | GGGTTTGAAGTGTGTA (SEQ ID NO: 2156) |
| 417 | SEQ ID NO:29 (SEQ ID NO: 29) | TAGGAACGGTAGGCGG (SEQ ID NO: 1854) |
| 418 | SEQ ID NO:29 (SEQ ID NO: 29) | TAGGAATGGTAGGTGG (SEQ ID NO: 1855) |
| 419 | SEQ ID NO:29 (SEQ ID NO: 29) | TAGGAACGGTAGGCGG (SEQ ID NO: 1854) |
| 420 | SEQ ID NO:29 (SEQ ID NO: 29) | TAGGAATGGTAGGTGG (SEQ ID NO: 1855) |
| 421 | SEQ ID NO:29 (SEQ ID NO: 29) | GTCGGAGGCGTTTGAG (SEQ ID NO: 1856) |
| 422 | SEQ ID NO:29 (SEQ ID NO: 29) | GTTGGAGGTGTTTGAGA (SEQ ID NO: 1857) |
| 423 | ARL7 (SEQ ID NO: 30) | TAGATTTCGTAGTTTTTTA (SEQ ID NO: 1858) |
| 424 | ARL7 (SEQ ID NO: 30) | TAGATTTTGTAGTTTTTTAA (SEQ ID NO: 1859) |
| 425 | ARL7 (SEQ ID NO: 30) | TGGGTACGTTTACGGT (SEQ ID NO: 1860) |
| 426 | ARL7 (SEQ ID NO: 30) | TGGGTATGTTTATGGTT (SEQ ID NO: 1861) |
| 427 | ARL7 (SEQ ID NO: 30) | GAAGAAATCGTTTTTGT (SEQ ID NO: 1862) |
| 428 | ARL7 (SEQ ID NO: 30) | GAAGAAATTGTTTTTGTT (SEQ ID NO: 1863) |
| 429 | ARL7 (SEQ ID NO: 30) | TAGTAGGATCGGTTTTT (SEQ ID NO: 1864) |
| 430 | ARL7 (SEQ ID NO: 30) | ATAGTAGGATTGGTTTTT (SEQ ID NO: 1865) |
| 431 | SEQ ID NO:31 (SEQ ID NO: 31) | AACGTTGCGTTGGGTA (SEQ ID NO: 1866) |
| 432 | SEQ ID NO:31 (SEQ ID NO: 31) | AATGTTGTGTTGGGTAA (SEQ ID NO: 1867) |
| 433 | SEQ ID NO:31 (SEQ ID NO: 31) | TAGCGTTTCGTGGTTA (SEQ ID NO: 1868) |
| 434 | SEQ ID NO:31 (SEQ ID NO: 31) | GTAGTGTTTTGTGGTTA (SEQ ID NO: 1869) |
| 435 | THH (SEQ ID NO: 32) | GTTCGTTGGCGTAAAT (SEQ ID NO: 1872) |

(continued)

| No: | Gene | Oligo: |
|-----|------|--------|
| 436 | THH (SEQ ID NO: 32) | GGTTTGTTGGTGTAAAT (SEQ ID NO: 1873) |
| 437 | (SEQ ID NO: 36) | TTATTTGCGTTTCGAA (SEQ ID NO: 2209) |
| 438 | (SEQ ID NO: 36) | AATTATTTGTGTTTTGAAT (SEQ ID NO: 2210) |
| 439 | (SEQ ID NO: 36) | GGACGTTGCGATTGTA (SEQ ID NO: 2161) |
| 440 | (SEQ ID NO: 36) | GATGTTGTGATTGTAGT (SEQ ID NO: 2162) |
| 441 | SENP3 (SEQ ID NO: 39) | TAGTCGAAAGTAGGACGT (SEQ ID NO: 1878) |
| 442 | SENP3 (SEQ ID NO: 39) | TAGTTGAAAGTAGGATGT (SEQ ID NO: 1879) |
| 443 | SENP3 (SEQ ID NO: 39) | TAGTCGAAAGTAGGACGT (SEQ ID NO: 1878) |
| 444 | SENP3 (SEQ ID NO: 39) | TAGTTGAAAGTAGGATGT (SEQ ID NO: 1879) |
| 445 | SENP3 (SEQ ID NO: 39) | AGGACGTTTTTGATCGG (SEQ ID NO: 1880) |
| 446 | SENP3 (SEQ ID NO: 39) | AGGATGTTTTTGATTGG (SEQ ID NO: 1881) |
| 447 | SENP3 (SEQ ID NO: 39) | AGGACGTTTTTGATCGG (SEQ ID NO: 1880) |
| 448 | SENP3 (SEQ ID NO: 39) | AGGATGTTTTTGATTGG (SEQ ID NO: 1881) |
| 449 | SENP3 (SEQ ID NO: 39) | AGTTATCGTTAGGAGGG (SEQ ID NO: 1882) |
| 450 | SENP3 (SEQ ID NO: 39) | AGTTATTGTTAGGAGGG (SEQ ID NO: 1883) |
| 451 | SENP3 (SEQ ID NO: 39) | AGTTATCGTTAGGAGGG (SEQ ID NO: 1882) |
| 452 | SENP3 (SEQ ID NO: 39) | AGTTATTGTTAGGAGGG (SEQ ID NO: 1883) |
| 453 | SEQ ID NO:42 (SEQ ID NO: 42) | GAGTCGGGTTGCGATG (SEQ ID NO: 1884) |
| 454 | SEQ ID NO:42 (SEQ ID NO: 42) | GGAGTTGGGTTGTGAT (SEQ ID NO: 1885) |
| 455 | (SEQ ID NO: 117) | TAGCGGTTTTTTAGCGTA (SEQ ID NO: 1888) |
| 456 | (SEQ ID NO: 117) | AGTGGTTTTTTAGTGTAT (SEQ ID NO: 1889) |
| 457 | (SEQ ID NO: 117) | AGATGCGCGGGTAGAT (SEQ ID NO: 1890) |
| 458 | (SEQ ID NO: 117) | AGATGTGTGGGTAGAT (SEQ ID NO: 1891) |
| 459 | (SEQ ID NO: 117) | AGATGCGCGGGTAGAT (SEQ ID NO: 1890) |
| 460 | (SEQ ID NO: 117) | AGATGTGTGGGTAGAT (SEQ ID NO: 1891) |
| 461 | (SEQ ID NO: 117) | AGATAGTTCGTATTCGT (SEQ ID NO: 1892) |
| 462 | (SEQ ID NO: 117) | GTAGATAGTTTGTATTTGT (SEQ ID NO: 1893) |
| 463 | (SEQ ID NO: 117) | TTTGTTCGTAACGTTT (SEQ ID NO: 1894) |
| 464 | (SEQ ID NO: 117) | TGTTTGTAATGTTTAGAG (SEQ ID NO: 1895) |
| 465 | 060279 (SEQ ID NO: 47) | AGTAAACGAATAAGAAGT (SEQ ID NO: 1896) |
| 466 | 060279 (SEQ ID NO: 47) | AAGTAAATGAATAAGAAGT (SEQ ID NO: 1897) |
| 467 | 060279 (SEQ ID NO: 47) | TACGTTTTTTCGGATTA (SEQ ID NO: 1898) |
| 468 | 060279 (SEQ ID NO: 47) | TATGTTTTTTTGGATTAAG (SEQ ID NO: 1899) |
| 469 | 060279 (SEQ ID NO: 47) | TAATTATCGGCGGTGT (SEQ ID NO: 1900) |
| 470 | 060279 (SEQ ID NO: 47) | ATTATTGGTGGTGTTTT (SEQ ID NO: 1901) |
| 471 | 060279 (SEQ ID NO: 47) | GGACGGCGGAAAATTA (SEQ ID NO: 1902) |
| 472 | 060279 (SEQ ID NO: 47) | AGGGATGGTGGAAAAT (SEQ ID NO: 1903) |
| 473 | SEQ ID NO:48 (SEQ ID NO: 48) | TATTTGGCGATTCGGA (SEQ ID NO: 1904) |

(continued)

| No: | Gene | Oligo: |
|---|---|---|
| 474 | SEQ ID NO:48 (SEQ ID NO: 48) | TGGTGATTTGGAGATT (SEQ ID NO: 1905) |
| 475 | SEQ ID NO:48 (SEQ ID NO: 48) | TAGGGTTACGTGTCGG (SEQ ID NO: 1906) |
| 476 | SEQ ID NO:48 (SEQ ID NO: 48) | TAGGGTTATGTGTTGG (SEQ ID NO: 1907) |
| 477 | SEQ ID NO:48 (SEQ ID NO: 48) | TAGGGTTACGTGTCGG (SEQ ID NO: 1906) |
| 478 | SEQ ID NO:48 (SEQ ID NO: 48) | TAGGGTTATGTGTTGG (SEQ ID NO: 1907) |
| 479 | SEQ ID NO:48 (SEQ ID NO: 48) | AACGAATTTTTCGATATT (SEQ ID NO: 1908) |
| 480 | SEQ ID NO:48 (SEQ ID NO: 48) | TTTAATGAATTTTTTGATATT (SEQ ID NO: 1909) |
| 481 | SEQ ID NO:48 (SEQ ID NO: 48) | AAAATCGTATGCGTGT (SEQ ID NO: 1910) |
| 482 | SEQ ID NO:48 (SEQ ID NO: 48) | GAAAATTGTATGTGTGTG (SEQ ID NO: 1911) |
| 483 | SEQ ID NO:9 (SEQ ID NO: 9) | GTTTCGCGTTTAGGGA (SEQ ID NO: 1912) |
| 484 | SEQ ID NO:9 (SEQ ID NO: 9) | GTTTTGTGTTTAGGGAT (SEQ ID NO: 1913) |
| 485 | SEQ ID NO:9 (SEQ ID NO: 9) | AGTGTTCGTCGTAGTT (SEQ ID NO: 1914) |
| 486 | SEQ ID NO:9 (SEQ ID NO: 9) | TGAGTGTTTGTTGTAGT (SEQ ID NO: 1915) |
| 487 | SEQ ID NO:4 (SEQ ID NO: 4) | TTTTGTTCGCGTTGAA (SEQ ID NO: 1916) |
| 488 | SEQ ID NO:4 (SEQ ID NO: 4) | TTGTTTGTGTTGAAGTA (SEQ ID NO: 1917) |
| 489 | SEQ ID NO:4 (SEQ ID NO: 4) | GGGTCGCGAGGTAGTT (SEQ ID NO: 1918) |
| 490 | SEQ ID NO:4 (SEQ ID NO: 4) | TGGGTTGTGAGGTAGT (SEQ ID NO: 1919) |
| 491 | SEQ ID NO:4 (SEQ ID NO: 4) | TTTGTGCGACGTTATT (SEQ ID NO: 1920) |
| 492 | SEQ ID NO:4 (SEQ ID NO: 4) | GGTTTGTGTGATGTTAT (SEQ ID NO: 1921) |
| 493 | SEQ ID NO:4 (SEQ ID NO: 4) | ATGGCGGTTTCGATTT (SEQ ID NO: 1922) |
| 494 | SEQ ID NO:4 (SEQ ID NO: 4) | GATGGTGGTTTTGATTT (SEQ ID NO: 1923) |
| 495 | SEQ ID NO:5 (SEQ ID NO: 5) | AATGAGCGAGAAAGTA (SEQ ID NO: 1924) |
| 496 | SEQ ID NO:5 (SEQ ID NO: 5) | AGAATGAGTGAGAAAGT (SEQ ID NO: 1925) |
| 497 | SEQ ID NO:5 (SEQ ID NO: 5) | ATTAAACGGGATGGTT (SEQ ID NO: 1926) |
| 498 | SEQ ID NO:5 (SEQ ID NO: 5) | AATATTAAATGGGATGGT (SEQ ID NO: 1927) |
| 499 | SEQ ID NO:5 (SEQ ID NO: 5) | GAGTTGCGAGGATTTT (SEQ ID NO: 1928) |
| 500 | SEQ ID NO:5 (SEQ ID NO: 5) | GGAGTTGTGAGGATTT (SEQ ID NO: 1929) |
| 501 | SEQ ID NO:5 (SEQ ID NO: 5) | GGGAATCGTTGATTTT (SEQ ID NO: 1930) |
| 502 | SEQ ID NO:5 (SEQ ID NO: 5) | AGGGGAATTGTTGATT (SEQ ID NO: 1931) |
| 503 | SEQ ID NO:7 (SEQ ID NO: 7) | TAGTCGTCGTGTAGGA (SEQ ID NO: 1932) |
| 504 | SEQ ID NO:7 (SEQ ID NO: 7) | TAGGTAGTTGTTGTGTA (SEQ ID NO: 1933) |
| 505 | SEQ ID NO:7 (SEQ ID NO: 7) | TATAGGTACGCGATGA (SEQ ID NO: 1934) |
| 506 | SEQ ID NO:7 (SEQ ID NO: 7) | AGGTATGTGATGAGGA (SEQ ID NO: 1935) |
| 507 | SEQ ID NO:7 (SEQ ID NO: 7) | ATGATTTGCGTTACGT (SEQ ID NO: 1938) |
| 508 | SEQ ID NO:7 (SEQ ID NO: 7) | ATGATTTGTGTTATGTTT (SEQ ID NO: 1939) |
| 509 | SEQ ID NO:7 (SEQ ID NO: 7) | TAACGTTGTGGTTCGAA (SEQ ID NO: 1940) |
| 510 | SEQ ID NO:7 (SEQ ID NO: 7) | TAATGTTGTGGTTTGAA (SEQ ID NO: 1941) |
| 511 | SEQ ID NO:7 (SEQ ID NO: 7) | TAACGTTGTGGTTCGAA (SEQ ID NO: 1940) |

(continued)

| No: | Gene | Oligo: |
|---|---|---|
| 512 | SEQ ID NO:7 (SEQ ID NO: 7) | TAATGTTGTGGTTTGAA (SEQ ID NO: 1941) |
| 513 | SEQ ID NO:1 (SEQ ID NO: 1) | GGTCGGCGTTGATTTTA (SEQ ID NO: 1942) |
| 514 | SEQ ID NO: (SEQ ID NO: 1) | GGTTGGTGTTGATTTTA (SEQ ID NO: 1943) |
| 515 | SEQ ID NO: 1 (SEQ ID NO: 1) | GGTCGGCGTTGATTTTA (SEQ ID NO: 1942) |
| 516 | SEQ ID NO: (SEQ ID NO: 1) | GGTTGGTGTTGATTTTA (SEQ ID NO: 1943) |
| 517 | SEQ ID NO: (SEQ ID NO: 1) | GATTCGAACGGATTTT (SEQ ID NO: 1944) |
| 518 | SEQ ID NO: (SEQ ID NO: 1) | GGGATTTGAATGGATTT (SEQ ID NO: 1945) |
| 519 | SEQ ID NO: (SEQ ID NO: 1) | TGGGTCGGGATTCGAA (SEQ ID NO: 1946) |
| 520 | SEQ ID NO: (SEQ ID NO: 1) | TGGGTTGGGATTTGAA (SEQ ID NO: 1947) |
| 521 | SEQ ID NO: (SEQ ID NO: 1) | TGGGTCGGGATTCGAA (SEQ ID NO: 1946) |
| 522 | SEQ ID NO: (SEQ ID NO: 1) | TGGGTTGGGATTTGAA (SEQ ID NO: 1947) |
| 523 | SEQ ID NO: (SEQ ID NO: 1) | GTCGGAAGTTTCGGGA (SEQ ID NO: 1948) |
| 524 | SEQ ID NO: (SEQ ID NO: 1) | GTTGGAAGTTTTGGGAT (SEQ ID NO: 1949) |
| 525 | SEQ ID NO: (SEQ ID NO: 1) | TGGATATCGTAGGGTA (SEQ ID NO: 1950) |
| 526 | SEQ ID NO: (SEQ ID NO: 1) | TGGATATTGTAGGGTAG (SEQ ID NO: 1951) |
| 527 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 528 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |
| 529 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 530 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |
| 531 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GGCGTCGAAAGTCGTT (SEQ ID NO: 1954) |
| 532 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GGTGTTGAAAGTTGTTG (SEQ ID NO: 1955) |
| 533 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAATCGTTTGTTTACGT (SEQ ID NO: 1956) |
| 534 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AATTGTTTGTTTATGTAGT (SEQ ID NO: 1957) |
| 535 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTACGGAGGCGTTTTA (SEQ ID NO: 1958) |

(continued)

| No: | Gene | Oligo: |
|-----|------|--------|
| 536 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTATGGAGGTGTTTT (SEQ ID NO: 1959) |
| 537 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | AGGCGAATTTATCGGG (SEQ ID NO: 1960) |
| 538 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | GGTGAATTTATTGGGG (SEQ ID NO: 1961) |
| 539 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTCGAAGTAGGCGT (SEQ ID NO: 1962) |
| 540 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTTGAAGTAGGTGTT (SEQ ID NO: 1963) |
| 541 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTCGACGAAGTTTT (SEQ ID NO: 1964) |
| 542 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTGATGAAGTTTTGTT (SEQ ID NO: 1965) |
| 543 | LIM DOMAIN KINASE 1 (EC 2.7.1.37) (LIMK-1) (SEQ ID NO: 12) | TGTAGTCGGGAGGTTA (SEQ ID NO: 1966) |
| 544 | LIM DOMAIN KINASE 1 (EC 2.7.1.37) (LIMK-1) (SEQ ID NO: 12) | TGTAGTTGGGAGGTTA (SEQ ID NO: 1967) |
| 545 | LIM DOMAIN KINASE 1 (EC 2.7.1.37) (LIMK-1) (SEQ ID NO: 12) | TGTAGTCGGGAGGTTA (SEQ ID NO: 1966) |
| 546 | LIM DOMAIN KINASE 1 (EC 2.7.1.37) (LIMK-1) (SEQ ID NO: 12) | TGTAGTTGGGAGGTTA (SEQ ID NO: 1967) |
| 547 | LIM DOMAIN KINASE 1 (EC 2.7.1.37) (LIMK-1) (SEQ ID NO: 12) | GGATTATCGCGGGGGT (SEQ ID NO: 1968) |
| 548 | LIM DOMAIN KINASE 1 (EC 2.7.1.37) (LIMK-1) (SEQ ID NO: 12) | GGATTATTGTGGGGGT (SEQ ID NO: 1969) |
| 549 | LIM DOMAIN KINASE 1 (EC 2.7.1.37) (LIMK-1) (SEQ ID NO: 12) | GGATTATCGCGGGGGT (SEQ ID NO: 1968) |

(continued)

| No: | Gene | Oligo: |
|-----|------|--------|
| 550 | LIM DOMAIN KINASE 1 (EC 2.7.1.37) (LIMK-1) (SEQ ID NO: 12) | GGATTATTGTGGGGGT (SEQ ID NO: 1969) |
| 551 | LIM DOMAIN KINASE 1 (EC 2.7.1.37) (LIMK-1) (SEQ ID NO: 12) | GTCGGTAGTTTATCGGAT (SEQ ID NO: 1970) |
| 552 | LIM DOMAIN KINASE 1 (EC 2.7.1.37) (LIMK-1) (SEQ ID NO: 12) | GTTGGTAGTTTATTGGAT (SEQ ID NO: 1971) |
| 553 | LIM DOMAIN KINASE 1 (EC 2.7.1.37) (LIMK-1) (SEQ ID NO: 12) | GTCGGTAGTTTATCGGAT (SEQ ID NO: 1970) |
| 554 | LIM DOMAIN KINASE 1 (EC 2.7.1.37) (LIMK-1) (SEQ ID NO: 12) | GTTGGTAGTTTATTGGAT (SEQ ID NO: 1971) |
| 555 | LIM DOMAIN KINASE 1 (EC 2.7.1.37) (LIMK-1) (SEQ ID NO: 12) | TAGGAGACGTTACGTT (SEQ ID NO: 1972) |
| 556 | LIM DOMAIN KINASE 1 (EC 2.7.1.37) (LIMK-1) (SEQ ID NO: 12) | AGATGTTATGTTAGGGT (SEQ ID NO: 1973) |
| 557 | BCL11B (SEQ ID NO: 50) | AAGTCGTCGGAGTTAG (SEQ ID NO: 1976) |
| 558 | BCL11B (SEQ ID NO: 50) | GTGAAGTTGTTGGAGT (SEQ ID NO: 1977) |
| 559 | BCL11B (SEQ ID NO: 50) | TTGAGGCGTTACGGTT (SEQ ID NO: 1978) |
| 560 | BCL11B (SEQ ID NO: 50) | TTGAGGTGTTATGGTT (SEQ ID NO: 1979) |
| 561 | BCL11B (SEQ ID NO: 50) | TTGAGGCGTTACGGTT (SEQ ID NO: 1978) |
| 562 | BCL11B (SEQ ID NO: 50) | TTGAGGTGTTATGGTT (SEQ ID NO: 1979) |
| 563 | MSF (SEQ ID NO: 13) | GTTTCGAAATTGGCGT (SEQ ID NO: 1980) |
| 564 | MSF (SEQ ID NO: 13) | TTTGAAATTGGTGTGG (SEQ ID NO: 1981) |
| 565 | MSF (SEQ ID NO: 13) | TTCGGTTTACGGGTTGTA (SEQ ID NO: 1982) |
| 566 | MSF (SEQ ID NO: 13) | TTTGGTTTATGGGTTGTA (SEQ ID NO: 1983) |
| 567 | MSF (SEQ ID NO: 13) | TTCGGTTTACGGGTTGTA (SEQ ID NO: 1982) |
| 568 | MSF (SEQ ID NO: 13) | TTTGGTTTATGGGTTGTA (SEQ ID NO: 1983) |
| 569 | MSF (SEQ ID NO: 13) | TTACGGTTCGATTTTG (SEQ ID NO: 1984) |
| 570 | MSF (SEQ ID NO: 13) | TATGGTTTGATTTTGGG (SEQ ID NO: 1985) |
| 571 | SEQ ID NO:14 (SEQ ID NO: 14) | TAAGGCGTTTTCGATA (SEQ ID NO: 1986) |
| 572 | SEQ ID NO:14 (SEQ ID NO: 14) | GTAAGGTGTTTTTGATAT (SEQ ID NO: 1987) |
| 573 | SEQ ID NO:16 (SEQ ID NO: 16) | ATCGTTGGTCGGATTT (SEQ ID NO: 1990) |
| 574 | SEQ ID NO:16 (SEQ ID NO: 16) | TAGGATTGTTGGTTGGA (SEQ ID NO: 1991) |
| 575 | SEQ ID NO:16 (SEQ ID NO: 16) | AGGAGTTTTCGTGTCGT (SEQ ID NO: 1992) |
| 576 | SEQ ID NO:16 (SEQ ID NO: 16) | AGGAGTTTTTGTGTTGT (SEQ ID NO: 1993) |
| 577 | SEQ ID NO: 16 (SEQ ID NO: 16) | AGGAGTTTTCGTGTCGT (SEQ ID NO: 1992) |
| 578 | SEQ ID NO:16 (SEQ ID NO: 16) | AGGAGTTTTTGTGTTGT (SEQ ID NO: 1993) |
| 579 | SEQ ID NO:16 (SEQ ID NO: 16) | TTACGGATAGGGCGAT (SEQ ID NO: 1994) |
| 580 | SEQ ID NO:16 (SEQ ID NO: 16) | TATGGATAGGGTGATTT (SEQ ID NO: 1995) |
| 581 | SEQ ID NO: 16 (SEQ ID NO: 16) | AGGCGTTGTCGGTGAT (SEQ ID NO: 1996) |
| 582 | SEQ ID NO: 16 (SEQ ID NO: 16) | AGGTGTTGTTGGTGATA (SEQ ID NO: 1997) |

(continued)

| No: | Gene | Oligo: |
|---|---|---|
| 583 | SEQ ID NO: 17 (SEQ ID NO: 17) | TACGTTTCGGGTTTGTTA (SEQ ID NO: 1998) |
| 584 | SEQ ID NO:17 (SEQ ID NO: 17) | TATGTTTTGGGTTTGTTA (SEQ ID NO: 1999) |
| 585 | SEQ ID NO:17 (SEQ ID NO: 17) | TACGTTTCGGGTTTGTTA (SEQ ID NO: 1998) |
| 586 | SEQ ID NO:17 (SEQ ID NO: 17) | TATGTTTTGGGTTTGTTA (SEQ ID NO: 1999) |
| 587 | SEQ ID NO:17 (SEQ ID NO: 17) | ATTTAGTCGTGCGTTT (SEQ ID NO: 2000) |
| 588 | SEQ ID NO:17 (SEQ ID NO: 17) | TGATTTAGTTGTGTGTT (SEQ ID NO: 2001) |
| 589 | SEQ ID NO:18 (SEQ ID NO: 18) | TTTACGCGGGGTTTTA (SEQ ID NO: 2002) |
| 590 | SEQ ID NO:18 (SEQ ID NO: 18) | TTTATGTGGGGTTTTAG (SEQ ID NO: 2003) |
| 591 | SEQ ID NO:18 (SEQ ID NO: 18) | TTACGTCGTTATTAGGT (SEQ ID NO: 2004) |
| 592 | SEQ ID NO:18 (SEQ ID NO: 18) | TTTTATGTTGTTATTAGGT (SEQ ID NO: 2005) |
| 593 | SEQ ID NO:18 (SEQ ID NO: 18) | TATTTGGACGTCGGGT (SEQ ID NO: 2006) |
| 594 | SEQ ID NO:18 (SEQ ID NO: 18) | TATTTGGATGTTGGGT (SEQ ID NO: 2007) |
| 595 | SEQ ID NO:18 (SEQ ID NO: 18) | TATTTGGACGTCGGGT (SEQ ID NO: 2006) |
| 596 | SEQ ID NO:18 (SEQ ID NO: 18) | TATTTGGATGTTGGGT (SEQ ID NO: 2007) |
| 597 | SEQ ID NO:18 (SEQ ID NO: 18) | GAGGCGTATTAGGTCGG (SEQ ID NO: 2008) |
| 598 | SEQ ID NO:18 (SEQ ID NO: 18) | AGGTGTATTAGGTTGGG (SEQ ID NO: 2009) |
| 599 | SEQ ID NO:18 (SEQ ID NO: 18) | AAAGCGGAGTCGTTAG (SEQ ID NO: 2010) |
| 600 | SEQ ID NO:18 (SEQ ID NO: 18) | AGTGGAGTTGTTAGGT (SEQ ID NO: 2011) |
| 601 | SEQ ID NO:19 (SEQ ID NO: 19) | AGGTTTTCGTTGTAGTA (SEQ ID NO: 2012) |
| 602 | SEQ ID NO:19 (SEQ ID NO: 19) | TAGGTTTTTGTTGTAGTA (SEQ ID NO: 2013) |
| 603 | SEQ ID NO:19 (SEQ ID NO: 19) | TGAGATTCGTTTTTTAAA (SEQ ID NO: 2014) |
| 604 | SEQ ID NO: 19 (SEQ ID NO: 19) | GGTGAGATTTGTTTTTTA (SEQ ID NO: 2015) |
| 605 | PRDM6 (SEQ ID NO: 20) | AGTTTTAAGCGTTTGGT (SEQ ID NO: 2016) |
| 606 | PRDM6 (SEQ ID NO: 20) | AGTTTTAAGTGTTTGGT (SEQ ID NO: 2017) |
| 607 | PRDM6 (SEQ ID NO: 20) | AGTTTTAAGCGTTTGGT (SEQ ID NO: 2016) |
| 608 | PRDM6 (SEQ ID NO: 20) | AGTTTTAAGTGTTTGGT (SEQ ID NO: 2017) |
| 609 | PRDM6 (SEQ ID NO: 20) | GAAGTTCGGATTTCGG (SEQ ID NO: 2018) |
| 610 | PRDM6 (SEQ ID NO: 20) | GGAAGTTTGGATTTTGG (SEQ ID NO: 2019) |
| 611 | PRDM6 (SEQ ID NO: 20) | TTGTCGGGTTACGGGA (SEQ ID NO: 2020) |
| 612 | PRDM6 (SEQ ID NO: 20) | GTTGGGTTATGGGAGA (SEQ ID NO: 2021) |
| 613 | PRDM6 (SEQ ID NO: 20) | TTCGTAGAATTGTCGAAG (SEQ ID NO: 2022) |
| 614 | PRDM6 (SEQ ID NO: 20) | TTTGTAGAATTGTTGAAG (SEQ ID NO: 2023) |
| 615 | PRDM6 (SEQ ID NO: 20) | TTCGTAGAATTGTCGAAG (SEQ ID NO: 2022) |
| 616 | PRDM6 (SEQ ID NO: 20) | TTTGTAGAATTGTTGAAG (SEQ ID NO: 2023) |
| 617 | RAP2B (SEQ ID NO: 21) | GGTCGGGTAATCGTTA (SEQ ID NO: 2024) |
| 618 | RAP2B (SEQ ID NO: 21) | GTTGGGTAATTGTTAGA (SEQ ID NO: 2025) |
| 619 | NR2E1 (SEQ ID NO: 22) | AATGTAGCGGCGTTAT (SEQ ID NO: 2028) |
| 620 | NR2E1 (SEQ ID NO: 22) | TAAATGTAGTGGTGTTAT (SEQ ID NO: 2029) |

(continued)

| No: | Gene | Oligo: |
|---|---|---|
| 621 | NR2E1 (SEQ ID NO: 22) | GTCGTTATCGGTTTGGA (SEQ ID NO: 2030) |
| 622 | NR2E1 (SEQ ID NO: 22) | GTTGTTATTGGTTTGGA (SEQ ID NO: 2031) |
| 623 | NR2E1 (SEQ ID NO: 22) | GTCGTTATCGGTTTGGA (SEQ ID NO: 2030) |
| 624 | NR2E1 (SEQ ID NO: 22) | GTTGTTATTGGTTTGGA (SEQ ID NO: 2031) |
| 625 | NR2E1 (SEQ ID NO: 22) | GACGTAAGTTTCGGGT (SEQ ID NO: 2032) |
| 626 | NR2E1 (SEQ ID NO: 22) | GGATGTAAGTTTTGGG (SEQ ID NO: 2033) |
| 627 | NR2E1 (SEQ ID NO: 22) | TTTTTAGTCGCGAGAA (SEQ ID NO: 2034) |
| 628 | NR2E1 (SEQ ID NO: 22) | TTTTTAGTTGTGAGAAGT (SEQ ID NO: 2035) |
| 629 | NR2E1 (SEQ ID NO: 22) | TTCGGGTGATATCGTTT (SEQ ID NO: 2036) |
| 630 | NR2E1 (SEQ ID NO: 22) | TTTGGGTGATATTGTTT (SEQ ID NO: 2037) |
| 631 | NR2E1 (SEQ ID NO: 22) | TTCGGGTGATATCGTTT (SEQ ID NO: 2036) |
| 632 | NR2E1 (SEQ ID NO: 22) | TTTGGGTGATATTGTTT (SEQ ID NO: 2037) |
| 633 | NR2E1 (SEQ ID NO: 22) | AGGCGAGTCGGAGTTT (SEQ ID NO: 2038) |
| 634 | NR2E1 (SEQ ID NO: 22) | AGGTGAGTTGGAGTTTT (SEQ ID NO: 2039) |
| 635 | NR2E1 (SEQ ID NO: 22) | TAAGTCGAGCGAGTTT (SEQ ID NO: 2040) |
| 636 | NR2E1 (SEQ ID NO: 22) | TAGTAAGTTGAGTGAGT (SEQ ID NO: 2041) |
| 637 | NR2E1 (SEQ ID NO: 22) | AGGGACGCGAAAATTT (SEQ ID NO: 2042) |
| 638 | NR2E1 (SEQ ID NO: 22) | GGAGGGATGTGAAAAT (SEQ ID NO: 2043) |
| 639 | PCDH7 (SEQ ID NO: 23) | ATCGTAGTCGGTTTTA (SEQ ID NO: 2044) |
| 640 | PCDH7 (SEQ ID NO: 23) | GATTATTGTAGTTGGTTT (SEQ ID NO: 2045) |
| 641 | RTTN (SEQ ID NO: 25) | TAGTGGCGCGGTAGTT (SEQ ID NO: 2046) |
| 642 | RTTN (SEQ ID NO: 25) | TAGTGGTGTGGTAGTTT (SEQ ID NO: 2047) |
| 643 | RTTN (SEQ ID NO: 25) | TAGGACGTGTTTTCGG (SEQ ID NO: 2048) |
| 644 | RTTN (SEQ ID NO: 25) | AGGATGTGTTTTTGGG (SEQ ID NO: 2049) |
| 645 | SNAP25 (SEQ ID NO: 33) | ATACGGAATATCGTATTT (SEQ ID NO: 2052) |
| 646 | SNAP25 (SEQ ID NO: 33) | GTGTATATATGGAATATTGT (SEQ ID NO: 2053) |
| 647 | SEQ ID NO:26 (SEQ ID NO: 26) | TTAAGTATCGAGGCGT (SEQ ID NO: 2054) |
| 648 | SEQ ID NO:26 (SEQ ID NO: 26) | TTTTAAGTATTGAGGTGT (SEQ ID NO: 2055) |
| 649 | SEQ ID NO:26 (SEQ ID NO: 26) | AATTTTGGTCGTTTAGT (SEQ ID NO: 2056) |
| 650 | SEQ ID NO:26 (SEQ ID NO: 26) | AAATTTTGGTTGTTTAGT (SEQ ID NO: 2057) |
| 651 | SEQ ID NO:26 (SEQ ID NO: 26) | TTCGTATTGACGTTAAT (SEQ ID NO: 2058) |
| 652 | SEQ ID NO:26 (SEQ ID NO: 26) | TTTGTATTGATGTTAATAGA (SEQ ID NO: 2059) |
| 653 | GIRK2 (SEQ ID NO: 27) | AGTTGTTCGTAGGCGA (SEQ ID NO: 2060) |
| 654 | GIRK2 (SEQ ID NO: 27) | AGTTGTTTGTAGGTGA (SEQ ID NO: 2061) |
| 655 | GIRK2 (SEQ ID NO: 27) | AGTTGTTCGTAGGCGA (SEQ ID NO: 2060) |
| 656 | GIRK2 (SEQ ID NO: 27) | AGTTGTTTGTAGGTGA (SEQ ID NO: 2061) |
| 657 | GIRK2 (SEQ ID NO: 27) | TTATTTCGTTCGTAGTT (SEQ ID NO: 2062) |
| 658 | GIRK2 (SEQ ID NO: 27) | TTTGTTTGTAGTTAGGTA (SEQ ID NO: 2063) |

(continued)

| No: | Gene | Oligo: |
|---|---|---|
| 659 | GIRK2 (SEQ ID NO: 27) | TTAGTCGAAAGGCGAG (SEQ ID NO: 2064) |
| 660 | GIRK2 (SEQ ID NO: 27) | TAGTTGAAAGGTGAGG (SEQ ID NO: 2065) |
| 661 | SEQ ID NO:28 (SEQ ID NO: 28) | ATTAGGCGAGTTTCGT (SEQ ID NO: 2066) |
| 662 | SEQ ID NO:28 (SEQ ID NO: 28) | TTAGGTGAGTTTTGTTT (SEQ ID NO: 2067) |
| 663 | SEQ ID NO:31 (SEQ ID NO: 31) | TTTACGTAGGGCGATT (SEQ ID NO: 2068) |
| 664 | SEQ ID NO:31 (SEQ ID NO: 31) | ATTTTTATGTAGGGTGAT (SEQ ID NO: 2069) |
| 665 | SEQ ID NO:31 (SEQ ID NO: 31) | GATACGGTTAGGCGGG (SEQ ID NO: 2070) |
| 666 | SEQ ID NO:31 (SEQ ID NO: 31) | GATATGGTTAGGTGGG (SEQ ID NO: 2071) |
| 667 | SEQ ID NO:31 (SEQ ID NO: 31) | GATACGGTTAGGCGGG (SEQ ID NO: 2070) |
| 668 | SEQ ID NO:31 (SEQ ID NO: 31) | GATATGGTTAGGTGGG (SEQ ID NO: 2071) |
| 669 | SEQ ID NO:31 (SEQ ID NO: 31) | TTCGGGCGTTTTATAT (SEQ ID NO: 2072) |
| 670 | SEQ ID NO:31 (SEQ ID NO: 31) | GGTTTGGGTGTTTTATA (SEQ ID NO: 2073) |
| 671 | HOXB13 (SEQ ID NO: 34) | GTCGTTATTATTTCGAGG (SEQ ID NO: 2074) |
| 672 | HOXB13 (SEQ ID NO: 34) | GTTGTTATTATTTTGAGGA (SEQ ID NO: 2075) |
| 673 | HOXB13 (SEQ ID NO: 34) | TTCGTAGAATCGAAGT (SEQ ID NO: 2211) |
| 674 | HOXB13 (SEQ ID NO: 34) | TAATTTGTAGAATTGAAGT (SEQ ID NO: 2212) |
| 675 | HOXB13 (SEQ ID NO: 34) | TTACGATTGAGCGTAT (SEQ ID NO: 2213) |
| 676 | HOXB13 (SEQ ID NO: 34) | TATGATTGAGTGTATAGG (SEQ ID NO: 2214) |
| 677 | HOXB13 (SEQ ID NO: 34) | AAGTTAGCGGGTTCGT (SEQ ID NO: 2076) |
| 678 | HOXB13 (SEQ ID NO: 34) | AAGTTAGTGGGTTTGT (SEQ ID NO: 2077) |
| 679 | HOXB13 (SEQ ID NO: 34) | AAGTTAGCGGGTTCGT (SEQ ID NO: 2076) |
| 680 | HOXB13 (SEQ ID NO: 34) | AAGTTAGTGGGTTTGT (SEQ ID NO: 2077) |
| 681 | HOXB13 (SEQ ID NO: 34) | TTGGTCGCGTAGTAAA (SEQ ID NO: 2078) |
| 682 | HOXB13 (SEQ ID NO: 34) | TGGTTGTGTAGTAAAGT (SEQ ID NO: 2079) |
| 683 | (SEQ ID NO: 35) | GGAGGTGCGAATTTAA (SEQ ID NO: 2080) |
| 684 | (SEQ ID NO: 35) | GGGAGGTGTGAATTTA (SEQ ID NO: 2081) |
| 685 | (SEQ ID NO: 35) | AAATAGTCGTTTGGGA (SEQ ID NO: 2082) |
| 686 | (SEQ ID NO: 35) | AAATAGTTGTTTGGGAG (SEQ ID NO: 2083) |
| 687 | (SEQ ID NO: 35) | AGAAAATATACGAGATTTAT (SEQ ID NO: 2084) |
| 688 | (SEQ ID NO: 35) | AGAAAATATATGAGATTTATT (SEQ ID NO: 2085) |
| 689 | (SEQ ID NO: 35) | TAAAGACGGGAAGAGA (SEQ ID NO: 2086) |
| 690 | (SEQ ID NO: 35) | ATAAAGATGGGAAGAGA (SEQ ID NO: 2087) |
| 691 | MGC10561 (SEQ ID NO: 37) | ATATTTAGCGTAGTTATTT (SEQ ID NO: 2171) |
| 692 | MGC10561 (SEQ ID NO: 37) | TAGTATATTTAGTGTAGTTAT (SEQ ID NO: 2172) |
| 693 | MGC10561 (SEQ ID NO: 37) | TTTTTTACGTTAAGGGG (SEQ ID NO: 2088) |
| 694 | MGC10561 (SEQ ID NO: 37) | TTTTTATGTTAAGGGGG (SEQ ID NO: 2089) |
| 695 | MGC10561 (SEQ ID NO: 37) | TTTGTTTTTCGAGTAGA (SEQ ID NO: 2090) |
| 696 | MGC10561 (SEQ ID NO: 37) | TGTTTTTTTGAGTAGAGG (SEQ ID NO: 2091) |

(continued)

| No: | Gene | Oligo: |
|---|---|---|
| 697 | LMX1A (SEQ ID NO: 38) | GTCGGGTTTTTCGGAA (SEQ ID NO: 2092) |
| 698 | LMX1A (SEQ ID NO: 38) | GTTGGGTTTTTTGGAAG (SEQ ID NO: 2093) |
| 699 | LMX1A (SEQ ID NO: 38) | GTCGAGATTTTATCGAAA (SEQ ID NO: 2094) |
| 700 | LMX1A (SEQ ID NO: 38) | GTTGAGATTTTATTGAAAG (SEQ ID NO: 2095) |
| 701 | LMX1A (SEQ ID NO: 38) | TTCGTTTTTAACGTGG (SEQ ID NO: 2215) |
| 702 | LMX1A (SEQ ID NO: 38) | TTTGTTTTTAATGTGGTT (SEQ ID NO: 2216) |
| 703 | LMX1A (SEQ ID NO: 38) | AGTATTCGGGCGGGTA (SEQ ID NO: 2096) |
| 704 | LMX1A (SEQ ID NO: 38) | GTAGTATTTGGGTGGG (SEQ ID NO: 2097) |
| 705 | LMX1A (SEQ ID NO: 38) | AACGAAATTACGTGTAT (SEQ ID NO: 2098) |
| 706 | LMX1A (SEQ ID NO: 38) | TGAAATGAAATTATGTGTA (SEQ ID NO: 2099) |
| 707 | GS1 (SEQ ID NO: 40) | TGCGAGTTAGCGGTTA (SEQ ID NO: 2100) |
| 708 | GS1 (SEQ ID NO: 40) | TTGTGAGTTAGTGGTT (SEQ ID NO: 2101) |
| 709 | GS1 (SEQ ID NO: 40) | AGTTTCGAGGTTTTCGG (SEQ ID NO: 2102) |
| 710 | GS1 (SEQ ID NO: 40) | AAGTTTTGAGGTTTTTGG (SEQ ID NO: 2103) |
| 711 | TITF1 (SEQ ID NO: 41) | GTCGTGGGGATCGTAT (SEQ ID NO: 2104) |
| 712 | TITF1 (SEQ ID NO: 41) | GTTGTGGGGATTGTAT (SEQ ID NO: 2105) |
| 713 | TITF1 (SEQ ID NO: 41) | GTCGTGGGGATCGTAT (SEQ ID NO: 2104) |
| 714 | TITF1 (SEQ ID NO: 41) | GTTGTGGGGATTGTAT (SEQ ID NO: 2105) |
| 715 | TITF1 (SEQ ID NO: 41) | GGTTCGTTTAAGTTCGG (SEQ ID NO: 2106) |
| 716 | TITF1 (SEQ ID NO: 41) | GGTTTGTTTAAGTTTGG (SEQ ID NO: 2107) |
| 717 | TITF1 (SEQ ID NO: 41) | GGTTCGTTTAAGTTCGG (SEQ ID NO: 2106) |
| 718 | TITF1 (SEQ ID NO: 41) | GGTTTGTTTAAGTTTGG (SEQ ID NO: 2107) |
| 719 | TITF1 (SEQ ID NO: 41) | TTAGGTCGCGTTTGTA (SEQ ID NO: 2108) |
| 720 | TITF1 (SEQ ID NO: 41) | AGGTTGTGTTTGTAGA (SEQ ID NO: 2109) |
| 721 | TITF1 (SEQ ID NO: 41) | TATTTCGTTTTCGTAATT (SEQ ID NO: 2110) |
| 722 | TITF1 (SEQ ID NO: 41) | TTTGTTTTTGTAATTAGATT (SEQ ID NO: 2111) |
| 723 | DDX51 (SEQ ID NO: 43) | AGATTTTCGGCGAGAT (SEQ ID NO: 2112) |
| 724 | DDX51 (SEQ ID NO: 43) | ATTTTTGGTGAGATAGG (SEQ ID NO: 2113) |
| 725 | DDX51 (SEQ ID NO: 43) | TACGTGTGGTTTTCGGTA (SEQ ID NO: 2114) |
| 726 | DDX51 (SEQ ID NO: 43) | TATGTGTGGTTTTTGGTA (SEQ ID NO: 2115) |
| 727 | DDX51 (SEQ ID NO: 43) | TACGTGTGGTTTTCGGTA (SEQ ID NO: 2114) |
| 728 | DDX51 (SEQ ID NO: 43) | TATGTGTGGTTTTTGGTA (SEQ ID NO: 2115) |
| 729 | DDX51 (SEQ ID NO: 43) | AACGTGCGGGGTTTTT (SEQ ID NO: 2116) |
| 730 | DDX51 (SEQ ID NO: 43) | TGAATGTGTGGGGTTT (SEQ ID NO: 2117) |
| 731 | SEQ ID NO:46 (SEQ ID NO: 46) | GAGTCGGGTTATCGTT (SEQ ID NO: 2120) |
| 732 | SEQ ID NO:46 (SEQ ID NO: 46) | GGAGTTGGGTTATTGT (SEQ ID NO: 2121) |
| 733 | SEQ ID NO:46 (SEQ ID NO: 46) | TTACGGATGTTTCGGT (SEQ ID NO: 2122) |
| 734 | SEQ ID NO:46 (SEQ ID NO: 46) | TTTATGGATGTTTTGGT (SEQ ID NO: 2123) |

(continued)

| No: | Gene | Oligo: |
|---|---|---|
| 735 | SEQ ID NO:46 (SEQ ID NO: 46) | TGACGTATTTTCGGTT (SEQ ID NO: 2124) |
| 736 | SEQ ID NO:46 (SEQ ID NO: 46) | GGTGATGTATTTTTGGT (SEQ ID NO: 2125) |
| 737 | SEQ ID NO:46 (SEQ ID NO: 46) | ATAGTCGGAATCGTTG (SEQ ID NO: 2126) |
| 738 | SEQ ID NO:46 (SEQ ID NO: 46) | TAGTTGGAATTGTTGTT (SEQ ID NO: 2127) |
| 739 | SEQ ID NO:2 (SEQ ID NO: 2) | ATTGGGTTTCGCGTAGG (SEQ ID NO: 2128) |
| 740 | SEQ ID NO:2 (SEQ ID NO: 2) | ATTGGGTTTTGTGTAGG (SEQ ID NO: 2129) |
| 741 | SEQ ID NO:2 (SEQ ID NO: 2) | ATTGGGTTTCGCGTAGG (SEQ ID NO: 2128) |
| 742 | SEQ ID NO:2 (SEQ ID NO: 2) | ATTGGGTTTTGTGTAGG (SEQ ID NO: 2129) |
| 743 | SEQ ID NO:2 (SEQ ID NO: 2) | TAGTAGTCGGCGGGAG (SEQ ID NO: 2130) |
| 744 | SEQ ID NO:2 (SEQ ID NO: 2) | TAGTAGTTGGTGGGAG (SEQ ID NO: 2131) |
| 745 | SEQ ID NO:2 (SEQ ID NO: 2) | TAGTAGTCGGCGGGAG (SEQ ID NO: 2130) |
| 746 | SEQ ID NO:2 (SEQ ID NO: 2) | TAGTAGTTGGTGGGAG (SEQ ID NO: 2131) |
| 747 | SEQ ID NO:2 (SEQ ID NO: 2) | TGTCGTACGTTATGTT (SEQ ID NO: 2217) |
| 748 | SEQ ID NO:2 (SEQ ID NO: 2) | GGTGTTGTATGTTATGT (SEQ ID NO: 2218) |
| 749 | SEQ ID NO:2 (SEQ ID NO: 2) | TTGATTGGCGGACGAG (SEQ ID NO: 1706) |
| 750 | SEQ ID NO:2 (SEQ ID NO: 2) | TTGATTGGTGGATGAG (SEQ ID NO: 1707) |
| 751 | SEQ ID NO:2 (SEQ ID NO: 2) | TTGATTGGCGGACGAG (SEQ ID NO: 1706) |
| 752 | SEQ ID NO:2 (SEQ ID NO: 2) | TTGATTGGTGGATGAG (SEQ ID NO: 1707) |
| 753 | SEQ ID NO:3 (SEQ ID NO: 3) | TTGCGTTAATTCGGTA (SEQ ID NO: 2132) |
| 754 | SEQ ID NO:3 (SEQ ID NO: 3) | AATTTGTGTTAATTTGGT (SEQ ID NO: 2133) |
| 755 | SEQ ID NO:3 (SEQ ID NO: 3) | AGTCGGGAGAGCGAAA (SEQ ID NO: 2134) |
| 756 | SEQ ID NO:3 (SEQ ID NO: 3) | AGTTGGGAGAGTGAAA (SEQ ID NO: 2135) |
| 757 | SEQ ID NO:3 (SEQ ID NO: 3) | AGTCGGGAGAGCGAAA (SEQ ID NO: 2134) |
| 758 | SEQ ID NO:3 (SEQ ID NO: 3) | AGTTGGGAGAGTGAAA (SEQ ID NO: 2135) |
| 759 | SEQ ID NO:3 (SEQ ID NO: 3) | GGTCGAAGAGTCGGGA (SEQ ID NO: 2136) |
| 760 | SEQ ID NO:3 (SEQ ID NO: 3) | GGTTGAAGAGTTGGGA (SEQ ID NO: 2137) |
| 761 | SEQ ID NO:3 (SEQ ID NO: 3) | GGTCGAAGAGTCGGGA (SEQ ID NO: 2136) |
| 762 | SEQ ID NO:3 (SEQ ID NO: 3) | GGTTGAAGAGTTGGGA (SEQ ID NO: 2137) |
| 763 | SEQ ID NO:3 (SEQ ID NO: 3) | ATGTTAGCGGGTCGAA (SEQ ID NO: 2138) |
| 764 | SEQ ID NO:3 (SEQ ID NO: 3) | ATGTTAGTGGGTTGAA (SEQ ID NO: 2139) |
| 765 | SEQ ID NO:3 (SEQ ID NO: 3) | ATGTTAGCGGGTCGAA (SEQ ID NO: 2138) |
| 766 | SEQ ID NO:3 (SEQ ID NO: 3) | ATGTTAGTGGGTTGAA (SEQ ID NO: 2139) |

Table 19: DCIS vs. benign breast conditions

| No: | Gene | Oligo: |
|---|---|---|
| 1 | SCGB3A1 (SEQ ID NO: 115) | TAGTGTTCGACGTTGT (SEQ ID NO: 1475) |
| 2 | SCGB3A1 (SEQ ID NO: 115) | TAGTGTTTGATGTTGTT (SEQ ID NO: 1476) |
| 3 | SASH1 (SEQ ID NO: 102) | TAGATTCGAGGTGCGG (SEQ ID NO: 1477) |

(continued)

| No: | Gene | Oligo: |
|---|---|---|
| 4 | SASH1 (SEQ ID NO: 102) | TAGATTTGAGGTGTGG (SEQ ID NO: 1478) |
| 5 | SASH1 (SEQ ID NO: 102) | TAGATTCGAGGTGCGG (SEQ ID NO: 1477) |
| 6 | SASH1 (SEQ ID NO: 102) | TAGATTTGAGGTGTGG (SEQ ID NO: 1478) |
| 7 | SASH1 (SEQ ID NO: 102) | ATTCGGTATTCGGGTAG (SEQ ID NO: 1479) |
| 8 | SASH1 (SEQ ID NO: 102) | ATTTGGTATTTGGGTAG (SEQ ID NO: 1480) |
| 9 | SASH1 (SEQ ID NO: 102) | ATTCGGTATTCGGGTAG (SEQ ID NO: 1479) |
| 10 | SASH1 (SEQ ID NO: 102) | ATTTGGTATTTGGGTAG (SEQ ID NO: 1480) |
| 11 | SASH1 (SEQ ID NO: 102) | AGTCGGAATCGGAGTT (SEQ ID NO: 1481) |
| 12 | SASH1 (SEQ ID NO: 102) | GTTGGAATTGGAGTTTA (SEQ ID NO: 1482) |
| 13 | ARH1/NOEY2 (SEQ ID NO: 97) | TAAAACGTTCGGTAGG (SEQ ID NO: 1485) |
| 14 | ARH1/NOEY2 (SEQ ID NO: 97) | TTTAAAATGTTTGGTAGG (SEQ ID NO: 1486) |
| 15 | ARH1/NOEY2 (SEQ ID NO: 97) | TGTATTCGTCGTTAGG (SEQ ID NO: 1487) |
| 16 | ARH1/NOEY2 (SEQ ID NO: 97) | AATGTATTTGTTGTTAGG (SEQ ID NO: 1488) |
| 17 | ARH1/NOEY2 (SEQ ID NO: 97) | TTAGACGGAGTTCGGA (SEQ ID NO: 1489) |
| 18 | ARH1/NOEY2 (SEQ ID NO: 97) | TAGATGGAGTTTGGAGA (SEQ ID NO: 1490) |
| 19 | ARH1/NOEY2 (SEQ ID NO: 97) | TAGACGTAGGCGTATT (SEQ ID NO: 1491) |
| 20 | ARH1/NOEY2 (SEQ ID NO: 97) | GGGTAGATGTAGGTGT (SEQ ID NO: 1492) |
| 21 | CCND2 (SEQ ID NO: 104) | TTAGGGTCGATCGTGT (SEQ ID NO: 1493) |
| 22 | CCND2 (SEQ ID NO: 104) | TAGGGTTGATTGTGTT (SEQ ID NO: 1494) |
| 23 | CCND2 (SEQ ID NO: 104) | TTATCGTAGTCGGTTT (SEQ ID NO: 1495) |
| 24 | CCND2 (SEQ ID NO: 104) | GATTTTATTGTAGTTGGT (SEQ ID NO: 1496) |
| 25 | CCND2 (SEQ ID NO: 104) | GAGTGAGGCGCGAAAT (SEQ ID NO: 1497) |
| 26 | CCND2 (SEQ ID NO: 104) | GAGTGAGGTGTGAAAT (SEQ ID NO: 1498) |
| 27 | CCND2 (SEQ ID NO: 104) | GAGTGAGGCGCGAAAT (SEQ ID NO: 1497) |
| 28 | CCND2 (SEQ ID NO: 104) | GAGTGAGGTGTGAAAT (SEQ ID NO: 1498) |
| 29 | CDKN1A (SEQ ID NO: 67) | ATTAGGGTCGCGTTGA (SEQ ID NO: 1501) |
| 30 | CDKN1A (SEQ ID NO: 67) | ATTAGGGTTGTGTTGA (SEQ ID NO: 1502) |
| 31 | CDKN1A (SEQ ID NO: 67) | ATTAGGGTCGCGTTGA (SEQ ID NO: 1501) |
| 32 | CDKN1A (SEQ ID NO: 67) | ATTAGGGTTGTGTTGA (SEQ ID NO: 1502) |
| 33 | CDKN2A (SEQ ID NO: 57) | GGCGTTGTTTAACGTAT (SEQ ID NO: 1503) |
| 34 | CDKN2A (SEQ ID NO: 57) | GGGTGTTGTTTAATGTA (SEQ ID NO: 1504) |
| 35 | CDKN2A (SEQ ID NO: 57) | AATAGTTACGGTCGGA (SEQ ID NO: 1505) |
| 36 | CDKN2A (SEQ ID NO: 57) | AGTTATGGTTGGAGGT (SEQ ID NO: 1506) |
| 37 | CDKN2A (SEQ ID NO: 57) | GTCGGAGGTCGATTTA (SEQ ID NO: 1507) |
| 38 | CDKN2A (SEQ ID NO: 57) | GGTTGGAGGTTGATTTA (SEQ ID NO: 1508) |
| 39 | EYA4 (SEQ ID NO: 58) | GGTATAAAATCGTAAATTTT (SEQ ID NO: 1513) |
| 40 | EYA4 (SEQ ID NO: 58) | GGGTATAAAATTGTAAATTT (SEQ ID NO: 1514) |
| 41 | EYA4 (SEQ ID NO: 58) | GTTTAGATACGAAATGTT (SEQ ID NO: 1517) |

(continued)

| No: | Gene | Oligo: |
|---|---|---|
| 42 | EYA4 (SEQ ID NO: 58) | GTTTAGATATGAAATGTTAT (SEQ ID NO: 1518) |
| 43 | FHIT (SEQ ID NO: 76) | TTTAAGTATCGATTTTAGT (SEQ ID NO: 2183) |
| 44 | FHIT (SEQ ID NO: 76) | TAAGTATTGATTTTAGTTTTA (SEQ ID NO: 2184) |
| 45 | FHIT (SEQ ID NO: 76) | GTTACGTTAGCGGGTT (SEQ ID NO: 1521) |
| 46 | FHIT (SEQ ID NO: 76) | GGTTATGTTAGTGGGT (SEQ ID NO: 1522) |
| 47 | FHIT (SEQ ID NO: 76) | TTCGGGGACGTTATAG (SEQ ID NO: 1523) |
| 48 | FHIT (SEQ ID NO: 76) | GGTTTGGGGATGTTAT (SEQ ID NO: 1524) |
| 49 | GSTP1 (SEQ ID NO: 59) | GGCGATTTCGGGGATT (SEQ ID NO: 1525) |
| 50 | GSTP1 (SEQ ID NO: 59) | GGTGATTTTGGGGATTT (SEQ ID NO: 1526) |
| 51 | GSTP1 (SEQ ID NO: 59) | GACGTTCGGGGTGTAG (SEQ ID NO: 1527) |
| 52 | GSTP1 (SEQ ID NO: 59) | GATGTTTGGGGTGTAG (SEQ ID NO: 1528) |
| 53 | GSTP1 (SEQ ID NO: 59) | GACGTTCGGGGTGTAG (SEQ ID NO: 1527) |
| 54 | GSTP1 (SEQ ID NO: 59) | GATGTTTGGGGTGTAG (SEQ ID NO: 1528) |
| 55 | GSTP1 (SEQ ID NO: 59) | AGTTCGCGGGATTTTT (SEQ ID NO: 1529) |
| 56 | GSTP1 (SEQ ID NO: 59) | GGAGTTTGTGGGATTT (SEQ ID NO: 1530) |
| 57 | GSTP1 (SEQ ID NO: 59) | AGTTTTCGTTATTAGTGA (SEQ ID NO: 1531) |
| 58 | GSTP1 (SEQ ID NO: 59) | TAGTTTTTGTTATTAGTGA (SEQ ID NO: 1532) |
| 59 | HIC1 (SEQ ID NO: 85) | TATCGAAGTTTTCGGG (SEQ ID NO: 1533) |
| 60 | HIC1 (SEQ ID NO: 85) | TATTGAAGTTTTTGGGT (SEQ ID NO: 1534) |
| 61 | MLH1 (SEQ ID NO: 89) | AGGCGGCGATAGATTA (SEQ ID NO: 1541) |
| 62 | MLH1 (SEQ ID NO: 89) | ATGAGGTGGTGATAGA (SEQ ID NO: 1542) |
| 63 | PGR (SEQ ID NO: 83) | TTTCGAGGTCGGATTT (SEQ ID NO: 1543) |
| 64 | PGR (SEQ ID NO: 83) | TTTGAGGTTGGATTTTT (SEQ ID NO: 1544) |
| 65 | PGR (SEQ ID NO: 83) | TTCGACGAAAAGACGT (SEQ ID NO: 2219) |
| 66 | PGR (SEQ ID NO: 83) | TTTTTGATGAAAAGATGT (SEQ ID NO: 2220) |
| 67 | PGR (SEQ ID NO: 83) | TTTTTTCGACGAAAAGA (SEQ ID NO: 1547) |
| 68 | PGR (SEQ ID NO: 83) | AGGATTTTTTTGATGAAA (SEQ ID NO: 1548) |
| 69 | SERPINB5 (SEQ ID NO: 68) | TTATTAACGTGTTTGAGA (SEQ ID NO: 1549) |
| 70 | SERPINB5 (SEQ ID NO: 68) | TTATTAATGTGTTTGAGAA (SEQ ID NO: 1550) |
| 71 | RARB (SEQ ID NO: 88) | ATGTCGAGAACGCGAG (SEQ ID NO: 1555) |
| 72 | RARB (SEQ ID NO: 88) | GGATGTTGAGAATGTGA (SEQ ID NO: 1556) |
| 73 | RARB (SEQ ID NO: 88) | AGCGATTCGAGTAGGG (SEQ ID NO: 1557) |
| 74 | RARB (SEQ ID NO: 88) | AGTGATTTGAGTAGGG (SEQ ID NO: 1558) |
| 75 | RARB (SEQ ID NO: 88) | AGCGATTCGAGTAGGG (SEQ ID NO: 1557) |
| 76 | RARB (SEQ ID NO: 88) | AGTGATTTGAGTAGGG (SEQ ID NO: 1558) |
| 77 | RARB (SEQ ID NO: 88) | TAGGATTCGGAACGTA (SEQ ID NO: 1559) |
| 78 | RARB (SEQ ID NO: 88) | GGTAGGATTTGGAATGT (SEQ ID NO: 1560) |
| 79 | TERT (SEQ ID NO: 92) | AGGTGTACGGTTTCGT (SEQ ID NO: 2187) |

(continued)

| No: | Gene | Oligo: |
|---|---|---|
| 80 | TERT (SEQ ID NO: 92) | AGGTGTATGGTTTTGT (SEQ ID NO: 2188) |
| 81 | TERT (SEQ ID NO: 92) | AGGTGTACGGTTTCGT (SEQ ID NO: 2187) |
| 82 | TERT (SEQ ID NO: 92) | AGGTGTATGGTTTTGT (SEQ ID NO: 2188) |
| 83 | TGFBR2 (SEQ ID NO: 93) | ATGGGGCGGACGAATA (SEQ ID NO: 1567) |
| 84 | TGFBR2 (SEQ ID NO: 93) | ATGGGGTGGATGAATA (SEQ ID NO: 1568) |
| 85 | TGFBR2 (SEQ ID NO: 93) | ATGGGGCGGACGAATA (SEQ ID NO: 1567) |
| 86 | TGFBR2 (SEQ ID NO: 93) | ATGGGGTGGATGAATA (SEQ ID NO: 1568) |
| 87 | THRB (SEQ ID NO: 106) | GGGCGGTTAAGTCGAG (SEQ ID NO: 1569) |
| 88 | THRB (SEQ ID NO: 106) | GGGTGGTTAAGTTGAG (SEQ ID NO: 1570) |
| 89 | THRB (SEQ ID NO: 106) | GGGCGGTTAAGTCGAG (SEQ ID NO: 1569) |
| 90 | THRB (SEQ ID NO: 106) | GGGTGGTTAAGTTGAG (SEQ ID NO: 1570) |
| 91 | TIMP3 (SEQ ID NO: 103) | TTATTAACGGAGGAAGG (SEQ ID NO: 1571) |
| 92 | TIMP3 (SEQ ID NO: 103) | TATTAATGGAGGAAGGG (SEQ ID NO: 1572) |
| 93 | TIMP3 (SEQ ID NO: 103) | TTCGTTATGTGTACGGAA (SEQ ID NO: 1573) |
| 94 | TIMP3 (SEQ ID NO: 103) | TTTGTTATGTGTATGGAA (SEQ ID NO: 1574) |
| 95 | TIMP3 (SEQ ID NO: 103) | TTCGTTATGTGTACGGAA (SEQ ID NO: 1573) |
| 96 | TIMP3 (SEQ ID NO: 103) | TTTGTTATGTGTATGGAA (SEQ ID NO: 1574) |
| 97 | TIMP3 (SEQ ID NO: 103) | GAGTATTTCGAGTTTGT (SEQ ID NO: 1575) |
| 98 | TIMP3 (SEQ ID NO: 103) | AGTATTTTGAGTTTGTATT (SEQ ID NO: 1576) |
| 99 | TIMP3 (SEQ ID NO: 103) | TAAGCGTTAATCGAGT (SEQ ID NO: 1577) |
| 100 | TIMP3 (SEQ ID NO: 103) | TAGGTAAGTGTTAATTGA (SEQ ID NO: 1578) |
| 101 | TP73 (SEQ ID NO: 86) | TTTCGGAGTTGCGAGT (SEQ ID NO: 1581) |
| 102 | TP73 (SEQ ID NO: 86) | TAGTTTTGGAGTTGTGA (SEQ ID NO: 1582) |
| 103 | CDH13 (SEQ ID NO: 70) | TAAAACGAGGGAGCGT (SEQ ID NO: 1583) |
| 104 | CDH13 (SEQ ID NO: 70) | AAAATGAGGGAGTGTT (SEQ ID NO: 1584) |
| 105 | CDH13 (SEQ ID NO: 70) | TAGTCGCGTGTATGAA (SEQ ID NO: 1585) |
| 106 | CDH13 (SEQ ID NO: 70) | TGTAGTTGTGTGTATGA (SEQ ID NO: 1586) |
| 107 | CDH13 (SEQ ID NO: 70) | ATGAAAACGTCGTCGG (SEQ ID NO: 1587) |
| 108 | CDH13 (SEQ ID NO: 70) | AATGAAAATGTTGTTGG (SEQ ID NO: 1588) |
| 109 | CDH13 (SEQ ID NO: 70) | TAGTCGAGAATTTCGT (SEQ ID NO: 1589) |
| 110 | CDH13 (SEQ ID NO: 70) | TGTAGTTGAGAATTTTGT (SEQ ID NO: 1590) |
| 111 | TMS1/ASC (SEQ ID NO: 84) | TTCGTTTCGGAGTCGA (SEQ ID NO: 1591) |
| 112 | TMS1/ASC (SEQ ID NO: 84) | TTTGTTTTGGAGTTGAT (SEQ ID NO: 1592) |
| 113 | APAF1 (SEQ ID NO: 82) | AGTAGCGTCGGGTTTT (SEQ ID NO: 1595) |
| 114 | APAF1 (SEQ ID NO: 82) | GAGTAGTGTTGGGTTT (SEQ ID NO: 1596) |
| 115 | SYK (SEQ ID NO: 60) | GAAGTTATCGCGTTGG (SEQ ID NO: 1597) |
| 116 | SYK (SEQ ID NO: 60) | AGAAGTTATTGTGTTGG (SEQ ID NO: 1598) |
| 117 | SYK (SEQ ID NO: 60) | GATCGATGCGGTTTAT (SEQ ID NO: 1599) |

(continued)

| No: | Gene | Oligo: |
|---|---|---|
| 118 | SYK (SEQ ID NO: 60) | GGGATTGATGTGGTTT (SEQ ID NO: 1600) |
| 119 | FABP3 (SEQ ID NO: 77) | GATGGGCGTATTAGTT (SEQ ID NO: 1605) |
| 120 | FABP3 (SEQ ID NO: 77) | GGGATGGGTGTATTAG (SEQ ID NO: 1606) |
| 121 | FABP3 (SEQ ID NO: 77) | GTGATGCGAGGGTTAT (SEQ ID NO: 1607) |
| 122 | FABP3 (SEQ ID NO: 77) | GTGATGTGAGGGTTAT (SEQ ID NO: 1608) |
| 123 | FABP3 (SEQ ID NO: 77) | GTGATGCGAGGGTTAT (SEQ ID NO: 1607) |
| 124 | FABP3 (SEQ ID NO: 77) | GTGATGTGAGGGTTAT (SEQ ID NO: 1608) |
| 125 | FABP3 (SEQ ID NO: 77) | TAAAGCGGTAGTTCGG (SEQ ID NO: 1609) |
| 126 | FABP3 (SEQ ID NO: 77) | AAGTGGTAGTTTGGGT (SEQ ID NO: 1610) |
| 127 | FABP3 (SEQ ID NO: 77) | TATTGGCGTTGACGTA (SEQ ID NO: 1611) |
| 128 | FABP3 (SEQ ID NO: 77) | TGGTGTTGATGTAGGT (SEQ ID NO: 1612) |
| 129 | RASSF1A (SEQ ID NO: 90) | TACGGGTATTTTCGCGT (SEQ ID NO: 1613) |
| 130 | RASSF1A (SEQ ID NO: 90) | ATATGGGTATTTTTGTGT (SEQ ID NO: 1614) |
| 131 | RASSF1A (SEQ ID NO: 90) | AGAGCGCGTTTAGTTT (SEQ ID NO: 1615) |
| 132 | RASSF1A (SEQ ID NO: 90) | GAGAGTGTGTTTAGTTT (SEQ ID NO: 1616) |
| 133 | RASSF1A (SEQ ID NO: 90) | AGTAAATCGGATTAGGA (SEQ ID NO: 1617) |
| 134 | RASSF1A (SEQ ID NO: 90) | AGTAAATTGGATTAGGAG (SEQ ID NO: 1618) |
| 135 | TWIST (SEQ ID NO: 100) | ATTGGGTCGTTGTAGA (SEQ ID NO: 1623) |
| 136 | TWIST (SEQ ID NO: 100) | ATTGGGTTGTTGTAGA (SEQ ID NO: 1624) |
| 137 | TWIST (SEQ ID NO: 100) | ATTGGGTCGTTGTAGA (SEQ ID NO: 1623) |
| 138 | TWIST (SEQ ID NO: 100) | ATTGGGTTGTTGTAGA (SEQ ID NO: 1624) |
| 139 | TWIST (SEQ ID NO: 100) | TAGGTCGGGACGTAAA (SEQ ID NO: 1625) |
| 140 | TWIST (SEQ ID NO: 100) | AGTAGGTTGGGATGTA (SEQ ID NO: 1626) |
| 141 | ESR2 (SEQ ID NO: 91) | TTTACGTGATCGAGTT (SEQ ID NO: 1631) |
| 142 | ESR2 (SEQ ID NO: 91) | AGTTTATGTGATTGAGTT (SEQ ID NO: 1632) |
| 143 | PLAU (SEQ ID NO: 62) | TATTTGTCGCGTTGAT (SEQ ID NO: 1633) |
| 144 | PLAU (SEQ ID NO: 62) | ATTTGTTGTGTTGATGA (SEQ ID NO: 1634) |
| 145 | PLAU (SEQ ID NO: 62) | TGTAATTCGGGGATTT (SEQ ID NO: 1635) |
| 146 | PLAU (SEQ ID NO: 62) | TTGTAATTTGGGGATTT (SEQ ID NO: 1636) |
| 147 | PLAU (SEQ ID NO: 62) | GAGCGTTGCGGAAGTA (SEQ ID NO: 1639) |
| 148 | PLAU (SEQ ID NO: 62) | GAGTGTTGTGGAAGTA (SEQ ID NO: 1640) |
| 149 | PLAU (SEQ ID NO: 62) | GAGCGTTGCGGAAGTA (SEQ ID NO: 1639) |
| 150 | PLAU (SEQ ID NO: 62) | GAGTGTTGTGGAAGTA (SEQ ID NO: 1640) |
| 151 | SLC19A1 (SEQ ID NO: 116) | GTCGTGCGGTTTTTAA (SEQ ID NO: 1663) |
| 152 | SLC19A1 (SEQ ID NO: 116) | GGTTGTGTGGTTTTTAA (SEQ ID NO: 1664) |
| 153 | SLC19A1 (SEQ ID NO: 116) | TTACGAAGGCGGTTTA (SEQ ID NO: 1665) |
| 154 | SLC19A1 (SEQ ID NO: 116) | TTTTATGAAGGTGGTTT (SEQ ID NO: 1666) |
| 155 | GJB2 (SEQ ID NO: 111) | GGATTTCGTCGGTATT (SEQ ID NO: 1667) |

(continued)

| No: | Gene | Oligo: |
|-----|------|--------|
| 156 | GJB2 (SEQ ID NO: 111) | GGGGATTTTGTTGGTA (SEQ ID NO: 1668) |
| 157 | HS3ST2 (SEQ ID NO: 113) | GAATCGGAGAGGCGAG (SEQ ID NO: 1671) |
| 158 | HS3ST2 (SEQ ID NO: 113) | AATTGGAGAGGTGAGG (SEQ ID NO: 1672) |
| 159 | HS3ST2 (SEQ ID NO: 113) | GGGTAATCGTTTGGTA (SEQ ID NO: 1673) |
| 160 | HS3ST2 (SEQ ID NO: 113) | GGGTAATTGTTTGGTAT (SEQ ID NO: 1674) |
| 161 | PRDM2 (SEQ ID NO: 114) | TGTAGAGACGACGATT (SEQ ID NO: 1675) |
| 162 | PRDM2 (SEQ ID NO: 114) | ATTGTAGAGATGATGATT (SEQ ID NO: 1676) |
| 163 | S100A7 (SEQ ID NO: 96) | TATAGTCGGGGTGATA (SEQ ID NO: 1689) |
| 164 | S100A7 (SEQ ID NO: 96) | TTTATAGTTGGGGTGAT (SEQ ID NO: 1690) |
| 165 | APC (SEQ ID NO: 65) | GATTCGTATTTCGTAGT (SEQ ID NO: 1695) |
| 166 | APC (SEQ ID NO: 65) | GATTCGTATTTCGTAGT (SEQ ID NO: 1695) |
| 167 | APC (SEQ ID NO: 65) | AGCGTTTTGGTTCGTAT (SEQ ID NO: 1696) |
| 168 | APC (SEQ ID NO: 65) | AGTGTTTTGGTTTGTAT (SEQ ID NO: 1697) |
| 169 | APC (SEQ ID NO: 65) | AGCGTTTTGGTTCGTAT (SEQ ID NO: 1696) |
| 170 | APC (SEQ ID NO: 65) | AGTGTTTTGGTTTGTAT (SEQ ID NO: 1697) |
| 171 | APC (SEQ ID NO: 65) | TTAATCGGCGGGTTTT (SEQ ID NO: 1698) |
| 172 | APC (SEQ ID NO: 65) | AGTTAATTGGTGGGTT (SEQ ID NO: 1699) |
| 173 | APC (SEQ ID NO: 65) | ATTTTCGAGTTCGGTA (SEQ ID NO: 1700) |
| 174 | APC (SEQ ID NO: 65) | TTTTTGAGTTTGGTAGT (SEQ ID NO: 1701) |
| 175 | SEQ ID NO:2 (SEQ ID NO: 2) | TTGATTGGCGGACGAG (SEQ ID NO: 1706) |
| 176 | SEQ ID NO:2 (SEQ ID NO: 2) | TTGATTGGTGGATGAG (SEQ ID NO: 1707) |
| 177 | SEQ ID NO:2 (SEQ ID NO: 2) | TTGATTGGCGGACGAG (SEQ ID NO: 1706) |
| 178 | SEQ ID NO:2 (SEQ ID NO: 2) | TTGATTGGTGGATGAG (SEQ ID NO: 1707) |
| 179 | IGFBP7 (SEQ ID NO: 94) | TAGTCGCGGAATGTTA (SEQ ID NO: 1708) |
| 180 | IGFBP7 (SEQ ID NO: 94) | TTGGTAGTTGTGGAAT (SEQ ID NO: 1709) |
| 181 | IGFBP7 (SEQ ID NO: 94) | ATTTTTTCGCGGGTAT (SEQ ID NO: 1710) |
| 182 | IGFBP7 (SEQ ID NO: 94) | TTTTTGTGGGTATTTTAG (SEQ ID NO: 1711) |
| 183 | IGFBP7 (SEQ ID NO: 94) | GGTATATTCGACGGGG (SEQ ID NO: 1712) |
| 184 | IGFBP7 (SEQ ID NO: 94) | GGGTATATTTGATGGGG (SEQ ID NO: 1713) |
| 185 | SOD2 (SEQ ID NO: 105) | TATTAGGCGGTTGCGG (SEQ ID NO: 1720) |
| 186 | SOD2 (SEQ ID NO: 105) | TATTAGGTGGTTGTGG (SEQ ID NO: 1721) |
| 187 | SOD2 (SEQ ID NO: 105) | TATTAGGCGGTTGCGG (SEQ ID NO: 1720) |
| 188 | SOD2 (SEQ ID NO: 105) | TATTAGGTGGTTGTGG (SEQ ID NO: 1721) |
| 189 | THBS1 (SEQ ID NO: 81) | TAAAGGGGCGTTCGTA (SEQ ID NO: 1726) |
| 190 | THBS 1 (SEQ ID NO: 81) | AAGGGGTGTTTGTATT (SEQ ID NO: 1727) |
| 191 | THBS1 (SEQ ID NO: 81) | GGTTAGTTCGGGCGTA (SEQ ID NO: 1728) |
| 192 | THBS1 (SEQ ID NO: 81) | GGTTAGTTTGGGTGTA (SEQ ID NO: 1729) |
| 193 | THBS1 (SEQ ID NO: 81) | GGTTAGTTCGGGCGTA (SEQ ID NO: 1728) |

(continued)

| No: | Gene | Oligo: |
|-----|------|--------|
| 194 | THBS1 (SEQ ID NO: 81) | GGTTAGTTTGGGTGTA (SEQ ID NO: 1729) |
| 195 | ESR1 (SEQ ID NO: 75) | AAATCGGCGGGTTATT (SEQ ID NO: 1732) |
| 196 | ESR1 (SEQ ID NO: 75) | AGAAATTGGTGGGTTA (SEQ ID NO: 1733) |
| 197 | ESR1 (SEQ ID NO: 75) | AGTTGCGGACGGTTTA (SEQ ID NO: 1734) |
| 198 | ESR1 (SEQ ID NO: 75) | AGTTGTGGATGGTTTA (SEQ ID NO: 1735) |
| 199 | ESR1 (SEQ ID NO: 75) | AGTTGCGGACGGTTTA (SEQ ID NO: 1734) |
| 200 | ESR1 (SEQ ID NO: 75) | AGTTGTGGATGGTTTA (SEQ ID NO: 1735) |
| 201 | CASP8 (SEQ ID NO: 71) | TGTATTCGAGGCGGTA (SEQ ID NO: 1740) |
| 202 | CASP8 (SEQ ID NO: 71) | TTGTATTTGAGGTGGT (SEQ ID NO: 1741) |
| 203 | HOXA5 (SEQ ID NO: 78) | TTCGAGTTCGGTTGAA (SEQ ID NO: 1746) |
| 204 | HOXA5 (SEQ ID NO: 78) | GTTTGAGTTTGGTTGAA (SEQ ID NO: 1747) |
| 205 | HOXA5 (SEQ ID NO: 78) | TAGTTTTCGGTCGGAA (SEQ ID NO: 1748) |
| 206 | HOXA5 (SEQ ID NO: 78) | TAGTTTTTGGTTGGAAG (SEQ ID NO: 1749) |
| 207 | HOXA5 (SEQ ID NO: 78) | TAATTCGATTTCGGTTT (SEQ ID NO: 1750) |
| 208 | HOXA5 (SEQ ID NO: 78) | TTTAATTTGATTTTGGTTT (SEQ ID NO: 1751) |
| 209 | HOXA5 (SEQ ID NO: 78) | ATCGGTAGTTGACGGTT (SEQ ID NO: 1752) |
| 210 | HOXA5 (SEQ ID NO: 78) | ATTGGTAGTTGATGGTT (SEQ ID NO: 1753) |
| 211 | HOXA5 (SEQ ID NO: 78) | ATCGGTAGTTGACGGTT (SEQ ID NO: 1752) |
| 212 | HOXA5 (SEQ ID NO: 78) | ATTGGTAGTTGATGGTT (SEQ ID NO: 1753) |
| 213 | RARA (SEQ ID NO: 108) | GAATTAGTATCGGTTTTT (SEQ ID NO: 1756) |
| 214 | RARA (SEQ ID NO: 108) | ATTAGTATTGGTTTTTGG (SEQ ID NO: 1757) |
| 215 | SNCG (SEQ ID NO: 73) | TGCGGTAGTATTCGAGT (SEQ ID NO: 1758) |
| 216 | SNCG (SEQ ID NO: 73) | TGTGGTAGTATTTGAGT (SEQ ID NO: 1759) |
| 217 | SNCG (SEQ ID NO: 73) | TGCGGTAGTATTCGAGT (SEQ ID NO: 1758) |
| 218 | SNCG (SEQ ID NO: 73) | TGTGGTAGTATTTGAGT (SEQ ID NO: 1759) |
| 219 | SNCG (SEQ ID NO: 73) | TATCGGGGATAGTCGTT (SEQ ID NO: 2199) |
| 220 | SNCG (SEQ ID NO: 73) | TATTGGGGATAGTTGTT (SEQ ID NO: 2200) |
| 221 | SNCG (SEQ ID NO: 73) | TATCGGGGATAGTCGTT (SEQ ID NO: 2199) |
| 222 | SNCG (SEQ ID NO: 73) | TATTGGGGATAGTTGTT (SEQ ID NO: 2200) |
| 223 | SNCG (SEQ ID NO: 73) | TATCGGCGTTAATAGG (SEQ ID NO: 2201) |
| 224 | SNCG (SEQ ID NO: 73) | TATTGGTGTTAATAGGAG (SEQ ID NO: 2202) |
| 225 | SLIT2 (SEQ ID NO: 112) | TTCGATAGTTAACGATG (SEQ ID NO: 1772) |
| 226 | SLIT2 (SEQ ID NO: 112) | TTTGATAGTTAATGATGGT (SEQ ID NO: 1773) |
| 227 | SLIT2 (SEQ ID NO: 112) | ATTTCGTCGTAGTTTG (SEQ ID NO: 1774) |
| 228 | SLIT2 (SEQ ID NO: 112) | TTTTGTTGTAGTTTGGA (SEQ ID NO: 1775) |
| 229 | SLIT2 (SEQ ID NO: 112) | TAGCGGGTTCGTAGTA (SEQ ID NO: 1776) |
| 230 | SLIT2 (SEQ ID NO: 112) | TTAGTGGGTTTGTAGTA (SEQ ID NO: 1777) |
| 231 | SLIT2 (SEQ ID NO: 112) | AAGGCGCGGAAGTTTA (SEQ ID NO: 1778) |

(continued)

| No: | Gene | Oligo: |
|---|---|---|
| 232 | SLIT2 (SEQ ID NO: 112) | AAGGTGTGGAAGTTTA (SEQ ID NO: 1779) |
| 233 | SLIT2 (SEQ ID NO: 112) | AAGGCGCGGAAGTTTA (SEQ ID NO: 1778) |
| 234 | SLIT2 (SEQ ID NO: 112) | AAGGTGTGGAAGTTTA (SEQ ID NO: 1779) |
| 235 | IGSF4 (SEQ ID NO: 74) | AGGTAGATCGAGGAGG (SEQ ID NO: 1780) |
| 236 | IGSF4 (SEQ ID NO: 74) | AGGTAGATTGAGGAGG (SEQ ID NO: 1781) |
| 237 | IGSF4 (SEQ ID NO: 74) | AGGTAGATCGAGGAGG (SEQ ID NO: 1780) |
| 238 | IGSF4 (SEQ ID NO: 74) | AGGTAGATTGAGGAGG (SEQ ID NO: 1781) |
| 239 | IGSF4 (SEQ ID NO: 74) | TAGGTTTTCGGATTGA (SEQ ID NO: 1784) |
| 240 | IGSF4 (SEQ ID NO: 74) | GTAGGTTTTTGGATTGA (SEQ ID NO: 1785) |
| 241 | MCT1 (SEQ ID NO: 101) | ATTTTACGTAGGCGTT (SEQ ID NO: 1786) |
| 242 | MCT1 (SEQ ID NO: 101) | GATTTTATGTAGGTGTTT (SEQ ID NO: 1787) |
| 243 | MCT1 (SEQ ID NO: 101) | AGTTAGTCGCGTTTTA (SEQ ID NO: 1788) |
| 244 | MCT1 (SEQ ID NO: 101) | AGAGTTAGTTGTGTTTTA (SEQ ID NO: 1789) |
| 245 | MCT1 (SEQ ID NO: 101) | TATACGAGGAAGGTCGG (SEQ ID NO: 1790) |
| 246 | MCT1 (SEQ ID NO: 101) | TATATGAGGAAGGTTGG (SEQ ID NO: 1791) |
| 247 | MCT1 (SEQ ID NO: 101) | TATACGAGGAAGGTCGG (SEQ ID NO: 1790) |
| 248 | MCT1 (SEQ ID NO: 101) | TATATGAGGAAGGTTGG (SEQ ID NO: 1791) |
| 249 | SEQ ID NO:6 (SEQ ID NO: 6) | AAGTTTATCGGCGTTT (SEQ ID NO: 1792) |
| 250 | SEQ ID NO:6 (SEQ ID NO: 6) | AGAAGTTTATTGGTGTTT (SEQ ID NO: 1793) |
| 251 | SEQ ID NO:6 (SEQ ID NO: 6) | ATTTCGGAATTTAAGCGT (SEQ ID NO: 1794) |
| 252 | SEQ ID NO:6 (SEQ ID NO: 6) | TTTTGGAATTTAAGTGTT (SEQ ID NO: 1795) |
| 253 | SEQ ID NO:6 (SEQ ID NO: 6) | TAATTTCGGACGCGGA (SEQ ID NO: 1796) |
| 254 | SEQ ID NO:6 (SEQ ID NO: 6) | TTTTGGATGTGGAGGA (SEQ ID NO: 1797) |
| 255 | SEQ ID NO:6 (SEQ ID NO: 6) | TTACGGTGAAGGCGGA (SEQ ID NO: 1798) |
| 256 | SEQ ID NO:6 (SEQ ID NO: 6) | TTATGGTGAAGGTGGA (SEQ ID NO: 1799) |
| 257 | SEQ ID NO:6 (SEQ ID NO: 6) | TTACGGTGAAGGCGGA (SEQ ID NO: 1798) |
| 258 | SEQ ID NO:6 (SEQ ID NO: 6) | TTATGGTGAAGGTGGA (SEQ ID NO: 1799) |
| 259 | SEQ ID NO:6 (SEQ ID NO: 6) | TTTCGGTTTTCGTTAAT (SEQ ID NO: 1800) |
| 260 | SEQ ID NO:6 (SEQ ID NO: 6) | TTTGGTTTTTGTTAATTTAG (SEQ ID NO: 1801) |
| 261 | SEQ ID NO:6 (SEQ ID NO: 6) | TGTGCGAAGTTAACGT (SEQ ID NO: 1802) |
| 262 | SEQ ID NO:6 (SEQ ID NO: 6) | TTGTGTGAAGTTAATGT (SEQ ID NO: 1803) |
| 263 | SEQ ID NO:8 (SEQ ID NO: 8) | ATAAAGCGGGGTTTTA (SEQ ID NO: 1804) |
| 264 | SEQ ID NO:8 (SEQ ID NO: 8) | GGATAAAGTGGGGTTT (SEQ ID NO: 1805) |
| 265 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGTGTATCGTTTTTCGG (SEQ ID NO: 1812) |
| 266 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAGTGTATTGTTTTTTGG (SEQ ID NO: 1813) |

(continued)

| No: | Gene | Oligo: |
|---|---|---|
| 267 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TGCGTATCGTTAGTTA (SEQ ID NO: 1814) |
| 268 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTTGTGTATTGTTAG (SEQ ID NO: 1815) |
| 269 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | ATTATTTCGGCGGTGA (SEQ ID NO: 1816) |
| 270 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GATTATTTTGGTGGTGA (SEQ ID NO: 1817) |
| 271 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAAGTCGCGTAAGGAG (SEQ ID NO: 1818) |
| 272 | PROSTAGLANDIN E2 RECEPTOR, EP4 SUBTYPE (PROSTANOID EP4 RECEPTOR) (PGE RECEPTOR, EP4 SUBTYPE) (SEQ ID NO:10) | AAGTTGTGTAAGGAGTA (SEQ ID NO: 1819) |
| 273 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATCGCGAGTTTGGA (SEQ ID NO: 1820) |
| 274 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATTGTGAGTTTGGAG (SEQ ID NO: 1821) |
| 275 | SEQ ID NO:51 (SEQ ID NO: 51) | GTCGGGGTCGATTCGA (SEQ ID NO: 1822) |
| 276 | SEQ ID NO:51 (SEQ ID NO: 51) | GTTGGGGTTGATTTGAT (SEQ ID NO: 1823) |
| 277 | SEQ ID NO:51 (SEQ ID NO: 51) | AGGATTCGTTTGCGTT (SEQ ID NO: 1824) |
| 278 | SEQ ID NO:51 (SEQ ID NO: 51) | AAGGATTTGTTTGTGTT (SEQ ID NO: 1825) |
| 279 | MGC34831 (SEQ ID NO: 52) | ATTAGCGTTTGGCGTT (SEQ ID NO: 1826) |
| 280 | MGC34831 (SEQ ID NO: 52) | AATTAGTGTTTGGTGTT (SEQ ID NO: 1827) |
| 281 | MGC34831 (SEQ ID NO: 52) | GTTTAGCGACGGTCGT (SEQ ID NO: 1828) |
| 282 | MGC34831 (SEQ ID NO: 52) | TGTTTAGTGATGGTTGT (SEQ ID NO: 1829) |
| 283 | SEQ ID NO:54 (SEQ ID NO: 54) | TGTGTAGCGGCGATTA (SEQ ID NO: 1830) |
| 284 | SEQ ID NO:54 (SEQ ID NO: 54) | GTGTGTAGTGGTGATT (SEQ ID NO: 1831) |
| 285 | SEQ ID NO:54 (SEQ ID NO: 54) | ATTAGCGTTTGGTCGG (SEQ ID NO: 1832) |
| 286 | SEQ ID NO:54 (SEQ ID NO: 54) | ATTAGTGTTTGGTTGGG (SEQ ID NO: 1833) |
| 287 | PDLIM1 (SEQ ID NO: 55) | TAGGTCGCGTAGTCGT (SEQ ID NO: 1834) |
| 288 | PDLIM1 (SEQ ID NO: 55) | TTAGGTTGTGTAGTTGT (SEQ ID NO: 1835) |
| 289 | DKK3 (SEQ ID NO: 24) | ATTCGTTTTAGTTCGAG (SEQ ID NO: 1842) |
| 290 | DKK3 (SEQ ID NO: 24) | ATTTGTTTTAGTTTGAGT (SEQ ID NO: 1843) |
| 291 | DKK3 (SEQ ID NO: 24) | TTCGATCGTTTTAGGA (SEQ ID NO: 1844) |
| 292 | DKK3 (SEQ ID NO: 24) | AGTTTTGATTGTTTTAGG (SEQ ID NO: 1845) |
| 293 | DKK3 (SEQ ID NO: 24) | GAAAAGACGCGATTTT (SEQ ID NO: 1848) |

(continued)

| No: | Gene | Oligo: |
|-----|------|--------|
| 294 | DKK3 (SEQ ID NO: 24) | GAAAAGATGTGATTTTATT (SEQ ID NO: 1849) |
| 295 | SEQ ID NO:29 (SEQ ID NO: 29) | TTGATGCGGAGTTCGA (SEQ ID NO: 1852) |
| 296 | SEQ ID NO:29 (SEQ ID NO: 29) | TTGATGTGGAGTTTGA (SEQ ID NO: 1853) |
| 297 | SEQ ID NO:29 (SEQ ID NO: 29) | TTGATGCGGAGTTCGA (SEQ ID NO: 1852) |
| 298 | SEQ ID NO:29 (SEQ ID NO: 29) | TTGATGTGGAGTTTGA (SEQ ID NO: 1853) |
| 299 | SEQ ID NO:29 (SEQ ID NO: 29) | TTCGAAGCGTGTATTA (SEQ ID NO: 2155) |
| 300 | SEQ ID NO:29 (SEQ ID NO: 29) | GGGTTTGAAGTGTGTA (SEQ ID NO: 2156) |
| 301 | SEQ ID NO:29 (SEQ ID NO: 29) | TAGGAACGGTAGGCGG (SEQ ID NO: 1854) |
| 302 | SEQ ID NO:29 (SEQ ID NO: 29) | TAGGAATGGTAGGTGG (SEQ ID NO: 1855) |
| 303 | SEQ ID NO:29 (SEQ ID NO: 29) | TAGGAACGGTAGGCGG (SEQ ID NO: 1854) |
| 304 | SEQ ID NO:29 (SEQ ID NO: 29) | TAGGAATGGTAGGTGG (SEQ ID NO: 1855) |
| 305 | SEQ ID NO:29 (SEQ ID NO: 29) | GTCGGAGGCGTTTGAG (SEQ ID NO: 1856) |
| 306 | SEQ ID NO:29 (SEQ ID NO: 29) | GTTGGAGGTGTTTGAGA (SEQ ID NO: 1857) |
| 307 | ARL7 (SEQ ID NO: 30) | TAGATTTCGTAGTTTTTTA (SEQ ID NO: 1858) |
| 308 | ARL7 (SEQ ID NO: 30) | TAGATTTTGTAGTTTTTTAA (SEQ ID NO: 1859) |
| 309 | ARL7 (SEQ ID NO: 30) | TGGGTACGTTTACGGT (SEQ ID NO: 1860) |
| 310 | ARL7 (SEQ ID NO: 30) | TGGGTATGTTTATGGTT (SEQ ID NO: 1861) |
| 311 | ARL7 (SEQ ID NO: 30) | GAAGAAATCGTTTTTGT (SEQ ID NO: 1862) |
| 312 | ARL7 (SEQ ID NO: 30) | GAAGAAATTGTTTTTGTT (SEQ ID NO: 1863) |
| 313 | ARL7 (SEQ ID NO: 30) | TAGTAGGATCGGTTTTT (SEQ ID NO: 1864) |
| 314 | ARL7 (SEQ ID NO: 30) | ATAGTAGGATTGGTTTTT (SEQ ID NO: 1865) |
| 315 | SEQ ID NO:31 (SEQ ID NO: 31) | AACGTTGCGTTGGGTA (SEQ ID NO: 1866) |
| 316 | SEQ ID NO:31 (SEQ ID NO: 31) | AATGTTGTGTTGGGTAA (SEQ ID NO: 1867) |
| 317 | SEQ ID NO:31 (SEQ ID NO: 31) | TAGCGTTTCGTGGTTA (SEQ ID NO: 1868) |
| 318 | SEQ ID NO:31 (SEQ ID NO: 31) | GTAGTGTTTTGTGGTTA (SEQ ID NO: 1869) |
| 319 | THH (SEQ ID NO: 32) | GTTCGTTGGCGTAAAT (SEQ ID NO: 1872) |
| 320 | THH (SEQ ID NO: 32) | GGTTTGTTGGTGTAAAT (SEQ ID NO: 1873) |
| 321 | (SEQ ID NO: 36) | GGACGTTGCGATTGTA (SEQ ID NO: 2161) |
| 322 | (SEQ ID NO: 36) | GATGTTGTGATTGTAGT (SEQ ID NO: 2162) |
| 323 | SENP3 (SEQ ID NO: 39) | TATTCGGATCGGGTTT (SEQ ID NO: 1876) |
| 324 | SENP3 (SEQ ID NO: 39) | TTTATTTGGATTGGGTT (SEQ ID NO: 1877) |
| 325 | SENP3 (SEQ ID NO: 39) | TAGTCGAAAGTAGGACGT (SEQ ID NO: 1878) |
| 326 | SENP3 (SEQ ID NO: 39) | TAGTTGAAAGTAGGATGT (SEQ ID NO: 1879) |
| 327 | SENP3 (SEQ ID NO: 39) | TAGTCGAAAGTAGGACGT (SEQ ID NO: 1878) |
| 328 | SENP3 (SEQ ID NO: 39) | TAGTTGAAAGTAGGATGT (SEQ ID NO: 1879) |
| 329 | SENP3 (SEQ ID NO: 39) | AGTTATCGTTAGGAGGG (SEQ ID NO: 1882) |
| 330 | SENP3 (SEQ ID NO: 39) | AGTTATTGTTAGGAGGG (SEQ ID NO: 1883) |
| 331 | SENP3 (SEQ ID NO: 39) | AGTTATCGTTAGGAGGG (SEQ ID NO: 1882) |

(continued)

| No: | Gene | Oligo: |
|---|---|---|
| 332 | SENP3 (SEQ ID NO: 39) | AGTTATTGTTAGGAGGG (SEQ ID NO: 1883) |
| 333 | SEQ ID NO:42 (SEQ ID NO: 42) | GAGTCGGGTTGCGATG (SEQ ID NO: 1884) |
| 334 | SEQ ID NO:42 (SEQ ID NO: 42) | GGAGTTGGGTTGTGAT (SEQ ID NO: 1885) |
| 335 | SEQ ID NO:42 (SEQ ID NO: 42) | ATGGGTTGCGATTTTTA (SEQ ID NO: 1886) |
| 336 | SEQ ID NO:42 (SEQ ID NO: 42) | ATGGGTTGTGATTTTTA (SEQ ID NO: 1887) |
| 337 | SEQ ID NO:42 (SEQ ID NO: 42) | ATGGGTTGCGATTTTTA (SEQ ID NO: 1886) |
| 338 | SEQ ID NO:42 (SEQ ID NO: 42) | ATGGGTTGTGATTTTTA (SEQ ID NO: 1887) |
| 339 | (SEQ ID NO: 117) | TAGCGGTTTTTTAGCGTA (SEQ ID NO: 1888) |
| 340 | (SEQ ID NO: 117) | AGTGGTTTTTTAGTGTAT (SEQ ID NO: 1889) |
| 341 | (SEQ ID NO: 117) | AGATGCGCGGGTAGAT (SEQ ID NO: 1890) |
| 342 | (SEQ ID NO: 117) | AGATGTGTGGGTAGAT (SEQ ID NO: 1891) |
| 343 | (SEQ ID NO: 117) | AGATGCGCGGGTAGAT (SEQ ID NO: 1890) |
| 344 | (SEQ ID NO: 117) | AGATGTGTGGGTAGAT (SEQ ID NO: 1891) |
| 345 | (SEQ ID NO: 117) | AGATAGTTCGTATTCGT (SEQ ID NO: 1892) |
| 346 | (SEQ ID NO: 117) | GTAGATAGTTTGTATTTGT (SEQ ID NO: 1893) |
| 347 | (SEQ ID NO: 117) | TTTGTTCGTAACGTTT (SEQ ID NO: 1894) |
| 348 | (SEQ ID NO: 117) | TGTTTGTAATGTTTAGAG (SEQ ID NO: 1895) |
| 349 | 060279 (SEQ ID NO: 47) | GGACGGCGGAAAATTA (SEQ ID NO: 1902) |
| 350 | 060279 (SEQ ID NO: 47) | AGGGATGGTGGAAAAT (SEQ ID NO: 1903) |
| 351 | SEQ ID NO:48 (SEQ ID NO: 48) | TATTTGGCGATTCGGA (SEQ ID NO: 1904) |
| 352 | SEQ ID NO:48 (SEQ ID NO: 48) | TGGTGATTTGGAGATT (SEQ ID NO: 1905) |
| 353 | SEQ ID NO:48 (SEQ ID NO: 48) | TAGGGTTACGTGTCGG (SEQ ID NO: 1906) |
| 354 | SEQ ID NO:48 (SEQ ID NO: 48) | TAGGGTTATGTGTTGG (SEQ ID NO: 1907) |
| 355 | SEQ ID NO:48 (SEQ ID NO: 48) | TAGGGTTACGTGTCGG (SEQ ID NO: 1906) |
| 356 | SEQ ID NO:48 (SEQ ID NO: 48) | TAGGGTTATGTGTTGG (SEQ ID NO: 1907) |
| 357 | SEQ ID NO:48 (SEQ ID NO: 48) | AAAATCGTATGCGTGT (SEQ ID NO: 1910) |
| 358 | SEQ ID NO:48 (SEQ ID NO: 48) | GAAAATTGTATGTGTGTG (SEQ ID NO: 1911) |
| 359 | SEQ ID NO:9 (SEQ ID NO: 9) | AGTGTTCGTCGTAGTT (SEQ ID NO: 1914) |
| 360 | SEQ ID NO:9 (SEQ ID NO: 9) | TGAGTGTTTGTTGTAGT (SEQ ID NO: 1915) |
| 361 | SEQ ID NO:4 (SEQ ID NO: 4) | TTTTGTTCGCGTTGAA (SEQ ID NO: 1916) |
| 362 | SEQ ID NO:4 (SEQ ID NO: 4) | TTGTTTGTGTTGAAGTA (SEQ ID NO: 1917) |
| 363 | SEQ ID NO:4 (SEQ ID NO: 4) | GGGTCGCGAGGTAGTT (SEQ ID NO: 1918) |
| 364 | SEQ ID NO:4 (SEQ ID NO: 4) | TGGGTTGTGAGGTAGT (SEQ ID NO: 1919) |
| 365 | SEQ ID NO:4 (SEQ ID NO: 4) | TTTGTGCGACGTTATT (SEQ ID NO: 1920) |
| 366 | SEQ ID NO:4 (SEQ ID NO: 4) | GGTTTGTGTGATGTTAT (SEQ ID NO: 1921) |
| 367 | SEQ ID NO:4 (SEQ ID NO: 4) | ATGGCGGTTTCGATTT (SEQ ID NO: 1922) |
| 368 | SEQ ID NO:4 (SEQ ID NO: 4) | GATGGTGGTTTTGATTT (SEQ ID NO: 1923) |
| 369 | SEQ ID NO:7 (SEQ ID NO: 7) | TAGTCGTCGTGTAGGA (SEQ ID NO: 1932) |

(continued)

| No: | Gene | Oligo: |
|---|---|---|
| 370 | SEQ ID NO:7 (SEQ ID NO: 7) | TAGGTAGTTGTTGTGTA (SEQ ID NO: 1933) |
| 371 | SEQ ID NO:7 (SEQ ID NO: 7) | TATAGGTACGCGATGA (SEQ ID NO: 1934) |
| 372 | SEQ ID NO:7 (SEQ ID NO: 7) | AGGTATGTGATGAGGA (SEQ ID NO: 1935) |
| 373 | SEQ ID NO:7 (SEQ ID NO: 7) | ATGATTTGCGTTACGT (SEQ ID NO: 1938) |
| 374 | SEQ ID NO:7 (SEQ ID NO: 7) | ATGATTTGTGTTATGTTT (SEQ ID NO: 1939) |
| 375 | SEQ ID NO:7 (SEQ ID NO: 7) | TAACGTTGTGGTTCGAA (SEQ ID NO: 1940) |
| 376 | SEQ ID NO:7 (SEQ ID NO: 7) | TAATGTTGTGGTTTGAA (SEQ ID NO: 1941) |
| 377 | SEQ ID NO:7 (SEQ ID NO: 7) | TAACGTTGTGGTTCGAA (SEQ ID NO: 1940) |
| 378 | SEQ ID NO:7 (SEQ ID NO: 7) | TAATGTTGTGGTTTGAA (SEQ ID NO: 1941) |
| 379 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 380 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |
| 381 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATCGAGGCGGT (SEQ ID NO: 1952) |
| 382 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTAATTGAGGTGGT (SEQ ID NO: 1953) |
| 383 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTACGGAGGCGTTTTA (SEQ ID NO: 1958) |
| 384 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTATGGAGGTGTTTT (SEQ ID NO: 1959) |
| 385 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | AGGCGAATTTATCGGG (SEQ ID NO: 1960) |
| 386 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | GGTGAATTTATTGGGG (SEQ ID NO: 1961) |
| 387 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTCGAAGTAGGCGT (SEQ ID NO: 1962) |

(continued)

| No: | Gene | Oligo: |
|-----|------|--------|
| 388 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTTGAAGTAGGTGTT (SEQ ID NO: 1963) |
| 389 | LIM DOMAIN KINASE 1 (EC 2.7.1.37) (LIMK-1) (SEQ ID NO: 12) | TGTAGTCGGGAGGTTA (SEQ ID NO: 1966) |
| 390 | LIM DOMAIN KINASE 1 (EC 2.7.1.37) (LIMK-1) (SEQ ID NO: 12) | TGTAGTTGGGAGGTTA (SEQ ID NO: 1967) |
| 391 | LIM DOMAIN KINASE 1 (EC 2.7.1.37) (LIMK-1) (SEQ ID NO: 12) | TGTAGTCGGGAGGTTA (SEQ ID NO: 1966) |
| 392 | LIM DOMAIN KINASE 1 (EC 2.7.1.37) (LIMK-1) (SEQ ID NO: 12) | TGTAGTTGGGAGGTTA (SEQ ID NO: 1967) |
| 393 | LIM DOMAIN KINASE 1 (EC 2.7.1.37) (LIMK-1) (SEQ ID NO: 12) | GGATTATCGCGGGGGT (SEQ ID NO: 1968) |
| 394 | LIM DOMAIN KINASE 1 (EC 2.7.1.37) (LIMK-1) (SEQ ID NO: 12) | GGATTATTGTGGGGGT (SEQ ID NO: 1969) |
| 395 | LIM DOMAIN KINASE 1 (EC 2.7.1.37) (LIMK-1) (SEQ ID NO: 12) | GGATTATCGCGGGGGT (SEQ ID NO: 1968) |
| 396 | LIM DOMAIN KINASE 1 (EC 2.7.1.37) (LIMK-1) (SEQ ID NO: 12) | GGATTATTGTGGGGGT (SEQ ID NO: 1969) |
| 397 | LIM DOMAIN KINASE 1 (EC 2.7.1.37) (LIMK-1) (SEQ ID NO: 12) | GTCGGTAGTTTATCGGAT (SEQ ID NO: 1970) |
| 398 | LIM DOMAIN KINASE 1 (EC 2.7.1.37) (LIMK-1) (SEQ ID NO: 12) | GTTGGTAGTTTATTGGAT (SEQ ID NO: 1971) |
| 399 | LIM DOMAIN KINASE 1 (EC 2.7.1.37) (LIMK-1) (SEQ ID NO: 12) | GTCGGTAGTTTATCGGAT (SEQ ID NO: 1970) |
| 400 | LIM DOMAIN KINASE 1 (EC 2.7.1.37) (LIMK-1) (SEQ ID NO: 12) | GTTGGTAGTTTATTGGAT (SEQ ID NO: 1971) |
| 401 | BCL11B (SEQ ID NO: 50) | TAGGTTTCGATTCGTT (SEQ ID NO: 1974) |
| 402 | BCL11B (SEQ ID NO: 50) | TTAGGTTTTGATTTGTTT (SEQ ID NO: 1975) |
| 403 | BCL11B (SEQ ID NO: 50) | AAGTCGTCGGAGTTAG (SEQ ID NO: 1976) |
| 404 | BL11B (SEQ ID NO: 50) | GTGAAGTTGTTGGAGT (SEQ ID NO: 1977) |
| 405 | MSF (SEQ ID NO: 13) | GTTTCGAAATTGGCGT (SEQ ID NO: 1980) |
| 406 | MSF (SEQ ID NO: 13) | TTTGAAATTGGTGTGG (SEQ ID NO: 1981) |
| 407 | MSF (SEQ ID NO: 13) | TTCGGTTTACGGGTTGTA (SEQ ID NO: 1982) |
| 408 | MSF (SEQ ID NO: 13) | TTTGGTTTATGGGTTGTA (SEQ ID NO: 1983) |
| 409 | MSF (SEQ ID NO: 13) | TTCGGTTTACGGGTTGTA (SEQ ID NO: 1982) |
| 410 | MSF (SEQ ID NO: 13) | TTTGGTTTATGGGTTGTA (SEQ ID NO: 1983) |
| 411 | MSF (SEQ ID NO: 13) | TTACGGTTCGATTTTG (SEQ ID NO: 1984) |
| 412 | MSF (SEQ ID NO: 13) | TATGGTTTGATTTTGGG (SEQ ID NO: 1985) |
| 413 | SEQ ID NO: 14 (SEQ ID NO: 14) | TAAGGCGTTTTCGATA (SEQ ID NO: 1986) |
| 414 | SEQ ID NO: 14 (SEQ ID NO: 14) | GTAAGGTGTTTTTGATAT (SEQ ID NO: 1987) |

(continued)

| No: | Gene | Oligo: |
|---|---|---|
| 415 | SEQ ID NO: 16 (SEQ ID NO: 16) | AGGAGTTTTCGTGTCGT (SEQ ID NO: 1992) |
| 416 | SEQ ID NO: 16 (SEQ ID NO: 16) | AGGAGTTTTTGTGTTGT (SEQ ID NO: 1993) |
| 417 | SEQ ID NO: 16 (SEQ ID NO: 16) | AGGAGTTTTCGTGTCGT (SEQ ID NO: 1992) |
| 418 | SEQ ID NO: 16 (SEQ ID NO: 16) | AGGAGTTTTTGTGTTGT (SEQ ID NO: 1993) |
| 419 | SEQ ID NO:16 (SEQ ID NO: 16) | AGGCGTTGTCGGTGAT (SEQ ID NO: 1996) |
| 420 | SEQ ID NO: 16 (SEQ ID NO: 16) | AGGTGTTGTTGGTGATA (SEQ ID NO: 1997) |
| 421 | SEQ ID NO: 17 (SEQ ID NO: 17) | TACGTTTCGGGTTTGTTA (SEQ ID NO: 1998) |
| 422 | SEQ ID NO: 17 (SEQ ID NO: 17) | TATGTTTTGGGTTTGTTA (SEQ ID NO: 1999) |
| 423 | SEQ ID NO: 17 (SEQ ID NO: 17) | TACGTTTCGGGTTTGTTA (SEQ ID NO: 1998) |
| 424 | SEQ ID NO: 17 (SEQ ID NO: 17) | TATGTTTTGGGTTTGTTA (SEQ ID NO: 1999) |
| 425 | SEQ ID NO: 17 (SEQ ID NO: 17) | ATTTAGTCGTGCGTTT (SEQ ID NO: 2000) |
| 426 | SEQ ID NO: 17 (SEQ ID NO: 17) | TGATTTAGTTGTGTGTT (SEQ ID NO: 2001) |
| 427 | SEQ ID NO: 18 (SEQ ID NO: 18) | TTTACGCGGGGTTTTA (SEQ ID NO: 2002) |
| 428 | SEQ ID NO: 18 (SEQ ID NO: 18) | TTTATGTGGGGTTTTAG (SEQ ID NO: 2003) |
| 429 | SEQ ID NO: 18 (SEQ ID NO: 18) | TTACGTCGTTATTAGGT (SEQ ID NO: 2004) |
| 430 | SEQ ID NO: 18 (SEQ ID NO: 18) | TTTTATGTTGTTATTAGGT (SEQ ID NO: 2005) |
| 431 | SEQ ID NO: 18 (SEQ ID NO: 18) | AAAGCGGAGTCGTTAG (SEQ ID NO: 2010) |
| 432 | SEQ ID NO: 18 (SEQ ID NO: 18) | AGTGGAGTTGTTAGGT (SEQ ID NO: 2011) |
| 433 | SEQ ID NO: 19 (SEQ ID NO: 19) | AGGTTTTCGTTGTAGTA (SEQ ID NO: 2012) |
| 434 | SEQ ID NO: 19 (SEQ ID NO: 19) | TAGGTTTTTGTTGTAGTA (SEQ ID NO: 2013) |
| 435 | NR2E1 (SEQ ID NO: 22) | AATGTAGCGGCGTTAT (SEQ ID NO: 2028) |
| 436 | NR2E1 (SEQ ID NO: 22) | TAAATGTAGTGGTGTTAT (SEQ ID NO: 2029) |
| 437 | NR2E1 (SEQ ID NO: 22) | GTCGTTATCGGTTTGGA (SEQ ID NO: 2030) |
| 438 | NR2E1 (SEQ ID NO: 22) | GTTGTTATTGGTTTGGA (SEQ ID NO: 2031) |
| 439 | NR2E1 (SEQ ID NO: 22) | GTCGTTATCGGTTTGGA (SEQ ID NO: 2030) |
| 440 | NR2E1 (SEQ ID NO: 22) | GTTGTTATTGGTTTGGA (SEQ ID NO: 2031) |
| 441 | NR2E1 (SEQ ID NO: 22) | GACGTAAGTTTCGGGT (SEQ ID NO: 2032) |
| 442 | NR2E1 (SEQ ID NO: 22) | GGATGTAAGTTTTGGG (SEQ ID NO: 2033) |
| 443 | NR2E 1 (SEQ ID NO: 22) | TTTTTAGTCGCGAGAA (SEQ ID NO: 2034) |
| 444 | NR2E1 (SEQ ID NO: 22) | TTTTTAGTTGTGAGAAGT (SEQ ID NO: 2035) |
| 445 | NR2E1 (SEQ ID NO: 22) | TTCGGGTGATATCGTTT (SEQ ID NO: 2036) |
| 446 | NR2E1 (SEQ ID NO: 22) | TTTGGGTGATATTGTTT (SEQ ID NO: 2037) |
| 447 | NR2E1 (SEQ ID NO: 22) | TTCGGGTGATATCGTTT (SEQ ID NO: 2036) |
| 448 | NR2E1 (SEQ ID NO: 22) | TTTGGGTGATATTGTTT (SEQ ID NO: 2037) |
| 449 | NR2E1 (SEQ ID NO: 22) | AGGCGAGTCGGAGTTT (SEQ ID NO: 2038) |
| 450 | NR2E1 (SEQ ID NO: 22) | AGGTGAGTTGGAGTTTT (SEQ ID NO: 2039) |
| 451 | NR2E1 (SEQ ID NO: 22) | AGGGACGCGAAAATTT (SEQ ID NO: 2042) |
| 452 | NR2E1 (SEQ ID NO: 22) | GGAGGGATGTGAAAAT (SEQ ID NO: 2043) |

(continued)

| No: | Gene | Oligo: |
|---|---|---|
| 453 | PCDH7 (SEQ ID NO: 23) | ATCGTAGTCGGTTTTA (SEQ ID NO: 2044) |
| 454 | PCDH7 (SEQ ID NO: 23) | GATTATTGTAGTTGGTTT (SEQ ID NO: 2045) |
| 455 | RTTN (SEQ ID NO: 25) | TAGATTGACGGGACGA (SEQ ID NO: 2221) |
| 456 | RTTN (SEQ ID NO: 25) | TAGATTGATGGGATGAG (SEQ ID NO: 2222) |
| 457 | RTTN (SEQ ID NO: 25) | TAGTGGCGCGGTAGTT (SEQ ID NO: 2046) |
| 458 | RTTN (SEQ ID NO: 25) | TAGTGGTGTGGTAGTTT (SEQ ID NO: 2047) |
| 459 | RTTN (SEQ ID NO: 25) | TAGGACGTGTTTTCGG (SEQ ID NO: 2048) |
| 460 | RTTN (SEQ ID NO: 25) | AGGATGTGTTTTTGGG (SEQ ID NO: 2049) |
| 461 | SNAP25 (SEQ ID NO: 33) | TATTTCGAGTTAGAGTTACGA (SEQ ID NO: 2050) |
| 462 | SNAP25 (SEQ ID NO: 33) | TATTTTGAGTTAGAGTTATGA (SEQ ID NO: 2051) |
| 463 | SNAP25 (SEQ ID NO: 33) | TATTTCGAGTTAGAGTTACGA (SEQ ID NO: 2050) |
| 464 | SNAP25 (SEQ ID NO: 33) | TATTTTGAGTTAGAGTTATGA (SEQ ID NO: 2051) |
| 465 | GIRK2 (SEQ ID NO: 27) | AGTTGTTCGTAGGCGA (SEQ ID NO: 2060) |
| 466 | GIRK2 (SEQ ID NO: 27) | AGTTGTTTGTAGGTGA (SEQ ID NO: 2061) |
| 467 | GIRK2 (SEQ ID NO: 27) | AGTTGTTCGTAGGCGA (SEQ ID NO: 2060) |
| 468 | GIRK2 (SEQ ID NO: 27) | AGTTGTTTGTAGGTGA (SEQ ID NO: 2061) |
| 469 | GIRK2 (SEQ ID NO: 27) | TTATTTCGTTCGTAGTT (SEQ ID NO: 2062) |
| 470 | GIRK2 (SEQ ID NO: 27) | TTTGTTTGTAGTTAGGTA (SEQ ID NO: 2063) |
| 471 | GIRK2 (SEQ ID NO: 27) | TTAGTCGAAAGGCGAG (SEQ ID NO: 2064) |
| 472 | GIRK2 (SEQ ID NO: 27) | TAGTTGAAAGGTGAGG (SEQ ID NO: 2065) |
| 473 | SEQ ID NO:28 (SEQ ID NO: 28) | ATTAGGCGAGTTTCGT (SEQ ID NO: 2066) |
| 474 | SEQ ID NO:28 (SEQ ID NO: 28) | TTAGGTGAGTTTTGTTT (SEQ ID NO: 2067) |
| 475 | SEQ ID NO:31 (SEQ ID NO: 31) | TTTACGTAGGGCGATT (SEQ ID NO: 2068) |
| 476 | SEQ ID NO:31 (SEQ ID NO: 31) | ATTTTTATGTAGGGTGAT (SEQ ID NO: 2069) |
| 477 | SEQ ID NO:31 (SEQ ID NO: 31) | GATACGGTTAGGCGGG (SEQ ID NO: 2070) |
| 478 | SEQ ID NO:31 (SEQ ID NO: 31) | GATATGGTTAGGTGGG (SEQ ID NO: 2071) |
| 479 | SEQ ID NO:31 (SEQ ID NO: 31) | GATACGGTTAGGCGGG (SEQ ID NO: 2070) |
| 480 | SEQ ID NO:31 (SEQ ID NO: 31) | GATATGGTTAGGTGGG (SEQ ID NO: 2071) |
| 481 | SEQ ID NO:31 (SEQ ID NO: 31) | TTCGGGCGTTTTATAT (SEQ ID NO: 2072) |
| 482 | SEQ ID NO:31 (SEQ ID NO: 31) | GGTTTGGGTGTTTTATA (SEQ ID NO: 2073) |
| 483 | HOXB13 (SEQ ID NO: 34) | GTCGTTATTATTTCGAGG (SEQ ID NO: 2074) |
| 484 | HOXB 13 (SEQ ID NO: 34) | GTTGTTATTATTTTGAGGA (SEQ ID NO: 2075) |
| 485 | HOXB 13 (SEQ ID NO: 34) | AAGTTAGCGGGTTCGT (SEQ ID NO: 2076) |
| 486 | HOXB 13 (SEQ ID NO: 34) | AAGTTAGTGGGTTTGT (SEQ ID NO: 2077) |
| 487 | HOXB 13 (SEQ ID NO: 34) | AAGTTAGCGGGTTCGT (SEQ ID NO: 2076) |
| 488 | HOXB 13 (SEQ ID NO: 34) | AAGTTAGTGGGTTTGT (SEQ ID NO: 2077) |
| 489 | LMX1A (SEQ ID NO: 38) | GTCGGGTTTTTCGGAA (SEQ ID NO: 2092) |
| 490 | LMX1A (SEQ ID NO: 38) | GTTGGGTTTTTTGGAAG (SEQ ID NO: 2093) |

(continued)

| No: | Gene | Oligo: |
|---|---|---|
| 491 | TITF1 (SEQ ID NO: 41) | GTCGTGGGGATCGTAT (SEQ ID NO: 2104) |
| 492 | TITF (SEQ ID NO: 41) | GTTGTGGGGATTGTAT (SEQ ID NO: 2105) |
| 493 | TITF1 (SEQ ID NO: 41) | GTCGTGGGGATCGTAT (SEQ ID NO: 2104) |
| 494 | TITF1 (SEQ ID NO: 41) | GTTGTGGGGATTGTAT (SEQ ID NO: 2105) |
| 495 | TITF1 (SEQ ID NO: 41) | GGTTCGTTTAAGTTCGG (SEQ ID NO: 2106) |
| 496 | TITF1 (SEQ ID NO: 41) | GGTTTGTTTAAGTTTGG (SEQ ID NO: 2107) |
| 497 | TITF1 (SEQ ID NO: 41) | GGTTCGTTTAAGTTCGG (SEQ ID NO: 2106) |
| 498 | TITF1 (SEQ ID NO: 41) | GGTTTGTTTAAGTTTGG (SEQ ID NO: 2107) |
| 499 | TITF1 (SEQ ID NO: 41) | TATTTCGTTTTCGTAATT (SEQ ID NO: 2110) |
| 500 | TITF1 (SEQ ID NO: 41) | TTTGTTTTTGTAATTAGATT (SEQ ID NO: 2111) |
| 501 | DDX51 (SEQ ID NO: 43) | AACGTGCGGGGTTTTT (SEQ ID NO: 2116) |
| 502 | DDX51 (SEQ ID NO: 43) | TGAATGTGTGGGGTTT (SEQ ID NO: 2117) |
| 503 | SEQ ID NO:46 (SEQ ID NO: 46) | GAGTCGGGTTATCGTT (SEQ ID NO: 2120) |
| 504 | SEQ ID NO:46 (SEQ ID NO: 46) | GGAGTTGGGTTATTGT (SEQ ID NO: 2121) |
| 505 | SEQ ID NO:46 (SEQ ID NO: 46) | TTACGGATGTTTCGGT (SEQ ID NO: 2122) |
| 506 | SEQ ID NO:46 (SEQ ID NO: 46) | TTTATGGATGTTTTGGT (SEQ ID NO: 2123) |
| 507 | SEQ ID NO:46 (SEQ ID NO: 46) | TGACGTATTTTCGGTT (SEQ ID NO: 2124) |
| 508 | SEQ ID NO:46 (SEQ ID NO: 46) | GGTGATGTATTTTTGGT (SEQ ID NO: 2125) |
| 509 | SEQ ID NO:46 (SEQ ID NO: 46) | ATAGTCGGAATCGTTG (SEQ ID NO: 2126) |
| 510 | SEQ ID NO:46 (SEQ ID NO: 46) | TAGTTGGAATTGTTGTT (SEQ ID NO: 2127) |
| 511 | SEQ ID NO:2 (SEQ ID NO: 2) | TAGTAGTCGGCGGGAG (SEQ ID NO: 2130) |
| 512 | SEQ ID NO:2 (SEQ ID NO: 2) | TAGTAGTTGGTGGGAG (SEQ ID NO: 2131) |
| 513 | SEQ ID NO:2 (SEQ ID NO: 2) | TAGTAGTCGGCGGGAG (SEQ ID NO: 2130) |
| 514 | SEQ ID NO:2 (SEQ ID NO: 2) | TAGTAGTTGGTGGGAG (SEQ ID NO: 2131) |
| 515 | SEQ ID NO:3 (SEQ ID NO: 3) | TTGCGTTAATTCGGTA (SEQ ID NO: 2132) |
| 516 | SEQ ID NO:3 (SEQ ID NO: 3) | AATTTGTGTTAATTTGGT (SEQ ID NO: 2133) |
| 517 | SEQ ID NO:3 (SEQ ID NO: 3) | AGTCGGGAGAGCGAAA (SEQ ID NO: 2134) |
| 518 | SEQ ID NO:3 (SEQ ID NO: 3) | AGTTGGGAGAGTGAAA (SEQ ID NO: 2135) |
| 519 | SEQ ID NO:3 (SEQ ID NO: 3) | AGTCGGGAGAGCGAAA (SEQ ID NO: 2134) |
| 520 | SEQ ID NO:3 (SEQ ID NO: 3) | AGTTGGGAGAGTGAAA (SEQ ID NO: 2135) |
| 521 | SEQ ID NO:3 (SEQ ID NO: 3) | GGTCGAAGAGTCGGGA (SEQ ID NO: 2136) |
| 522 | SEQ ID NO:3 (SEQ ID NO: 3) | GGTTGAAGAGTTGGGA (SEQ ID NO: 2137) |
| 523 | SEQ ID NO:3 (SEQ ID NO: 3) | GGTCGAAGAGTCGGGA (SEQ ID NO: 2136) |
| 524 | SEQ ID NO:3 (SEQ ID NO: 3) | GGTTGAAGAGTTGGGA (SEQ ID NO: 2137) |
| 525 | SEQ ID NO:3 (SEQ ID NO: 3) | ATGTTAGCGGGTCGAA (SEQ ID NO: 2138) |
| 526 | SEQ ID NO:3 (SEQ ID NO: 3) | ATGTTAGTGGGTTGAA (SEQ ID NO: 2139) |
| 527 | SEQ ID NO:3 (SEQ ID NO: 3) | ATGTTAGCGGGTCGAA (SEQ ID NO: 2138) |
| 528 | SEQ ID NO:3 (SEQ ID NO: 3) | ATGTTAGTGGGTTGAA (SEQ ID NO: 2139) |

Table 20: Breast cancer vs. benign breast conditions

| No: | Gene | Oligo: |
|---|---|---|
| 1 | SCGB3A1 (SEQ ID NO: 115) | GGCGTAGAAGGCGTTT (SEQ ID NO: 2140) |
| 2 | SCGB3A1(SEQ ID NO: 115) | GGTGTAGAAGGTGTTT (SEQ ID NO: 2141) |
| 3 | SCGB3A1 (SEQ ID NO: 115) | GGCGTAGAAGGCGTTT (SEQ ID NO: 2140) |
| 4 | SCGB3A1 (SEQ ID NO: 115) | GGTGTAGAAGGTGTTT (SEQ ID NO: 2141) |
| 5 | SCGB3A1 (SEQ ID NO: 115) | TAGTGTTCGACGTTGT (SEQ ID NO: 1475) |
| 6 | SCGB3A1 (SEQ ID NO: 115) | TAGTGTTTGATGTTGTT (SEQ ID NO: 1476) |
| 7 | ARH1/NOEY2 (SEQ ID NO: 97) | TAAAACGTTCGGTAGG (SEQ ID NO: 1485) |
| 8 | ARH1/NOEY2 (SEQ ID NO: 97) | TTTAAAATGTTTGGTAGG (SEQ ID NO: 1486) |
| 9 | ARH1/NOEY2 (SEQ ID NO: 97) | TGTATTCGTCGTTAGG (SEQ ID NO: 1487) |
| 10 | ARH1/NOEY2 (SEQ ID NO: 97) | AATGTATTTGTTGTTAGG (SEQ ID NO: 1488) |
| 11 | ARH1/NOEY2 (SEQ ID NO: 97) | TTAGACGGAGTTCGGA (SEQ ID NO: 1489) |
| 12 | ARH1/NOEY2 (SEQ ID NO: 97) | TAGATGGAGTTTGGAGA (SEQ ID NO: 1490) |
| 13 | ARH1/NOEY2 (SEQ ID NO: 97) | TAGACGTAGGCGTATT (SEQ ID NO: 1491) |
| 14 | ARH1/NOEY2 (SEQ ID NO: 97) | GGGTAGATGTAGGTGT (SEQ ID NO: 1492) |
| 15 | CCND2 (SEQ ID NO: 104) | TTAGGGTCGATCGTGT (SEQ ID NO: 1493) |
| 16 | CCND2 (SEQ ID NO: 104) | TAGGGTTGATTGTGTT (SEQ ID NO: 1494) |
| 17 | CCND2 (SEQ ID NO: 104) | TTATCGTAGTCGGTTT (SEQ ID NO: 1495) |
| 18 | CCND2 (SEQ ID NO: 104) | GATTTTATTGTAGTTGGT (SEQ ID NO: 1496) |
| 19 | CCND2 (SEQ ID NO: 104) | GAGTGAGGCGCGAAAT (SEQ ID NO: 1497) |
| 20 | CCND2 (SEQ ID NO: 104) | GAGTGAGGTGTGAAAT (SEQ ID NO: 1498) |
| 21 | CCND2 (SEQ ID NO: 104) | GAGTGAGGCGCGAAAT (SEQ ID NO: 1497) |
| 22 | CCND2 (SEQ ID NO: 104) | GAGTGAGGTGTGAAAT (SEQ ID NO: 1498) |
| 23 | CCND2 (SEQ ID NO: 104) | AAGGATCGAGCGTGGA (SEQ ID NO: 1499) |
| 24 | CCND2 (SEQ ID NO: 104) | AAGGATTGAGTGTGGA (SEQ ID NO: 1500) |
| 25 | CCND2 (SEQ ID NO: 104) | AAGGATCGAGCGTGGA (SEQ ID NO: 1499) |
| 26 | CCND2 (SEQ ID NO: 104) | AAGGATTGAGTGTGGA (SEQ ID NO: 1500) |
| 27 | CDKN I A (SEQ ID NO: 67) | ATTAGGGTCGCGTTGA (SEQ ID NO: 1501) |
| 28 | CDKN1A (SEQ ID NO: 67) | ATTAGGGTTGTGTTGA (SEQ ID NO: 1502) |
| 29 | CDKN1A (SEQ ID NO: 67) | ATTAGGGTCGCGTTGA (SEQ ID NO: 1501) |
| 30 | CDKN1A (SEQ ID NO: 67) | ATTAGGGTTGTGTTGA (SEQ ID NO: 1502) |
| 31 | CDKN2A (SEQ ID NO: 57) | GGCGTTGTTTAACGTAT (SEQ ID NO: 1503) |
| 32 | CDKN2A (SEQ ID NO: 57) | GGGTGTTGTTTAATGTA (SEQ ID NO: 1504) |
| 33 | DAPK1 (SEQ ID NO: 98) | TTTCGGAGATTCGGTT (SEQ ID NO: 1509) |
| 34 | DAPK1 (SEQ ID NO: 98) | TTTGGAGATTTGGTTTT (SEQ ID NO: 1510) |
| 35 | DAPK1 (SEQ ID NO: 98) | TTTTAGCGTCGGGGAG (SEQ ID NO: 1511) |
| 36 | DAPK1 (SEQ ID NO: 98) | TTTTAGTGTTGGGGAG (SEQ ID NO: 1512) |
| 37 | DAPK1 (SEQ ID NO: 98) | TTTTAGCGTCGGGGAG (SEQ ID NO: 1511) |

(continued)

| No: | Gene | Oligo: |
|-----|------|--------|
| 38 | DAPK1 (SEQ ID NO: 98) | TTTTAGTGTTGGGGAG (SEQ ID NO: 1512) |
| 39 | EYA4 (SEQ ID NO: 58) | GGTATAAAATCGTAAATTTT (SEQ ID NO: 1513) |
| 40 | EYA4 (SEQ ID NO: 58) | GGGTATAAAATTGTAAATTT (SEQ ID NO: 1514) |
| 41 | EYA4 (SEQ ID NO: 58) | TATGTAGTCGCGTAGT (SEQ ID NO: 1515) |
| 42 | EYA4 (SEQ ID NO: 58) | TTTATGTAGTTGTGTAGT (SEQ ID NO: 1516) |
| 43 | EYA4 (SEQ ID NO: 58) | GTTTAGATACGAAATGTT (SEQ ID NO: 1517) |
| 44 | EYA4 (SEQ ID NO: 58) | GTTTAGATATGAAATGTTAT (SEQ ID NO: 1518) |
| 45 | EYA4 (SEQ ID NO: 58) | AGTTTTGACGTCGTTT (SEQ ID NO: 1519) |
| 46 | EYA4 (SEQ ID NO: 58) | TTGATGTTGTTTTGGAA (SEQ ID NO: 1520) |
| 47 | FHIT (SEQ ID NO: 76) | GTTACGTTAGCGGGTT (SEQ ID NO: 1521) |
| 48 | FHIT (SEQ ID NO: 76) | GGTTATGTTAGTGGGT (SEQ ID NO: 1522) |
| 49 | GSTP1 (SEQ ID NO: 59) | GGCGATTTCGGGGATT (SEQ ID NO: 1525) |
| 50 | GSTP1 (SEQ ID NO: 59) | GGTGATTTTGGGGATTT (SEQ ID NO: 1526) |
| 51 | GSTP1 (SEQ ID NO: 59) | GACGTTCGGGGTGTAG (SEQ ID NO: 1527) |
| 52 | GSTP1 (SEQ ID NO: 59) | GATGTTTGGGGTGTAG (SEQ ID NO: 1528) |
| 53 | GSTP1 (SEQ ID NO: 59) | GACGTTCGGGGTGTAG (SEQ ID NO: 1527) |
| 54 | GSTP1 (SEQ ID NO: 59) | GATGTTTGGGGTGTAG (SEQ ID NO: 1528) |
| 55 | GSTP1 (SEQ ID NO: 59) | AGTTCGCGGGATTTTT (SEQ ID NO: 1529) |
| 56 | GSTP1 (SEQ ID NO: 59) | GGAGTTTGTGGGATTT (SEQ ID NO: 1530) |
| 57 | GSTP1 (SEQ ID NO: 59) | AGTTTTCGTTATTAGTGA (SEQ ID NO: 1531) |
| 58 | GSTP1 (SEQ ID NO: 59) | TAGTTTTTGTTATTAGTGA (SEQ ID NO: 1532) |
| 59 | HIC1 (SEQ ID NO: 85) | TATCGAAGTTTTCGGG (SEQ ID NO: 1533) |
| 60 | HIC1 (SEQ ID NO: 85) | TATTGAAGTTTTTGGGT (SEQ ID NO: 1534) |
| 61 | HIC1 (SEQ ID NO: 85) | TAGCGGTTATTTCGGT (SEQ ID NO: 1535) |
| 62 | HIC1 (SEQ ID NO: 85) | TTTAGTGGTTATTTTGGT (SEQ ID NO: 1536) |
| 63 | HIC1 (SEQ ID NO: 85) | TACGTTTTTCGTAGCGT (SEQ ID NO: 1537) |
| 64 | HIC1 (SEQ ID NO: 85) | ATTATGTTTTTTGTAGTGT (SEQ ID NO: 1538) |
| 65 | HIC1 (SEQ ID NO: 85) | TTCGGTTTTGTCGTATA (SEQ ID NO: 1539) |
| 66 | HIC1 (SEQ ID NO: 85) | TTTGGTTTTGTTGTATAG (SEQ ID NO: 1540) |
| 67 | SERPINB5 (SEQ ID NO: 68) | ATTGTCGTACGTATGT (SEQ ID NO: 1551) |
| 68 | SERPINB5 (SEQ ID NO: 68) | AGAGGATTGTTGTATGTA (SEQ ID NO: 1552) |
| 69 | SERPINB5 (SEQ ID NO: 68) | TTTTTTGTTCGAATATGT (SEQ ID NO: 1553) |
| 70 | SERPINB5 (SEQ ID NO: 68) | TTTGTTTGAATATGTTGG (SEQ ID NO: 1554) |
| 71 | TERT (SEQ ID NO: 92) | TATAACGAACGTCGTT (SEQ ID NO: 2142) |
| 72 | TERT (SEQ ID NO: 92) | AGGTATAATGAATGTTGT (SEQ ID NO: 2143) |
| 73 | TGFBR2 (SEQ ID NO: 93) | AAGACGGTTAGGTCGG (SEQ ID NO: 2144) |
| 74 | TGFBR2 (SEQ ID NO: 93) | AAGATGGTTAGGTTGG (SEQ ID NO: 2145) |
| 75 | TGFBR2 (SEQ ID NO: 93) | AAGACGGTTAGGTCGG (SEQ ID NO: 2144) |

(continued)

| No: | Gene | Oligo: |
|-----|------|--------|
| 76 | TGFBR2 (SEQ ID NO: 93) | AAGATGGTTAGGTTGG (SEQ ID NO: 2145) |
| 77 | THRB (SEQ ID NO: 106) | GGGCGGTTAAGTCGAG (SEQ ID NO: 1569) |
| 78 | THRB (SEQ ID NO: 106) | GGGTGGTTAAGTTGAG (SEQ ID NO: 1570) |
| 79 | THRB (SEQ ID NO: 106) | GGGCGGTTAAGTCGAG (SEQ ID NO: 1569) |
| 80 | THRB (SEQ ID NO: 106) | GGGTGGTTAAGTTGAG (SEQ ID NO: 1570) |
| 81 | TIMP3 (SEQ ID NO: 103) | TTATTAACGGAGGAAGG (SEQ ID NO: 1571) |
| 82 | TIMP3 (SEQ ID NO: 103) | TATTAATGGAGGAAGGG (SEQ ID NO: 1572) |
| 83 | TIMP3 (SEQ ID NO: 103) | TTCGTTATGTGTACGGAA (SEQ ID NO: 1573) |
| 84 | TIMP3 (SEQ ID NO: 103) | TTTGTTATGTGTATGGAA (SEQ ID NO: 1574) |
| 85 | TIMP3 (SEQ ID NO: 103) | TTCGTTATGTGTACGGAA (SEQ ID NO: 1573) |
| 86 | TIMP3 (SEQ ID NO: 103) | TTTGTTATGTGTATGGAA (SEQ ID NO: 1574) |
| 87 | TIMP3 (SEQ ID NO: 103) | GAGTATTTCGAGTTTGT (SEQ ID NO: 1575) |
| 88 | TIMP3 (SEQ ID NO: 103) | AGTATTTTGAGTTTGTATT (SEQ ID NO: 1576) |
| 89 | TIMP3 (SEQ ID NO: 103) | TAAGCGTTAATCGAGT (SEQ ID NO: 1577) |
| 90 | TIMP3 (SEQ ID NO: 103) | TAGGTAAGTGTTAATTGA (SEQ ID NO: 1578) |
| 91 | TP73 (SEQ ID NO: 86) | TAGGATTCGCGTTTTT (SEQ ID NO: 1579) |
| 92 | TP73 (SEQ ID NO: 86) | GGTAGGATTTGTGTTTT (SEQ ID NO: 1580) |
| 93 | CDH13 (SEQ ID NO: 70) | TAGTCGCGTGTATGAA (SEQ ID NO: 1585) |
| 94 | CDH13 (SEQ ID NO: 70) | TGTAGTTGTGTGTATGA (SEQ ID NO: 1586) |
| 95 | TMS1/ASC (SEQ ID NO: 84) | TTCGTTTCGGAGTCGA (SEQ ID NO: 1591) |
| 96 | TMS1/ASC (SEQ ID NO: 84) | TTTGTTTTGGAGTTGAT (SEQ ID NO: 1592) |
| 97 | APAF1 (SEQ ID NO: 82) | GTGTCGTAGCGGTATT (SEQ ID NO: 1593) |
| 98 | APAF1 (SEQ ID NO: 82) | GGTGTTGTAGTGGTAT (SEQ ID NO: 1594) |
| 99 | APAF1 (SEQ ID NO: 82) | AGTAGCGTCGGGTTTT (SEQ ID NO: 1595) |
| 100 | APAF1 (SEQ ID NO: 82) | GAGTAGTGTTGGGTTT (SEQ ID NO: 1596) |
| 101 | SYK (SEQ ID NO: 60) | GAAGTTATCGCGTTGG (SEQ ID NO: 1597) |
| 102 | SYK (SEQ ID NO: 60) | AGAAGTTATTGTGTTGG (SEQ ID NO: 1598) |
| 103 | SYK (SEQ ID NO: 60) | GATCGATGCGGTTTAT (SEQ ID NO: 1599) |
| 104 | SYK (SEQ ID NO: 60) | GGGATTGATGTGGTTT (SEQ ID NO: 1600) |
| 105 | SYK (SEQ ID NO: 60) | TTATTCGGTCGGGATT (SEQ ID NO: 1603) |
| 106 | SYK (SEQ ID NO: 60) | TTTATTTGGTTGGGATT (SEQ ID NO: 1604) |
| 107 | FABP3 (SEQ ID NO: 77) | TAAAGCGGTAGTTCGG (SEQ ID NO: 1609) |
| 108 | FABP3 (SEQ ID NO: 77) | AAGTGGTAGTTTGGGT (SEQ ID NO: 1610) |
| 109 | FABP3 (SEQ ID NO: 77) | TATTGGCGTTGACGTA (SEQ ID NO: 1611) |
| 110 | FABP3 (SEQ ID NO: 77) | TGGTGTTGATGTAGGT (SEQ ID NO: 1612) |
| 111 | RASSF1A (SEQ ID NO: 90) | TACGGGTATTTTCGCGT (SEQ ID NO: 1613) |
| 112 | RASSF1A (SEQ ID NO: 90) | ATATGGGTATTTTTGTGT (SEQ ID NO: 1614) |
| 113 | RASSF1A (SEQ ID NO: 90) | AGAGCGCGTTTAGTTT (SEQ ID NO: 1615) |

(continued)

| No: | Gene | Oligo: |
|---|---|---|
| 114 | RASSF1A (SEQ ID NO: 90) | GAGAGTGTGTTTAGTTT (SEQ ID NO: 1616) |
| 115 | RASSF1A (SEQ ID NO: 90) | AGTAAATCGGATTAGGA (SEQ ID NO: 1617) |
| 116 | RASSF1A (SEQ ID NO: 90) | AGTAAATTGGATTAGGAG (SEQ ID NO: 1618) |
| 117 | TWIST (SEQ ID NO: 100) | AGTAAAGGCGTTGCGT (SEQ ID NO: 1619) |
| 118 | TWIST (SEQ ID NO: 100) | AGTAAAGGTGTTGTGT (SEQ ID NO: 1620) |
| 119 | TWIST (SEQ ID NO: 100) | AGTAAAGGCGTTGCGT (SEQ ID NO: 1619) |
| 120 | TWIST (SEQ ID NO: 100) | AGTAAAGGTGTTGTGT (SEQ ID NO: 1620) |
| 121 | TWIST (SEQ ID NO: 100) | TATTTTTCGAGGCGTA (SEQ ID NO: 1621) |
| 122 | TWIST (SEQ ID NO: 100) | TTTTGAGGTGTAGTTTT (SEQ ID NO: 1622) |
| 123 | TWIST (SEQ ID NO: 100) | ATTGGGTCGTTGTAGA (SEQ ID NO: 1623) |
| 124 | TWIST (SEQ ID NO: 100) | ATTGGGTTGTTGTAGA (SEQ ID NO: 1624) |
| 125 | TWIST (SEQ ID NO: 100) | ATTGGGTCGTTGTAGA (SEQ ID NO: 1623) |
| 126 | TWIST (SEQ ID NO: 100) | ATTGGGTTGTTGTAGA (SEQ ID NO: 1624) |
| 127 | TWIST (SEQ ID NO: 100) | TAGGTCGGGACGTAAA (SEQ ID NO: 1625) |
| 128 | TWIST (SEQ ID NO: 100) | AGTAGGTTGGGATGTA (SEQ ID NO: 1626) |
| 129 | ESR2 (SEQ ID NO: 91) | ATAAGCGATTTAACGAT (SEQ ID NO: 1629) |
| 130 | ESR2 (SEQ ID NO: 91) | AAGTGATTTAATGATAAGT (SEQ ID NO: 1630) |
| 131 | PLAU (SEQ ID NO: 62) | TATTTGTCGCGTTGAT (SEQ ID NO: 1633) |
| 132 | PLAU (SEQ ID NO: 62) | ATTTGTTGTGTTGATGA (SEQ ID NO: 1634) |
| 133 | PLAU (SEQ ID NO: 62) | TGTAATTCGGGGATTT (SEQ ID NO: 1635) |
| 134 | PLAU (SEQ ID NO: 62) | TTGTAATTTGGGGATTT (SEQ ID NO: 1636) |
| 135 | PLAU (SEQ ID NO: 62) | TTGGAGATCGCGTTTT (SEQ ID NO: 1637) |
| 136 | PLAU (SEQ ID NO: 62) | TTGGAGATTGTGTTTTT (SEQ ID NO: 1638) |
| 137 | PLAU (SEQ ID NO: 62) | GAGCGTTGCGGAAGTA (SEQ ID NO: 1639) |
| 138 | PLAU (SEQ ID NO: 62) | GAGTGTTGTGGAAGTA (SEQ ID NO: 1640) |
| 139 | PLAU (SEQ ID NO: 62) | GAGCGTTGCGGAAGTA (SEQ ID NO: 1639) |
| 140 | PLAU (SEQ ID NO: 62) | GAGTGTTGTGGAAGTA (SEQ ID NO: 1640) |
| 141 | STAT1 (SEQ ID NO: 109) | GTTATTTTCGAGAGTTG (SEQ ID NO: 1641) |
| 142 | STAT1 (SEQ ID NO: 109) | GTTATTTTTGAGAGTTGT (SEQ ID NO: 1642) |
| 143 | LOT1 (SEQ ID NO: 95) | ATGGGTACGTTTAAGG (SEQ ID NO: 1649) |
| 144 | LOT1 (SEQ ID NO: 95) | TGGGTATGTTTAAGGG (SEQ ID NO: 1650) |
| 145 | GJB2 (SEQ ID NO: 111) | GGATTTCGTCGGTATT (SEQ ID NO: 1667) |
| 146 | GJB2 (SEQ ID NO: 111) | GGGGATTTTGTTGGTA (SEQ ID NO: 1668) |
| 147 | PRDM2 (SEQ ID NO: 114) | TGTAGAGACGACGATT (SEQ ID NO: 1675) |
| 148 | PRDM2 (SEQ ID NO: 114) | ATTGTAGAGATGATGATT (SEQ ID NO: 1676) |
| 149 | PRDM2 (SEQ ID NO: 114) | AGAGCGCGGTAGTAGT (SEQ ID NO: 1677) |
| 150 | PRDM2 (SEQ ID NO: 114) | TGAGAGTGTGGTAGTA (SEQ ID NO: 1678) |
| 151 | PRDM2 (SEQ ID NO: 114) | TGTTCGCGATGTTTTA (SEQ ID NO: 1679) |

(continued)

| No: | Gene | Oligo: |
|-----|------|--------|
| 152 | PRDM2 (SEQ ID NO: 114) | TGTTTGTGATGTTTTAGT (SEQ ID NO: 1680) |
| 153 | ALX4 (SEQ ID NO: 64) | AAGTCGATCGTTTTGT (SEQ ID NO: 1683) |
| 154 | ALX4 (SEQ ID NO: 64) | TGGAAGTTGATTGTTTT (SEQ ID NO: 1684) |
| 155 | ALX4 (SEQ ID NO: 64) | ATATCGTTCGGGGGAA (SEQ ID NO: 2146) |
| 156 | ALX4 (SEQ ID NO: 64) | ATATCGTTCGGGGGAA (SEQ ID NO: 2146) |
| 157 | ALX4 (SEQ ID NO: 64) | TATTGCGAGGATTCGG (SEQ ID NO: 1685) |
| 158 | ALX4 (SEQ ID NO: 64) | ATTGTGAGGATTTGGT (SEQ ID NO: 1686) |
| 159 | ALX4 (SEQ ID NO: 64) | TTCGTAGCGTAGGGTT (SEQ ID NO: 1687) |
| 160 | ALX4 (SEQ ID NO: 64) | TTTGTAGTGTAGGGTTT (SEQ ID NO: 1688) |
| 161 | IGFBP7 (SEQ ID NO: 94) | ATTTTTTCGCGGGTAT (SEQ ID NO: 1710) |
| 162 | IGFBP7 (SEQ ID NO: 94) | TTTTTGTGGGTATTTTAG (SEQ ID NO: 1711) |
| 163 | IGFBP7 (SEQ ID NO: 94) | GGTATATTCGACGGGG (SEQ ID NO: 1712) |
| 164 | IGFBP7 (SEQ ID NO: 94) | GGGTATATTTGATGGGG (SEQ ID NO: 1713) |
| 165 | IGFBP7 (SEQ ID NO: 94) | GGTACGAGCGTTTTTT (SEQ ID NO: 1714) |
| 166 | IGFBP7 (SEQ ID NO: 94) | TGGGTATGAGTGTTTT (SEQ ID NO: 1715) |
| 167 | IGFBP7 (SEQ ID NO: 94) | AAAGCGTATTTAATTCGT (SEQ ID NO: 1716) |
| 168 | IGFBP7 (SEQ ID NO: 94) | AGTGTATTTAATTGTGTT (SEQ ID NO: 1717) |
| 169 | NME1 (SEQ ID NO: 107) | AGTCGAGATTGCGTTA (SEQ ID NO: 2147) |
| 170 | NME (SEQ ID NO: 107) | AGTTGAGATTGTGTTAG (SEQ ID NO: 2148) |
| 171 | NME1 (SEQ ID NO: 107) | ATCGTTTGAATTCGGGA (SEQ ID NO: 2149) |
| 172 | NME1 (SEQ ID NO: 107) | ATTGTTTGAATTTGGGA (SEQ ID NO: 2150) |
| 173 | NME1 (SEQ ID NO: 107) | ATCGTTTGAATTCGGGA (SEQ ID NO: 2149) |
| 174 | NME1 (SEQ ID NO: 107) | ATTGTTTGAATTTGGGA (SEQ ID NO: 2150) |
| 175 | NME1 (SEQ ID NO: 107) | AATTCGAGATTAGTTCGG (SEQ ID NO: 1724) |
| 176 | NME1 (SEQ ID NO: 107) | AATTTGAGATTAGTTTGG (SEQ ID NO: 1725) |
| 177 | NME1 (SEQ ID NO: 107) | AATTCGAGATTAGTTCGG (SEQ ID NO: 1724) |
| 178 | NME1 (SEQ ID NO: 107) | AATTTGAGATTAGTTTGG (SEQ ID NO: 1725) |
| 179 | NME1 (SEQ ID NO: 107) | TTTTAGTACGTTGGAAA (SEQ ID NO: 2151) |
| 180 | NME1 (SEQ ID NO: 107) | TTAGTATGTTGGAAAGTA (SEQ ID NO: 2152) |
| 181 | THBS1 (SEQ ID NO: 81) | TAAAGGGGCGTTCGTA (SEQ ID NO: 1726) |
| 182 | THBS1 (SEQ ID NO:81) | AAGGGGTGTTTGTATT (SEQ ID NO: 1727) |
| 183 | THBS1 (SEQ ID NO: 81) | GGTTAGTTCGGGCGTA (SEQ ID NO: 1728) |
| 184 | THBS1 (SEQ ID NO: 81) | GGTTAGTTTGGGTGTA (SEQ ID NO: 1729) |
| 185 | THBS1 (SEQ ID NO: 81) | GGTTAGTTCGGGCGTA (SEQ ID NO: 1728) |
| 186 | THBS (SEQ ID NO: 81) | GGTTAGTTTGGGTGTA (SEQ ID NO: 1729) |
| 187 | THBS1 (SEQ ID NO: 81) | TTGTGCGTTCGGAGTA (SEQ ID NO: 1730) |
| 188 | THBS1 (SEQ ID NO: 81) | TGTGTTTGGAGTAGAG (SEQ ID NO: 1731) |
| 189 | IL6 (SEQ ID NO: 99) | TTCGGTTATACGTAGG (SEQ ID NO: 1736) |

(continued)

| No: | Gene | Oligo: |
|---|---|---|
| 190 | IL6 (SEQ ID NO: 99) | TTTTGGTTATATGTAGGG (SEQ ID NO: 1737) |
| 191 | IL6 (SEQ ID NO: 99) | AGTTTAGTCGGTTTCGT (SEQ ID NO: 1738) |
| 192 | IL6 (SEQ ID NO: 99) | AGTTTAGTTGGTTTTGT (SEQ ID NO: 1739) |
| 193 | IL6 (SEQ ID NO: 99) | AGTTTAGTCGGTTTCGT (SEQ ID NO: 1738) |
| 194 | IL6 (SEQ ID NO: 99) | AGTTTAGTTGGTTTTGT (SEQ ID NO: 1739) |
| 195 | HOXA5 (SEQ ID NO: 78) | TAGTTTTCGGTCGGAA (SEQ ID NO: 1748) |
| 196 | HOXA5 (SEQ ID NO: 78) | TAGTTTTTGGTTGGAAG (SEQ ID NO: 1749) |
| 197 | HOXA5 (SEQ ID NO: 78) | ATCGGTAGTTGACGGTT (SEQ ID NO: 1752) |
| 198 | HOXA5 (SEQ ID NO: 78) | ATTGGTAGTTGATGGTT (SEQ ID NO: 1753) |
| 199 | HOXA5 (SEQ ID NO: 78) | ATCGGTAGTTGACGGTT (SEQ ID NO: 1752) |
| 200 | HOXA5 (SEQ ID NO: 78) | ATTGGTAGTTGATGGTT (SEQ ID NO: 1753) |
| 201 | GPC3 (SEQ ID NO: 118) | AATAGTCGCGTTTAGG (SEQ ID NO: 1760) |
| 202 | GPC3 (SEQ ID NO: 118) | TAGTTGTGTTTAGGGAT (SEQ ID NO: 1761) |
| 203 | CLDN7 (SEQ ID NO: 87) | TTACGTTAAGTCGGGT (SEQ ID NO: 1764) |
| 204 | CLDN7 (SEQ ID NO: 87) | AGTTATGTTAAGTTGGG (SEQ ID NO: 1765) |
| 205 | SLIT2 (SEQ ID NO: 112) | ATTTCGTCGTAGTTTG (SEQ ID NO: 1774) |
| 206 | SLIT2 (SEQ ID NO: 112) | TTTTGTTGTAGTTTGGA (SEQ ID NO: 1775) |
| 207 | SLIT2 (SEQ ID NO: 112) | TAGCGGGTTCGTAGTA (SEQ ID NO: 1776) |
| 208 | SLIT2 (SEQ ID NO: 112) | TTAGTGGGTTTGTAGTA (SEQ ID NO: 1777) |
| 209 | SLIT2 (SEQ ID NO: 112) | AAGGCGCGGAAGTTTA (SEQ ID NO: 1778) |
| 210 | SLIT2 (SEQ ID NO: 112) | AAGGTGTGGAAGTTTA (SEQ ID NO: 1779) |
| 211 | SLIT2 (SEQ ID NO: 112) | AAGGCGCGGAAGTTTA (SEQ ID NO: 1778) |
| 212 | SLIT2 (SEQ ID NO: 112) | AAGGTGTGGAAGTTTA (SEQ ID NO: 1779) |
| 213 | IGSF4 (SEQ ID NO: 74) | AGGTAGATCGAGGAGG (SEQ ID NO: 1780) |
| 214 | IGSF4 (SEQ ID NO: 74) | AGGTAGATTGAGGAGG (SEQ ID NO: 1781) |
| 215 | IGSF4 (SEQ ID NO: 74) | AGGTAGATCGAGGAGG (SEQ ID NO: 1780) |
| 216 | IGSF4 (SEQ ID NO: 74) | AGGTAGATTGAGGAGG (SEQ ID NO: 1781) |
| 217 | IGSF4 (SEQ ID NO: 74) | TAGTCGTAGAGTCGGG (SEQ ID NO: 1782) |
| 218 | IGSF4 (SEQ ID NO: 74) | GTTGTAGAGTTGGGTT (SEQ ID NO: 1783) |
| 219 | MCT1 (SEQ ID NO: 101) | AGTTAGTCGCGTTTTA (SEQ ID NO: 1788) |
| 220 | MCT1 (SEQ ID NO: 101) | AGAGTTAGTTGTGTTTTA (SEQ ID NO: 1789) |
| 221 | SEQ ID NO:6 (SEQ ID NO: 6) | AAGTTTATCGGCGTTT (SEQ ID NO: 1792) |
| 222 | SEQ ID NO:6 (SEQ ID NO: 6) | AGAAGTTTATTGGTGTTT (SEQ ID NO: 1793) |
| 223 | SEQ ID NO:6 (SEQ ID NO: 6) | ATTTCGGAATTTAAGCGT (SEQ ID NO: 1794) |
| 224 | SEQ ID NO:6 (SEQ ID NO: 6) | TTTTGGAATTTAAGTGTT (SEQ ID NO: 1795) |
| 225 | SEQ ID NO:6 (SEQ ID NO: 6) | TAATTTCGGACGCGGA (SEQ ID NO: 1796) |
| 226 | SEQ ID NO:6 (SEQ ID NO: 6) | TTTTGGATGTGGAGGA (SEQ ID NO: 1797) |
| 227 | SEQ ID NO:6 (SEQ ID NO: 6) | TTACGGTGAAGGCGGA (SEQ ID NO: 1798) |

(continued)

| No: | Gene | Oligo: |
|---|---|---|
| 228 | SEQ ID NO:6 (SEQ ID NO: 6) | TTATGGTGAAGGTGGA (SEQ ID NO: 1799) |
| 229 | SEQ ID NO:6 (SEQ ID NO: 6) | TTACGGTGAAGGCGGA (SEQ ID NO: 1798) |
| 230 | SEQ ID NO:6 (SEQ ID NO: 6) | TTATGGTGAAGGTGGA (SEQ ID NO: 1799) |
| 231 | SEQ ID NO:6 (SEQ ID NO: 6) | TTTCGGTTTTCGTTAAT (SEQ ID NO: 1800) |
| 232 | SEQ ID NO:6 (SEQ ID NO: 6) | TTTGGTTTTTGTTAATTTAG (SEQ ID NO: 1801) |
| 233 | SEQ ID NO:6 (SEQ ID NO: 6) | TGTGCGAAGTTAACGT (SEQ ID NO: 1802) |
| 234 | SEQ ID NO:6 (SEQ ID NO: 6) | TTGTGTGAAGTTAATGT (SEQ ID NO: 1803) |
| 235 | SEQ ID NO:8 (SEQ ID NO: 8) | ATAGGGCGGGATTTTA (SEQ ID NO: 2153) |
| 236 | SEQ ID NO:8 (SEQ ID NO: 8) | GATAGGGTGGGATTTT (SEQ ID NO: 2154) |
| 237 | SEQ ID NO:8 (SEQ ID NO: 8) | ATAAAGCGGGGTTTTA (SEQ ID NO: 1804) |
| 238 | SEQ ID NO:8 (SEQ ID NO: 8) | GGATAAAGTGGGGTTT (SEQ ID NO: 1805) |
| 239 | SEQ ID NO:8 (SEQ ID NO: 8) | AGGAGGCGAGAAATTT (SEQ ID NO: 1806) |
| 240 | SEQ ID NO:8 (SEQ ID NO: 8) | GAGGAGGTGAGAAATT (SEQ ID NO: 1807) |
| 241 | SEQ ID NO:8 (SEQ ID NO: 8) | AGAAATTTCGGGGTAG (SEQ ID NO: 1808) |
| 242 | SEQ ID NO:8 (SEQ ID NO: 8) | GAAATTTTGGGGTAGTA (SEQ ID NO: 1809) |
| 243 | SEQ ID NO:8 (SEQ ID NO: 8) | GGTAGTATCGTTTATAGA (SEQ ID NO: 1810) |
| 244 | SEQ ID NO:8 (SEQ ID NO: 8) | GGGGTAGTATTGTTTATA (SEQ ID NO: 1811) |
| 245 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGTGTATCGTTTTTCGG (SEQ ID NO: 1812) |
| 246 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAGTGTATTGTTTTTTGG (SEQ ID NO: 1813) |
| 247 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TGCGTATCGTTAGTTA (SEQ ID NO: 1814) |
| 248 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AGGTTGTGTATTGTTAG (SEQ ID NO: 1815) |
| 249 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | ATTATTTCGGCGGTGA (SEQ ID NO: 1816) |
| 250 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GATTATTTTGGTGGTGA (SEQ ID NO: 1817) |
| 251 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | TAAGTCGCGTAAGGAG (SEQ ID NO: 1818) |
| 252 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | AAGTTGTGTAAGGAGTA (SEQ ID NO: 1819) |

(continued)

| No: | Gene | Oligo: |
|---|---|---|
| 253 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATCGCGAGTTTGGA (SEQ ID NO: 1820) |
| 254 | PROSTAGLANDIN E2 RECEPTOR, EP4 SUBTYPE (PROSTANOID EP4 RECEPTOR) (PGE RECEPTOR, EP4 SUBTYPE) (SEQ ID NO: 10) | GTATTGTGAGTTTGGAG (SEQ ID NO: 1821) |
| 255 | SEQ ID NO:51 (SEQ ID NO: 51) | GTCGGGGTCGATTCGA (SEQ ID NO: 1822) |
| 256 | SEQ ID NO:51 (SEQ ID NO: 51) | GTTGGGGTTGATTTGAT (SEQ ID NO: 1823) |
| 257 | SEQ ID NO:51 (SEQ ID NO: 51) | AGGATTCGTTTGCGTT (SEQ ID NO: 1824) |
| 258 | SEQ ID NO:51 (SEQ ID NO: 51) | AAGGATTTGTTTGTGTT (SEQ ID NO: 1825) |
| 259 | MGC34831 (SEQ ID NO: 52) | ATTAGCGTTTGGCGTT (SEQ ID NO: 1826) |
| 260 | MGC34831 (SEQ ID NO: 52) | AATTAGTGTTTGGTGTT (SEQ ID NO: 1827) |
| 261 | MGC34831 (SEQ ID NO: 52) | GTTTAGCGACGGTCGT (SEQ ID NO: 1828) |
| 262 | MGC34831 (SEQ ID NO: 52) | TGTTTAGTGATGGTTGT (SEQ ID NO: 1829) |
| 263 | SEQ ID NO:54 (SEQ ID NO: 54) | TGTGTAGCGGCGATTA (SEQ ID NO: 1830) |
| 264 | SEQ ID NO:54 (SEQ ID NO: 54) | GTGTGTAGTGGTGATT (SEQ ID NO: 1831) |
| 265 | SEQ ID NO:54 (SEQ ID NO: 54) | ATTAGCGTTTGGTCGG (SEQ ID NO: 1832) |
| 266 | SEQ ID NO:54 (SEQ ID NO: 54) | ATTAGTGTTTGGTTGGG (SEQ ID NO: 1833) |
| 267 | PDLIM1 (SEQ ID NO: 55) | TAGGTCGCGTAGTCGT (SEQ ID NO: 1834) |
| 268 | PDLIM1 (SEQ ID NO: 55) | TTAGGTTGTGTAGTTGT (SEQ ID NO: 1835) |
| 269 | DKK3 (SEQ ID NO: 24) | ATTCGTTTTAGTTCGAG (SEQ ID NO: 1842) |
| 270 | DKK3 (SEQ ID NO: 24) | ATTTGTTTTAGTTTGAGT (SEQ ID NO: 1843) |
| 271 | DKK3 (SEQ ID NO: 24) | TTCGATCGTTTTAGGA (SEQ ID NO: 1844) |
| 272 | DKK3 (SEQ ID NO: 24) | AGTTTTGATTGTTTTAGG (SEQ ID NO: 1845) |
| 273 | DKK3 (SEQ ID NO: 24) | ATTCGTTCGGAGACGG (SEQ ID NO: 1846) |
| 274 | DKK3 (SEQ ID NO: 24) | TTTGTTTGGAGATGGG (SEQ ID NO: 1847) |
| 275 | DKK3 (SEQ ID NO: 24) | GAAAAGACGCGATTTT (SEQ ID NO: 1848) |
| 276 | DKK3 (SEQ ID NO: 24) | GAAAAGATGTGATTTTATT (SEQ ID NO: 1849) |
| 277 | SEQ ID NO:28 (SEQ ID NO: 28) | TATCGACGTTTTTGGT (SEQ ID NO: 1850) |
| 278 | SEQ ID NO:28 (SEQ ID NO: 28) | TATTGATGTTTTTGGTTT (SEQ ID NO: 1851) |
| 279 | SEQ ID NO:29 (SEQ ID NO: 29) | TTGATGCGGAGTTCGA (SEQ ID NO: 1852) |
| 280 | SEQ ID NO:29 (SEQ ID NO: 29) | TTGATGTGGAGTTTGA (SEQ ID NO: 1853) |
| 281 | SEQ ID NO:29 (SEQ ID NO: 29) | TTGATGCGGAGTTCGA (SEQ ID NO: 1852) |
| 282 | SEQ ID NO:29 (SEQ ID NO: 29) | TTGATGTGGAGTTTGA (SEQ ID NO: 1853) |
| 283 | SEQ ID NO:29 (SEQ ID NO: 29) | TTCGAAGCGTGTATTA (SEQ ID NO: 2155) |
| 284 | SEQ ID NO:29 (SEQ ID NO: 29) | GGGTTTGAAGTGTGTA (SEQ ID NO: 2156) |
| 285 | SEQ ID NO:29 (SEQ ID NO: 29) | TTTTCGCGTTTGCGAA (SEQ ID NO: 2157) |
| 286 | SEQ ID NO:29 (SEQ ID NO: 29) | TTTGTGTTTGTGAAAGG (SEQ ID NO: 2158) |
| 287 | SEQ ID NO:29 (SEQ ID NO: 29) | TAGGAACGGTAGGCGG (SEQ ID NO: 1854) |

(continued)

| No: | Gene | Oligo: |
|---|---|---|
| 288 | SEQ ID NO:29 (SEQ ID NO: 29) | TAGGAATGGTAGGTGG (SEQ ID NO: 1855) |
| 289 | SEQ ID NO:29 (SEQ ID NO: 29) | TAGGAACGGTAGGCGG (SEQ ID NO: 1854) |
| 290 | SEQ ID NO:29 (SEQ ID NO: 29) | TAGGAATGGTAGGTGG (SEQ ID NO: 1855) |
| 291 | SEQ ID NO:29 (SEQ ID NO: 29) | GTCGGAGGCGTTTGAG (SEQ ID NO: 1856) |
| 292 | SEQ ID NO:29 (SEQ ID NO: 29) | GTTGGAGGTGTTTGAGA (SEQ ID NO: 1857) |
| 293 | ARL7 (SEQ ID NO: 30) | TAGATTTCGTAGTTTTTTA (SEQ ID NO: 1858) |
| 294 | ARL7 (SEQ ID NO: 30) | TAGATTTTGTAGTTTTTTAA (SEQ ID NO: 1859) |
| 295 | ARL7 (SEQ ID NO: 30) | TGGGTACGTTTACGGT (SEQ ID NO: 1860) |
| 296 | ARL7 (SEQ ID NO: 30) | TGGGTATGTTTATGGTT (SEQ ID NO: 1861) |
| 297 | ARL7 (SEQ ID NO: 30) | GAAGAAATCGTTTTTGT (SEQ ID NO: 1862) |
| 298 | ARL7 (SEQ ID NO: 30) | GAAGAAATTGTTTTTGTT (SEQ ID NO: 1863) |
| 299 | ARL7 (SEQ ID NO: 30) | TAGTAGGATCGGTTTTT (SEQ ID NO: 1864) |
| 300 | ARL7 (SEQ ID NO: 30) | ATAGTAGGATTGGTTTTT (SEQ ID NO: 1865) |
| 301 | SEQ ID NO:31 (SEQ ID NO: 31) | AACGTTGCGTTGGGTA (SEQ ID NO: 1866) |
| 302 | SEQ ID NO:31 (SEQ ID NO: 31) | AATGTTGTGTTGGGTAA (SEQ ID NO: 1867) |
| 303 | SEQ ID NO:31 (SEQ ID NO: 31) | TAGCGTTTCGTGGTTA (SEQ ID NO: 1868) |
| 304 | SEQ ID NO:31 (SEQ ID NO: 31) | GTAGTGTTTTGTGGTTA (SEQ ID NO: 1869) |
| 305 | THH (SEQ ID NO: 32) | TTCGGAAGAAAAGCGAAT (SEQ ID NO: 1870) |
| 306 | THH (SEQ ID NO: 32) | TTTGGAAGAAAAGTGAAT (SEQ ID NO: 1871) |
| 307 | THH (SEQ ID NO: 32) | TTCGGAAGAAAAGCGAAT (SEQ ID NO: 1870) |
| 308 | THH (SEQ ID NO: 32) | TTTGGAAGAAAAGTGAAT (SEQ ID NO: 1871) |
| 309 | THH (SEQ ID NO: 32) | GTTCGTTGGCGTAAAT (SEQ ID NO: 1872) |
| 310 | THH (SEQ ID NO: 32) | GGTTTGTTGGTGTAAAT (SEQ ID NO: 1873) |
| 311 | (SEQ ID NO: 36) | TTCGTTGGAGATCGTAA (SEQ ID NO: 2159) |
| 312 | (SEQ ID NO: 36) | GTTTGTTGGAGATTGTA (SEQ ID NO: 2160) |
| 313 | (SEQ ID NO: 36) | GGACGTTGCGATTGTA (SEQ ID NO: 2161) |
| 314 | (SEQ ID NO: 36) | GATGTTGTGATTGTAGT (SEQ ID NO: 2162) |
| 315 | SENP3 (SEQ ID NO: 39) | TATTCGGATCGGGTTT (SEQ ID NO: 1876) |
| 316 | SENP3 (SEQ ID NO: 39) | TTTATTTGGATTGGGTT (SEQ ID NO: 1877) |
| 317 | SENP3 (SEQ ID NO: 39) | TAGTCGAAAGTAGGACGT (SEQ ID NO: 1878) |
| 318 | SENP3 (SEQ ID NO: 39) | TAGTTGAAAGTAGGATGT (SEQ ID NO: 1879) |
| 319 | SENP3 (SEQ ID NO: 39) | TAGTCGAAAGTAGGACGT (SEQ ID NO: 1878) |
| 320 | SENP3 (SEQ ID NO: 39) | TAGTTGAAAGTAGGATGT (SEQ ID NO: 1879) |
| 321 | SENP3 (SEQ ID NO: 39) | AGGACGTTTTTGATCGG (SEQ ID NO: 1880) |
| 322 | SENP3 (SEQ ID NO: 39) | AGGATGTTTTTGATTGG (SEQ ID NO: 1881) |
| 323 | SENP3 (SEQ ID NO: 39) | AGGACGTTTTTGATCGG (SEQ ID NO: 1880) |
| 324 | SENP3 (SEQ ID NO: 39) | AGGATGTTTTTGATTGG (SEQ ID NO: 1881) |
| 325 | SENP3 (SEQ ID NO: 39) | AGTTATCGTTAGGAGGG (SEQ ID NO: 1882) |

(continued)

| No: | Gene | Oligo: |
|---|---|---|
| 326 | SENP3 (SEQ ID NO: 39) | AGTTATTGTTAGGAGGG (SEQ ID NO: 1883) |
| 327 | SENP3 (SEQ ID NO: 39) | AGTTATCGTTAGGAGGG (SEQ ID NO: 1882) |
| 328 | SENP3 (SEQ ID NO: 39) | AGTTATTGTTAGGAGGG (SEQ ID NO: 1883) |
| 329 | SEQ ID NO:42 (SEQ ID NO: 42) | GAGTCGGGTTGCGATG (SEQ ID NO: 1884) |
| 330 | SEQ ID NO:42 (SEQ ID NO: 42) | GGAGTTGGGTTGTGAT (SEQ ID NO: 1885) |
| 331 | SEQ ID NO:42 (SEQ ID NO: 42) | ATGGGTTGCGATTTTTA (SEQ ID NO: 1886) |
| 332 | SEQ ID NO:42 (SEQ ID NO: 42) | ATGGGTTGTGATTTTTA (SEQ ID NO: 1887) |
| 333 | SEQ ID NO:42 (SEQ ID NO: 42) | ATGGGTTGCGATTTTTA (SEQ ID NO: 1886) |
| 334 | SEQ ID NO:42 (SEQ ID NO: 42) | ATGGGTTGTGATTTTTA (SEQ ID NO: 1887) |
| 335 | (SEQ ID NO: 117) | TAGCGGTTTTTTAGCGTA (SEQ ID NO: 1888) |
| 336 | (SEQ ID NO: 117) | AGTGGTTTTTTAGTGTAT (SEQ ID NO: 1889) |
| 337 | (SEQ ID NO: 117) | AGATGCGCGGGTAGAT (SEQ ID NO: 1890) |
| 338 | (SEQ ID NO: 117) | AGATGTGTGGGTAGAT (SEQ ID NO: 1891) |
| 339 | (SEQ ID NO: 117) | AGATGCGCGGGTAGAT (SEQ ID NO: 1890) |
| 340 | (SEQ ID NO: 117) | AGATGTGTGGGTAGAT (SEQ ID NO: 1891) |
| 341 | (SEQ ID NO: 117) | AGATAGTTCGTATTCGT (SEQ ID NO: 1892) |
| 342 | (SEQ ID NO: 117) | GTAGATAGTTTGTATTTGT (SEQ ID NO: 1893) |
| 343 | (SEQ ID NO: 117) | TTTGTTCGTAACGTTT (SEQ ID NO: 1894) |
| 344 | (SEQ ID NO: 117) | TGTTTGTAATGTTTAGAG (SEQ ID NO: 1895) |
| 345 | 060279 (SEQ ID NO: 47) | TACGTTTTTTCGGATTA (SEQ ID NO: 1898) |
| 346 | 060279 (SEQ ID NO: 47) | TATGTTTTTTTGGATTAAG (SEQ ID NO: 1899) |
| 347 | 060279 (SEQ ID NO: 47) | GGACGGCGGAAAATTA (SEQ ID NO: 1902) |
| 348 | 060279 (SEQ ID NO: 47) | AGGGATGGTGGAAAAT (SEQ ID NO: 1903) |
| 349 | SEQ ID NO:48 (SEQ ID NO: 48) | TATTTGGCGATTCGGA (SEQ ID NO: 1904) |
| 350 | SEQ ID NO:48 (SEQ ID NO: 48) | TGGTGATTTGGAGATT (SEQ ID NO: 1905) |
| 351 | SEQ ID NO:48 (SEQ ID NO: 48) | TAGGGTTACGTGTCGG (SEQ ID NO: 1906) |
| 352 | SEQ ID NO:48 (SEQ ID NO: 48) | TAGGGTTATGTGTTGG (SEQ ID NO: 1907) |
| 353 | SEQ ID NO:48 (SEQ ID NO: 48) | TAGGGTTACGTGTCGG (SEQ ID NO: 1906) |
| 354 | SEQ ID NO:48 (SEQ ID NO: 48) | TAGGGTTATGTGTTGG (SEQ ID NO: 1907) |
| 355 | SEQ ID NO:48 (SEQ ID NO: 48) | AACGAATTTTTCGATATT (SEQ ID NO: 1908) |
| 356 | SEQ ID NO:48 (SEQ ID NO: 48) | TTTAATGAATTTTTTGATATT (SEQ ID NO: 1909) |
| 357 | SEQ ID NO:48 (SEQ ID NO: 48) | AAAATCGTATGCGTGT (SEQ ID NO: 1910) |
| 358 | SEQ ID NO:48 (SEQ ID NO: 48) | GAAAATTGTATGTGTGTG (SEQ ID NO: 1911) |
| 359 | SEQ ID NO:9 (SEQ ID NO: 9) | GTTTCGCGTTTAGGGA (SEQ ID NO: 1912) |
| 360 | SEQ ID NO:9 (SEQ ID NO: 9) | GTTTTGTGTTTAGGGAT (SEQ ID NO: 1913) |
| 361 | SEQ ID NO:9 (SEQ ID NO: 9) | AGTGTTCGTCGTAGTT (SEQ ID NO: 1914) |
| 362 | SEQ ID NO:9 (SEQ ID NO: 9) | TGAGTGTTTGTTGTAGT (SEQ ID NO: 1915) |
| 363 | SEQ ID NO:4 (SEQ ID NO: 4) | TTTTGTTCGCGTTGAA (SEQ ID NO: 1916) |

(continued)

| No: | Gene | Oligo: |
|---|---|---|
| 364 | SEQ ID NO:4 (SEQ ID NO: 4) | TTGTTTGTGTTGAAGTA (SEQ ID NO: 1917) |
| 365 | SEQ ID NO:4 (SEQ ID NO: 4) | GGGTCGCGAGGTAGTT (SEQ ID NO: 1918) |
| 366 | SEQ ID NO:4 (SEQ ID NO: 4) | TGGGTTGTGAGGTAGT (SEQ ID NO: 1919) |
| 367 | SEQ ID NO:4 (SEQ ID NO: 4) | TTTGTGCGACGTTATT (SEQ ID NO: 1920) |
| 368 | SEQ ID NO:4 (SEQ ID NO: 4) | GGTTTGTGTGATGTTAT (SEQ ID NO: 1921) |
| 369 | SEQ ID NO:4 (SEQ ID NO: 4) | ATGGCGGTTTCGATTT (SEQ ID NO: 1922) |
| 370 | SEQ ID NO:4 (SEQ ID NO: 4) | GATGGTGGTTTTGATTT (SEQ ID NO: 1923) |
| 371 | SEQ ID NO:5 (SEQ ID NO: 5) | AATGAGCGAGAAAGTA (SEQ ID NO: 1924) |
| 372 | SEQ ID NO:5 (SEQ ID NO: 5) | AGAATGAGTGAGAAAGT (SEQ ID NO: 1925) |
| 373 | SEQ ID NO:5 (SEQ ID NO: 5) | TATGAGGTCGTATTGG (SEQ ID NO: 2163) |
| 374 | SEQ ID NO:5 (SEQ ID NO: 5) | TATGAGGTTGTATTGGT (SEQ ID NO: 2164) |
| 375 | SEQ ID NO:7 (SEQ ID NO: 7) | TAGTCGTCGTGTAGGA (SEQ ID NO: 1932) |
| 376 | SEQ ID NO:7 (SEQ ID NO: 7) | TAGGTAGTTGTTGTGTA (SEQ ID NO: 1933) |
| 377 | SEQ ID NO:7 (SEQ ID NO: 7) | TATAGGTACGCGATGA (SEQ ID NO: 1934) |
| 378 | SEQ ID NO:7 (SEQ ID NO: 7) | AGGTATGTGATGAGGA (SEQ ID NO: 1935) |
| 379 | SEQ ID NO:7 (SEQ ID NO: 7) | TATGGTTACGTACGAG (SEQ ID NO: 1936) |
| 380 | SEQ ID NO:7 (SEQ ID NO: 7) | ATGGTTATGTATGAGTTT (SEQ ID NO: 1937) |
| 381 | SEQ ID NO:7 (SEQ ID NO: 7) | ATGATTTGCGTTACGT (SEQ ID NO: 1938) |
| 382 | SEQ ID NO:7 (SEQ ID NO: 7) | ATGATTTGTGTTATGTTT (SEQ ID NO: 1939) |
| 383 | SEQ ID NO:7 (SEQ ID NO: 7) | TAACGTTGTGGTTCGAA (SEQ ID NO: 1940) |
| 384 | SEQ ID NO:7 (SEQ ID NO: 7) | TAATGTTGTGGTTTGAA (SEQ ID NO: 1941) |
| 385 | SEQ ID NO:7 (SEQ ID NO: 7) | TAACGTTGTGGTTCGAA (SEQ ID NO: 1940) |
| 386 | SEQ ID NO:7 (SEQ ID NO: 7) | TAATGTTGTGGTTTGAA (SEQ ID NO: 1941) |
| 387 | SEQ ID NO:1 (SEQ ID NO: 1) | GGTCGGCGTTGATTTTA (SEQ ID NO: 1942) |
| 388 | SEQ ID NO: (SEQ ID NO: 1) | GGTTGGTGTTGATTTTA (SEQ ID NO: 1943) |
| 389 | SEQ ID NO: (SEQ ID NO: 1) | GGTCGGCGTTGATTTTA (SEQ ID NO: 1942) |
| 390 | SEQ ID NO: (SEQ ID NO: 1) | GGTTGGTGTTGATTTTA (SEQ ID NO: 1943) |
| 391 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTACGGAGGCGTTTTA (SEQ ID NO: 1958) |
| 392 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTATGGAGGTGTTTT (SEQ ID NO: 1959) |

(continued)

| No: | Gene | Oligo: |
|---|---|---|
| 393 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | AGGCGAATTTATCGGG (SEQ ID NO: 1960) |
| 394 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | GGTGAATTTATTGGGG (SEQ ID NO: 1961) |
| 395 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTCGAAGTAGGCGT (SEQ ID NO: 1962) |
| 396 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TAGTTGAAGTAGGTGTT (SEQ ID NO: 1963) |
| 397 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTCGACGAAGTTTT (SEQ ID NO: 1964) |
| 398 | ORPHAN NUCLEAR RECEPTOR NR5A2 (ALPHA-1-FETOPROTEIN TRANSCRIPTION FACTOR) (HEPATOCYTIC TRANSCRIPTION FACTOR) (B1-BINDING FACTOR) (HB1F) (CYP7A PROMOTER BINDING FACTOR) (SEQ ID NO: 11) | TTTTGATGAAGTTTTGTT (SEQ ID NO: 1965) |
| 399 | LIM DOMAIN KINASE 1 (EC 2.7.1.37) (LIMK-1) (SEQ ID NO: 12) | TGTAGTCGGGAGGTTA (SEQ ID NO: 1966) |
| 400 | LIM DOMAIN KINASE 1 (EC 2.7.1.37) (LIMK-1) (SEQ ID NO: 12) | TGTAGTTGGGAGGTTA (SEQ ID NO: 1967) |
| 401 | LIM DOMAIN KINASE 1 (EC 2.7.1.37) (LIMK-1) (SEQ ID NO: 12) | TGTAGTCGGGAGGTTA (SEQ ID NO: 1966) |
| 402 | LIM DOMAIN KINASE 1 (EC 2.7.1.37) (LIMK-1) (SEQ ID NO: 12) | TGTAGTTGGGAGGTTA (SEQ ID NO: 1967) |
| 403 | LIM DOMAIN KINASE 1 (EC 2.7.1.37) (LIMK-1) (SEQ ID NO: 12) | GGATTATCGCGGGGGT (SEQ ID NO: 1968) |
| 404 | LIM DOMAIN KINASE 1 (EC 2.7.1.37) (LIMK-1) (SEQ ID NO: 12) | GGATTATTGTGGGGGT (SEQ ID NO: 1969) |
| 405 | LIM DOMAIN KINASE 1 (EC 2.7.1.37) (LIMK-1) (SEQ ID NO: 12) | GGATTATCGCGGGGGT (SEQ ID NO: 1968) |
| 406 | LIM DOMAIN KINASE 1 (EC 2.7.1.37) (LIMK-1) (SEQ ID NO: 12) | GGATTATTGTGGGGGT (SEQ ID NO: 1969) |
| 407 | LIM DOMAIN KINASE 1 (EC 2.7.1.37) (LIMK-1) (SEQ ID NO: 12) | GTCGGTAGTTTATCGGAT (SEQ ID NO: 1970) |

(continued)

| No: | Gene | Oligo: |
|---|---|---|
| 408 | LIM DOMAIN KINASE 1 (EC 2.7.1.37) (LIMK-1) (SEQ ID NO: 12) | GTTGGTAGTTTATTGGAT (SEQ ID NO: 1971) |
| 409 | LIM DOMAIN KINASE 1 (EC 2.7.1.37) (LIMK-1) (SEQ ID NO: 12) | GTCGGTAGTTTATCGGAT (SEQ ID NO: 1970) |
| 410 | LIM DOMAIN KINASE 1 (EC 2.7.1.37) (LIMK-1) (SEQ ID NO: 12) | GTTGGTAGTTTATTGGAT (SEQ ID NO: 1971) |
| 411 | LIM DOMAIN KINASE 1 (EC 2.7.1.37) (LIMK-1) (SEQ ID NO: 12) | TAGGAGACGTTACGTT (SEQ ID NO: 1972) |
| 412 | LIM DOMAIN KINASE 1 (EC 2.7.1.37) (LIMK-1) (SEQ ID NO: 12) | AGATGTTATGTTAGGGT (SEQ ID NO: 1973) |
| 413 | BCL11B (SEQ ID NO: 50) | TAGGTTTCGATTCGTT (SEQ ID NO: 1974) |
| 414 | BCL11B (SEQ ID NO: 50) | TTAGGTTTTGATTTGTTT (SEQ ID NO: 1975) |
| 415 | BCL11B (SEQ ID NO: 50) | AAGTCGTCGGAGTTAG (SEQ ID NO: 1976) |
| 416 | BCL11B (SEQ ID NO: 50) | GTGAAGTTGTTGGAGT (SEQ ID NO: 1977) |
| 417 | MSF (SEQ ID NO: 13) | GTTTCGAAATTGGCGT (SEQ ID NO: 1980) |
| 418 | MSF (SEQ ID NO: 13) | TTTGAAATTGGTGTGG (SEQ ID NO: 1981) |
| 419 | MSF (SEQ ID NO: 13) | TTCGGTTTACGGGTTGTA (SEQ ID NO: 1982) |
| 420 | MSF (SEQ ID NO: 13) | TTTGGTTTATGGGTTGTA (SEQ ID NO: 1983) |
| 421 | MSF (SEQ ID NO: 13) | TTCGGTTTACGGGTTGTA (SEQ ID NO: 1982) |
| 422 | MSF (SEQ ID NO: 13) | TTTGGTTTATGGGTTGTA (SEQ ID NO: 1983) |
| 423 | MSF (SEQ ID NO: 13) | TTACGGTTCGATTTTG (SEQ ID NO: 1984) |
| 424 | MSF (SEQ ID NO: 13) | TATGGTTTGATTTTGGG (SEQ ID NO: 1985) |
| 425 | SEQ ID NO:14 (SEQ ID NO: 14) | TAAGGCGTTTTCGATA (SEQ ID NO: 1986) |
| 426 | SEQ ID NO: 14 (SEQ ID NO: 14) | GTAAGGTGTTTTTGATAT (SEQ ID NO: 1987) |
| 427 | SEQ ID NO: 16 (SEQ ID NO: 16) | AGGAGTTTTCGTGTCGT (SEQ ID NO: 1992) |
| 428 | SEQ ID NO: 16 (SEQ ID NO: 16) | AGGAGTTTTTGTGTTGT (SEQ ID NO: 1993) |
| 429 | SEQ ID NO:16 (SEQ ID NO: 16) | AGGAGTTTTCGTGTCGT (SEQ ID NO: 1992) |
| 430 | SEQ ID NO:16 (SEQ ID NO: 16) | AGGAGTTTTTGTGTTGT (SEQ ID NO: 1993) |
| 431 | SEQ ID NO: 17 (SEQ ID NO: 17) | TACGTTTCGGGTTTGTTA (SEQ ID NO: 1998) |
| 432 | SEQ ID NO: 17 (SEQ ID NO: 17) | TATGTTTTGGGTTTGTTA (SEQ ID NO: 1999) |
| 433 | SEQ ID NO: 17 (SEQ ID NO: 17) | TACGTTTCGGGTTTGTTA (SEQ ID NO: 1998) |
| 434 | SEQ ID NO: 17 (SEQ ID NO: 17) | TATGTTTTGGGTTTGTTA (SEQ ID NO: 1999) |
| 435 | SEQ ID NO: 17 (SEQ ID NO: 17) | ATTTAGTCGTGCGTTT (SEQ ID NO: 2000) |
| 436 | SEQ ID NO: 17 (SEQ ID NO: 17) | TGATTTAGTTGTGTGTT (SEQ ID NO: 2001) |
| 437 | SEQ ID NO: 18 (SEQ ID NO: 18) | TTTACGCGGGGTTTTA (SEQ ID NO: 2002) |
| 438 | SEQ ID NO: 18 (SEQ ID NO: 18) | TTTATGTGGGGTTTTAG (SEQ ID NO: 2003) |
| 439 | SEQ ID NO: 18 (SEQ ID NO: 18) | TTACGTCGTTATTAGGT (SEQ ID NO: 2004) |
| 440 | SEQ ID NO: 18 (SEQ ID NO: 18) | TTTTATGTTGTTATTAGGT (SEQ ID NO: 2005) |
| 441 | SEQ ID NO: 18 (SEQ ID NO: 18) | TATTTGGACGTCGGGT (SEQ ID NO: 2006) |

(continued)

| No: | Gene | Oligo: |
|---|---|---|
| 442 | SEQ ID NO: 18 (SEQ ID NO: 18) | TATTTGGATGTTGGGT (SEQ ID NO: 2007) |
| 443 | SEQ ID NO: 18 (SEQ ID NO: 18) | TATTTGGACGTCGGGT (SEQ ID NO: 2006) |
| 444 | SEQ ID NO: 18 (SEQ ID NO: 18) | TATTTGGATGTTGGGT (SEQ ID NO: 2007) |
| 445 | SEQ ID NO: 18 (SEQ ID NO: 18) | AAAGCGGAGTCGTTAG (SEQ ID NO: 2010) |
| 446 | SEQ ID NO: 18 (SEQ ID NO: 18) | AGTGGAGTTGTTAGGT (SEQ ID NO: 2011) |
| 447 | SEQ ID NO:19 (SEQ ID NO: 19) | AGGTTTTCGTTGTAGTA (SEQ ID NO: 2012) |
| 448 | SEQ ID NO: 19 (SEQ ID NO: 19) | TAGGTTTTTGTTGTAGTA (SEQ ID NO: 2013) |
| 449 | SEQ ID NO:19 (SEQ ID NO: 19) | TTTGATATCGAGGGAG (SEQ ID NO: 2165) |
| 450 | SEQ ID NO: 19 (SEQ ID NO: 19) | TTTGATATTGAGGGAGG (SEQ ID NO: 2166) |
| 451 | SEQ ID NO:19 (SEQ ID NO: 19) | GGTGTACGGAGGAAAG (SEQ ID NO: 2167) |
| 452 | SEQ ID NO:19 (SEQ ID NO: 19) | GGTGTATGGAGGAAAG (SEQ ID NO: 2168) |
| 453 | SEQ ID NO:19 (SEQ ID NO: 19) | GGTGTACGGAGGAAAG (SEQ ID NO: 2167) |
| 454 | SEQ ID NO:19 (SEQ ID NO: 19) | GGTGTATGGAGGAAAG (SEQ ID NO: 2168) |
| 455 | SEQ ID NO:19 (SEQ ID NO: 19) | TGAGATTCGTTTTTTAAA (SEQ ID NO: 2014) |
| 456 | SEQ ID NO:19 (SEQ ID NO: 19) | GGTGAGATTTGTTTTTTA (SEQ ID NO: 2015) |
| 457 | PRDM6 (SEQ ID NO: 20) | TTGTCGGGTTACGGGA (SEQ ID NO: 2020) |
| 458 | PRDM6 (SEQ ID NO: 20) | GTTGGGTTATGGGAGA (SEQ ID NO: 2021) |
| 459 | PRDM6 (SEQ ID NO: 20) | TTCGTAGAATTGTCGAAG (SEQ ID NO: 2022) |
| 460 | PRDM6 (SEQ ID NO: 20) | TTTGTAGAATTGTTGAAG (SEQ ID NO: 2023) |
| 461 | PRDM6 (SEQ ID NO: 20) | TTCGTAGAATTGTCGAAG (SEQ ID NO: 2022) |
| 462 | PRDM6 (SEQ ID NO: 20) | TTTGTAGAATTGTTGAAG (SEQ ID NO: 2023) |
| 463 | NR2E1 (SEQ ID NO: 22) | AATGTAGCGGCGTTAT (SEQ ID NO: 2028) |
| 464 | NR2E1 (SEQ ID NO: 22) | TAAATGTAGTGGTGTTAT (SEQ ID NO: 2029) |
| 465 | NR2E1 (SEQ ID NO: 22) | GTCGTTATCGGTTTGGA (SEQ ID NO: 2030) |
| 466 | NR2E1 (SEQ ID NO: 22) | GTTGTTATTGGTTTGGA (SEQ ID NO: 2031) |
| 467 | NR2E1 (SEQ ID NO: 22) | GTCGTTATCGGTTTGGA (SEQ ID NO: 2030) |
| 468 | NR2E1 (SEQ ID NO: 22) | GTTGTTATTGGTTTGGA (SEQ ID NO: 2031) |
| 469 | NR2E1 (SEQ ID NO: 22) | GACGTAAGTTTCGGGT (SEQ ID NO: 2032) |
| 470 | NR2E1 (SEQ ID NO: 22) | GGATGTAAGTTTTGGG (SEQ ID NO: 2033) |
| 471 | NR2E1 (SEQ ID NO: 22) | TTTTTAGTCGCGAGAA (SEQ ID NO: 2034) |
| 472 | NR2E1 (SEQ ID NO: 22) | TTTTTAGTTGTGAGAAGT (SEQ ID NO: 2035) |
| 473 | NR2E1 (SEQ ID NO: 22) | TTCGGGTGATATCGTTT (SEQ ID NO: 2036) |
| 474 | NR2E1 (SEQ ID NO: 22) | TTTGGGTGATATTGTTT (SEQ ID NO: 2037) |
| 475 | NR2E1 (SEQ ID NO: 22) | TTCGGGTGATATCGTTT (SEQ ID NO: 2036) |
| 476 | NR2E1 (SEQ ID NO: 22) | TTTGGGTGATATTGTTT (SEQ ID NO: 2037) |
| 477 | NR2E1 (SEQ ID NO: 22) | AGGCGAGTCGGAGTTT (SEQ ID NO: 2038) |
| 478 | NR2E1 (SEQ ID NO: 22) | AGGTGAGTTGGAGTTTT (SEQ ID NO: 2039) |
| 479 | NR2E1 (SEQ ID NO: 22) | TAAGTCGAGCGAGTTT (SEQ ID NO: 2040) |

(continued)

| No: | Gene | Oligo: |
|---|---|---|
| 480 | NR2E1 (SEQ ID NO: 22) | TAGTAAGTTGAGTGAGT (SEQ ID NO: 2041) |
| 481 | NR2E1 (SEQ ID NO: 22) | AGGGACGCGAAAATTT (SEQ ID NO: 2042) |
| 482 | NR2E1 (SEQ ID NO: 22) | GGAGGGATGTGAAAAT (SEQ ID NO: 2043) |
| 483 | PCDH7 (SEQ ID NO: 23) | ATCGTAGTCGGTTTTA (SEQ ID NO: 2044) |
| 484 | PCDH7 (SEQ ID NO: 23) | GATTATTGTAGTTGGTTT (SEQ ID NO: 2045) |
| 485 | RTTN (SEQ ID NO: 25) | TAGGACGTGTTTTCGG (SEQ ID NO: 2048) |
| 486 | RTTN (SEQ ID NO: 25) | AGGATGTGTTTTTGGG (SEQ ID NO: 2049) |
| 487 | SNAP25 (SEQ ID NO: 33) | TATTTCGAGTTAGAGTTACGA (SEQ ID NO: 2050) |
| 488 | SNAP25 (SEQ ID NO: 33) | TATTTTGAGTTAGAGTTATGA (SEQ ID NO: 2051) |
| 489 | SNAP25 (SEQ ID NO: 33) | TATTTCGAGTTAGAGTTACGA (SEQ ID NO: 2050) |
| 490 | SNAP25 (SEQ ID NO: 33) | TATTTTGAGTTAGAGTTATGA (SEQ ID NO: 2051) |
| 491 | SNAP25 (SEQ ID NO: 33) | ATACGGAATATCGTATTT (SEQ ID NO: 2052) |
| 492 | SNAP25 (SEQ ID NO: 33) | GTGTATATATGGAATATTGT (SEQ ID NO: 2053) |
| 493 | SNAP25 (SEQ ID NO: 33) | GTTATTATTCGGTACGT (SEQ ID NO: 2169) |
| 494 | SNAP25 (SEQ ID NO: 33) | AGTTATTATTTGGTATGTAT (SEQ ID NO: 2170) |
| 495 | SEQ ID NO:26 (SEQ ID NO: 26) | TTAAGTATCGAGGCGT (SEQ ID NO: 2054) |
| 496 | SEQ ID NO:26 (SEQ ID NO: 26) | TTTTAAGTATTGAGGTGT (SEQ ID NO: 2055) |
| 497 | SEQ ID NO:26 (SEQ ID NO: 26) | AATTTTGGTCGTTTAGT (SEQ ID NO: 2056) |
| 498 | SEQ ID NO:26 (SEQ ID NO: 26) | AAATTTTGGTTGTTTAGT (SEQ ID NO: 2057) |
| 499 | GIRK2 (SEQ ID NO: 27) | AGTTGTTCGTAGGCGA (SEQ ID NO: 2060) |
| 500 | GIRK2 (SEQ ID NO: 27) | AGTTGTTTGTAGGTGA (SEQ ID NO: 2061) |
| 501 | GIRK2 (SEQ ID NO: 27) | AGTTGTTCGTAGGCGA (SEQ ID NO: 2060) |
| 502 | GIRK2 (SEQ ID NO: 27) | AGTTGTTTGTAGGTGA (SEQ ID NO: 2061) |
| 503 | GIRK2 (SEQ ID NO: 27) | TTATTTCGTTCGTAGTT (SEQ ID NO: 2062) |
| 504 | GIRK2 (SEQ ID NO: 27) | TTTGTTTGTAGTTAGGTA (SEQ ID NO: 2063) |
| 505 | GIRK2 (SEQ ID NO: 27) | TTAGTCGAAAGGCGAG (SEQ ID NO: 2064) |
| 506 | GIRK2 (SEQ ID NO: 27) | TAGTTGAAAGGTGAGG (SEQ ID NO: 2065) |
| 507 | SEQ ID NO:28 (SEQ ID NO: 28) | ATTAGGCGAGTTTCGT (SEQ ID NO: 2066) |
| 508 | SEQ ID NO:28 (SEQ ID NO: 28) | TTAGGTGAGTTTTGTTT (SEQ ID NO: 2067) |
| 509 | SEQ ID NO:31 (SEQ ID NO: 31) | TTTACGTAGGGCGATT (SEQ ID NO: 2068) |
| 510 | SEQ ID NO:31 (SEQ ID NO: 31) | ATTTTTATGTAGGGTGAT (SEQ ID NO: 2069) |
| 511 | SEQ ID NO:31 (SEQ ID NO: 31) | GATACGGTTAGGCGGG (SEQ ID NO: 2070) |
| 512 | SEQ ID NO:31 (SEQ ID NO: 31) | GATATGGTTAGGTGGG (SEQ ID NO: 2071) |
| 513 | SEQ ID NO:31 (SEQ ID NO: 31) | GATACGGTTAGGCGGG (SEQ ID NO: 2070) |
| 514 | SEQ ID NO:31 (SEQ ID NO: 31) | GATATGGTTAGGTGGG (SEQ ID NO: 2071) |
| 515 | SEQ ID NO:31 (SEQ ID NO: 31) | TTCGGGCGTTTTATAT (SEQ ID NO: 2072) |
| 516 | SEQ ID NO:31 (SEQ ID NO: 31) | GGTTTGGGTGTTTTATA (SEQ ID NO: 2073) |
| 517 | HOXB13 (SEQ ID NO: 34) | GTCGTTATTATTTCGAGG (SEQ ID NO: 2074) |

(continued)

| No: | Gene | Oligo: |
|---|---|---|
| 518 | HOXB13 (SEQ ID NO: 34) | GTTGTTATTATTTTGAGGA (SEQ ID NO: 2075) |
| 519 | HOXB13 (SEQ ID NO: 34) | AAGTTAGCGGGTTCGT (SEQ ID NO: 2076) |
| 520 | HOXB13 (SEQ ID NO: 34) | AAGTTAGTGGGTTTGT (SEQ ID NO: 2077) |
| 521 | HOXB13 (SEQ ID NO: 34) | AAGTTAGCGGGTTCGT (SEQ ID NO: 2076) |
| 522 | HOXB13 (SEQ ID NO: 34) | AAGTTAGTGGGTTTGT (SEQ ID NO: 2077) |
| 523 | HOXB13 (SEQ ID NO: 34) | TTGGTCGCGTAGTAAA (SEQ ID NO: 2078) |
| 524 | HOXB13 (SEQ ID NO: 34) | TGGTTGTGTAGTAAAGT (SEQ ID NO: 2079) |
| 525 | (SEQ ID NO: 35) | AAATAGTCGTTTGGGA (SEQ ID NO: 2082) |
| 526 | (SEQ ID NO: 35) | AAATAGTTGTTTGGGAG (SEQ ID NO: 2083) |
| 527 | (SEQ ID NO: 35) | TAAAGACGGGAAGAGA (SEQ ID NO: 2086) |
| 528 | (SEQ ID NO: 35) | ATAAAGATGGGAAGAGA (SEQ ID NO: 2087) |
| 529 | MGC10561 (SEQ ID NO: 37) | ATATTTAGCGTAGTTATTT (SEQ ID NO: 2171) |
| 530 | MGC10561 (SEQ ID NO: 37) | TAGTATATTTAGTGTAGTTAT (SEQ ID NO: 2172) |
| 531 | LMX1A (SEQ ID NO: 38) | GTCGGGTTTTTCGGAA (SEQ ID NO: 2092) |
| 532 | LMX1A (SEQ ID NO: 38) | GTTGGGTTTTTTGGAAG (SEQ ID NO: 2093) |
| 533 | LMX1A (SEQ ID NO: 38) | GTCGAGATTTTATCGAAA (SEQ ID NO: 2094) |
| 534 | LMX1A (SEQ ID NO: 38) | GTTGAGATTTTATTGAAAG (SEQ ID NO: 2095) |
| 535 | LMX1A (SEQ ID NO: 3 8) | AGTATTCGGGCGGGTA (SEQ ID NO: 2096) |
| 536 | LMX1A (SEQ ID NO: 38) | GTAGTATTTGGGTGGG (SEQ ID NO: 2097) |
| 537 | LMX1A (SEQ ID NO: 38) | AACGAAATTACGTGTAT (SEQ ID NO: 2098) |
| 538 | LMX1A (SEQ ID NO: 38) | TGAAATGAAATTATGTGTA (SEQ ID NO: 2099) |
| 539 | GS1 (SEQ ID NO: 40) | TGCGAGTTAGCGGTTA (SEQ ID NO: 2100) |
| 540 | GS1 (SEQ ID NO: 40) | TTGTGAGTTAGTGGTT (SEQ ID NO: 2101) |
| 541 | TITF1 (SEQ ID NO: 41) | GTCGTGGGGATCGTAT (SEQ ID NO: 2104) |
| 542 | TITF1 (SEQ ID NO: 41) | GTTGTGGGGATTGTAT (SEQ ID NO: 2105) |
| 543 | TITF1 (SEQ ID NO: 41) | GTCGTGGGGATCGTAT (SEQ ID NO: 2104) |
| 544 | TITF1 (SEQ ID NO: 41) | GTTGTGGGGATTGTAT (SEQ ID NO: 2105) |
| 545 | TITF1 (SEQ ID NO: 41) | GGTTCGTTTAAGTTCGG (SEQ ID NO: 2106) |
| 546 | TITF1 (SEQ ID NO: 41) | GGTTTGTTTAAGTTTGG (SEQ ID NO: 2107) |
| 547 | TITF1 (SEQ ID NO: 41) | GGTTCGTTTAAGTTCGG (SEQ ID NO: 2106) |
| 548 | TITF1 (SEQ ID NO: 41) | GGTTTGTTTAAGTTTGG (SEQ ID NO: 2107) |
| 549 | TITF1 (SEQ ID NO: 41) | TTAGGTCGCGTTTGTA (SEQ ID NO: 2108) |
| 550 | TITF1 (SEQ ID NO: 41) | AGGTTGTGTTTGTAGA (SEQ ID NO: 2109) |
| 551 | TITF1 (SEQ ID NO: 41) | TATTTCGTTTTCGTAATT (SEQ ID NO: 2110) |
| 552 | TITF1 (SEQ ID NO: 41) | TTTGTTTTTGTAATTAGATT (SEQ ID NO: 2111) |
| 553 | DDX51 (SEQ ID NO: 43) | TAGGCGAGCGTTAGGT (SEQ ID NO: 2173) |
| 554 | DDX51 (SEQ ID NO: 43) | GGTGAGTGTTAGGTTA (SEQ ID NO: 2174) |
| 555 | DDX51 (SEQ ID NO: 43) | AACGTGCGGGGTTTTT (SEQ ID NO: 2116) |

(continued)

| No: | Gene | Oligo: |
|---|---|---|
| 556 | DDX51 (SEQ ID NO: 43) | TGAATGTGTGGGGTTT (SEQ ID NO: 2117) |
| 557 | SEQ ID NO:45 (SEQ ID NO: 45) | TGTAGTATCGGGGGAG (SEQ ID NO: 2175) |
| 558 | SEQ ID NO:45 (SEQ ID NO: 45) | TGTAGTATTGGGGGAG (SEQ ID NO: 2176) |
| 559 | SEQ ID NO:45 (SEQ ID NO: 45) | TGTAGTATCGGGGGAG (SEQ ID NO: 2175) |
| 560 | SEQ ID NO:45 (SEQ ID NO: 45) | TGTAGTATTGGGGGAG (SEQ ID NO: 2176) |
| 561 | SEQ ID NO:46 (SEQ ID NO: 46) | GAGTCGGGTTATCGTT (SEQ ID NO: 2120) |
| 562 | SEQ ID NO:46 (SEQ ID NO: 46) | GGAGTTGGGTTATTGT (SEQ ID NO: 2121) |
| 563 | SEQ ID NO:46 (SEQ ID NO: 46) | TTACGGATGTTTCGGT (SEQ ID NO: 2122) |
| 564 | SEQ ID NO:46 (SEQ ID NO: 46) | TTTATGGATGTTTTGGT (SEQ ID NO: 2123) |
| 565 | SEQ ID NO:46 (SEQ ID NO: 46) | TGACGTATTTTCGGTT (SEQ ID NO: 2124) |
| 566 | SEQ ID NO:46 (SEQ ID NO: 46) | GGTGATGTATTTTTGGT (SEQ ID NO: 2125) |
| 567 | SEQ ID NO:46 (SEQ ID NO: 46) | ATAGTCGGAATCGTTG (SEQ ID NO: 2126) |
| 568 | 568SEQ ID NO:46 (SEQ ID NO: 46) | TAGTTGGAATTGTTGTT (SEQ ID NO: 2127) |
| 569 | SEQ ID NO:2 (SEQ ID NO: 2) | ATTGGGTTTCGCGTAGG (SEQ ID NO: 2128) |
| 570 | SEQ ID NO:2 (SEQ ID NO: 2) | ATTGGGTTTTGTGTAGG (SEQ ID NO: 2129) |
| 571 | SEQ ID NO:2 (SEQ ID NO: 2) | ATTGGGTTTCGCGTAGG (SEQ ID NO: 2128) |
| 572 | SEQ ID NO:2 (SEQ ID NO: 2) | ATTGGGTTTTGTGTAGG (SEQ ID NO: 2129) |
| 573 | SEQ ID NO:2 (SEQ ID NO: 2) | TAGTAGTCGGCGGGAG (SEQ ID NO: 2130) |
| 574 | SEQ ID NO:2 (SEQ ID NO: 2) | TAGTAGTTGGTGGGAG (SEQ ID NO: 2131) |
| 575 | SEQ ID NO:2 (SEQ ID NO: 2) | TAGTAGTCGGCGGGAG (SEQ ID NO: 2130) |
| 576 | SEQ ID NO:2 (SEQ ID NO: 2) | TAGTAGTTGGTGGGAG (SEQ ID NO: 2131) |
| 577 | SEQ ID NO:3 (SEQ ID NO: 3) | TTGCGTTAATTCGGTA (SEQ ID NO: 2132) |
| 578 | SEQ ID NO:3 (SEQ ID NO: 3) | AATTTGTGTTAATTTGGT (SEQ ID NO: 2133) |
| 579 | SEQ ID NO:3 (SEQ ID NO: 3) | AGTCGGGAGAGCGAAA (SEQ ID NO: 2134) |
| 580 | SEQ ID NO:3 (SEQ ID NO: 3) | AGTTGGGAGAGTGAAA (SEQ ID NO: 2135) |
| 581 | SEQ ID NO:3 (SEQ ID NO: 3) | AGTCGGGAGAGCGAAA (SEQ ID NO: 2134) |
| 582 | SEQ ID NO:3 (SEQ ID NO: 3) | AGTTGGGAGAGTGAAA (SEQ ID NO: 2135) |
| 583 | SEQ ID NO:3 (SEQ ID NO: 3) | GGTCGAAGAGTCGGGA (SEQ ID NO: 2136) |
| 584 | SEQ ID NO:3 (SEQ ID NO: 3) | GGTTGAAGAGTTGGGA (SEQ ID NO: 2137) |
| 585 | SEQ ID NO:3 (SEQ ID NO: 3) | GGTCGAAGAGTCGGGA (SEQ ID NO: 2136) |
| 586 | SEQ ID NO:3 (SEQ ID NO: 3) | GGTTGAAGAGTTGGGA (SEQ ID NO: 2137) |
| 587 | SEQ ID NO:3 (SEQ ID NO: 3) | ATGTTAGCGGGTCGAA (SEQ ID NO: 2138) |
| 588 | SEQ ID NO:3 (SEQ ID NO: 3) | ATGTTAGTGGGTTGAA (SEQ ID NO: 2139) |
| 589 | SEQ ID NO:3 (SEQ ID NO: 3) | ATGTTAGCGGGTCGAA (SEQ ID NO: 2138) |
| 590 | SEQ ID NO:3 (SEQ ID NO: 3) | ATGTTAGTGGGTTGAA (SEQ ID NO: 2139) |

Table 21

| Genomic SEQ ID NO: | Primer SEQ ID NO: | Primer SEQ ID NO: | Probe SEQ ID NO: | Syber green | Syber green detection Temperatire ˚C | % of samples methylated (Probe detection) | % of samples methylated (Syber green detection) |
|---|---|---|---|---|---|---|---|
| 20 | 2223 | 2224 | 2225 | No | | 91 | |
| 24 | 2226 | 2227 | 2228 | Yes | 85˚C | 30 | 30 |
| 13 | 2229 | 2230 | | Yes | 81˚C | | 36 |
| 13 | 2239 | 2240 | 2241 | | | 47 | |
| 15 | 2231 | 2232 | 2233 | No | | 40 | |
| 22 | 2233 | 2234 | | Yes | 81˚C | | 72 |
| 22 | 2236 | 2237 | 2238 | | | 68 | |

**Claims**

1. A method for detecting, or for detecting and distinguishing between or among breast cell proliferative disorders in a subject, comprising

   a) determining a CpG methylation status of the LMX1A gene or its promoter region; and
   b) determining therefrom the presence, absence or subclass of a breast cell proliferative disorder.

2. The method according to claim 1, wherein a CpG methylation status of SEQ ID NO: 38 is determined.

3. The method according to claim 1 or 2 for detecting breast cell proliferative disorders, comprising

   (a) contacting genomic DNA isolated from a biological sample obtained from the subject, or a fragment thereof, with one reagent or a plurality of reagents sufficient for distinguishing between methylated and non methylated CpG dinucleotide sequences within a target sequence, the target sequence comprises, or hybridizes under stringent conditions to a sequence comprising at least 16 contiguous nucleotides of SEQ ID NO: 38, said contiguous nucleotides comprising at least one CpG dinucleotide sequence; and
   (b) determining, based at least in part on said distinguishing, the methylation state of at least one target CpG dinucleotide sequence, or an average, or a value reflecting an average methylation state of a plurality of target CpG dinucleotide sequences.

4. The method according to claim 3, wherein the distinguishing between methylated and non methylated CpG dinucleotide sequences comprises methylation state-dependent conversion or non-conversion of at least one such CpG dinucleotide sequence to the corresponding converted or non-converted dinucleotide sequence within a sequence selected from the group consisting of SEQ ID NO: 567, 568, 803, 804, and contiguous regions thereof.

5. The method according to claim 1 or 2, comprising

   (a) extracting genomic DNA from a biological sample obtained from a subject;
   (b) contacting the genomic DNA, or a fragment thereof, comprising one or more sequences selected from SEQ ID NO: 38 or a sequence that hybridizes under stringent conditions thereto, with one or more methylation-sensitive restriction enzymes, wherein the genomic DNA is either digested thereby to produce digestion fragments, or is not digested thereby; and
   (c) determining, based on a presence or absence of, or on property of at least one such fragment, the methylation state of at least one CpG dinucleotide sequence of one or more sequences of SEQ ID NO: 38, or an average, or a value reflecting an average methylation state of a plurality of CpG dinucleotide sequences thereof.

6. The method according to claim 3 to 5, wherein said biological sample is selected from the group consisting of cell lines, histological slides, biopsies, paraffin-embedded tissue, body fluids, nipple aspirate fluid , lymphatic fluid, ductal lavage fluid, fine needle aspirate, blood plasma, blood serum, whole blood, isolated blood cells, cells isolated from the blood, and all possible combinations thereof.

7. An oligomer, comprising a sequence of at least 9, 15, 20, 25, 30 or 35 contiguous nucleotides that is complementary to, or hybridizes under moderately stringent or stringent conditions to a sequence selected from the group consisting of SEQ ID NO: 567, 568, 803, and 804.

8. A kit comprising:

(a) a bisulfite-containing reagent; and
(b) at least two primer oligonucleotides whose sequences in each case correspond, are complementary, or hybridize under stringent or highly stringent conditions to a 16-base long segment of the sequences SEQ ID NO: 567, 568, 803, or 804.

9. The kit of claim 8, further comprising standard reagents for performing a methylation assay selected from the group consisting of MS-SNuPE, MSP, MethyLight, HeavyMethyl, nucleic acid sequencing, and combinations thereof.

10. Use of mRNA expression analysis, protein expression analysis or analysis of a CpG methylation status of the LMX1A gene, genomic sequences thereof, its promoter or regulatory elements for the detection, or for the detection and distinguishing between or among breast cell proliferative disorders.

11. Use of an oligomer according to claim 7 for the detection, the detection and differentiation between or among subclasses of, diagnosis of, prognosis of, treatment of, monitoring of, and treatment and monitoring of breast cell proliferative disorders.

12. Use of a method according to claims 1 to 6 for diagnosis, prognosis, differentiation, and treatment of breast cell proliferative disorders, as well as for the identification of and differentiation between subclasses or said disorders.

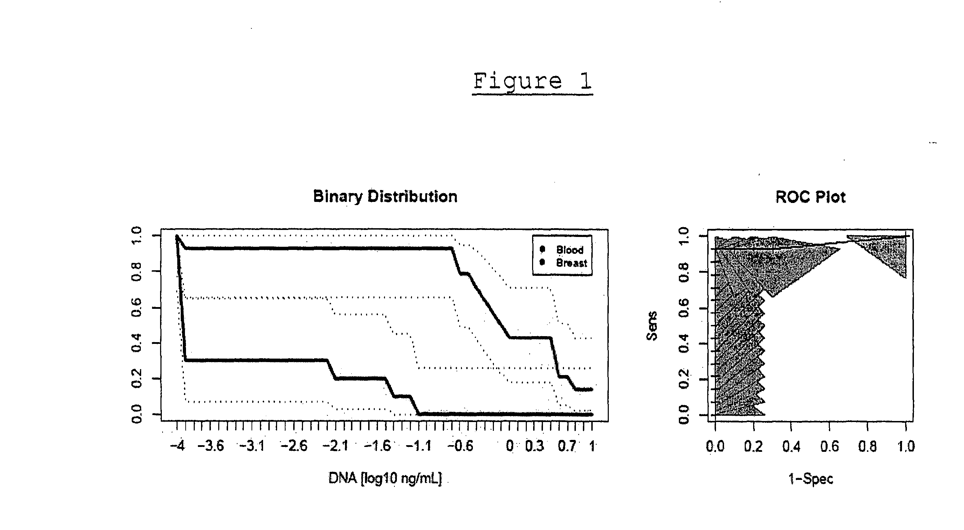

Figure 1

ROC Plot

Binary Distribution

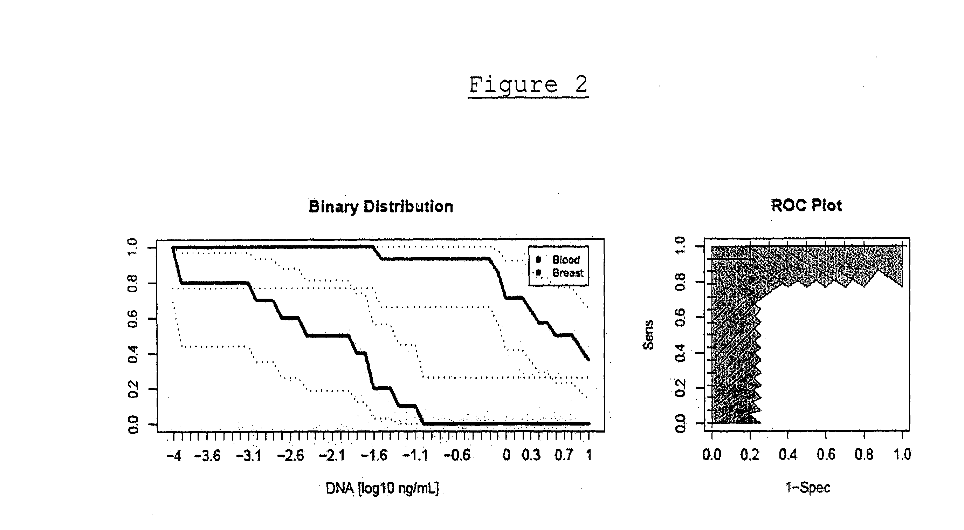

Figure 2

ROC Plot

Binary Distribution

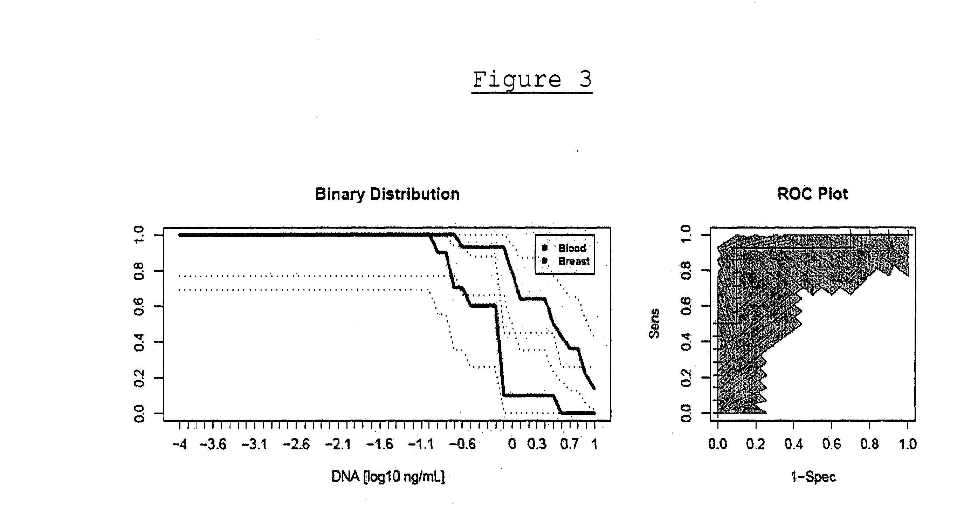

Figure 3

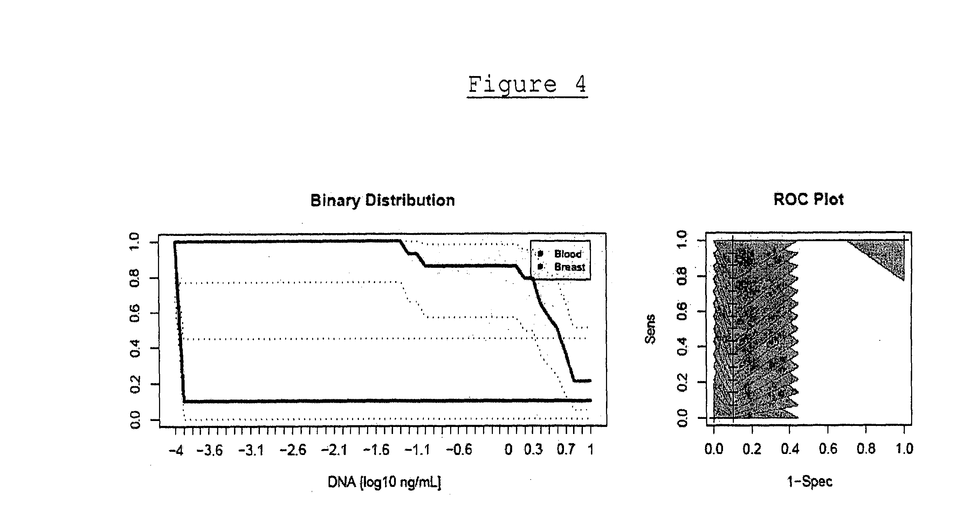

Figure 4

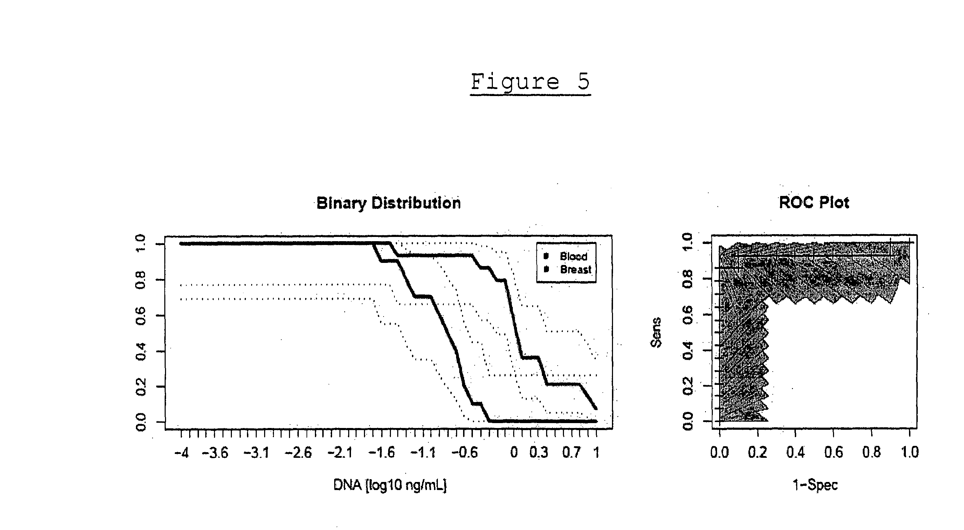

Figure 5

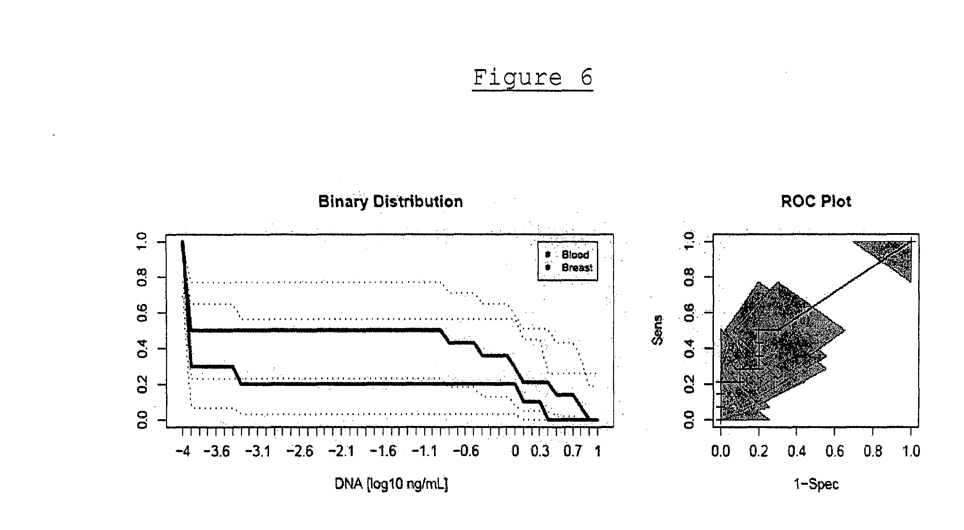

Figure 6

ROC Plot

1-Spec

Sens

Binary Distribution

Blood
Breast

DNA [log10 ng/mL]

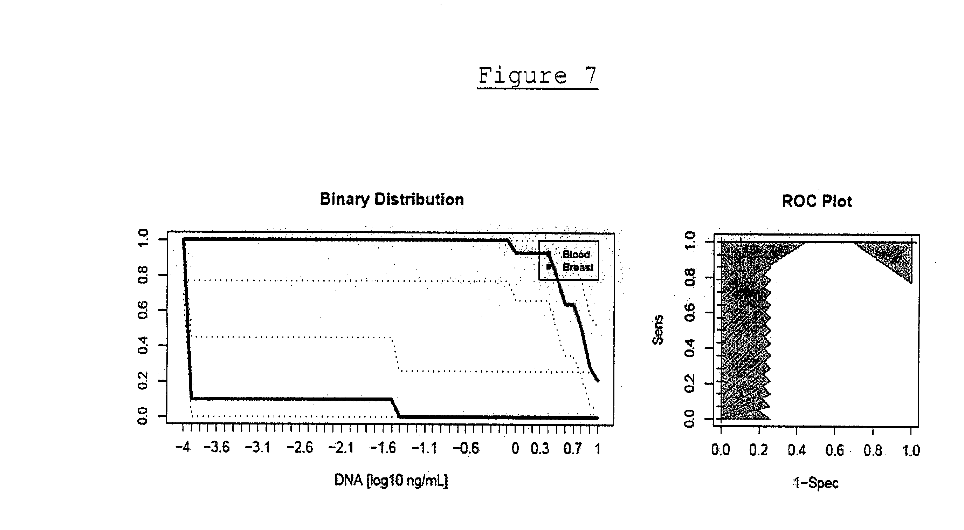

Figure 7

**ROC Plot**

Sens

1-Spec

**Binary Distribution**

Blood
Breast

DNA [log10 ng/mL]

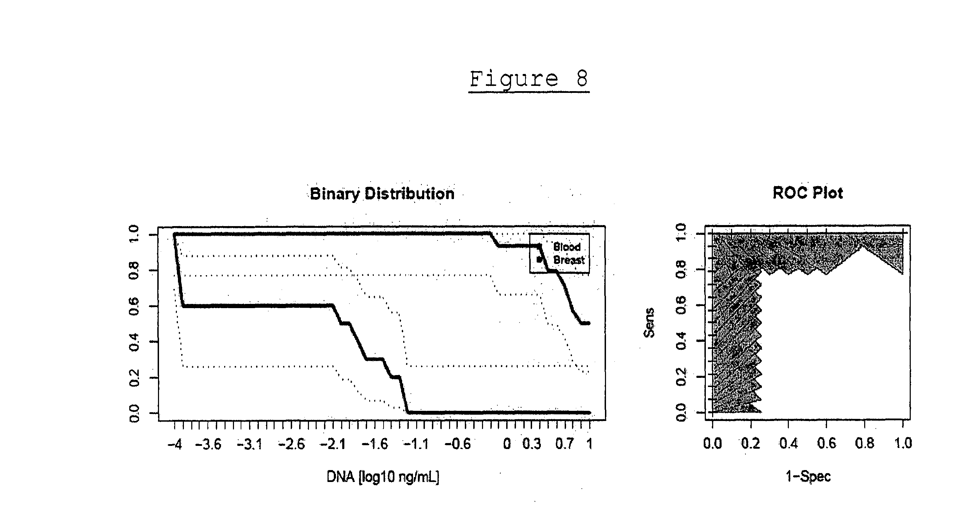

Figure 8

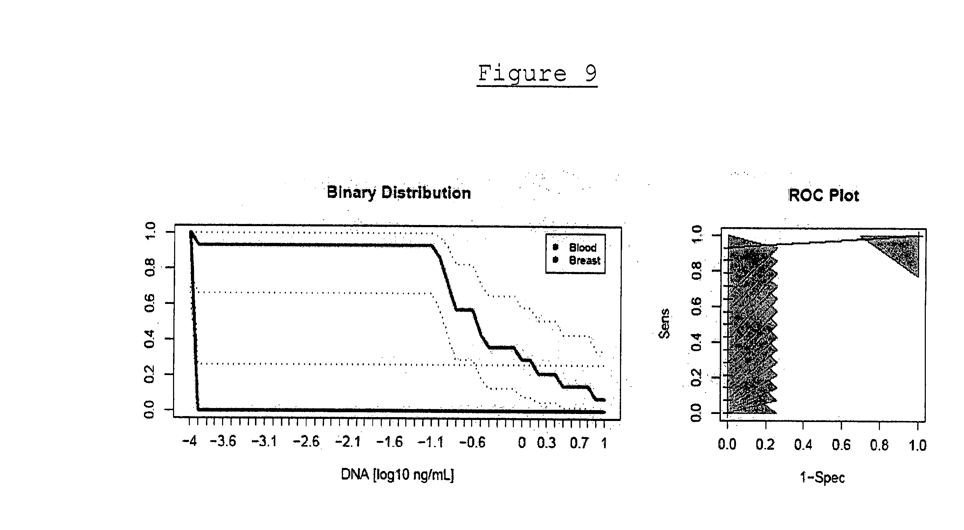

Figure 9

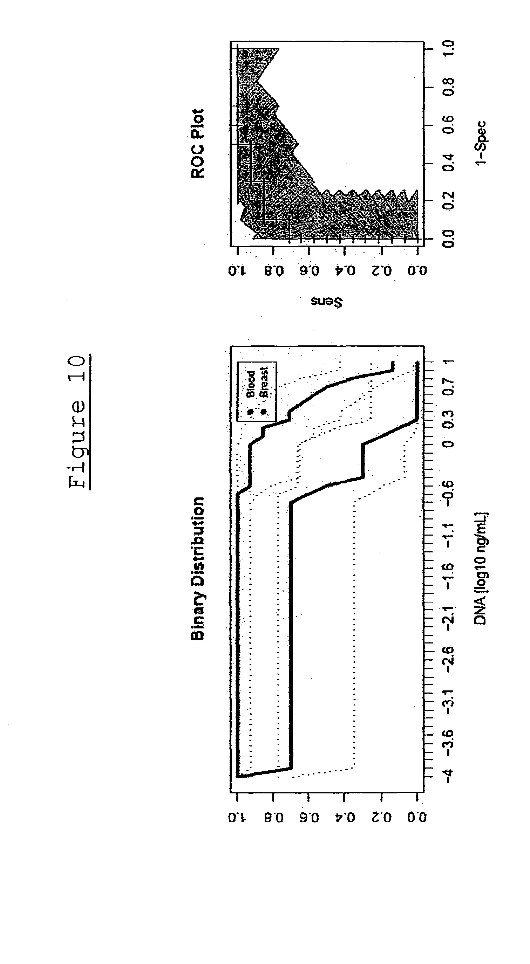

Figure 10

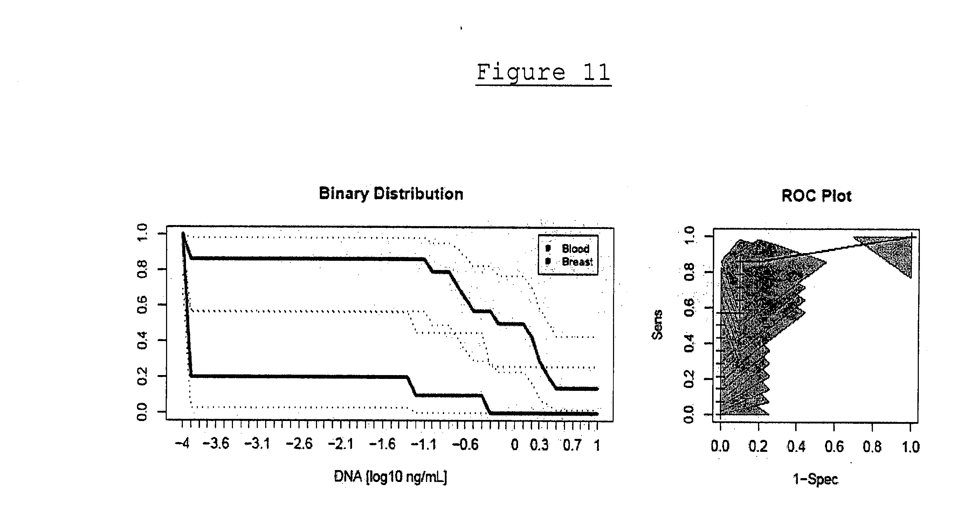

Figure 11

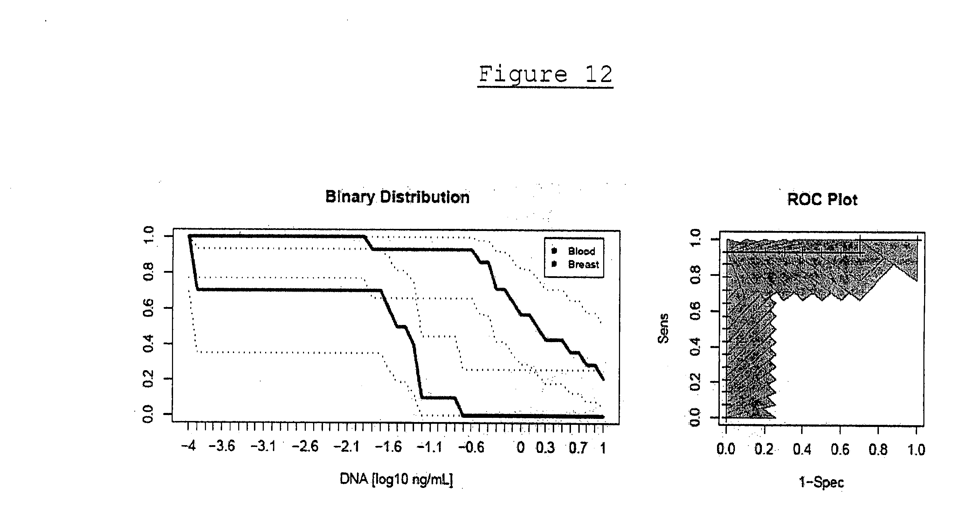

Figure 12

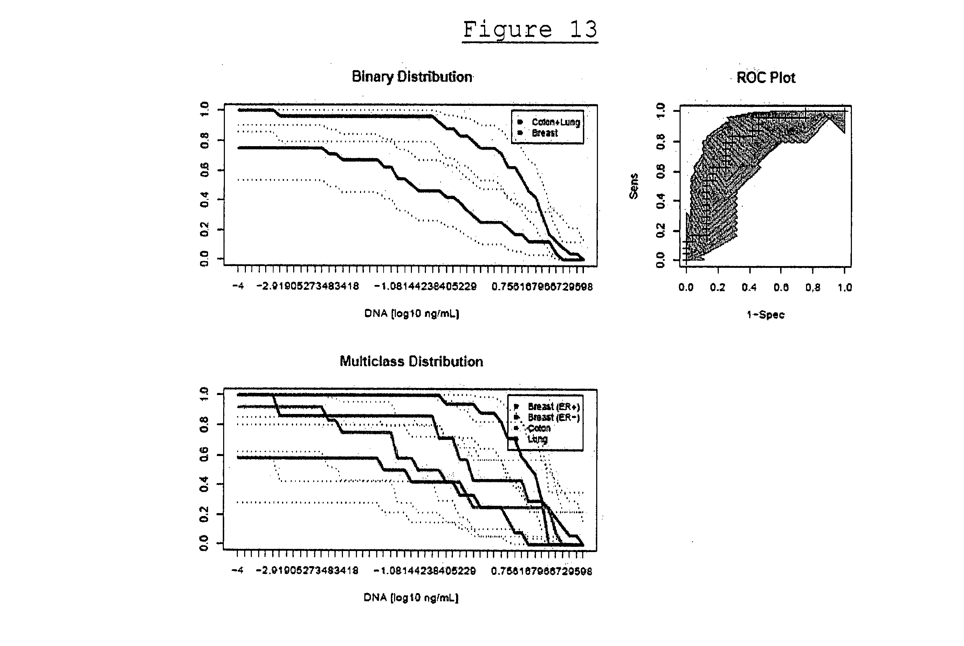

Figure 13

Binary Distribution

Multiclass Distribution

ROC Plot

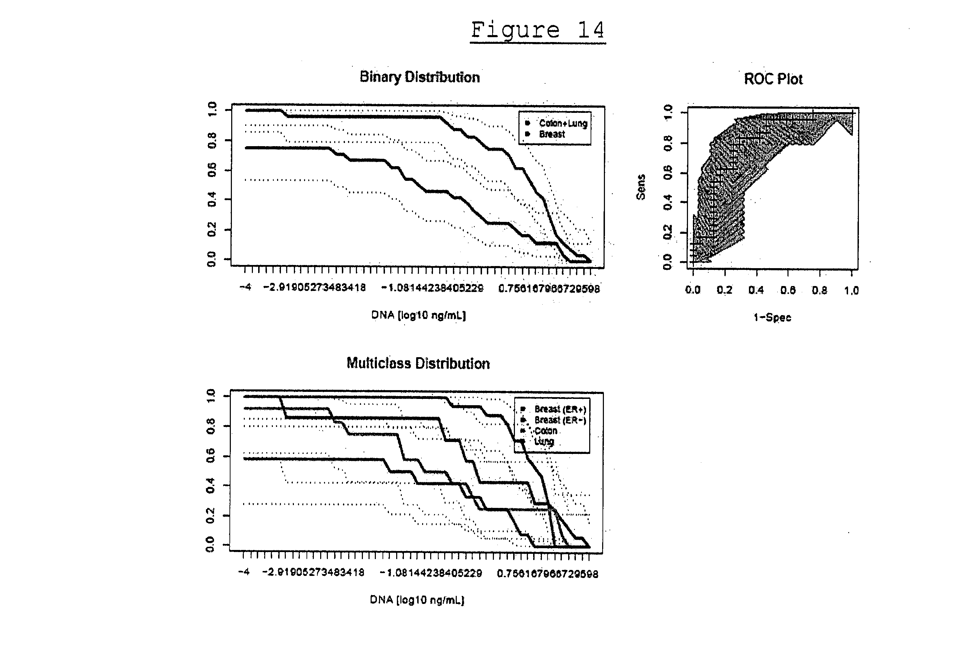

Figure 14

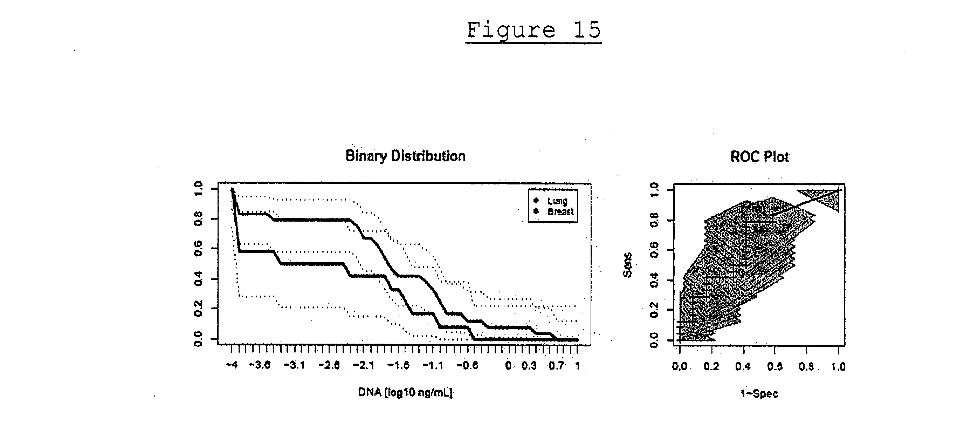

Figure 15

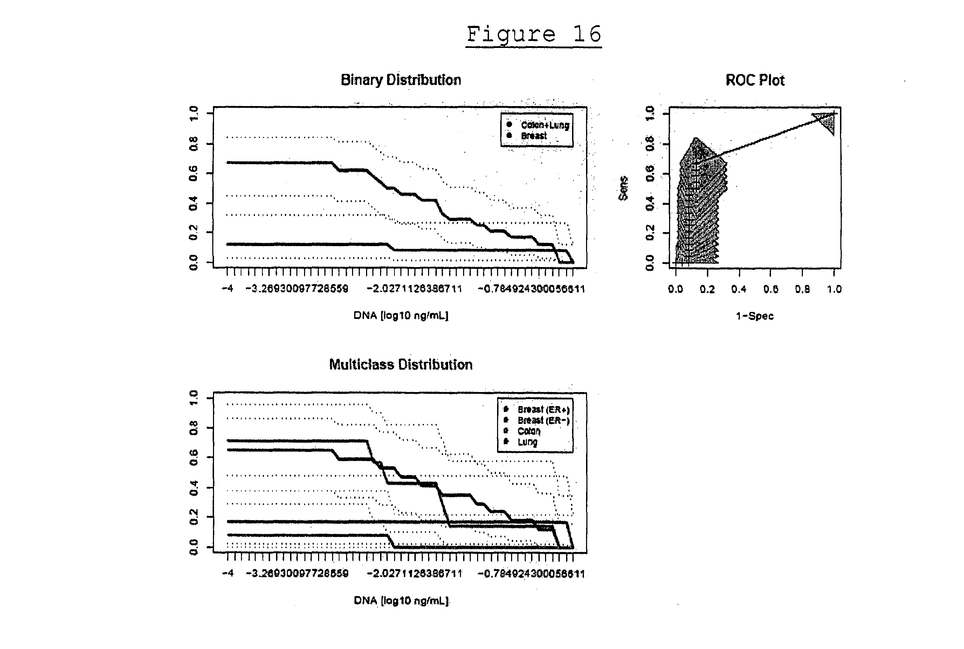

# Figure 16

**Binary Distribution**

ROC Plot

**Multiclass Distribution**

EP 2 302 073 A1

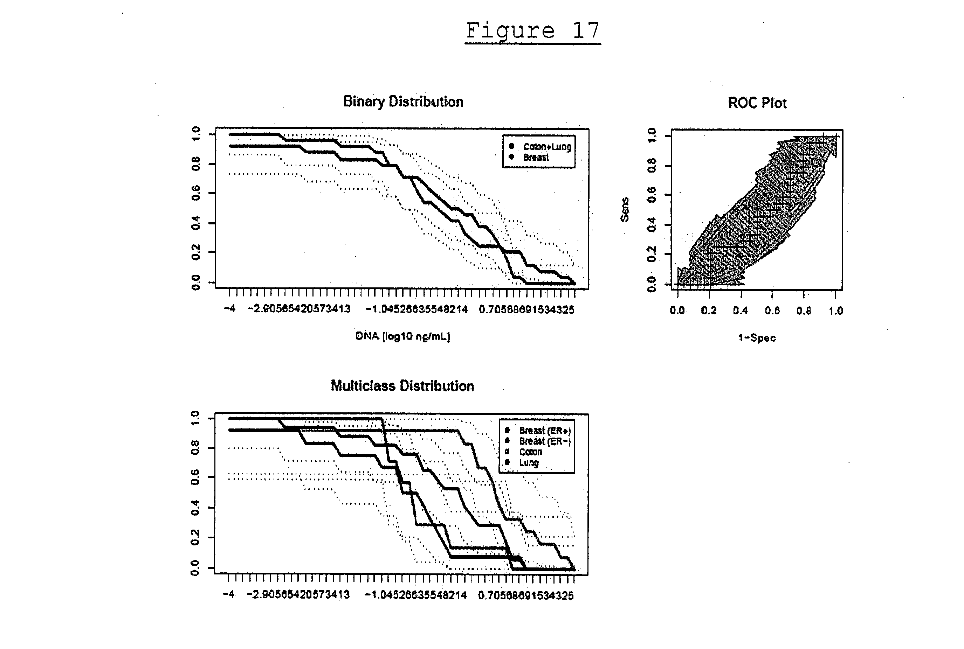

Figure 17

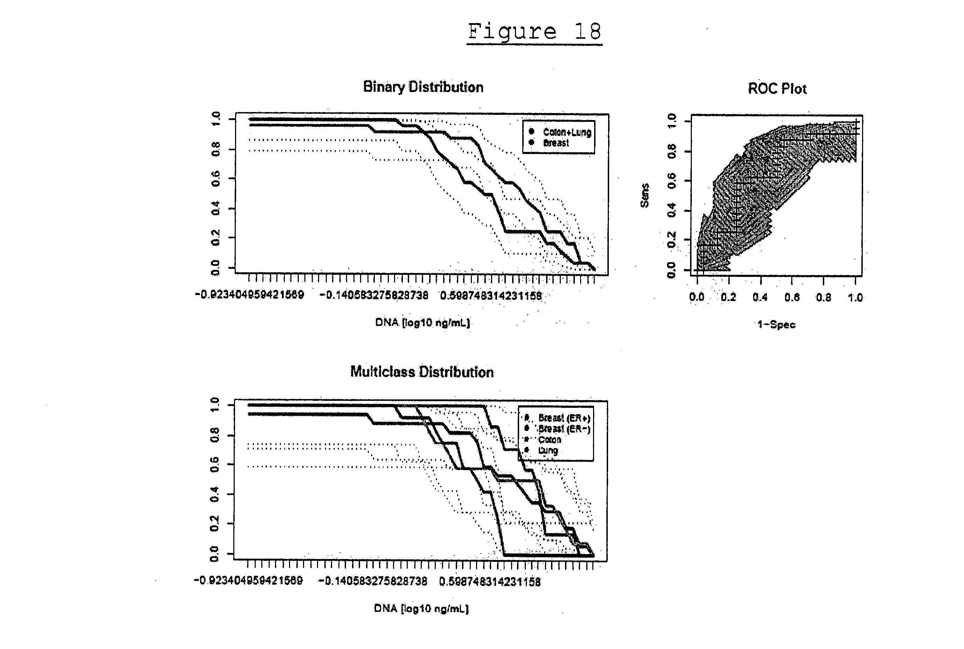

Figure 18

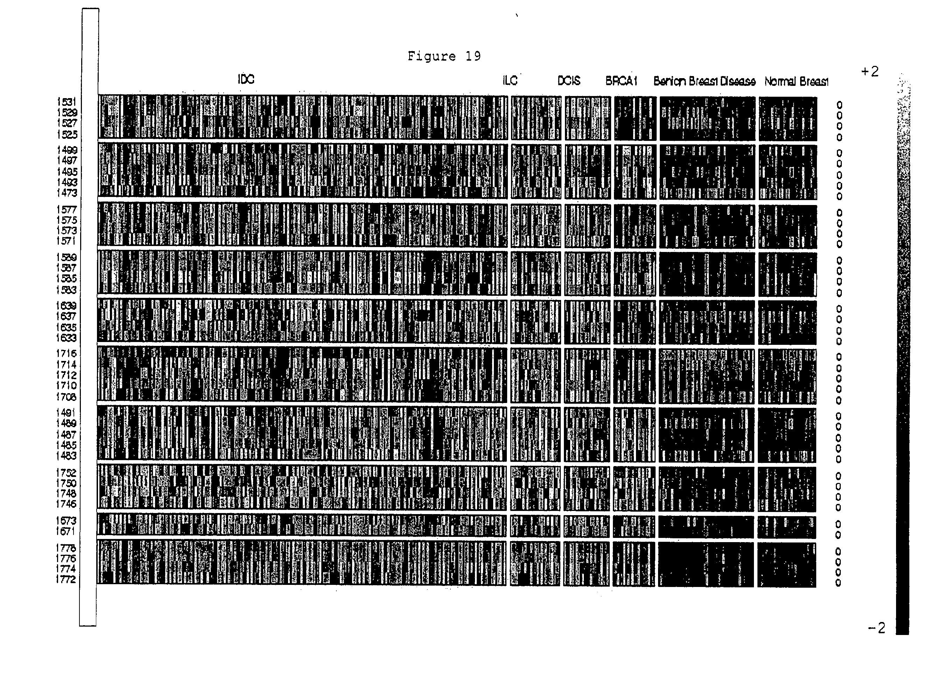

Figure 19

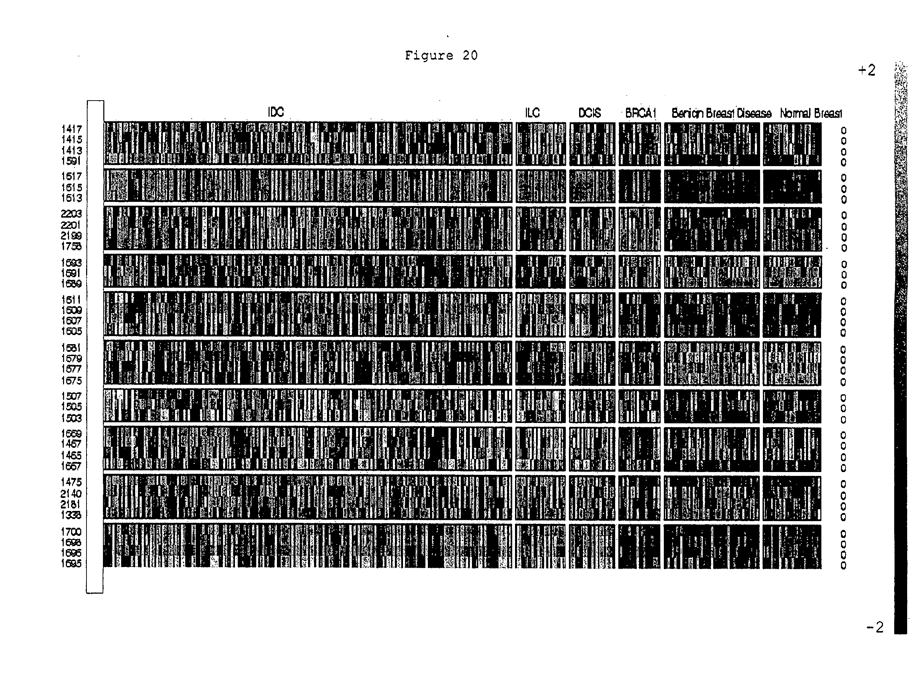

Figure 20

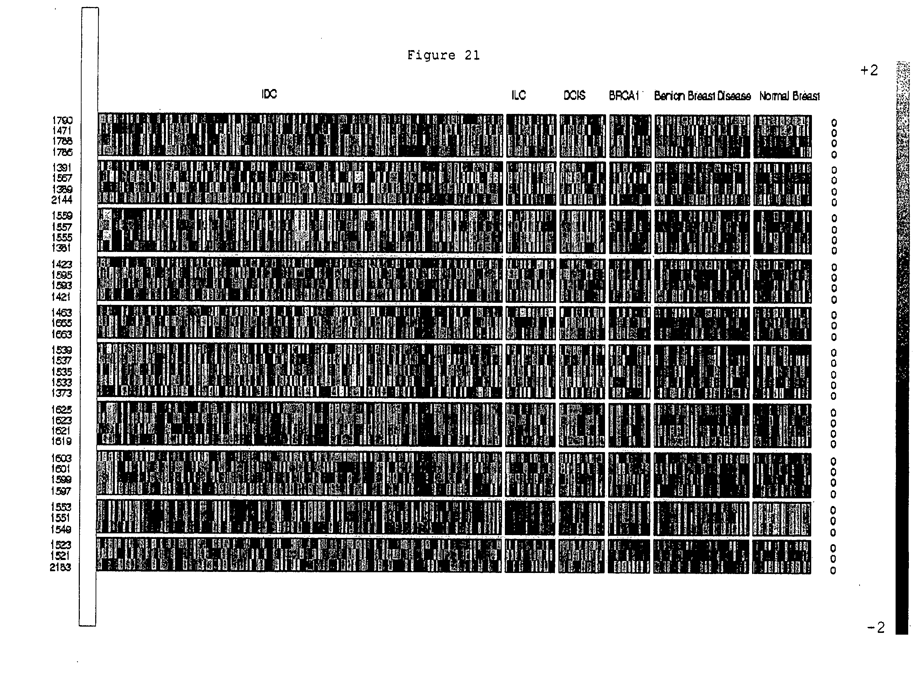

Figure 21

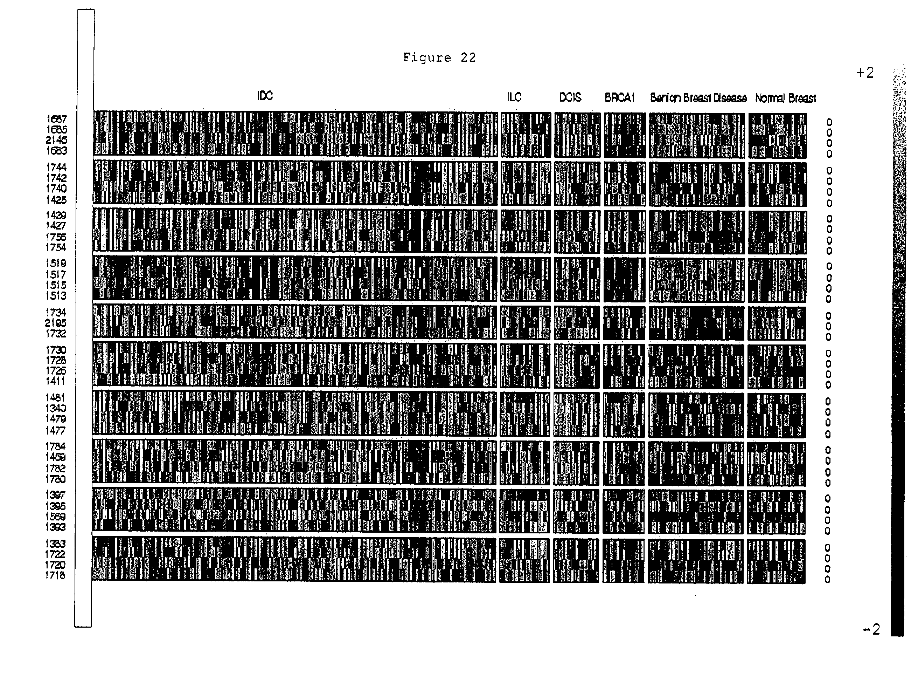

Figure 22

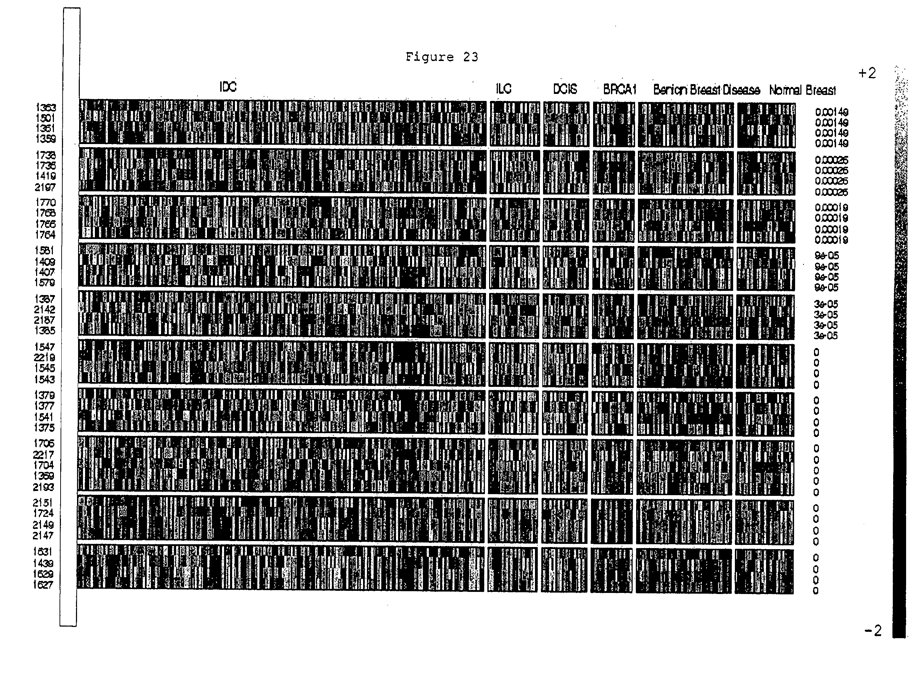

Figure 23

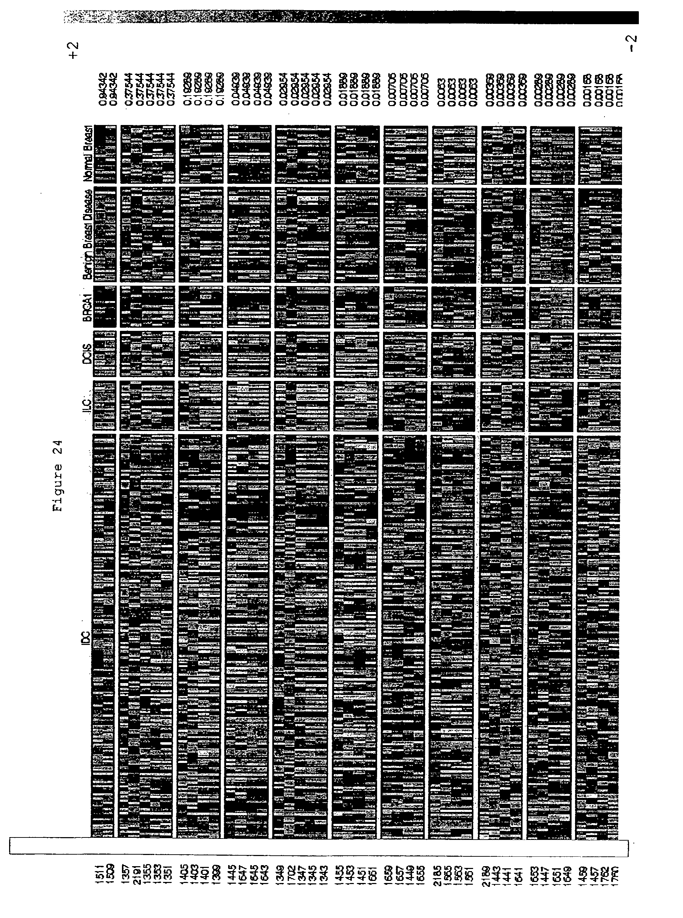

Figure 24

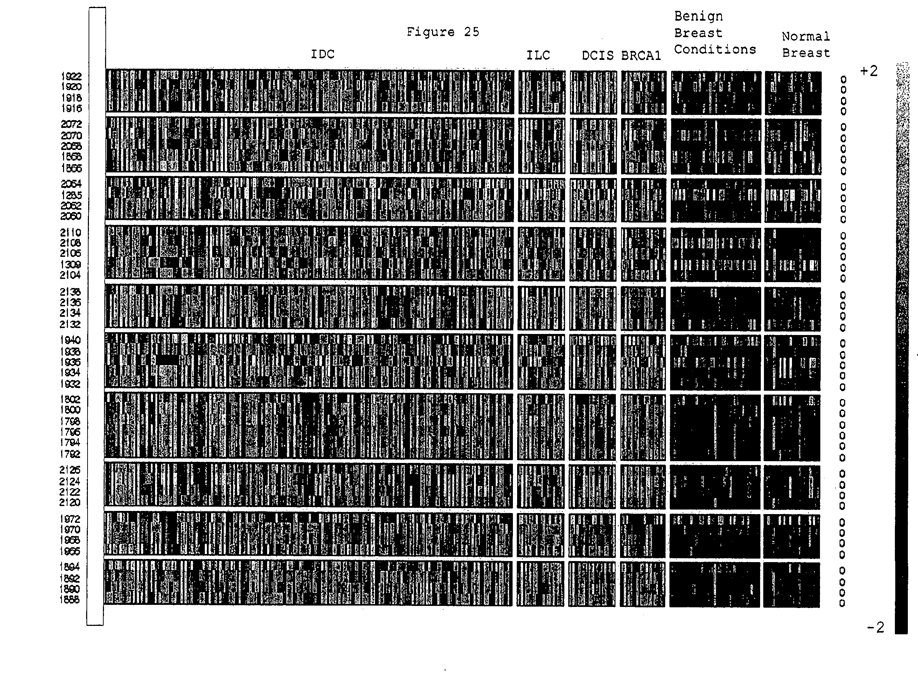

Figure 25

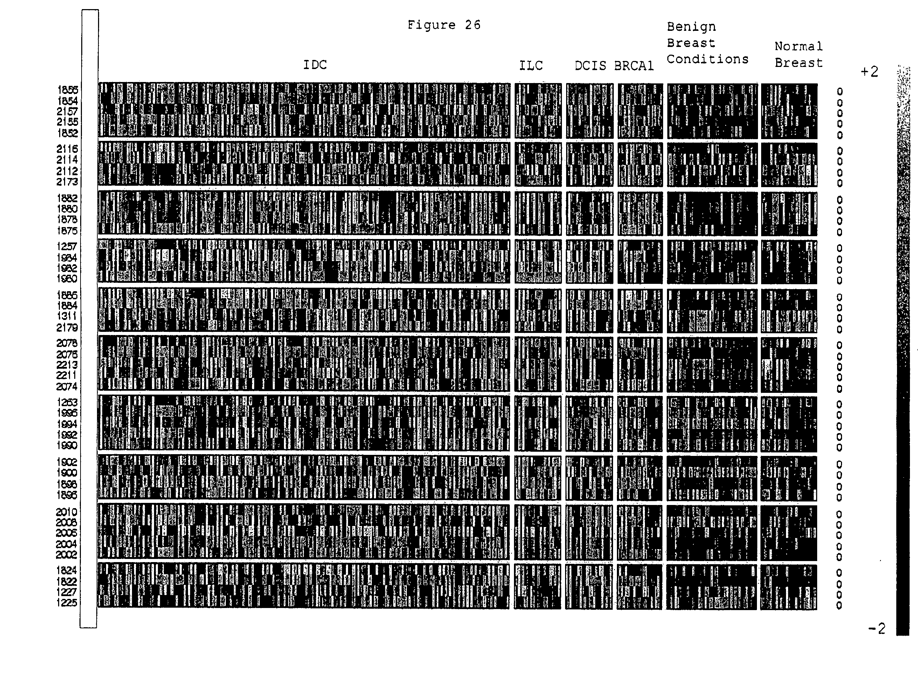

Figure 26

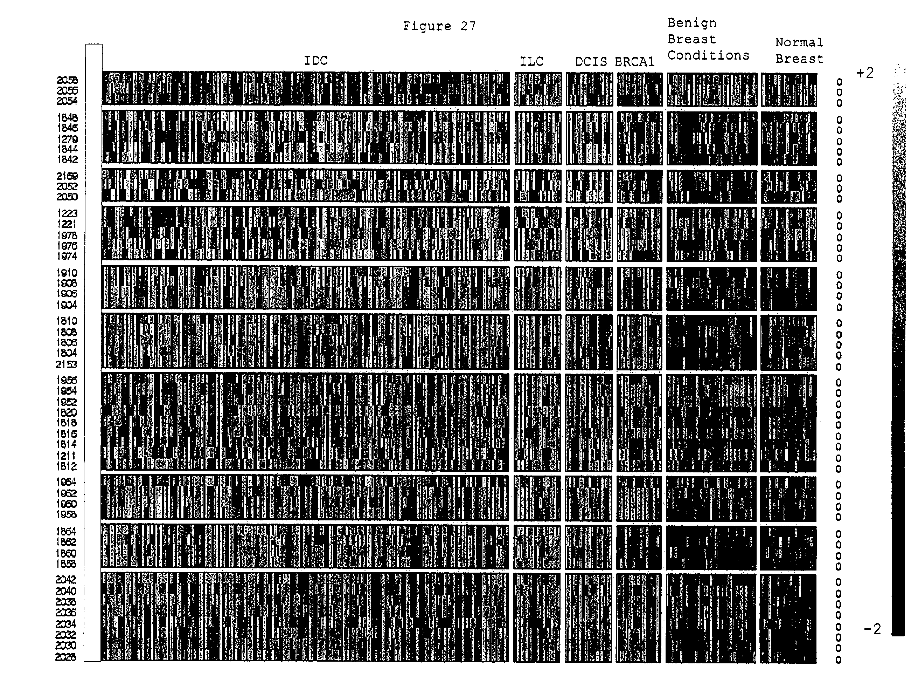

Figure 27

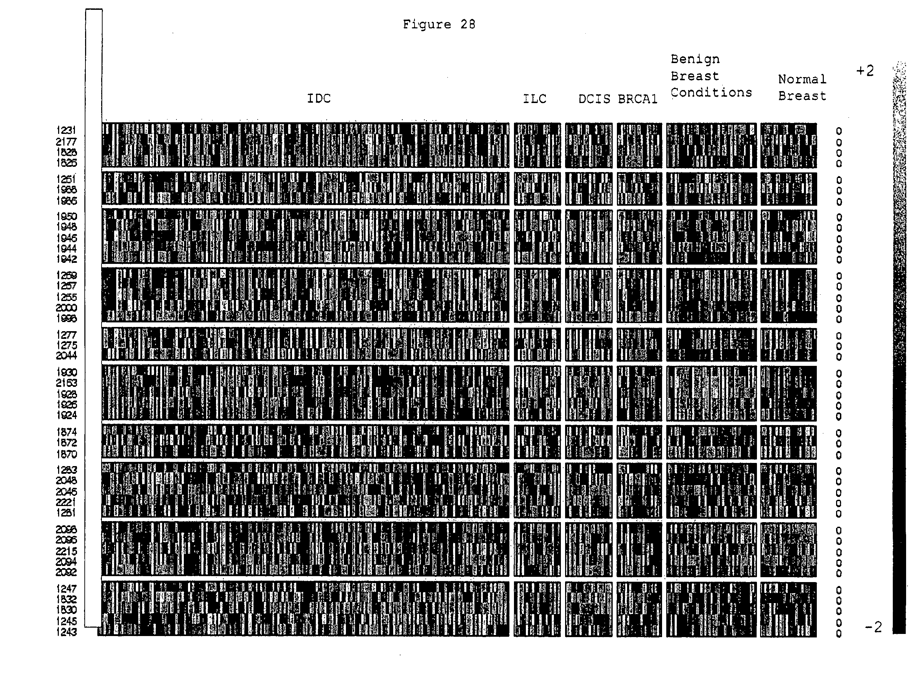

Figure 28

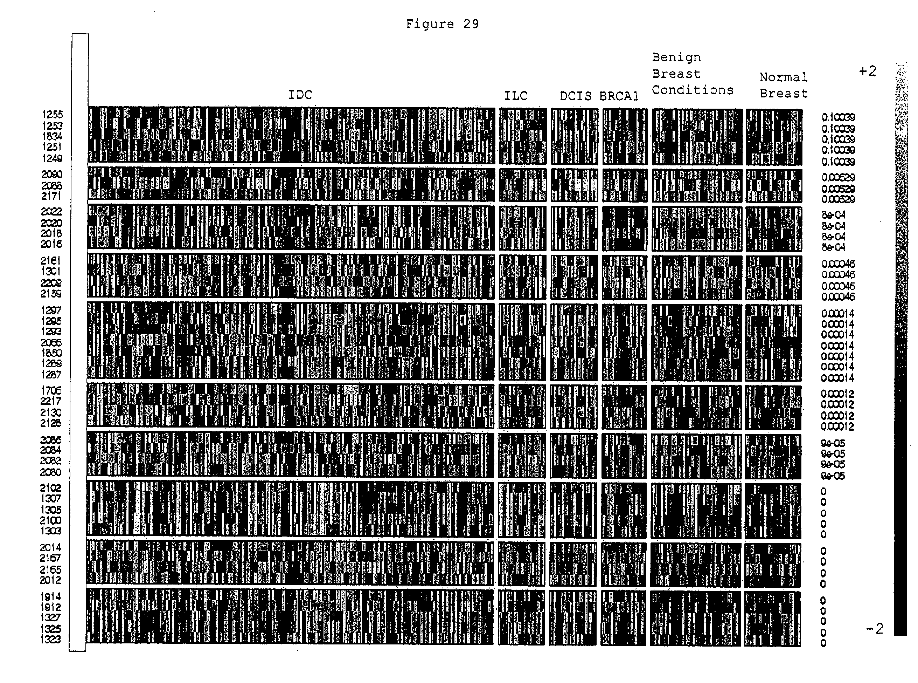

Figure 29

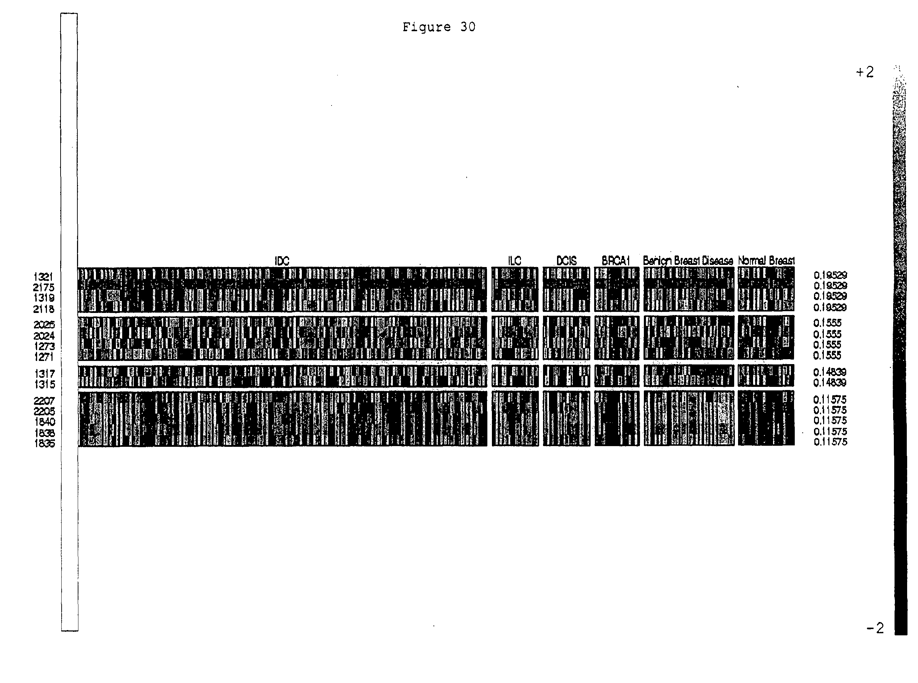

Figure 30

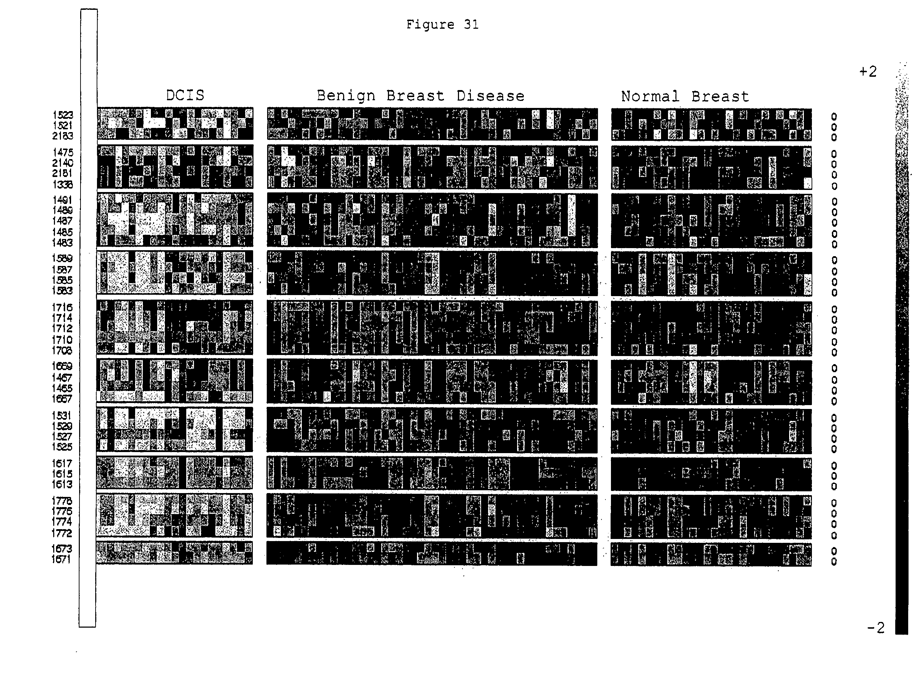

Figure 31

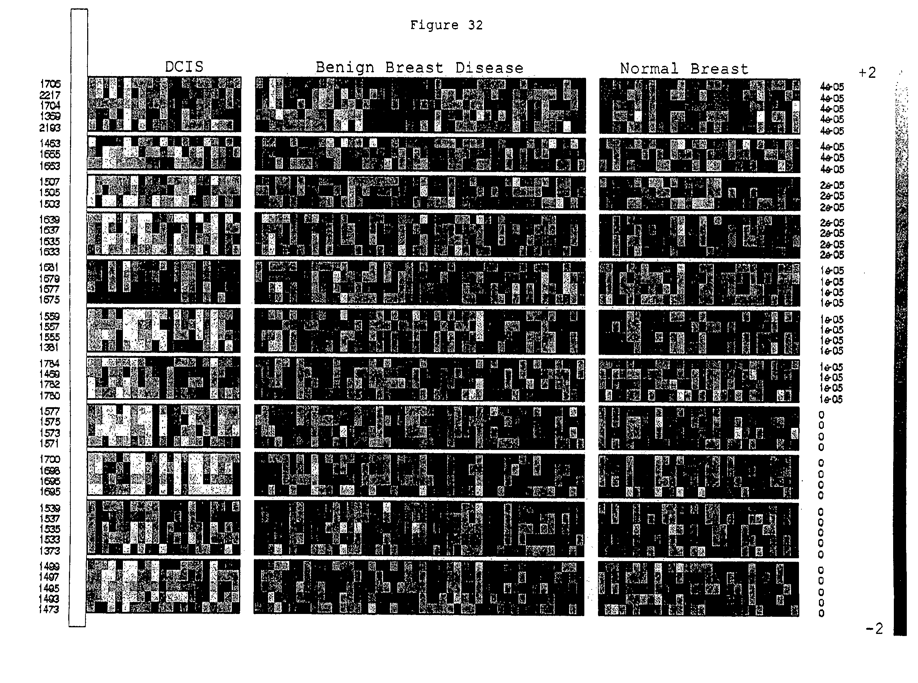

Figure 32

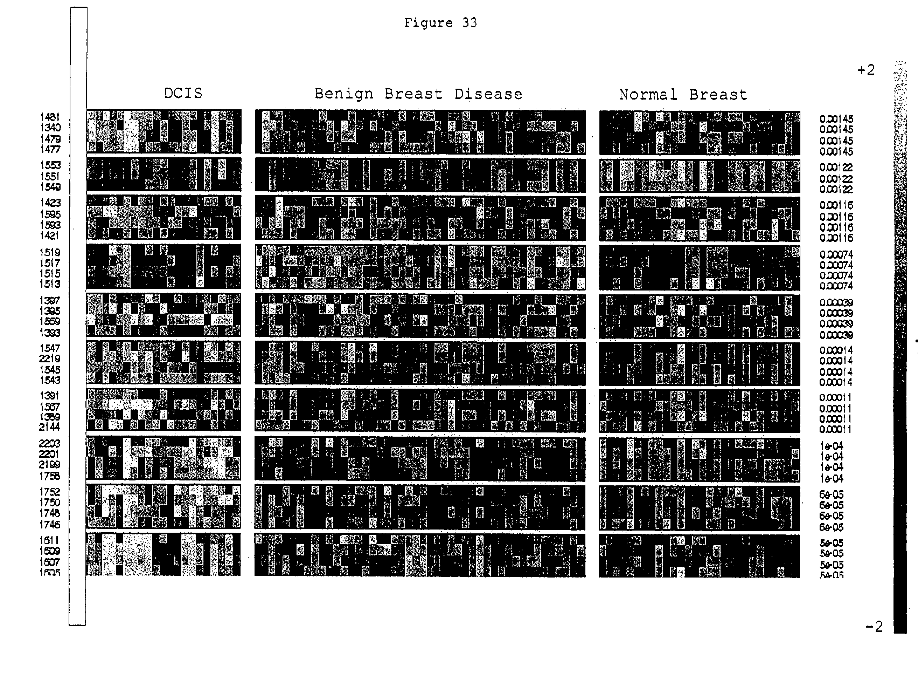

Figure 33

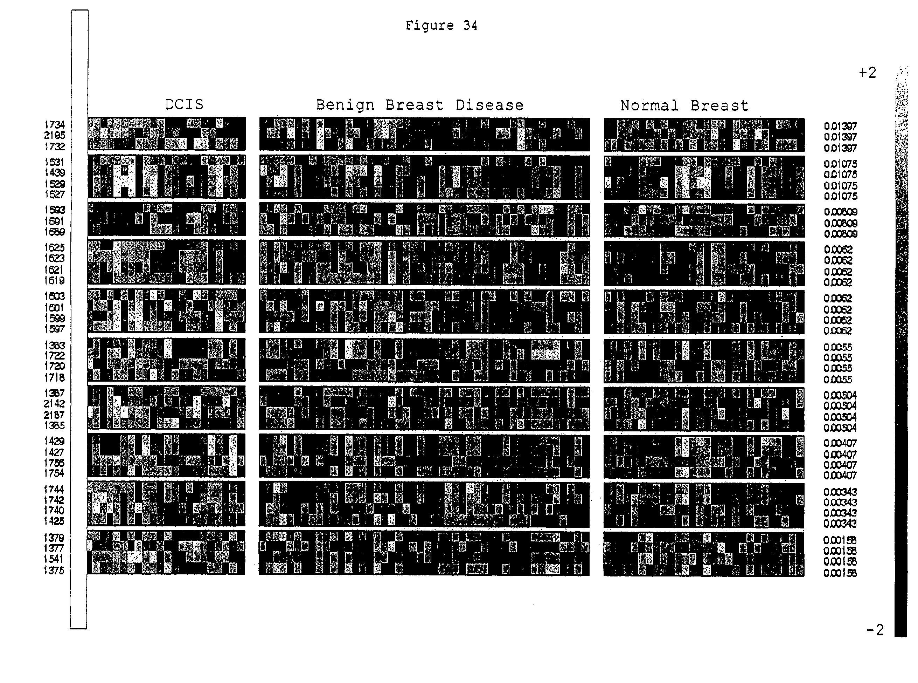

Figure 34

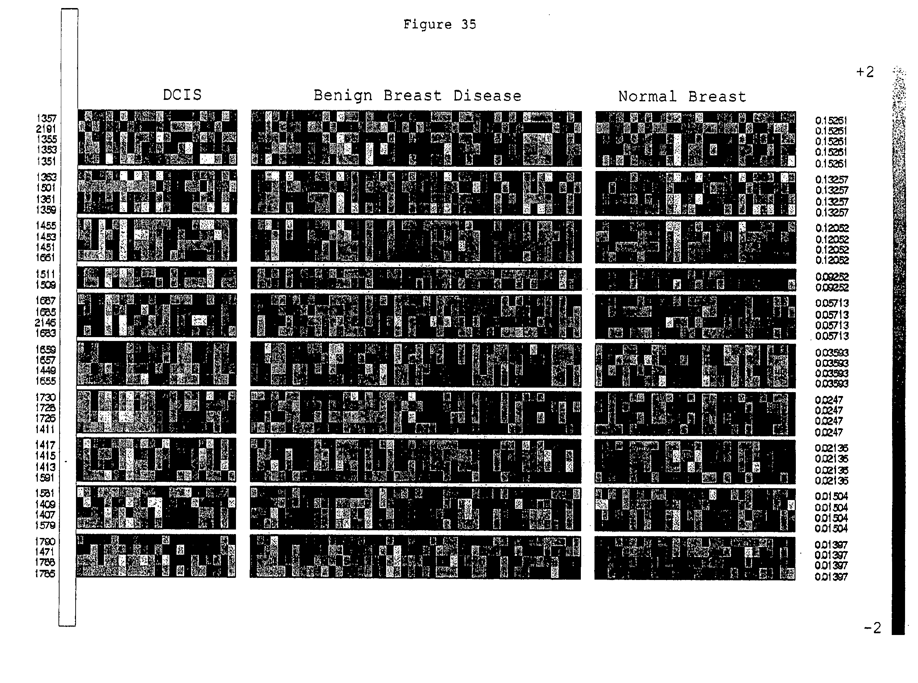

Figure 35

EP 2 302 073 A1

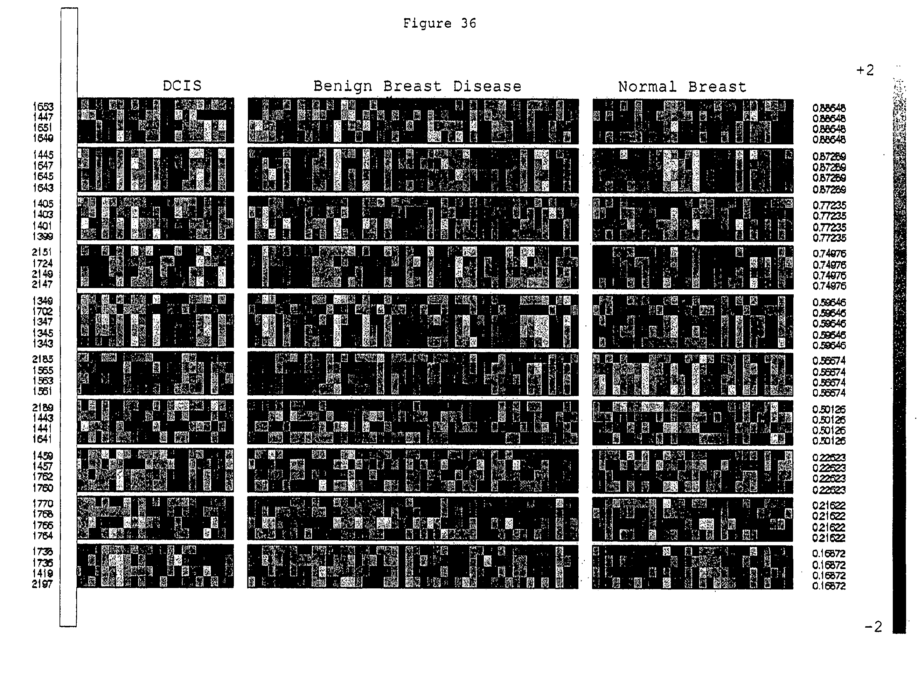

Figure 36

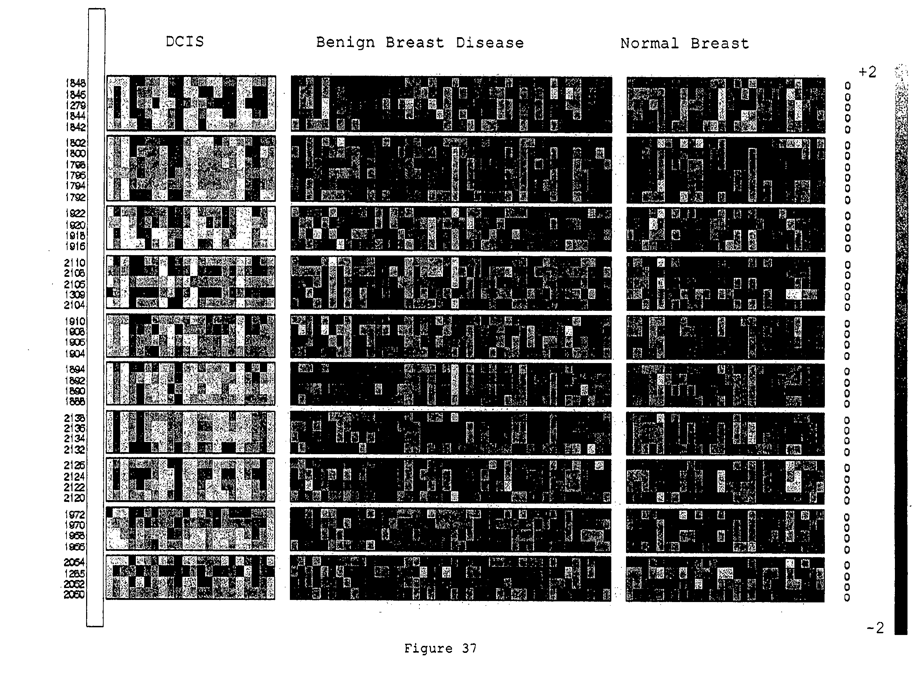

Figure 37

DCIS    Benign Breast Disease    Normal Breast

+2

-2

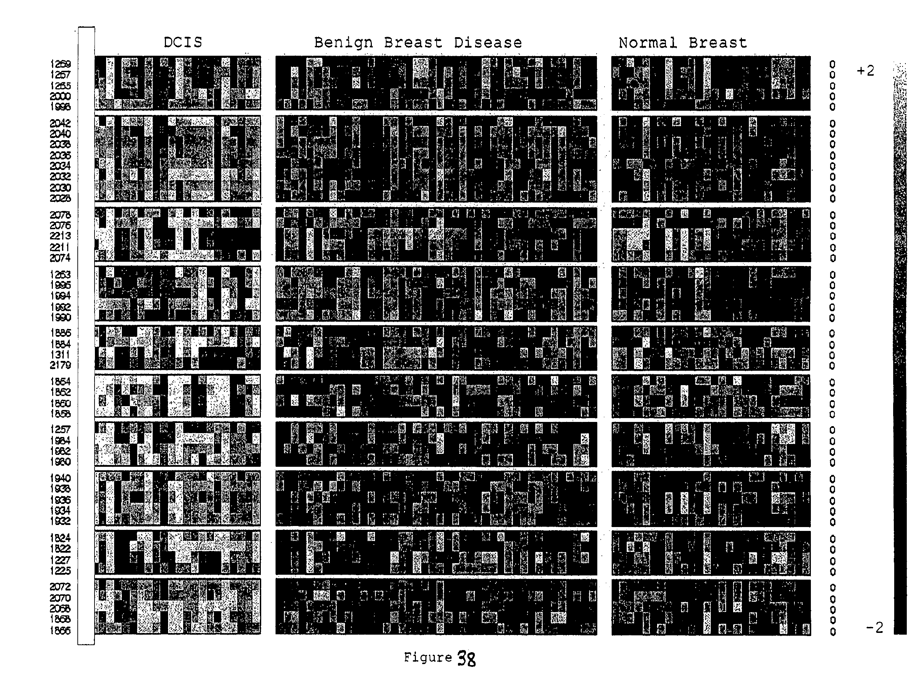

Figure 38

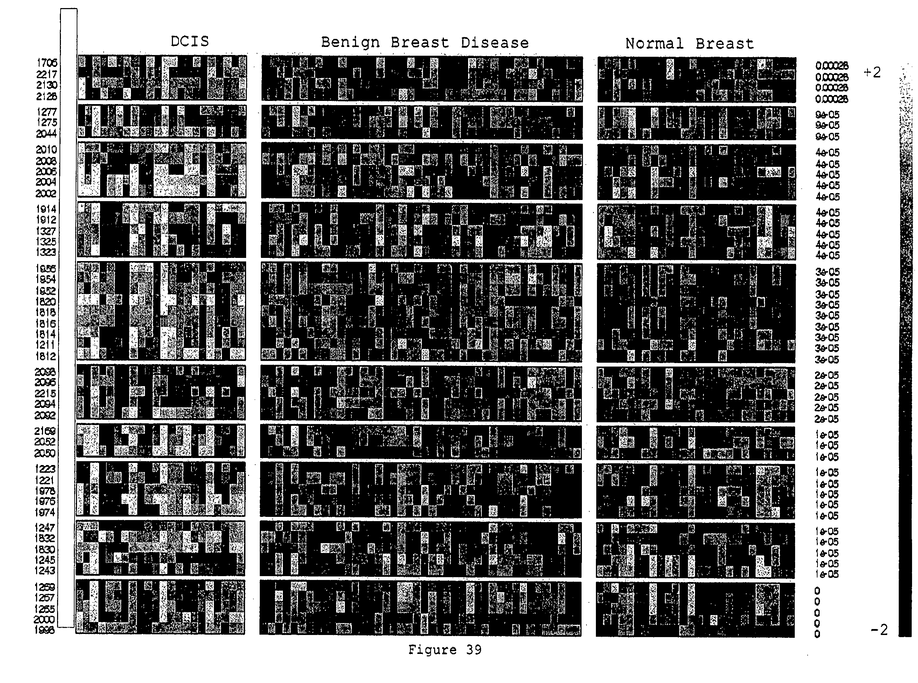

Figure 39

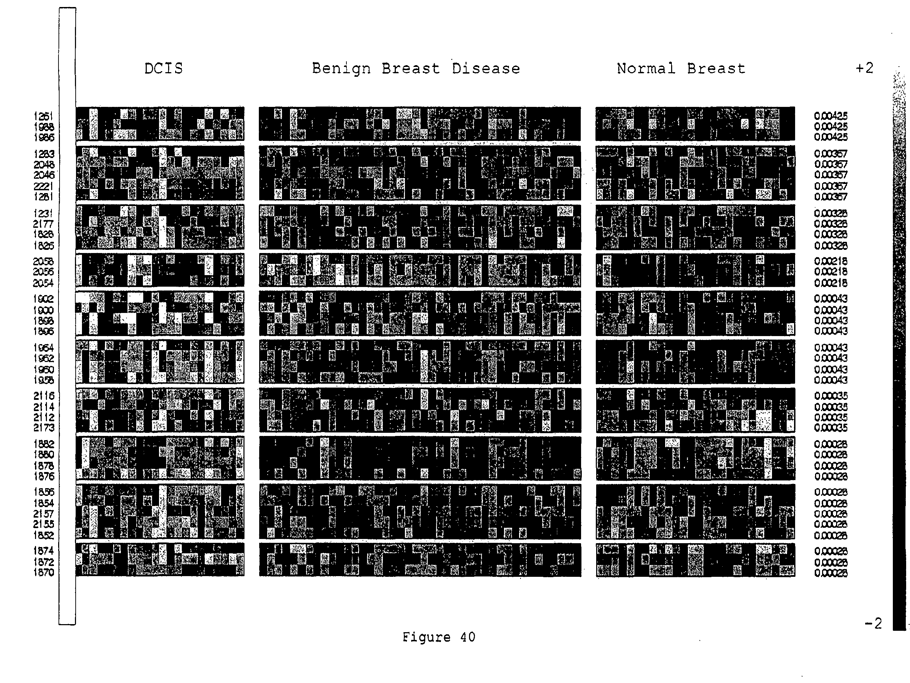

Figure 40

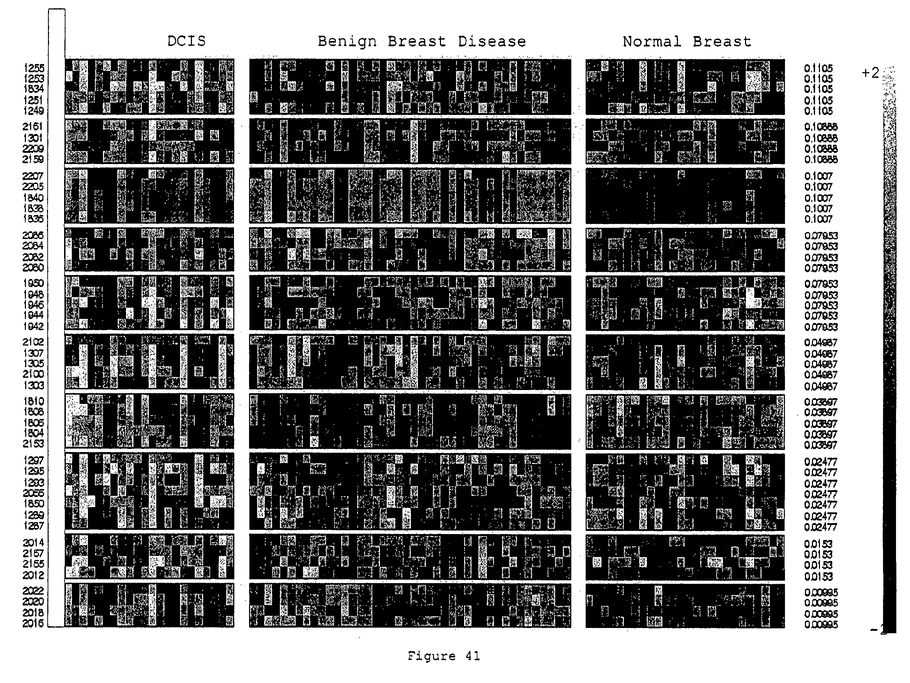

Figure 41

EP 2 302 073 A1

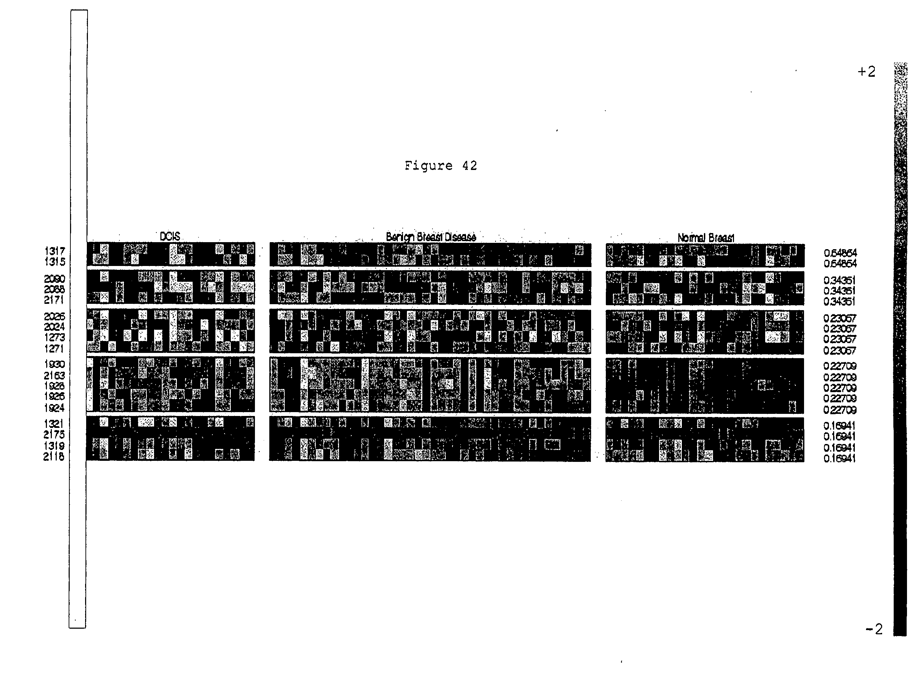

Figure 42

+2

-2

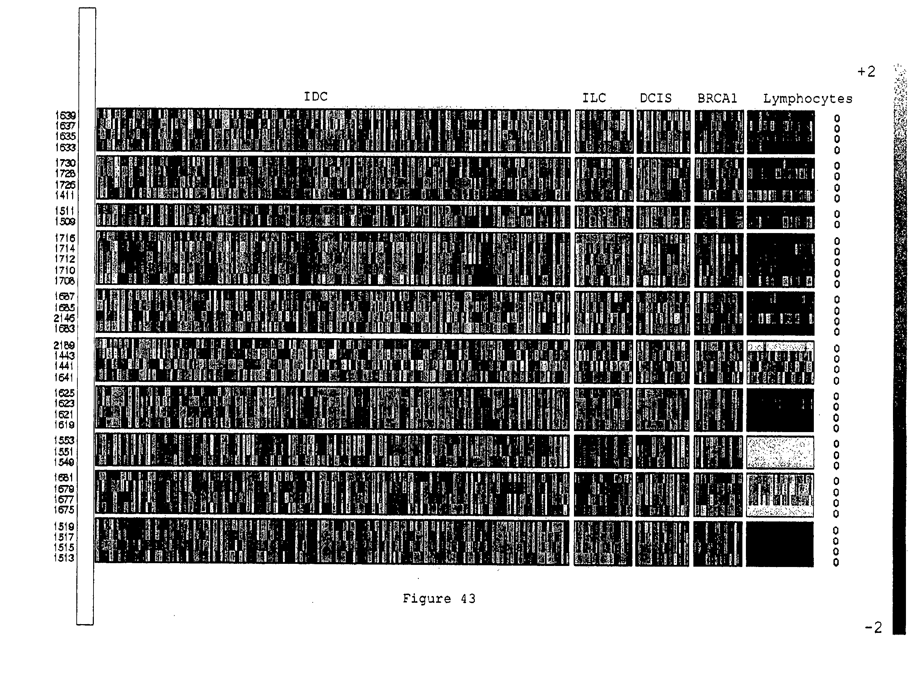

Figure 43

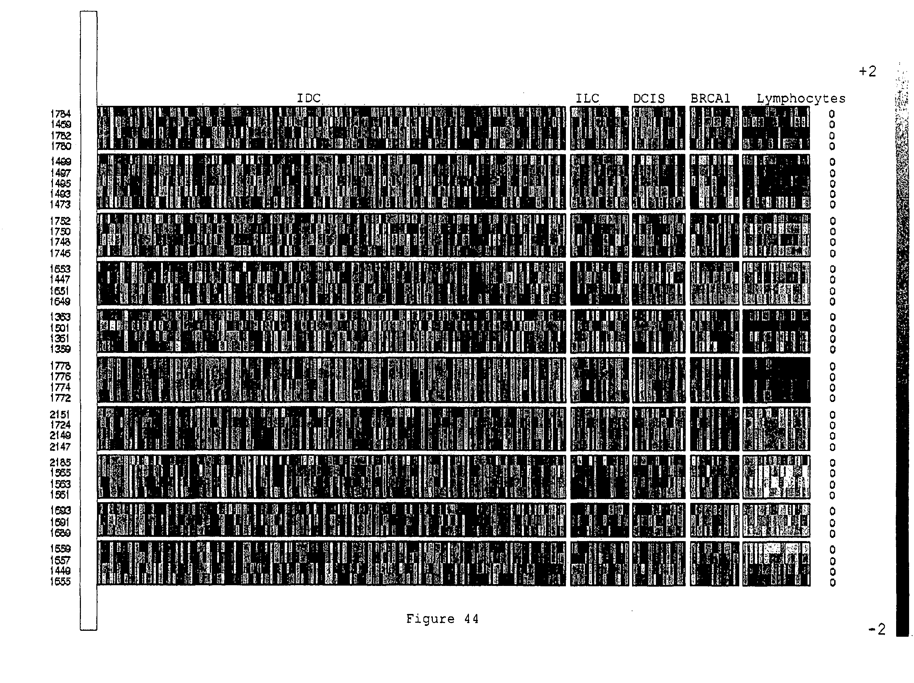

Figure 44

IDC  ILC  DCIS  BRCA1  Lymphocytes

+2

−2

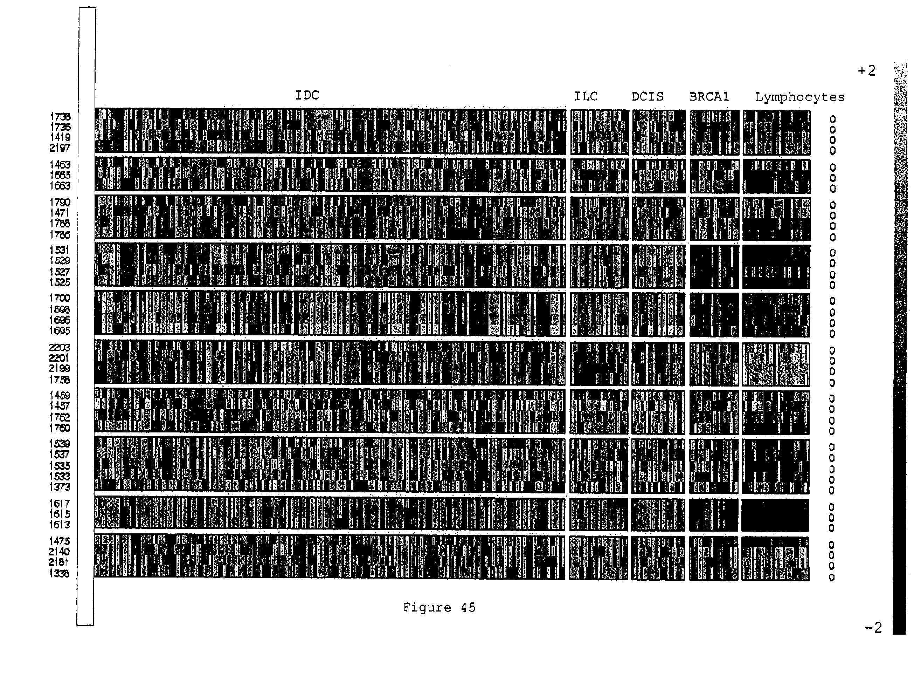

Figure 45

EP 2 302 073 A1

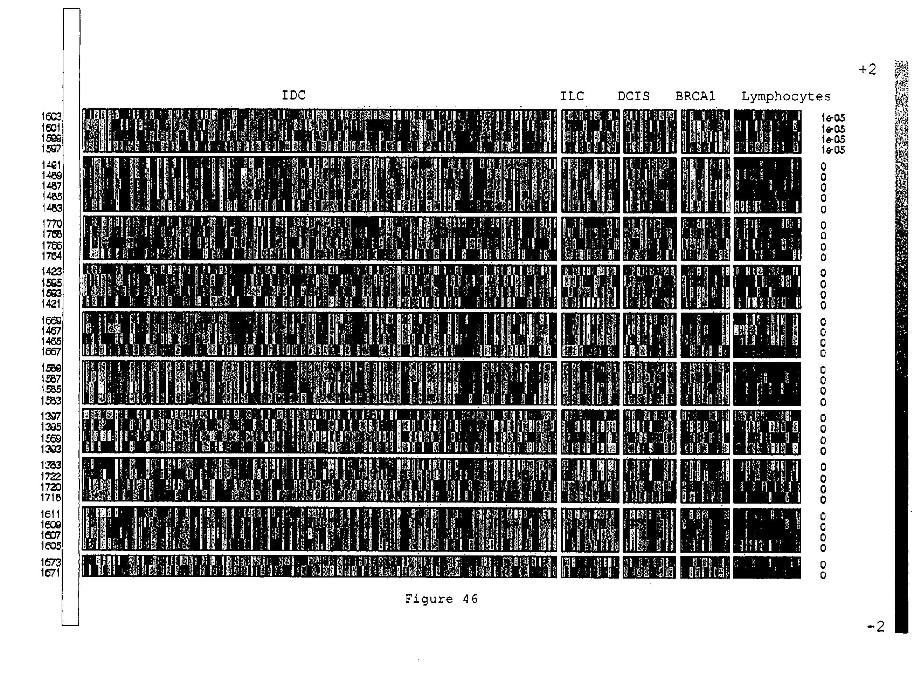

Figure 46

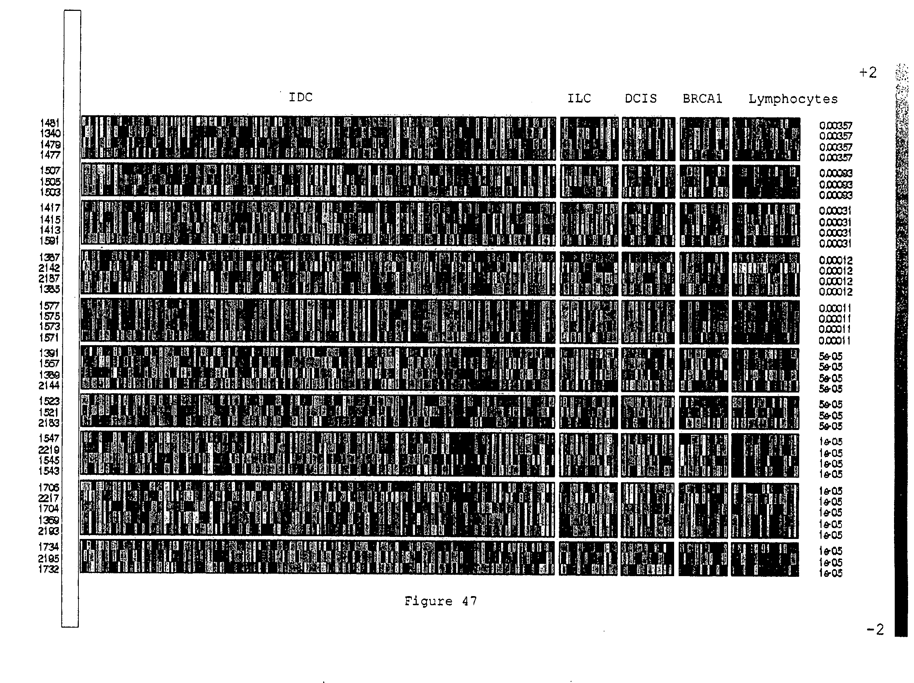

Figure 47

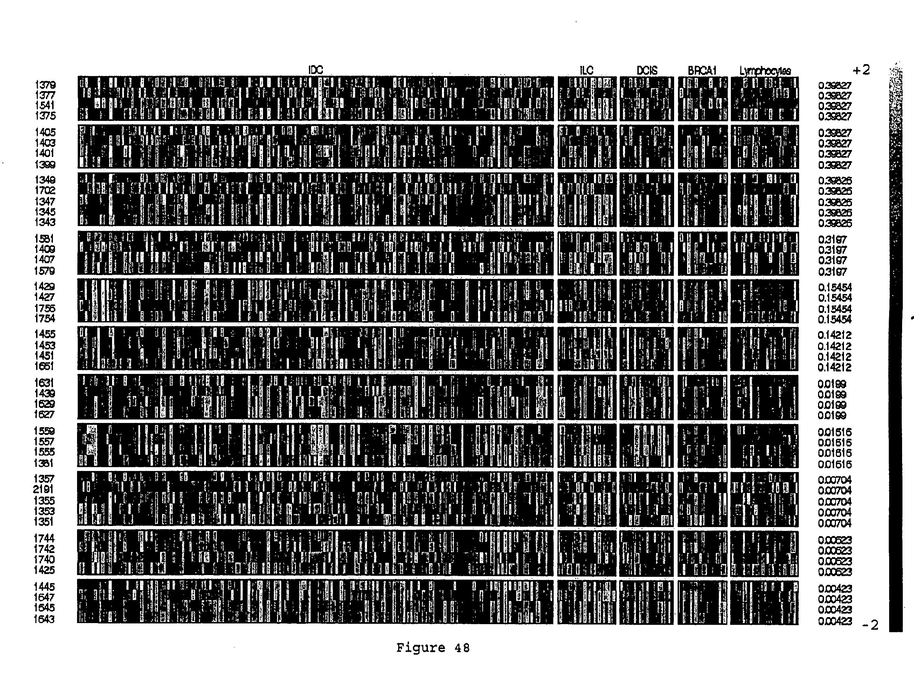

Figure 48

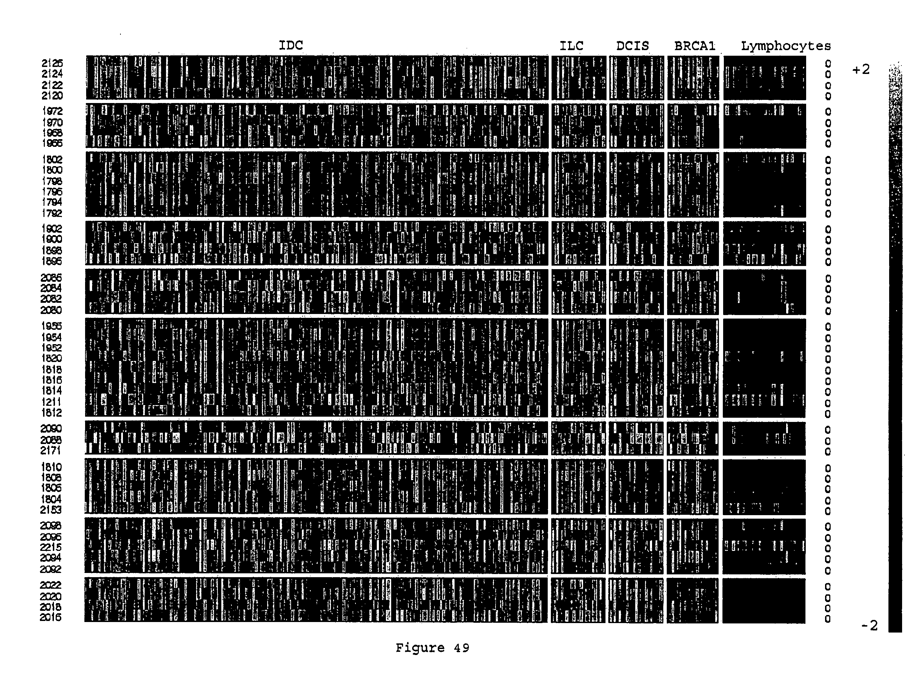

Figure 49

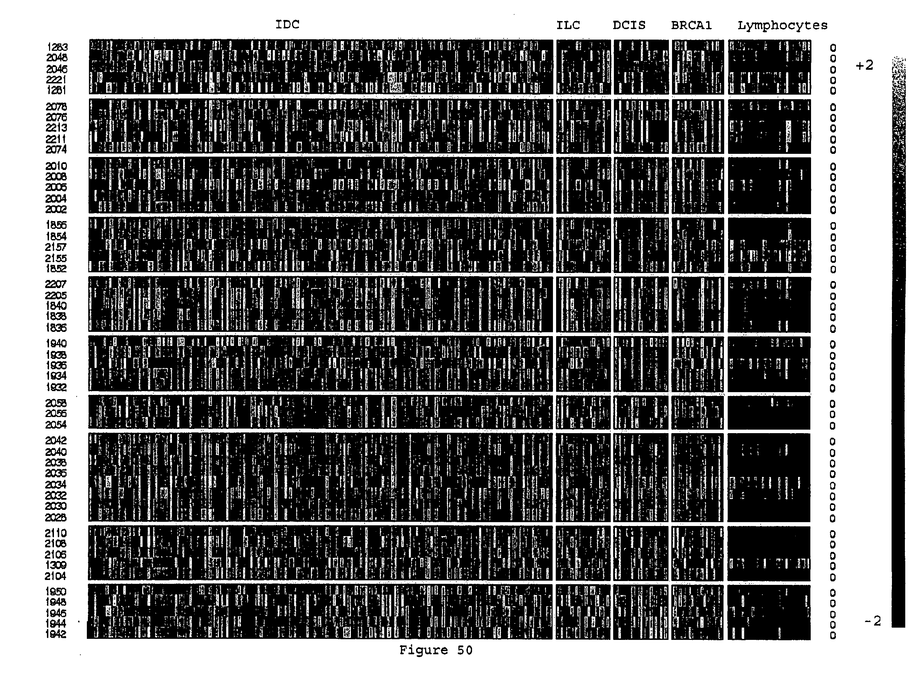

Figure 50

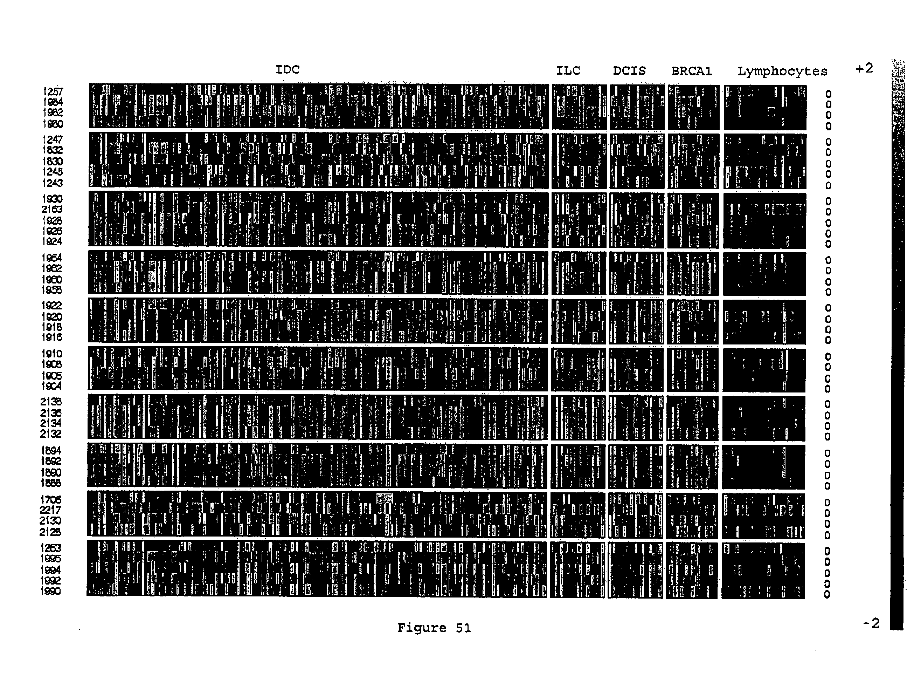

Figure 51

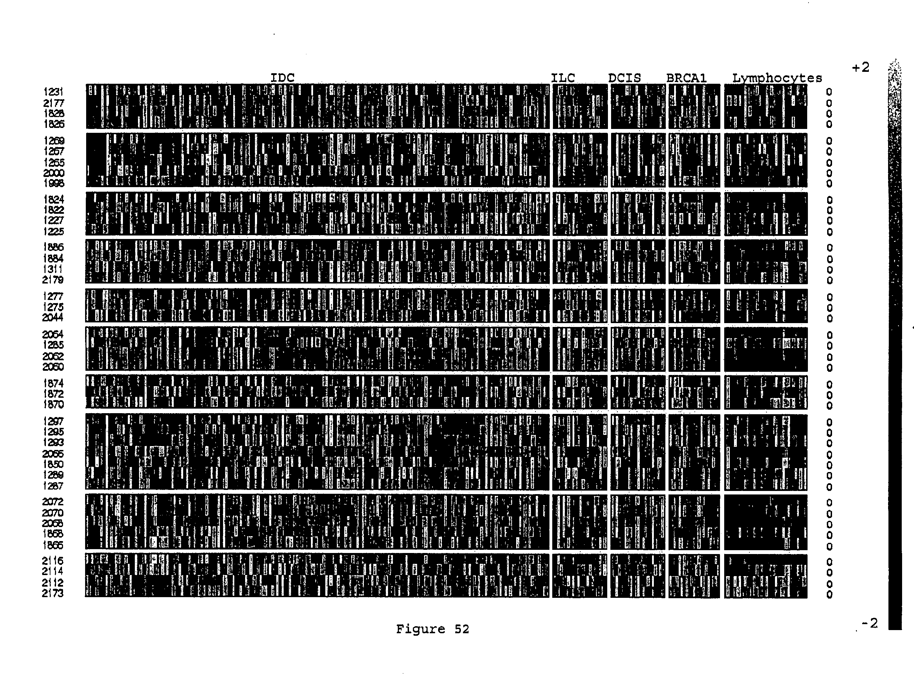

Figure 52

273

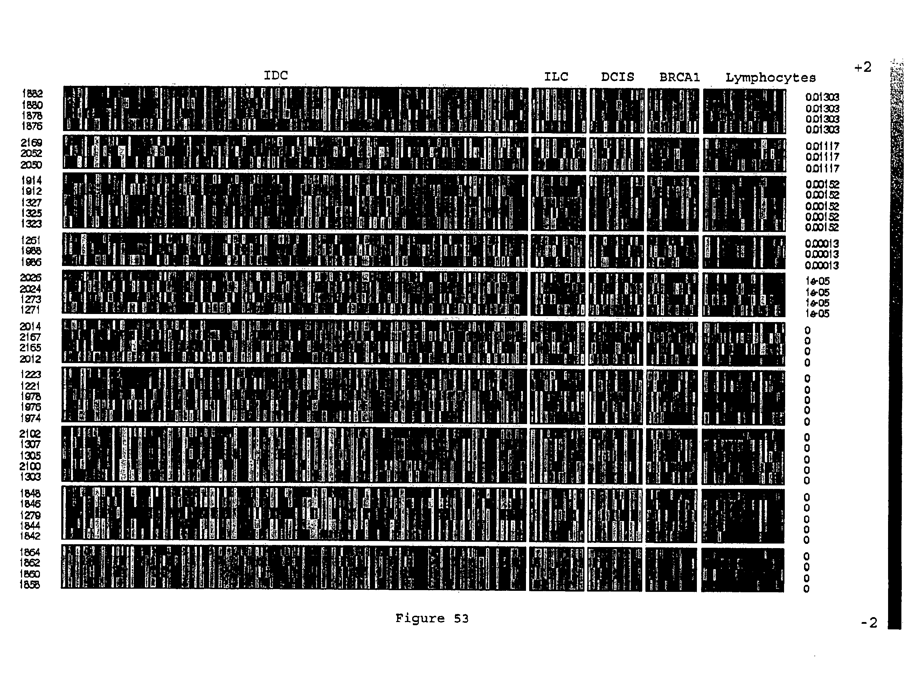

Figure 53

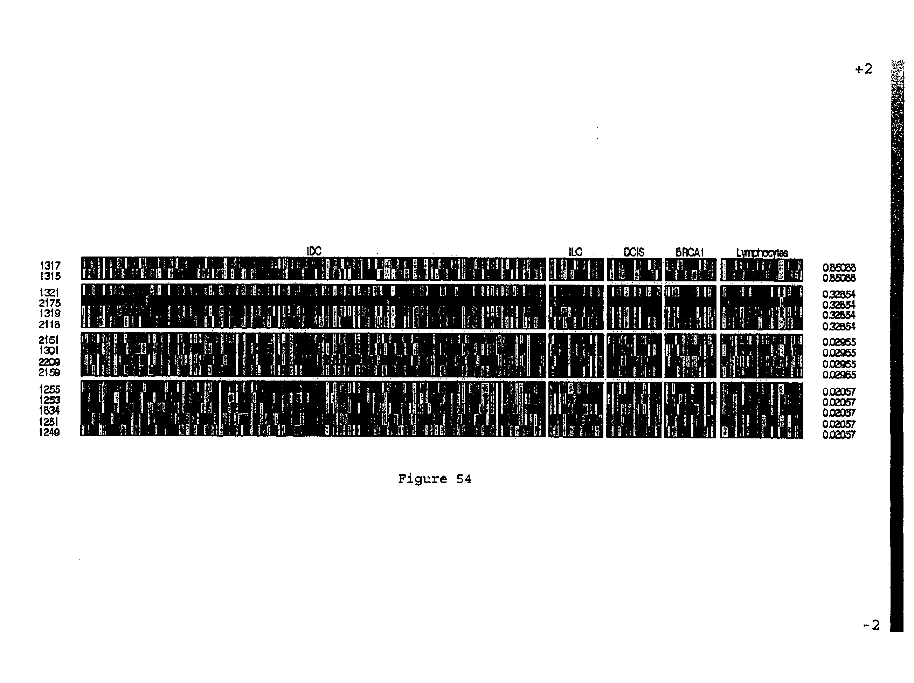

Figure 54

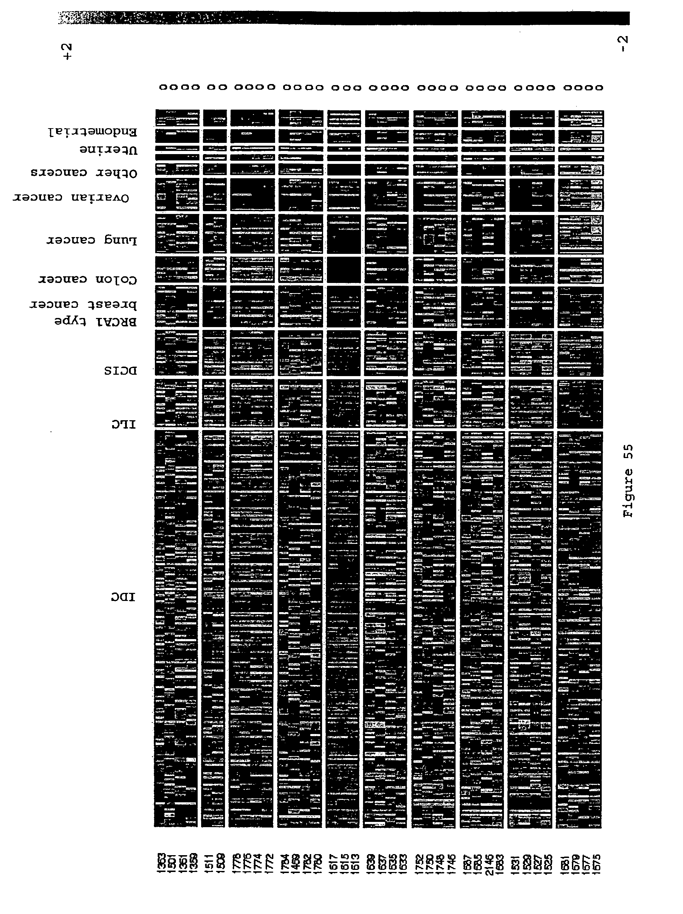

Figure 55

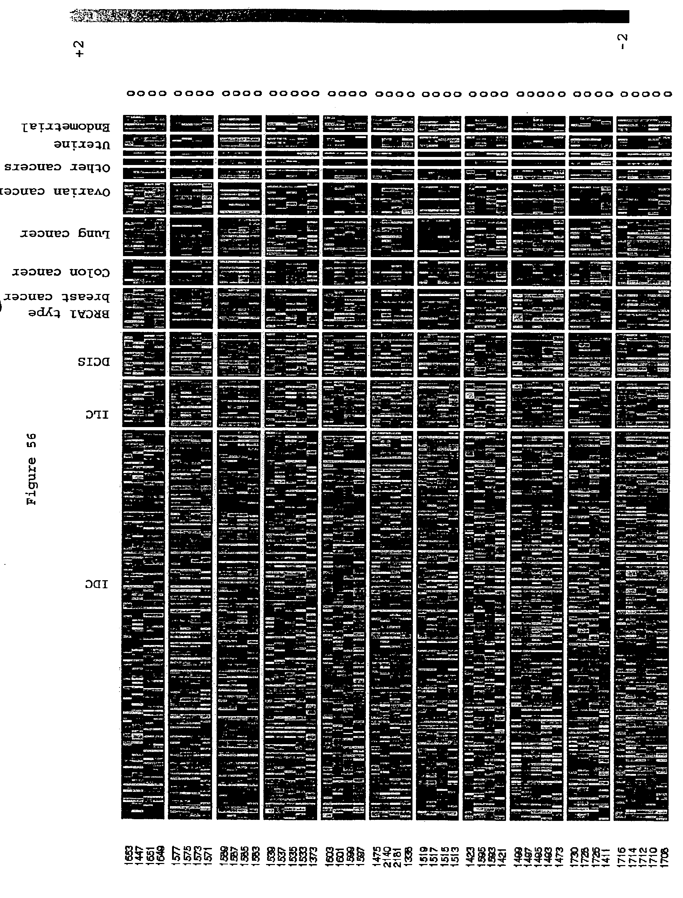

Figure 56

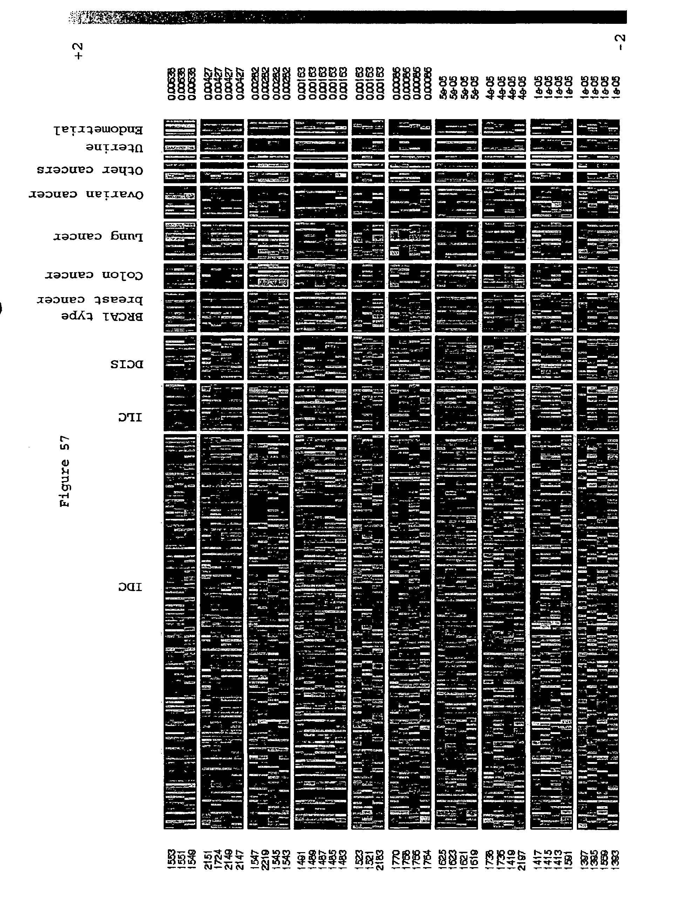

Figure 57

IDC

ILC

DCIS

BRCA1 type
breast cancer

Colon cancer

Lung cancer

Ovarian cancer

Other cancers

Uterine

Endometrial

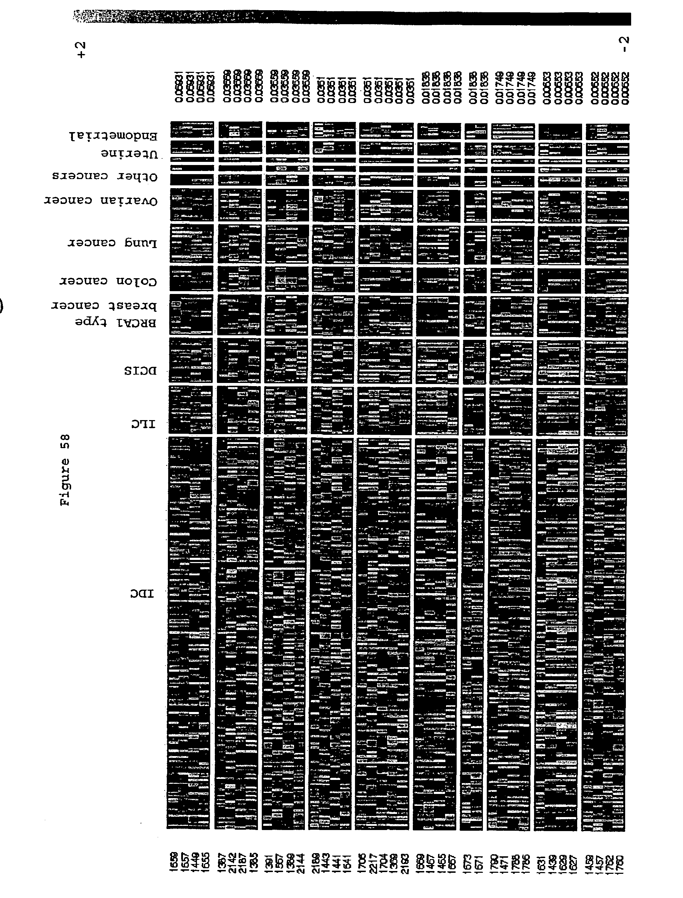

Figure 58

-2

+2

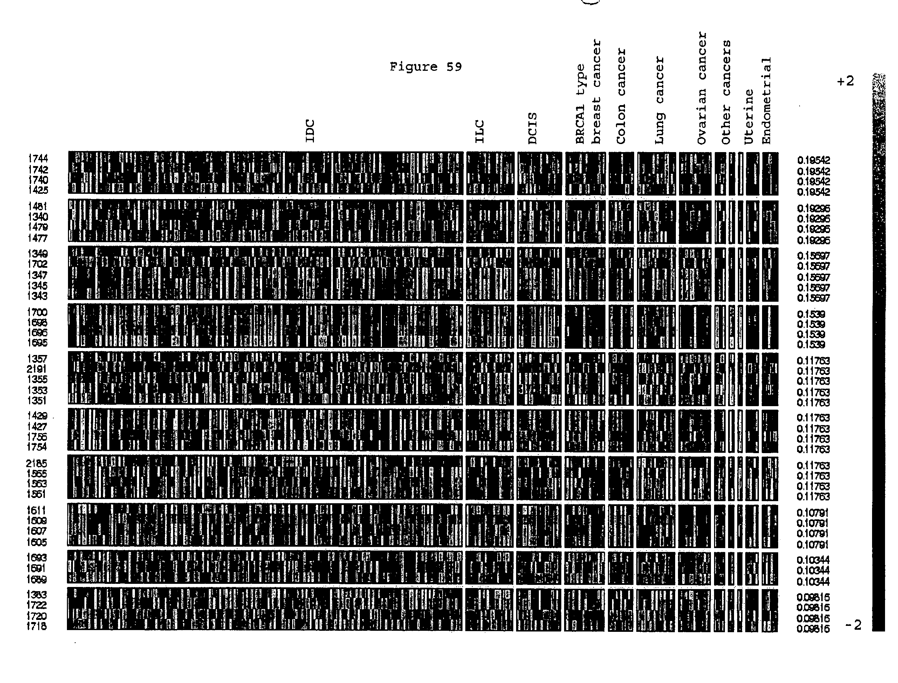

Figure 59

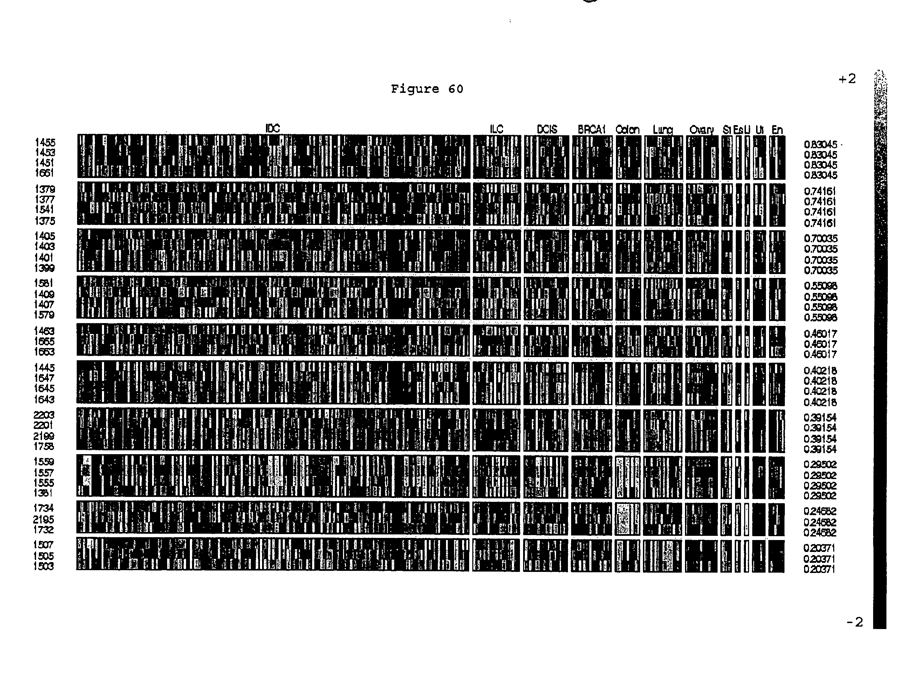

Figure 60

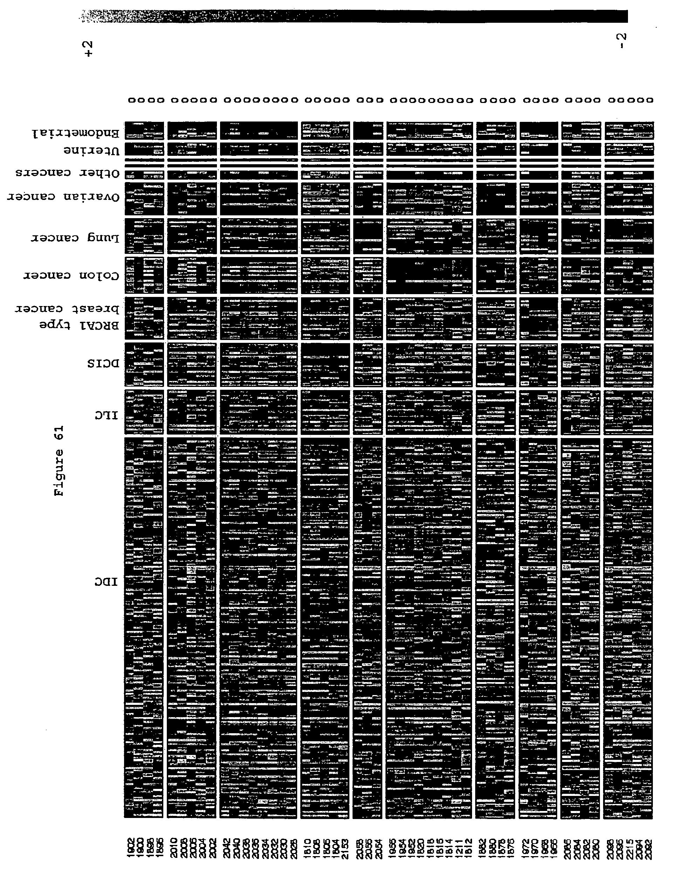

Figure 61

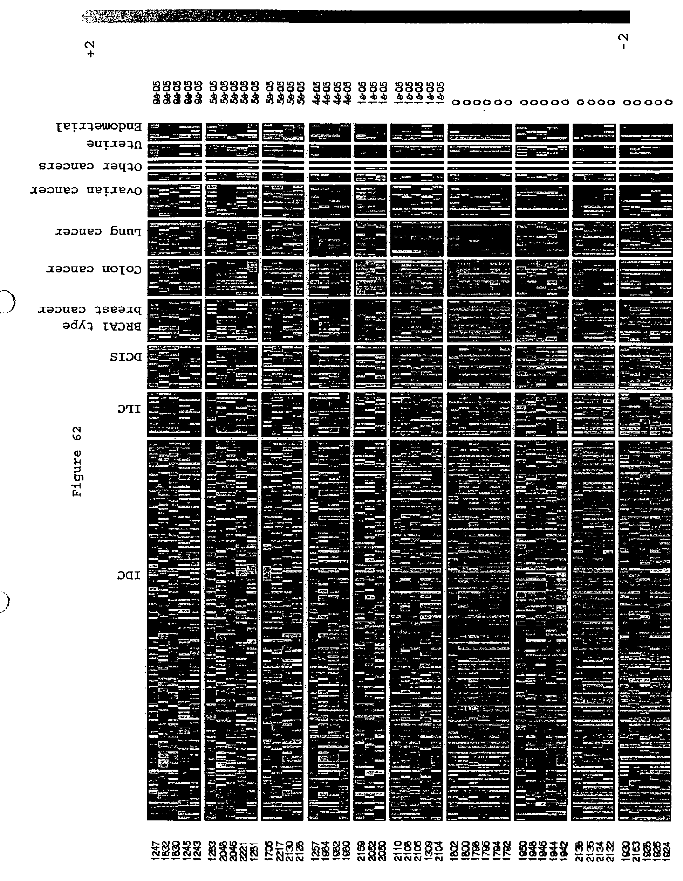

Figure 62

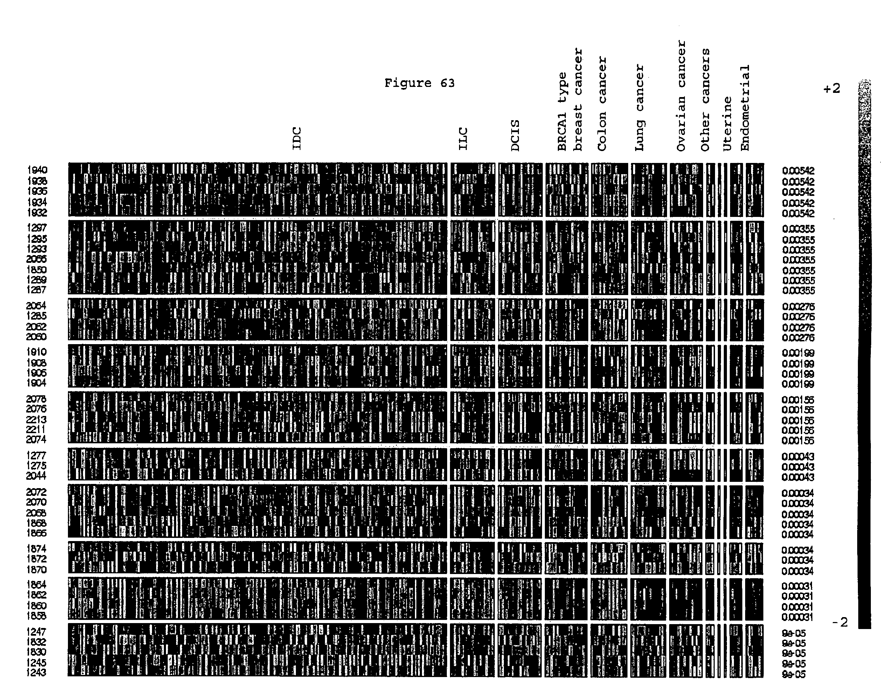

Figure 63

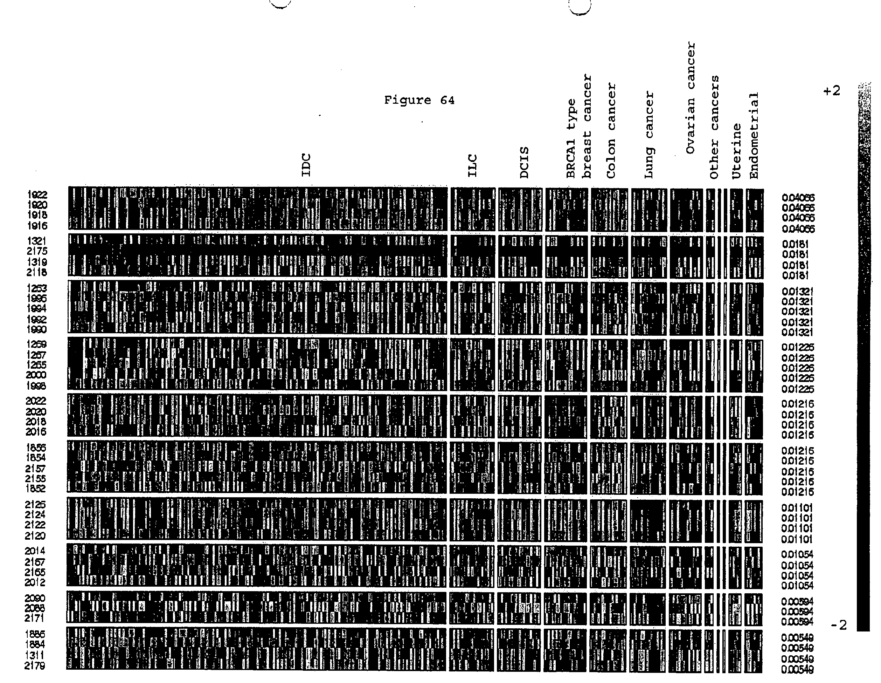

Figure 64

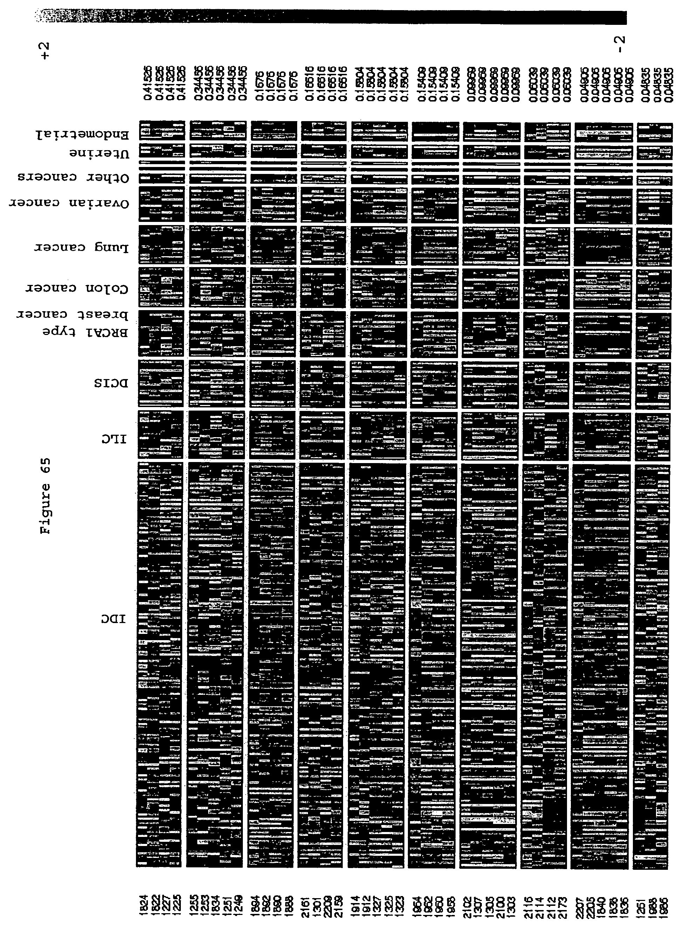

Figure 65

-2

+2

Endometrial
Uterine
Other cancers
Ovarian cancer
Lung cancer
Colon cancer
BRCA1 type breast cancer
DCIS
ILC
IDC

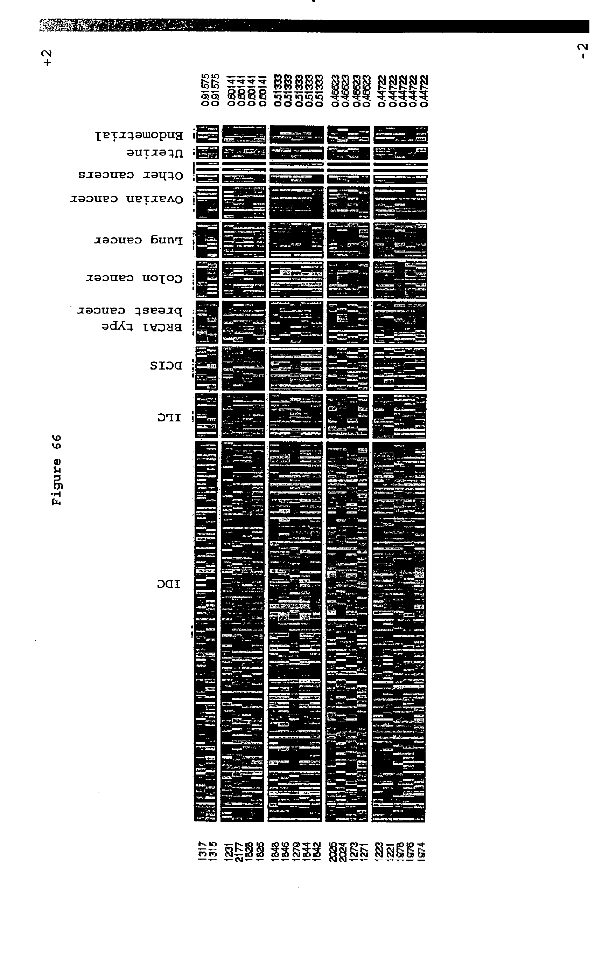

Figure 66

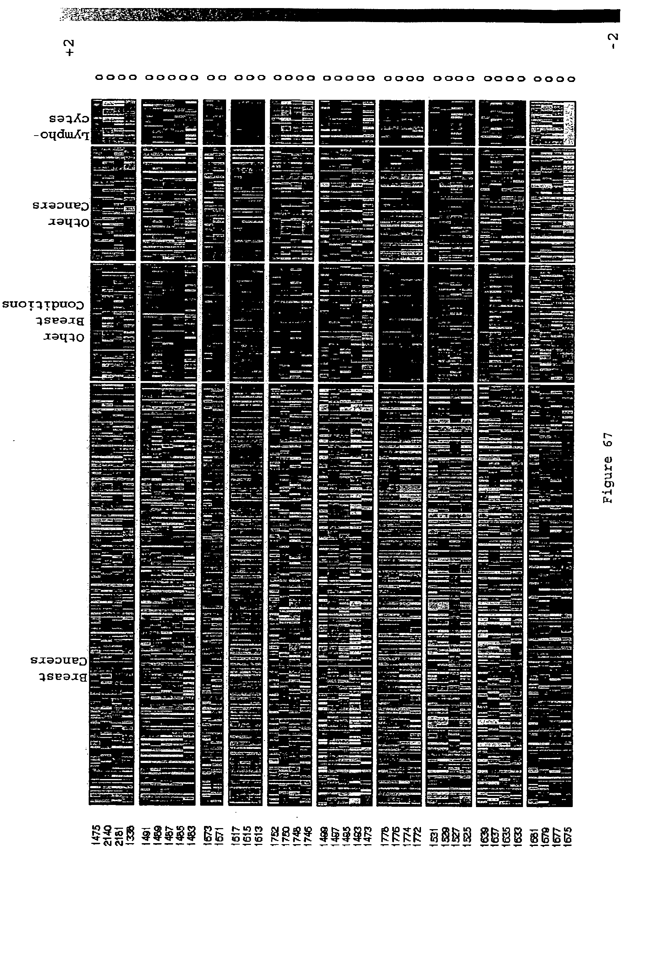

Figure 67

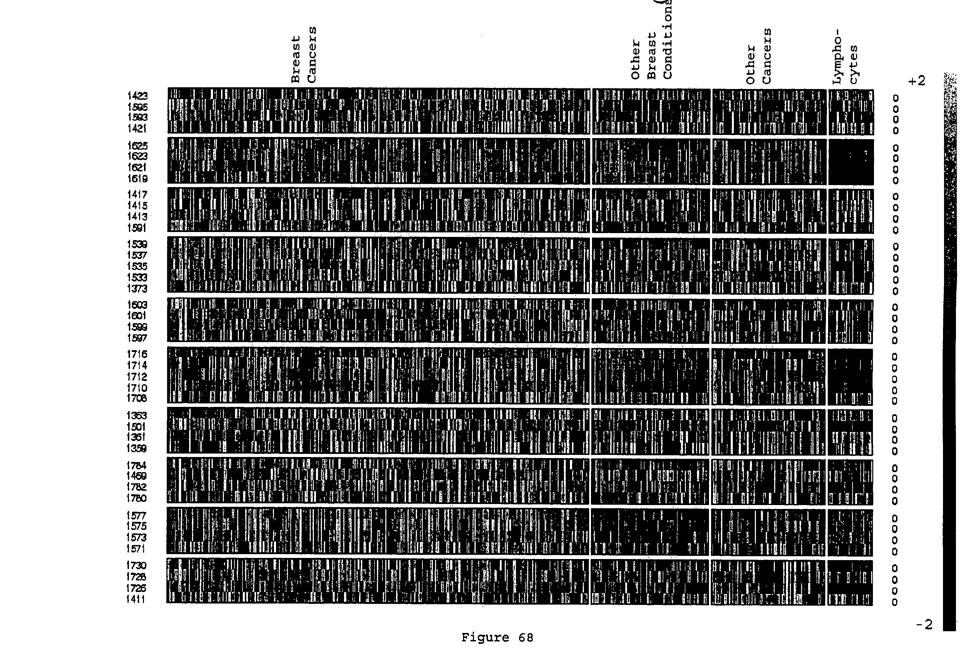

Figure 68

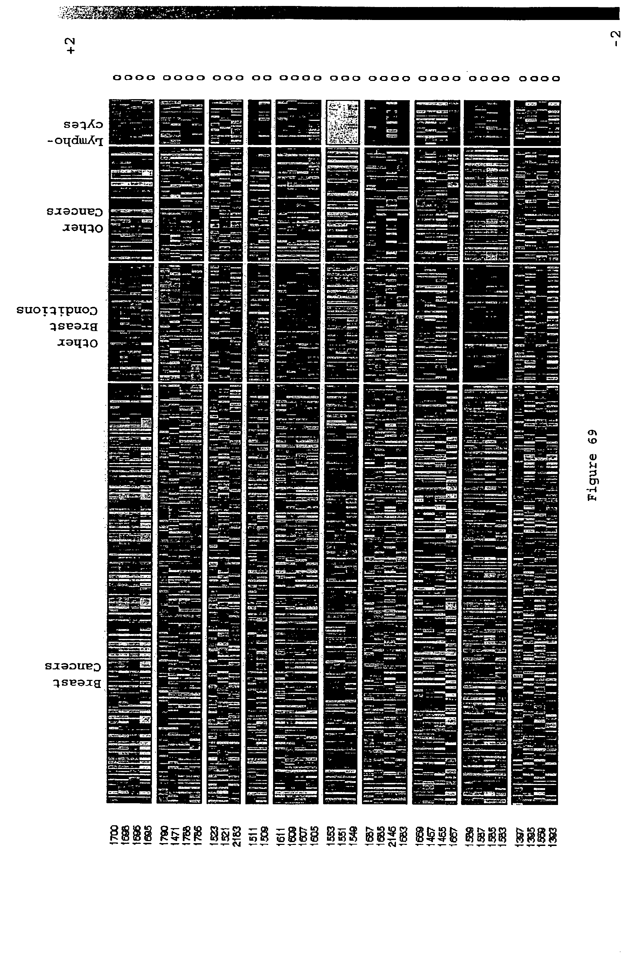

Figure 69

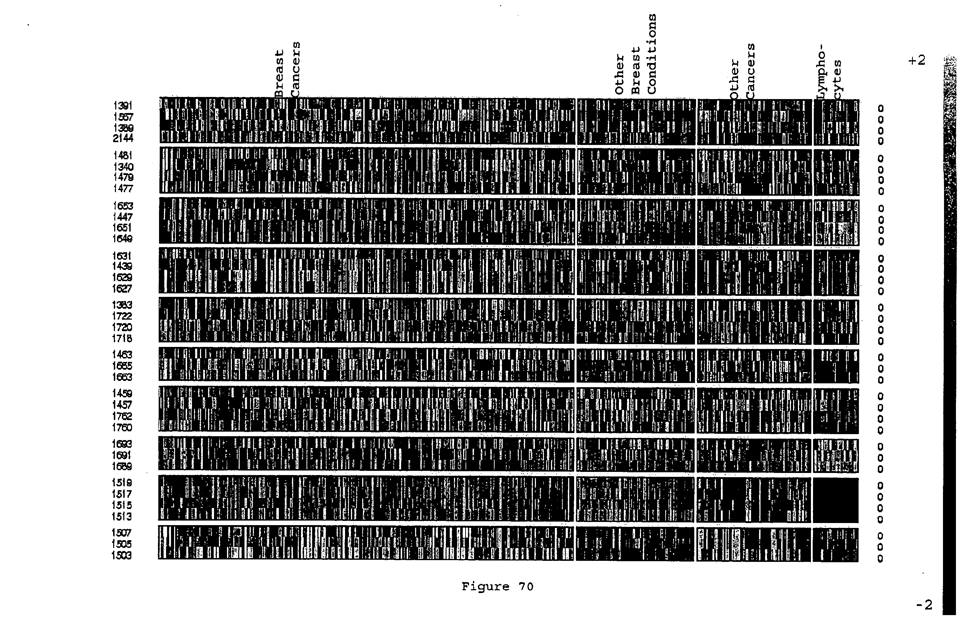

Figure 70

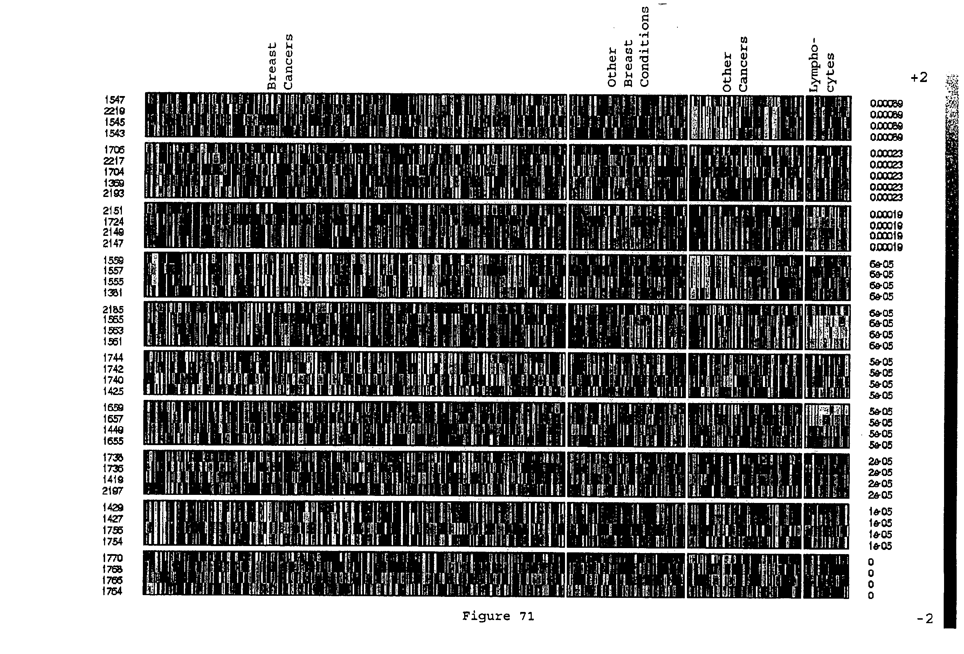

Figure 71

EP 2 302 073 A1

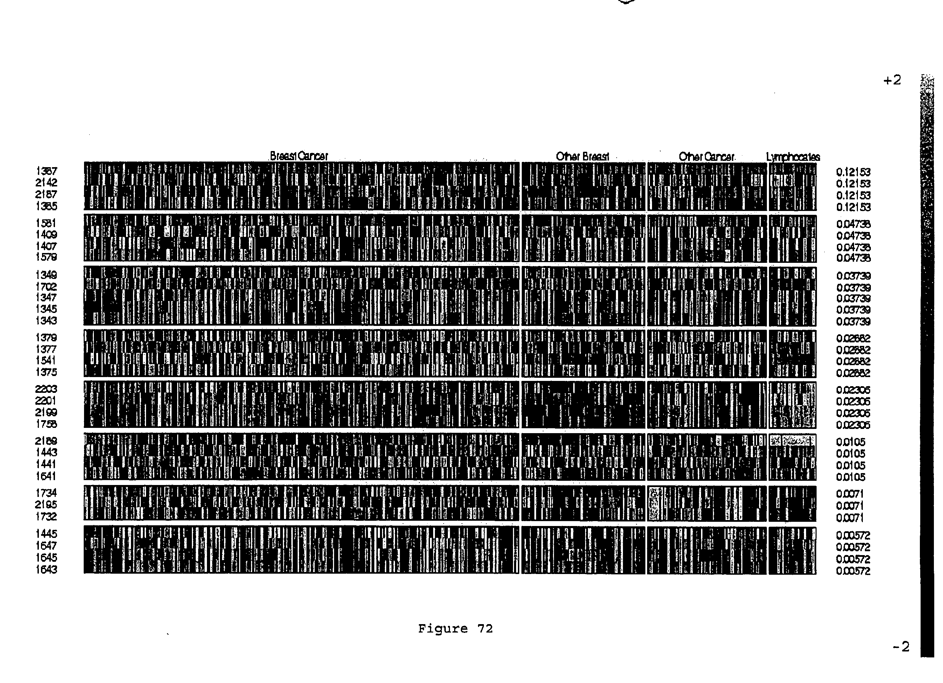

Figure 72

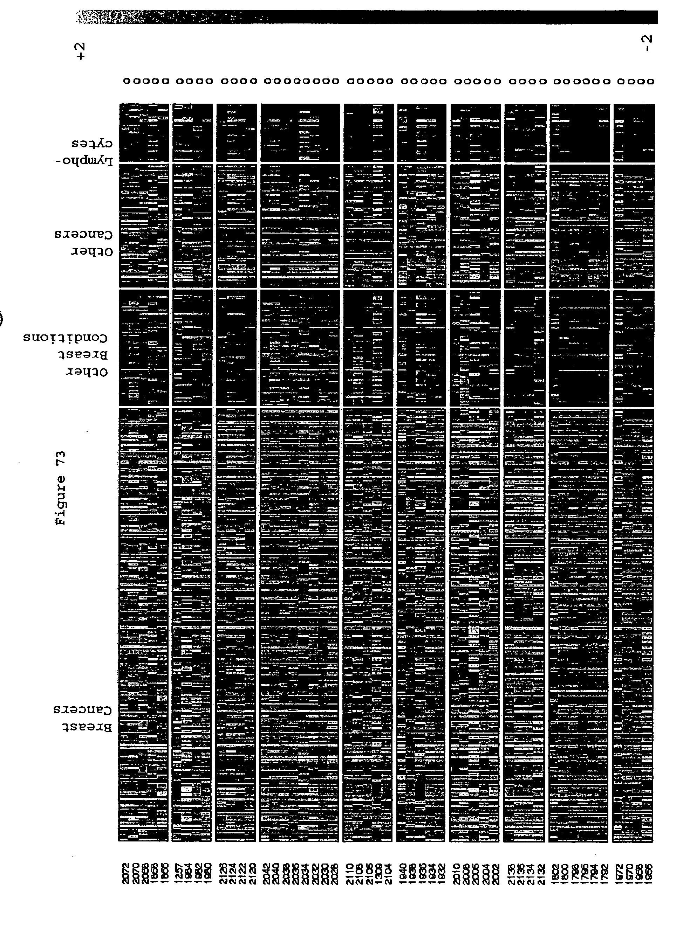

Figure 73

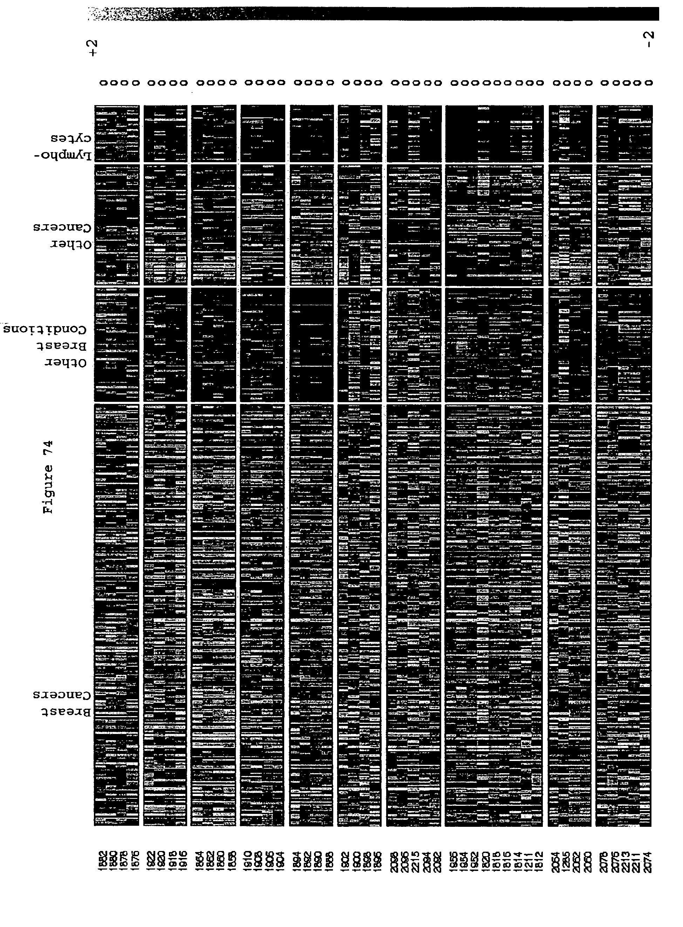

Figure 74

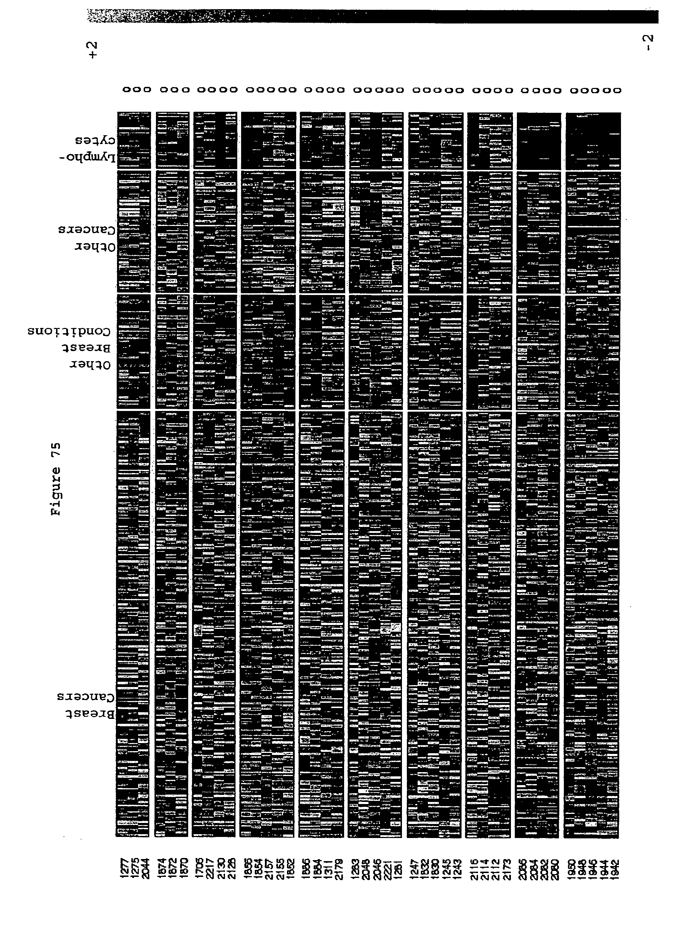

Figure 75

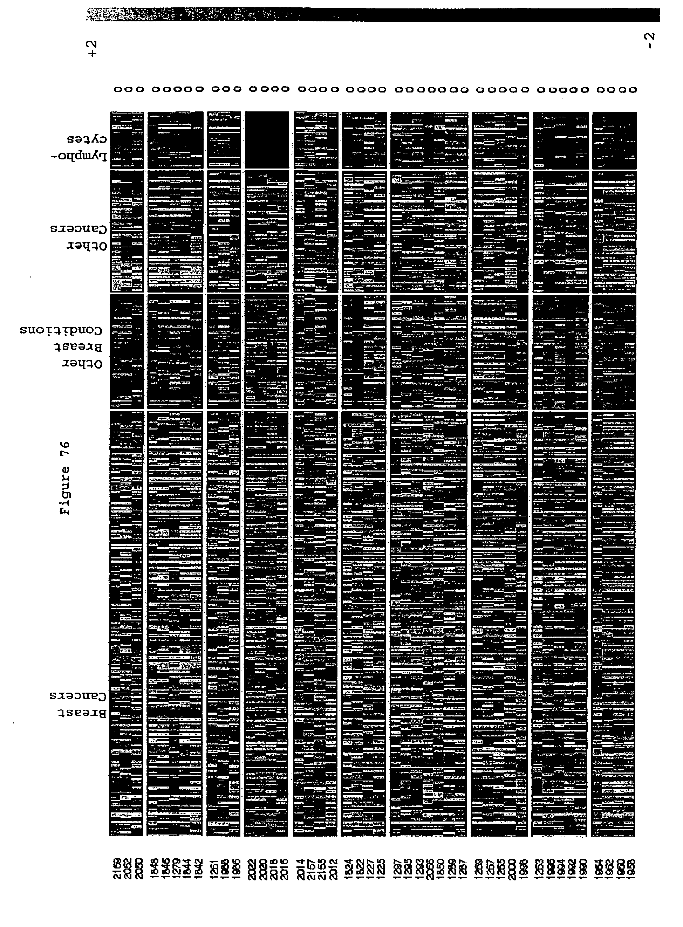

Figure 76

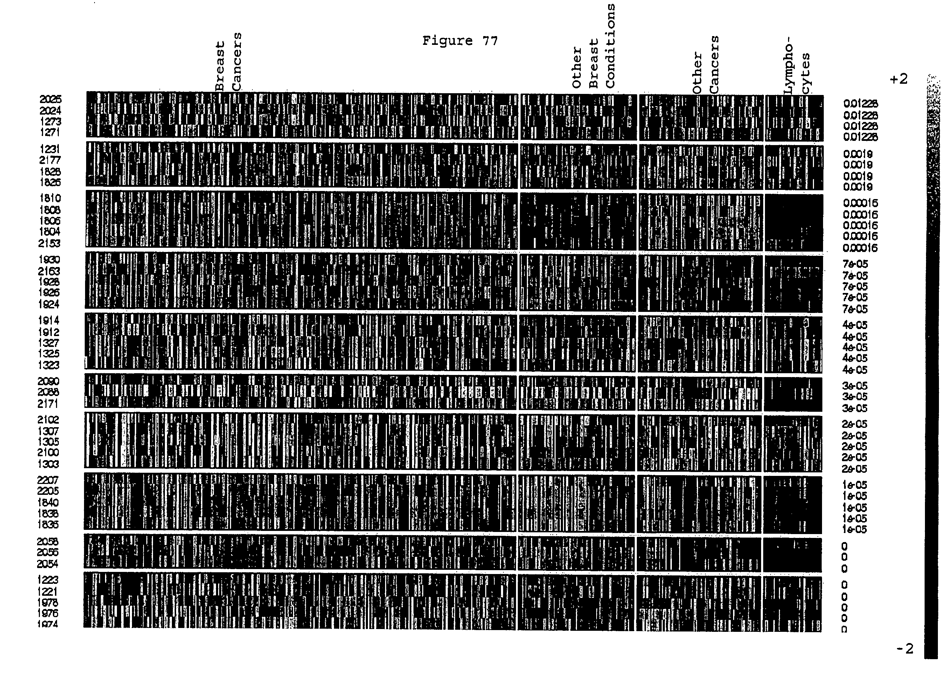

Figure 77

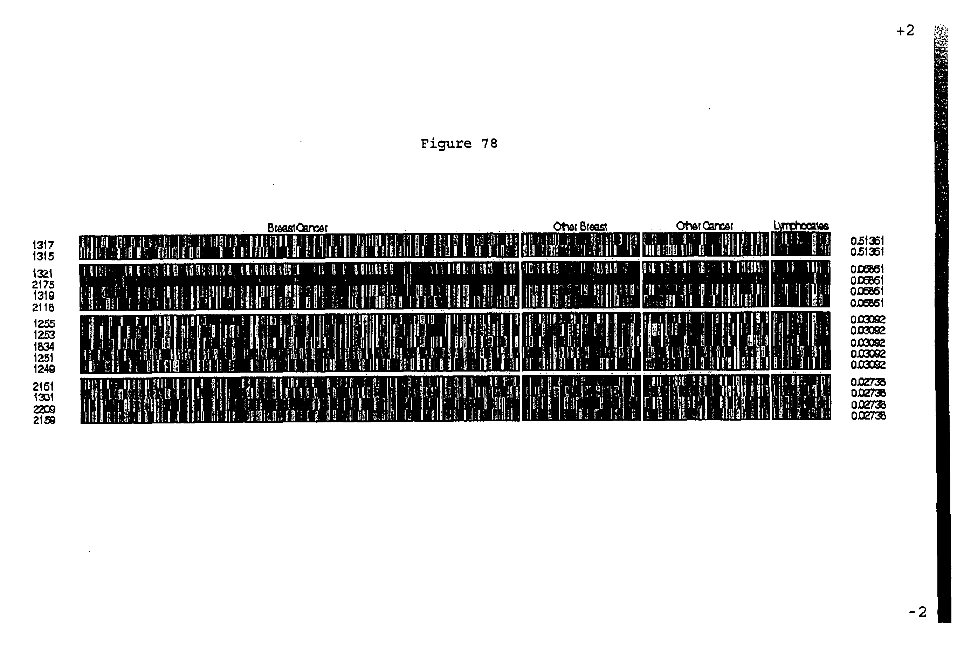

Figure 78

Breast Cancer  Other Breast  Other Cancer  Lymphocytes

1317   0.51351
1315   0.51351

1321   0.06861
2175   0.06861
1319   0.06861
2118   0.06861

1255   0.03092
1253   0.03092
1834   0.03092
1251   0.03092
1249   0.03092

2161   0.02738
1301   0.02738
2209   0.02738
2159   0.02738

+2

-2

# EP 2 302 073 A1

Europäisches
Patentamt
European
Patent Office
Office européen
des brevets

## EUROPEAN SEARCH REPORT

Application Number

EP 10 17 4924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/00927 A2 (EPIGENOMICS AG [DE]; OLEK ALEXANDER [DE]; PIEPENBROCK CHRISTIAN [DE];) 3 January 2002 (2002-01-03) * claims 1-31 * | 7-9 | INV. C12Q1/68 |
| X | PAZ M F ET AL: "Genetic unmasking of epigenetically silenced tumor suppressor genes in colon cancer cells deficient in DNA methyltransferases", HUMAN MOLECULAR GENETICS, OXFORD UNIVERSITY PRESS, SURREY, vol. 12, no. 17, 1 September 2003 (2003-09-01), pages 2209-2219, XP002543571, ISSN: 0964-6906, DOI: DOI:10.1093/HMG/DDG226 * the whole document * | 7-9 | |
| X | WO 2004/020662 A (EPIGENOMICS AG [DE]; MAIER SABINE [DE]) 11 March 2004 (2004-03-11) * the whole document * | 1-12 | |
| X | YAN P S ET AL: "Dissecting complex epigenetic alterations in breast cancer using CpG Island microarrays", CANCER RESEARCH, AMERICAN ASSOCIATION FOR CANCER RESEARCH, BALTIMORE, MD, vol. 61, no. 23, 1 December 2001 (2001-12-01), pages 8375-8380, XP002243660, ISSN: 0008-5472 * abstract * | 1-12 | TECHNICAL FIELDS SEARCHED (IPC)  C12Q |

-/--

The present search report has been drawn up for all claims

| Place of search | Date of completion of the search | Examiner |
|---|---|---|
| Munich | 26 January 2011 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

300

Europäisches
Patentamt

European
Patent Office

Office européen
des brevets

**EUROPEAN SEARCH REPORT**

Application Number

EP 10 17 4924

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FACKLER M J ET AL: "DNA METHYLATION OF RASSF1A, HIN-1, RAR-BETA, CYCLIN D2 AND TWIST IN IN SITU AND INVASIVE LOBULAR BREAST CARCINOMA", INTERNATIONAL JOURNAL OF CANCER, NEW YORK, NY, US, vol. 107, no. 6, 20 December 2003 (2003-12-20), pages 970-975, XP008038883, ISSN: 0020-7136 * abstract * | 1-12 | |
| X | MÜLLER H M ET AL: "DNA Methylation in Serum of Breast Cancer Patients: An Independent Prognostic Marker", CANCER RESEARCH, AMERICAN ASSOCIATION FOR CANCER RESEARCH, BALTIMORE, MD, vol. 63, 15 November 2003 (2003-11-15), pages 7641-7645, XP002361844, ISSN: 0008-5472 * abstract * | 1-12 | |
| A | WO 02/18632 A (EPIGENOMICS AG [DE]; OLEK ALEXANDER [DE]; PIEPENBROCK CHRISTIAN [DE];) 7 March 2002 (2002-03-07) * the whole document * * page 31, line 19 - line 35; claims 1-13,37-40 * | 1-12 | TECHNICAL FIELDS SEARCHED (IPC) |

The present search report has been drawn up for all claims

| Place of search | Date of completion of the search | Examiner |
|---|---|---|
| Munich | 26 January 2011 | Bradbrook, Derek |

EPO FORM 1503 03.82 (P04C01)

## ANNEX TO THE EUROPEAN SEARCH REPORT
## ON EUROPEAN PATENT APPLICATION NO.

EP 10 17 4924

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2011

| Patent document cited in search report | | Publication date | Patent family member(s) | | Publication date |
|---|---|---|---|---|---|
| WO 0200927 | A2 | 03-01-2002 | AU | 7637101 A | 14-01-2002 |
| | | | AU | 7752101 A | 14-01-2002 |
| | | | AU | 7970701 A | 14-01-2002 |
| | | | AU | 8391501 A | 08-01-2002 |
| | | | AU | 8391601 A | 14-01-2002 |
| | | | AU | 8757501 A | 08-01-2002 |
| | | | AU | 8757601 A | 08-01-2002 |
| | | | AU | 8961701 A | 08-01-2002 |
| | | | WO | 0202806 A2 | 10-01-2002 |
| | | | WO | 0202807 A2 | 10-01-2002 |
| | | | WO | 0200926 A2 | 03-01-2002 |
| | | | WO | 0200928 A2 | 03-01-2002 |
| | | | WO | 0202808 A2 | 10-01-2002 |
| | | | WO | 0200705 A2 | 03-01-2002 |
| | | | WO | 0202809 A2 | 10-01-2002 |
| | | | EP | 1294947 A2 | 26-03-2003 |
| | | | EP | 1297182 A2 | 02-04-2003 |
| | | | EP | 1294948 A2 | 26-03-2003 |
| | | | EP | 1294950 A2 | 26-03-2003 |
| | | | EP | 1356099 A2 | 29-10-2003 |
| | | | EP | 1294951 A2 | 26-03-2003 |
| | | | EP | 1355932 A2 | 29-10-2003 |
| | | | EP | 1297185 A2 | 02-04-2003 |
| | | | JP | 2004501666 T | 22-01-2004 |
| | | | US | 2004023230 A1 | 05-02-2004 |
| | | | US | 2003143606 A1 | 31-07-2003 |
| | | | US | 2008145839 A1 | 19-06-2008 |
| | | | US | 2004115630 A1 | 17-06-2004 |
| | | | US | 2008026396 A1 | 31-01-2008 |
| WO 2004020662 | A | 11-03-2004 | AU | 2003250989 A1 | 19-03-2004 |
| | | | EP | 1540014 A2 | 15-06-2005 |
| | | | US | 2006292564 A1 | 28-12-2006 |
| WO 0218632 | A | 07-03-2002 | AT | 382711 T | 15-01-2008 |
| | | | AU | 1218802 A | 13-03-2002 |
| | | | AU | 2002212188 B2 | 28-02-2008 |
| | | | CA | 2420840 A1 | 27-02-2003 |
| | | | DK | 1423528 T3 | 13-05-2008 |
| | | | EP | 1423528 A2 | 02-06-2004 |
| | | | ES | 2299520 T3 | 01-06-2008 |
| | | | IL | 154663 A | 24-12-2009 |
| | | | IS | 6705 A | 31-01-2003 |
| | | | JP | 2004516821 T | 10-06-2004 |
| | | | MX | PA03001834 A | 01-08-2003 |
| | | | NZ | 524229 A | 27-01-2006 |

EPO FORM P0459

For more details about this annex : see Official Journal of the European Patent Office, No. 12/82

302

## ANNEX TO THE EUROPEAN SEARCH REPORT
## ON EUROPEAN PATENT APPLICATION NO.

EP 10 17 4924

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2011

| Patent document cited in search report | Publication date | Patent family member(s) | Publication date |
|---|---|---|---|
| WO 0218632 A | | US 2004234960 A1 | 25-11-2004 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5786146 A
- WO 0026401 A1
- WO 9500669 A
- US 6251594 B
- WO 9928498 A, Olek
- WO 9746705 A
- WO 9515373 A
- US 5514758 A
- US 5565552 A
- US 5567810 A

- US 5574142 A
- US 5585481 A
- US 5587371 A
- US 5597696 A
- US 5958773 A
- US 20030013091 A
- US 09898743 B
- US 6265171 B, Herman
- US 6331393 B

**Non-patent literature cited in the description**

- Early Breast Cancer Trialists. Collaborative Group, 1998
- **Widschwendter ; Jones.** *Oncogene,* 12 August 2002, vol. 21 (35), 5462-82
- **Silva et al.** *Br J Cancer,* June 1999, vol. 80 (8), 1262-4
- **Krassenstein et al.** *Clin Cancer Res.,* 01 January 2004, vol. 10, 28-32
- **Evron et al.** *Lancet,* 28 April 2001, vol. 357 (9265), 1335-6
- **J.P. Egan.** Signal Detection Theory and ROC Analysis. Academic Press, 1975
- **Gonzalgo et al.** *Cancer Research,* 1997, vol. 57, 594-599
- **Eads et al.** *Cancer Res.,* 1999, vol. 59, 2302-2306
- **Gonzalgo ; Jones.** *Nucleic Acids Res.,* 1997, vol. 25, 2529-2531
- **Herman et al.** *Proc. Natl. Acad. Sci. USA,* 1996, vol. 93, 9821-9826
- **Xiong ; Laird.** *Nucleic Acids Res.,* 1997, vol. 25, 2532-2534
- **Toyota et al.** *Cancer Res.,* 1999, vol. 59, 2307-12
- **Sambrook et al.** Molecular Cloning, A Laboratory Manual. Cold Spring Harbor Press, 1989
- Current Protocols in Molecular Biology. John Wiley and Sons, 1995
- **Olek A et al.** A modified and improved method for bisulfite based cytosine methylation analysis. *Nucleic Acids Res.,* 1996, vol. 24, 5064-6
- **Rein, T. et al.** *Nucleic Acids Res.,* 1998, vol. 26, 2255
- **Zeschnigk M et al.** *Eur J Hum Genet.,* 1997, vol. 5, 94-98

- **Olek ; Walter.** *Nat Genet. 1997,* 1997, vol. 17, 275-6
- **Gonzalgo ; Jones.** *Nucleic Acids Res.,* 1997, vol. 25, 2529-31
- **Xiong ; Laird.** *Nucleic Acids Res.,* 1997, vol. 25, 2532-4
- **Grigg ; Clark.** *Bioessays,* 1994, vol. 16, 431-6
- **Zeschnigk M et al.** *Hum Mol Genet.,* 1997, vol. 6, 387-95
- **Feil R et al.** *Nucleic Acids Res.,* 1994, vol. 22, 695
- **Martin V et al.** *Gene,* 1995, vol. 157, 261-4
- **Frommer et al.** *Proc. Natl. Acad. Sci. USA,* 1992, vol. 89, 1827-1831
- **Sadri ; Hornsby.** *Nucl. Acids Res.,* 1996, vol. 24, 5058-5059
- **Sambrook et al.** Molecular Cloning: A Laboratory Manual. 1989
- **Belyavsky et al.** *Nucl Acid Res,* 1989, vol. 17, 2919-2932
- **Krug ; Berger.** Methods in Enzymology. Academic Press, 1987, vol. 152, 316-325
- Basic and Clinical Immunology. Appleton and Lange, 1991, 217-262
- **Milstein ; Kohler.** *Nature,* 1975, vol. 256, 495-497
- **Gulfre ; Milstein.** Methods in Enzymology: Immunochemical Techniques. Academic Press, 1981, vol. 73, 1-46
- **Krol et al.** *BioTechniques,* 1988, vol. 6, 958-976
- **Zon.** *Pharm. Res.,* 1988, vol. 5, 539-549
- *Nature Genetics,* January 1999, vol. 21

- **Yu et al.** *BioTechniques,* 1997, vol. 23, 714-720
- **Karas ; Hillenkamp.** *Anal Chem.,* 1988, vol. 60, 2299-301
- **Gut ; Beck.** *Current Innovations and Future Trends,* 1995, vol. 1, 147-57
- **Gut ; Beck.** *Nucleic Acids Res.,* 1995, vol. 23, 1367-73
- **Heid et al.** *Genome Res.,* 1996, vol. 6, 986-994
- **Sanger F. et al.** *Proc Natl Acad Sci USA,* 1977, vol. 74, 5463-5467

- **Ripley, B. D.** Pattern Recognition and Neural Networks. Cambridge University Press, 1996
- **Model F ; Koenig T ; Piepenbrock C ; Adorjan P.** Statistical process control for large scale Microarray experiments. *Bioinformatics,* 2002, vol. 18 (1), 155-163
- **Venables, W. N. ; Ripley, B. D.** Modem Applied Statistics with S-PLUS. Springer, 2002
- **Cristiannini, N. ; Shawe-Taylor, J.** An introduction to support vector machines. Cambridge University Press, 2000
- **Duda, R. O. ; Hart, P. E. ; Stork, D. G.** Pattern Classification. John Wiley & Sons, 2001